(19) Europäisches Patentamt
European Patent Office
Office européen des brevets

(11) EP 3 766 481 A1

(12) EUROPEAN PATENT APPLICATION

(43) Date of publication:
**20.01.2021 Bulletin 2021/03**

(51) Int Cl.:
*A61K 9/08* (2006.01) *A61K 9/00* (2006.01)
*A61K 39/395* (2006.01) *A61K 47/26* (2006.01)

(21) Application number: **19187338.9**

(22) Date of filing: **19.07.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Ichnos Sciences SA**
**2300 La Chaux-de-Fonds (CH)**

(72) Inventors:
- **Dubey, Sachin**
  **2300 La Chaux-de-Fonds (CH)**
- **Vadgama, Paresh**
  **2300 La Chaux-de-Fonds (CH)**
- **Strobbe, Benoit**
  **2300 La Chaux-de-Fonds (CH)**
- **Abrantes, Filipa**
  **2300 La Chaux-de-Fonds (CH)**
- **Giovannini, Roberto**
  **2300 La Chaux-de-Fonds (CH)**

(54) **LIQUID ANTIBODY FORMULATION**

(57) The present invention relates to a stable pharmaceutical formulation comprising an antibody; preferably the pharmaceutical formulation is liquid.

**FIG. 1D**

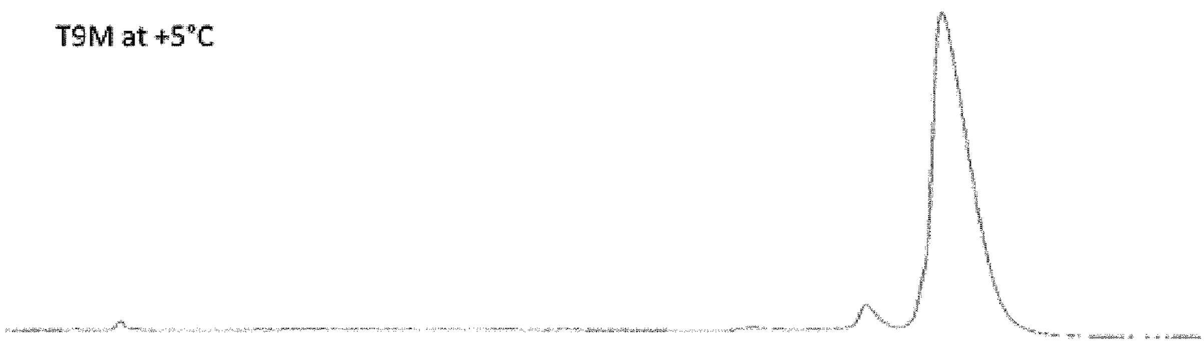


EP 3 766 481 A1

## Description

### TECHNICAL FIELD

The present invention relates to a stable pharmaceutical formulation comprising an antibody; preferably the pharmaceutical formulation is liquid.

### BACKGROUND

In the context of pharmaceutical product development, a pharmaceutical formulation refers to a mixture of an active pharmaceutical ingredient(s) and inactive ingredients, also known as excipients that are combined to produce a final drug product.

While the active pharmaceutical ingredient (API) has a pharmacological activity that contributes to the diagnosis, treatment, or prevention of a certain disease(s), the inactive ingredients have the role of contributing to product stability, biopharmaceutical profile, appearance and to patient acceptability (P. Furrer, European Pharmaceutical Review, Issue 2, 2013, April 18 2013). In particular the excipients, according to the International Pharmaceutical Excipients Council (IPEC, 1995) "are all substances contained in a dosage form other than the active substance or finished dosage form, which have been appropriately evaluated for safety and are included in a drug delivery system to either aid the processing of the drug delivery system during its manufacture, protect, support, enhance stability, bioavailability, or patient acceptability, assist in product identification, or enhance any other attributes of the overall safety and effectiveness of the drug delivery system during storage or use". Excipients therefore include solvents, diluents, buffering agents, pH-adjusting agents, surfactants, preservatives, tonicity agents, stabilizing agents etc.

When developing a drug product, the choice of the pharmaceutical formulation (API(s) concentration, excipients, excipients concentration) depends on the properties of the API(s), on the drug product presentation, on the route of administration of the drug product and on the administration dosage. Among the factors driving such choice, there is the stability of the final drug product, for which integrity of the API and safety of the formulation for the patients must be assured. At this aim, analytical tests are carried out to assess the stability of the pharmaceutical formulation when subjected to different stresses (i.e. freeze-thaw cycles, shaking) and in different conditions (i.e. short term and long term storage, storage at different temperatures). A first indication of product degradation is the visual appearance, which could disclose large aggregate formation as well as microbial contamination. A part from visible alterations, the presence/absence of sub-visible particle in parenteral products is regulated by Pharmacopeia, in fact, significant presence of sub-visible particles, whether they are product-related or foreign, may lead to potential immunogenicity and may compromise the efficacy of the drug product as well as its safety (A. McAuley et al. BioPharm International Volume 22, Issue 6, Jun 01, 2009).

Different drug product types have different characteristics that affect their stability (M. Rios, BioProcess Int., May 12, 2015), therefore additional appropriate stability tests needs to be adapted to the product. For instance, when considering biological therapeutic products, such as therapeutic antibodies, the stability of the antibody can be determined by assays that show the consistency in protein concentration, the presence/absence of aggregates, fragments, or charge variants and by assays that confirm the capability of the antibody to efficiently bind its target.

For the production of therapeutic antibodies, the development of stable pharmaceutical formulations is necessary to obtain an effective and stable drug products, therefore optimizing antibody pharmaceutical formulations, and in parallel performing exhaustive stability tests, is a primary need of the pharmaceutical industry.

### SUMMARY

The present invention relates to a stable liquid pharmaceutical formulation comprising an anti-OX40 antagonist antibody or fragment thereof, a buffer and one or more stabilizing or tonicity agents.

In one aspect of the present invention, the disclosed pharmaceutical formulation comprises an anti-OX40 antagonist antibody or fragment thereof that is present within said pharmaceutical formulation at a concentration between about 100 mg/mL and about 200 mg/mL.

In another aspect the pharmaceutical formulation has a pH between about 5.0 and about 7.0. In a more particular aspect, the pharmaceutical formulation has a pH of about 6.0 $\pm$ 0.5.

In one embodiment the buffer of the pharmaceutical formulation is selected from the group comprising acetate, histidine-HCl, citrate and phosphate, wherein said buffer is present within said pharmaceutical formulation at concentration between about 1 mM and about 50 mM. In a more particular embodiment the buffer is histidine-HCl or citrate, present within said pharmaceutical formulation at a concentration of about 25 mM.

In another embodiment, the pharmaceutical formulation comprises a stabilizing or tonicity agent selected from the group comprising sodium chloride, calcium chloride, mannitol, sucrose, polyethylene glycol 400, sorbitol, trehalose, EDTA, glycine, arginine, arginine-HCl, glutamine, glutathione, methionine, cysteine, proline, lysine, lysine-HCl, Polysorbate 20, Polysorbate 40 and Polysorbate 80, Poloxamer, Poloxamer 188, Poloxamer 407.

In a particular embodiment, the stabilizing or tonicity agent is sodium chloride present within said formulation at a concentration between about 100 mM and about 200 mM; and/or arginine-HCl present with said formulation at a concentration between about 100 mM and about 200 mM; and/or proline present within said formulation at a concentration between about 100 mM and about 200 mM; and/or lysine-HCl present within said formulation at a concentration between about 100 mM and about 200 mM; and/or mannitol present within said formulation at a concentration between about 0.5% and about 5%; and/or sucrose present within said formulation at a concentration between about 5% and about 10%; and/or methionine present within said formulation at a concentration between about 1 mM and about 20 mM, and/or Polysorbate 80 present within said formulation at a concentration between about 0.01% and about 0.15%.

In a particular embodiment the pharmaceutical formulation comprises an anti-OX40 antagonist antibody or fragment thereof present within said pharmaceutical formulation at a concentration of about 150 mg/mL, an histidine-HCl buffer present within said pharmaceutical formulation at a concentration of about 25 mM, arginine-HCl or sodium chloride present within said pharmaceutical formulation at a concentration of about 150 mM, optionally methionine present within said pharmaceutical formulation at a concentration of about 10 mM, and Polysorbate 80 present within said pharmaceutical formulation at a concentration between about 0.03% and about 0.06%, and wherein said pharmaceutical formulation has pH between about 5 and about 7.

In another particular embodiment the pharmaceutical formulation comprises an anti-OX40 antagonist antibody or fragment thereof present within said pharmaceutical formulation at a concentration of about 150 mg/mL, histidine HCl buffer present within said pharmaceutical formulation at a concentration of about 25 mM, mannitol present within said pharmaceutical formulation at a concentration between about 1% and about 5% or sucrose present within said pharmaceutical formulation at a concentration of about 8.5%, optionally proline or lysine-HCl present within said pharmaceutical formulation at a concentration of about 150 mM, or arginine-HCl present within said pharmaceutical formulation at a concentration of about 50 mM, and Polysorbate 80 present within said pharmaceutical formulation at a concentration of about 0.036%, and wherein said pharmaceutical formulation has pH of about 6.

In another particular embodiment the pharmaceutical formulation comprises an anti-OX40 antagonist antibody or fragment thereof present within said pharmaceutical formulation at a concentration of about 150 mg/mL, citrate buffer present within said pharmaceutical formulation at a concentration of about 25 mM, sodium chloride or arginine-HCl present within said pharmaceutical formulation at a concentration of about 150 mM, and Polysorbate 80 present within said pharmaceutical formulation at a concentration of about 0.036%, and wherein said pharmaceutical formulation has pH of about 6.

In a more particular embodiment the pharmaceutical formulation comprises an anti-OX40 antagonist antibody or fragment thereof present within said pharmaceutical formulation at a concentration of about 150 mg/mL, an histidine-HCl buffer present within said pharmaceutical formulation at a concentration of about 25 mM, proline or sodium chloride present within said pharmaceutical formulation at a concentration of about 150 mM, or arginine-HCl present within said pharmaceutical formulation at a concentration between about 50 mM and about 150 mM, optionally methionine present within said pharmaceutical formulation at a concentration of about 10 mM or mannitol present within said pharmaceutical formulation at a concentration between about 2% and about 3.5%, and Polysorbate 80 present within said pharmaceutical formulation at a concentration between about 0.03% and about 0.06%, wherin said pharmaceutical formulation has pH between about 5 and about 7, and wherein said pharmaceutical formulation is stable for at least about 6 months at a temperature of about 25°C.

In another more particular embodiment the pharmaceutical formulation comprises an anti-OX40 antagonist antibody or fragment thereof present within said pharmaceutical formulation at a concentration of about 150 mg/mL, histidine HCl buffer present within said pharmaceutical formulation at a concentration of about 25 mM, arginine-HCl or sodium chloride present within said pharmaceutical formulation at a concentration of about 150 mM and Polysorbate 80 present within said pharmaceutical formulation at a concentration of about 0.036%, wherein said pharmaceutical formulation has pH of about 6 and wherein said pharmaceutical formulation is stable for at least about 18 months at a temperature of about 5°C.

The present invention also relates to a pharmaceutical formulation comprised within a prefilled syringe which comprises a glass barrel characterized by a staked needle having gauge between about 25G and about 30G and a siliconization level between about 0.2mg and about 1 mg per prefilled syringe, and a rubber stopper. In a particular embodiment, the pharmaceutical formulation is comprised within a prefilled syringe which comprises a glass barrel characterized by a staked needle having gauge of about 27G and a siliconization level between about 0.3 mg and about 0.7 mg per prefilled syringe, and a halobutyl stopper.

In one aspect of the present invention, the anti-OX40 antagonist antibody or fragment thereof is a humanized monoclonal anti-OX40 antagonist antibody or fragment thereof.

The current invention also relates to a pharmaceutical formulation for use in the treatment of an OX40-mediated disorders.

In a particular embodiment, a therapeutically effective amount of the pharmaceutical formulation according to the present invention is administrated subcutaneously using a prefilled syringe.

The current invention also discloses a method of manufacturing the pharmaceutical formulation according to the present invention, and/or a method of manufacturing a vial or a prefilled syringe containing said pharmaceutical formulation according to the present invention.

Also disclosed herein is a method to test said pharmaceutical formulation to any assay for stability determination.

The present invention also relates to an article of manufacture comprising: (a) a container which holds the prefilled syringe and (b) instructions for using said prefilled syringe.

The present invention related to a liquid pharmaceutical formulation comprising an anti-OX40 antagonist antibody or fragment thereof, a buffer and one or more stability or tonicity agents.

As used herein, the following terms have the following meanings: "a", "an", and "the" as used herein refers to both singular and plural unless the context clearly dictates otherwise.

Unless otherwise defined, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures utilized in connection with, and techniques of cell and tissue culture, molecular biology, and protein and oligo- or polynucleotide chemistry, laboratory procedures and techniques of analytical chemistry, synthetic organic chemistry, and medicinal and pharmaceutical chemistry described herein are those well-known and commonly used in the art.

Temperatures above 0°C are expressed in the present invention either by preceding the temperature value by "+" or not, e.g. 25°C and +25°C can be used interchangeably according to the present invention.

A "liquid" formulation is one that has been prepared in a liquid format. Such a formulation may be suitable for direct administration to a subject or, alternatively, can be packaged for storage either in a liquid form, in a frozen state or in a dried form (e.g. lyophilized) for later reconstitution into a liquid form or other forms suitable for administration to a subject.

The term "buffer" as used herein refers to a buffered solution that resists changes in pH by the action of its acid-base conjugate components. A buffer of this invention has a pH in the range from about 5.0 to about 7.0; and preferably is 6.0±0.5. Examples of buffers that can control the pH in this range include acetate (e.g. sodium acetate), succinate (such as sodium succinate), gluconate, amino acids, such as histidine (e.g. histidine-HCl), citrate, phosphate, other organic acid buffer, their salts and combinations of buffers. In one embodiment of the present invention the buffer is present within the pharmaceutical formulation at concentration between about 1 mM and about 100 mM; in a more specific embodiment the concentration of the buffer is between about 5 mM and about 50 mM; preferably the concentration of the buffer is about 25 mM. In another aspect of the present invention, the concentration of the buffer is at least about 1 mM, at least about 5 mM, at least about 10 mM, at least about 20 mM, at least about 25 mM, at least about 30 mM, at least about 40 mM, at least about 50 mM, at least about 60 mM, at least about 70 mM, at least about 80 mM, at least about 90 mM, at least about 100 mM. The present invention also includes a buffer with a concentration at any intermediate value of the above said values. In a particular embodiment of the present invention, the buffer is Histidine, e.g. histidine-HCl, present within the pharmaceutical formulation at a concentration of about 25 mM; in another particular embodiment the buffer is citrate, present within the pharmaceutical formulation at a concentration of about 25 mM.

A stabilizing or tonicity agent may be added to the formulation to stabilize the protein in the lyophilized form. Said stabilizing or tonicity agent is selected from the group comprising sodium acetate, sodium bicarbonate, sodium carbonate, sodium chloride (NaCl), potassium acetate, potassium bicarbonate, potassium carbonate, potassium chloride, calcium chloride (CaCl2) sugars such as sucrose, glucose and trehalose, polyols such as mannitol, maltitol, sorbitol, xylitol, erythritol, and isomalt, polyethylene glycol, such as PEG400, Ethylenediaminetetraacetic acid (EDTA), amino acids such as histidine (e.g. histidine-HCl), arginine (e.g. arginine hydrochloride) and glycine, methionine, proline, lysine (e.g. lysine-HCl), glutamic acid, glutamine, cysteine, amines, glutathione, cyclodextrin, such as such as Hydroxypropyl β-cyclodextrin (HPBCD), Hydroxypropyl-sulfobutyl β-cyclodextrin (HPSBCD), Sulfobutylether β-cyclodextrin (SBECD), β-cyclodextrin (BetaCD), α-cyclodextrin (Alpha CD) and γ-cyclodextrin (Gamm CD) and surfactants. Non limiting examples of a typical surfactant include: non- ionic surfactants (HLB 6 to 18) such as sorbitan fatty acid esters (e.g. sorbitan monocaprylate, sorbitan monolaurate, sorbitan monopalmitate), glycerine fatty acid esters (e.g. glycerine monocaprylate, glycerine monomyristate, glycerine monostearate), poly glycerine fatty acid esters (e.g. decaglyceryl monostearate, decaglyceryl distearate, decaglyceryl monolinoleate), polyoxyethylene sorbitan fatty acid esters (e.g. polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, polyoxyethylene sorbitan tristearate), polyoxyethylene sorbitol fatty acid esters (e.g. polyoxyethylene sorbitol tetrastearate, polyoxyethylene sorbitol tetraoleate), polyoxyethylene glycerine fatty acid esters (e.g. polyoxyethylene glyceryl monostearate), polyethylene glycol fatty acid esters (e.g. polyethylene glycol distearate), polyoxyethylene alkyl ethers (e.g. polyoxyethylene lauryl ether), polyoxy ethylene polyoxypropylene alkyl ethers (e.g. polyoxyethylene polyoxypropylene glycol ether, polyoxyethylene polyoxypropylene propyl ether, polyoxyethylene polyoxypropylene cetyl ether), polyoxyethylene alkylphenyl ethers (e.g. polyoxyethylene nonylphenyl ether), polyoxyethylene hydrogenated castor oils (e.g. polyoxyethylene castor oil, polyoxyethylene hydrogenated castor oil), polyoxyethylene beeswax derivatives (e.g. polyoxyethylene sorbitol beeswax), polyoxyethylene lanolin derivatives (e.g. polyoxyethylene lanolin), and polyoxyethylene fatty acid amides (e.g. polyoxyethylene stearyl amide); anionic surfactants such as Cio-Cis alkyl sulfates salts (e.g. sodium cetyl sulfate, sodium lauryl sulfate, sodium oleyl sulfate), polyoxyethylene Cio-Cis alkyl ether sulfates salts with an average of 2 - 4 moles of ethylene oxide (e.g. sodium polyoxyethylene lauryl sulfate), and Cs-Cis alkyl sulfosuccmate ester salts (e.g. sodium lauryl sulfosuccmate ester); natural surfactants such as lecithin, glycerophospho lipid, sphingophospho lipids (e.g. sphingomyelin) and sucrose esters of C 12-C 18f atty acids; Poloxamers such as Poloxamer 188, Poloxamer 407, Poloxamer 124, Poloxamer 237, Poloxamer 338; salts and combinations of the above cited components .

In a certain aspect of the present invention, the stabilizing or tonicity agent is present within the pharmaceutical formulation at a concentration between about 0.001 mg/mL and about 300 mg/mL, i.e. between about 50 mg/mL and 100 mg/mL, between about 70 mg/mL and 200 mg/mL, between about 5 mg/mL and 50 mg/mL, between about 1 mg/mL and 20 mg/mL, between about 0.1 mg/mL and 10 mg/mL, between about 0.001 mg/mL and 0.1 mg/mL. In another aspect of the present invention, the stabilizing or tonicity agent is present within the pharmaceutical formulation at a concentration between about 1 mM and about 300 mM, i.e. between about 5 mM and 200 mM, specifically about 10 mM, or about 50 mM, or about 100 mM, or about 150 mM, or about 200 mM. In another aspect of the present invention, the stabilizing or tonicity agent is present within the pharmaceutical formulation at a concentration between about 0.001% and about 15%, i.e. between about 0.01% and about 5% or between about 0.03% and about 3%. In a particular aspect, the pharmaceutical formulation of the present invention comprises one or more stabilizing or tonicity agent(s) selected form the group comprising sodium chloride, arginine-HCl, proline, mannitol, lysine-HCl, sucrose, methionine and a surfactant. In a more particular aspect the pharmaceutical formulation of the present invention comprises NaCl present within the pharmaceutical formulation at a concentration between about 100 mM and about 200 mM, specifically between about 130 mM and about 170 mM, even more specifically NaCl is present within the pharmaceutical formulation at a concentration of about 150 mM. In another particular aspect the pharmaceutical formulation of the present invention comprises arginine-HCl present within the pharmaceutical formulation at a concentration between about 100 mM and about 200 mM, specifically between about 130 mM and about 170 mM, even more specifically arginine-HCl is present within the pharmaceutical formulation at a concentration of about 150 mM. In another particular aspect the pharmaceutical formulation of the present invention comprises proline present within the pharmaceutical formulation at a concentration between about 100 mM and about 200 mM, specifically between about 130 mM and about 170 mM, even more specifically proline is present within the pharmaceutical formulation at a concentration of about 150 mM. In another particular aspect the pharmaceutical formulation of the present invention comprises mannitol present within the pharmaceutical formulation at a concentration between about 0.5% and about 10%, specifically between about 1% and about 5%, even more specifically mannitol is present within the pharmaceutical formulation at a concentration of selected from the group comprising about 1%, about 2%, about 2.5%, about 3%, about 4%, about 4.5%. In another particular aspect the pharmaceutical formulation of the present invention comprises lysine-HCl present within the pharmaceutical formulation at a concentration between about 100 mM and about 200 mM, specifically between about 130 mM and about 170 mM, even more specifically lysine-HCl is present within the pharmaceutical formulation at a concentration of about 150 mM. In another particular aspect the pharmaceutical formulation of the present invention comprises sucrose present within the pharmaceutical formulation at a concentration between about 1% and about 15%, specifically between about 5% and about 10%, even more specifically sucrose is present within the pharmaceutical formulation at a concentration of about 8.5%. In another particular aspect the pharmaceutical formulation of the present invention comprises methionine present within the pharmaceutical formulation at a concentration between about 1 mM and about 50 mM, specifically between about 5 mM and about 25 mM, even more specifically methionine is present within the pharmaceutical formulation at a concentration of about 10 mM. In another aspect the pharmaceutical formulation of the present invention comprises a surfactant present within the pharmaceutical formulation at a concentration between about 0.001% and about 1%, specifically between about 0.005% and 0.5%, more specifically between about 0.01% and 0.1%, even more specifically the surfactant is present within the pharmaceutical formulation at a concentration selected from the group comprising about 0.03%, about 0.04%, about 0.05%, and about 0.1%. Preferably, the surfactant is selected from polyoxyethylene sorbitan fatty acid esters. Particularly preferably the surfactant is Polysorbate 20, 21, 40, 60, 65, 80, 81 and 85, most preferably Polysorbate 80. Polysorbate 80 is also known by the brand name Tween 80™ (ICI Americas, Inc.). In a specific embodiment of the present invention, the surfactant is Polysorbate 80, present within said pharmaceutical formulation at a concentration of about 0.036%, or about 0.054%, or about 0.1%. The present invention also includes a stabilizing or tonicity agent at any intermediate value of the above said values.

The term "antibody" as referred to herein includes whole antibodies and any antigen binding fragments or single chains thereof. An "antibody" refers to a glycoprotein comprising at least two heavy (H) chains and two light (L) chains inter-connected by disulfide bonds, or an antigen binding fragment thereof. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR) with are hypervariable in sequence and/or involved in antigen recognition and/or usually form structurally defined loops, interspersed with regions that are more conserved, termed framework regions (FR or FW). Each VH and VL is composed of three CDRs and four FWs, arranged from amino- terminus to carboxy-terminus in the following order: FW1, CDR1, FW2, CDR2, FW3, CDR3, FW4. The amino acid sequences of FW1, FW2, FW3, and FW4 all together constitute the "non-CDR region" or "non-extended CDR region" of VH or VL as referred to herein.

The term "heavy chain variable framework region" as referred herein may comprise one or more (e.g., one, two, three and/or four) heavy chain framework region sequences (e.g., framework 1 (FW1), framework 2 (FW2), framework 3 (FW3) and/or framework 4 (FW4)). Preferably the heavy chain variable region framework comprises FW1, FW2 and/or FW3, more preferably FW1, FW2 and FW3. The term "light chain variable framework region" as referred herein may comprise one or more (e.g., one, two, three and/or four) light chain framework region sequences (e.g., framework 1 (FW1), framework 2 (FW2), framework 3 (FW3) and/or framework 4 (FW4)). Preferably the light chain variable region framework comprises FW1, FW2 and/or FW3, more preferably FW1, FW2 and FW3. The variable regions of the heavy and light chains contain a binding domain that interacts with an antigen. The constant regions of the antibodies may mediate the binding of the immunoglobulin to host tissues or factors, including various cells of the immune system (e.g., effector cells) and the First component (C1q) of the classical complement system.

Antibodies are grouped into classes, also referred to as isotypes, as determined genetically by the constant region. Human constant light chains are classified as kappa (CK) and lambda (CX) light chains. Heavy chains are classified as mu ($\mu$), delta ($\delta$), gamma ($\gamma$), alpha (a), or epsilon ($\varepsilon$), and define the antibody's isotype as IgM, IgD, IgG, IgA, and IgE, respectively. Thus, "isotype" as used herein is meant any of the classes and/or subclasses of immunoglobulins defined by the chemical and antigenic characteristics of their constant regions. The known human immunoglobulin isotypes are IgG1 (IGHG1), IgG2 (IGHG2), IgG3 (IGHG3), IgG4 (IGHG4), IgA1 (IGHA1), IgA2 (IGHA2), IgM (IGHM), IgD (IGHD), and IgE (IGHE). The so-called human immunoglobulin pseudo-gamma IGHGP gene represents an additional human immunoglobulin heavy constant region gene which has been sequenced but does not encode a protein due to an altered switch region (Bensmana M et al., (1988) Nucleic Acids Res. 16(7): 3108). In spite of having an altered switch region, the human immunoglobulin pseudo-gamma IGHGP gene has open reading frames for all heavy constant domains (CH1 -CH3) and hinge. All open reading frames for its heavy constant domains encode protein domains which align well with all human immunoglobulin constant domains with the predicted structural features. This additional pseudo-gamma isotype is referred herein as IgGP or IGHGP. Other pseudo immunoglobulin genes have been reported such as the human immunoglobulin heavy constant domain epsilon PI and P2 pseudo-genes (IGHEP1 and IGHEP2). The IgG class is the most commonly used for therapeutic purposes. In humans this class comprises subclasses IgG1, IgG2, IgG3 and IgG4. In mice this class comprises subclasses IgG1, IgG2a, IgG2b, IgG2c and IgG3.

The terms "antagonistic antibody" or "antagonist antibody" are used herein equivalently and include an antibody that is capable of inhibiting and/or neutralising the biological signaling activity of OX40, for example by blocking binding or substantially reducing binding of OX40 to OX40 ligand and thus inhibiting or reducing the signalisation pathway triggered by OX40 and/or inhibiting or reducing an OX40-mediated cell response like lymphocyte proliferation, cytokine expression, or lymphocyte survival.

The term "anti-OX40 antagonist antibody or fragment thereof" is used herein to indicate antibodies or antibody fragments thereof that bind to OX40 e.g. human OX40, and are capable of inhibiting and/or neutralising the biological signalling activity of OX40, for example by blocking binding or substantially reducing binding of OX40 to OX40 ligand and thus inhibiting or reducing the signalisation pathway triggered by OX40 and/or inhibiting or reducing an OX40-mediated cell response like lymphocyte proliferation, cytokine expression, or lymphocyte survival.

Antibody fragments include, but are not limited to, (i) the Fab fragment consisting of VL, VH, CL and CHI domains, including Fab' and Fab'-SH, (ii) the Fd fragment consisting of the VH and CHI domains, (iii) the Fv fragment consisting of the VL and VH domains of a single antibody; (iv) the dAb fragment (Ward ES et al., (1989) Nature, 341 : 544-546) which consists of a single variable, (v) F(ab')2 fragments, a bivalent fragment comprising two linked Fab fragments (vi) single chain Fv molecules (scFv), wherein a VH domain and a VL domain are linked by a peptide linker which allows the two domains to associate to form an antigen binding site (Bird RE et al, (1988) Science 242: 423-426; Huston JS et al, (1988) Proc. Natl. Acad. Sci. USA, 85: 5879-83), (vii) bispecific single chain Fv dimers (PCT/US92/09965), (viii) "diabodies" or "triabodies", multivalent or multispecific fragments constructed by gene fusion (Tomlinson I & Hollinger P (2000) Methods Enzymol. 326: 461-79; WO94/13804; Holliger P et al, (1993) Proc. Natl. Acad. Sci. USA, 90: 6444-48) and (ix) scFv genetically fused to the same or a different antibody (Coloma MJ & Morrison SL (1997) Nature Biotechnology, 15(2): 159-163).

In another embodiment of the present invention, the anti-OX40 antibody is present in the pharmaceutical formulation at a concentration between about 1 mg/mL and about 200 mg/mL. In a specific embodiment the anti-OX40 antibody is present in the pharmaceutical formulation at a concentration between about 1 mg/mL and 100 mg/mL, more specifically at a concentration between about 5 mg/mL and about 50 mg/mL, more specifically at a concentration of about 10 mg/mL. The present invention also includes the anti-OX40 antibody with a concentration at any value between the above cited concentrations. According to another aspect of the present invention, the anti-OX40 antibody or fragment thereof is present within the pharmaceutical formulation at a concentration between about 100 mg/ml and about 200 mg/mL, specifically at a concentration between about 130 mg/ml and 180 mg/ml, more specifically at a concentration of about 150 mg/mL. In one aspect of the present invention, the concentration of anti-OX40 antibody or fragment thereof is selected from the group of at least about 1 mg/mL, at least about 10 mg/mL, at least about 100 mg/mL, at least about 130 mg/mL, at least about 150 mg/mL, at least about 170 mg/mL, at least about 200 mg/mL. The present invention also includes concentrations of an anti-OX40 antagonist antibody or fragment thereof at any intermediate value of the above said values. In a preferred embodiment the concentration of the anti-OX40 antibody or fragment thereof is about 150 mg/mL.

In one particular embodiment of the present invention, the pharmaceutical formulation comprises an anti-OX40 antagonist antibody or fragment thereof present within said pharmaceutical formulation at a concentration of about 10 mg/mL, histidine buffer present within said pharmaceutical formulation at a concentration of about 15 mM, sodium chloride present within said pharmaceutical formulation at a concentration of about 150 mM and Polysorbate 80 present within said pharmaceutical formulation at a concentration of about 0.01%, and said pharmaceutical formulation has pH of about 6.25.

In another particular embodiment of the present invention, the pharmaceutical formulation comprises an anti-OX40 antagonist antibody or fragment thereof present within said pharmaceutical formulation at a concentration between about 150 mg/mL and about 200 mg/mL, more specifically between about 160 mg/mL and about 195 mg/mL, or between about 160 mg/mL and about 175 mg/mL, histidine buffer present within said pharmaceutical formulation at a concentration of about 25 mM, sodium chloride or arginine-HCl present within said pharmaceutical formulation at a concentration of about 150 mM, and said pharmaceutical formulation has pH or about 6.0±1.

In another particular embodiment of the present invention, the pharmaceutical formulation comprises an anti-OX40 antagonist antibody or fragment thereof present within said pharmaceutical formulation at a concentration between about 150 mg/mL and about 200 mg/mL, more specifically between about 165 mg/mL and about 181 mg/mL, histidine-HCl buffer present within said pharmaceutical formulation at a concentration of about 25 mM, arginine-HCl present within said pharmaceutical formulation at a concentration of about 150 mM, and Polysorbate 80 present within said pharmaceutical formulation at a concentration of about 0.1%, and said pharmaceutical formulation has pH or about 6.0±1.

In another embodiment the pharmaceutical formulation comprises an anti-OX40 antagonist antibody or fragment thereof present within said pharmaceutical formulation at a concentration of about 150 mg/mL, an histidine-HCl buffer present within said pharmaceutical formulation at a concentration of about 25 mM, arginine-HCl or sodium chloride present within said pharmaceutical formulation at a concentration of about 150 mM, optionally methionine present within said pharmaceutical formulation at a concentration of about 10 mM, and Polysorbate 80 present within said pharmaceutical formulation at a concentration between about 0.03% and about 0.06%, and wherein said pharmaceutical formulation has pH between about 5 and about 7.

In another embodiment the pharmaceutical formulation comprises an anti-OX40 antagonist antibody or fragment thereof present within said pharmaceutical formulation at a concentration of about 150 mg/mL, histidine HCl buffer present within said pharmaceutical formulation at a concentration of about 25 mM, mannitol present within said pharmaceutical formulation at a concentration between about 1% and about 5% or sucrose present within said pharmaceutical formulation at a concentration of about 8.5%, optionally proline or lysine-HCl present within said pharmaceutical formulation at a concentration of about 150 mM or arginine-HCl present within said pharmaceutical formulation at a concentration of about 50 mM, and Polysorbate 80 present within said pharmaceutical formulation at a concentration of about 0.036%, and wherein said pharmaceutical formulation has pH of about 6.

In another embodiment the pharmaceutical formulation comprises an anti-OX40 antagonist antibody or fragment thereof present within said pharmaceutical formulation at a concentration of about 150 mg/mL, citrate buffer present within said pharmaceutical formulation at a concentration of about 25 mM, sodium chloride or arginine-HCl present within said pharmaceutical formulation at a concentration of about 150 mM, and Polysorbate 80 present within said pharmaceutical formulation at a concentration of about 0.036%, and wherein said pharmaceutical formulation has pH of about 6.

In a more particular embodiment of the present invention, the pharmaceutical formulation comprises an anti-OX40 antagonist antibody or fragment thereof present within said pharmaceutical formulation at a concentration of about 150 mg/mL, histidine-HCl buffer present within said pharmaceutical formulation at a concentration of about 25 mM, sodium chloride present within said pharmaceutical formulation at a concentration of about 150 mM and Polysorbate 80 present within said pharmaceutical formulation at a concentration of about 0.036%, and said pharmaceutical formulation has pH of about 6.

In another more particular embodiment of the present invention, the pharmaceutical formulation comprises an anti-OX40 antagonist antibody or fragment thereof present within said pharmaceutical formulation at a concentration of about 150 mg/mL, histidine-HCl buffer present within said pharmaceutical formulation at a concentration of about 25 mM, arginine-HCl present within said pharmaceutical formulation at a concentration of about 150 mM and Polysorbate 80 present within said pharmaceutical formulation at a concentration of about 0.036%, and said pharmaceutical formulation has pH of about 6.

In another more particular embodiment of the present invention, the pharmaceutical formulation comprises an anti-OX40 antagonist antibody or fragment thereof present within said pharmaceutical formulation at a concentration of about 150 mg/mL, histidine-HCl buffer present within said pharmaceutical formulation at a concentration of about 25 mM, arginine-HCl present within said pharmaceutical formulation at a concentration of about 150 mM and Polysorbate 80 present within said pharmaceutical formulation at a concentration of about 0.036%, and said pharmaceutical formulation has pH of about 5.5.

In another more particular embodiment of the present invention, the pharmaceutical formulation comprises an anti-OX40 antagonist antibody or fragment thereof present within said pharmaceutical formulation at a concentration of about 150 mg/mL, histidine-HCl buffer present within said pharmaceutical formulation at a concentration of about 25 mM, arginine-HCl present within said pharmaceutical formulation at a concentration of about 150 mM and Polysorbate 80 present within said pharmaceutical formulation at a concentration of about 0.036%, and said pharmaceutical formulation has pH of about 6.5.

In another more particular embodiment of the present invention, the pharmaceutical formulation comprises an anti-OX40 antagonist antibody or fragment thereof present within said pharmaceutical formulation at a concentration of about 150 mg/mL, citrate buffer present within said pharmaceutical formulation at a concentration of about 25 mM, sodium chloride present within said pharmaceutical formulation at a concentration of about 150 mM and Polysorbate 80 present within said pharmaceutical formulation at a concentration of about 0.036%, and said pharmaceutical formulation has pH of about 6.

In another more particular embodiment of the present invention, the pharmaceutical formulation comprises an anti-OX40 antagonist antibody or fragment thereof present within said pharmaceutical formulation at a concentration of about 150 mg/mL, citrate buffer present within said pharmaceutical formulation at a concentration of about 25 mM, arginine-HCl present within said pharmaceutical formulation at a concentration of about 150 mM and Polysorbate 80 present within said pharmaceutical formulation at a concentration of about 0.036%, and said pharmaceutical formulation has pH of about 6.

In another more particular embodiment of the present invention, the pharmaceutical formulation comprises an anti-OX40 antagonist antibody or fragment thereof present within said pharmaceutical formulation at a concentration of about 150 mg/mL, histidine-HCl buffer present within said pharmaceutical formulation at a concentration of about 25 mM, proline present within said pharmaceutical formulation at a concentration of about 150 mM, mannitol present within said pharmaceutical formulation at a concentration of about 2.5% and Polysorbate 80 present within said pharmaceutical formulation at a concentration of about 0.036%, and said pharmaceutical formulation has pH of about 6.

In another more particular embodiment of the present invention, the pharmaceutical formulation comprises an anti-OX40 antagonist antibody or fragment thereof present within said pharmaceutical formulation at a concentration of about 150 mg/mL, histidine-HCl buffer present within said pharmaceutical formulation at a concentration of about 25 mM, lysine-HCl present within said pharmaceutical formulation at a concentration of about 150 mM, mannitol present within said pharmaceutical formulation at a concentration of about 1% and Polysorbate 80 present within said pharmaceutical formulation at a concentration of about 0.036%, and said pharmaceutical formulation has pH of about 6.

In another more particular embodiment of the present invention, the pharmaceutical formulation comprises an anti-OX40 antagonist antibody or fragment thereof present within said pharmaceutical formulation at a concentration of about 150 mg/mL, histidine-HCl buffer present within said pharmaceutical formulation at a concentration of about 25 mM, arginine-HCl present within said pharmaceutical formulation at a concentration of about 50 mM, mannitol present within said pharmaceutical formulation at a concentration of about 3% and Polysorbate 80 present within said pharmaceutical formulation at a concentration of about 0.036%, and said pharmaceutical formulation has pH of about 6.

In another more particular embodiment of the present invention, the pharmaceutical formulation comprises an anti-OX40 antagonist antibody or fragment thereof present within said pharmaceutical formulation at a concentration of about 150 mg/mL, histidine-HCl buffer present within said pharmaceutical formulation at a concentration of about 25 mM, arginine-HCl present within said pharmaceutical formulation at a concentration of about 150 mM, methionine present within said pharmaceutical formulation at a concentration of about 10 mM and Polysorbate 80 present within said pharmaceutical formulation at a concentration of about 0.036%, and said pharmaceutical formulation has pH of about 6.

In another more particular embodiment of the present invention, the pharmaceutical formulation comprises an anti-OX40 antagonist antibody or fragment thereof present within said pharmaceutical formulation at a concentration of about 150 mg/mL, histidine-HCl buffer present within said pharmaceutical formulation at a concentration of about 25 mM, mannitol present within said pharmaceutical formulation at a concentration of about 4.2% and Polysorbate 80 present within said pharmaceutical formulation at a concentration of about 0.036%, and said pharmaceutical formulation has pH of about 6.

In another more particular embodiment of the present invention, the pharmaceutical formulation comprises an anti-OX40 antagonist antibody or fragment thereof present within said pharmaceutical formulation at a concentration of about 150 mg/mL, histidine-HCl buffer present within said pharmaceutical formulation at a concentration of about 25 mM, sucrose present within said pharmaceutical formulation at a concentration of about 8.5% and Polysorbate 80 present within said pharmaceutical formulation at a concentration of about 0.036%, and said pharmaceutical formulation has pH of about 6.

In another more particular embodiment of the present invention, the pharmaceutical formulation comprises an anti-OX40 antagonist antibody or fragment thereof present within said pharmaceutical formulation at a concentration of about 150 mg/mL, histidine-HCl buffer present within said pharmaceutical formulation at a concentration of about 25 mM, arginine-HCl present within said pharmaceutical formulation at a concentration of about 150 mM and Polysorbate 80 present within said pharmaceutical formulation at a concentration of about 0.054%, and said pharmaceutical formulation has pH of about 6.

The pharmaceutical formulation according to the present invention is stable. A "stable" formulation is one in which the protein therein essentially retains its physical stability and/or chemical stability and/or biological activity upon storage. Various analytical techniques for measuring protein stability are available in the art and are reviewed for example in Peptide and Protein Drug Delivery, 247-301, Vincent Lee Ed., Marcel Dekker, Inc., New York, New York, Pubs. (1991) and Jones A (1993) Adv Drug Delivery Rev, 10: 29-90. Stability can be measured at a selected temperature for a selected time period.

Analytical tests useful to determine said stability include but are not limited to: monitoring of the visual appearance as a significant change in the appearance of sample may indicate product degradation and/or microbial contamination leading to safety risk for the patients; sub-visible particles analysis, as the presence of higher sub-visible particles in parental solutions may lead to immunogenic responses; protein content measurement (e.g. by measuring absorbance at 280 nm wavelength (A280) by UV-VIS Spectroscopy or by SoloVPE) as any significant variation from its target concentration would not provide effective dose to patients; pH measurement as changes in pH may be indicative of degradation of buffering agents and lead to protein instability; size variants monitoring (e.g. by SE-HPLC and/or by cGE reduced and non-reduced) as changes in monomeric content toward aggregates (higher size than monomer) or fragments (smaller size than monomer) is an indication of its degradation; charge variants monitoring (e.g. by cIEF) as changes in content of charged variants is an indication of its degradation; antibody potency measurement (e.g. by ELISA) as any significant change of binding property of the antibody toward its target would indicate antibody degradation. Additionally, the amino acid sequence as well as post-translational modifications (i.e. deamidation, oxidation, glycation, N-terminal variants, C-terminal variants and glycosylation site occupancy) can be verified, for instance by peptide mapping. Other characteristics of the formulation can be monitored, such as osmolarity and viscosity, as well as the protein thermal stability for instance by nano-format of Differential Scanning Fluorimetry (DSF), syringeability.

In one aspect of the present invention, the pharmaceutical formulation is stable under stresses comprising: rolling stress, storage at temperature higher than the room temperature, such as storage at +40 °C for at least 1 week; shaking such as shaking at 900 rpm for at least 24 hours, at least 48 hours, at least 72 hours at room temperature (25°C); freezing-thawing circles such as freezing at -20°C or at -80°C and thawing at about 5°C, or at about 20°C, or at about 25°C, more in particular such as 3 to 5 freeze-thaw cycles by freezing at -80 °C and thawing at +5 °C, such as 3 to 5 freeze-thaw cycles by freezing at -80 °C and thawing at +25 °C, and such as freeze-thaw cycles by freezing at -20 °C and thawing at 20°C.

According to one aspect of the present invention, the pharmaceutical formulation is stable at temperature equal to or less than about 40°C, i.e. at a temperature between about -80°C and 40°C, for at least about 1 month, or at least about 3 months, or at least about 6 months, or at least about 9 months, or at least about 12 months, or at least about 18 months, or at least about 24 months, or at least about 30 months, or at least about 36 months. The present invention also includes the disclosed pharmaceutical formulations stable at any value between the above cited temperatures, and at any time intervals between the above cited times. In one embodiment of the present invention, the pharmaceutical formulation of the present invention is stable when stored for at least about 1 month, or at least about 2 months, or at least about 3 months, or at least about 6 months, or at least about 9 months, or at least about 12 months, or at least about 18 months, or at least about 24 months, or at least about 30 months, or at least about 36 months, at a temperature of about of at least about 5°C, more specifically at a temperature of about 5°C or at a temperature of about 25°C; in a specific embodiment the pharmaceutical formulation of the present invention is stable when stored for at least about 9 month at a temperature of about 5°C or at a temperature of about 25°C, for properties comprising glycation, sequence terminal modifications, and potency. In another specific aspect, the pharmaceutical formulation according to the present invention is stable when stored for at least about 36 months at a temperature of about 5°C. In a more specific embodiment, the disclosed pharmaceutical formulation is stable at about +25$\pm$2°C for at least 1 month; in another specific embodiment, the disclosed pharmaceutical formulation is stable at about +5$\pm$3°C for at least about 1 month, preferably for at least about 3 months; in another specific embodiment, the disclosed pharmaceutical formulation is stable at about -80$\pm$20°C or about -20$\pm$5°C for at least about 1 month, or at least about 3 months, preferably for at least about 6 months. In another more specific embodiment, the disclosed pharmaceutical formulation is stable at about 40$\pm$2°C for at least about 1 month; in another specific embodiment, the disclosed pharmaceutical formulation is stable at about 25$\pm$2°C for at least about 1 month, more specifically for least about 3 months, preferably for at least about 6 months; in another specific embodiment, the disclosed pharmaceutical formulation is stable at about 5±3°C for at least about 1 month, specifically for at least about 3 months, more specifically for at least about 6 months, even more specifically for at least about 12 months, preferably for at least about 18 months, more preferably for at least about 24 months.

In a more particular embodiment the pharmaceutical formulation comprising an anti-OX40 antagonist antibody or fragment thereof present within said pharmaceutical formulation at a concentration of about 150 mg/mL, an histidine-HCl buffer present within said pharmaceutical formulation at a concentration of about 25 mM, proline or sodium chloride present within said pharmaceutical formulation at a concentration of about 150 mM, or arginine-HCl present within said pharmaceutical formulation at a concentration between about 50 mM and about 150 mM, optionally methionine present within said pharmaceutical formulation at a concentration of about 10 mM of mannitol present within said pharmaceutical formulation at a concentration between about 2% and about 3.5%, and Polysorbate 80 present within said pharmaceutical formulation at a concentration between about 0.03% and about 0.06%, and having pH between about 5 and about 7, is stable for at least about 6 months at a temperature of about 25°C or less.

In another more particular embodiment the pharmaceutical formulation comprising an anti-OX40 antagonist antibody or fragment thereof present within said pharmaceutical formulation at a concentration of about 150 mg/mL, histidine HCl buffer present within said pharmaceutical formulation at a concentration of about 25 mM, arginine-HCl or sodium chloride present within said pharmaceutical formulation at a concentration of about 150 mM and Polysorbate 80 present within said pharmaceutical formulation at a concentration of about 0.036%, and having pH of about 6 is stable for at least about 18 months at a temperature of about 5°C or less.

The liquid formulation of the present invention can be comprised within a container. For instance a container made of glass, plastic, other polymers. Non limiting examples of said container are syringes, such as pre-filled syringe, glass cartridge syringe, autoinjectors, bottles, vials, and test tubes, pens, bags such as storage bags. The liquid formulation according to the present invention can be contained in a prefilled syringe for subcutaneous injection using an autoinjectors.

In one embodiment of the present invention, the pharmaceutical formulation is comprised within a glass vial, such as a 2 mL glass vials stoppered with 13 mm injection rubber stopper or such as a 10 mL glass vial stoppered with a 20 mm injection flurotec-coated stopper.

In another embodiment of the present invention the pharmaceutical formulation is comprised within a prefilled syringe (PFS)). A PFS may be filled with a volume such as about 0.1 mL, or about 0.2 mL, or about 0.5 mL, or about 1 mL, or about 1.5 mL, or about 2 mL, or about 2.5 mL, or about 3 mL of the formulation according to the present invention. In particular, the prefilled syringe comprises a glass barrel characterized by a needle having gauge between 25 and 30G and a siliconization level between 0.2mg and 1 mg per prefilled syringe, and a halobutyl stopper; wherein the needle may be a staked needle or a needle externally mounted for instance on a Luer lock of a syringe tip. Suitable material of which the PFS is made include and are not limited to glass and plastic. In a specific embodiment about 2 mL, such as 2.1 mL of said pharmaceutical formulation is filled in 2.25 mL glass pre-fillable syringe which comprises a glass barrel characterized by a staked needle having gauge of about 27G ½" and a siliconization level between 0.3 mg and 0.7 mg per prefilled syringe, and a halobutyl stopper. Examples of commercially available pre-filled syringe barrels/stoppers are Gerresheimer, Schott, OMPI, BD NovaPure, West Pharmaceutical Services.

To determine the stability of the prefilled syringe filled with the formulation according to the present invention, the analytical tests mentioned above are applicable. Additionally, the performance of syringe (i.e. Syringeability) may be judged based on two forces namely, the force required to initiate plunger movement (break-loose force) and the force required to maintain the movement of plunger (gliding force). Any significant change in these two forces may indicate poor syringe performance which may lead to inaccuracy in dose volume and/or pain to patients during injection.

In one aspect of the present invention, the pharmaceutical formulation is expelled from the PFS in a time between 5s and 30 s, preferably in a time between 10s and 20 s, more preferably in 15 s. In another aspect of the present invention, when the pharmaceutical formulation is expelled from the PFS in a time between 10s and 20 s, the break lose force is between about 2N and about 4N and the gliding force is between about 4N and about 30N, for instance between about 4N and 13N.

According to one aspect of the present invention, the pharmaceutical formulation comprised within a 2.25 mL glass prefilled syringe is stable at temperature equal to or less than about 40°C, i.e. at a temperature between about -80°C and 40°C, for at least about 1 month, or at least about 3 months, or at least about 6 months, or at least about 9 months, or at least about 12 months, or at least about 18 months, or at least about 24 months. The present invention also includes the disclosed pharmaceutical formulation stable at any value between the above cited temperatures, and at any time intervals between the above cited times. In a more specific embodiment, the disclosed pharmaceutical formulation is stable at about 40±2°C for at least about 1 month; in another specific embodiment, the disclosed pharmaceutical formulation is stable at about 25±2°C for at least about 1 month, more specifically for least about 3 months; in another specific embodiment, the disclosed pharmaceutical formulation is stable at about 5±3°C for at least about 1 month, specifically for at least about 3 months, more specifically for at least about 6 months, even more specifically for at least about 12 months, preferably for at least about 18 months, more preferably for at least about 24 months.

In a more particular embodiment the pharmaceutical formulation comprising an anti-OX40 antagonist antibody or fragment thereof present within said pharmaceutical formulation at a concentration of about 150 mg/mL, an histidine-HCl buffer present within said pharmaceutical formulation at a concentration of about 25 mM, proline or sodium chloride present within said pharmaceutical formulation at a concentration of about 150 mM, or arginine-HCl present within said pharmaceutical formulation at a concentration between about 50 mM and about 150 mM, optionally methionine present within said pharmaceutical formulation at a concentration of about 10 mM or mannitol present within said pharmaceutical formulation at a concentration between about 2% and about 3.5%, and Polysorbate 80 present within said pharmaceutical formulation at a concentration between about 0.03% and about 0.06%, and having pH between about 5 and about 7, is comprised within a prefilled syringe and it is stable for at least about 6 months at a temperature of about 25°C or less.

In another more particular embodiment the pharmaceutical formulation comprising an anti-OX40 antagonist antibody or fragment thereof present within said pharmaceutical formulation at a concentration of about 150 mg/mL, histidine HCl buffer present within said pharmaceutical formulation at a concentration of about 25 mM, arginine-HCl or sodium chloride present within said pharmaceutical formulation at a concentration of about 150 mM and Polysorbate 80 present within said pharmaceutical formulation at a concentration of about 0.036%, and having pH of about 6, is comprised within a prefilled syringe and it is stable for at least about 18 months at a temperature of about 5°C or less.

In a particular embodiment, the anti-OX40 antibody or fragment thereof is a humanized monoclonal anti-OX40 antagonist antibody or fragment thereof.

The term "humanized antibody" or "humanized anti-OX40 antibody" as used herein includes antibodies in which CDR sequences derived from the germline of another mammalian species, such as a mouse, have been grafted onto human framework sequences. Additional framework region modifications may be made within the human framework sequences as well as within the CDR sequences derived from the germline of another mammalian species.

More specifically, the anti-OX40 antagonist antibody or fragment is GBR830 (CAS Registry Number 2126777-87-3).

In a certain embodiment, the pharmaceutical formulation of any one of the preceding claims for use in the treatment of patients suffering of an OX40-mediated disorders.

As used herein, the term "OX40-mediated disorder" includes conditions such as allergy, asthma, COPD, rheumatoid arthritis, psoriasis and diseases associated with autoimmunity and inflammation. In particular, according to the present invention, exemplary OX40 mediated disorders include infections (viral, bacterial, fungal and parasitic), endotoxic shock associated with infection, arthritis, rheumatoid arthritis, asthma, chronic obstructive pulmonary disease (COPD), pelvic inflammatory disease, Alzheimer's Disease, inflammatory bowel disease, Crohn's disease, ulcerative colitis, Peyronie's Disease, coeliac disease, gallbladder disease, Pilonidal disease, peritonitis, psoriasis, vasculitis, surgical adhesions, stroke, Type I Diabetes, lyme disease, arthritis, meningoencephalitis, autoimmune uveitis, immune mediated inflammatory disorders of the central and peripheral nervous system such as multiple sclerosis, lupus (such as systemic lupus erythematosus) and Guillain-Barr syndrome, Atopic dermatitis, autoimmune hepatitis, fibrosing alveolitis, Grave's disease, IgA nephropathy, idiopathic thrombocytopenic purpura, Meniere's disease, pemphigus, primary biliary cirrhosis, sarcoidosis, scleroderma, Wegener's granulomatosis, pancreatitis, trauma (surgery), graft-versus-host disease (GVHD), transplant rejection, cardiovascular disease including ischaemic diseases such as myocardial infarction as well as atherosclerosis, intravascular coagulation, bone resorption, osteoporosis, osteoarthritis, periodontitis, hypochlorhydia, hidradenitis and neuromyelitis optica.

Other exemplary OX40 mediated disorder include infections (viral, bacterial, fungal and parasitic), endotoxic shock associated with infection, arthritis, rheumatoid arthritis, asthma, bronchitis, influenza, respiratory syncytial virus, pneumonia, chronic obstructive pulmonary disease (COPD), idiopathic pulmonary fibrosis (IPF), cryptogenic fibrosing alveolitis (CFA), idiopathic fibrosing interstitial pneumonia, emphysema, pelvic inflammatory disease, Alzheimer's Disease, inflammatory bowel disease, Crohn's disease, ulcerative colitis, Peyronie's Disease, coeliac disease, gallbladder disease, Pilonidal disease, peritonitis, psoriasis, vasculitis, surgical adhesions, stroke, Type I Diabetes, lyme disease, arthritis, meningoencephalitis, autoimmune uveitis, immune mediated inflammatory disorders of the central and peripheral nervous system such as multiple sclerosis, lupus (such as systemic lupus erythematosus) and Guillain-Barr syndrome, Atopic dermatitis, autoimmune hepatitis, fibrosing alveolitis, Grave's disease, IgA nephropathy, idiopathic thrombocytopenic purpura, Meniere's disease, pemphigus, primary biliary cirrhosis, sarcoidosis, scleroderma, Wegener's granulomatosis, pancreatitis, trauma (surgery), graft-versus-host disease (GVHD), transplant rejection, cardiovascular disease including ischaemic diseases such as myocardial infarction as well as atherosclerosis, intravascular coagulation, bone resorption, osteoporosis, osteoarthritis, periodontitis, hypochlorhydia, hidradenitis and neuromyelitis optica.

In accordance to a preferred aspect of the present invention, the anti-OX40 antagonist antibody is used for the treatment or prevention of an OX40-mediated disorder selected from the group comprising atopic dermatitis, rheumatoid arthritis, autoimmune uveitis, multiple sclerosis, lupus (such as systemic lupus erythematosus), ulcerative colitis, scleroderma and graft-versus-host disease (GVHD), scleroderma, hidradenitis, and ulcerative colitis.

The present invention also provides a method for treating an OX40-mediated disorder, wherein the OX40-mediated disorder is selected from the group comprising atopic dermatitis, rheumatoid arthritis, autoimmune uveitis, multiple sclerosis, lupus (such as systemic lupus erythematosus) and graft-versus-host disease (GVHD), scleroderma, hidradenitis, and ulcerative colitis.

As used herein, the term "subject" includes any human or nonhuman animal. The term "nonhuman animal" includes all vertebrates, e.g., mammals and non-mammals, such as nonhuman primates, sheep, dogs, cats, horses, cows, chickens, amphibians, reptiles, etc. Preferably the subject is human.

A "patient" for the purposes of the present invention includes both humans and other animals, preferably mammals and most preferably humans. Thus the antibodies of the present invention have both human therapy and veterinary applications. The term "treatment" or "treating" in the present invention is meant to include therapeutic treatment, as well as prophylactic, or suppressive measures for a disease or disorder. Thus, for example, successful administration of an antibody prior to onset of the disease results in treatment of the disease. As another example, successful administration of an antibody after clinical manifestation of the disease to combat the symptoms of the disease comprises treatment of the disease.

"Treatment" and "treating" also encompasses administration of an antibody after the appearance of the disease in order to eradicate the disease. Successful administration of an antibody after onset and after clinical symptoms have developed, with possible abatement of clinical symptoms and perhaps amelioration of the disease, comprises treatment of the disease. Those "in need of treatment" include mammals already having the disease or disorder, as well as those prone to having the disease or disorder, including those in which the disease or disorder is to be prevented.

The antibody or of the present invention can be administered via one or more routes of administration using one or more of a variety of methods known in the art. As will be appreciated by the skilled artisan, the route and/or mode of administration will vary depending upon the desired results. Preferred routes of administration include intravenous, intramuscular, intradermal, intraperitoneal, subcutaneous, spinal or other parenteral routes of administration, for example by injection or infusion. More preferred routes of administration are intravenous or subcutaneous. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. Alternatively, an antibody of the invention can be administered via a non- parenteral route, such as a topical, epidermal or mucosal route of administration, for example, intranasally, orally, vaginally, rectally, sublingually or topically.

The antibody of the present invention can be administered at a single or multiple doses. The term "dose" or "dosage" as used in the present invention are interchangeable and indicates an amount of drug substance administered per body weight of a subject or a total dose administered to a subject irrespective to their body weight.

In one embodiment, a therapeutically effective amount of the pharmaceutical formulation according to the present invention is administrated subcutaneously using a prefilled syringe. In accordance with one aspect of the present invention, the pharmaceutical formulations provided may be administered to individuals. In particular, a therapeutically effective amount of said pharmaceutical formulation is administrated subcutaneously as single-dose drug product in a pre-fillable syringe system. Administration is preferably in a "therapeutically effective amount", this being sufficient to show benefit to a subject. Such benefit may be at least amelioration of at least one symptom. The actual amount administered, and rate and time-course of administration, will depend on the nature and severity of what is being treated. Prescription of treatment, e.g. decisions on dosage etc, is within the responsibility of medical doctors. Appropriate doses of antibody are well known in the art (Ledermann JA et al., (1999) Int J Cancer 47: 659-664; Bagshawe KD et a/., (1991) Antibody, Immunoconjugates and Radiopharmaceuticals, 4: 915-922). The precise dose will depend upon a number of factors, including the size and location of the area to be treated, body weight of the subject, the precise nature of the antibody (e.g. whole antibody or fragment) and any additional therapeutic agents administered before, at the time of or after administration of the antibody.

The current invention also discloses a method of manufacturing the pharmaceutical formulation according to the present invention, and/or a method of manufacturing a vial or a prefilled syringe containing said pharmaceutical formulation according to the present invention.

Also disclosed herein is a method to test said pharmaceutical formulation to any assay for stability determination. Non limiting examples of stability assay include analytical test such as monitoring of the visual appearance, sub-visible particles analysis, protein content measurement (e.g. by measuring absorbance at 280 nm wavelength), pH measurement, osmolarity measurement, viscosity measurement, protein thermal stability measurement for instance by nanoformat of Differential Scanning Fluorimetry (DSF), size variants monitoring (e.g. by SE-HPLC and/or by cGE reduced and non-reduced), charge variants monitoring (e.g. by cIEF), antibody potency measurement (e.g. by ELISA) as any significant change of binding property of the antibody toward its target would indicate antibody degradation, peptide mapping, post-translational modifications mapping.

The present invention also relates to an article of manufacture comprising: (a) a container which holds the prefilled syringe and (b) instructions for using said prefilled syringe.

According to the present invention, the disclosed pharmaceutical formulation is suitable to make the a stable bulk drug substance and/or a stable drug product.

Figure 1: Electrophoregrams of samples obtained by cGE analysis (A) REF1; (B) T0; (C) T9M at 25°C; (D) T9M at 5°C.

Figure 2: Profiles of samples obtained by cIEF analysis (A) REF1; (B) T0; (C) T9M at 25°C; (D) T9M at 5°C.

Figure 3: Profiles of samples obtained by iCE analysis (A) REF1; (B) T9M at 25°C; (C) T9M at 5°C.

Figure 4: Overlay of profiles obtained by iCE analysis.

Figure 5: Profiles of samples obtained by CEX-HPLC analysis (A) REF1; (B) T0; (C) T9M at 25°C; (D) T9M at 5°C.

Figure 6: Trial 1 and trial 2 UFDF process flowcharts

Figure 7: Gel theory plot example

Figure 8: Comparison of conductivity trend during diafiltration step between both trials. (A) Trial 1, Buffer 1; (B) Trial 2, Buffer 1; (C) Trial 1, Buffer 2; (D) Trial 2, Buffer 2.

Figure 9: Cgel estimation chart: Normalized filtrate flux = f[log($C_r$)], (A) Buffer1; (B) Buffer2.

Figure 10: Size variants repartition of BDS post UFDF by SE-HPLC analysis, %Aggregates (first column), % monomer (second column), % fragments (third column)

Figure 11: Charge variants repartition of BDS post UFDF by IEF with iCE3, %Acid (first column), % Main (second column), %Basic (third column)

Figure 12: pI range of BDS post UFDF by IEF with iCE3, pI start (first column), pI end (second column), pI of main peak (third column)

Figure 13: SE-HPLC analysis data, %Aggregates (first column), % monomer (second column), % fragments (third column); (A) Ab1-Sample1; (B) Ab1-Sample2.

Figure 14: cIEF by iCE3 analysis data, %Acid (first column), % Main (second column), %Basic (third column; (A) Ab1-Sample1; (B) Ab1-Sample2.

Figure 15: cGE (non-reduced) analysis data of Ab1-Sample1, Assigned peak (first column), IgG peak (second column), others peak (third column).

Figure 16: cGE (reduced) analysis data of Ab1-Sample1, %LC (first column), %HC (second column), Total other (third column).

Figure 17: cGE (non-reduced) analysis data of Ab1-Sample2, Assigned peak (first column), IgG peak (second column), others peak (third column).

Figure 18: cGE (reduced) analysis data of Ab1-Sample2, %LC (first column), %HC (second column), Total other (third column).

Figure 19: Results of the syringeability measurements before (A and B) and after 2 weeks of storage at 40 °C (C and D); measurements were performed with ejection speeds of 10 (A and C) and 20 s/2 ml (B and D); results represent mean force profiles, n = 3.

Figure 20: Results of the cumulative particle concentrations ≥2 μm obtained by MFI analysis comparing T0 (first column) to T-FT (second column) and T2w_40C (third column) of the 50-mg/ml stock dilutions in different PFS; values were not corrected for the dilution factor. Ejection speeds of 10 (A) and 20 s/2 ml (B).

Figure 21: : Results of the cumulative particle concentrations ≥ 5 μm obtained by MFI analysis before and after stressing; cumulative particle number was divided into non-silicone oil droplet-like particle (bottom part of the column) and silicone oil droplet-like particle (upper part of the column) fractions; values were not corrected for the dilution factor. (A) T0, ejection speeds 10 sec; (B) T0, ejection speeds 20 sec; (C) TFT; (D) T2w at 40°C, ejection speeds 10 sec; (E) T2w at 40°C, ejection speeds 20 sec.

Figure 22: Results of the cumulative particle concentrations ≥ 10 μm obtained by MFI analysis before and after stressing; cumulative particle number was divided into non-silicone oil droplet-like particle (bottom part of the column) and silicone oil droplet-like particle (upper part of the column) fractions; values were not corrected for the dilution factor. (A) T0, ejection speeds 10 sec; (B) T0, ejection speeds 20 sec; (C) TFT; (D) T2w at 40°C, ejection speeds 10 sec; (E) T2w at 40°C, ejection speeds 20 sec.

Figure 23: Results of the cumulative particle concentrations ≥25 μm obtained by MFI analysis before and after stressing; cumulative particle number was divided into non-silicone oil droplet-like particle (bottom part of the column) and silicone oil droplet-like particle (upper part of the column) fractions; values were not corrected for the dilution factor. (A) T0, ejection speeds 10 sec; (B) T0, ejection speeds 20 sec; (C) TFT; (D) T2w at 40°C, ejection speeds 10 sec; (E) T2w at 40°C, ejection speeds 20 sec.

Figure 24: Thermal stability evaluation F2, F6, F9 and F10 in Barrel1 and Barrel 3(ii), (A) Tagg, onset; (B) Tm, onset; (C) Tm1.

Figure 25: Thermal stability evaluation F1 to F13 in Barrel1, (A) Tagg, onset; (B) Tm, onset; (C) Tm1

Figure 26: Melting curves F2 vs F7. (A) Ratio fluorescence signal 350nm/330nm; (B) 1$^{st}$ derivative fluorescence signal 350nm/330nm; (C) fluorescence signal 350nm; (D) Back reflection intensity.

Figure 27: Turbidity (OD350) measured for F1 to F13 in Barrel1 (left panel), and for F2, F6, F9, F10 in Barrel3(ii) (right panel) at T0 (first column), after TFT (second column) and after T-roll stresses (third column).

Figure 28: Turbidity (OD550) measured for F1 to F13 in Barrel1 (left panel), and for F2, F6, F9, F10 in Barrel3(ii) (right panel) at T0 (first column), after TFT (second column) and after T-roll stresses (third column).

Figure 29: Micro-Flow Imaging (MFI), particle level measurement in protein-containing samples compared to formulation buffer.

Figure 30: MFI particle levels ≥ 2 μm results before and after stressing . T0 (first column), TFT (second column) and T-roll stresses (third column).

Figure 31: MFI particle levels ≥ 5 μm results before and after stressing and shape analysis. (A) T0; (B) TFT; (C) T-roll.

Figure 32: MFI particle levels ≥ 10 μm results before and after stressing and shape analysis. (A) T0; (B) TFT; (C) T-roll.

Figure 33: SEC monomer content analysis for F1 to F13 in Barrel1 (A), and for F2, F6, F9, F10 in Barrel1 and Barrel3(ii) (B) at T0 (first column), after TFT (second column) and after T-roll stresses (third column).

Figure 34: SEC aggregates analysis (HMWS) for F1 to F13 in Barrel1 (A), and for F2, F6, F9, F10 in Barrel1 and Barrel3(ii) (B) at T0 (first column), after TFT (second column) and after T-roll stresses (third column).

Figure 35: SEC aggregates analysis (LMWS) for F1 to F13 in Barrel1 (A), and for F2, F6, F9, F10 in Barrel1 and Barrel3(ii) (B) at T0 (first column), after TFT (second column) and after T-roll stresses (third column).

Figure 36: Purity analysis (Bioanalyzer)

Figure 37: Purity analysis (Bioanalyzer), results before and after stressing in non-reducing conditions for F1 to F13 in Barrel1 (A), and for F2, F6, F9, F10 in Barrel1 and Barrel3(ii) (B) at T0 (first column), after TFT (second column) and after T-roll stresses (third column).

Figure 38: Purity analysis (Bioanalyzer), results before and after stressing in reducing conditions for F1 to F13 in Barrel1 (A), and for F2, F6, F9, F10 in Barrel1 and Barrel3(ii) (B) at T0 (first column), after TFT (second column) and after T-roll stresses (third column).

Figure 39: iCE main species content for F1 to F13 in Barrel1 (A), and for F2, F6, F9, F10 in Barrel1 and Barrel3(ii) (B) at T0 (first column), after TFT (second column) and after T-roll stresses (third column).

Figure 40: iCE profile of F7

Figure 41: iCE acidic species, results before and after stressing for F1 to F13 in Barrel1 (A), and for F2, F6, F9, F10 in Barrel1 and Barrel3(ii) (B) at T0 (first column), after TFT (second column) and after T-roll stresses (third column).

Figure 42: iCE basic species, results before and after stressing for F1 to F13 in Barrel1 (A), and for F2, F6, F9, F10 in Barrel1 and Barrel3(ii) (B) at T0 (first column), after TFT (second column) and after T-roll stresses (third column).

Figure 43: Thermal stability results, before and after 1 month storage (F350): T0 (first column), T1M at 5°C (second column), T1M at 25°C (third column) and T1M at 40°C (forth column). (A) Tm onset, (B) Tm1.

Figure 44: Thermal stability results, before and after 1 month storage (back reflection): T0 (first column), T1M at 5°C (second column), T1M at 25°C (third column) and T1M at 40°C (forth column). Tagg onset.

Figure 45: Thermal stability results, before and after 1 month storage (Barrel1 VS Barrel3(ii) PFS) : T0 (first column), T1M at 5°C (second column), T1M at 25°C (third column) and T1M at 40°C (forth column). (A) Tagg, onset; (B) Tm, onset; (C) Tm1.

Figure 46: Turbidity (OD350) results, before and after 1 month storage for F1 to F13 in Barrel1 (A), and for F2, F6, F9, F10 in Barrel1 and Barrel3(ii) (B): T0 (first column), T1M at 5°C (second column), T1M at 25°C (third column) and T1M at 40°C (forth column).

Figure 47: Turbidity (OD550) results, before and after 1 month storage for F1 to F13 in Barrel1 (A), and for F2, F6, F9, F10 in Barrel1 and Barrel3(ii) (B): T0 (first column), T1M at 5°C (second column), T1M at 25°C (third column) and T1M at 40°C (forth column).

Figure 48: MFI results at 5°C, before and after 1 month storage

Figure 49: MFI particle levels ≥ 2 μm results, before and after 1 month storage. T0 (first column), T1M at 5°C (second column), T1M at 25°C (third column) and T1M at 40°C (forth column).

Figure 50: MFI particle levels ≥ 5 μm results and shape analysis, before and after 1 month storage. Silicon oil droplet-like particles (upper part of the column), non-silicon oil droplet-like particles (bottom part of the column). T0 (A), T1M at 5°C (B), T1M at 25°C (C) and T1M at 40°C (D).

Figure 51: MFI particle levels ≥ 10 μm results and shape analysis, before and after 1 month storage. Silicon oil droplet-like particles (upper part of the column), non-silicon oil droplet-like particles (bottom part of the column). T0 (A), T1M at 5°C (B), T1M at 25°C (C) and T1M at 40°C (D).

Figure 52: MFI particle levels ≥ 25 μm results and shape analysis, before and after 1 month storage. Silicon oil droplet-like particles (upper part of the column), non-silicon oil droplet-like particles (bottom part of the column). T0 (A), T1M at 5°C (B), T1M at 25°C (C) and T1M at 40°C (D).

Figure 53: SEC monomer content analysis, before and after 1 month storage for F1 to F13 in Barrel1 (A), and for F2, F6, F9, F10 in Barrel1 and Barrel3(ii) (B): T0 (first column), T1M at 5°C (second column), T1M at 25°C (third column) and T1M at 40°C (forth column).

Figure 54: SEC aggregates (HMWS) analysis, before and after 1 month storage for F1 to F13 in Barrel1 (A), and for F2, F6, F9, F10 in Barrel1 and Barrel3(ii) (B): T0 (first column), T1M at 5°C (second column), T1M at 25°C (third column) and T1M at 40°C (forth column).

Figure 55: SEC aggregates (LMWS) analysis, before and after 1 month storage for F1 to F13 in Barrel1 (A), and for F2, F6, F9, F10 in Barrel1 and Barrel3(ii) (B): T0 (first column), T1M at 5°C (second column), T1M at 25°C (third column) and T1M at 40°C (forth column).

Figure 56: Purity analysis (Bioanalyzer) in non-reducing conditions, before and after 1 month storage for F1 to F13 in Barrel1 (A), and for F2, F6, F9, F10 in Barrel1 and Barrel3(ii) (B): T0 (first column), T1M at 5°C (second column), T1M at 25°C (third column) and T1M at 40°C (forth column).

Figure 57: Purity analysis (Bioanalyzer) in reducing conditions, before and after 1 month storage for F1 to F13 in Barrel1 (A), and for F2, F6, F9, F10 in Barrel1 and Barrel3(ii) (B): T0 (first column), T1M at 5°C (second column), T1M at 25°C (third column) and T1M at 40°C (forth column).

Figure 58: iCE3 analysis (main species), before and after 1 month storage for F1 to F13 in Barrel1 (A), and for F2, F6, F9, F10 in Barrel1 and Barrel3(ii) (B): T0 (first column), T1M at 5°C (second column), T1M at 25°C (third column) and T1M at 40°C (forth column).

Figure 59: iCE3 exemplary profiles, before and after 1 month storage

Figure 60: iCE3 analysis (acidic species), before and after 1 month storage for F1 to F13 in Barrel1 (A), and for F2, F6, F9, F10 in Barrel1 and Barrel3(ii) (B): T0 (first column), T1M at 5°C (second column), T1M at 25°C (third column) and T1M at 40°C (forth column).

Figure 61: iCE3 analysis (basic species), before and after 1 month storage for F1 to F13 in Barrel1 (A), and for F2, F6, F9, F10 in Barrel1 and Barrel3(ii) (B): T0 (first column), T1M at 5°C (second column), T1M at 25°C (third column) and T1M at 40°C (forth column).

Figure 62: Dynamic viscosity results before and after 3 months storage for F1 to F13 in Barrel1 (A), and for F2, F6, F9, F10 in Barrel1 and Barrel3(ii) (B): T3M at 5°C (first column), T3M at 25°C (second column), and T3M at 40°C (third column).

Figure 63: Syringeability results, 3 months storage for F1 to F13 in Barrel1 (A), and for F2, F6, F9, F10 in Barrel1 and Barrel3(ii) (B): T3M at 5°C (first column), T3M at 25°C (second column), and T3M at 40°C (third column).

Figure 64: Syringeability results, 3 months storage (A) Barrel1, T3M, F2; (B) Barrel3(ii), T3M, F2; (C) Barrel1, T3M, F6; (D) Barrel3(ii), T3M, F6.

Figure 65: Syringeability results, 3 months storage (A) Barrel1, T3M, F9; (B) Barrel3(ii), T3M, F9; (C) Barrel1, T3M, F10; (D) Barrel3(ii), T3M, F10.

Figure 66: Thermal stability results, before and after 3 months storage (F350). (A) Tm, onset; (B) Tm1. T0 (first column), T1M at 5°C (second column), T3M at 5°C (third column), T1M at 25°C (forth column), T3M at 25°C (fifth column), T1M at 40°C (sixth column), and T3M at 40°C (seventh column).

Figure 67: Thermal stability results, before and after 3 months storage (T agg onset, back reflection). T0 (first column), T1M at 5°C (second column), T3M at 5°C (third column), T1M at 25°C (forth column), T3M at 25°C (fifth column), T1M at 40°C (sixth column), and T3M at 40°C (seventh column).

Figure 68: Thermal stability results, before and after 3 months storage (Barrel1 VS Barrel3(ii) PFS). (A) Tagg onset, (B) Tm, onset; (B) Tm1. T0 (first column), T1M at 5°C (second column), T3M at 5°C (third column), T1M at 25°C (forth column), T3M at 25°C (fifth column), T1M at 40°C (sixth column), and T3M at 40°C (seventh column).

Figure 69: Turbidity (OD350) results, before and after 3 months storage. (A) Barrel1, (B) Barrel 1 and Barrel3(ii). T0 (first column), T1M at 5°C (second column), T3M at 5°C (third column), T1M at 25°C (forth column), T3M at 25°C (fifth column), T1M at 40°C (sixth column), and T3M at 40°C (seventh column).

Figure 70: Turbidity (OD550) results, before and after 3 months storage. (A) Barrel1, (B) Barrel 1 and Barrel3(ii). T0 (first column), T1M at 5°C (second column), T3M at 5°C (third column), T1M at 25°C (forth column), T3M at 25°C (fifth column), T1M at 40°C (sixth column), and T3M at 40°C (seventh column).

Figure 71: MFI particle levels $\geq 2\ \mu m$ results, before and after 3 months storage. T0 (first column), T1M at 5°C (second column), T3M at 5°C (third column), T1M at 25°C (forth column), T3M at 25°C (fifth column), T1M at 40°C (sixth column), and T3M at 40°C (seventh column).

Figure 72: MFI particle levels ≥ 5 μm results and shape analysis, before and after 3 months storage. Silicon oil droplet-like particles (upper part of the column), non-silicon oil droplet-like particles (bottom part of the column). T0 (A), T3M at 5°C (B), T3M at 25°C (C) and T3M at 40°C (D).

Figure 73: MFI particle levels ≥ 10 μm results and shape analysis, before and after 3 months storage. Silicon oil droplet-like particles (upper part of the column), non-silicon oil droplet-like particles (bottom part of the column). T0 (A), T3M at 5°C (B), T3M at 25°C (C) and T3M at 40°C (D).

Figure 74: MFI particle levels ≥ 25 μm results and shape analysis, before and after 3 months storage. Silicon oil droplet-like particles (upper part of the column), non-silicon oil droplet-like particles (bottom part of the column). T0 (A), T3M at 5°C (B), T3M at 25°C (C) and T3M at 40°C (D).

Figure 75: SEC monomer content analysis, before and after 3 months storage for F1 to F13 in Barrel1 (A), and for F2, F6, F9, F10 in Barrel1 and Barrel3(ii) (B). T0 (first column), T1M at 5°C (second column), T3M at 5°C (third column), T1M at 25°C (forth column), T3M at 25°C (fifth column), T1M at 40°C (sixth column), and T3M at 40°C (seventh column).

Figure 76: SEC aggregates (HMWS) analysis, before and after 3 months storage for F1 to F13 in Barrel1 (A), and for F2, F6, F9, F10 in Barrel1 and Barrel3(ii) (B). T0 (first column), T1M at 5°C (second column), T3M at 5°C (third column), T1M at 25°C (forth column), T3M at 25°C (fifth column), T1M at 40°C (sixth column), and T3M at 40°C (seventh column).

Figure 77: SEC aggregates (LMWS) analysis, before and after 3 months storage for F1 to F13 in Barrel1 (A), and for F2, F6, F9, F10 in Barrel1 and Barrel3(ii) (B). T0 (first column), T1M at 5°C (second column), T3M at 5°C (third column), T1M at 25°C (forth column), T3M at 25°C (fifth column), T1M at 40°C (sixth column), and T3M at 40°C (seventh column).

Figure 78: Purity analysis (Bioanalyzer) in non-reducing conditions, before and after 3 months storage for F1 to F13 in Barrel1 (A), and for F2, F6, F9, F10 in Barrel1 and Barrel3(ii) (B). T0 (first column), T1M at 5°C (second column), T3M at 5°C (third column), T1M at 25°C (forth column), T3M at 25°C (fifth column), T1M at 40°C (sixth column), and T3M at 40°C (seventh column).

Figure 79: Purity analysis (Bioanalyzer) in reducing conditions, before and after 3 months storage for F1 to F13 in Barrel1 (A), and for F2, F6, F9, F10 in Barrel1 and Barrel3(ii) (B). T0 (first column), T1M at 5°C (second column), T3M at 5°C (third column), T1M at 25°C (forth column), T3M at 25°C (fifth column), T1M at 40°C (sixth column), and T3M at 40°C (seventh column).

Figure 80: iCE3 analysis (main species), before and after 3 months storage at 5°C (A), at 25°C (B) and at 40°C (C). T0 (first column), T1M (second column), T3M (third column).

Figure 81: iCE3 analysis (acidic species), before and after 3 months storage at 5°C (A), at 25°C (B) and at 40°C (C). T0 (first column), T1M (second column), T3M (third column).

Figure 82: iCE3 analysis (basic species), before and after 3 months storage at 5°C (A), at 25°C (B) and at 40°C (C). T0 (first column), T1M (second column), T3M (third column).

Figure 83: Thermal stability results, before and after 6 months storage (F350) for F2, F7, F9, F10 and F13 in Barrel1. (A) Tm onset, (B) Tm1. T0 (first column), T1M at 5°C (second column), T3M at 5°C (third column), T6M at 5°C (forth column), T1M at 25°C (fifth column), T3M at 25°C (sixth column), and T6M at 25°C (seventh column).

Figure 84: Thermal stability results, before and after 6 months storage (Tagg onset, back reflection) for F2, F7, F9, F10 and F13 in Barrel1. T0 (first column), T1M at 5°C (second column), T3M at 5°C (third column), T6M at 5°C (forth column), T1M at 25°C (fifth column), T3M at 25°C (sixth column), and T6M at 25°C (seventh column).

Figure 85: Turbidity (OD350) results, before and after 6 months storage for F2, F7, F9, F10 and F13 in Barrel1. T0 (first column), T1M at 5°C (second column), T3M at 5°C (third column), T6M at 5°C (forth column), T1M at 25°C (fifth column), T3M at 25°C (sixth column), and T6M at 25°C (seventh column).

Figure 86: Turbidity (OD550) results, before and after 6 months storage for F2, F7, F9, F10 and F13 in Barrel1. T0 (first column), T1M at 5°C (second column), T3M at 5°C (third column), T6M at 5°C (forth column), T1M at 25°C (fifth column), T3M at 25°C (sixth column), and T6M at 25°C (seventh column).

Figure 87: MFI particle levels ≥ 2 μm results, before and after 6 months storage (A, top panel) T0; (A, bottom panel T6M at 25°C; (B) T6M at 5°C.

Figure 88: MFI particle levels ≥ 5 μm results and shape analysis, before and after 6 months storage. Silicon oil droplet-like particles (upper part of the column), non-silicon oil droplet-like particles (bottom part of the column). T0 (A), T6M at 25°C (B), T6M at 5°C (C).

Figure 89: MFI particle levels ≥ 10 μm results and shape analysis, before and after 6 months storage. Silicon oil droplet-like particles (upper part of the column), non-silicon oil droplet-like particles (bottom part of the column). T0 (A), T6M at 25°C (B), T6M at 5°C (C).

Figure 90: MFI particle levels ≥ 25 μm results and shape analysis, before and after 6 months storage. Silicon oil droplet-like particles (upper part of the column), non-silicon oil droplet-like particles (bottom part of the column). T0 (A), T6M at 25°C (B), T6M at 5°C (C).

Figure 91: SEC monomer content analysis, before and after 6 months storage for F2, F7, F9, F10 and F13 in Barrel1. T0 (first column), T1M at 5°C (second column), T3M at 5°C (third column), T6M at 5°C (forth column), T1M at 25°C (fifth column), T3M at 25°C (sixth column), and T6M at 25°C (seventh column).

Figure 92: SEC aggregates (HMWS) analysis, before and after 6 months storage for F2, F7, F9, F10 and F13 in Barrel1. T0 (first column), T1M at 5°C (second column), T3M at 5°C (third column), T6M at 5°C (forth column), T1M at 25°C (fifth column), T3M at 25°C (sixth column), and T6M at 25°C (seventh column).

Figure 93: SEC aggregates (LMWS) analysis, before and after 6 months storage for F2, F7, F9, F10 and F13 in Barrel1. T0 (first column), T1M at 5°C (second column), T3M at 5°C (third column), T6M at 5°C (forth column), T1M at 25°C (fifth column), T3M at 25°C (sixth column), and T6M at 25°C (seventh column).

Figure 94: Purity analysis (Bioanalyzer) in non-reducing conditions, before and after 6 months storage for F2, F7, F9, F10 and F13 in Barrel1. T0 (first column), T1M at 5°C (second column), T3M at 5°C (third column), T6M at 5°C (forth column), T1M at 25°C (fifth column), T3M at 25°C (sixth column), and T6M at 25°C (seventh column).

Figure 95: Purity analysis (Bioanalyzer) in reducing conditions, before and after 6 months storage for F2, F7, F9, F10 and F13 in Barrel1. T0 (first column), T1M at 5°C (second column), T3M at 5°C (third column), T6M at 5°C (forth column), T1M at 25°C (fifth column), T3M at 25°C (sixth column), and T6M at 25°C (seventh column).

Figure 96: iCE3 analysis (main species), before and after 6 months storage for F2, F7, F9, F10 and F13 in Barrel1. T0 (first column), T1M at 5°C (second column), T3M at 5°C (third column), T6M at 5°C (forth column), T1M at 25°C (fifth column), T3M at 25°C (sixth column), and T6M at 25°C (seventh column).

Figure 97: iCE3 analysis (acidic species), before and after 6 months storage for F2, F7, F9, F10 and F13 in Barrel1. T0 (first column), T1M at 5°C (second column), T3M at 5°C (third column), T6M at 5°C (forth column), T1M at 25°C (fifth column), T3M at 25°C (sixth column), and T6M at 25°C (seventh column).

Figure 98: iCE3 analysis (basic species), before and after 6 months storage for F2, F7, F9, F10 and F13 in Barrel1. T0 (first column), T1M at 5°C (second column), T3M at 5°C (third column), T6M at 5°C (forth column), T1M at 25°C (fifth column), T3M at 25°C (sixth column), and T6M at 25°C (seventh column).

## EXAMPLES

### Example 1. Investigation of the effect of buffer, pH and excipient on an antibody pre-formulation

Pre-formulation studies have been performed to assess the effect of buffer type, pH and excipient on an anti-OX40 antibody, Ab1.

Material and methods

To determine the effect of buffer type, pH and excipient on Ab1, its stability was determined by SE-HPLC (briefly, by employing a linear flow of aqueous buffer at a flow rate of 1 mL/min and an injection volume of 50 μl; separation was achieved by using a TSK gel G3000 column) and by subjecting the sample to different types of stress, including (i) storage at 40°C for 1 month (M); (ii) Freeze-Thaw (-40 to +25 C° 3 times); (iii) shear stress, applied by placing vials filled with Ab1 on a horizontal shaker, speed of 900 rpm was applied for 3 days; and (iv) light exposure of 1Klux at 25°C.

Results

The effect of pH and solution molarity is shown in Table 1, the effect of buffer type and pH is shown in Table 2 and the effect of the excipients is shown in Table 3. These studies were carried out using a low concentration of Ab1 equal to 10 mg/ml.

Table 1. Effect of pH and solution molarity. Stress: 1 Month (M) at 40°C. N represents the vial number.

| N | Buffer Concentration | Buffer Type | pH | NaCl | Concentration g/L (SEC) | % Monomer |
|---|---|---|---|---|---|---|
| 1 | 5 mM | Acetate | 5 | 0 mM NaCl | 8.37 | 94.8 |
| 2 | 5 mM | Acetate | 5 | 25 mM NaCl | 9.29 | 94.1 |
| 3 | 5 mM | Acetate | 5 | 50 mM NaCl | 9.04 | 93.7 |
| 4 | 5 mM | Acetate | 5 | 75 mM NaCl | 9 | 93.1 |
| 5 | 5 mM | Acetate | 5 | 100 mM NaCl | 8.87 | 92.5 |
| 6 | 5 mM | Acetate | 5 | 125 mM NaCl | 8.84 | 92.9 |
| 7 | 5 mM | Acetate | 5 | 150 mM NaCl | 8.36 | 92.2 |
| 8 | 5 mM | Acetate | 5 | 175 mM NaCl | 9.3 | 91.7 |
| 9 | 5 mM | Acetate | 5 | 200 mM NaCl | 8.73 | 91.6 |
| 10 | 5 mM | Acetate | 5 | 225 mM NaCl | 8.62 | 91.6 |
| 11 | 5 mM | Acetate | 5 | 250 mM NaCl | 9.1 | 91.3 |
| 12 | 5 mM | Acetate | 5.25 | 0 mM NaCl | 8.1 | 95.2 |
| 13 | 5 mM | Acetate | 5.25 | 25 mM NaCl | 8.77 | 94.3 |
| 14 | 5 mM | Acetate | 5.25 | 50 mM NaCl | 8.35 | 94 |
| 15 | 5 mM | Acetate | 5.25 | 75 mM NaCl | 8.73 | 94.4 |
| 16 | 5 mM | Acetate | 5.25 | 100 mM NaCl | 8.48 | 93.4 |
| 17 | 5 mM | Acetate | 5.25 | 125 mM NaCl | 8.56 | 93.6 |
| 18 | 5 mM | Acetate | 5.25 | 150 mM NaCl | 9.43 | 93.6 |
| 19 | 5 mM | Acetate | 5.25 | 175 mM NaCl | 8.79 | 93.8 |
| 20 | 5 mM | Acetate | 5.25 | 200 mM NaCl | 9.85 | 93.5 |
| 21 | 5 mM | Acetate | 5.25 | 225 mM NaCl | 11.13 | 92.5 |
| 22 | 5 mM | Acetate | 5.25 | 250 mM NaCl | 8.02 | 93.3 |
| 23 | 5 mM | Acetate | 5.5 | 0 mM NaCl | 10.69 | 94.4 |

(continued)

| N | Buffer Concentration | Buffer Type | pH | NaCl | Concentration g/L (SEC) | % Monomer |
|---|---|---|---|---|---|---|
| 24 | 5 mM | Acetate | 5.5 | 25 mM NaCl | 8.82 | 94.5 |
| 25 | 5 mM | Acetate | 5.5 | 50 mM NaCl | 9.27 | 94.8 |
| 26 | 5 mM | Acetate | 5.5 | 75 mM NaCl | 8.85 | 93.7 |
| 27 | 5 mM | Acetate | 5.5 | 100 mM NaCl | 9.09 | 94.3 |
| 28 | 5 mM | Acetate | 5.5 | 125 mM NaCl | 8.12 | 94.9 |
| 29 | 5 mM | Acetate | 5.5 | 150 mM NaCl | 9.85 | 95 |
| 30 | 5 mM | Acetate | 5.5 | 175 mM NaCl | 9.01 | 94.5 |
| 31 | 5 mM | Acetate | 5.5 | 200 mM NaCl | 9.5 | 93.8 |
| 32 | 5 mM | Acetate | 5.5 | 225 mM NaCl | 9.4 | 94.8 |
| 33 | 5 mM | Acetate | 5.5 | 250 mM NaCl | 8.66 | 94.7 |
| 34 | 5 mM | Histidine | 5.75 | 0 mM NaCl | 9.43 | 95.9 |
| 35 | 5 mM | Histidine | 5.75 | 25 mM NaCl | 9.04 | 95 |
| 36 | 5 mM | Histidine | 5.75 | 50 mM NaCl | 8.56 | 95.4 |
| 37 | 5 mM | Histidine | 5.75 | 75 mM NaCl | 9 | 95.2 |
| 38 | 5 mM | Histidine | 5.75 | 100 mM NaCl | 9.26 | 95.2 |
| 39 | 5 mM | Histidine | 5.75 | 125 mM NaCl | 9.09 | 95 |
| 40 | 5 mM | Histidine | 5.75 | 150 mM NaCl | 9.06 | 95.5 |
| 41 | 5 mM | Histidine | 5.75 | 175 mM NaCl | 9.51 | 95.4 |
| 42 | 5 mM | Histidine | 5.75 | 200 mM NaCl | 9.57 | 95.3 |
| 43 | 5 mM | Histidine | 5.75 | 225 mM NaCl | 11.62 | 95.3 |
| 44 | 5 mM | Histidine | 5.75 | 250 mM NaCl | 12.31 | 95.1 |
| 45 | 5 mM | Histidine | 6 | 0 mM NaCl | 9.19 | 95.5 |
| 46 | 5 mM | Histidine | 6 | 25 mM NaCl | 10.43 | 95.5 |
| 47 | 5 mM | Histidine | 6 | 50 mM NaCl | 10.93 | 95.6 |
| 48 | 5 mM | Histidine | 6 | 75 mM NaCl | 9.94 | 95.4 |
| 49 | 5 mM | Histidine | 6 | 100 mM NaCl | 10.69 | 95.2 |
| 50 | 5 mM | Histidine | 6 | 125 mM NaCl | 9.84 | 95.4 |
| 51 | 5 mM | Histidine | 6 | 150 mM NaCl | 9.42 | 95.2 |
| 52 | 5 mM | Histidine | 6 | 175 mM NaCl | 10.37 | 95.4 |
| 53 | 5 mM | Histidine | 6 | 200 mM NaCl | 9.88 | 95.4 |
| 54 | 5 mM | Histidine | 6 | 225 mM NaCl | 10.81 | 95.2 |
| 55 | 5 mM | Histidine | 6 | 250 mM NaCl | 9.66 | 95.2 |
| 56 | 5 mM | Histidine | 6.25 | 0 mM NaCl | 9.55 | 95 |
| 57 | 5 mM | Histidine | 6.25 | 25 mM NaCl | 9.99 | 94.9 |
| 58 | 5 mM | Histidine | 6.25 | 50 mM NaCl | 8.67 | 95.2 |
| 59 | 5 mM | Histidine | 6.25 | 75 mM NaCl | 9.85 | 95.5 |
| 60 | 5 mM | Histidine | 6.25 | 100 mM NaCl | 9.98 | 95 |
| 61 | 5 mM | Histidine | 6.25 | 125 mM NaCl | 8.95 | 95.2 |

(continued)

| N | Buffer Concentration | Buffer Type | pH | NaCl | Concentration g/L (SEC) | % Monomer |
|---|---|---|---|---|---|---|
| 62 | 5 mM | Histidine | 6.25 | 150 mM NaCl | 10.06 | 94.8 |
| 63 | 5 mM | Histidine | 6.25 | 175 mM NaCl | 9.95 | 95.1 |
| 64 | 5 mM | Histidine | 6.25 | 200 mM NaCl | 9.88 | 94.8 |
| 65 | 5 mM | Histidine | 6.25 | 225 mM NaCl | 10.24 | 95.1 |
| 66 | 5 mM | Histidine | 6.25 | 250 mM NaCl | 9.18 | 94.8 |
| 67 | 5 mM | Histidine | 6.5 | 0 mM NaCl | 8.21 | 92.9 |
| 68 | 5 mM | Histidine | 6.5 | 25 mM NaCl | 8.82 | 93.6 |
| 69 | 5 mM | Histidine | 6.5 | 50 mM NaCl | 8.71 | 94.1 |
| 70 | 5 mM | Histidine | 6.5 | 75 mM NaCl | 10.1 | 93.6 |
| 71 | 5 mM | Histidine | 6.5 | 100 mM NaCl | 8.9 | 94.2 |
| 72 | 5 mM | Histidine | 6.5 | 125 mM NaCl | 8.92 | 94 |
| 73 | 5 mM | Histidine | 6.5 | 150 mM NaCl | 10.25 | 94.2 |
| 74 | 5 mM | Histidine | 6.5 | 175 mM NaCl | 9.02 | 94.2 |
| 75 | 5 mM | Histidine | 6.5 | 200 mM NaCl | 9.29 | 94 |
| 76 | 5 mM | Histidine | 6.5 | 225 mM NaCl | 12.46 | 94.4 |
| 77 | 5 mM | Histidine | 6.5 | 250 mM NaCl | 10.94 | 94.3 |
| 78 | 5 mM | Histidine | 6.75 | 0 mM NaCl | 8.61 | 90.4 |
| 79 | 5 mM | Histidine | 6.75 | 25 mM NaCl | 8.81 | 91 |
| 80 | 5 mM | Histidine | 6.75 | 50 mM NaCl | 8.57 | 92 |
| 81 | 5 mM | Histidine | 6.75 | 75 mM NaCl | 8.72 | 92.7 |
| 82 | 5 mM | Histidine | 6.75 | 100 mM NaCl | 10.02 | 93 |
| 83 | 5 mM | Histidine | 6.75 | 125 mM NaCl | 10.82 | 93 |
| 84 | 5 mM | Histidine | 6.75 | 150 mM NaCl | 9.2 | 93.3 |
| 85 | 5 mM | Histidine | 6.75 | 175 mM NaCl | 8.91 | 93 |
| 86 | 5 mM | Histidine | 6.75 | 200 mM NaCl | 10.09 | 93.4 |
| 87 | 5 mM | Histidine | 6.75 | 225 mM NaCl | 9.53 | 93.2 |
| 88 | 5 mM | Histidine | 6.75 | 250 mM NaCl | 9.76 | 93.4 |
| 89 | 5 mM | Histidine | 7 | 0 mM NaCl | 8.51 | 87.6 |
| 90 | 5 mM | Histidine | 7 | 25 mM NaCl | 7.96 | 88.9 |
| 91 | 5 mM | Histidine | 7 | 50 mM NaCl | 9.2 | 89.7 |
| 92 | 5 mM | Histidine | 7 | 75 mM NaCl | 8.67 | 90.5 |
| 93 | 5 mM | Histidine | 7 | 100 mM NaCl | 8.31 | 90.9 |
| 94 | 5 mM | Histidine | 7 | 125 mM NaCl | 9.82 | 91 |
| 95 | 5 mM | Histidine | 7 | 150 mM NaCl | 12.25 | 91.1 |
| 96 | 5 mM | Histidine | 7 | 175 mM NaCl | 8.58 | 91.8 |
| 97 | 5 mM | Histidine | 7 | 200 mM NaCl | 8.46 | 91.6 |
| 98 | 5 mM | Histidine | 7 | 225 mM NaCl | 9.18 | 91.3 |
| 99 | 5 mM | Histidine | 7 | 250 mM NaCl | 8.21 | 91.4 |

Table 2: Effect of buffer type and pH. Stress: Freeze-thaw and 1 M at 40°C. N represents the vial number.

| | | | | | T0 | | Freeze-Thaw | | 1M at 40°C | |
|---|---|---|---|---|---|---|---|---|---|---|
| N | Buffer Concentration | Buffer Type | pH | NaCl (mM) | UV mg/ml | SEC - Monomer % | UV mg/ml | SEC - Monomer % | UV mg/ml | SEC - Monomer % |
| 1 | 5 mM | Acetate | 5.5 | 150 | 10.64 | 99.9 | 10.7 | 99.40 | 11.441 | 94.6 |
| 2 | 25 mM | Acetate | 5.5 | 150 | 10.62 | 99.8 | 10.76 | 99.60 | 11.469 | 94 |
| 3 | 50 mM | Acetate | 5.5 | 150 | 10.67 | 99.8 | 10.91 | 99.70 | 11.479 | 93.7 |
| 4 | 5 mM | Citrate | 5.5 | 150 | 10.77 | 99.9 | 11.07 | 99.60 | 11.611 | 95.2 |
| 5 | 25 mM | Citrate | 5.5 | 150 | 10.46 | 99.9 | 10.55 | 99.80 | 11.523 | 94.8 |
| 6 | 50 mM | Citrate | 5.5 | 150 | 10.92 | 99.9 | 10.75 | 99.80 | 12.07 | 94.7 |
| 7 | 5 mM | Citrate | 5.75 | 150 | 11.05 | 99.9 | 10.95 | 99.60 | 11.712 | 96 |
| 8 | 25 mM | Citrate | 5.75 | 150 | 10.95 | 99.9 | 11.2 | 99.80 | 11.639 | 95.8 |
| 9 | 50 mM | Citrate | 5.75 | 150 | 10.91 | 99.9 | 10.75 | 99.80 | 11.437 | 95.2 |
| 10 | 5 mM | Histidine | 5.75 | 150 | 10.73 | 99.9 | 10.77 | 99.40 | 11.379 | 95.1 |
| 11 | 25 mM | Histidine | 5.75 | 150 | 10.71 | 99.9 | 10.69 | 99.70 | 11.355 | 94.6 |
| 12 | 50 mM | Histidine | 5.75 | 150 | 10.53 | 99.9 | 10.61 | 99.80 | 11.338 | 95 |
| 13 | 5 mM | Citrate | 6 | 150 | 10.65 | 99.9 | 10.71 | 99.60 | 11.325 | 95.5 |
| 14 | 25 mM | Citrate | 6 | 150 | 11.78 | 99.9 | 11.83 | 99.80 | 12.491 | 95.7 |
| 15 | 50 mM | Citrate | 6 | 150 | 6.39 | 99.9 | 6.15 | 99.80 | 6.428 | 95.8 |
| 16 | 5 mM | Histidine | 6 | 150 | 10.5 | 99.9 | 10.64 | 99.50 | 10.95 | 95 |
| 17 | 25 mM | Histidine | 6 | 150 | 10.62 | 99.9 | 10.88 | 99.80 | 11.482 | 95.3 |
| 18 | 50 mM | Histidine | 6 | 150 | 10.68 | 99.9 | 10.96 | 99.80 | 11.737 | 95.1 |
| 19 | 5 mM | Histidine | 6.25 | 150 | 10.55 | 99.9 | 10.72 | 99.50 | 11.509 | 96 |
| 20 | 25 mM | Histidine | 6.25 | 150 | 10.84 | 99.9 | 10.87 | 99.80 | 11.405 | 95.8 |
| 21 | 50 mM | Histidine | 6.25 | 150 | 10.64 | 99.9 | 10.96 | 99.80 | 11.762 | 95.6 |
| 22 | 5 mM | Phosphate | 6.25 | 150 | 10.69 | 99.9 | 10.92 | 99.50 | 11.387 | 95.5 |
| 23 | 25 mM | Phosphate | 6.25 | 150 | 10.74 | 99.9 | 10.84 | 99.70 | 11.447 | 95.2 |
| 24 | 50 mM | Phosphate | 6.25 | 150 | 10.43 | 99.9 | 10.75 | 99.70 | 11.634 | 94.8 |

(continued)

| | | | | | T0 | | Freeze-Thaw | | 1M at 40°C | |
|---|---|---|---|---|---|---|---|---|---|---|
| N | Buffer Concentration | Buffer Type | pH | NaCl (mM) | UV mg/ml | SEC - Monomer % | UV mg/ml | SEC - Monomer % | UV mg/ml | SEC - Monomer % |
| 25 | 5 mM | Histidine | 6.5 | 150 | 10.38 | 99.9 | 10.62 | 99.50 | 11.376 | 95 |
| 26 | 25 mM | Histidine | 6.5 | 150 | 10.33 | 99.9 | 10.61 | 99.80 | 11.315 | 94.8 |
| 27 | 50 mM | Histidine | 6.5 | 150 | 11.07 | 99.9 | 11.08 | 99.80 | 11.21 | 94.5 |
| 28 | 5 mM | Phosphate | 6.5 | 150 | 10.88 | 99.8 | 11.1 | 99.60 | 11.982 | 94.9 |
| 29 | 25 mM | Phosphate | 6.5 | 150 | 10.72 | 99.9 | 10.88 | 99.70 | 11.635 | 94.8 |
| 30 | 50 mM | Phosphate | 6.5 | 150 | 10.73 | 99.9 | 10.86 | 99.70 | 11.569 | 94.1 |

| | T0 | Freeze-Thaw | Shear stress | Light Exposure | 1M at 40°C |
|---|---|---|---|---|---|

| N | Type excipient | Molarity (mM) | Buffer type | pH | Excipient | Excipient Concentration (g/L) | UV mg/ml | Monomer % | UV mg/ml | Monomer % | UV mg/ml | Monomer % | UV mg/ml | Monomer % | UV mg/ml | Monomer % |
|---|---|---|---|---|---|---|---|---|---|---|---|---|---|---|---|---|
| 1 | Sugar/polyhydric alcohols | 5 | Citrate | 5.75 | Manni-tol | 10 | 9.602 | 99.6 | 9.221 | 99.7 | 9.221 | 99.6 | 9.423 | 98.3 | 7.912 | 93.9 |
| 2 | | 5 | Citrate | 5.75 | Manni-tol | 50 | 10.194 | 99.6 | 10.086 | 99.5 | 10.086 | 99.7 | 10.453 | 98.2 | 8.988 | 94.1 |
| 3 | | 5 | Citrate | 5.75 | Sucrose | 75 | 9.46 | 99.6 | 9.4 | 99.7 | 9.4 | | 8.833 | 98.2 | 8.239 | 94.5 |
| 4 | | 5 | Citrate | 5.75 | Sucrose | 150 | 9.687 | 99.6 | 9.446 | 99.7 | 9.446 | | 9.336 | 98.6 | 8.447 | 94.2 |
| 5 | | 5 | Citrate | 5.75 | PEG400 | 10 | 10.02 | 99.6 | 9.986 | 99.7 | 9.986 | | 10.029 | 97.7 | 8.696 | 94.5 |
| 6 | | 5 | Citrate | 5.75 | PEG400 | 50 | 9.981 | 99.6 | 9.962 | 99.7 | 9.962 | 99.7 | 9.971 | 97.4 | 8.681 | 94.6 |
| 7 | | 5 | Citrate | 5.75 | Sorbitol | 50 | 10.246 | 99.6 | 10.054 | 99.7 | 10.054 | 99.3 | 10.126 | 98 | 8.85 | 94.4 |
| 8 | | 5 | Citrate | 5.75 | Sorbitol | 100 | 10.029 | 99.6 | 9.788 | 99.7 | 9.788 | 99.6 | 10.047 | 98.7 | 8.949 | 95 |
| 9 | | 5 | Citrate | 5.75 | Treha-lose | 10 | 10.114 | 99.6 | 9.72 | 99.7 | 9.72 | 99.6 | 10.26 | 93.4 | 8.544 | 94.9 |
| 10 | | 5 | Citrate | 5.75 | Treha-lose | 50 | 9.94 | 99.6 | 9.134 | 99.7 | 9.134 | 99.4 | 9.759 | 98.4 | 8.717 | 95.1 |
| 11 | Detergents | 5 | Citrate | 5.75 | Tween 20 | 0.012 | 10.062 | 99.6 | 9.618 | 99.7 | 9.618 | | 9.704 | 96.5 | 8.733 | 94.9 |
| 12 | | 5 | Citrate | 5.75 | Tween 20 | 0.061 | 9.97 | 99.6 | 9.769 | 99.4 | 9.769 | 99.7 | 9.584 | 96.3 | 8.696 | 94.9 |
| 13 | | 5 | Citrate | 5.75 | Tween 20 | 0.1 | 10.098 | 99.6 | 9.433 | 99.7 | 9.433 | 99.7 | 9.603 | 96.6 | 8.63 | 94.8 |
| 14 | | 5 | Citrate | 5.75 | Tween 80 | 0.1 | 9.819 | 99.1 | 9.425 | 99.5 | 9.425 | 99.2 | 9.484 | 96.8 | 8.476 | 94.9 |
| 15 | | 5 | Citrate | 5.75 | Tween 80 | 0.5 | 9.625 | 99.4 | 10.102 | 99.3 | 10.102 | 99.5 | 9.555 | 97.9 | 8.567 | 94.2 |
| 16 | | 5 | Citrate | 5.75 | Tween 80 | 1 | 9.662 | 99.1 | 10.193 | 99 | 10.193 | 99.2 | 9.48 | 96.8 | 8.49 | 93.9 |
| 17 | | 5 | Citrate | 5.75 | Tween 80 | 10 | 10.324 | 94.6 | 10.972 | 95 | 10.972 | 94.8 | 9.775 | 87 | 9.021 | 90.2 |
| 18 | Chaotropes | 5 | Citrate | 5.75 | CaCl2 | 10 | 9.481 | 99.7 | 9.518 | 99.7 | 9.518 | | 9.851 | 97.7 | 8.451 | 90.1 |
| 19 | | 5 | Citrate | 5.75 | CaCl2 | 20 | 9.774 | 99.6 | 10.091 | 98.1 | 10.091 | | 9.543 | 97.7 | 8.566 | 86.4 |
| 20 | | 5 | Citrate | 5.75 | EDTA | 0.01 | 9.671 | 99.7 | 9.547 | 99.6 | 9.547 | | 9.906 | 97.9 | 8.408 | 95.9 |
| 21 | | 5 | Citrate | 5.75 | EDTA | 0.05 | 9.814 | 99.7 | 9.814 | 99.6 | 9.814 | | 9.787 | 97.8 | 8.592 | 95.4 |
| 22 | Ami | 5 | Citrate | 5.75 | Glycine | 4 | 9.671 | 99.7 | 10.022 | 99.5 | 10.022 | | 9.354 | 97.2 | 8.321 | 95.8 |
| 23 | | 5 | Citrate | 5.75 | Glycine | 20 | 9.522 | 99.7 | 9.655 | 99.7 | 9.655 | | 9.811 | 97.7 | 8.477 | 95.6 |

| | | | | | | | | | | | | | | | | |
|---|---|---|---|---|---|---|---|---|---|---|---|---|---|---|---|---|
| 24 | | 5 | Citrate | 5.75 | Arginine | 4 | 9.507 | 99.7 | 9.387 | 99.6 | 9.387 | | 9.685 | 98.2 | 8.422 | 95.7 |
| 25 | | 5 | Citrate | 5.75 | Arginine | 20 | 8.892 | 99.7 | 9.218 | 99.6 | 9.218 | | 8.828 | 98.8 | 7.975 | 95.4 |
| 26 | | 5 | Citrate | 5.75 | Glutamine | 4 | 10.523 | 99.7 | 10.368 | 99.7 | 10.368 | | 10.67 | 97.4 | 9.381 | 95.3 |
| 27 | | 5 | Citrate | 5.75 | Glutamine | 8 | 10.193 | 99.7 | 10.334 | 99.7 | 10.334 | | 10.577 | 98.1 | 9.019 | 95.7 |
| 28 | Antioxydants | 5 | Citrate | 5.75 | Glutathione | 0.003 | 9.73 | 99.7 | 9.727 | 99.7 | 9.727 | | 9.696 | 97.7 | 8.596 | 96.1 |
| 29 | | 5 | Citrate | 5.75 | Glutathione | 0.15 | 11.546 | 99.7 | 11.221 | 99.5 | 11.221 | 99.4 | 12.766 | 95.4 | 9.957 | 94.9 |
| 30 | | 5 | Citrate | 5.75 | Glutathione | 0.31 | 9.882 | 98.7 | 9.408 | 99.5 | 9.408 | 99.4 | 10.137 | 97.6 | 8.397 | 95.5 |
| 31 | | 5 | Citrate | 5.75 | Methionine | 0.0015 | 9.563 | 99.7 | 9.532 | 99.6 | 9.532 | 99.6 | 9.624 | 96.5 | 8.484 | 94.9 |
| 32 | | 5 | Citrate | 5.75 | Methionine | 0.075 | 9.593 | 99.5 | 9.606 | 99.7 | 9.606 | | 10.432 | 97.1 | 8.499 | 94.8 |
| 33 | | 5 | Citrate | 5.75 | Methionine | 0.15 | 9.589 | 99.7 | 9.447 | 99.7 | 9.447 | | 9.997 | 97.1 | 8.426 | 95.5 |
| 34 | | 5 | Citrate | 5.75 | Cysteine | 0.0012 | 10.875 | 99.7 | 10.833 | 99.7 | 10.833 | | 10.522 | 95.3 | 9.536 | 95.4 |
| 35 | | 5 | Citrate | 5.75 | Cysteine | 0.06 | 9.457 | 99.6 | 9.361 | 99.5 | 9.361 | 99.5 | 9.557 | 96.9 | 8.452 | 95.5 |
| 36 | | 5 | Citrate | 5.75 | Cysteine | 0.12 | 9.701 | 99.5 | 9.418 | 99.5 | 9.418 | | 10.621 | 98.2 | 8.603 | 95.7 |
| 37 | Sugar/polyhydric alcohols | 5 | Histidine | 6.25 | Mannitol | 10 | 10.541 | 99.6 | 10.357 | 99.7 | 10.357 | | 11.124 | 98 | 10.49 | 96 |
| 38 | | 5 | Histidine | 6.25 | Mannitol | 50 | 10.063 | 99.7 | 9.846 | 99.5 | 9.846 | | 10.223 | 98.5 | 9.926 | 96 |
| 39 | | 5 | Histidine | 6.25 | Sucrose | 75 | 11.012 | 99.7 | 11.128 | 99.7 | 11.128 | | 10.715 | 98.2 | 10.974 | 95.9 |
| 40 | | 5 | Histidine | 6.25 | Sucrose | 150 | 10.365 | 99.7 | 10.087 | 99.8 | 10.087 | | 9.94 | 98.6 | 10.136 | 95.6 |
| 41 | | 5 | Histidine | 6.25 | PEG400 | 10 | 11.158 | 99.7 | 10.913 | 99.7 | 10.913 | | 11.277 | 97.6 | 10.995 | 95.9 |
| 42 | | 5 | Histidine | 6.25 | PEG400 | 50 | 10.479 | 99.6 | 9.67 | 99.6 | 9.67 | | 9.949 | 95.6 | 9.736 | 95.5 |
| 43 | | 5 | Histidine | 6.25 | Sorbitol | 50 | 10.736 | 99.7 | 10.691 | 99.7 | 10.691 | | 10.393 | 98.6 | 10.809 | 95.9 |
| 44 | | 5 | Histidine | 6.25 | Sorbitol | 100 | 10.845 | 99.6 | 10.753 | 99.7 | 10.753 | | 10.616 | 98.7 | 10.704 | 96.2 |

| | | | | | | | | | | | | | | | | |
|---|---|---|---|---|---|---|---|---|---|---|---|---|---|---|---|---|
| 45 | | 5 | Histidine | 6.25 | Trehalose | 10 | 11.611 | 99.6 | 10.457 | 99.7 | 10.457 | | 10.947 | 98.1 | 10.63 | 95.6 |
| 46 | | 5 | Histidine | 6.25 | Trehalose | 50 | 10.846 | 99.7 | 10.488 | 99.7 | 10.488 | 99.5 | 8.691 | 98.3 | 10.736 | 96.2 |
| 47 | Detergents | 5 | Histidine | 6.25 | Tween 20 | 0.012 | 10.826 | 99.7 | 10.628 | 99.7 | 10.628 | | 10.683 | 98.2 | 10.785 | 96 |
| 48 | | 5 | Histidine | 6.25 | Tween 20 | 0.061 | 10.766 | 99.6 | 10.609 | 99.4 | 10.609 | 99.6 | 10.546 | 98.3 | 10.534 | 95.6 |
| 49 | | 5 | Histidine | 6.25 | Tween 20 | 0.1 | 11.005 | 99.7 | 11.034 | 99.7 | 11.034 | 99.5 | 10.634 | 98.3 | 10.807 | 94.7 |
| 50 | | 5 | Histidine | 6.25 | Tween 80 | 0.1 | 10.927 | 99.6 | 10.686 | 99.4 | 10.686 | 99.4 | 10.672 | 98.1 | 10.576 | 92.6 |
| 51 | | 5 | Histidine | 6.25 | Tween 80 | 0.5 | 10.915 | 99.3 | 10.713 | 99.5 | 10.713 | 99.5 | 10.733 | 97.9 | 10.664 | 88.9 |
| 52 | | 5 | Histidine | 6.25 | Tween 80 | 1 | 10.91 | 99.3 | 10.685 | 99.4 | 10.685 | 99.4 | 10.683 | 97.7 | 10.676 | 86.7 |
| 53 | | 5 | Histidine | 6.25 | Tween 80 | 10 | 11.732 | 95.7 | 11.098 | 95.9 | 11.098 | 96 | 10.668 | 94.1 | 10.773 | 80.3 |
| 54 | Chaotropes | 5 | Histidine | 6.25 | CaCl2 | 10 | 11.054 | 99.7 | 10.717 | 99 | 10.717 | | 10.738 | 97.7 | 10.73 | 95.6 |
| 55 | | 5 | Histidine | 6.25 | CaCl2 | 20 | 10.855 | 99.7 | 10.644 | 93.9 | 10.644 | | 10.84 | 97.8 | 10.777 | 95.5 |
| 56 | | 5 | Histidine | 6.25 | EDTA | 0.01 | 10.332 | 99.7 | 10.143 | 99.7 | 10.143 | | 12.316 | 97.9 | 10.189 | 96.3 |
| 57 | | 5 | Histidine | 6.25 | EDTA | 0.05 | 10.731 | 99.7 | 10.43 | 99.7 | 10.43 | | 9.503 | 98.1 | 10.527 | 96.3 |
| 58 | Amino acids | 5 | Histidine | 6.25 | Glycine | 4 | 10.772 | 99.6 | 10.809 | 99.7 | 10.809 | | 10.83 | 98 | 10.647 | 96.1 |
| 59 | | 5 | Histidine | 6.25 | Glycine | 20 | 10.895 | 99.7 | 10.65 | 99.7 | 10.65 | | 10.854 | 98.7 | 10.707 | 95 |
| 60 | | 5 | Histidine | 6.25 | Arginine | 4 | 10.676 | 99.7 | 10.23 | 99.7 | 10.23 | | 10.933 | 98.5 | 10.374 | 95.9 |
| 61 | | 5 | Histidine | 6.25 | Arginine | 20 | 11.094 | 99.7 | 10.613 | 99.7 | 10.613 | | 10.923 | 99 | 10.672 | 95.9 |
| 62 | | 5 | Histidine | 6.25 | Glutamine | 4 | 10.769 | 99.7 | 10.418 | 99.7 | 10.418 | | 10.867 | 98.6 | 10.694 | 96 |
| 63 | | 5 | Histidine | 6.25 | Glutamine | 8 | 10.833 | 99.7 | 10.619 | 99.7 | 10.619 | | 10.281 | 98.5 | 10.766 | 95.6 |
| 64 | Antioxydan | 5 | Histidine | 6.25 | Gluthatione | 0.003 | 11.51 | 99.6 | 10.7 | 99.5 | 10.7 | | 11.422 | 98.3 | 10.645 | 96.6 |
| 65 | | 5 | Histidine | 6.25 | Gluthatione | 0.15 | 10.798 | 99.6 | 10.696 | 99.5 | 10.696 | | 10.74 | 98.1 | 10.724 | 95.5 |

| | | | | | | | | | | | | | | | | |
|---|---|---|---|---|---|---|---|---|---|---|---|---|---|---|---|---|
| 66 | | 5 | Histi-dine | 6.25 | Glutha-tione | 0.31 | 10.943 | 99.5 | 10.793 | 99.5 | 10.793 | | 11.748 | 98.2 | 10.885 | 95.5 |
| 67 | | 5 | Histi-dine | 6.25 | Methio-nine | 0.0015 | 10.873 | 99.6 | 10.679 | 99.7 | 10.679 | | 11.076 | 98.3 | 10.699 | 96.1 |
| 68 | | 5 | Histi-dine | 6.25 | Methio-nine | 0.075 | 11.366 | 99.7 | 10.647 | 99.7 | 10.647 | | 11.076 | 97.9 | 10.854 | 96.1 |
| 69 | | 5 | Histi-dine | 6.25 | Methio-nine | 0.15 | 11.233 | 99.6 | 10.79 | 99.5 | 10.79 | | 10.972 | 98.2 | 10.782 | 96 |
| 70 | | 5 | Histi-dine | 6.25 | Cysteine | 0.0012 | 10.906 | 99.6 | 10.603 | 99.7 | 10.603 | | 11.389 | 98.5 | 10.779 | 96.3 |
| 71 | | 5 | Histi-dine | 6.25 | Cysteine | 0.06 | 11.701 | 99.5 | 10.634 | 99.4 | 10.634 | | 11.216 | 98.4 | 10.74 | 95.3 |
| 72 | | 5 | Histi-dine | 6.25 | Cysteine | 0.12 | 10.885 | 99.5 | 10.779 | 99.4 | 10.779 | 99.3 | 11.214 | 98.4 | 11.042 | 94.4 |

Table 3: Effect of excipient. Stress: Freeze-thaw, Shear stress, Light Exposure, 1M at 40°C. N represents the vial number.

**Example 2: Study of the stability on low concentration formulation**

Part 1

To characterize the stability of a liquid formulation containing a low concentration of Ab1, the stability of Ab1 Drug Product (DP) stored during approximately nine months at 25°C was analyzed. The data were compared to two standards, to the T0 of stability study and to the same time point at 5°C.

*Materials and Methods*

Reference materials: REF1 and REF2.

Ab1 DP formulation: 10 mg/mL Ab1, 15 mM Histidine, 150 mM NaCl, 0.01% polysorbate, such as Tween 80, pH 6.25.

The samples were tested as follow: pH measurement, appearance, A280 measurement, SDS-PAGE, SE-HPLC, cGE, cIEF (iCE)/CEX-HPLC, Binding ELISA/Cell-based ELISA. The amino acid sequence and post-translational modifications (deamidation, oxidation, glycation, N-terminal variants, C-terminal variants and glycosylation site occupancy) was verified by peptide mapping RP-HPLC coupled to LC-ESI-MS (MS/MS).

*Results and Discussion*

Table 4 summarizes results of analyses.

Table 4: Results of stability tests.

| Tests | Specifications | Ab1 stability sample T0 | Ab1 stability sample T9 at 25°C | Ab1 stability sample T9 at 5°C |
|---|---|---|---|---|
| Appearan ce | Colorless to slightly yellowish, clear to slightly opalescent liquid; practically free of visual particulates | Clear, colorless and practically free of visible particulates | Clear, colorless and practically free of visible particulates | Slightly opalescent, colorless and practically free of visible particulates |

(continued)

| Tests | Specifications | Ab1 stability sample T0 | Ab1 stability sample T9 at 25°C | Ab1 stability sample T9 at 5°C |
|---|---|---|---|---|
| pH | pH at T0 ± 0.15 | 6.34 | 6.35 | 6.33 |
| $A_{280}$ | Concentration at T0 ± 20% | 9.4 | 9.8 | 9.8 |
| SDS-PAGE (reduced) | Main bands approximately 25kDa and 50kDa; Banding pattern consistent with Reference Standard | Main bands at 25 kDa and at 50 kDa: banding pattern consistent with Ref. Std. | Main bands at 25 kDa and at 50 kDa: banding pattern not consistent with Ref. Std. | Main bands at 25 kDa and at 50 kDa: banding pattern consistent with Ref. Std. |
| SDS-PAGE (nonreduced) | Main band at approximately 150kDa; Banding pattern consistent with Reference Standard | Main band at 150 kDa: banding pattern consistent with Ref. Std. | Main band at 150 kDa: banding pattern not consistent with Ref. Std. | Main band at 150 kDa: banding pattern consistent with Ref. Std. |
| SE-HPLC | % monomer IgG peak ≥ 98% | 100% | 95% | 99% |
| Binding ELISA | ≥ 50 and ≤ 150% of EC50 of Reference Standard | 114% | 133% | 102% |
| CGE | Banding pattern consistent with Reference Standard. Report % Monomer IgG peak | Comparable to Ref. Std -92.1% main peak | Not Comparable to Ref. Std -77.9% main peak | Comparable to Ref. Std -93.4% main peak |
| cIEF | Comparable to Reference Standard | Comparable to Ref. Std - pI range 7.7 to 8.4 (main 7.9) | Not Comparable to Ref. Std - pI range 7.3 to 8.4 (main 7.9) | Comparable to Ref. Std - pI range 7.3 to 8.3 (main 7.8) |
| iCE | Comparable to Reference Standard | NT | Not Comparable to Ref. Std - pI range 7.0 to 8.9 (main 8.3) | Comparable to Ref. Std - pI range 7.2 to 8.8 (main 8.2) |
| CEX-HPLC | Comparable to Reference Standard | Comparable to Ref. Std - 42.0% Main peak | Not Comparable to Ref. Std - 38.8% Main peak | Comparable to Ref. Std - 41.2% Main peak |
| NT: Not Tested | | | | |

The results of cGE are presented in Figure 1 and in Table 5.

Table 5: Values of Retention Time (RT) and %Area of peaks for cGE analysis

| | REF1 | | T0 | | T9M +25°C | | T9M +5°C | |
|---|---|---|---|---|---|---|---|---|
| Peak names | RT | Area | RT | Area | RT | Area | RT | Area |
| LC | 15.3 | 1.2 | 15.3 | 1.5 | 15.5 | 1.2 | 15.4 | 1.1 |
| HC | n/a | 0 | n/a | 0 | 18.7 | 0.6 | 18.7 | 0.1 |
| 75kD Fragment | 22.1 | 0.2 | 22.2 | 0.2 | 22.4 | 0.2 | 22.3 | 0.2 |
| 100kD Fragment | 24.9 | 0.4 | 25.0 | 0.6 | 25.3 | 1.3 | 25.1 | 0.5 |
| 125kD Fragment | 26.6 | 3.9 | 26.7 | 5.6 | 27.0 | 4.6 | 26.8 | 4.1 |
| NG IgG | n/a | 0 | n/a | 0 | 27.8 | 14.3 | 27.8 | 3.9 |
| IgG | 27.7 | 94.3 | 27.9 | 92.1 | 28.2 | 77.9 | 28.0 | 90.0 |

On cGE, T0 and T9M at 5°C are similar to reference standard profile except for the shoulder before the main peak which is not visible in REF1. It seems to be specific to the material. The shoulder clearly appears as an independent peak on sample T9M at 25°C (Figure 1). Values of peak area confirm this observation (Table 5). HC and 100kD fragments increase as well. Regarding values and profiles, cGE clearly highlight the fact that non-glycosylated (NG) species are created during the degradation of Ab1 as well as HC and 100kD fragments.

The results of cIEF are presented in Figure 2 and in Table 6.

Table 6: Values of percentages area of peaks for cIEF analysis

| Peak names | REF1 | T0 | T9M +25°C | T9M +5°C |
|---|---|---|---|---|
| Basic | 27.7 | 32.6 | 22.8 | 27.4 |
| Main | 45.8 | 44.5 | 36.6 | 44.4 |
| Acidic | 26.5 | 22.9 | 40.6 | 27.9 |

Due to the complexity of cIEF profile, visual observation is difficult. Nonetheless, sample T9M at +25°C does not has a double main peak and the first peaks of the profile seem lower that in the other profiles. So this sample seems to contain less main and basic species (Figure 2). Values of Table 6 confirm the visual observation. Acidic peak represents 41% of total species instead of 22-28% in other samples. Main specie represents 37% of total species instead of 44-45% in other samples. The variation can be explained by a change in the global charge of the molecule like deamidation or oxidation.

The results of iCE are presented in Figure 3, Figure 4 and in Table 7.

Table 7: Values of Retention Time (RT) and percentages area of peaks for iCE analysis

| | REF1 | | T9M +25°C | | T9M +5°C | |
|---|---|---|---|---|---|---|
| Peak names | RT | Area | RT | Area | RT | Area |
| Acidic | 8.055 | 30.89 | 8.064 | 48.92 | 8.044 | 34.55 |
| Main | 8.259 | 44.26 | 8.260 | 32.54 | 8.246 | 42.23 |
| Basic | 8.408 | 24.85 | 8.402 | 18.54 | 8.395 | 23.22 |

T0 sample was not analyzed by iCE as the method was not available for routine at this period. Profiles of iCE are visually comparable (Figure 3). Overlay allows to notice that acidic species are higher and main and basic species are lower in T9M at +25°C compared to the two other profiles. Data of Table 7 confirm this observation. Retention time is similar for all samples. The hypothesis can be made that charge of the molecule changes due to oxidation and/or deamidation.

The results of CEX-HPLC are presented in Figure 5 and in Table 8.

Table 8: Values of Retention Time (RT) and percentages area for CEX-HPLC analysis. NA: Not Applicable.

| | REF1 | | T0 | | T9M +25°C | | T9M +5°C | |
|---|---|---|---|---|---|---|---|---|
| Peak number | RT | Area | RT | Area | RT | Area | RT | Area |
| 1 | 9.600 | 3.18 | 9.717 | 3.0 | 9.567 | 4.76 | 9.633 | 3.66 |
| 2 | 10.595 | 7.97 | 10.546 | 7.8 | 10.601 | 8.99 | 10.554 | 8.33 |
| 3 | 11.067 | 1.49 | 10.911 | 1.9 | 10.938 | 4.89 | 11.033 | 1.80 |
| 4 | 11.426 | 8.18 | 11.373 | 8.5 | 11.415 | 13.39 | 11.391 | 9.49 |
| 5 | 12.800 | 40.64 | 12.632 | 41.9 | 12.742 | 38.76 | 12.738 | 41.21 |
| 6 | 15.328 | 18.60 | 14.976 | 19.1 | 15.260 | 17.80 | 15.241 | 18.75 |
| 7 | 16.739 | 10.32 | 16.291 | 9.4 | 16.657 | 1.58 | 16.654 | 8.47 |
| 8 | 20.563 | 8.06 | 20.115 | 8.4 | 20.693 | 9.83 | 20.558 | 8.30 |
| 9 | 22.259 | 1.58 | NA | NA | NA | NA | NA | NA |

Data shows that two peaks increase (peak 3 and 4) and one peak almost disappear (peak 7 at 16.6 minutes) in sample T9M at +25°C. This evolution could be linked to the oxidation and/or deamidation.

Additionally, peptide mapping was performed with RP-HPLC LC-ESI-MS (MS) to confirm amino acid sequence. Table 9 summarizes the results, results confirms 100% identity with the expected sequence. As expected, sequence of all sample are 100% identical.

Table 9: Peptide mapping RP-HPLC LC-ESI-MS (MS) for Ab1 - confirmation of sequence identity

| Tests | Methods | Specification | REF1 | REF2 | Ab1 stability sample T9M (+25°C) |
|---|---|---|---|---|---|
| Amino acid Sequence | Peptide mapping RP-HPLC LC-ESI-MS(MS) | Report % heavy and light chains detected as expected sequence | 100% identity to reference sequence | 100% identity to reference sequence | 100% identity to reference sequence |

Data related to deamidation and isomerization analysis are presented in Table 10. Peptide mapping was performed with RP-HPLC LC-ESI-MS (MS) to confirm level of deamidation.

Table 10: Peptide mapping RP-HPLC LC-ESI-MS (MS) - deamidation

| Tests | Methods | Specification | REF1 | REF2 | Ab1 stability sample T9M (+25°C) |
|---|---|---|---|---|---|
| Asn Deamidation (% at amino acid position) | Peptide mapping RP-HPLC LC-ESI-MS(MS) | Report Result (% deamidation) for light chain (L) and heavy chain (H) | L151/157 - 1% | L151/157 - n.d. | L151/157 - 1% |
| | | | H326 - 1% | H326 - 1% | H326 - 9% |
| | | | H385/390/391 - 3% | H385/390/391 - 1% | H385/390/391 -4% |
| | | | H422/435 - 1% | H422/435 -1% | H422/435 - 1% |
| | | | H435 - n.d. | H435 - n.d. | H435 - 1% |

A high deamidation level is found on the 326th amino acid of the heavy chain (H326) for the stability sample T9M at +25°C compared to the other samples. Deamidation seems to occur during degradation of the molecule.

Data related to oxidation analysis are reported in Table 11. Peptide mapping was performed with RP-HPLC LC-ESI-MS (MS) to confirm oxidation sites along with the level of oxidations.

Table 11: Peptide mapping RP-HPLC LC-ESI-MS (MS)-Oxidation levels

| Tests | Methods | Specification | REF1 | REF2 | Ab1 stability sample T9M (+25°C) |
|---|---|---|---|---|---|
| | | | L32/34 (Met/Trp) - 1% | L32/34 (Met/Trp) - 2% | L32/34 (Met/Trp) - 1% |
| | | | L32/34/36 (Met/Trp/Trp) - 2% | L32/34/36 (Met/Trp/Trp) - 3% | L32/34/36 (Met/Trp/Trp) - 2% |
| | | | L46 (Trp) - 1% | L46 (Trp) - 1% | L46 (Trp) - 1% |
| | | | L90/95 (Trp/Trp) - n.d. | L90/95 (Trp/Trp) - n.d. | L90/95 (Trp/Trp) - 1% |
| | | | H34/38 (Met/Trp) - 5% | H34/38 (Met/Trp) - 9% | H34/38 (Met/Trp) -17% |
| | | | H49 (Trp) - 1% | H49 (Trp) - 1% | H49 (Trp) - 1% |

(continued)

| Tests | Methods | Specification | REF1 | REF2 | Ab1 stability sample T9M (+25°C) |
|---|---|---|---|---|---|
| Met/Trp Oxidation (% at amino acid position) | Peptide mapping RP-HPLC LC-ESI-MS(MS) | Report Result for light chain (L) and heavy chain (H) | H49/54 (Trp/Trp) - n.d. | H49/54 (Trp/Trp) - n.d. | H49/54 (Trp/Trp) - 1% |
| | | | H54/55 (Trp/Trp) - 1% | H54/55 (Trp/Trp) - 1% | H54/55 (Trp/Trp) - n.d. |
| | | | H55 (Trp) - n.d. | H55 (Trp) - n.d. | H55 (Trp) - n.d |
| | | | H84/87 (Met/Met) - 1% | H84/87 (Met/Met) - 2% | H84/87 (Met/Met) -2% |
| | | | H102 (Trp) - 1% | H102 (Trp) - 1% | H102 (Trp) - 1% |
| | | | H102/108 (Trp/Trp) - 3% | H102/108 (Trp/Trp) - 3% | H102/108 (Trp/Trp) - 8% |
| | | | H159 (Trp) - 1% | H159 (Trp) - 1% | H159 (Trp) - 1% |
| | | | H253 (Met) - 6% | H253 (Met) - 10% | H253 (Met) - 19% |
| | | | H382 (Trp) - n.d. | H382 (Trp) - n.d. | H382 (Trp) - 1% |
| | | | H418/429 (Trp/Met) - 2% | H418/429 (Trp/Met) -3% | H418/429 (Trp/Met)-4% |
| | | | H429 (Met) - 1% | H429 (Met) - 2% | H429 (Met) - 3% |

A higher oxidation level is found on three sites of the heavy chain for the stability sample T9M at +25°C compared to the other samples. Oxidation seems to occur in specific sites during degradation of the molecule.

Data related to glycation analysis are summarized in Table 12. Peptide mapping was performed with RP-HPLC LC-ESI-MS (MS) to confirm glycation sites along with the level of glycation.

Table 12: Peptide mapping RP-HPLC LC-ESI-MS (MS)-Glycation levels

| Tests | Methods | Specification | REF1 | REF2 | Ab1 stability sample T9M (+25°C) |
|---|---|---|---|---|---|
| Glycation | Peptide mapping RP-HPLC LC-ESI-MS(MS) | Report Result for light chain (L) and heavy chain (H) | L102 - 2% | L102 - 1% | L102 - 1% |
| | | | L102/106 - 2% | L102/106 - 2% | L102/106 - 2% |
| | | | L106/125 - 1% | L106/125 - 1% | L106/125 - 1% |
| | | | L144/148 - 1% | L144/148 - 1% | L144/148 - 1% |
| | | | L144/148/168 - 1% | L144/148/168 - 1% | L144/148/168 - 1% |
| | | | L148/168 - 1% | L148/168 - 1% | L148/168 - 1% |
| | | | L168/182 - 1% | L168/182 - 1% | L168/182 - 1% |
| | | | L168/182/187/189 - 4% | L168/182/187/189 - 4% | L168/182/187/189 - 3% |
| | | | L182/187/189 - 1% | L182/187/189 - 3% | L182/187/189 - 2% |
| | | | L182/187/189/206 - 3% | L182/187/189/206 - 3% | L182/187/189/206 - 3% |
| | | | L187/189 - 3% | L187/189 - 3% | L187/189 - 2% |
| | | | L187/189/206 - 1% | L187/189/206 - 2% | L187/189/206 - 2% |
| | | | H66/73/77 - 3% | H66/73/77 - 2% | H66/73/77 - 2% |
| | | | H73/77 - 1% | H73/77 - 1% | H73/77 - 1% |
| | | | H206/211/214/215/21 9-2% | H206/211/214/215/21 9-1% | H206/211/214/215/21 9-1% |
| | | | H214/215/219/223 - 1% | H214/215/219/223 - 2% | H214/215/219/223 - 1% |
| | | | H247/249 - 2% | H247/249 - 1% | H247/249 - 2% |
| | | | H247/249/275 - 2% | H247/249/275 - 2% | H247/249/275 - 2% |
| | | | H275 - n.d. | H275 - n.d. | H275 - 1% |
| | | | H275/289/291 - 2% | H275/289/291 - 2% | H275/289/291 - 2% |
| | | | H321/323/327 - 2% | H321/323/327 - 1% | H321/323/327 - 1% |
| | | | H321/323/327/335/33 9/341 - 2% | H321/323/327/335/33 9/341- 1% | H321/323/327/335/33 9/341 - 2% |
| | | | H410/415 - 1% | H410/415 - n.d. | H410/415 - n.d. |

Glycation does not go over 4% for all amino acids of all samples. Degradation does not lead to glycation.

Data on N-terminal and C-terminal modifications are reported in Table 13. Peptide mapping was performed with RP-HPLC LC-ESI-MS (MS) to understand N and C terminal modifications.

Table 13: Peptide mapping RP-HPLC LC-ESI-MS (MS) - N-terminal and C-terminal modifications Results are similar for all samples. PyoGlutamate is slightly higher for the stability example T9M at +25°C but the difference is not significant and can be due to the method variation. No degradation on C-terminal and N-terminal sequences are detected.

| Tests | Methods | Specification | REF1 | REF2 | Ab1 stability sample T9M (+25°C) |
|---|---|---|---|---|---|
| N term sequencing | Peptide mapping RP-HPLC LC-ESI-MS(MS) | Report % PyroGlu for both heavy and light chains | 1% pyroGlu detected for N-terminal residue of light chain | 1% pyroGlu detected for N-terminal residue of light chain | 3% pyroGlu detected for N-terminal residue of light chain |
| | | | 99% pyroGlu detected for N-terminal residue of heavy chain | 99% pyroGlu detected for N-terminal residue of heavy chain | >99% pyroGlu detected for N-terminal residue of heavy chain |
| C term sequencing | | Report Result for heavy chain | Heavy chain: 89% terminal Lys clipped | Heavy chain: 88% terminal Lys clipped | Heavy chain: 88% terminal Lys clipped |

*Conclusions*

Data provide supportive evidence for the conclusion that Ab1 is stable regarding the glycation, the sequence terminal modifications, pH, appearance and concentration. Testing highlights significant differences in charge variant, oxidation and deamidation levels for stability sample T9M at +25°C compared to reference standard. Hypothesis can be made that the peak at 14min on CEX-HPLC profile could be related to oxidation or deamidation. These modifications are also translated by a decrease in acidic species and an increase in basic species on iCE profile. Nonetheless, potency of Ab1 is not impacted. A potential explanation could be that modifications identified do not occurs in target binding regions (CDR) or do not cause any change on this particular regions. During nine months, Ab1 goes through several modifications due to the stability of the molecule (including methionine/tryptophan oxidation and asparagine deamidation). All the modifications leads to a change on the global charge of the molecule and are detected thanks to charge variant tests (iCE and CEX-HPLC). The potency of the molecule is not affected by the charge variation.

Part 2

Additionally, the drug Product formulated in 15 mM L-Histidine, 150 mM Sodium Chloride, 0.01% Polysorbate 80, pH 6.25, at a concentration of 10.7 mg/mL was stored at +25 ± 2°C at different time (T) intervals, namely 1 month (M), 2, 3, 6 and 12 months; and also at + 5 ± 3°C (at 1, 2, 3, 6, 12, 18, 24, 30, 36 M) to study the stability. The results are summarized in Table 14 to Table 16.

Table 14. Stability at Long Term Stability Condition of +5 ± 3°C

| **Stability Tests** | **Specification** | **Time Interval (months)** | | | | |
|---|---|---|---|---|---|---|
| | | **Initial T0** | **T1** | **T2** | **T3** | **T6** |
| Physical appearance of liquid | Colorless to slightly yellowish, clear to slightly opalescent liquid, practically free of visual particulates | Colorless, slightly opalescent and practically free of visual particulates | Colorless, slightly opalescent and practically free of visual particulates | Clear, Colorless, practically free of particles | Clear, Colorless, practically free of particles | Clear, Colorless, practically free of particles |

(continued)

| Stability Tests | Specification | Time Interval (months) | | | | |
|---|---|---|---|---|---|---|
| | | Initial T0 | T1 | T2 | T3 | T6 |
| pH | T0 ± 0.15 (>6.14 and 6.44) | 6.29 | 6.27 | 6.33 | 6.31 | 6.32 |
| $A_{280}$ | T0 ± 10% (≥ 9.6 and ≤ 11.8 mg/mL) | 10.7 mg/mL | 10.9 mg/mL | 10.9 mg/mL | 10.8 mg/mL | 10.7 mg/mL |
| SE-HPLC | ≥ 98% | 100% | 99% | 98% | 99% | 95% |
| CGE (nonreduced) | IgG peak ≥ 90%<br>Assigned peaks ≤ 8%<br>Total other peaks ≤ 3% | IgG peak: 93%<br>Assigned peaks: 5%<br>Total other peaks: 2% | IgG peak: 93%<br>Assigned peaks: 6%<br>Total other peaks: 2% | IgG peak: 92%<br>Assigned peaks: 7%<br>Total other peaks: 2% | IgG peak: 93%<br>Assigned peaks: 6%<br>Total other peaks: 2% | IgG peak: 79%<br>Assigned peaks: 8%<br>Total other peaks: 13% |
| CGE (reduced) | Similar profile to that of the Ref.Std<br>Heavy chain peak ≥ 60%<br>Light chain peak ≥ 30%<br>Total other peaks ≤ 3% | Similar profile to that of the Ref. Std.<br>Heavy chain peak: 63%<br>Light chain peak: 36%<br>Total other peaks: 1% | similar profile to that of the Ref. Std.<br>Heavy chain peak: 65%<br>Light chain peak: 35%<br>Total other peaks: 1% | Similar profile to that of the Ref. Std.<br>Heavy chain peak: 64%<br>Light chain peak: 35%<br>Total other peaks: 1% | Similar profile to that of the Ref. Std.<br>Heavy chain peak: 63%<br>Light chain peak: 36%<br>Total other peaks: 1% | Similar profile to that of the Ref. Std.<br>Hevy chain peak: 63%<br>Light chain peak: 34%<br>Total other peaks: 3% |
| CEX-HPLC | Similar profile to that of the | Similar profile to that of the Ref. Std.<br>Acidic peak: 33%, Main peak: 38%, Basic peak: 30% | NA | NA | NA | Not similar profile to Ref. Std.<br>Acidic peak:36%, Main peak: 41%, Basic peak: 23% |
| cIEF/iCE3 | Similar profile to that of the Ref. Std.<br>Acidic peaks: ≥ 14% and ≤ 59%<br>Main peak: ≥ 33% and ≤ 54%<br>Basic peaks: ≥ 7% and ≤ 35%<br>pI main peak: ≥ 8.1 and ≤ 8.5 | Similar profile to that of the Ref. Std.<br>Acidic peaks: 40%<br>Main peak: 40%<br>Basic peaks: 20%<br>pI main peak: 8.1 | Similar profile to that of the Ref. Std.<br>Acidic peaks: 41%<br>Main peak: 40%<br>Basic peaks: 19%<br>pI main peak: 8.3 | Similar profile to that of the Ref. Std.<br>Acidic peaks: 40%<br>Main peak: 41%<br>Basic peaks:<br>19% pI main peak: 8.3 | Similar profile to that of the Ref. Std.<br>Acidic peaks: 40%<br>Main peak: 41%<br>Basic peaks: 19%<br>pI main peak: 8.3 | Not similar profile to that of the Ref. Std.<br>Acidic peaks: 52%<br>Main peak: 32%<br>Basic peaks: 16%<br>pI main peak: 8.3 |
| Binding ELISA | ≥ 50% and ≤ 150% of Ref. Std. $EC_{50}$ | 85% | 91% | 109% | 132% | 184% |

(continued)

| Stability Tests | Specification | Time Interval (months) | | | | |
|---|---|---|---|---|---|---|
| | | Initial T0 | T1 | T2 | T3 | T6 |
| Ki ELISA (competition) | Report result as % $IC_{50}$ of Ref. Std. | 80% | NA | NA | NA | 121% |
| Sub-visible particles | ≤ 600 particles of 25 µm in size or larger per container ≤ 6000 particles of 10 µm in size or larger per container | NA | NA | NA | NA | NA |
| Bioburden | Report value | NA | NA | NA | NA | NA |
| | Report value | NA | NA | NA | NA | NA |
| Sterility | No growth at 14 days | NA | NA | NA | NA | NA |

Table 15. Stability at Long Term Stability Condition of +5 ± 3°C

| Stability Tests | Specification | Time Interval (months) | | | | | |
|---|---|---|---|---|---|---|---|
| | | T9 | T12 | T18 | T24 | T30 | T36 |
| Physical appearance of liquid | Colorless to slightly yellowish, clear to slightly opalescent liquid, practically free of visual particulates | Clear, Colorless, practically free of particles | Slightly opalescent, Slightly yellowish, practically free of particles | Clear, Slightly yellowish, practically free of particles | Clear, Slightly yellowish, practically free of particles | Slightly opalescent, Slightly yellowish, practically free of particles | Clear, Slightly yellowish, practically free of particles |
| pH | T0 ± 0.15 (≥ 6.14 and ≤ 6.44) | 6.31 | 6.31 | 6.27 | 6.31 | 6.31 | 6.32 |
| $A_{280}$ | T0 ± 10% (≥ 9.6 and ≤ 11.8 mg/mL) | 10.7 mg/mL | 10.3 mg/mL | 10.5 mg/mL | 10.5 mg/mL | 10.3 mg/mL | 10.6 mg/mL |
| SE-HPLC | ≥ 98% | 99% | 99% | 98% | 98% | 99% | 98% |
| CGE (non-reduced) | IgG peak ≥ 90%<br>Assigned peaks ≤ 8%<br>Total other peaks ≤ 3% | IgG peak: 93%<br>Assigned peaks: 6%<br>Total other peaks: 2% | IgG peak: 93%<br>Assigned peaks: 5%<br>Total other peaks: 2% | IgG peak: 93%<br>Assigned peaks: 5%<br>Total other peaks: 2% | IgG peak: 93%<br>Assigned peaks: 5%<br>Total other peaks: 2% | IgG peak: 92%<br>Assigned peaks: 6%<br>Total other peaks: 2% | IgG peak: 93%<br>Assigned peaks: 6%<br>Total other peaks: 2% |
| CGE (reduced) | Similar profile to that of the Ref.Std<br>Heavy chain peak ≥ 60%<br>Light chain peak ≥ 30%<br>Total other peaks ≤ 3% | Similar profile to that of the Ref. Std.<br>Heavy chain peak: 64%<br>Light chain peak: 36%<br>Total other peaks: 1% | Similar profile to that of the Ref. Std.<br>Heavy chain peak: 63%<br>Light chain peak: 35%<br>Total other peaks: 2% | Similar profile to that of the Ref. Std.<br>Heavy chain peak: 63%<br>Light chain peak: 34%<br>Total other peaks: 3% | Similar profile to that of the Ref. Std.<br>Heavy chain peak: 63%<br>Light chain peak: 35%<br>Total other peaks: 2% | Similar profile to that of the Ref. Std.<br>Heavy chain peak: 63%<br>Light chain peak: 35%<br>Total other peaks: 2% | Similar profile to that of the Ref. Std.<br>Heavy chain peak: 64%<br>Light chain peak: 35%<br>Total other peaks: 1% |
| CEX-HPLC | Similar profile to that of the Ref. Std. | NA | Similar profile to that of the Ref. Std.<br>Acidic peak: 33%<br>Main peak: 40%<br>Basic peak: 27% | Similar profile to that of the Ref. Std.<br>Acidic peak: 28%,<br>Main peak: 45%<br>Basic peak: 27% | Similar profile to that of the Ref. Std.<br>Acidic peak: 33%,<br>Main peak: 42%,<br>Basic peak: 25% | Similar profile to that of the Ref. Std.<br>Acidic peak: 29%,<br>Main peak: 46%,<br>Basic peak: 25% | Similar profile to that of the Ref. Std.<br>Acidic peak: 33%,<br>Main peak: 43%,<br>Basic peak: 24% |

(continued)

| Stability Tests | Specification | Time Interval (months) | | | | | |
|---|---|---|---|---|---|---|---|
| | | T9 | T12 | T18 | T24 | T30 | T36 |
| cIEF/iCE3 | Similar profile to that of the Ref. Std.<br>Acidic peaks: ≥ 14% and ≤ 59%<br>Main peak: ≥ 33% and ≤ 54%<br>Basic peaks: ≥ 7% and ≤ 35%<br>pI main peak: ≥ 8.1 and ≤ 8.5 | Similar profile to that of the Ref. Std.<br>Acidic peaks: 44%<br>Main peak: 38%<br>Basic peaks: 19%<br>pI main peak: 8.3 | Similar profile to that of the Ref. Std.<br>Acidic peaks: 41%<br>Main peak: 40%<br>Basic peaks: 19%<br>pI main peak: 8.3 | Similar profile to that of the Ref. Std.<br>Acidic peaks: 44%<br>Main peak: 38%<br>Basic peaks: 18%<br>pI main peak: 8.3 | Similar profile to that of the Ref. Std.<br>Acidic peaks: 47%<br>Main peak: 35%<br>Basic peaks: 18%<br>pI main peak: 8.3 | Similar profile to that of the Ref. Std.<br>Acidic peaks: 45%<br>Main peak: 38%<br>Basic peaks: 18%<br>pI main peak: 8.3 | Similar profile to that of the Ref. Std.<br>Acidic peaks: 45%<br>Main peak: 37%<br>Basic peaks: 18%<br>pI main peak: 8.3 |
| Binding ELISA | ≥ 50% and ≤ 150% of Ref. Std. $EC_{50}$ | 144% | 89% | 132% | 112% | 118% | 150% |
| Ki ELISA (competition) | Report result as % $IC_{50}$ of Ref. Std. | NA | 101% | 186% | 131% | 142% | 65% |
| Sub-visible particles | ≤ 600 particles of 25 μm in size or larger per container<br>≤ 6000 particles of 10 μm in size or larger per container | NA | ≥ 10 μm = 150<br>≥ 25 μm = 0 | NA | ≥ 10 μm = 20<br>≥ 25 μm = 0 | NA | ≥ 10 μm = 31<br>≥ 25 μm = 7 |
| Bioburden | Report value (TM) | NA | 0 CFU/mL | NA | 0 CFU/mL | NA | NA |
| | Report value (YM) | NA | 0 CFU/mL | NA | 0 CFU/mL | NA | NA |
| Sterility | No growth at 14 days | NA | NA | NA | NA | NA | No growth |

Table 16. Stability at Accelerated Stability Condition of +25 ± 2°C

| Stability Tests | Specification | Time Interval (months) | | | | | | |
|---|---|---|---|---|---|---|---|---|
| | | Initial T0 | T1 | T2 | T3 | T6 | T9 | T12 |
| Physical appearance of liquid | Colorless to slightly yellowish, clear to slightly opalescent liquid, practically free of visual particulates | Colorless, slightly opalescent and practically free of visual particulates | Colorless, slightly opalescent and practically free of visual particulates | Clear, slightly yellowish practically free of particles | Clear, Colorless, practically free of particles | Clear, Colorless, practically free of particles | Clear, slightly yellowish practically free of particles | Clear, slightly yellowish practically free of particles |
| pH | T0 ± 0.15 (≥ 6.14 and ≤ 6.44) | 6.29 | 6.25 | 6.34 | 6.32 | 6.31 | 6.31 | 6.31 |
| $A_{280}$ | T0 ± 10% (≥ 9.6 and ≤ 11.8 mg/mL) | 10.7 mg/mL | 10.9 mg/mL | 10.8 mg/mL | 10.8 mg/mL | 10.8 mg/mL | 10.8 mg/mL | 10.3 mg/mL |
| SE-HPLC | ≥ 98% | 100% | 99% | 98% | 99% | 95% | 94% | 96% |
| CGE (non-reduced) | IgG peak ≥ 90%<br>Assigned peaks ≤ 8%<br>Total other peaks ≤ 3% | IgG peak: 93%<br>Assigned peaks: 5%<br>Total other peaks: 2% | IgG peak: 91%<br>Assigned peaks: 7%<br>Total other peaks: 2% | IgG peak: 92%<br>Assigned peaks: 6%<br>Total other peaks: 2% | IgG peak: 92%<br>Assigned peaks: 6%<br>Total other peaks: 2% | IgG peak: 78%<br>Assigned peaks: 8%<br>Total other peaks: 14% | IgG peak: 77%<br>Assigned peaks: 7%<br>Total other peaks: 16% | IgG peak: 73%<br>Assigned peaks: 8%<br>Total other peaks: 19% |
| CGE (reduced) | Similar profile to that of the Ref.Std<br>Heavy chain peak ≥ 60%<br>Light chain peak ≥ 30%<br>Total other peaks ≤ 3% | Similar profile to that of the Ref. Std.<br>Heavy chain peak: 63%<br>Light chain peak: 36%<br>Total other peaks: 1% | Similar profile to that of the Ref. Std.<br>Heavy chain peak: 64%<br>Light chain peak: 35%<br>Total other peaks: 1% | Similar profile to that of the Ref. Std.<br>Heavy chain peak: 64%<br>Light chain peak: 35%<br>Total other peaks: 1% | Similar profile to that of the Ref. Std.<br>Heavy chain peak: 64%<br>Light chain peak: 36%<br>Total other peaks: 1% | Similar profile to that of the Ref. Std.<br>Heavy chain peak: 62%<br>Light chain peak: 34%<br>Total other peaks: 1% | Similar profile to that of the Ref. Std.<br>Heavy chain peak: 64%<br>Light chain peak: 35%<br>Total other peaks: 1% | Not similar profile to that of the Ref. Std.<br>Heavy chain peak: 62%<br>Light chain peak:<br>33% Total other peaks: 4% |

(continued)

| Stability Tests | Specification | Time Interval (months) | | | | | | |
|---|---|---|---|---|---|---|---|---|
| | | Initial T0 | T1 | T2 | T3 | T6 | T9 | T12 |
| CEX-HPLC | Similar profile to that of the Ref. Std. | Similar profile to that of the Ref. Std.<br>Acidic peak: 33%<br>Main peak: 38%<br>Basic peak: 30% | NA | NA | NA | Not similar profile to that of the Ref. Std.<br>Acidic peak: 36%<br>Main peak: 41%<br>Basic peak: 23% | NA | Not similar profile to that of the Ref. Std.<br>Acidic peak: 43%<br>Main peak: 37%<br>Basic peak: 20% |
| cIEF/iCE3 | Similar profile to that of the Ref. Std.<br>Acidic peaks: ≥ 14% and ≤ 59%<br>Main peak: ≥ 33% and ≤ 54%<br>Basic peaks: ≥ 7% and ≤ 35%<br>pI main peak: ≥ 8.1 and ≤ 8.5 | Similar profile to that of the Ref. Std.<br>Acidic peaks:40 %<br>Main peak: 40%<br>Basic peaks: 20%<br>pI main peak: 8.1 | Similar profile to that of the Ref. Std.<br>Acidic peaks: 41%<br>Main peak: 40%<br>Basic peaks: 19%<br>pI main peak: 8.3 | Similar profile to that of the Ref. Std.<br>Acidic peaks: 41%<br>Main peak: 41%<br>Basic peaks: 18%<br>pI main peak: 8.3 | Similar profile to that of the Ref. Std.<br>Acidic peaks: 47%<br>Main peak: 35%<br>Basic peaks: 18%<br>pI main peak: 8.3 | Not similar profile to that of the Ref. Std.<br>Acidic peaks: 25%<br>Main peak: 58%<br>Basic peaks: 16%<br>pI main peak: 8.3 | Similar profile to that of the Ref. Std.<br>Acidic peaks: 29%<br>Main peak: 55%<br>Basic peaks: 15%<br>pI main peak: 8.3 | Not similar profile to that of the Ref. Std.<br>Acidic peaks: 32%<br>Main peak: 54%<br>Basic peaks: 14%<br>pI main peak: 8.3 |
| Binding ELISA | ≥ 50% and ≤ 150% of Ref. Std. $EC_{50}$ | 85% | 88% | 113% | 137% | 159% | 134% | 47% |
| Ki ELISA (competition) | Report result as % $IC_{50}$ of Ref. Std. | 80% | NA | NA | NA | 201% | NA | 199% |
| Sub-visible particles | ≤ 600 particles of 25 μm in size or larger per container<br>≤ 6000 particles of 10 μm in size or larger per container | NA | NA | NA | NA | NA | NA | ≥ 10 μm = 50<br>≥ 25 μm = 0 |

(continued)

| Stability Tests | Specification | Time Interval (months) | | | | | | |
|---|---|---|---|---|---|---|---|---|
| | | Initial T0 | T1 | T2 | T3 | T6 | T9 | T12 |
| Sterility | No growth at 14 days | NA | NA | NA | NA | NA | NA | No growth after 14 days |

Ab1 drug product is stable for 36 months when stored at + 5 ± 3°C in terms of purity (based on SE-HPLC and cGE), charge profile (based on iCE3 and CEX-HPLC), protein content (A280), binding to target (ELISA) and subvisible particle content. Physical appearance and pH values are also within specifications.

**Example 3: Concentration of the Ab formulation**

Concentration of Ab1 liquid formulation

In order to develop high concentration Ab1 liquid formulation, UFDF (Ultrafiltration Diafiltration), UFDF2, was tested. In particular the target concentration was between about 160 and 195 mg/mL.

*Material and Methods*

Bulk drug substance (BDS)

The characteristics of the starting material BDS Ab1-lot1 (30C (thawed at 30°C)) are in Table 17:

Table 17: Starting BDS material data

| BDS material | Ab1-lot1 (30C) |
|---|---|
| pH | 6.0 |
| Conductivity (mS/cm) | 0.57 |
| Osmolality (mOsm/kg) | 12 |
| Protein concentration (mg/mL) | 69.6 |

Buffers

The two buffers used for this study are shown in Table 18:

Table 18: UFDF2 buffers composition

| Buffer No. | Composition |
|---|---|
| Buffer 1 | 25 mM L-Histidine, 150 mM sodium chloride, pH 6.0 |
| Buffer 2 | 25 mM L-Histidine, 150 mM L-Arginine hydrochloride, pH 6.0 |

Equipment

30 kDa cut-off Omega centramate T series cassette with 0.019 $m^2$ area from Pall was used for this UFDF2 development. A new single multiple-use cassette was used for each trial. Cogent μscale Tangential Flow Filtration system (DI-DSP-078) from Millipore was used to perform the UFDF2 development. A 120U/DV peristatic pump from Watson Marlow was used to provide a continuous feed during diafiltration step. Nanodrop 2000 from Thermo Scientific was used for protein concentration determination. SevenExcellence pH-meter was used for pH and conductivity measurements. HIAC 9703 particle counter (HACH, Skan AG) was used for subvisible particles estimation. Lovis 2000 ME microviscosimeter from Anton Paar was used for dynamic viscosity measurements.

Analytics method

Samples protein content was determined by $A_{280}$ using Thermo Scientific Nanodrop 2000. SE-HPLC and Subvisible particles analysis were performed on pre- and post- concentration steps to determine if any aggregation was induced due to UFDF process. IEF by iCE3 was also run to see any change in the charge variants for the same reason. Conductivity were measured on input, permeate and retentate samples. pH and osmolality were also measured on initial, diafiltrated and concentrated final material.

UFDF process

Two different trials were performed to concentrate Ab1 BDS in desired buffers. Same starting BDS was used, but parameters were optimized in between. UFDF process flowcharts and parameters are detailed in Figure 6 and Table 19. Two concentration steps had to be performed in trial 1 as the parameters were not optimized. As a consequence, feed overpressures happened with both buffers in trial 1 before concentration target was reached. Thus concentration have been performed in two steps during this trial.

Table 19: Comparison table of operational UFDF parameters used for trial 1 and trial 2

| Parameters | Trial 1 | | Trial 2 | |
|---|---|---|---|---|
| | Buffer 1 | Buffer 2 | Buffer 1 | Buffer 2 |
| Diafiltration step | | | | |
| Initial volume (mL) | 200 | 200 | 175 | 175 |
| Pump flow (mL/min) | 107 | 107 | 49.5 | 49.5 |
| Peristaltic pump speed (rpm) | 20 | 20 | 13 | 15 |
| Stirring speed | 50 | 50 | 50 | 50 |
| No. of diavolumes | 4 | 4 | 4 | 4 |
| TMP (bar) | 0.8-0.9 | 0.8-0.9 | 0.6-0.7 | 0.6-0.7 |
| Temperature (°C) | 26-29 | 25-28 | 25-27 | 25-27 |
| Concentration step | | | | |
| Initial volume (mL) | 200 | 200 | 175 | 175 |
| Final volume (mL) | 50 | 50 | 70 | 60 |
| Pump flow (mL/min) | 82.5 to 24.8 | 99.0 to 16.5 | 49.5 to 16.5 | 49.5 to 16.5 |
| Stirring speed | 50 | 50 | 50 | 50 |
| TMP (bar) | 0.7-1.2 | 0.5 to 1.1 | 0.6-0.7 | 0.6-0.7 |
| Temperature (°C) | 25-29 | 25-28 | 25-28 | 25-27 |

$C_{gel}$ determination

During concentration step, many data points were taken, by regularly recording feed and retentate pressures, permeate volume and flux, and temperature. By determining theoretical concentration on the retentate ($C_r$) at each point (Equation 1), it is possible to use the gel theory to determine the theoretical gel concentration, or $C_{gel}$, maximum possible retentate concentration before membrane clogs.

Equation 1: determination of theoretical retentate concentration:

$$V_{retentate} = V_{initial} - V_{permeate} \text{ and } C_r = (C_{initial} \cdot V_{initial}) / V_{retentate})$$

Using the linear trend line of *Permeate flux* = f[ln($C_r$)] plot in semi-log scale Figure 7, it is possible to define K which is the slope of the trend line and b its intercept, and finally the gel theory can predict the $C_{gel}$ value (Equation 2). Permeate flux is expressed in $L.m^{-2}.h^{-1}$ (abbreviated LMH in Figure 7).

Equation 2: gel theory to predict gel concentration

$$C_{gel} = e^{(b/-K)}$$

*Results*

Diafiltration was performed with 4 diavolumes on each run. 5 diavolumes is a safer margin to be sure the diafiltration step is complete, but the permeate conductivities obtained are within the target conductivity ± 3% (see table 5 for buffer conductivity target). The conductivity trend during diafiltration can be found in Figure 8.

Using calculations explained above and after data collection, theoretical $C_{gel}$ has been estimated on trial 2 with both buffer conditions (see Table 20). For both buffers, theoretical $C_{gel}$ reaches about 200 mg/mL.

Table 20: Theoretical Cgel estimation on Trial 2

| Buffer used | Buffer 1 | Buffer 2 |
|---|---|---|
| Cgel estimation chart: Normalized filtrate flux = f[log($C_r$)] | Figure 9A | Figure 9B |
| K (slope factor) | -14.63 | -19.19 |
| B (X-intercept) | 77.727 | 101.85 |
| Theoretical Cgel | 202.9 mg/mL | 201.8 mg/mL |

Yields (or material recovery) of the UFDF processes were estimated. These were estimated on the concentrated product before flush. In production condition, the system must be flushed with fresh buffer and this flushing product must be mixed with the concentrated product. In this study, system was flushed to "clean" the system and membrane, but flushing product was not mixed with the concentrated product because of concern on the final BDS concentration. The yields obtain are compared in Table 21. As better process parameters was used for Trial 2, its yield values are more relevant in the concern of a feasibility study.

Table 21: UFDF process yields (without flush)

| | UFDF in Buffer 1 | UFDF in Buffer 2 |
|---|---|---|
| Trial 1 Yield | 58.5 % | 68.6 % |
| Trial 2 Yield | 93.8 % | 84.9 % |

Once the diafiltration has ended, the product's pH, conductivity, osmolality and protein concentration was measured to be compared with starting material characteristics. Analysis were also performed after concentration step. These data can be found inTable 22.

Consistency of the process can be interpreted from these results. However some unexpected results were observed; an increasing of pH was observed on each run during the concentration step. This phenomenon is linked to the Gibbs-Donnan's effect, which occurs in this range of protein concentration. This induces a gradient of proton concentration between the two sides of the membrane which explains the pH change during the process.

Differences of conductivity values was also observed between the retentate and permeate after diafiltration, and conductivity of retentate decreased further during concentration step of each runs. This could be linked to the protein concentration but has to be investigated.

Maximum protein concentration achieved in buffer 1 is about 164 mg/mL and could be assimilated to an experimental $C_{gel}$, as concentration had to be stopped to avoid feed pump overpressure. Buffer 2 allowed to reach higher protein concentration of 192 mg/mL in trial 1. In trial 2, concentration in buffer 2 was stopped until target concentration was reached (173 mg/mL).

Table 22: IPC physico-chemical data collected during the different UFDF trials

| Composition | Samples | Protein Concentration (mg/ml) | pH | Conductivity (mS/cm) | Osmolality (mosm/kg) |
|---|---|---|---|---|---|
| Starting material | Initial Ab1-lot1(30C) | 70 | 6.38 | 0.57 | 12 |

(continued)

| Composition | Samples | Protein Concentration (mg/ml) | pH | Conductivity (mS/cm) | Osmolality (mosm/kg) |
|---|---|---|---|---|---|
| Buffer 1 | Trial 1 | | | | |
| | UFDF buffer | NA | 6.00 | 16.30 | 316 |
| | Diafiltration end product | 68 | 6.06 | 13.65 | 315 |
| | 1st Concentration end product | 103 | 6.06 | 11.99 | 317 |
| | 2nd Concentration end product | 164 | 6.11 | 10.11 | 308 |
| | Trial 2 | | | | |
| | UFDF buffer | NA | 6.00 | 16.20 | 305 |
| | Diafiltration end product | 71.2 | 6.01 | 13.60 | 305 |
| | Concentration end product | 164.1 | 6.02 | 10.80 | 313 |
| Buffer 2 | Trial 1 | | | | |
| | UFDF buffer | NA | 6.00 | 12.06 | 297 |
| | Diafiltration end product | 64 | 5.99 | 10.64 | 297 |
| | 1st Concentration end product | 141 | 6.03 | 8.51 | 306 |
| | 2nd Concentration end product | 192 | 6.08 | 7.31 | 291 |
| | Trial 2 | | | | |
| | UFDF buffer | NA | 6.00 | 12.06 | 297 |
| | Diafiltration end product | 69.4 | 6.01 | 10.0 | 288 |
| | Concentration end product | 173.4 | 6.05 | 8.0 | 292 |

Final BDS post UFDF products were analysed with starting material in SE-HPLC runs. The profile obtained proved consistency in monomer, aggregates and fragments repartition between starting and ending BDS with both buffers, and there is reproducibility between both trials' size variants (see Figure 10). Final BDS post UFDF products were analysed with starting material in IEF runs on iCE 3 system. The profile obtained proved consistency in charge variants repartition and pI ranges between starting and ending BDS with both buffers, and was comparable with the reference standard used (Figure 11 and Figure 12).

Subvisible particles analysis was also performed to have an insight on the UFDF products content. The values obtained are compiled in Table 23.

Table 23: Subvisible particles (SVP) count from each post-UFDF products

| | | SVP (particles/mL) | | | |
|---|---|---|---|---|---|
| | | ≥ 2 μm | ≥ 5 μm | ≥ 10 μm | ≥ 25 μm |
| Trial 1 | Buffer 1 | 24,920 | 6,990 | 1,305 | 30 |
| | Buffer 2 | 13,990 | 4,605 | 945 | 40 |
| Trial 2 | Buffer 1 | 29,365 | 7,400 | 1,740 | 135 |
| | Buffer 2 | 17,745 | 3,950 | 1,115 | 55 |

In addition to above analysis, the viscosity of these two formulations were also measured using a Rolling-ball viscometer. The Lovis dynamic viscosities were observed to be 12.25 mPa·s and 11.38 mPa·s at 25°C for formulation containing NaCl and L-Arginine HCl respectively. However, during this analysis, the standard (water) was expired and also it did not passed specifications.

*Conclusions*

The main aim of this study was achieved as it showed that it is possible to concentrate Ab1 in both the selected buffers at a protein concentration >150 mg/mL. However different optimization have to be performed to assess the feasibility of this process for a scale-up, e.g. including a flush step before retrieving the post-UFDF end product.

This study give an insight on the consistency of the BDS stability against the UFDF process in these conditions, and it can give hints to justify an UFDF buffer selection, as buffer 2 (25 mM L-Hisitidine, 150 mM L-Arginine-HCl, pH 6.0) allows to reach higher concentration compared to buffer 1 (25 mM L-Hisitidine, 150 mM NaCl, pH 6.0).

**Example 4: PFS development**

Part 1: Force Degradation Studies and PFS characteristics analysis

Two formulation compositions were evaluated for forced degradation studies and for pre-fillable syringe (PFS) development:

(F1) 25 mM L-Histidine, 150 mM Sodium chloride, pH 6.0 and
(F2) 25 mM L-Histidine, 150 mM L-Arginine Hydrochloride, pH 6.0.

*Material and Methods*

Ab1-lot1(30C) was diafiltered into two different formulation buffers separately and were concentrated further to achieve a protein concentration of > 164 mg/mL. To each of the UF/DF 2 retentate solution, a calculated amount of Polysorbate 80 was added while adjusting protein concentration to 150 mg/mL to generate Formulated bulk drug substance. The formulated bulk solution was filtered through 0.22 μ PES membrane based filter and was filled into 2.25 mL glass PFS system manually without $N_2$ gas flushing. This would provide a worst case scenario. The formulation composition and role of each ingredients of both Ab1 drug product in PFS (300 mg/2 mL) is summarised in below Table 35.

Table 24: Formulation composition and role of each ingredient

| Ingredients | Composition (mg/mL) | | Role of each ingredient |
|---|---|---|---|
| | Ab1-Sample1 | Ab1-Sample2 | |
| Ab1 | 150 | 150 | Active Pharmaceutical Ingredients |
| L-Histidine | 3.9 | 3.9 | Buffering agent |
| 3.7 %w/w Hydrochloric acid | 0.4 | 0.4 | For pH adjustment |
| Sodium chloride | 8.8 | NA | Stabilizer / Tonicity modifier |
| L-Arginine hydrochloride | NA | 31.6 | Stabilizer / Tonicity modifier |
| Polysorbate 80 | 0.36 | 0.36 | Stabilizer/surfactant |
| 50 %w/w Sodium hydroxide | q.s. | q.s. | For pH adjustment |
| Target pH | 6.0 | 6.0 | NA |

A small scale forced degradation study using Formulated bulk drug substance (150 mg/mL) was performed in order to understand the molecular stability of Ab1 under various stress conditions. About 1 mL of filtered Formulated bulk drug substance was filled into 2 mL glass vials and was stoppered with 20 mm injection rubber stopper. The vials were subjected to multiple stresses as listed below for defined period:

- Storage at + 40 °C for 1 week

- Shaking at 900 rpm for 72 hours at room temperature

- 3 Freeze-thaw cycles by freezing at -80 °C ± -20 °C and thawing at +5 °C ± 3 °C
- 3 Freeze-thaw cycles by freezing at -80 °C ± -20 °C and thawing at +25 °C ± 2 °C
- The control (unstressed) sample was stored at +5 °C ± 3 °C

Ab1 drug product quality was monitored using several analytical techniques as listed in Table 36.

Table 25: Details of analytical test performed and their justification

| Analytical test | Justification for test |
|---|---|
| Visual Appearance | A significant change in the appearance of sample may indicate product degradation and/or microbial contamination leading to safety risk for the patients. Hence it is necessary to monitor appearance of solution. |
| Protein content (by A280) | Ab1 is required to be administered at a target conc. of 150 mg/mL. Any significant variation from its target concentration would not provide effective dose to patients. Hence it is important to measure A280 values. |
| Size variants by SE-HPLC | Changes in monomeric content of Ab1 is an indication of its degradation. The aggregates (higher size than monomer) or fragments (smaller size than monomer) may be detected by sensitive technique like SE-HPLC. Hence it was monitored |
| Size variants by cGE (reduced & non-reduced) | Changes in monomeric content of Ab1 is an indication of its degradation. The aggregates (higher size than monomer) or fragments (smaller size than monomer) may be detected by sensitive technique like cGE which can be used as an orthogonal technique to SE-HPLC. Hence it was analysed. |
| Charge variants by cIEF | Changes in content of charged variants of Ab1 is an indication of its degradation. The acidic and basic charged variants can be analysed by sensitive technique like cIEF. Hence it was measured. |

*Results forced degradation study*

The data of appearance, pH and protein content (by A280) are populated in below table.

Table 26: Physico-chemical analysis data

| Stress conditions | Ab1-Sample1 | | | Ab1-Sample2 | | |
|---|---|---|---|---|---|---|
| | Appearance | pH | Protein content (mg/mL) | Appearance | pH | Protein content (mg/mL) |
| Control | Clear, slightly yellowish | 6.05 | 156.0 | Clear, slightly yellowish | 6.08 | 150.7 |
| Shaking @ 900 rpm 72h RT | Clear, slightly yellowish | 6.04 | 154.3 | Clear, slightly yellowish | 6.08 | 153.3 |
| Storage @ 40°C, 1 week | Clear, slightly yellowish | 6.04 | 149.2 | Clear, slightly yellowish | 6.08 | 147.5 |
| 3F/T cycles @ -80 °C (F) 5 °C (T) | Clear, slightly yellowish | 6.03 | 150.4 | Clear, slightly yellowish | 6.11 | 151.0 |
| 3F/T cycles @ -80 °C (F) 25 °C (T) | Clear, slightly yellowish | 6.04 | 150.7 | Clear, slightly yellowish | 6.08 | 149.5 |

The data of SE-HPLC analysis are shown in Figure 14. The cIEF by iCE3 analysis data are summarized in Figure 15. The cGE (non-reduced) and cGE (reduced) analysis data of Ab1-Sample1 are shown in Figure 16 and Figure 17 respectively. The cGE (non-reduced) and cGE (reduced) analysis data of Ab1-Sample2 are shown in Figure 18 and Figure 19 respectively.

Based on the data of forced degradation study it can be concluded that no significant change in physico-chemical attributes of Ab1-Sample1 and Ab1-Sample2 were observed across all stressed samples. The data from protein stability (SE-HPLC, cGE (reduced and non-reduced) and cIEF by iCE3) indicated that there was no major impact on size and charge variants between the control and stressed samples.

Based on all analytical data, it was observed that heat stress (storage at 40°C for 1 week) was relatively most destabilizing for the Ab1 in both Ab1-Sample1 and Ab1-Sample2. However both these products seem to be stable concerning multiple F/T cycles and shaking stress.

*PFS development*

An Ab1 drug product in PFS (300 mg/2 mL) has been developed for subcutaneous injection using an autoinjector. The final product shall be designed to deliver 2 mL of solution containing Ab1 concentration at 150 mg/mL with high accuracy of dose and minimum pain to the patients. Hence it’s important to select an appropriate combination of PFS barrel and plunger stopper.

The key parameters that would impact the delivery of 2 mL of Ab1 solution are Syringe plunger diameter, Needle length, Needle diameter, Viscosity of solution. The Hagen-Poiseuille Equation is mentioned below that can be used for calculation of plunger force required for delivery of s solution through a PFS.

$$F = (128\ Q\mu LA) / \pi * D^4$$

F = Plunger force (in N)
Q = Volumetric flow ate (in $cm^3$/min)
$\mu$ = Dynamic viscosity of solution (in N*min/$cm^2$)
L = Needle length (in cm)
D = Needle bore diameter (in cm)
A = Syringe plunger area (in $cm^2$)
$\pi$ (pi) = 3.14159

Assuming 2 mL of Ab1 (150 mg/mL) solution with a dynamic viscosity of 0.02 Pa.s (20 centipoise) is to be delivered using an autoinjector, the following two scenarios could be derived suing above equation.

Table 27: Theoretical plunger force and delivery time of Ab1 (150 mg/mL)

| Needle Gauge (G) | Plunger force (N) required to deliver 2 mL of solution in 15 sec | Time to inject 2 mL (sec) using 10N plunger force |
|---|---|---|
| 26 | 4.86 | 7.28 |
| 27 | 10.88 | 16.32 |
| 29 | 19.36 | 29.04 |
| 30 | 34.72 | 52.08 |

Based on above table formulation composition shall be such selected that not only prevents the Ab1 (at 150 mg/mL) from degradation but also assist in its complete delivery with maximum patient compliance (lower injection force).

Part 2: Testing of different PSF configurations

Based on the analysis above, four different PFS configurations were tested with one exemplary Ab1 formulation (composition given in Table 39).

Table 28: Composition of exemplary Ab1 formulation tested.

| Protein content | Polysorbate 80 level | Other excipients | pH |
|---|---|---|---|
| 165-181 mg/mL | 0.1% (w/v) | 25 mM L-Histidine<br>150 mM arginine-HCl | 6.0 ± 0.1 |

Table 40 summarizes the different PFS materials and configurations tested. The Ab1 test formulation (Table 39) covered two main aspects: (i) a high Ab1 protein content of 165 mg/ml (higher range of target concentration of 150 ± 15 mg/ml), and (ii) a high PS80 content of 0.1% (w/v) (about 3x the target concentration of 0.036 % (w/v)), maximizing potential interactions with the packaging components of PFS. The high protein concentration was achieved by using an ultrafiltration process.

The test formulation was fully-formulated in 25 mM L-Histidine, 150 mM L-Arginine-HCl, 0.1% (w/v) Polysorbate 80 at a pH of 6.0, via spiking of excipients. The test formulation was filled manually into four different types of PFS barrels and was closed with a Stopper1 PFS stopper (Table 40). The samples were tested directly after preparation (T0) and after stressing under freeze-thaw (T-FT) and heat, namely storage for 2 weeks at 40°C (T2w_40C), conditions. Methods used for characterization included visual inspection, Micro-Flow Imaging (MFI) and syringeability.

Table 29: Overview of PFS configurations tested

| **Different PFS configurations** | |
|---|---|
| PFS1 | Barrel1 + Stopper1 |
| PFS2 | Barrel2 + Stopper1 |
| PFS3(i) | Barrel3(i) + Stopper1 |
| PFS4 | Barrel4 + Stopper1 |

*Materials*

Ab1 BDS material at a concentration of 71 mg/ml in 5 mM histidine at pH 6 was used. Highly purified water (as defined by the European Pharmacopoeia) and in the following named "water" was prepared in-house by using a Milli-Q system (Merck Millipore). The water is characterized by a resistivity value of 18.2 MΩ cm (at 25 °C).

Table 30: Overview of PFS materials tested.

| PFS | Description | Siliconization per PFS |
|---|---|---|
| Barrel1 | 2.25 glass barrel with 27G ½" staked needle | 0.5 ± 0.2 mg |
| Barrel2 | 2.25 glass barrel with 27G ½" staked needle | 0.7 ± 0.3 mg |
| Barrel3(i) | 2.25 glass barrel with 27G ½" staked needle | 0.7 ± 0.2 mg |
| Barrel4 | 2.25 glass barrel with 27G ½" staked needle | 0.55 ± 0.25 mg |
| Stopper1 | 1-3ml plunger halobutyl | none |

*Sample preparation*

Ab1 BDS material was concentrated up to 185 mg/ml by a TFF process. Sample aliquots of 2.0 ml Ab1 test formulation were filled manually into 2.25-ml siliconized syringes (Barrel1, Barrel4, Barrel2, Barrel3(i)) under LAF conditions. A Multipette Xstream (Eppendorf) equipped with a sterile 10-ml Combitip (suitable for high viscosities) was used for transferring the formulation into the syringes. The syringes were closed with plungers (Stopper1) by using a Groninger SVH 100V inserting and closing machine. The machine was set to create a headspace of 2 - 3 mm above the liquid inside the syringes (complete detachment of stopper and liquid were ensured).

*Stress /storage conditions*

Freeze-thaw stress: the formulations in PFS (each containing 2.0 ml of formulation) were exposed in horizontal positioning to five (manual) cycles of freeze-thaw stress between room temperature (20 °C to 25 °C) and -20 °C (-15 °C to - 25 °C). Samples equilibrated to room temperature were placed manually into a -20 °C-freezer and kept at -20 °C for a minimum of two hours. Subsequently, the frozen samples were thawed again at room temperature for a minimum of two hours (complete thawing was ensured by visual inspection). This procedure was performed 5 times.

Heat stress: the formulations were stored in PFS (each containing 2.0 ml formulation) in a horizontal positioning at 40 °C/75% RH. in a climate chamber for two weeks.

*Analytical methods*

Table 42 provides an overview of the sampling time points and analytical methods of this study.

Table 31: Overview of stress conditions and analytical methods for characterization of samples; n.a. = not applicable.

| **Analytical methods** | **Stress conditions** | | |
|---|---|---|---|
| | **T0** | **T-FT** | **T2w_40C** |
| MFI | n=3 for each test speed | n=3 | n=3 for each test speed |
| Visual inspection | all PFS units (n=7) | all PFS units (n=4) | all PFS units (n=7) |
| Syringeability (2 test speeds) | n=3 for each test speed | n.a. | n=3 for each test speed |

Visual inspection

The vials were inspected for the presence or absence of visible particles under gentle, manual, radial agitation for 5 seconds in front of a white background and for 5 seconds in front of a black background according to the European Pharmacopoeia (9th edition; monograph 2.9.20) at 2,000 - 3,750 lux. The inspection was performed independently by two trained examiners.

To classify the observed visible particles, a number score on the basis of the "Deutscher Arzneimittel-Codex" (DAC 2006) was used, as listed in Table 43. Fiber-like structures and particles that are likely non-inherent to the product are not accounted.

Table 32: Number score for visible particles, excluding fibers and particles that are likely non-inherent.

| **Score for visible particles** | **Description** |
|---|---|
| **0** | No particles visible within 5 sec |
| **1** | Few particles visible within 5 sec |
| **2** | Medium number of particles visible within 5 sec |
| **10** | Large number of particles directly visible |

Syringeability

Syringeability testing (n = 6) was performed on PFS samples by using a Zwickline syringeability system (Zwick GmbH, Ulm, Germany). The force profile was recorded for expelling the entire content of each PFS at a pre-defined speed by using TestXpert II software Version V3.7. For three PFS units, the test speed was set to expel the PFS content in 20 seconds and for another three PFS in 10 seconds. The approximate travel distance of the PFS stopper for expelling the entire content of each PFS was estimated by using a ruler and defined individually for each type of syringe (barrel geometries were considered). The test speeds applied are summarized in Table 44. Measurements were terminated after the maximum force of 80 N was exceeded during analysis (STOP criterion for measurements). The three PFS samples expelled at the same speed were collected in a sterile, particle-free container (e.g., a 15-ml Falcon tube) and used directly for MFI analysis.

Table 33: Settings applied for expelling PFS content

| **PFS type** | **Travel distance [mm]** | **Speed applied to expel 2.0 ml of sample within 20 s [mm/min]** | **Speed applied to expel 2.0 ml of sample within 10 s [mm/min]** |
|---|---|---|---|
| Barrel1 | 35 | 105 | 210 |
| Barrel2 | 35 | 105 | 210 |
| Barrel3 (i) | 36 | 108 | 216 |
| Barrel4 | 35 | 105 | 210 |

The (i) break loose force and the (ii) gliding force were determined (i) as the temporary, initial maximum at the beginning of the force profile and (ii) as the plateau level of the force profile, respectively, and reported.

Micro-Flow Imaging (MFI)

Micro-Flow Imaging measurements were conducted with a MFI-5200 particle analyzer system equipped with a silane-coated high-resolution 100-μm SP3 flow cell.

MFI View System Software (MVSS) version was used to perform the measurements and Lumetics LINK software was used to analyze the samples. The concentrations of particles ≥ 1 μm, 2 μm, 5 μm, 10 μm, and 25 μm as well as exemplary particle images of particles ≥ 5 μm were reported. Particle populations' ≥ 5 μm were divided into silicone oil droplet-like particles and non-silicone oil droplet-like particles, if applicable.

*Results*

Visual inspection

The results of the visual inspection are shown in Table 45. At T0, all samples were free of visible particles and exhibited a yellow coloration. After freeze-thaw as well as heat-stressing the samples showed no or few visible particles and a yellow coloration irrespective of the PFS barrel type.

Table 34: Results of visual inspection of Ab1 formulation

| **Sample** | | **Visual inspection (individual PFS units)** | |
|---|---|---|---|
| **Time point** | **PFS barrel type** | **DAC score examiner 1** | **DAC score examiner 2** |
| **T0** | Barrel1 | 0/0/0/0/0/0/0 | 0/0/0/0/0/0/0 |
| | Barrel2 | 0/0/0/0/0/0/0 | 0/0/0/0/0/0/0 |
| | Barrel3(i) | 0/0/0/0/0/0/0 | 0/0/0/0/0/0/0 |
| | Barrel4 | 0/0/0/0/0/0/0 | 0/0/0/0/0/0/0 |
| **T-FT** | Barrel1 | 0/0/1/0 | 0/0/0/0 |
| | Barrel2 | 0/0/1/0 | 0/0/0/0 |
| | Barrel3(i) | 0/0/0/0 | 0/0/0/0 |
| | Barrel4 | 0/0/1/0 | 0/0/0/0 |
| **T2w_40C** | Barrel1 | 0/0/0/0/0/0/0 | 0/0/0/0/0/0/0 |
| | Barrel2 | 0/0/0/0/0/0/0 | 0/0/1/0/0/0/0 |
| | Barrel3(i) | 1/0/1/0/1/1/1 | 0/0/0/0/0/0/1 |
| | Barrel4 | 1/0/1/0/0/1/1 | 1/0/0/0/0/0/1 |

Syringeability

Syringeability measurements were performed at T0 and T2w_40C. The content (2 ml) of each syringe was expelled within 10 and 20 s (n = 3 each). The staked needle was 27G ½" for all PFS configurations tested. The content expelled in each triplicate measurement was collected in a 15-ml Falcon tube and used for MFI measurements as described above. Results of the break loose and gliding forces are shown in Table 46. The mean force profiles are given in Figure 20.

Table 35: Results of break loose and gliding forces of Ab1 formulation in WP 3A for expelling the entire PFS content within 10 and 20 s, respectively.

| Time point | PFS barrel type | Break loose force [N] Mean (n=3) | | | | Gliding force [N] Mean (n=3) | | | |
|---|---|---|---|---|---|---|---|---|---|
| | | 10 s | | 20 s | | 10 s | | 20 s | |
| | | Mean | SD | Mean | SD | Mean | SD | Mean | SD |
| T0 | Barrel1 | 3.6 | 0.3 | 2.2 | 1.1 | 12.7 | 0.2 | 6.9 | 0.3 |
| | Barrel2 | 3.5 | 0.2 | 2.8 | 0.5 | 12.5 | 0.6 | 6.5 | 0.2 |
| | Barrel3(i) | 3.4 | 0.1 | 2.5 | 0.3 | 11.2 | 0.7 | 5.6 | 0.2 |
| | Barrel4 | 3.2 | 0.9 | 3.1 | 0.1 | 30.3 | 0.9 | 15.7 | 0.2 |
| T2w_ 40C | Barrel1 | 4.5 | 0.5 | 3.6 | 0.4 | 11.8 | 0.2 | 6.3 | 0.1 |
| | Barrel2 | 4.1 | 0.7 | 3.7 | 0.3 | 11.1 | 0.2 | 6.2 | 0.2 |
| | Barrel3(i) | 4.2 | 0.5 | 3.3 | 0.5 | 10.4 | 0.4 | 5.4 | 0.6 |
| | Barrel4 | 4.7 | 0.1 | 3.8 | 0.3 | 29.4 | 1.6 | 16.0 | 0.6 |

Gliding forces were comparable for Barrel1, Barrel2 and Barrel3(i). In detail, values for gliding forces remained below 13 N for expelling the PFS content within 10 s and below 7 N for expelling the PFS content within 20 s. Gliding forces required to expel the content of Barrel4 PFS were higher compared to the other PFS, i.e., 30 N for expelling the PFS content within 10 s and 16 N for expelling the PFS content within 20 s. After heat stressing, the break loose forces increased slightly, whereas the gliding forces decreased slightly, irrespective of the PFS barrel type.

*Micro-Flow Imaging (MFI)*

MFI analysis was performed on the 50-mg/ml stock dilutions of the Ab1 test formulation in order to assess the number of sub-visible particles. The results of the MFI measurements are shown in Figure 21 to Figure 24, as well as Table 47 to Table 51. For particle levels ≥ 5 μm a shape analysis was performed to distinguish between non-silicone oil droplet-like particles and silicone oil droplet-like particles.

At T0, all samples showed less than 10,000 particles ≥2 μm per ml, except sample in Barrel2 PFS, which showed a slightly higher number of sub-visible particles (15,000 particles ≥2 μm per ml). After stressing, the particle levels ≥ 2 μm increased slightly compared to T0, but remained overall low for both stress conditions.

The impact of FT stress was least for Barrel1 PFS and highest for Barrel4 PFS. The impact of heat stress was highest for Barrel3(i), especially when expelling the PFS content in 10 s. The impact of ejection speed was overall less pronounced for Barrel1 and Barrel2 PFS compared to Barrel3(i) and Barrel4 (higher particle levels observed when expelled at higher speed of 10 s/2 ml).

Performing shape analysis to distinguish between non-silicone oil droplet-like particle and silicone oil droplet-like particle fractions, Barrel2 PFS exhibited the largest numbers of silicone oil droplet-like particles in the size regions ≥ 5 μm, ≥ 10 μm and ≥ 25 μm, most pronounced at T0.

Overall, all samples were characterized by very low numbers of sub-visible particles with only small differences between the four PFS barrel types.

Table 36: Cumulative concentration of particles ≥ 2 μm determined by MFI; values are reported as the mean of n = 3 measurements after expelling the PFS content; values are reported not corrected for the dilution factor (ca. 3-fold); n.a. = not analyzed.

| Time point | PFS barrel type | Cumulative concentration of particles ≥2 μm [#/ml], n=3 | | | | | |
|---|---|---|---|---|---|---|---|
| | | 10 s (syringeability) | | Manually in ca. 15 s | | 20 s (syringeability) | |
| | | Mean | SD | Mean | SD | Mean | SD |
| T0 | Barrel1 | 4,330 | 1,053 | n.a. | n.a. | 4,388 | 259 |
| | Barrel2 | 14,777 | 2,637 | n.a. | n.a. | 13,840 | 977 |
| | Barrel3(i) | 10,024 | 955 | n.a. | n.a. | 4,536 | 913 |
| | Barrel4 | 7,260 | 1,025 | n.a. | n.a. | 2,462 | 507 |

(continued)

| Time point | PFS barrel type | Cumulative concentration of particles ≥2 μm [#/ml], n=3 | | | | | |
|---|---|---|---|---|---|---|---|
| | | 10 s (syringeability) | | Manually in ca. 15 s | | 20 s (syringeability) | |
| | | Mean | SD | Mean | SD | Mean | SD |
| T-FT | Barrel1 | n.a. | n.a. | 10,700 | 131 | n.a. | n.a. |
| | Barrel2 | n.a. | n.a. | 20,071 | 189 | n.a. | n.a. |
| | Barrel3(i) | n.a. | n.a. | 14,333 | 126 | n.a. | n.a. |
| | Barrel4 | n.a. | n.a. | 15,073 | 609 | n.a. | n.a. |
| T2w_40C | Barrel1 | 10,220 | 491 | n.a. | n.a. | 7,157 | 640 |
| | Barrel2 | 10,047 | 232 | n.a. | n.a. | 5,621 | 248 |
| | Barrel3(i) | 19,743 | 1,896 | n.a. | n.a. | 10,983 | 545 |
| | Barrel4 | 10,080 | 1,013 | n.a. | n.a. | 7,892 | 1,101 |

Table 41: Cumulative concentration of non-silicone oil droplet-like particles ≥ 5 μm determined by MFI (and LINK software); values are reported as the mean of n = 3 measurements after expelling the PFS content; values are reported not corrected for the dilution factor (ca. 3-fold); n.a. = not analyzed.

| Time point | PFS barrel type | Cumulative concentration of non-silicone oil droplet-like particles ≥ 5 μm [#/ml], n=3 | | | | | |
|---|---|---|---|---|---|---|---|
| | | 10 s (syringeability) | | Manually in ca. 15 s | | 20 s (syringeability) | |
| | | Mean | SD | Mean | SD | Mean | SD |
| T0 | Barrel1 | 577 | 217 | n.a. | n.a. | 665 | 146 |
| | Barrel2 | 1,556 | 646 | n.a. | n.a. | 1,436 | 396 |
| | Barrel3 | 1,176 | 73 | n.a. | n.a. | 1,034 | 427 |
| | Barrel4 | 1,017 | 181 | n.a. | n.a. | 425 | 84 |
| T-FT | Barrel1 | n.a. | n.a. | 950 | 38 | n.a. | n.a. |
| | Barrel2 | n.a. | n.a. | 1,648 | 188 | n.a. | n.a. |
| | Barrel3 | n.a. | n.a. | 1,176 | 92 | n.a. | n.a. |
| | Barrel4 | n.a. | n.a. | 1,080 | 148 | n.a. | n.a. |
| T2w_40C | Barrel1 | 1,428 | 257 | n.a. | n.a. | 1,238 | 350 |
| | Barrel2 | 1,271 | 86 | n.a. | n.a. | 904 | 89 |
| | Barrel3 | 2,275 | 238 | n.a. | n.a. | 1,541 | 219 |
| | Barrel4 | 1,247 | 124 | n.a. | n.a. | 1,084 | 250 |

Table 42: Cumulative concentration of silicone oil droplet-like particles ≥5 µm determined by MFI (and LINK software); values are reported as the mean of n = 3 measurements after expelling the PFS content; values are reported not corrected for the dilution factor (ca. 3-fold); n.a. = not analyzed.

| Time point | PFS barrel type | Cumulative concentration of silicone oil droplet-like particles ≥5 µm [#/ml], n=3 | | | | | |
|---|---|---|---|---|---|---|---|
| | | 10 s (syringeability) | | Manually in ca. 15 s | | 20 s (syringeability) | |
| | | Mean | SD | Mean | SD | Mean | SD |
| T0 | Barrel1 | 567 | 289 | n.a. | n.a. | 785 | 119 |
| | Barrel2 | 1,222 | 367 | n.a. | n.a. | 1,858 | 310 |
| | Barrel3(i) | 829 | 157 | n.a. | n.a. | 278 | 37 |
| | Barrel4 | 491 | 132 | n.a. | n.a. | 244 | 55 |
| T-FT | Barrel1 | n.a. | n.a. | 1,206 | 32 | n.a. | n.a. |
| | Barrel2 | n.a. | n.a. | 1,551 | 21 | n.a. | n.a. |
| | Barrel3(i) | n.a. | n.a. | 1,009 | 78 | n.a. | n.a. |
| | Barrel4 | n.a. | n.a. | 876 | 75 | n.a. | n.a. |
| T2w_40C | Barrel1 | 1,448 | 260 | n.a. | n.a. | 1,472 | 209 |
| | Barrel2 | 968 | 124 | n.a. | n.a. | 574 | 83 |
| | Barrel3(i) | 1,220 | 53 | n.a. | n.a. | 1,287 | 193 |
| | Barrel4 | 746 | 173 | n.a. | n.a. | 574 | 45 |

Table 37: Cumulative concentration of non-silicone oil droplet-like particles ≥10 µm determined by MFI (and LINK software); values are reported as the mean of n = 3 measurements after expelling the PFS content; values are reported not corrected for the dilution factor (ca. 3-fold); n.a. = not analyzed.

| Time point | PFS barrel type | Cumulative concentration of non-silicone oil droplet-like particles ≥10 µm [#/ml], n=3 | | | | | |
|---|---|---|---|---|---|---|---|
| | | 10 s (syringeability) | | Manually in ca. 15 s | | 20 s (syringeability) | |
| | | Mean | SD | Mean | SD | Mean | SD |
| T0 | Barrel1 | 107 | 29 | n.a. | n.a. | 140 | 32 |
| | Barrel2 | 255 | 121 | n.a. | n.a. | 132 | 30 |
| | Barrel3(i) | 223 | 185 | n.a. | n.a. | 412 | 140 |
| | Barrel4 | 228 | 38 | n.a. | n.a. | 137 | 37 |
| T-FT | Barrel1 | n.a. | n.a. | 144 | 30 | n.a. | n.a. |
| | Barrel2 | n.a. | n.a. | 239 | 63 | n.a. | n.a. |
| | Barrel3(i) | n.a. | n.a. | 139 | 60 | n.a. | n.a. |
| | Barrel4 | n.a. | n.a. | 130 | 70 | n.a. | n.a. |
| T2w_40C | Barrel1 | 102 | 54 | n.a. | n.a. | 153 | 108 |
| | Barrel2 | 123 | 58 | n.a. | n.a. | 183 | 113 |
| | Barrel3(i) | 128 | 19 | n.a. | n.a. | 78 | 5 |
| | Barrel4 | 125 | 41 | n.a. | n.a. | 147 | 63 |

Table 38: Cumulative concentration of silicone oil droplet-like particles ≥10 μm determined by MFI (and LINK software); values are reported as the mean of n = 3 measurements after expelling the PFS content; values are reported not corrected for the dilution factor (ca. 3-fold); n.a. = not analyzed.

| Time point | PFS barrel type | Cumulative concentration of silicone oil droplet-like particles ≥10 μm [#/ml], n=3 | | | | | |
|---|---|---|---|---|---|---|---|
| | | 10 s (syringeability) | | Manually in ca. 15 s | | 20 s (syringeability) | |
| | | Mean | SD | Mean | SD | Mean | SD |
| T0 | Barrel1 | 31 | 20 | n.a. | n.a. | 230 | 27 |
| | Barrel2 | 191 | 57 | n.a. | n.a. | 329 | 31 |
| | Barrel3(i) | 121 | 58 | n.a. | n.a. | 36 | 8 |
| | Barrel4 | 68 | 32 | n.a. | n.a. | 38 | 8 |
| T-FT | Barrel1 | n.a. | n.a. | 91 | 2 | n.a. | n.a. |
| | Barrel2 | n.a. | n.a. | 190 | 29 | n.a. | n.a. |
| | Barrel3(i) | n.a. | n.a. | 122 | 51 | n.a. | n.a. |
| | Barrel4 | n.a. | n.a. | 74 | 27 | n.a. | n.a. |
| T2w_40C | Barrel1 | 147 | 48 | n.a. | n.a. | 178 | 37 |
| | Barrel2 | 120 | 25 | n.a. | n.a. | 135 | 39 |
| | Barrel3(i) | 109 | 39 | n.a. | n.a. | 145 | 53 |
| | Barrel4 | 79 | 36 | n.a. | n.a. | 72 | 23 |

Table 39: Cumulative concentration of non-silicone oil droplet-like particles ≥25 μm determined by MFI (and LINK software); values are reported as the mean of n = 3 measurements after expelling the PFS content; values are reported not corrected for the dilution factor (ca. 3-fold); n.a. = not analyzed.

| Time point | PFS barrel type | Cumulative concentration of non-silicone oil droplet-like particles ≥25 μm [#/ml], n=3 | | | | | |
|---|---|---|---|---|---|---|---|
| | | 10 s (syringeability) | | Manually in ca. 15 s | | 20 s (syringeability) | |
| | | Mean | SD | Mean | SD | Mean | SD |
| T0 | Barrel1 | 28 | 8 | n.a. | n.a. | 48 | 30 |
| | Barrel2 | 84 | 52 | n.a. | n.a. | 29 | 2 |
| | Barrel3(i) | 73 | 72 | n.a. | n.a. | 150 | 12 |
| | Barrel4 | 81 | 33 | n.a. | n.a. | 50 | 21 |
| T-FT | Barrel1 | n.a. | n.a. | 36 | 11 | n.a. | n.a. |
| | Barrel2 | n.a. | n.a. | 78 | 14 | n.a. | n.a. |
| | Barrel3(i) | n.a. | n.a. | 36 | 18 | n.a. | n.a. |
| | Barrel4 | n.a. | n.a. | 30 | 26 | n.a. | n.a. |
| T2w_40C | Barrel1 | 20 | 20 | n.a. | n.a. | 50 | 46 |
| | Barrel2 | 49 | 23 | n.a. | n.a. | 73 | 50 |
| | Barrel3(i) | 34 | 18 | n.a. | n.a. | 20 | 3 |
| | Barrel4 | 39 | 8 | n.a. | n.a. | 37 | 12 |

Table 40: Cumulative concentration of silicone oil droplet-like particles ≥25 μm determined by MFI (and LINK software); values are reported as the mean of n = 3 measurements after expelling the PFS content; values are reported not corrected for the dilution factor (ca. 3-fold); n.a. = not analyzed.

| Time point | PFS barrel type | Cumulative concentration of silicone oil droplet-like particles ≥25 μm [#/ml], n=3 | | | | | |
|---|---|---|---|---|---|---|---|
| | | 10 s (syringeability) | | Manually in ca. 15 s | | 20 s (syringeability) | |
| | | Mean | SD | Mean | SD | Mean | SD |
| T0 | Barrel1 | 0 | 0 | n.a. | n.a. | 7 | 7 |
| | Barrel2 | 7 | 0 | n.a. | n.a. | 11 | 4 |
| | Barrel3(i) | 8 | 8 | n.a. | n.a. | 2 | 2 |
| | Barrel4 | 4 | 8 | n.a. | n.a. | 6 | 7 |
| T-FT | Barrel1 | n.a. | n.a. | 3 | 3 | n.a. | n.a. |
| | Barrel2 | n.a. | n.a. | 14 | 8 | n.a. | n.a. |
| | Barrel3(i) | n.a. | n.a. | 12 | 8 | n.a. | n.a. |
| | Barrel4 | n.a. | n.a. | 4 | 4 | n.a. | n.a. |
| T2w_40C | Barrel1 | 8 | 8 | n.a. | n.a. | 6 | 7 |
| | Barrel2 | 14 | 12 | n.a. | n.a. | 9 | 2 |
| | Barrel3(i) | 10 | 3 | n.a. | n.a. | 8 | 2 |
| | Barrel4 | 4 | 4 | n.a. | n.a. | 3 | 6 |

*Summary and conclusions*

Four different PFS configurations were tested with one exemplary Ab1 formulation. The main finding was that by testing different siliconization levels, no particular sensitivity of Ab1 towards silicone oil could be identified. Therefore, it can be concluded that all standard PFS solutions would be feasible as primary packaging material configurations for Ab1 drug product formulated at 150 ± 15 mg/ml protein.

The results obtained with respect to differences observed between the different siliconization levels can be summarized as follows:

Differences between PFS configurations:

**Syringeability**

- Higher forces required for Barrel4 compared to other PFS barrel types
- Results for gliding forces very similar for Barrel1, Barrel2 and Barrel3(i)
- No impact of 2 weeks of storage at 40 °C on any force profile

Higher siliconization levels of Barrel2 and Barrel3 (i) (0.7 mg/PFS) may explain lower gliding forces compared to 0.55 mg per PFS (Barrel4). The variability of siliconization between different PFS lots as well as within one PFS lot must be considered high, such as for example 0.5 mg ± 0.2 mg per PFS for Barrel1 PFS. The syringeability of Barrel1 PFS, however, was in the same range as the syringeability of PFS barrels with 0.7 mg/PFS.

**Subvisible particle levels**

- Barrel2 PFS showed highest levels of silicone oil droplet-like particles compared to other PFS barrel types
- No major differences observed in non-silicone oil droplet-like particle fractions

**Impact of different PFS configurations on Ab1 stability upon stressing:**

- All samples showed no visible and low subvisible particle levels before stressing
- No impact on subvisible particle levels upon FT stressing observed
- Small impact of heat stress observed: few visible particles in Barrel3 (i) and Barrel4 and slightly increased non-silicone oil droplet-like particle levels in all PFS barrel types (most pronounced for Barrel3 (i))
- No impact of the ejection speed (10 versus 20 s/2 ml) on subvisible particle levels determined

This study suggests PFS barrels from Barrel1 (nominal silicone level 0.5 $\pm$ 0.2 mg per PFS) as well as from BD and Barrel2 (nominal silicone level 0.7 $\pm$ 0.2 or $\pm$ 0.3 mg per PFS) to give gliding force profiles in the target range, i.e., 5-6 N.
Barrel1 and Barrel3(i) were selected and further tests.

Part 3 Molecular stability analysis

*Material and methods*

The PFS post exposure to freeze-thaw and heat stresses, were analysed for various Ab1 quality parameters such as protein content (by A280), purity by (SE-HPLC, cGE-reduced, and cGE-non-reduced, iCE3) and potency (Binding ELISA).

*Results and conclusions*

The results of protein content analysis, shown in Table 52, indicate that there was no significant change in terms of protein content post stresses which shall be attributed to the analytical method variations.

Table 41. Protein content results

| | **Protein content (mg/mL)** | | |
|---|---|---|---|
| | **T0** | **Freeze-Thaw (FT) stress** | **Heat stress (HS)** |
| **Barrel1** | 145.7 | 144.8 | 141.3 |
| **Barrel3(i)** | 144.4 | 142.6 | 142.8 |
| **Barrel2** | 143.7 | 141.6 | 142.1 |
| **Barel4** | 143.3 | 141.8 | 142.7 |

Binding ELISA, Table 53, shows that both stresses, did not alter the target binding potency of the molecule irrespective of type of PFS barrels used.

Table 42. Binding ELISA results

| | **Binding ELISA** | | |
|---|---|---|---|
| | **T0** | **Freeze-Thaw (FT) stress** | **Heat stress (HS)** |
| **Barrel1** | 96 | 116 | 73 |
| **Barrel3(i)** | 97 | 84 | 109 |
| **Barrel2** | 90 | 89 | 113 |
| **Barel4** | 98 | 89 | 121 |

In Table 54 the results of iCE3 analysis are shown indicating no significant change in % of charged variants of Ab1 were noticed post Freeze-Thaw (FT) stress but there was a non-specific increase in the acidic variants across all type of barrels post Heat stress (HS) with Barrel3(i) and Barrel2 barrels showing slightly higher %Acidic variants than Barrel1/Barrel4.

Table 43: iCE3 results

| | iCE3 | | |
|---|---|---|---|
| **PFS type** | **%Acidic variants** | | |
| | **T0** | **Freeze-Thaw (FT) stress** | **Heat stress (HS)** |
| **Barrel1** | 30.5 | 30.8 | 36.3 |
| **Barrel3(i)** | 29.6 | 30.3 | 37.4 |
| **Barrel2** | 30.8 | 30.3 | 38.5 |
| **Barel4** | 30.6 | 31.0 | 36.5 |
| **PFS type** | **%Main peak** | | |
| | **T0** | **Freeze-Thaw (FT) stress** | **Heat stress (HS)** |
| **Barrel1** | 45.6 | 45.3 | 41.6 |
| **Barrel3(i)** | 46.0 | 45.3 | 40.4 |
| **Barrel2** | 45.2 | 45.8 | 39.6 |
| **Barel4** | 45.4 | 45.4 | 41.2 |
| **PFS type** | **%Basic variants** | | |
| | **T0** | **Freeze-Thaw (FT) stress** | **Heat stress (HS)** |
| **Barrel1** | 23.9 | 23.8 | 22.1 |
| **Barrel3(i)** | 24.4 | 24.4 | 22.2 |
| **Barrel2** | 24.0 | 23.9 | 22.0 |
| **Barel4** | 24.0 | 23.6 | 22.3 |

Non-reduced cGE analysis (Table 55) shows that Ab1 is stable (no change in content of different species) post Freeze-Thaw (FT) stress but there was a decrease in %IgG peak across all type of barrels post Heat stress (HS) suggesting a non-barrel-specific degradation of molecule under heat condition.

Table 44: Non-reduced cGE results.

| | cGE (NR) | | |
|---|---|---|---|
| **PFS type** | **%IgG (≥ 90%)** | | |
| | **T0** | **Freeze-Thaw (FT) stress** | **Heat stress (HS)** |
| **Barrel1** | 93.3 | 93.1 | 83.4 |
| **Barrel3(i)** | 93.3 | 93.1 | 83.4 |
| **Barrel2** | 93.2 | 93.1 | 84.5 |
| **Barel4** | 93.3 | 92.8 | 83.7 |
| **PFS type** | **%Assigned peaks (≤ 8%)** | | |
| | **T0** | **Freeze-Thaw (FT) stress** | **Heat stress (HS)** |
| **Barrel1** | 5.1 | 5.3 | 7.4 |
| **Barrel3(i)** | 5.2 | 5.3 | 6.6 |
| **Barrel2** | 5.3 | 5.3 | 6.2 |
| **Barel4** | 5.1 | 5.5 | 6.9 |

(continued)

| PFS type | %Total other peaks (≤ 3%) | | |
|---|---|---|---|
| | T0 | Freeze-Thaw (FT) stress | Heat stress (HS) |
| Barrel1 | 1.6 | 1.5 | 9.4 |
| Barrel3(i) | 1.6 | 1.6 | 10.0 |
| Barrel2 | 1.6 | 1.6 | 9.2 |
| Barel4 | 1.6 | 1.7 | 9.5 |
| Assigned peaks = Frag. 75kDa + Frag. 100 kDa + Frag. 125kDa. Other peaks = LC + HC + IgG'. | | | |

Reduced cGE analysis (Table 56) shows that there was no observable degradation trend post stress conditions irrespective of type of barrels used.

Table 45: Reduced cGE results

| | cGE (R) | | |
|---|---|---|---|
| PFS type | %HC (≥ 60%) | | |
| | T0 | Freeze-Thaw (FT) stress | Heat stress (HS) |
| Barrel1 | 64.0 | 64.0 | 64.0 |
| Barrel3(i) | 64.0 | 64.0 | 63.0 |
| Barrel2 | 64.0 | 64.0 | 64.0 |
| Barel4 | 64.0 | 63.0 | 63.0 |
| PFS type | %LC (≥ 30%) | | |
| | T0 | Freeze-Thaw (FT) stress | Heat stress (HS) |
| Barrel1 | 35.0 | 35.0 | 34.0 |
| Barrel3(i) | 35.0 | 35.0 | 35.0 |
| Barrel2 | 35.0 | 35.0 | 35.0 |
| Barel4 | 35.0 | 36.0 | 35.0 |
| PFS type | %Total other peaks (≤ 3%) | | |
| | T0 | Freeze-Thaw (FT) stress | Heat stress (HS) |
| Barrel1 | 1.0 | 2.0 | 1.0 |
| Barrel3(i) | 1.0 | 1.0 | 2.0 |
| Barrel2 | 2.0 | 1.0 | 2.0 |
| Barel4 | 1.0 | 2.0 | 2.0 |

In Table 57 the results of SE-HPLC analysis are reported, indicating that Ab1 remained stable post Freeze Thaw stress whereas heat stress cased loss of %monomer and %aggregates while increased %fragments irrespective of barrel types used.

Table 46: SE-HPLC results

| | SE-HPLC | | |
|---|---|---|---|
| PFS type | %Monomer (≥ 95%) | | |
| | T0 | Freeze-Thaw (FT) stress | Heat stress (HS) |
| Barrel1 | 96.9 | 96.4 | 95.6 |
| Barrel3(i) | 97.1 | 97.3 | 95.6 |

(continued)

| | SE-HPLC | | |
|---|---|---|---|
| PFS type | %Monomer (≥ 95%) | | |
| | T0 | Freeze-Thaw (FT) stress | Heat stress (HS) |
| Barrel2 | 96.2 | 96.6 | 95.9 |
| Barel4 | 96.9 | 96.6 | 95.6 |
| PFS type | %Aggregates | | |
| | T0 | Freeze-Thaw (FT) stress | Heat stress (HS) |
| Barrel1 | 3.15 | 3.58 | 2.74 |
| Barrel3(i) | 2.95 | 2.66 | 2.70 |
| Barrel2 | 3.83 | 3.42 | 2.45 |
| Barel4 | 3.06 | 3.36 | 2.76 |
| PFS type | %Fragments | | |
| | T0 | Freeze-Thaw (FT) stress | Heat stress (HS) |
| Barrel1 | 0.0 | 0.0 | 1.64 |
| Barrel3(i) | 0.0 | 0.0 | 1.73 |
| Barrel2 | 0.0 | 0.0 | 1.61 |
| Barel4 | 0.0 | 0.0 | 1.61 |

In conclusion all different types of barrels used (with variable silicon oil lubrication content) showed no specific Ab1 degradations. The molecule was stable under freeze-thaw stress whereas under heat stress, it showed increased fragmentations and acidic variants levels which was largely similar across all barrel types. This confirmed that Ab1 have no particular sensitivity with silicon oil with the range of 0.3 to 1.0 mg/barrel tested.

**Example 5: Stability studies**

Materials and methods

Stress and stability tests were performed for 13 formulations (Table 58) containing 150 mg/ml of Ab1, in different PFS (Table 59).

Table 47. Formulations. (PS: Polysorbate)

| Formulation no. | Buffer | pH | Salt | Amino acid | Polyol | Antioxidant | Surfactant |
|---|---|---|---|---|---|---|---|
| **F1** | 25 mM histidine-HCl | 6.0 | 150 mM NaCl | - | - | - | 0.036% PS80 |
| **F2** | 25 mM histidine-HCl | 6.0 | - | 150 mM arginine-HCl | - | - | 0.036% PS80 |
| **F3** | 25 mM histidine-HCl | 5.5 | - | 150 mM arginine-HCl | - | - | 0.036% PS80 |
| **F4** | 25 mM histidine-HCl | 6.5 | - | 150 mM arginine-HCl | - | - | 0.036% PS80 |

(continued)

| Formulation no. | Buffer | pH | Salt | Amino acid | Polyol | Antioxidant | Surfactant |
|---|---|---|---|---|---|---|---|
| **F5** | 25 mM citrate | 6.0 | 150 mM NaCl | - | - | - | 0.036% PS80 |
| **F6** | 25 mM citrate | 6.0 | - | 150 mM arginine-HCl | - | - | 0.036% PS80 |
| **F7** | 25 mM histidine-HCl | 6.0 | - | 150 mM proline | 2.5% mannitol | - | 0.036% PS80 |
| **F8** | 25 mM histidine-HCl | 6.0 | - | 150 mM lysine-HCl | 1% mannitol | - | 0.036% PS80 |
| **F9** | 25 mM histidine-HCl | 6.0 | - | 50 mM arginine-HCl | 3% mannitol | - | 0.036% PS80 |
| **F10** | 25 mM histidine-HCl | 6.0 | - | 150 mM arginine-HCl | | 10 mM methionine | 0.036% PS80 |
| **F11** | 25 mM histidine-HCl | 6.0 | - | - | 4.2% mannitol | - | 0.036% PS80 |
| **F12** | 25 mM histidine-HCl | 6.0 | - | - | 8.5% sucrose | - | 0.036% PS80 |
| **F13** | 25 mM histidine-HCl | 6.0 | - | 150 mM arginine-HCl | - | - | 0.054% PS80 |

Table 48: Tested PFS

| Part | Description | Siliconization |
|---|---|---|
| Barrel1 | 2.25 glass barrel with 27G ½" staked needle | 0.5 mg/PFS |
| Barrel3(ii) | 2.25 glass barrel with 27G ½" staked needle | 0.5 mg/PFS |
| Stopper1 | 2.25 glass barrel with 27G ½" staked needle | none |
| **Tested PFS** | | |
| PFS1 | Barrel1 + Stopper1 | Tested with F1- F13 |
| PFS3(ii) | Barrel3(ii) + Stopper1 | Tested with F2, F6, F9, F10 |

*Sample preparation*

Ab1 BDS material was provided at a nominal protein concentration of about 69.47 mg/ml in 5 mM histidine, pH 6.45. The sample was concentrated to about 190 mg/ml by using TFF. Full formulation was achieved either by spiking (two-step addition of concentrated excipient stock) or by buffer exchange (discontinuous diafiltration via Amicon and dialysis). A third spiking step was performed by spiking of respective volumes of 10% (w/v) PS80 stock solutions to both protein samples and formulation buffers/placebo, followed by the filtration of the samples through 0.22-$\mu$m PES filters under laminar air-flow conditions and by the filling of product/placebo formulation buffers into 2.25-ml PFS and stoppering under vacuum.

*Stress /storage conditions*

Stress conditions:

- Freeze-thaw stress (TFT) was performed manually and in horizontal position, 5 cycles from +20°C to -20 °C in freezer, holding times >2 h or until completely frozen or thawed
- Rolling stress (T-roll) was performed in a vertical test tube rotatorat 40 rpm for 2 days at room temperature (> 100,000 bubble rolls in total per barrel)

Storage conditions: Samples were stored in horizontal position for 1 month, 3 and 6 months of storage at 5 °C, and 25 °C/60% r.h. and for up to 3 months at 40 °C/75% r.h.

*Analytical methods*

Samples were analyzed before (T0) and after stress and storage, by using the analytical methods specified in Table 60.

| Sample | Visual inspection | UV (A280) | MFI | SEC | Bio-analyzer | iCE3 | Nano DSF | Viscosity | pH | Osmolality | Syringeability | Binding ELISA |
|---|---|---|---|---|---|---|---|---|---|---|---|---|
| T0 | X | X | X | X | X | X | X | X | X | X | X | X |
| TFT | X | X | X | X | X | X | - | - | X | - | - | X |
| T-roll | X | X | X | X | X | X | - | - | X | - | - | X |
| T1m 5°C, 25°C, 40°C) | X | X | X | X | X | X | X | - | X | - | - | X |
| T3m 5°C, 25°C, 40°C) | X | X | X | X | X | X | X | X | X | X | X | X |
| T6m (5°C, 25°C) | X | X | X | X | X | X | X | X | X | - | - | X |

Table 49: Overview of the analytical methods applied

T0 characterization

*pH, osmolality and viscosity*

First an initial characterization (T0) has been done, measuring pH, osmolality and viscosity of the above described samples. The results of the characterization are shown in Table 61.

| | | Formulation | pH | | Osmolality | Viscosity |
|---|---|---|---|---|---|---|
| | | | Target | T0 | T0 [mOsmol/kg] | T0 [mPa*s] |
| Test group A: Barrel1 | 1 | 25 mM His-HCl, 150 mM NaCl, 0.036% PS80 | 6.0 ± 0.1 | 6.14 | 380 | 11.4 |
| | 2 | 25 mM His-HCl, 150 mM Arg-HCl, 0.036% PS80 | 6.0 ± 0.1 | 6.01 | 356 | 6.7 |
| | 3 | 25 mM His-HCl, 150 mM Arg-HCl, 0.036% PS80 | 5.5 ± 0.1 | 5.48 | 360 | 6.8 |
| | 4 | 25 mM His-HCl, 150 mM Arg-HCl, 0.036% PS80 | 6.5 ± 0.1 | 6.49 | 339 | 7.0 |
| | 5 | 25 mM Citrate, 150 mM NaCl, 0.036% PS80 | 6.0 ± 0.1 | 6.09 | 384 | 11.8 |
| | 6 | 25 mM Citrate, 150 mM Arg-citrate/HCl, 0.036% PS80 | 6.0 ± 0.1 | 6.01 | 232 | 7.8 |
| | 7 | 25 mM His-HCl, 150 mM proline, 2.5% mannitol, 0.036% PS80 | 6.0 ± 0.1 | 6.04 | 428 | 10.4 |
| | 8 | 25 mM His-HCl, 150 mM Lys-HCl, 1.0% mannitol, 0.036% PS80 | 6.0 ± 0.1 | 6.05 | 456 | 8.3 |
| | 9 | 25 mM His-HCl, 50 mM Arg-HCl, 3.0% mannitol, 0.036% PS80 | 6.0 ± 0.1 | 6.04 | 369 | 9.3 |
| | 10 | 25 mM His-HCl, 150 mM Arg-Cl, 10 mM methionine, 0.036% PS80 | 6.0 ± 0.1 | 6.03 | 357 | 7.1 |
| | 11 | 25 mM His-HCl, 4.2% mannitol, 0.036% PS80 | 6.0 ± 0.1 | 6.09 | 353 | 16.1 |
| | 12 | 25 mM His-HCl, 8.5% sucrose, 0.036% PS80 | 6.0 ± 0.1 | 6.09 | 416 | 18.2 |
| | 13 | 25 mM His-HCl, 150 mM Arg-Cl, 0.054% PS80 | 6.0 ± 0.1 | 6.02 | 343 | 6.4 |
| Test group B: Barrel3(ii) | 2 | 25 mM His-HCl, 150 mM Arg-HCl, 0.036% PS80 | 6.0 ± 0.1 | 6.03 | 354 | 6.4 |
| | 6 | 25 mM Citrate, 150 mM Arg-citrate/HCl, 0.036% PS80 | 6.0 ± 0.1 | 6.00 | 232 | 7.9 |
| | 9 | 25 mM His-HCl, 50 mM Arg-HCl, 3.0% mannitol, 0.036% PS80 | 6.0 ± 0.1 | 6.03 | 369 | 9.4 |
| | 10 | 25 mM His-HCl, 150 mM Arg-Cl, 10 mM methionine, 0.036% PS80 | 6.0 ± 0.1 | 6.02 | 357 | 7.2 |

Table 50: T0 characterization

*Thermal stability*

Next thermal stability of samples was evaluated using NanoDSF that utilizes measurement of intrinsic fluorescence of protein upon heating from 20 to 95 °C to determine $T_m$ values (aggregation onset and unfolding events).

Apparent protein melting temperatures (Tm) obtained from the ratio of the fluorescence emission at 350 nm and 330 nm (350/330 nm ratio) as a function of temperature. The Tm values and the onset temperature of unfolding (Tm,onset) are calculated by software. In addition, the intensity of the back reflection was monitored to get information about temperature onset of aggregation (Tagg,onset) by the software. There we two melting events were observed (Tm1 & Tm2) and their results are summarized in Table 62 and in Figure 25.

| Thermal event | **Formulation** *evaluating fluorescence ratio 350/330 nm | | | | | | | | | | | | | | | | |
|---|---|---|---|---|---|---|---|---|---|---|---|---|---|---|---|---|---|
| | **F1-Barrel1** | **F2-Barrel1** | **F3-Barrel1** | **F4-Barrel1** | **F5-Barrel1** | **F6-Barrel1** | **F7-Barrel1** | **F8-Barrel1** | **F9-Barrel1** | **F10-Barrel1** | **F11-Barrel1** | **F12-Barrel1** | **F13-Barrel1** | **F2-Barrel3(ii)** | **F6- Barrel3(ii)** | **F9- Barrel3(ii)** | **F10- Barrel3(ii)** |
| $T_m$, onset | 64.1 | 65.5 | 63.9 | 66.0 | 64.0 | 66.0 | 62.0<br>61.8 | 65.7 | 62.0 | 65.6 | 64.1 | 64.8 | 65.0 | 65.0 | 65.8 | 67.1 | 65.5 |
| $T_{m1}$ [°C]* | 67.9 | 69.1 | 67.8 | 70.1 | 68.6 | 70.0 | 66.4<br>66.1 | 69.7 | 70.7 | 69.1 | 67.6 | 68.2 | 69.1 | 69.1 | 70.0 | 70.8 | 69.2 |
| $T_{m2}$ [°C]* | 82.9 | 82.4 | 81.6 | 82.7 | 83.3 | 82.9 | 85.1 | 83.3 | 83.5 | 82.6 | 84.7 | 85.5 | 82.6 | 82.6 | 82.9 | 83.5 | 82.6 |
| $T_{agg}$, onset* | 60.0 | 61.0 | 58.2 | 62.0 | 60.7 | 61.8 | 63.3<br>63.2 | 61.6 | 62.4 | 60.6 | 64.1 | 65.1 | 61.6 | 61.8 | 62.0 | 62.8 | 60.9 |
| Thermal event | **Formulation (**evaluating fluorescence ratio 350nm) | | | | | | | | | | | | | | | | |
| | **F1-Barrel1** | **F2-Barrel1** | **F3-Barrel1** | **F4-Barrel1** | **F5-Barrel1** | **F6-Barrel1** | **F7-Barrel1** | **F8-Barrel1** | **F9-Barrel1** | **F10-Barrel1** | **F11-Barrel1** | **F12-Barrel1** | **F13-Barrel1** | **F2-Barrel3(ii)** | **F6- Barrel3(ii)** | **F9- Barrel3(ii)** | **F10- Barrel3(ii)** |
| $T_m$, onset [°C] | 62.7 | 62.4 | 60.6 | 64.0 | 64.1 | 64.2 | 64.6<br>65.0 | 63.6 | 64.0 | 62.6 | 65.0 | 65.3 | 62.7 | 62.9 | 64.4 | 64.3 | 63.0 |
| $T_{m1}$ [°C] | 69.1 | 69.8 | 68.5 | 70.7 | 70.1 | 70.8 | 71.2<br>71.2 | 70.5 | 71.2 | 69.8 | 71.6 | 72.1 | 69.8 | 69.8 | 70.8 | 71.3 | 69.9 |
| $T_{m2}$ [°C] | 82.9 | 82.2 | 81.5 | 82.6 | 83.2 | 82.7 | 83.2<br>83.0 | 83.3 | 83.1 | 82.1 | 82.7 | 83.6 | 82.2 | 82.1 | 82.4 | 82.3 | 82.1 |

Table 51: nanoDSF results

All thermal events were comparable and irrespective of the type of PFS (Barrel1 or Barrel3(ii)). The results reported above indicate that the thermal stability is not impacted by methionine (F10) or higher PS80 content.Thermal/conformational stability is slightly reduced at reduced pH 5.5 (F3) compared to pH 6.5 (F4). A potentially positive impact of compositions with proline (F7) and His/Man or Suc combinations F11 and F12 is detected. The determination of $T_{m,onset}$ was difficult for formulations F7, F11 and F12 due to a different melting profile to the other formulations (see Figure 27 for exemplarily melting curves). Thermal events were determined manually under evaluation of 350 nm/330 nm (+ 1st derivative) as well as 350 nm (+ 1st derivative).

Results - Stress analyses

*pH*

At T0, the pH of all samples was within the acceptance limits of target pH ± 0.1 pH units. The pH was unchanged after freeze-thaw and rolling stress, as shown in Table 63.

| Time point | pH | | | | | | | | | | | | | | | | |
|---|---|---|---|---|---|---|---|---|---|---|---|---|---|---|---|---|---|
| | F1-Barrel1 | F2-Barrel1 | F3-Barrel1 | F4-Barrel1 | F5-Barrel1 | F6-Barrel1 | F7-Barrel1 | F8-Barrel1 | F9-Barrel1 | F10-Barrel1 | F11-Barrel1 | F12-Barrel1 | F13-Barrel1 | F2-Barrel3(ii) | F6- Barrel3(ii) | F9- Barrel3(ii) | F10- Barrel3(ii) |
| Target | 6.0 | 6.0 | 5.5 | 6.5 | 6.0 | 6.0 | 6.0 | 6.0 | 6.0 | 6.0 | 6.0 | 6.0 | 6.0 | 6.0 | 6.0 | 6.0 | 6.0 |
| T0 | 6.14 | 6.01 | 5.48 | 6.49 | 6.09 | 6.01 | 6.04 | 6.05 | 6.04 | 6.03 | 6.09 | 6.09 | 6.02 | 6.03 | 6.00 | 6.03 | 6.02 |
| TFT | 6.11 | 6.04 | 5.47 | 6.47 | 6.07 | 6.03 | 6.02 | 6.02 | 6.01 | 6.02 | 6.04 | 6.06 | 6.02 | 6.01 | 6.01 | 6.02 | 6.01 |
| T-roll | 6.11 | 6.04 | 5.47 | 6.47 | 6.04 | 6.01 | 6.00 | 6.03 | 6.02 | 6.01 | 6.09 | 6.06 | 6.00 | 6.00 | 6.02 | 6.03 | 6.00 |

Table 52: pH analysis results after freeze-thaw and rolling stress

*Protein content*

At T0, the protein content of all samples was within the acceptance limits of 150 ± 15 mg/ml. The protein content was unchanged after freeze-thaw and rolling stress (still within target range) as shown in Table 64.

| Time point | Protein content A280-A320 (measured at ca. 1 mg/ml) | | | | | | | | | | | | | | | | |
|---|---|---|---|---|---|---|---|---|---|---|---|---|---|---|---|---|---|
| | F1-Barrel1 | F2-Barrel1 | F3-Barrel1 | F4-Barrel1 | F5-Barrel1 | F6-Barrel1 | F7-Barrel1 | F8-Barrel1 | F9-Barrel1 | F10-Barrel1 | F11-Barrel1 | F12-Barrel1 | F13-Barrel1 | F2-Barrel3(ii) | F6- Barrel3(ii) | F9- Barrel3(ii) | F10- Barrel3(ii) |
| T0 | 154 | 144 | 147 | 150 | 151 | 154 | 150 | 158 | 161 | 153 | 153 | 152 | 157 | 145 | 148 | 147 | 150 |
| TFT | 153 | 156 | 152 | 160 | 150 | 157 | 160 | 160 | 155 | 160 | 158 | 165 | 142 | 157 | 156 | 156 | 158 |
| T-roll | 156 | 158 | 162 | 159 | 151 | 161 | 160 | 161 | 160 | 153 | 155 | 164 | 154 | 159 | 161 | 158 | 145 |

Table 53: Protein content analysis results after freeze-thaw and rolling stress

*Turbidity*

No difference in the OD350 and OD550 before and after freeze-thaw and rolling stress were detected as shown in Figure 28 and in Figure 29, respectively.

*Visual inspection*

At T0, all samples were practically free of visible particles and exhibited a yellowish coloration. All formulations remained unchanged after freeze-thaw and rolling stress as shown in Table 65.

| Time point | Visual inspection DAC score examiner 1 / examiner 2 | | | | | | | | | | | | | | | | |
|---|---|---|---|---|---|---|---|---|---|---|---|---|---|---|---|---|---|
| | F1-Barrel1 | F2-Barrel1 | F3-Barrel1 | F4-Barrel1 | F5-Barrel1 | F6-Barrel1 | F7-Barrel1 | F8-Barrel1 | F9-Barrel1 | F10-Barrel1 | F11-Barrel1 | F12-Barrel1 | F13-Barrel1 | F2-Barrel3(ii) | F6- Barrel3(ii) | F9- Barrel3(ii) | F10- Barrel3(ii) |
| T0 | 0/0 | 0/0 | 0/0 | 0/1 | 0/0 | 0/0 | 0/0 | 0/0 | 0/0 | 0/0 | 0/0 | 0/0 | 0/0 | 0/0 | 0/0 | 0/0 | 0/0 |
| TFT | 0/0 | 0/0 | 0/0 | 0/0 | 0/0 | 0/0 | 0/0 | 0/0 | 0/0 | 0/0 | 0/0 | 0/0 | 0/0 | 0/0 | 1/1 | 0/0 | 0/0 |
| T-roll | 0/0 | 0/0 | 0/0 | 0/0 | 1/1 | 0/0 | 0/0 | 0/0 | 0/0 | 0/1 | 0/0 | 0/0 | 0/0 | 0/0 | 0/0 | 0/0 | 1/1 |

Table 54: Visual inspection results after freeze-thaw and rolling stress

*MFI*

All formulations were measured diluted in their respective formulation buffer (at ca. 50 mg/ml). The particle content of the dilution media (Buffer) was very low and comparable for all formulation buffers. The particle levels of protein-containing samples was higher compared to the formulation buffers, overall highest for F5 (* exceeds y-axis, Figure 30), see Table 66 and Table 67.

Table 55: MFI results after stressing

| Formulation | T0 | | | | TFT | | | | T-roll | | | |
|---|---|---|---|---|---|---|---|---|---|---|---|---|
| | Cumulative concentration [particles/ml] | | | | Cumulative concentration [particles/ml] | | | | Cumulative concentration [particles/ml] | | | |
| | Total | Non-silicone oil particle | | | Total | Non-silicone oil particles | | | Total | Non-silicone oil particles | | |
| | ≥ 2 μm | ≥ 5 μm | 10 μm | ≥ 25 μm | ≥ 2 μm | ≥ 5 μm | ≥ 10 μm | 25 μm | ≥ 2 μm | ≥ 5 μm | 10 μm | ≥25 μm |
| F1-Barrel1 | 3,801 | 546 | 160 | 50 | 7,718 | 360 | 73 | 23 | 12,751 | 286 | 60 | 20 |
| F2-Barrel1 | 2,811 | 446 | 137 | 23 | 6,708 | 366 | 93 | 20 | 4,234 | 343 | 103 | 33 |
| F3-Barrel1 | 2,611 | 333 | 103 | 20 | 5,756 | 150 | 60 | 13 | 7,471 | 393 | 77 | 10 |
| F4-Barrel1 | 1,949 | 273 | 150 | 37 | 7,001 | 127 | 60 | 23 | 7,281 | 343 | 40 | 7 |
| F5-Barrel1 | 48,880 | 1,366 | 353 | 80 | 25,408 | 300 | 70 | 3 | 6,548 | 360 | 113 | 23 |
| F6-Barrel1 | 2,105 | 280 | 147 | 50 | 5,206 | 266 | 100 | 23 | 15,645 | 676 | 110 | 20 |
| F7-Barrel1 | 4,433 | 360 | 133 | 17 | 12,018 | 373 | 53 | 20 | 12,517 | 829 | 187 | 33 |

(continued)

| Formulation | T0 | | | | TFT | | | | T-roll | | | |
|---|---|---|---|---|---|---|---|---|---|---|---|---|
| | Cumulative concentration [particles/ml] | | | | Cumulative concentration [particles/ml] | | | | Cumulative concentration [particles/ml] | | | |
| | Total | Non-silicone oil particle | | | Total | Non-silicone oil particles | | | Total | Non-silicone oil particles | | |
| | ≥ 2 µm | ≥ 5 µm | 10 µm | ≥ 25 µm | ≥ 2 µm | ≥ 5 µm | ≥ 10 µm | 25 µm | ≥ 2 µm | ≥ 5 µm | 10 µm | ≥ 25 µm |
| F8-Barrel1 | 2,501 | 266 | 120 | 37 | 5,839 | 313 | 63 | 10 | 14,073 | 463 | 87 | 17 |
| F9-Barrel1 | 6,998 | 573 | 213 | 33 | 5,886 | 137 | 47 | 10 | 5,669 | 436 | 113 | 27 |
| F10-Barrel1 | 4,084 | 580 | 250 | 70 | 6,046 | 240 | 77 | 10 | 10,019 | 426 | 77 | 10 |
| F11-Barrel1 | 6,342 | 510 | 183 | 63 | 9,097 | 450 | 120 | 23 | 4,017 | 276 | 110 | 37 |
| F12-Barrel1 | 4,567 | 303 | 43 | 7 | 5,702 | 783 | 290 | 50 | 10,619 | 363 | 57 | 30 |
| F13-Barrel1 | 1,952 | 276 | 157 | 37 | 6,695 | 283 | 83 | 17 | 6,125 | 343 | 80 | 3 |
| F2-Barrel3(ii) | 1,615 | 530 | 286 | 80 | 6,525 | 839 | 276 | 73 | 2,298 | 217 | 80 | 20 |
| F6-Barrel3(ii) | 1,572 | 390 | 177 | 57 | 10,259 | 723 | 260 | 57 | 6,469 | 523 | 140 | 40 |
| F9-Barrel3(ii) | 3,984 | 410 | 163 | 57 | 3,071 | 526 | 187 | 37 | 1,749 | 167 | 43 | 10 |
| F10-Barrel3 (ii) | 3,311 | 263 | 127 | 40 | 4,563 | 127 | 50 | 10 | 4,627 | 183 | 47 | 7 |

Table 56: MFI results after stressing

| Formulation | T0 | | | TFT | | | T-roll | | |
|---|---|---|---|---|---|---|---|---|---|
| | Cumulative concentration [particles/ml] | | | Cumulative concentration [particles/ml] | | | Cumulative concentration [particles/ml] | | |
| | Silicone oil-like particles | | | Silicone oil-like particles | | | Silicone oil-like particles | | |
| | ≥ 5 µm | ≥ 10 µm | ≥ 25 µm | ≥ 5 µm | ≥ 10 µm | ≥ 25 µm | ≥ 5 µm | ≥ 10 µm | ≥ 25 µm |
| F1-Barrel1 | 183 | 10 | 3 | 1,212 | 80 | 0 | 1,639 | 97 | 0 |
| F2-Barrel1 | 243 | 20 | 0 | 886 | 50 | 3 | 456 | 60 | 3 |
| F3-Barrel1 | 117 | 20 | 0 | 513 | 27 | 0 | 1,526 | 30 | 0 |
| F4-Barrel1 | 230 | 13 | 0 | 453 | 20 | 3 | 2,565 | 100 | 0 |
| F5-Barrel1 | 1,446 | 20 | 7 | 3,038 | 523 | 0 | 1,549 | 27 | 3 |
| F6-Barrel1 | 233 | 3 | 0 | 873 | 83 | 0 | 4,843 | 280 | 3 |
| F7-Barrel1 | 266 | 3 | 0 | 1,582 | 13 | 3 | 2,278 | 100 | 0 |
| F8-Barrel1 | 240 | 57 | 3 | 799 | 63 | 0 | 2,325 | 207 | 0 |
| F9-Barrel1 | 706 | 27 | 0 | 726 | 23 | 0 | 233 | 30 | 0 |
| F10-Barrel1 | 380 | 17 | 7 | 550 | 40 | 0 | 3,930 | 713 | 7 |
| F11-Barrel1 | 1,522 | 127 | 0 | 656 | 40 | 0 | 729 | 53 | 0 |
| F12-Barrel1 | 1,009 | 23 | 0 | 210 | 30 | 0 | 1,046 | 17 | 0 |
| F13-Barrel1 | 110 | 13 | 0 | 1,063 | 50 | 0 | 1,679 | 60 | 0 |
| F2-Barrel3(ii) | 43 | 3 | 3 | 343 | 17 | 0 | 110 | 13 | 0 |

(continued)

| Formulation | T0 | | | TFT | | | T-roll | | |
|---|---|---|---|---|---|---|---|---|---|
| | Cumulative concentration [particles/ml] | | | Cumulative concentration [particles/ml] | | | Cumulative concentration [particles/ml] | | |
| | Silicone oil-like particles | | | Silicone oil-like particles | | | Silicone oil-like particles | | |
| | ≥ 5 μm | ≥ 10 μm | ≥ 25 μm | ≥ 5 μm | ≥ 10 μm | ≥ 25 μm | ≥ 5 μm | ≥ 10 μm | ≥ 25 μm |
| F6-Barrel3(ii) | 13 | 3 | 0 | 553 | 33 | 0 | 819 | 73 | 0 |
| F9-Barrel3(ii) | 220 | 17 | 3 | 33 | 7 | 3 | 97 | 10 | 0 |
| F10-Barrel3 (ii) | 290 | 23 | 3 | 150 | 13 | 3 | 220 | 20 | 0 |

Particle levels ≥ 2 μm were overall low in this study, except for F5 (Figure 31). Silicone oil was found in all PFS samples at T0, mainly in the small size region (≥ 5 μm and potentially below, but differentiation not possible), likely causing increased particle levels. FT and rolling stress did not affect the non-silicone oil-like particle fractions, but some differences were observed for the silicone oil fractions. Silicone oil fractions increased after FT: F5 (large droplets). Silicone oil fractions increased after rolling stress: largest impact on F10 (large droplets), F6. Silicone oil levels ≥ 5 μm were overall lower in Barrel3(ii) PFS compared to Barrel1 PFS (Figure 32 and Figure 33).

*SEC analysis*

At T0 the SEC profiles of most formulations were comparable, with a monomer content of ca. 97%; a slight reduction of monomer content was observed for F5 (96.4%) > F6, F11 (96.8%). After stressing (FT and rolling stress), the monomer content remained unchanged for all formulations in both types of PFS. There was no difference between samples stressed in Barrel1 or Barrel3(ii) PFS (Figure 34). Initial HMWS levels were comparable for F2, F4, F7, F8, F9, F10, F12, and F13 at ca. 1.2%; slightly increased HMWS levels were found for F5 (1.8%) > F6, F11 (1.5%). All HMWS levels remained unchanged after FT and rolling stress. There was no difference between samples stressed in Barrel1 or Barrel3(ii) PFS (Figure 35). Initial LMWS levels were at ca. 1.7% for all formulations and remained unchanged after FT and rolling stress. There was no difference between samples stressed in Barrel1 or Barrel3(ii) PFS (Figure 36).

*Bioanalyzer purity*

At T0, the sample was 95-96% under non-reducing conditions, and 98% under reducing conditions, and comparable for all formulations. There was no difference between Barrel1 and Barrel3(ii) PFS (Figure 37). Similarly, there was no difference in the determined purity (Bioanalyzer) under non-reducing conditions before and after freeze-thaw and rolling stress (Figure 38), and no difference in the purity (Bioanalyzer) under reducing conditions before and after freeze-thaw and rolling stress (Figure 39).

*iCE*

At T0 the iCE profiles were comparable (main species ca. 49-50%) (slightly different pattern for F7), pI range was between 7.6 to 8.9; the pI range of F8 was slightly more acidic (7.2 to 8.1). After stressing (FT and rolling stress), the main species content remained unchanged for all formulations. There was no difference in the iCE profiles between Barrel1 and Barrel3(ii) PFS samples (Figure 40). Initial acidic species levels were comparable for all formulations at ca. 28-29%. All acidic species levels remained unchanged after FT and rolling stress. There was no difference between samples stressed in Barrel1 or Barrel3(ii) PFS (Figure 42). Initial basic species levels were at ca. 21-22% for all formulations and remained unchanged after FT and rolling stress. There was no difference between samples stressed in Barrel1 or Barrel3(ii) PFS (Figure 43).

*Conclusions*

- All formulations were successfully prepared meeting the target specifications of 150 mg/ml ± 15 mg/ml protein content and a target pH ± 0.1 pH units

- All formulations were free of visible particles and exhibited low levels of subvisible particles (MFI), turbidity (OD350, OD550) and soluble aggregates (SEC); the monomer content (SEC) was 96-97%; and the main isoform (iCE3) content was 49-50% for all formulations (results in consensus with expected values considering the CoA, received on 9th October 2018 and the expected impact of the sample preparation procedures)
- Viscosity was successfully reduced by addition of 150 mM of viscosity reducing agents, namely. Viscosities were lowest for 150 mM arginine in combination with histidine-bufferat ca. 6-7 mPa*s and highest for F11 and F12 at 16 and 18 mPa*s (no arginine)

Other formulation-related observations

- Slightly reduced thermal/conformational stability for F3 at pH 5.5
- F7 (proline) different melting curve and slightly different iCE pattern
- the pI range of F8 was slightly shifted towards acidic pI region (pI region 7.2 to 8.1), the pattern as such was comparable to other formulations (pI region 7.7 to 8.7)
- No major impact of methionine on protein stability in F10 so far
- Impact of different stressors on Ab1
- Freeze-thaw and rolling stress had no measurable impact on any formulation (protein stability)
- Impact of different stressors on PFS barrels
- Rolling stress increased the silicone oil droplet content in Barrel1 > Barrel3(ii) PFS

<u>Results - Stability 1 month</u>

The results of the stability test after one month of storage are shown below.

*pH*

At T0, the pH of all samples was within the acceptance limits of target pH ± 0.1 pH units. The pH remained unchanged after 1 month of storage, irrespective of the storage condition, as shown in Table 68.

| Time point | pH | | | | | | | | | | | | | | | | |
|---|---|---|---|---|---|---|---|---|---|---|---|---|---|---|---|---|---|
| | F1-Barrel1 | F2-Barrel1 | F3-Barrel1 | F4-Barrel1 | F5-Barrel1 | F6-Barrel1 | F7-Barrel1 | F8-Barrel1 | F9-Barrel1 | F10-Barrel1 | F11-Barrel1 | F12-Barrel1 | F13-Barrel1 | F2-Barrel3(ii) | F6- Barrel3(ii) | F9- Barrel3(ii) | F10- Barrel3(ii) |
| Tar-get | 6.0 | 6.0 | 5.5 | 6.5 | 6.0 | 6.0 | 6.0 | 6.0 | 6.0 | 6.0 | 6.0 | 6.0 | 6.0 | 6.0 | 6.0 | 6.0 | 6.0 |
| T0 | 6.14 | 6.01 | 5.48 | 6.49 | 6.09 | 6.01 | 6.04 | 6.05 | 6.04 | 6.03 | 6.09 | 6.09 | 6.02 | 6.03 | 6.00 | 6.03 | 6.02 |
| T1m 5C | 6.15 | 6.05 | 5.48 | 6.51 | 6.11 | 6.02 | 6.05 | 6.05 | 6.06 | 6.04 | 6.15 | 6.12 | 6.05 | 6.03 | 6.01 | 6.07 | 6.03 |
| T1m 25C | 6.13 | 6.03 | 5.53 | 6.47 | 6.10 | 6.04 | 6.03 | 6.06 | 6.06 | 6.05 | 6.11 | 6.09 | 6.04 | 6.02 | 6.03 | 6.06 | 6.04 |
| T1m 40C | 6.15 | 6.04 | 5.47 | 6.46 | 6.05 | 6.03 | 6.00 | 6.04 | 6.03 | 6.04 | 6.12 | 6.11 | 6.04 | 6.07 | 6.02 | 6.02 | 6.04 |

Table 57. pH before and after one month of storage

*Osmolarity*

Initial osmolalities were largely in the expected/acceptable range except for F6 which showed lower osmolality than expected possibly due to the complex formulation of citrate with arginine and F7 and F8 which showed higher osmolality up to 460 mOsmol/kg. After 1 month of storage at 40 °C, the osmolalities remained unchanged. At T0, the pH of all samples was within the acceptance limits of target pH ± 0.1 pH units. The pH remained unchanged after 1 month of storage, irrespective of the storage condition, as shown in Table 69.

| Time point | Osmolality | | | | | | | | | | | | | | | | |
|---|---|---|---|---|---|---|---|---|---|---|---|---|---|---|---|---|---|
| | F1-Barrel1 | F2-Barrel1 | F3-Barrel1 | F4-Barrel1 | F5-Barrel1 | F6-Barrel1 | F7-Barrel1 | F8-Barrel1 | F9-Barrel1 | F10-Barrel1 | F11-Barrel1 | F12-Barrel1 | F13-Barrel1 | F2-Barrel3(ii) | F6- Barrel3(ii) | F9- Barrel3(ii) | F10- Barrel3(ii) |
| T0 | 380 | 356 | 360 | 339 | 384 | 232 | 428 | 456 | 369 | 357 | 353 | 416 | 343 | 354 | 232 | 369 | 357 |
| T1m 5C | n.a. | n.a. | n.a. | n.a. | n.a. | n.a. | n.a. | n.a. | n.a. | n.a. | n.a. | n.a. | n.a. | n.a. | n.a. | n.a. | n.a. |
| T1m 25C | n.a. | n.a. | n.a. | n.a. | n.a. | n.a. | n.a. | n.a. | n.a. | n.a. | n.a. | n.a. | n.a. | n.a. | n.a. | n.a. | n.a. |
| T1m 40C | 373 | 357 | 356 | 336 | 363 | 230 | 428 | 452 | 362 | 358 | 355 | 415 | 350 | 349 | 233 | 368 | 358 |

Table 58. Osmolality before and after one month of storage

*Thermal stability*

nanoDSF results before and after T1m of storage are shown in Figure 44 to Figure 46. At T0, no impact of methionine (F10) or higher PS80 content, slightly reduced thermal/conformational stability at pH 5.5 (F3) compared to pH 6.5 (F4). Values remained unchanged for all formulations after 1 month of storage, irrespective of the storage condition. Similar trends as were observed with the fluorescence signal (F350) and back reflection. Onset of aggregation remained unchanged after storage at 5 °C and 25°C. All thermal events remained comparable to T0 after T1m, irrespective of the type of PFS (Barrel1 or Barrel3(ii)).

*Protein content*

At T0, the protein content of all samples was within the acceptance limits of 150 ± 15mg/ml. The protein content was unchanged after storage of 1 month at 5 °C, 25 °C and 40°C (Table 70).

| Time point | Protein content[mg/ml] A280-A320 | | | | | | | | | | | | | | | | |
|---|---|---|---|---|---|---|---|---|---|---|---|---|---|---|---|---|
| | F1-Barrel1 | F2-Barrel1 | F3-Barrel1 | F4-Barrel1 | F5-Barrel1 | F6-Barrel1 | F7-Barrel1 | F8-Barrel1 | F9-Barrel1 | F10-Barrel1 | F11-Barrel1 | F12-Barrel1 | F13-Barrel1 | F2-Barrel3(ii) | F6- Barrel3(ii) | F9- Barrel3(ii) | F10- Barrel3(ii) |
| T0 | 154 | 144 | 147 | 150 | 151 | 154 | 150 | 158 | 145 | 153 | 153 | 152 | 157 | 145 | 148 | 147 | 150 |
| T1m 5C | 145 | 143 | 150 | 156 | 158 | 159 | 149 | 146 | 154 | 153 | 148 | 151 | 162 | 148 | 158 | 153 | 158 |
| T1m 25C | 155 | 141 | 145 | 145 | 143 | 142 | 149 | 151 | 143 | 144 | 147 | 154 | 142 | 144 | 150 | 144 | 147 |
| T1m 40C | 143 | 143 | 144 | 144 | 141 | 146 | 147 | 147 | 144 | 143 | 152 | 151 | 143 | 145 | 153 | 144 | 141 |

Table 59: Protein concentration before and after one month of storage

*Turbidity*

Initial OD350 values were comparable for most formulations, but slightly higher for F1, F5 and F8; there was no difference between samples in Barrel1 and Barrel3(ii) PFS. No difference in the OD350 before and after storage for 1 month at 5 °C. OD350 values increased after storage at 25 °C < 40 °C. Increase in OD350 at 40 °C was less pronounced for the citrate-based formulations F5, F6 compared to the histidine-based formulations (Figure 47). Initial OD550 values were comparable for most formulations, but slightly higher for F1, and slightly lower for F7; there was no difference between samples in Barrel1 and Barrel3(ii) PFS. No change upon storage for 1 month at 5 °C and 25 °C. OD550 values increased after storage at 40 °C independent of formulation and syringe type (Figure 48).

*Visual inspection*

At T0, all samples were practically free of visible particles and exhibited a yellowish coloration. After storage for 1 month at 5 °C and 25 °C all formulations remained unchanged. After storage for 1 month at 40 °C, most formulation remained particle-free, but F3 (pH 5.5) showed a slightly increased number of particles (score of 2), F1/F5/F6 appeared turbid after T1m at 40 °C compared to other formulations (Table 71).

| Time point | Visual inspection DAC score examiner1 / examiner2 | | | | | | | | | | | | | | | | |
|---|---|---|---|---|---|---|---|---|---|---|---|---|---|---|---|---|---|
| | F1-Barrel1 | F2-Barrel1 | F3-Barrel1 | F4-Barrel1 | F5-Barrel1 | F6-Barrel1 | F7-Barrel1 | F8-Barrel1 | F9-Barrel1 | F10-Barrel1 | F11-Barrel1 | F12-Barrel1 | F13-Barrel1 | F2-Barrel3(ii) | F6- Barrel3(ii) | F9- Barrel3(ii) | F10-Barrel3(ii) |
| T0 | 0/0 | 0/0 | 0/0 | 0/0 | 0/0 | 0/0 | 0/0 | 0/0 | 0/0 | 0/0 | 0/0 | 0/0 | 0/0 | 0/0 | 0/0 | 0/0 | 0/0 |
| T1m 5C | 0/0 | 0/0 | 0/0 | 0/0 | 0/0 | 0/0 | 0/0 | 0/0 | 0/0 | 0/0 | 0/0 | 0/0 | 0/0 | 0/0 | 0/0 | 0/0 | 0/0 |
| T1m 25C | 0/0 | 0/0 | 0/0 | 0/0 | 1/0 | 0/0 | 0/0 | 1/0 | 0/0 | 0/0 | 1/1 | 0/0 | 0/0 | 0/0 | 0/0 | 0/0 | 0/0 |
| T1m 40C | 0/0 | 0/0 | 2/2 | 0/1 | 0/0 | 0/1 | 1/1 | 0/0 | 0/0 | 0/0 | 0/0 | 0/0 | 0/0 | 1/0 | 0/1 | 0/0 | 0/0 |

Table 60: Visual inspection before and after one month of storage

*MFI*

All formulations were measured diluted in their respective formulation buffer (at ca. 50 mg/ml). The particle content of the dilution media (Buffer) was very low and comparable for all formulation buffers. The particle levels of protein-containing samples was higher compared to the formulation buffers (dilution medium), overall highest for F5 (* exceeding y-axis), confirming results reported for T0 (see Figure 49).

Particle levels ≥ 2 μm were overall low in this study (Figure 50), except for F5 at T0 and after storage for 1 month at 5 °C. Silicone oil was found in all PFS samples, mainly in the small size region (≥ 5 μm and potentially below, but differentiation not possible), likely causing increased particle levels (Figure 51). After storage for 1 month at 5 °C, non-silicone oil-like particle fractions increased more than at 25 °C and 40 °C (especially F8, F9, F13, F1, F11); solely for F6 in Barrel3(ii) PFS particles of all size classes appeared to have increased at 40 °C. Silicone oil fractions (mainly in the size range ≥ 5 and 10 μm) slightly increased after storage for 1 month at 40 °C (Figure 52, Figure 53). Silicone oil levels ≥ 5 μm were overall lower in Barrel3(ii) PFS compared to Barrel1 PFS. For a summary of the results see Table 72 and Table 73.

Table 61: MFI results before and after 1 month of storage: non-silicone oil droplet-like particles

| Formulation | T0 | | | | T1m_5C | | | |
|---|---|---|---|---|---|---|---|---|
| | Cumulative concentration [particles/ml] | | | | Cumulative concentration [particles/ml] | | | |
| | Total | Non-silicone oil particles | | | Total | Non-silicone oil particles | | |
| | ≥ 2 μm | ≥ 5 μm | ≥ 10 μm | ≥ 25 μm | ≥ 2 μm | ≥ 5 μm | ≥ 10 μm | ≥ 25 μm |
| F1-Barrel1 | 3,801 | 546 | 160 | 50 | 15,302 | 1,989 | 713 | 133 |
| F2-Barrel1 | 2,811 | 446 | 137 | 23 | 11,611 | 833 | 280 | 77 |
| F3-Barrel1 | 2,611 | 333 | 103 | 20 | 9,683 | 483 | 190 | 97 |
| F4-Barrel1 | 1,949 | 273 | 150 | 37 | 5,349 | 713 | 300 | 60 |
| F5-Barrel1 | 48,88 | 1,366 | 353 | 80 | 52,434 | 1,775 | 400 | 107 |
| F6-Barrel1 | 2,105 | 280 | 147 | 50 | 7,558 | 1,352 | 699 | 270 |
| F7-Barrel1 | 4,433 | 360 | 133 | 17 | 10,376 | 623 | 246 | 53 |
| F8-Barrel1 | 2,501 | 266 | 120 | 37 | 21,194 | 4,387 | 1,802 | 490 |
| F9-Barrel1 | 6,998 | 573 | 213 | 33 | 20,978 | 2,911 | 1,156 | 336 |

(continued)

| Formulation | T0 | | | | T1m_5C | | | |
|---|---|---|---|---|---|---|---|---|
| | Cumulative concentration [particles/ml] | | | | Cumulative concentration [particles/ml] | | | |
| | Total | Non-silicone oil particles | | | Total | Non-silicone oil particles | | |
| | ≥ 2 μm | ≥ 5 μm | ≥ 10 μm | ≥ 25 μm | ≥ 2 μm | ≥ 5 μm | ≥ 10 μm | ≥ 25 μm |
| F10-Barrel1 | 4,084 | 580 | 250 | 70 | 10,752 | 1,779 | 783 | 210 |
| F11-Barrel1 | 6,342 | 510 | 183 | 63 | 18,246 | 1,962 | 663 | 190 |
| F12-Barrel1 | 4,567 | 303 | 43 | 7 | 19,236 | 560 | 227 | 60 |
| F13-Barrel1 | 1,952 | 276 | 157 | 37 | 12,014 | 2,681 | 1,166 | 293 |
| F2-Barrel3(ii) | 1,615 | 530 | 286 | 80 | 3,887 | 1,113 | 470 | 103 |
| F6-Barrel3(ii) | 1,572 | 390 | 177 | 57 | 5,556 | 1,259 | 613 | 197 |
| F9-Barrel3(ii) | 3,984 | 410 | 163 | 57 | 3,661 | 896 | 406 | 150 |
| F10-Barrel3(ii) | 3,311 | 263 | 127 | 40 | 5,839 | 1,759 | 759 | 200 |
| **Formulation** | **T1m_25C** | | | | **T1m_40C** | | | |
| | Cumulative concentration [particles/ml] | | | | Cumulative concentration [particles/ml] | | | |
| | Total | Non-silicone oil particles | | | Total | Non-silicone oil particles | | |
| | ≥ 2 μm | ≥ 5 μm | ≥ 10 μm | ≥ 25 μm | ≥ 2 μm | ≥ 5 μm | ≥ 10 μm | ≥ 25 μm |
| F1-Barrel1 | 3,741 | 283 | 110 | 37 | 10,915 | 483 | 123 | 33 |
| F2-Barrel1 | 5,659 | 699 | 363 | 160 | 5,752 | 333 | 160 | 50 |
| F3-Barrel1 | 10,65 | 1,715 | 799 | 233 | 5,323 | 213 | 60 | 13 |
| F4-Barrel1 | 4,467 | 729 | 366 | 123 | 3,707 | 243 | 87 | 40 |
| F5-Barrel1 | 6,012 | 1,093 | 513 | 137 | 7,185 | 596 | 203 | 77 |
| F6-Barrel1 | 3,664 | 706 | 430 | 200 | 8,207 | 386 | 127 | 30 |
| F7-Barrel1 | 7,541 | 426 | 230 | 93 | 9,603 | 583 | 140 | 40 |
| F8-Barrel1 | 7,161 | 470 | 296 | 143 | 5,416 | 473 | 143 | 63 |
| F9-Barrel1 | 5,676 | 570 | 310 | 120 | 6,962 | 173 | 23 | 13 |
| F10-Barrel1 | 3,727 | 520 | 303 | 143 | 3,461 | 193 | 87 | 27 |
| F11-Barrel1 | 9,590 | 936 | 450 | 137 | 7,381 | 420 | 103 | 17 |
| F12-Barrel1 | 5,822 | 323 | 130 | 47 | 13,190 | 606 | 123 | 40 |
| F13-Barrel1 | 5,253 | 416 | 217 | 107 | 7,038 | 316 | 90 | 37 |
| F2-Barrel3(ii) | 2,462 | 606 | 340 | 67 | 3,591 | 613 | 236 | 73 |
| F6-Barrel3(ii) | 1,332 | 300 | 130 | 47 | 25,385 | 8,500 | 3,361 | 736 |
| F9-Barrel3(ii) | 7,405 | 1,262 | 530 | 117 | 3,158 | 500 | 180 | 53 |
| F10-Barrel3(ii) | 2,888 | 456 | 250 | 90 | 1,386 | 190 | 77 | 20 |

Table 62: MFI results before and after 1 month of storage: silicone oil droplet-like particles

| **Formulation** | **T0** | | | **T1m_5C** | | |
|---|---|---|---|---|---|---|
| | Cumulative concentration [particles/ml] | | | Cumulative concentration [particles/ml] | | |
| | Non-silicone oil particles | | | Non-silicone oil particles | | |
| | ≥ 5 μm | ≥ 10 μm | ≥ 25 μm | ≥ 5 μm | ≥ 10 μm | ≥ 25 μm |
| F1-Barrel1 | 183 | 10 | 3 | 1,352 | 33 | 0 |
| F2-Barrel1 | 243 | 20 | 0 | 1,119 | 53 | 3 |
| F3-Barrel1 | 117 | 20 | 0 | 1,089 | 93 | 10 |
| F4-Barrel1 | 230 | 13 | 0 | 440 | 17 | 10 |
| F5-Barrel1 | 1,446 | 20 | 7 | 110 | 20 | 7 |
| F6-Barrel1 | 233 | 3 | 0 | 816 | 77 | 7 |
| F7-Barrel1 | 266 | 3 | 0 | 2,158 | 183 | 7 |
| F8-Barrel1 | 240 | 57 | 3 | 1,126 | 130 | 7 |
| F9-Barrel1 | 706 | 27 | 0 | 1,066 | 77 | 10 |
| F10-Barrel1 | 380 | 17 | 7 | 1,109 | 47 | 10 |
| F11-Barrel1 | 1,522 | 127 | 0 | 2,558 | 87 | 20 |
| F12-Barrel1 | 1,009 | 23 | 0 | 1,003 | 37 | 7 |
| F13-Barrel1 | 110 | 13 | 0 | 909 | 37 | 0 |
| F2-Barrel3(ii) | 43 | 3 | 3 | 113 | 23 | 3 |
| F6-Barrel3(ii) | 13 | 3 | 0 | 63 | 27 | 13 |
| F9-Barrel3(ii) | 220 | 17 | 3 | 103 | 10 | 0 |
| F10-Barrel3(ii) | 290 | 23 | 3 | 63 | 30 | 3 |
| **Formula tion** | **T1m_25C** | | | **T1m_40C** | | |
| | Cumulative concentration [particles/ml] | | | Cumulative concentration [particles/ml] | | |
| | Non-silicone oil particles | | | Non-silicone oil particles | | |
| | ≥ 5 μm | ≥ 10 μm | ≥ 25 μm | ≥ 5 μm | ≥ 10 μm | ≥ 25 μm |
| F1-Barrel1 | 476 | 47 | 0 | 1,729 | 137 | 10 |
| F2-Barrel1 | 227 | 37 | 10 | 1,049 | 67 | 7 |
| F3-Barrel1 | 916 | 93 | 0 | 933 | 77 | 3 |
| F4-Barrel1 | 356 | 17 | 7 | 273 | 17 | 0 |
| F5-Barrel1 | 170 | 27 | 7 | 1,222 | 197 | 3 |
| F6-Barrel1 | 416 | 67 | 17 | 939 | 33 | 7 |
| F7-Barrel1 | 1,152 | 40 | 7 | 2,305 | 306 | 0 |
| F8-Barrel1 | 580 | 50 | 3 | 956 | 127 | 3 |
| F9-Barrel1 | 736 | 47 | 3 | 1,003 | 53 | 0 |
| F10-Barrel1 | 350 | 20 | 7 | 626 | 123 | 3 |
| F11-Barrel1 | 886 | 50 | 7 | 513 | 7 | 0 |
| F12-Barrel1 | 366 | 67 | 7 | 2,175 | 187 | 3 |
| F13-Barrel1 | 230 | 17 | 0 | 603 | 7 | 0 |

(continued)

| Formula tion | T1m_25C | | | T1m_40C | | |
|---|---|---|---|---|---|---|
| | Cumulative concentration [particles/ml] | | | Cumulative concentration [particles/ml] | | |
| | Non-silicone oil particles | | | Non-silicone oil particles | | |
| | ≥ 5 μm | ≥ 10 μm | ≥ 25 μm | ≥ 5 μm | ≥ 10 μm | ≥ 25 μm |
| F2-Barrel3(ii) | 163 | 27 | 7 | 97 | 17 | 3 |
| F6-Barrel3(ii) | 27 | 7 | 7 | 140 | 30 | 7 |
| F9-Barrel3(ii) | 273 | 57 | 7 | 340 | 100 | 20 |
| F10-Barrel3(ii) | 476 | 47 | 0 | 1,729 | 137 | 10 |

*SEC results*

The initial SEC profiles of most formulations were comparable, with a monomer content of ca. 97.6%; a slightly lower monomer content was observed for F5 (97.0%) > F6, F11 (97.3%). The monomer content decreased after storage for 1 month at 25 °C < 40 °C for all formulations in both types of PFS, but remained unchanged at 5 °C; lowest monomer content after storage at 25 °C/ 40 °C was observed for F1, F3, F5, F11, F12 (ca. 95% at 40 °C). There was no difference between samples stored in Barrel1 or Barrel3(ii) PFS (Figure 54). Initial HMWS levels were comparable for F1-F4, F7-F10, F12 and F13 at ca. 1.0%; slightly increased HMWS levels were found for F5 (1.4%) > F6, F11 (1.3%). HMWS levels increased after storage for 1 month at 25 °C < 40 °C (especially F5, F1, F11, F3, F12). There was no difference between samples stored in Barrel1 or Barrel3(ii) PFS (Figure 55). Initial LMWS levels were at ca. 1.5% for all formulations. LMWS levels increased after storage for 1 month at 25 °C < 40 °C relatively independent of the type of formulation. There was no difference between samples stored in Barrel1 or Barrel3(ii) PFS (Figure 56).

*Bioanalyzer purity*

The purity (Bioanalyzer) under non-reducing conditions remained largely unchanged after storage for 1 month. There was no difference between samples stored in Barrel1 or Barrel3(ii) PFS (Figure 57). The purity (Bioanalyzer) under reducing conditions was not affected after storage for 1 month irrespective of storage temperature. There was no difference between samples stored in Barrel1 or Barrel3(ii) PFS (Figure 58).

*iCE*

At T0 the iCE profiles were comparable (main species ca. 50%) (slightly different pattern for F7), pI range was between 7.6 to 8.9; only the pI range of F8 was slightly more acidic (7.2 to 8.1). Main species content decreased similarly in all formulations after 1 month of storage at 25 °C < 40 °C, but remained unchanged at 5 °C. There was no difference in the iCE profiles between Barrel1 and Barrel3(ii) PFS samples (Figure 59 and Figure 60). Initial acidic species levels were comparable for all formulations at ca. 28%. Acidic species content increased in all formulations after 1 month of storage at 25 °C <40 °C with little difference between the formulations; no change after T1m of storage at 5 °C. There was no difference between samples stored in Barrel1 or Barrel3(ii) PFS (Figure 61). Initial basic species levels were at ca. 21% for all formulations, and remained relatively unaffected after storage at 5 °c and 25 °C. Basic species content decreased after storage for 1 month at 40 °C with little difference between the formulations; no change after T1m of storage at 5 °C. There was no difference between samples stored in Barrel1 or Barrel3(ii) PFS (Figure 62).

*Conclusions*

- All formulations are within the target specifications of 150 mg/ml ± 15 mg/ml protein content and a target pH ± 0.1 pH units;
- At T0, all formulations were free of visible particles and exhibited low levels of subvisible particles (MFI), turbidity (OD350, OD550) and soluble aggregates (SEC); the monomer content (SEC) was ca.98%; and the main isoform (iCE3) content was ca. 50% for all formulations
- Viscosity was successfully reduced by addition of 150 mM of viscosity reducing agents. Viscosities were lowest for 150 mM arginine in combination with histidine-buffer at ca. 6-7 mPa*s and highest for F11 and F12 at 16 and 18 mPa*s (no arginine).

After storage for 1 month at 5°C

- All samples remained stable with no/little changes after storage at 5 °C; only subvisible particle concentration increased slightly for some formulations

After storage for 1 month at 25°C < 40°C

- pH, osmolality (only measured for 40 °C samples), protein content, thermal stability (nanoDSF), as well as sample purity (Bioanalyzer) remained unchanged after storage at 25 °C and 40 °C for all formulations

- All formulations were similarly affected: increase in OD550 values (turbidity), LMWS species (SEC), as well as acidic and basic species (iCE)

Impact of PFS barrel Barrel1 versus Barrel3(ii):

- No differences observed between Barrel1 and Barrel3(ii) PFS at 5 °C and 25 °C, solely storage at 40 °C increased content of silicone oil droplet-like particles in Barrel1 > Barrel3(ii) PFS

<u>Results - Stability 3 months</u>

The results of the stability test after one month of storage are shown below.

*pH*

At T0, the pH of all samples was within the acceptance limits of target pH ± 0.1 pH units. The pH remained unchanged after 3 months of storage, irrespective of the storage condition (Table 74).

| Time point | pH | | | | | | | | | | | | | | | | |
|---|---|---|---|---|---|---|---|---|---|---|---|---|---|---|---|---|---|
| | F1-Barrel1 | F2-Barrel1 | F3-Barrel1 | F4-Barrel1 | F5-Barrel1 | F6-Barrel1 | F7-Barrel1 | F8-Barrel1 | F9-Barrel1 | F10-Barrel1 | F11-Barrel1 | F12-Barrel1 | F13-Barrel1 | F2-Barrel3(ii) | F6- Barrel3(ii) | F9- Barrel3(ii) | F10- Barrel3(ii) |
| Target | 6.0 | 6.0 | 5.5 | 6.5 | 6.0 | 6.0 | 6.0 | 6.0 | 6.0 | 6.0 | 6.0 | 6.0 | 6.0 | 6.0 | 6.0 | 6.0 | 6.0 |
| T0 | 6.14 | 6.01 | 5.48 | 6.49 | 6.09 | 6.01 | 6.04 | 6.05 | 6.04 | 6.03 | 6.09 | 6.09 | 6.02 | 6.03 | 6.00 | 6.03 | 6.02 |
| T3m 5C | 6.17 | 6.04 | 5.47 | 6.48 | 6.08 | 6.00 | 6.01 | 6.06 | 6.04 | 6.02 | 6.06 | 6.08 | 6.01 | 6.02 | 6.01 | 6.01 | 6.04 |
| T3m 25C | 6.16 | 6.03 | 5.51 | 6.42 | 6.06 | 6.01 | 6.04 | 5.93 | 6.03 | 6.06 | 6.11 | 6.10 | 6.03 | 6.02 | 6.01 | 6.02 | 6.03 |
| T3m 40C | 6.16 | 6.08 | 5.55 | 6.51 | 6.10 | 6.01 | 6.03 | 6.05 | 6.04 | 6.03 | 6.10 | 6.07 | 6.04 | 6.03 | 6.01 | 6.03 | 6.04 |

Table 63: pH before and after 3 months of storage

*Osmolarity*

Initial osmolalities were largely in the expected/acceptable range except for (i) F6 which showed lower osmolality than expected possibly due to the complex formulation of citrate with arginine and (ii) F7 and F8 which showed higher osmolality of up to 460 mOsmol/kg. No strict acceptance criteria had been defined; selected lead candidates may require adjustments of (i) sample preparation procedure, and/or (ii) mannitol content. After 3 months of storage, the osmolalities remained unchanged, irrespective of the storage condition (Table 75).

| Time point | Osmolality | | | | | | | | | | | | | | | | |
|---|---|---|---|---|---|---|---|---|---|---|---|---|---|---|---|---|---|
| | F1-Barrel1 | F2-Barrel1 | F3-Barrel1 | F4-Barrel1 | F5-Barrel1 | F6-Barrel1 | F7-Barrel1 | F8-Barrel1 | F9-Barrel1 | F10-Barrel1 | F11-Barrel1 | F12-Barrel1 | F13-Barrel1 | F2-Barrel3(ii) | F6- Barrel3(ii) | F9- Barrel3(ii) | F10- Barrel3(ii) |
| T0 | 380 | 356 | 360 | 339 | 384 | 232 | 428 | 456 | 369 | 357 | 353 | 416 | 343 | 354 | 232 | 369 | 357 |
| T3m 5C | 376 | 345 | 354 | 339 | 362 | 229 | 429 | 451 | 365 | 353 | 357 | 410 | 349 | 348 | 234 | 369 | 356 |
| T3m 25C | 383 | 354 | 358 | 341 | 370 | 231 | 431 | 465 | 373 | 362 | 363 | 426 | 354 | 357 | 237 | 373 | 361 |
| T3m 40C | 382 | 344 | 360 | 340 | 364 | 233 | 431 | 457 | 371 | 365 | 360 | 424 | 352 | 351 | 232 | 372 | 362 |

Table 64. Osmolality before and after 3 months of storage

*Dynamic viscosity*

Initial values for dynamic viscosity were < 20 mPa*s for all formulations. The most effective viscosity reducing agent was arginine (F2, F3, F4, F6, F9, F10, F13), followed by lysine (F8) and proline (F7); formulations containing no amino acids exhibited higher initial values for dynamic viscosity (F1, F5: NaCl; F11, F12: polyols). After 3 months of storage, the dynamic viscosity remained largely unchanged, independent of storage condition (Table 76).

| Time point | Dynamic viscosity [mPa*s] at 20 °C | | | | | | | | | | | | | | | | |
|---|---|---|---|---|---|---|---|---|---|---|---|---|---|---|---|---|---|
| | F1-Barrel1 | F2-Barrel1 | F3-Barrel1 | F4-Barrel1 | F5-Barrel1 | F6-Barrel1 | F7-Barrel1 | F8-Barrel1 | F9-Barrel1 | F10-Barrel1 | F11-Barrel1 | F12-Barrel1 | F13-Barrel1 | F2-Barrel3(ii) | F6- Barrel3(ii) | F9- Barrel3(ii) | F10- Barrel3(ii) |
| T0 | 11.4 | 6.7 | 6.8 | 7.0 | 11.8 | 7.8 | 10.4 | 8.3 | 9.3 | 7.1 | 16.1 | 18.2 | 6.4 | 6.4 | 7.9 | 9.4 | 7.2 |
| T3m 5C | 10.9 | 6.6 | 6.8 | 6.8 | 11.7 | 8.0 | 10.5 | 8.3 | 9.4 | 6.7 | 15.9 | 18.9 | 6.6 | 6.3 | 7.6 | 9.5 | 7.1 |
| T3m 25C | 10.6 | 6.6 | 7.0 | 6.8 | 11.7 | 7.8 | 11.1 | 8.6 | 9.2 | 6.7 | 16.4 | 18.6 | 6.5 | 6.5 | 7.9 | 9.3 | 6.7 |
| T3m 40C | 10.6 | 6.4 | 7.2 | 6.8 | 10.5 | 7.4 | 10.2 | 8.0 | 8.8 | 6.7 | 14.8 | 16.5 | 6.5 | 6.4 | 7.3 | 9.0 | 6.9 |

Table 65: Dynamic viscosity results before and after 3 months of storage

No difference observed between samples stored in Barrel1 or Barrel3(ii) PFS (Figure 63).

*Syringeability*

Gliding forces after storage for 3 months at 5 °C were as expected and compliant with results for dynamic viscosity: highest (17-19 N) for F11 and F12; no difference between samples stored in Barrel1 or Barrel3(ii) PFS. Comparing 25 °C to 5 °C storage of 3 months, gliding forces increased slightly for samples in Barrel3(ii) PFS, whilst they were comparable for Barrel1 PFS (no formulation effect observed). Comparing 40 °C to 5 °C storage of 3 months, gliding forces increased clearly for all formulations with similar forces required for Barrel1 and Barrel3(ii) PFS (Figure 64 - Figure 66).

*Thermal stability*

At T0, no impact of methionine (F10 vs. F2) or higher PS80 content. At T0, slightly reduced thermal/conformational stability at pH 5.5 (F3) compared to pH 6.5 (F4). Potentially slightly positive impact of composition of histidine with proline (F7) and the His/Man or His/Suc combinations F11 and F12. Values remained unchanged for all formulations after 3 months of storage at 5 °C and 25 °C, but were slightly reduced after storage at 40 °C (similar for all formulations) (Figure 67).

Similar trends as were observed with the fluorescence signal (F350) were observed with the back reflection. Onset of aggregation remained unchanged after storage at 5 °C and 25 °C. Reduction in Tagg, onset for all formulations after 3 months of storage at 40 °C, most pronounced for F3, and least pronounced for F7, F9 and F10 (Figure 68). No impact of the type of PFS (Barrel1 or Barrel3(ii)) on nanoDSF results (Figure 69).

*Protein content*

At T0, the protein content of all samples was within the acceptance limits of 150 ± 15 mg/ml. The protein content did not change during storage of 3 months at 5 °C, 25 °C and 40 °C (Table 77).

| Time point | Protein content[mg/ml] A280-A320 | | | | | | | | | | | | | | | | |
|---|---|---|---|---|---|---|---|---|---|---|---|---|---|---|---|---|---|
| | F1-Barrel1 | F2-Barrel1 | F3-Barrel1 | F4-Barrel1 | F5-Barrel1 | F6-Barrel1 | F7-Barrel1 | F8-Barrel1 | F9-Barrel1 | F10-Barrel1 | F11-Barrel1 | F12-Barrel1 | F13-Barrel1 | F2-Barrel3(ii) | F6- Barrel3(ii) | F9- Barrel3(ii) | F10- Barrel3(ii) |
| T0 | 154 | 144 | 147 | 150 | 151 | 154 | 150 | 158 | 145 | 153 | 153 | 152 | 157 | 145 | 148 | 147 | 150 |
| T3m 5C | 158 | 148 | 154 | 151 | 148 | 150 | 143 | 148 | 153 | 150 | 160 | 161 | 152 | 148 | 148 | 151 | 151 |
| T3m 25C | 150 | 146 | 151 | 155 | 149 | 153 | 156 | 158 | 153 | 154 | 158 | 159 | 147 | 149 | 149 | 152 | 155 |
| T3m 40C | 148 | 154 | 153 | 142 | 150 | 143 | 143 | 145 | 141 | 140 | 142 | 149 | 149 | 145 | 141 | 141 | 140 |

Table 66: Protein concentration before and after 3 months of storage

*Turbidity*

Initial OD350 values were comparable for most formulations, but slightly higher for F1, F5 and F8; there was no difference between samples in Barrel1 and Barrel3(ii) PFS. No difference in the OD350 before and after storage for up to 3 months at 5 °C. OD350 values increased after storage at 25 °C < 40 °C. Increase in OD350 at 40 °C was highest for F1, F8 and F12, and least pronounced for the citrate-based formulations F5, F6 compared to the histidine-based formulations (Figure 70). Initial OD550 values were comparable for most formulations, but slightly higher for F1 and slightly lower for F7; there was no difference between samples in Barrel1 and Barrel3(ii) PFS. No change upon storage for up to 3 months at 5 °C and 25 °C. OD550 values increased after T3m storage at 40 °C independent of formulation and PFS type (Figure 71).

*Visual inspection*

At T0, all samples were practically free of visible particles and exhibited a yellowish coloration. At 5 °C storage (T3m), all formulations remained free of visible particles, but samples F5, F6 appeared slightly turbid. At 25 °C storage (T3m), most formulations remained unchanged but F1, F5 and F6 exhibited clearly visible particles (score 10). At 40 °C storage (T3m), all formulations exhibited visible particles (turbid appearance), solely F7, F11, F12 in Barrel1, and F9 in Barrel3(ii) PFS remained unchanged (Table 78).

| Time point | Visual inspection DAC score examiner1 / examiner2 | | | | | | | | | | | | | | | | |
|---|---|---|---|---|---|---|---|---|---|---|---|---|---|---|---|---|---|
| | F1-Barrel1 | F2-Barrel1 | F3-Barrel1 | F4-Barrel1 | F5-Barrel1 | F6-Barrel1 | F7-Barrel1 | F8-Barrel1 | F9-Barrel1 | F10-Barrel1 | F11-Barrel1 | F12-Barrel1 | F13-Barrel1 | F2-Barrel3(ii) | F6- Barrel3(ii) | F9- Barrel3(ii) | F10- Barrel3(ii) |
| T0 | 0/0 | 0/0 | 0/0 | 0/1 | 0/0 | 0/0 | 0/0 | 0/0 | 0/0 | 0/0 | 0/0 | 0/0 | 0/0 | 0/0 | 0/0 | 0/0 | 0/0 |
| T3m 5C | 0/0 | 1/0 | 0/0 | 0/0 | 0/0 | 0/0 | 0/0 | 0/0 | 0/0 | 0/0 | 0/0 | 0/0 | 0/0 | 0/0 | 0/0 | 0/0 | 0/0 |
| T3m 25C | 10/10 | 0/0 | 0/0 | 0/0 | 10/10 | 10/10 | 0/0 | 0/0 | 0/0 | 1/1 | 0/0 | 0/0 | 1/0 | 1/0 | 10/10 | 0/0 | 1/2 |
| T3m 40C | 10/10 | 10/2 | 10/10 | 2/2 | 2/10 | 10/10 | 0/0 | 10/10 | 10/10 | 10/10 | 0/0 | 1/1 | 10/10 | 10/10 | 10/10 | 0/0 | 10/10 |

Table 67**:** Visual inspection before and after 3 months of storage

*MFI*

Particle levels ≥ 2 μm were overall low in this study, except for F5 at T0 and after storage for up to 3 months at 5 °C (Figure 72). After storage for 3 months the non-silicone oil-like particle fractions were largely unaffected irrespective of storage condition or formulation (observed for particle levels ≥ 5 μm and larBarrel1), solely slightly increased levels for F1 and F5 (Figure 73). Silicone oil-like particle fractions increased after storage for 3 months at 25 °C < 40 °C (mainly in the size range ≥ 5 and 10 μm) with no/little difference between samples stored in Barrel1 and PFS (Figure 74, Figure 75).

*SEC results*

The initial SEC profiles of most formulations were comparable, with a monomer content of ca. 97.6%; a slightly lower monomer content was observed for F5 (97.0%) > F6, F11 (97.3%). The monomer content remained largely unchanged after storage for up to 3 months at 5 °C. The monomer content of all formulations decreased after storage for 3 months at 25 °C < 40 °C; lowest monomer content after storage was observed for F3 (ca. 88% at 40 °C). There was no difference between samples stored in Barrel1 or Barrel3(ii) PFS (Figure 76). Initial HMWS levels were comparable for F1-F4, F7-F10, F12 and F13 at ca. 1.0%; slightly increased HMWS levels were found for F5 (1.4%) > F6, F11 (1.3%). HMWS levels increased slightly after storage of 3 months at 5 °C. HMWS levels increased after storage of 3 months at 25 °C < 40 °C (especially F1, F3, F5, F11, F12: 4-5% after 3 months of storage at 40 °C). There was no difference between samples stored in Barrel1 or Barrel3(ii) PFS (Figure 77). Initial LMWS levels were at ca. 1.5% for all formulations. LMWS levels remained unchanged after storage for 3 months at 5 °C. LMWS levels increased after storage for 3 months at 25 °C < 40 °C for all formulations; only formulation effect for F3 with highest LMWS content after 3 months of storage at 40 °C (6.8%). There was no difference between samples stored in Barrel1 or Barrel3(ii) PFS (Figure 78).

*Bioanalyzer purity*

The purity (Bioanalyzer) under non-reducing conditions was not affected by storage of 3 months at 5 and 25 °C. After storage of 3 months at 40 °C, the purity decreased in all formulations (lowest sample purity observed for F11 with ca. 80% and F12 with ca. 84%). There was no difference between samples stored in Barrel1 or Barrel3(ii) PFS (Figure 79). The purity (Bioanalyzer) under reducing conditions was not affected by storage of 3 months at 5 °C and 25 °C. After storage of 3 months at 40 °C, the purity decreased in all formulations. There was no difference between samples stored in Barrel1 or Barrel3(ii) PFS (Figure 80).

*iCE*

After storage for 3 months at 5 °C, main species content remained largely unchanged. After storage for 3 months at 25 °C < 40 °C, main species content decreased for all formulations. There was no difference in the iCE profiles between samples stored in Barrel1 or Barrel3(ii) PFS (Figure 81). After storage for 3 months at 5 °C, acidic species content remained largely unchanged. After storage for 3 months at 25 °C < 40 °C, acidic species content increased for all formulations. There was no difference in the iCE profiles between samples stored in Barrel1 or Barrel3(ii) PFS (Figure 82). After storage for 3 months at 5 °C, basic species content remained largely unchanged. After storage for 3 months at 25 °C < 40 °C, basic species content decreased for all formulations. There was no difference in the iCE profiles between samples stored in Barrel1 or Barrel3(ii) PFS (Figure 83).

*Conclusions*

- All formulations are within the target specifications of 150 mg/ml ± 15 mg/ml protein content and a target pH ± 0.1 pH units
- At T0, all formulations were free of visible particles and exhibited low levels of subvisible particles (MFI, except F5), turbidity (OD350, OD550) and soluble aggregates (SEC); the monomer content (SEC) was ca. 98%; and the main isoform (iCE3) content was ca. 50% for all formulations
- Viscosity was successfully reduced by addition of 150 mM of viscosity reducing agents. Viscosities were lowest for 150 mM arginine in combination with histidine- buffer at ca. 6-7 mPa*s (F2, F3, F4, F10, F13) and highest for F11 and F12 at 16 and 18 mPa*s (no arginine)

After storage for 3 months at 5 °C:

- All samples remained stable with no/little changes after storage at 5 °C; only in visual inspection F5 and F6 appeared slightly turbid

After storage for 3 months at 25 °C and 40 °C:

- pH, osmolality, protein content, non-silicone oil-like particle fractions (MFI) as well as dynamic viscosity remained largely unchanged after storage at 25 °C and 40 °C for all formulations
- All formulations were affected by 3 months of storage as follows: increase in OD350 and OD550 values (turbidity), gliding forces, silicone oil-like particle fractions (MFI), HMWS and LMWS content (SEC), and acidic species (iCE). Decrease in thermal/conformational stability (nanoDSF) and sample purity under reducing and non-reducing conditions (Bioanalyzer) at 40 °C storage, and loss of monomer content (SEC) at 25 °C < 40 °C

Impact of PFS barrel Barrel1 versus Barrel3(ii):

- Gliding forces increased slightly for samples in Barrel3(ii) PFS after storage at 25 °C compared to 5 °C (no increase of gliding forces observed for samples stored in Barrel1 PFS)
- For both PFS types the gliding forces (silicone oil droplet-like particle content) increased similarly after T3m of storage at 40 °C. NOTE: silicone oil droplet-like particle content was slightly increased in Barrel1 compared to Barrel3(ii) after T1m of storage at 40 °C and after rolling stress

Results - Stability 6 months

The results of the stability test after 6 months of storage for formulation F2, F7, F9, F10 and F13 are shown below.

*pH*

At T0, the pH of all samples was within the acceptance limits of target pH ± 0.1 pH units. The pH remained unchanged after 6 months of storage, irrespective of the storage condition (Table 79)

Table 68: pH before and after 6 months of storage

| Time point | pH | | | | | | |
|---|---|---|---|---|---|---|---|
| | F2-Barrel1 | F3-Barrel1 | F4-Barrel1 | F7-Barrel1 | F9-Barrel1 | F10-Barrel1 | F13-Barrel1 |
| Target | 6.0 | 5.5 | 6.5 | 6.0 | 6.0 | 6.0 | 6.0 |
| T0 | 6.01 | 5.48 | 6.49 | 6.04 | 6.04 | 6.03 | 6.02 |
| T6m_5C | 6.03 | 5.51 | 6.49 | 6.02 | 5.99 | 5.99 | 5.98 |
| T6m_25C | 5.98 | 5.51 | 6.50 | 5.99 | 5.98 | 6.00 | 6.01 |

*Dynamic viscosity*

Initial values for dynamic viscosity were < 15 mPa*s for all formulations. The most effective viscosity reducing agent was arginine (F2, F9, F10, F13), followed by proline (F7). Viscosity remained unchanged for up to T3m, but appeared reduced by 1.5-3 mPa*s after T6m (Table 80).

Table 69: dynamic viscosity before and after 6 months of storage

| Time point | Dynamic viscosity[mPa*s] at 20°C | | | | |
|---|---|---|---|---|---|
| | F2-Barrel1 | F7-Barrel1 | F9-Barrel1 | F10-Barrel1 | F13-Barrel1 |
| T0 | 6.7 | 10.4 | 9.3 | 7.1 | 6.4 |
| T3m_5C | 6.6 | 10.5 | 9.4 | 6.7 | 6.6 |
| T6m_5C | 4.0 | 6.9 | 6.2 | 4.7 | 4.4 |
| T3m_25C | 6.6 | 11.1 | 9.2 | 6.7 | 6.5 |
| T6m_25C | 5.3 | 8.2 | 6.7 | 5.0 | 4.9 |

*Thermal stability*

At T0, no impact of methionine or higher PS80 content was observed. Values remained unchanged for all formulations after 6 months of storage at 5 °C and 25 °C (Figure 84). Onset of aggregation remained unchanged after storage at 5 °C and 25 °C (Figure 85).

*Protein content*

At T0, the protein content of all samples was within the acceptance limits of 150 ± 15mg/ml. The protein content was unchanged after storage of 6 month at 5 °C and 25 °C (Table 81).

Table 70: Protein content before and after 6 months storage

| Time point | Protein content[mg/ml] A280-A320 | | | | | | |
|---|---|---|---|---|---|---|---|
| | F2-Barrel1 | F3-Barrel1 | F4-Barrel1 | F7-Barrel1 | F9-Barrel1 | F10-Barrel1 | F13-Barrel1 |
| T0 | 144 | 147 | 150 | 150 | 145 | 153 | 157 |
| T6m_5C | 144 | 154 | 155 | 157 | 157 | 151 | 148 |
| T6m_25C | 149 | 149 | 153 | 149 | 146 | 151 | 149 |

*Turbidity*

Initial OD350 values were comparable for all five formulations. No difference in the OD350 observed before and after storage for up to 6 months at 5 °C. OD350 values increased after storage at 25 °C, with no clear difference between the formulations (Figure 86). Initial OD550 values were comparable for most formulations (slightly lower for F7). No change upon storage for up to 6 months at 5 °C and 25 °C, all formulations revealed in OD550 values (Figure 87).

*Visual inspection*

At T0, all samples were practically free of visible particles and exhibited a yellowish coloration. After 6 months of storage at 5 °C, all formulations except F7 (DAC score 2) remained free of visible particles; F2, F9 and F13 appeared slightly turbid. After 6 months of storage at 25 °C, all formulations showed no or few visible particles; F2, F10 and F13 appeared slightly turbid (Table 82).

Table 71: Visual inspection before and after 6 months of storage

| Time point | Visual inspection DAC score examiner1/examiner2 | | | | |
|---|---|---|---|---|---|
| | F2-Barrel1 | F7-Barrel1 | F9-Barrel1 | F10-Barrel1 | F13-Barrel1 |
| T0 | 0/0 | 0/0 | 0/0 | 0/0 | 0/0 |
| T3m_5C | 1/0 | 0/0 | 0/0 | 0/0 | 0/0 |
| T6m_5C | 0/0 | 2/2 | 0/0 | 0/0 | 0/0 |
| T3m_25C | 0/0 | 0/0 | 0/0 | 1/1 | 1/0 |
| T6m_25C | 1/0 | 0/0 | 0/0 | 1/0 | 1/0 |

*MFI*

After 6 months of storage at 5 °C and 25 °C the particle levels ≥ 2 μm increased slightly, most pronounced for F7, F9 (5 °C) and F2 (25 °C). Particle levels of F10 and F13 remained similar to initial levels and thus lower compared to the other formulations (Figure 88). After 6 months of storage at 5 °C and 25 °C the levels of proteinaceous particles ≥ 5 μm increased compared to T0 and T3m. Trend more pronounced for F2, F7, and F9 compared to F10 and F13 (5 °C > 25 °C). Silicone oil droplet-like particle levels higher compared to T0/T3m, but no clear formulation differences (Figure 89). After 6 months of storage at 5 °C and 25 °C the levels of proteinaceous particles ≥ 5 μm increased compared to T0 and T3m. Trend more pronounced for F2, F7, and F9 compared to F10 and F13 (5 °C > 25 °C). Silicone oil droplet-like particle levels higher compared to T0/T3m, but no clear formulation differences (Figure 90). Similar trend for particles ≥ 25 μm than for ≥ 5 and 10 μm was observed. Slightly increased particle levels for F2 and F7 compared to F9, F10 and F13. Silicone oil droplet-like particle levels remained low and comparable to T0/T3m (Figure 91).

*SEC results*

The initial SEC profiles of all five formulations were comparable, with a monomer content of ca. 97.6%. The monomer content decreased marginally after storage for up to 6 months at 5 °C. Upon storage of 6 months at 25 °C, the monomer content of all formulations decreased to 95.7-95.3% with no clear difference between the formulations (Figure 92). Initial HMWS levels were comparable at ca. 1.0% for all five formulations. HMWS levels increased slightly after storage of 6 months at 5 °C (to ca. 1.2%).HMWS levels increased after storage of 6 months at 25 °C (most pronounced for F7 and F9: 1.7%; least pronounced for F10: 1.3%) (Figure 93). Initial LMWS levels were at ca. 1.5% for all formulations. LMWS levels increased slightly to ca. 1.7% upon storage for 6 months at 5 °C for all formulations. Upon storage for 6 months at 25 °C, LMWS levels increased for all formulations to ca. 2.9% (Figure 94).

*Bioanalyzer purity*

The purity (Bioanalyzer) under non-reducing conditions was overall not affected by storage at 5 and 25 °C for up to 6 months (Figure 95). The purity (Bioanalyzer) under reducing conditions was not affected by storage at 5 °C for up to 6 months. After 6 months of storage at 25 °C, the purity seemed slightly reduced for F2, F7, F9 and F10, not F13 (Figure 96).

*iCE*

After storage for 6 months at 5 °C, main species content remained largely unchanged. After storage for 6 months at 25 °C, main species content decreased for all formulations (no differences between formulations) (Figure 97). After storage for 6 months at 5 °C, acidic species content remained largely unchanged. After storage for 6 months at 25 °C, acidic species content increased for all formulations (Figure 98). After storage for 6 months at 5 °C, basic species content remained largely unchanged. After storage for 6 months at 25 °C, basic species content decreased for all formulations (Figure 99).

*ELISA*

Table 72. Binding ELISA

| Formulation code | T0 | Values @ 3M | | | Values @ 6M | |
|---|---|---|---|---|---|---|
| | | +40°C | +25°C | +5°C | +25°C | +5°C |
| Barrel1-F1 | 83 | 89 | 113 | 98 | | |
| Barrel1-F2 | 94 | 138 | 90 | 120 | 99 | 80 |
| Barrel1-F3 | 124 | 59 | 125 | 140 | 135 | 78 |
| Barrel1-F4 | 83 | 194 | 86 | 80 | 140 | 64 |
| Barrel1-F5 | 68 | 85 | 86 | 119 | | |
| Barrel1-F6 | 109 | 227 | 100 | 105 | | |
| Barrel1-F7 | 90 | 136 | 89 | 92 | 92 | 72 |
| Barrel1-F8 | 91 | 192 | 112 | 93 | | |
| Barrel1-F9 | 96 | 203 | 87 | 118 | 103 | 106 |
| Barrel1-F10 | 97 | 163 | 99 | 88 | 115 | 93 |
| Barrel1-F11 | 90 | 181 | 143 | 114 | | |
| Barrel1-F12 | 93 | 135 | 138 | 97 | | |
| Barrel1-F13 | 86 | 171 | 137 | 92 | 112 | 107 |
| Barrel3(ii)-F2 | 93 | 134 | 128 | 85 | | |
| Barrel3(ii)-F6 | 90 | 141 | NT | 92 | | |
| Barrel3(ii)-F9 | 78 | 154 | 161 | 103 | | |
| Barrel3(ii)-F10 | 89 | 163 | 116 | 108 | | |

Binding ELISA Table 83 showed that many formulations were out of specifications after 3M of incubation at 40°C.

*Conclusions*

All formulations are within the target specifications of 150 mg/ml ± 15 mg/ml protein content and a target pH ± 0.1 pH units

- At T0, all formulations were free of visible particles and exhibited low levels of subvisible particles (MFI, except F5), turbidity (OD350, OD550) and soluble aggregates (SEC); the monomer content (SEC) was ca. 98%; and the main isoform (iCE3) content was ca. 50% for all formulations
- Viscosity was successfully reduced by addition of 150 mM of viscosity reducing agents. Viscosities were lowest for 150 mM arginine in combination with histidine- buffer at ca. 6-7 mPa*s (F2, F10, F13)

After storage for 6 months at 5 °C:

All formulations remained relatively stable with only little changes observed:

- in visual inspection F2, F9, and F13 appeared slightly turbid, and F7 exhibited clearly visible particles (no changes for F10)
- Proteinaceous particles ≥ 5 μm formed particularly for F2 > F7 > F9, particle levels in F10 and F13 affected least
- Soluble aggregates increased from 1.0% to 1.3% for F7 and F9, and to 1.1% for F10, and F13
- LMWS slightly increased by 0.3% for all formulations
- Viscosity reduced between 2.1 - 3.5 mPa*s, affect lowest for F13, highest for F7

After storage for 6 months at 25 °C:

All formulations were clearly affected after storage at 25 °C; the following changes were observed:

- in visual inspection F2, F10, and F13 appeared slightly turbid, no change in content of visible particles for any of the formulations
- Proteinaceous particles ≥ 5 μm formed particularly for F7, particle levels in F10 and F13 affected least
- OD350 (not OD550) increased for all formulations similarly
- Soluble aggregates increased from 1.0% to 1.7% for F7 and F9, and to 1.3% for F10
- LMWS increased by 1.4% for all formulations
- Viscosity reduced between 1.5 - 2.6 mPa*s, affect lowest for F2/F13, highest for F9
- Reduction of main iCE species from 50% to 40% (no formulation differences), whilst acidic species increased (from 29% to 42%) and basic species decreased (from 21.5% to 18.5%)

**Example 6: Storage stability studies**

The molecular stability profile of Ab1 formulated bulk drug substance (150 mg/mL) was further investigated:

(a) when filled in 10 mL glass vials (target fill volume: 2.1 mL); and
(b) when filled in 2.25 mL glass pre-fillable syringe (PFS) system (target fill volume: 2.1 mL)

and stored under different temperature storage conditions.

Material and methods

This study was performed using:

(a) Development batches of Ab1 formulated BDS (150 mg/mL) with lot Ab1-Sample1 and Ab1-Sample2, manufactured at in-house.

- Lot Ab1-Sample1: formulated in 25 mM Histidine, 150 mM Sodium chloride, 0.36 mg/mL Polysorbate 80, pH 6.0
- Lot Ab1-Sample2: formulated in 25 mM Histidine, 150 mM L-Arginine hydrochloride, 0.36 mg/mL Polysorbate 80, pH 6.0.

The formulated bulk drug substance at target concentration of 150 mg/mL of Ab1 was filled into 10 mL glass vials (2.1 mL fill per vial). The required number of Ab1 formulated BDS vials were stored under multiple temperature conditions of -80 ± 20°C, -20 ± 5°C, +5 ± 3°C and +25 ± 2°C in order to assess its stability.

(b) Development batches of Ab1 drug product in PFS (PFS1) (300 mg/2 mL) with lot Ab1-Sample3 and Ab1-Sample4, manufactured in-house.

- Lot Ab1-Sample3: formulated in 25 mM Histidine, 150 mM Sodium chloride, 0.36 mg/mL Polysorbate 80, pH 6.0
- Lot Ab1-Sample4: formulated in 25 mM Histidine, 150 mM L-Arginine hydrochloride, 0.36 mg/mL Polysorbate 80, pH 6.0.

The formulated bulk drug substance at target conc. of 150 mg/mL of Ab1 was filled into PFS1. The required number of Ab1 PFS were stored under multiple temperature conditions of +5 ± 3°C, +25 ± 2°C and +40 ± 2°C in order to access its stability.

The container closure systems for formulated bulk drug substance and PFS are detailed in the Table 84 and Table 85, respectively.

Table 73: Container closure system details for formulated bulk drug substance

| Name | Description |
|---|---|
| Vial | 10 mL Type I glass vial |
| Stopper | 20 mm Injection Stoppers-Flurotec-coated |
| Overcap | 20 mm Flip off seal-Blue |

Table 74: Container closure system details for PFS

| Name | Description |
|---|---|
| Syringe barrel | Barrel1 |
| Plunger stopper | Stopper1 |

The key quality attributes to be monitored during this stability are listed down along with their justification in Table 86.

Table 75: Details of test to be performed and their justification

| Test | Justification for test |
|---|---|
| Visual Appearance | A significant change in the appearance of sample may indicate product degradation and/or microbial contamination leading to safety risk for the patients. Hence it is necessary to monitor appearance of solution. |
| pH | Changes in pH may be indicative of degradation of buffering agents. A significant variation from the target pH may lead to protein instability that ultimately possess a risk to the patient's safety. Hence it is shall be monitored. |
| Subvisible particles | It is Pharmacopeial requirement for parenteral drug products. Presence of higher sub-visible particles in parental solutions may lead to immunogenic responses. An increase in sub-visible particles in PFS may be indicative of protein aggregation and/or leaching of silicon oil from glass/stopper surfaces. Thus it shall be monitored during stability to confirm product quality. |
| Protein content (by A280) | The protein is required to be administered at a target conc. of 150 mg/mL. Any significant variation from its target conc. would not provide effective dose to patients. Hence it is important to measure A280 values. |
| Size variants by SE-HPLC | Changes in monomeric content of Ab1 is an indication of its degradation. The aggregates (higher size than monomer) or fragments (smaller size than monomer) may be detected by sensitive technique like SE-HPLC. Hence it shall be used during stability testing. |
| Charge variants by cIEF | Changes in content of charged variants of Ab1 is an indication of its degradation. The acidic and basic charged variants can be analyzed by sensitive technique like cIEF. Hence it shall be use during stability testing. |

(continued)

| Test | Justification for test |
|---|---|
| Size variants by cGE (reduced & non-reduced) | Changes in monomeric content of Ab1 is an indication of its degradation. The aggregates (higher size than monomer) or fragments (smaller size than monomer) may be detected by sensitive technique like CGE which can be used as an orthogonal technique to SE-HPLC. Hence it shall be use during stability testing. |
| Potency by Binding ELISA | An intact molecule would bind to the target receptor (OX40-Fc) to show its efficacy. Any significant change of such binding property would indicate its degradation. Hence it essential measure this parameter to monitor its potency. |
| Syringability (for PFS only) | Ab1 is intended to be delivered into sub-cutaneous layer of skin through a prefilled syringe system. The performance of syringe (i.e. Syringability) is judged based on two forces namely, the force required to initiate plunBarrel1 movement (break-loose force) and the force required to maintain the movement of plunBarrel1 (gliding force). Any significant change in these two forces indicate poor syringe performance leading to inaccuracy in dose volume and/or pain to patients during injection. Hence it essential monitor this parameter. |

Results stability of Ab1 formulated bulk drug substance

The results of the stability studies on Ab1 formulated bulk drug substance are reported in Table 87 to Table 94.

Table 76: Ab1 formulated BDS (150 mg/mL) lot Ab1-Sample1 under storage of -80 ± 20°C

| Stability Indicating Tests | Specifications | Time Interval (months) | | | |
|---|---|---|---|---|---|
| | | T0 | T1 | T3 | T6 |
| Visual Appearance | Report results (as clear or opalescent, colorless or yellowish or brownish, presence of particles or free of particles) | Slightly opalescent, slightly brownish, presence of dust like particles | Slightly opalescent, brownish, presence of particles | Slightly opalescent, slightly brownish, practically free of visual particulates | Slightly opalescent, slightly brownish, dust-liked material |
| pH | Value at T0 ± 0.2 (5.9 to 6.3) | 6.1 | 6.2 | 6.2 | 6.0 |
| Subvisible particles | Number of particles/ mL of size ≥ 2 µm (c) | 5775 | 4655 | 2857 | 605 |
| | Number of particles/ mL of size ≥ 5 µm (c) | 1215 | 350 | 265 | 215 |
| | Report results (number of particles/ mL of size ≥ 10 µm) | 250 | 30 | 40 | 120 |
| | Report results (number of particles/ mL of size ≥ 25 µm) | 0 | 0 | 5 | 5 |
| Protein content (by A280) | Value at T0 ± 10% (≥ 125.3 mg/mL to ≤ 153.2 mg/mL) | 139.3 mg/mL | 142.6 mg/mL | 143.1 mg/mL | 144.0 mg/mL |
| SE-HPLC | % Main monomer IgG peak ≥ 95% | 97.6 | 97.4 | 98.3 | 96.6 |

(continued)

| Stability Indicating Tests | Specifications | Time Interval (months) | | | |
|---|---|---|---|---|---|
| | | T0 | T1 | T3 | T6 |
| cGE (reduced) | Profile comparable to Ref. standard | Complies | Complies | Complies | Complies |
| | Heavy chain peak ≥ 60% | 64.1 | 64.6 | 64.0 | 64.0 |
| | Light chain peak ≥ 30% | 35.2 | 33.7 | 33.8 | 35.0 |
| | Total other peak ≤ 3% | 0.7 | 1.7 | 2.3 | 1.0 |
| cGE (non-reduced) | IgG peak ≥ 90% | 91.7 | 93.3 | 93.4 | 94.0 |
| | Assigned peaks ≤ 8% | 6.6 | 5.4 | 5.1 | 5.0 |
| | Total other peak ≤ 3% | 1.7 | 1.4 | 1.5 | 1.0 |
| cIEF by iCE3 | Profile comparable to Ref. standard | Complies | Complies | Complies | Complies |
| | Acidic peaks: ≥ 14% and ≤ 59% | 34.0 | 31.6 | 32.5 | 33.9 |
| | Main peak:≥ 33% and ≤ 54% | 44.0 | 46.9 | 46.4 | 44.2 |
| | Basic peaks: ≥ 7% and ≤ 35% | 22.0 | 21.4 | 21.0 | 21.9 |
| | pI main peak: ≥ 8.1 and ≤ 8.5 | 8.347 | 8.450 | 8.457 | 8.319 |
| Binding ELISA | ≥ 50% and ≤ 150% of Ref. standard EC50 | 79 | 88 | 103 | 86 |
| Osmolality (mOsm/kg) | Report results | 328 | 341 | 323 | 334 |

Table 77: Ab1 formulated BDS (150 mg/mL) lot Ab1-Sample1 under storage of -20 ± 5°C

| Stability Indicating Tests | Specifications | Time Interval (months) | | | |
|---|---|---|---|---|---|
| | | T0 | T1 | T3 | T6 |
| Visual Appearance | Report results (as clear or opalescent, colorless or yellowish or brownish, presence of particles or free of particles) | Slightly opalescent, slightly brownish, presence of dust like particles | Slightly opalescent, brownish, practically free of particles | Slightly opalescent, slightly brownish, practically free of visual particulates | Slightly opalescent, slightly brownish, dust-liked material |
| pH | Value at T0 ± 0.2 (5.9 to 6.3) | 6.1 | 6.2 | 6.1 | 6.0 |
| Subvisible particles | Number of particles/ mL of size ≥ 2 μm | 5775 | 3895 | 2977 | 3340 |
| | Number of particles/ mL of size ≥ 5 μm | 1215 | 645 | 447 | 530 |

(continued)

| Stability Indicating Tests | Specifications | Time Interval (months) | | | |
|---|---|---|---|---|---|
| | | T0 | T1 | T3 | T6 |
| | Report results (number of particles/ mL of size ≥ 10 µm) | 250 | 110 | 137 | 155 |
| | Report results (number of particles/ mL of size ≥ 25 µm) | 0 | 20 | 5 | 0 |
| Protein content (by A280) | Value at T0 ± 10% (≥ 125.3 mg/mL to ≤ 153.2 mg/mL) | 139.3 mg/mL | 142.1 mg/mL | 142.5 mg/mL | 144.5 mg/mL |
| SE-HPLC | % Main monomer IgG peak ≥ 95% | 97.6 | 97.3 | 97.7 | 95.3 |
| cGE (reduced) | Profile comparable to Ref. standard | Complies | Complies | Complies | Complies |
| | Heavy chain peak ≥ 60% | 64.1 | 64.7 | 64.1 | 63.0 |
| | Light chain peak ≥ 30% | 35.2 | 33.4 | 33.9 | 35.0 |
| | Total other peak ≤ 3% | 0.7 | 1.8 | 2.0 | 2.0 |
| cGE (non-reduced) | IgG peak ≥ 90% | 91.7 | 93.3 | 93.5 | 94.0 |
| | Assigned peaks ≤ 8% | 6.6 | 5.3 | 5.0 | 5.0 |
| | Total other peak ≤ 3% | 1.7 | 1.4 | 1.6 | 1.0 |
| cIEF by iCE3 | Profile comparable to Ref. standard | Complies | Complies | Complies | Complies |
| | Acidic peaks: ≥ 14% and ≤ 59% | 34.0 | 31.4 | 31.7 | 34.5 |
| | Main peak:≥ 33% and ≤ 54% | 44.0 | 46.4 | 46.8 | 43.9 |
| | Basic peaks: ≥ 7% and ≤ 35% | 22.0 | 22.2 | 21.4 | 21.6 |
| | pI main peak: ≥ 8.1 and ≤ 8.5 | 8.347 | 8.455 | 8.449 | 8.315 |
| Binding ELISA | ≥ 50% and ≤ 150% of Ref. standard EC50 | 79 | 92 | 81 | 106 |
| Osmolality (mOsm/kg) | Report results | 328 | 329 | 325 | 324 |

Table 78: Ab1 formulated BDS (150 mg/mL) lot Ab1-Sample1 under storage of +5 ± 3°C

| Stability Indicating Tests | Specifications | Time Interval (months) | | |
|---|---|---|---|---|
| | | T0 | T1 | T3 |
| Visual Appearance | Report results (as clear or opalescent, colorless or yellowish or brownish, presence of particles or free of particles) (a) | Slightly opalescent, slightly brownish, presence of dust like particles | Slightly opalescent, brownish, practically free of particles | Slightly opalescent, slightly brownish, practically free of visual particulates |
| pH | Value at T0 ± 0.2 (5.9 to 6.3) | 6.1 | 6.2 | 6.2 |
| Subvisible particles | Number of particles/ mL of size ≥ 2 μm | 5775 | 3020 | 2193 |
| | Number of particles/ mL of size ≥ 5 μm | 1215 | 570 | 443 |
| | Report results (number of particles/ mL of size ≥ 10 μm) | 250 | 85 | 193 |
| | Report results (number of particles/ mL of size ≥ 25 μm) | 0 | 0 | 20 |
| Protein content (by A280) | Value at T0 ± 10% (≥ 125.3 mg/mL to ≤ 153.2 mg/mL) | 139.3 mg/mL | 141.9 mg/mL | 143.0 mg/mL |
| SE-HPLC | % Main monomer IgG peak ≥ 95% | 97.6 | 97.2 | 97.8 |
| cGE (reduced) | Profile comparable to Ref. standard | Complies | Complies | Complies |
| | Heavy chain peak ≥ 60% | 64.1 | 64.3 | 61.6 |
| | Light chain peak ≥ 30% | 35.2 | 33.7 | 36.2 |
| | Total other peak ≤ 3% | 0.7 | 2.2 | 2.2 |
| cGE (non-reduced) | IgG peak ≥ 90% | 91.7 | 93.4 | 93.3 |
| | Assigned peaks ≤ 8% | 6.6 | 5.2 | 5.1 |
| | Total other peak ≤ 3% | 1.7 | 1.3 | 1.6 |
| cIEF by iCE3 | Profile comparable to Ref. standard | Complies | Complies | Complies |
| | Acidic peaks: ≥ 14% and ≤ 59% | 34.0 | 31.4 | 34.5 |
| | Main peak:≥ 33% and ≤ 54% | 44.0 | 46.6 | 45.0 |
| | Basic peaks: ≥ 7% and ≤ 35% | 22.0 | 21.9 | 20.5 |
| | pI main peak: ≥ 8.1 and ≤ 8.5 | 8.347 | 8.446 | 8.446 |
| Binding ELISA | ≥ 50% and ≤ 150% of Ref. standard EC50 | 79 | 86 | 103 |
| Osmolality (mOsm/kg) | Report results | 328 | 332 | 326 |

Table 79: Ab1 formulated BDS (150 mg/mL) lot Ab1-Sample1 under storage of +25 ± 2°C

| Stability Indicating Tests | Specifications | Time Interval (months) | |
|---|---|---|---|
| | | T0 | T1 |
| Visual Appearance | Report results (as clear or opalescent, colorless or yellowish or brownish, presence of particles or free of particles) | Slightly opalescent, slightly brownish, presence of dust like particles | Slightly opalescent, brownish, presence of particles |
| pH | Value at T0 ± 0.2 (5.9 to 6.3) | 6.1 | 6.2 |
| particles | Number of particles/ mL of size ≥ 2 μm | 5775 | 5035 |
| | Number of particles/ mL of size ≥ 5 μm | 1215 | 1060 |
| | Report results (number of particles/ mL of size ≥ 10 μm) | 250 | 185 |
| | Report results (number of particles/ mL of size ≥ 25 μm) | 0 | 0 |
| Protein content (by A280) | Value at T0 ± 10% (≥ 125.3 mg/mL to ≤ 153.2 mg/mL) | 139.3 mg/mL | 143.7 mg/mL |
| SE-HPLC | % Main monomer IgG peak ≥ 95% | 97.6 | 96.3 |
| cGE (reduced) | Profile comparable to Ref. standard | Complies | Complies |
| | Heavy chain peak ≥ 60% | 64.1 | 64.2 |
| | Light chain peak ≥ 30% | 35.2 | 33.8 |
| | Total other peak ≤ 3% | 0.7 | 1.9 |
| cGE (non-reduced) | IgG peak ≥ 90% | 91.7 | 92.6 |
| | Assigned peaks ≤ 8% | 6.6 | 5.6 |
| | Total other peak ≤ 3% | 1.7 | 1.8 |
| cIEF by iCE3 | Profile comparable to Ref. standard | Complies | Complies |
| | Acidic peaks: ≥ 14% and ≤ 59% | 34.0 | 33.0 |
| | Main peak:≥ 33% and ≤ 54% | 44.0 | 45.7 |
| | Basic peaks: ≥ 7% and ≤ 35% | 22.0 | 21.3 |
| | pI main peak: ≥ 8.1 and ≤ 8.5 | 8.347 | 8.459 |
| Binding ELISA | ≥ 50% and ≤ 150% of Ref. standard EC50 | 79 | 118 |
| Osmolality (mOsm/kg) | Report results | 328 | 331 |

Table 80: Ab1 formulated BDS (150 mg/mL) lot Ab1-Sample2 under storage of -80 ± 20°C

| Stability Indicating Tests | Specifications | Time Interval (months) | | | |
|---|---|---|---|---|---|
| | | T0 | T1 | T3 | T6 |
| Visual Appearance | Report results (as clear or opalescent, colorless or yellowish or brownish, presence of particles or free of particles) | Slightly opalescent, slightly brownish, presence of dust like particles | Slightly opalescent, brownish, practically free of particles | Slightly opalescent, slightly brownish, practically free of visual particulates | Slightly opalescent, slightly brownish, dust-liked material |
| pH | Value at T0 ± 0.2 (5.9 to 6.3) | 6.1 | 6.2 | 6.2 | 6.1 |
| Subvisible particles | Number of particles/ mL of size ≥ 2 μm | 7440 | 1305 | 1648 | 955 |
| | Number of particles/ mL of size ≥ 5 μm | 1640 | 290 | 123 | 400 |
| | Report results (number of particles/ mL of size ≥ 10 μm) | 245 | 110 | 12 | 125 |
| | Report results (number of particles/ mL of size ≥ 25 μm) | 0 | 5 | 0 | 10 |
| Protein content (by A280) | Value at T0 ± 10% (≥ 126.0 mg/mL to ≤ 154.0 mg/mL) | 140.0 mg/mL | 141.5 mg/mL | 144.0 mg/mL | 146.8 mg/mL |
| SE-HPLC | % Main monomer IgG peak ≥ 95% | 98.0 | 97.6 | 98.6 | 97.0 |
| cGE (reduced) | Profile comparable to Ref. standard | Complies | Complies | Complies | Complies |
| | Heavy chain peak ≥ 60% | 64.2 | 64.3 | 63.6 | 64.0 |
| | Light chain peak ≥ 30% | 35.0 | 33.5 | 33.7 | 35.0 |
| | Total other peak ≤ 3% | 0.8 | 2.1 | 2.7 | 1.0 |
| cGE (non-reduced) | IgG peak ≥ 90% | 91.8 | 93.3 | 93.6 | 94.0 |
| | Assigned peaks ≤ 8% | 6.4 | 5.5 | 5.1 | 5.0 |
| | Total other peak ≤ 3% | 1.7 | 1.3 | 1.4 | 1.0 |
| cIEF by iCE3 | Profile comparable to Ref. standard | Complies | Complies | Complies | Complies |
| | Acidic peaks: ≥ 14% and ≤ 59% | 33.7 | 30.7 | 31.9 | 33.6 |
| | Main peak:≥ 33% and ≤ 54% | 43.8 | 47.5 | 46.6 | 44.3 |
| | Basic peaks: ≥ 7% and ≤ 35% | 22.4 | 21.9 | 21.5 | 22.1 |
| | pI main peak: ≥ 8.1 and ≤ 8.5 | 8.344 | 8.452 | 8.452 | 8.315 |

(continued)

| Stability Indicating Tests | Specifications | Time Interval (months) | | | |
|---|---|---|---|---|---|
| | | T0 | T1 | T3 | T6 |
| Binding ELISA | ≥ 50% and ≤ 150% of Ref. standard EC50 | 91 | 80 | 86 | 109 |
| Osmolality (mOsm/kg) | Report results | 307 | 310 | 306 | 317 |

Table 81: Ab1 formulated BDS (150 mg/mL) lot Ab1-Sample2 under storage of -20 ± 5°C

| Stability Indicating Tests | Specifications | Time Interval (months) | | | |
|---|---|---|---|---|---|
| | | T0 | T1 | T3 | T6 |
| Visual Appearance | Report results (as clear or opalescent, colorless or yellowish or brownish, presence of particles or free of particles) (a) | Slightly opalescent, slightly brownish, presence of dust like particles | Slightly opalescent, brownish, practically free of particles | Slightly opalescent, slightly brownish, practically free of visual particulates | Slightly opalescent, slightly brownish, dust-liked material |
| pH | Value at T0 ± 0.2 (5.9 to 6.3) | 6.1 | 6.2 | 6.2 | 6.1 |
| Subvisible particles | Number of particles/ mL of size ≥ 2 μm | 7440 | 840 | 1838 | 700 |
| | Number of particles/ mL of size ≥ 5 μm | 1640 | 110 | 282 | 230 |
| | Report results (number of particles/ mL of size ≥ 10 μm) | 245 | 20 | 107 | 115 |
| | Report results (number of particles/ mL of size ≥ 25 μm) | 0 | 5 | 2 | 10 |
| Protein content (by A280) | Value at T0 ± 10% (≥ 126.0 mg/mL to ≤ 154.0 mg/mL) | 140.0 mg/mL | 144.0 mg/mL | 143.9 mg/mL | 145.3 mg/mL |
| SE-HPLC | % Main monomer IgG peak ≥ 95% | 98.0 | 97.6 | 98.5 | 96.8 |
| cGE (reduced) | Profile comparable to Ref. standard | Complies | Complies | Complies | Complies |
| | Heavy chain peak ≥ 60% | 64.2 | 64.2 | 64.1 | 64.0 |
| | Light chain peak ≥ 30% | 35.0 | 33.5 | 33.9 | 35.0 |
| | Total other peak ≤ 3% | 0.8 | 2.3 | 2.0 | 1.0 |
| cGE (non-reduced) | IgG peak ≥ 90% | 91.8 | 93.2 | 93.7 | 94.0 |
| | Assigned peaks ≤ 8% | 6.4 | 5.4 | 5.2 | 5.0 |
| | Total other peak ≤ 3% | 1.7 | 1.3 | 1.4 | 1.0 |

(continued)

| Stability Indicating Tests | Specifications | Time Interval (months) | | | |
|---|---|---|---|---|---|
| | | T0 | T1 | T3 | T6 |
| cIEF by iCE3 | Profile comparable to Ref. standard | Complies | Complies | Complies | Complies |
| | Acidic peaks: ≥ 14% and ≤ 59% | 33.7 | 29.4 | 32.4 | 33.9 |
| | Main peak:≥ 33% and ≤ 54% | 43.8 | 48.0 | 45.9 | 43.9 |
| | Basic peaks: ≥ 7% and ≤ 35% | 22.4 | 22.6 | 21.7 | 22.2 |
| | pI main peak: ≥ 8.1 and ≤ 8.5 | 8.344 | 8.461 | 8.454 | 8.321 |
| Binding ELISA | ≥ 50% and ≤ 150% of Ref. standard EC50 | 91 | 86 | 91 | 77 |
| Osmolality (mOsm/kg) | Report results | 307 | 312 | 308 | 313 |

Table 82: Ab1 formulated BDS (150 mg/mL) lot Ab1-Sample2 under storage of +5 ± 3°C

| Stability Indicating Tests | Specifications | Time Interval (months) | | |
|---|---|---|---|---|
| | | T0 | T1 | T3 |
| Visual Appearance | Report results (as clear or opalescent, colorless or yellowish or brownish, presence of particles or free of particles) | Slightly opalescent, slightly brownish, presence of dust like particles | Slightly opalescent, brownish, practically free of particles | Slightly opalescent, slightly brownish, practically free of visual particulates |
| pH | Value at T0 ± 0.2 (5.9 to 6.3) | 6.1 | 6.2 | 6.2 |
| Subvisible particles | Number of particles/ mL of size ≥ 2 μm | 7440 | 2530 | 528 |
| | Number of particles/ mL of size ≥ 5 μm | 1640 | 235 | 103 |
| | Report results (number of particles/ mL of size ≥ 10 μm) | 245 | 5 | 30 |
| | Report results (number of particles/ mL of size ≥ 25 μm) | 0 | 0 | 5 |
| Protein content (by A280) | Value at T0 ± 10% (≥ 126.0 mg/mL to ≤ 154.0 mg/mL) | 140.0 mg/mL | 143.8 mg/mL | 144.5 mg/mL |
| SE-HPLC | % Main monomer IgG peak ≥ 95% | 98.0 | 97.6 | 98.4 |

(continued)

| Stability Indicating Tests | Specifications | Time Interval (months) | | |
|---|---|---|---|---|
| | | T0 | T1 | T3 |
| cGE (reduced) | Profile comparable to Ref. standard | Complies | Complies | Complies |
| | Heavy chain peak ≥ 60% | 64.2 | 64.4 | 63.9 |
| | Light chain peak ≥ 30% | 35.0 | 33.6 | 33.9 |
| | Total other peak ≤ 3% | 0.8 | 2.0 | 2.1 |
| cGE (nonreduced) | IgG peak ≥ 90% | 91.8 | 93.1 | 93.5 |
| | Assigned peaks ≤ 8% | 6.4 | 5.6 | 5.2 |
| | Total other peak ≤ 3% | 1.7 | 1.4 | 1.4 |
| cIEF by iCE3 | Profile comparable to Ref. standard | Complies | Complies | Complies |
| | Acidic peaks: ≥ 14% and ≤ 59% | 33.7 | 30.6 | 33.0 |
| | Main peak:≥ 33% and ≤ 54% | 43.8 | 47.5 | 45.6 |
| | Basic peaks: ≥ 7% and ≤ 35% | 22.4 | 21.9 | 21.3 |
| | pl main peak: ≥ 8.1 and ≤ 8.5 | 8.344 | 8.459 | 8.445 |
| Binding ELISA | ≥ 50% and ≤ 150% of Ref. standard EC50 | 91 | 148 | 94 |
| Osmolality (mOsm/kg) | Report results | 307 | 308 | 307 |

Table 83: Ab1 formulated BDS (150 mg/mL) lot Ab1-Sample2 under storage of +25 ± 2°C

| Stability Indicating Tests | Specifications | Time Interval (months) | |
|---|---|---|---|
| | | T0 | T1 |
| Visual Appearance | Report results (as clear or opalescent, colorless or yellowish or brownish, presence of particles or free of particles) | Slightly opalescent, slightly brownish, presence of dust like particles | Slightly opalescent, brownish, practically free of particles |
| pH | Value at T0 ± 0.2 (5.9 to 6.3) | 6.1 | 6.2 |
| Subvisible particles | Number of particles/ mL of size ≥ 2 μm | 7440 | 1300 |
| | Number of particles/ mL of size ≥ 5 μm | 1640 | 170 |
| | Report results (number of particles/ mL of size ≥ 10 μm) | 245 | 20 |
| | Report results (number of particles/ mL of size ≥ 25 μm) | 0 | 0 |
| Protein content (by A280) | Value at T0 ± 10% (≥ 126.0 mg/mL to ≤ 154.0 mg/mL) | 140.0 mg/mL | 142.3 mg/mL |
| SE-HPLC | % Main monomer IgG peak ≥ 95% | 98.0 | 97.0 |

(continued)

| Stability Indicating Tests | Specifications | Time Interval (months) | |
|---|---|---|---|
| | | T0 | T1 |
| cGE (reduced) | Profile comparable to Ref. standard | Complies | Complies |
| | Heavy chain peak ≥ 60% | 64.2 | 64.3 |
| | Light chain peak ≥ 30% | 35.0 | 33.6 |
| | Total other peak ≤ 3% | 0.8 | 2.0 |
| cGE (nonreduced) | IgG peak ≥ 90% | 91.8 | 93.0 |
| | Assigned peaks ≤ 8% | 6.4 | 5.5 |
| | Total other peak ≤ 3% | 1.7 | 1.5 |
| cIEF by iCE3 | Profile comparable to Ref. standard | Complies | Complies |
| | Acidic peaks: ≥ 14% and ≤ 59% | 33.7 | 32.4 |
| | Main peak:≥ 33% and ≤ 54% | 43.8 | 45.9 |
| | Basic peaks: ≥ 7% and ≤ 35% | 22.4 | 21.7 |
| | pI main peak: ≥ 8.1 and ≤ 8.5 | 8.344 | 8.456 |
| Binding ELISA | ≥ 50% and ≤ 150% of Ref. standard EC50 | 91 | 144 |
| Osmolality (mOsm/kg) | Report results | 307 | 309 |

According the results above, Ab1 formulated BDS (150 mg/mL) in vial with lot Ab1-Sample1 and Ab1-Sample2 are within specification for all the stability indicating parameters. Both the lots met specifications in terms of purity (based on SE-HPLC and cGE), charge profile (based on iCE), protein content ($A_{280}$) and binding to target (Binding ELISA) when stored frozen up to 6 months at -80 ± 20° & -20 ± 5°C. The pH values were also within predefined specifications however the color of solution varied from slightly brownish to brownish with presence of random (dust like) particulates without any observable trend.

Under liquid state and at elevated storage conditions of +5 ± 3°C as well as +25 ± 2°C, both lots (Ab1-Sample1 and Ab1-Sample2) were within specifications for all the stability parameters for up to 3 months and 1 month of storage respectively.

Additionally, solution osmolality were measured at all time-points for both the lots; it was observed to be consistent across all storage conditions. The average value of osmolality for lot Ab1-Sample1 for all time-points and storage conditions was (329 ± 5) mOsm/kg whereas for lot Ab1-Sample2 it was (310 ± 3) mOsm/kg. Considering the method variation of ± 10 mOsm/kg, osmolality values for both these lots are almost the same for each given storage conditions.

Based on available data, it can be concluded that Ab1 formulated BDS (150 mg/mL) in vial having lot Ab1-Sample1 and Ab1-Sample2, are found to be:

- Stable for 6 months when stored frozen at -80 ± 20°C & -20 ± 5°C
- Stable for 3 months when stored refriBarrellated at +5 ± 3°C
- Stable for 1 month when stored at +25 ± 2°C

Results stability study pre-filled syringe drug product

The results of the stability studies on Ab1 formulated bulk drug substance are reported from Table 95 to Table 108.

Table 84: Ab1 DP in PFS1 (300 mg/2mL) lot Ab1-Sample3 under storage of +5 ± 3°C

| Stability Tests | Specifications | Time Interval (months) | | | |
|---|---|---|---|---|---|
| | | T0 | T1 | T3 | T6 |
| Visual Appear ance | Report results (as clear or opalescent, colorless or yellowish or brownish, presence of particles or free of particles) | Slightly opalescent, slightly brownish, practically free of particles | Slightly opalescent, slightly brownish, practically free of particles | Slightly opalescent, slightly yellowish, practically free of visual particles | Slightly opalescent, slightly brownish, practically free of particles |
| pH | Value at T0 ± 0.2 (5.9 to 6.3) | 6.1 | 6.2 | 6.3 | 6.0 |
| Sub-visible particles | Number of particles/ mL of size ≥ 2 μm | 10120 | 3575 | 4188 | 4265 |
| | Number of particles/ mL of size ≥ 5 μm | 2345 | 300 | 782 | 1370 |
| | Report results (number of particles/ mL of size ≥ 10 μm) | 460 | 50 | 130 | 310 |
| | Report results (number of particles/ mL of size ≥ 25 μm) | 5 | 0 | 5 | 5 |
| Protein content (by A280) | Value at T0 ± 10% (≥ 126.5 mg/mL to ≤ 154.6 mg/mL) | 140.5 mg/mL | 140.4 mg/mL | 140.7 mg/mL | 140.2 mg/mL |
| SE-HPLC | % Main monomer IgG peak ≥ 95% | 98.7 | 98.5 | 98.1 | 96.5 |
| cGE (reduced) | Profile comparable to Ref. standard | Complies | Complies | Complies | Complies |
| | Heavy chain peak ≥ 60% | 63.3 | 63.5 | 65.0 | 64.0 |
| | Light chain peak ≥ 30% | 35.2 | 35.5 | 35.0 | 35.0 |
| | Total other peak ≤ 3% | 1.6 | 1.0 | 1.0 | 1.0 |
| cGE (nonreduced) | IgG peak ≥ 90% | 93.2 | 93.6 | 94.0 | 93.0 |
| | Assigned peaks ≤ 8% | 5.2 | 5.1 | 4.0 | 5.0 |
| | Total other peak ≤ 3% | 1.6 | 1.4 | 1.0 | 1.0 |
| cIEF by iCE3 | Profile comparable to Ref. standard | Complies | Complies | Complies | Complies |
| | Acidic peaks: ≥ 14% and ≤ 59% | 33.0 | 34.7 | 35.0 | 34.7 |
| | Main peak:≥ 33% and ≤ 54% | 45.6 | 43.9 | 43.9 | 44.0 |
| | Basic peaks: ≥ 7% and ≤ 35% | 21.4 | 21.3 | 21.1 | 21.3 |
| | pI main peak: ≥ 8.1 and ≤ 8.5 | 8.367 | 8.294 | 8.308 | 8.314 |
| Binding ELISA | ≥ 50% and ≤ 150% of Ref. standard EC50 | 122 | 134 | 84 | 125 |

Table 85: Ab1 DP in PFS1 (300 mg/2mL) lot Ab1-Sample3 under storage of +5 ± 3°C

| Stability Tests | Specifications | Time Interval (months) | | |
|---|---|---|---|---|
| | | T9 | T12 | T18 |
| Visual Appearance | Report results (as clear or opalescent, colorless or yellowish or brownish, presence of particles or free of particles) | Slightly opalescent, slightly brownish, practically free of particles | Slightly opalescent, slightly brownish, practically free of particles | Slightly opalescent, slightly brownish, practically free of particles |
| pH | Value at T0 ± 0.2 (5.9 to 6.3) | 6.0 | 6.0 | 6.1 |
| Subvisible particles | Number of particles/ mL of size ≥ 2 μm | 3460 | 11540 | 4078 |
| | Number of particles/ mL of size ≥ 5 μm | 760 | 2185 | 880 |
| | Report results (number of particles/ mL of size ≥ 10 μm) | 150 | 360 | 206 |
| | Report results (number of particles/ mL of size ≥ 25 μm) | 0 | 0 | 10 |
| Protein content (by A280) | Value at T0 ± 10% (≥ 126.5 mg/mL to ≤ 154.6 mg/mL) | 142.9 mg/mL | 133.2 mg/mL (a) | 144.3 mg/mL |
| SE-HPLC | % Main monomer IgG peak ≥ 95% | 97.7 | 87.7 (b) | 97.0 |
| cGE (reduced) | Profile comparable to Ref. standard | Complies | Complies | Complies |
| | Heavy chain peak ≥ 60% | 63.9 | 64.1 | 63.5 |
| | Light chain peak ≥ 30% | 34.7 | 34.5 | 34.9 |
| | Total other peak ≤ 3% | 1.4 | 1.4 | 1.7 |
| cGE (nonreduced) | IgG peak ≥ 90% | 93.1 | 93.2 | 93.0 |
| | Assigned peaks ≤ 8% | 5.4 | 5.2 | 5.4 |
| | Total other peak ≤ 3% | 1.6 | 1.6 | 1.6 |
| cIEF by iCE3 | Profile comparable to Ref. standard | Complies | Complies | Complies |
| | Acidic peaks: ≥ 14% and ≤ 59% | 36.6 | 37.7 | 35.8 |
| | Main peak:≥ 33% and ≤ 54% | 42.4 | 41.8 | 43.6 |
| | Basic peaks: ≥ 7% and ≤ 35% | 21.0 | 20.6 | 20.6 |
| | pI main peak: ≥ 8.1 and ≤ 8.5 | 8.313 | 8.309 | 8.289 |
| Binding ELISA | ≥ 50% and ≤ 150% of Ref. standard EC50 | 75 | 131 | 112 |

Table 86: Ab1 DP in PFS1 (300 mg/2mL) lot Ab1-Sample3 under storage of +5 ± 3°C

| Stability Indicating Tests | Specifications | Time Interval (months) | | | |
|---|---|---|---|---|---|
| | | T0 | T1 | T3 | T6 |
| Syringeability | Report results | NA | NA | NA | Break-loose force: N/A Gliding force: 5.9 N ± 0.2 N |

(continued)

| Stability Indicating Tests | Specifications | Time Interval (months) | | | | | | |
|---|---|---|---|---|---|---|---|---|
| | | T0 | T1 | T3 | T6 | | | |
| Osmolality (mOsm/kg) | Report results | 325 | 327 | 322 | 339 | | | |
| Sub-visible particle count using MFI | Report results | NA | NA | NA | Size | Total | Protein-like | Silicon-like |
| | | | | | ≥ 2 μm | 13,300 | | |
| | | | | | ≥ 5 μm | 2,000 | 1,950 | 50 |
| | | | | | ≥ 10 μm | 370 | 350 | 20 |
| | | | | | ≥ 25 μm | 0 | 0 | 0 |

Table 87: Ab1 DP in PFS1 (300 mg/2mL) lot Ab1-Sample3 under storage of +5 ± 3°C

| Stability Indicating Tests | Specifications | Time Interval (months) | | |
|---|---|---|---|---|
| | | **T9** | **T12** | **T18** |
| Syringeability | Report results | NA | NA | NA |
| Osmolality (mOsm/kg) | Report results | 329 mOsm/kg | 333 mOsm/kg | 335 mOsm/kg |
| Sub-visible particle count using MFI | Report results | NA | NA | NA |

Table 88: Ab1 DP in PFS1 (300 mg/2mL) lot Ab1-Sample3 under storage of +25 ± 2°C

| Stability Tests | Specifications | Time Interval (months) | | | |
|---|---|---|---|---|---|
| | | T0 | T1 | T3 | T6 |
| Visual Appear ance | Report results (as clear or opalescent, colorless or yellowish or brownish, presence of particles or free of particles) | Slightly opalescent, slightly brownish, practically free of particles | Slightly opalescent, slightly brownish, practically free of particles | Slightly opalescent, slightly yellowish, practically free of visual particles | Slightly opalescent, yellowish, practically free of visual particles |
| pH | Value at T0 ± 0.2 (5.9 to 6.3) | 6.1 | 6.2 | 6.2 | 6.0 |
| Subvisibl e particles (b) | Number of particles/ mL of size ≥ 2 μm | 10120 | 6810 | 8605 | 50595 |
| | Number of particles/ mL of size ≥ 5 μm | 2345 | 1105 | 2092 | 2210 |
| | Report results (number of particles/ mL of size ≥ 10 μm) | 460 | 210 | 222 | 330 |
| | Report results (number of particles/ mL of size ≥ 25 μm) | 5 | 0 | 0 | 5 |
| Protein content (by A280) | Value at T0 ± 10% (≥ 126.5 mg/mL to ≤ 154.6 mg/mL) | 140.5 mg/mL | 144.5 mg/mL | 144.8 mg/mL | 144.6 mg/mL |

(continued)

| Stability Tests | Specifications | Time Interval (months) | | | |
|---|---|---|---|---|---|
| | | T0 | T1 | T3 | T6 |
| SE-HPLC | % Main monomer IgG peak ≥ 95% | 98.7 | 98.0 | 97.5 | 80.8 |
| cGE (reduced ) | Profile comparable to Ref. standard | Complies | Complies | Complies | Does not comply |
| | Heavy chain peak ≥ 60% | 63.3 | 63.1 | 64.0 | 61.0 |
| | Light chain peak ≥ 30% | 35.2 | 35.4 | 34.0 | 31.0 |
| | Total other peak ≤ 3% | 1.6 | 1.5 | 2.0 | 8.0 |
| cGE (nonreduced) | IgG peak ≥ 90% | 93.2 | 93.5 | 95.0 | 56.0 |
| | Assigned peaks ≤ 8% | 5.2 | 5.0 | 4.0 | 4.0 |
| | Total other peak ≤ 3% | 1.6 | 1.5 | 2.0 | 41.0 |
| cIEF by iCE3 | Profile comparable to Ref. standard | Complies | Complies | Complies | Does not comply |
| | Acidic peaks: ≥ 14% and ≤ 59% | 33.0 | 37.3 | 38.8 | 71.1 |
| | Main peak:≥ 33% and ≤ 54% | 45.6 | 42.1 | 41.4 | 18.0 |
| | Basic peaks: ≥ 7% and ≤ 35% | 21.4 | 21.3 | 19.8 | 10.9 |
| | pI main peak: ≥ 8.1 and ≤ 8.5 | 8.367 | 8.302 | 8.309 | 8.335 |
| Binding ELISA | ≥ 50% and ≤ 150% of Ref. standard EC50 | 122 | 81 | 89 | 140 |

Table 89: Ab1 DP in PFS1 (300 mg/2mL) lot Ab1-Sample3 under storage of +25 ± 2°C

| Stability Indicating Tests | Specifications | Time Interval (months) | | | | | | |
|---|---|---|---|---|---|---|---|---|
| | | T0 | T1 | T3 | T6 | | | |
| Syringeability | Report results | NA | NA | NA | Break-loose force: 7.5 N Gliding force: 12.9 N ± 2.8 N | | | |
| Osmolality mOsm/kg | Report results | 325 | 329 | 322 | 339 | | | |
| Sub-visible particle count using MFI | Report results | NA | NA | NA | Size | Total | Protein-like | Silicon-like |
| | | | | | ≥ 2 µm | 1,102,890 | | |
| | | | | | ≥ 5 µm | 35,450 | 32,870 | 2,570 |
| | | | | | ≥ 10 µm | 4,220 | 2,220 | 2,220 |
| | | | | | ≥ 25 µm | 0 | 0 | 0 |

Table 90: Ab1 DP in PFS1 (300 mg/2mL) lot Ab1-Sample3 under storage of +40 ± 2°C

| Stability Indicating Tests | Specifications | Time Interval (months) | |
|---|---|---|---|
| | | T0 | T1 |
| Visual Appearance | Report results (as clear or opalescent, colorless or yellowish or brownish, presence of particles or free of particles) | Slightly opalescent, slightly brownish, practically free of particles | Slightly opalescent, slightly brownish, practically free of particles |
| pH | Value at T0 ± 0.2 (5.9 to 6.3) | 6.1 | 6.2 |
| Subvisible particles | Number of particles/ mL of size ≥ 2 μm | 10120 | 2585 |
| | Number of particles/ mL of size ≥ 5 μm | 2345 | 300 |
| | Report results (number of particles/ mL of size ≥ 10 μm) (a) | 460 | 75 |
| | Report results (number of particles/ mL of size ≥ 25 μm) | 5 | 10 |
| Protein content (by A280) | Value at T0 ± 10% (≥ 126.5 mg/mL to ≤ 154.6 mg/mL) | 140.5 mg/mL | 139.0 mg/mL |
| SE-HPLC | % Main monomer IgG peak ≥ 95% | 98.7 | 97.0 |
| cGE (reduced) | Profile comparable to Ref. standard | Complies | Complies |
| | Heavy chain peak ≥ 60% | 63.3 | 63.5 |
| | Light chain peak ≥ 30% | 35.2 | 34.1 |
| | Total other peak ≤ 3% | 1.6 | 2.5 |
| | IgG peak ≥ 90% | 93.2 | 81.9 |
| cGE (nonreduced) | Assigned peaks ≤ 8% | 5.2 | 5.7 |
| | Total other peak ≤ 3% | 1.6 | 2.0 |
| cIEF by iCE3 | Profile comparable to Ref. standard | Complies | Does not comply |
| | Acidic peaks: ≥ 14% and ≤ 59% | 33.0 | 47.1 |
| | Main peak:≥ 33% and ≤ 54% | 45.6 | 34.1 |
| | Basic peaks: ≥ 7% and ≤ 35% | 21.4 | 18.9 |
| | pI main peak: ≥ 8.1 and ≤ 8.5 | 8.367 | 8.309 |
| Binding ELISA | ≥ 50% and ≤ 150% of Ref. standard EC50 | 122 | 127 |
| Osmolarity | Report results | 325 mOsm/kg | 327 mOsm/kg |

Table 91: Ab1 DP in PFS1 (300 mg/2mL) lot Ab1-Sample4 under storage of +5 ± 3°C

| Stability Tests | Specifications | Time Interval (months) | | | |
|---|---|---|---|---|---|
| | | T0 | T1 | T3 | T6 |
| Visual Appear ance | Report results (as clear or opalescent, colorless or yellowish or brownish, presence of particles or free of particles) | Slightly opalescent, slightly brownish, practically free of particles | Slightly opalescent, slightly brownish, presence of particles | Slightly opalescent, slightly yellowish, practically free of visual particles | Slightly opalescent, slightly brownish, practically free of particles |

(continued)

| Stability Tests | Specifications | Time Interval (months) | | | |
|---|---|---|---|---|---|
| | | T0 | T1 | T3 | T6 |
| pH | Value at T0 ± 0.2 (5.9 to 6.3) | 6.1 | 6.2 | 6.2 | 6.1 |
| Sub-visible particles | Number of particles/ mL of size ≥ 2 μm | 8135 | 6560 | 5735 | 765 |
| | Number of particles/ mL of size ≥ 5 μm | 1545 | 1075 | 1592 | 340 |
| | Report results (number of particles/ mL of size ≥ 10 μm) | 20 | 100 | 278 | 75 |
| | Report results (number of particles/ mL of size ≥ 25 μm) | 0 | 10 | 12 | 5 |
| Protein content (by A280) | Value at T0 ± 10% (≥ 129.8 mg/mL to ≤ 158.7 mg/mL) | 144.3 mg/mL | 144.9 mg/mL | 143.7 mg/mL | 144.7 mg/mL |
| SE-HPLC | % Main monomer IgG peak ≥ 95% | 98.9 | 98.8 | 98.5 | 97.0 |
| cGE (reduced) | Profile comparable to Ref. standard | Complies | Complies | Complies | Complies |
| | Heavy chain peak ≥ 60% | 62.9 | 63.5 | 65.0 | 64.0 |
| | Light chain peak ≥ 30% | 35.3 | 35.4 | 34.0 | 35.0 |
| | Total other peak ≤ 3% | 1.9 | 1.0 | 1.0 | 1.0 |
| cGE (nonreduced) | IgG peak ≥ 90% | 92.5 | 93.4 | 94.0 | 93.0 |
| | Assigned peaks ≤ 8% | 5.4 | 5.2 | 5.0 | 5.0 |
| | Total other peak ≤ 3% | 2.0 | 1.4 | 1.0 | 2.0 |
| cIEF by iCE3 | Profile comparable to Ref. standard | Complies | Complies | Complies | Complies |
| | Acidic peaks: ≥ 14% and ≤ 59% | 33.0 | 34.0 | 33.5 | 34.1 |
| | Main peak:≥ 33% and ≤ 54% | 45.4 | 44.4 | 45.0 | 44.1 |
| | Basic peaks: ≥ 7% and ≤ 35% | 21.7 | 21.7 | 21.5 | 21.8 |
| | pI main peak: ≥ 8.1 and ≤ 8.5 | 8.356 | 8.306 | 8.306 | 8.316 |
| Binding ELISA | ≥ 50% and ≤ 150% of Ref. standard EC50 | 87 | 130 | 85 | 105 |

Table 92: Ab1 DP in PFS1 (300 mg/2mL) lot Ab1-Sample4 under storage of +5 ± 3°C

| **Stability Indicating Tests** | **Specifications** | Time Interval (months) | | |
|---|---|---|---|---|
| | | T9 | T12 | T18 |
| Visual Appearance | Report results (as clear or opalescent, colorless or yellowish or brownish, presence of particles or free of particles) | Clear, slightly brownish, practically free of particles | Slightly opalescent, slightly brownish, practically free of particles | Slightly opalescent, slightly brownish, practically free of particles |
| pH | Value at T0 ± 0.2 (5.9 to 6.3) | 6.0 | 6.1 | 6.2 |
| Subvisible particles (c) | Number of particles/ mL of size ≥ 2 μm | 2755 | 13580 | 4212 |
| | Number of particles/ mL of size ≥ 5 μm | 940 | 2650 | 1250 |
| | Report results (number of particles/ mL of size ≥ 10 μm) | 300 | 410 | 284 |
| | Report results (number of particles/ mL of size ≥ 25 μm) | 5 | 5 | 20 |
| Protein content (by A280) | Value at T0 ± 10% (≥ 129.8 mg/mL to ≤ 158.7 mg/mL) | 135.5 mg/mL | 124.7 mg/mL (a) | 141.9 mg/mL |
| SE-HPLC | % Main monomer IgG peak ≥ 95% | 98.2 | 89.6 (b) | 97.7 |
| cGE (reduced) | Profile comparable to Ref. standard | Complies | Complies | Complies |
| | Heavy chain peak ≥ 60% | 63.9 | 63.6 | 63.6 |
| | Light chain peak ≥ 30% | 34.7 | 35.1 | 34.6 |
| | Total other peak ≤ 3% | 1.3 | 1.4 | 1.9 |
| cGE (non-reduced) | IgG peak ≥ 90% | 93.4 | 94.0 | 92.7 |
| | Assigned peaks ≤ 8% | 5.1 | 4.7 | 5.5 |
| | Total other peak ≤ 3% | 1.4 | 1.3 | 1.9 |
| cIEF by iCE3 | Profile comparable to Ref. standard | Complies | Complies | Complies |
| | Acidic peaks: ≥ 14% and ≤ 59% | 36.3 | 36.0 | 34.0 |
| | Main peak:≥ 33% and ≤ 54% | 42.2 | 42.5 | 44.7 |
| | Basic peaks: ≥ 7% and ≤ 35% | 21.5 | 21.5 | 21.3 |
| | pI main peak: ≥ 8.1 and ≤ 8.5 | 8.310 | 8.308 | 8.292 |
| Binding ELISA | ≥ 50% and ≤ 150% of Ref. standard EC50 | 113 | 129 | 107 |

Table 93: Ab1 DP in PFS1 (300 mg/2mL) lot Ab1-Sample4 under storage of +5 ± 3°C

| Stability Indicating Tests | Specifications | Time Interval (months) | | | |
|---|---|---|---|---|---|
| | | T0 | T1 | T3 | T6 |
| Syringeability | Report results | NA | NA | NA | Break-loose force:7.5 N Gliding force: 4.1 N ± 0.2 N |

(continued)

| Stability Indicating Tests | Specifications | Time Interval (months) | | | | | | |
|---|---|---|---|---|---|---|---|---|
| | | T0 | T1 | T3 | T6 | | | |
| Osmolality mOsm/kg | Report results (c) | 308 | 318 | 308 | 326 | | | |
| Sub-visible particle count using MFI | Report results | NA | NA | NA | Size | Total | Protein-like | Silicon-like |
| | | | | | ≥ 2 µm | 255,520 | | |
| | | | | | ≥ 5 µm | 6,350 | 6,270 | 70 |
| | | | | | ≥ 10 µm | 450 | 370 | 70 |
| | | | | | ≥ 25 µm | 50 | 50 | 0 |

Table 94: Ab1 DP in PFS (300 mg/2mL) lot Ab1-Sample4 under storage of +5 ± 3°C

| Stability Indicating Tests | Specifications | Time Interval (months) | | |
|---|---|---|---|---|
| | | T0 | T1 | T3 |
| Syringeability | Report results | NA | NA | NA |
| Osmolality mOsm/kg | Report results (c) | 305 mOsm/kg | 317 mOsm/kg | 320 mOsm/kg |
| Sub-visible particle count using MFI | Report results | NA | NA | NA |

Table 95: Ab1 DP in PFS1 (300 mg/2mL) lot Ab1-Sample4 under storage of +25 ± 2°C

| Stability Indicating Tests | Specifications | Time Interval (months) | | | |
|---|---|---|---|---|---|
| | | T0 | T1 | T3 | T6 (b) |
| Visual Appearance | Report results (as clear or opalescent, colorless or yellowish or brownish, presence of particles or free of particles) | Slightly opalescent, slightly brownish, practically free of particles | Slightly opalescent, slightly brownish, presence of particles | Slightly opalescent, slightly yellowish, practically free of visual particles | Slightly opalescent, slightly brownish, practically free of particles |
| pH | Value at T0 ± 0.2 (5.9 to 6.3) | 6.1 | 6.2 | 6.3 | 6.2 |
| Subvisible particles | Number of particles/ mL of size ≥ 2 µm | 8135 | 745 | 1692 | 9875 |
| | Number of particles/ mL of size ≥ 5 µm | 1545 | 45 | 398 | 3155 |
| | Report results (number of particles/ mL of size ≥ 10 µm) | 20 | 5 | 50 | 1305 |
| | Report results (number of particles/ mL of size ≥ 25 µm) | 0 | 0 | 2 | 150 |
| Protein content (by A280) | Value at T0 ± 10% (≥ 129.8 mg/mL to ≤ 158.7 mg/mL) | 144.3 mg/mL | 143.3 mg/mL | 144.4 mg/mL | 146.2 mg/mL |

(continued)

| Stability Indicating Tests | Specifications | Time Interval (months) | | | |
|---|---|---|---|---|---|
| | | T0 | T1 | T3 | T6 (b) |
| SE-HPLC | % Main monomer IgG peak ≥ 95% | 98.9 | 98.6 | 98.1 | 84.9 |
| cGE (reduced) | Profile comparable to Ref. standard | Complies | Complies | Complies | Does not comply |
| | Heavy chain peak ≥ 60% | 62.9 | 63.5 | 65.0 | 62.0 |
| | Light chain peak ≥ 30% | 35.3 | 35.3 | 34.0 | 30.0 |
| | Total other peak ≤ 3% | 1.9 | 1.2 | 1.0 | 9.0 |
| cGE (non-reduced) | IgG peak ≥ 90% | 92.5 | 93.5 | 94.0 | 54.0 |
| | Assigned peaks ≤ 8% | 5.4 | 5.1 | 5.0 | 4.0 |
| | Total other peak ≤ 3% | 2.0 | 1.4 | 1.0 | 42.0 |
| cIEF by iCE3 | Profile comparable to Ref. standard | Complies | Complies | Complies | Does not comply |
| | Acidic peaks: ≥ 14% and ≤ 59% | 33.0 | 35.3 | 39.9 | 69.4 |
| | Main peak:≥ 33% and ≤ 54% | 45.4 | 43.4 | 39.3 | 19.7 |
| | Basic peaks: ≥ 7% and ≤ 35% | 21.7 | 21.3 | 20.8 | 10.9 |
| | pI main peak: ≥ 8.1 and ≤ 8.5 | 8.356 | 8.316 | 8.310 | 8.334 |
| Binding ELISA | ≥ 50% and ≤ 150% of Ref. standard EC50 | 87 | 68 | 86 | 164 |

Table 96: Ab1 DP in PFS1 (300 mg/2mL) lot Ab1-Sample4 under storage of +25 ± 2°C

| Stability Indicating Tests | Specification s | Time Interval (months) | | | | | | |
|---|---|---|---|---|---|---|---|---|
| | | T0 | T1 | T3 | T6 | | | |
| Syringeabilit y | Report results (a) | NA | NA | NA | Break-loose force: 5.9 N Gliding force: 5.3 N ± 0.5 N | | | |
| Osmolality mOsm/kg | Report results (d) | 30 8 | 30 8 | 30 6 | 327 | | | |
| Sub-visible particle count using MFI | Report results (d) | NA | NA | NA | Size | Total | Protein-like | Silicon-like |
| | | | | | ≥ 2 μm | 215,100 | | |
| | | | | | ≥ 5 μm | 70,590 | 69,270 | 1,320 |
| | | | | | ≥ 10 μm | 23,200 | 21,950 | 1,250 |
| | | | | | ≥ 25 μm | 2,070 | 1,950 | 120 |

Table 97: Ab1 DP in PFS1 (300 mg/2mL) lot Ab1-Sample4 under storage of +40 ± 2°C

| Stability Indicating Tests | Specifications | Time Interval (months) | |
|---|---|---|---|
| | | T0 | T1 |
| Visual Appearance | Report results (as clear or opalescent, colorless or yellowish or brownish, presence of particles or free of particles) | Slightly opalescent, slightly brownish, practically free of particles | Slightly opalescent, slightly brownish, practically free of particles |
| pH | Value at T0 ± 0.2 (5.9 to 6.3) | 6.1 | 6.2 |
| Subvisible particles | Number of particles/ mL of size ≥ 2 μm | 8135 | 1625 |
| | Number of particles/ mL of size ≥ 5 μm | 1545 | 475 |
| | Report results (number of particles/ mL of size ≥ 10 μm) | 20 | 95 |
| | Report results (number of particles/ mL of size ≥ 25 μm) | 0 | 0 |
| Protein content (by A280) | Value at T0 ± 10% (≥ 129.8 mg/mL to ≤ 158.7 mg/mL) | 144.3 mg/mL | 145.3 mg/mL |
| SE-HPLC | % Main monomer IgG peak ≥ 95% | 98.9 | 97.7 |
| cGE (reduced) | Profile comparable to Ref. standard | Complies | Complies |
| | Heavy chain peak ≥ 60% | 62.9 | 62.8 |
| | Light chain peak ≥ 30% | 35.3 | 34.4 |
| | Total other peak ≤ 3% | 1.9 | 2.8 |
| cGE (non-reduced) | IgG peak ≥ 90% | 92.5 | 80.8 |
| | Assigned peaks ≤ 8% | 5.4 | 6.2 |
| | Total other peak ≤ 3% | 2.0 | 2.1 |
| cIEF by iCE3 | Profile comparable to Ref. standard | Complies | Does not comply |
| | Acidic peaks: ≥ 14% and ≤ 59% | 33.0 | 46.4 |
| | Main peak:≥ 33% and ≤ 54% | 45.4 | 34.8 |
| | Basic peaks: ≥ 7% and ≤ 35% | 21.7 | 18.7 |
| | pI main peak: ≥ 8.1 and ≤ 8.5 | 8.356 | 8.309 |
| Binding ELISA | ≥ 50% and ≤ 150% of Ref. standard EC50 | 87 | 124 |
| Osmolality | Report results | 308 mOsm/kg | 309 mOsm/kg |

According to the results reported above, Ab1 drug product in PFS (300 mg/2 mL) with lot Ab1-Sample3 and Ab1-Sample 4 are within specification for all the stability indicating parameters when stored up to 18 months at +5 ± 3 °C. Both the lots met specifications in terms of purity (based on SE-HPLC and cGE), charge profile (based on iCE), protein content ($A_{280}$) and binding to target (Binding ELISA). The pH values were also within predefined specifications however the color of solution varied from slightly yellowish to slightly brownish with presence of random (dust like) particulates at some time points without any observable trend.

Under accelerated storage conditions of +25 ± 2 °C, both the samples were found to be stable for up to 3 months. Due to incorrect PFS storage at +40 ± 2 °C between 3 and 6 months instead of intended storage condition of +25 ± 2 °C, both lots were OOS for SE-HPLC, cGE (non-reduced and reduced) and cIEF (by iCE3) at 6 months. Under regular stress condition of +40 ± 2 °C for 1 month, both lots did not met predefined stability specifications for cGE (non-reduced) and cIEF (by iCE3) tests.

Additionally, solution osmolality were measured at all time-points for both lots; it was observed to be consistent across all storage conditions. The average value of osmolality for Ab1-Sample3 for all time-points and storage conditions was 329 ± 6 mOsm/kg whereas for Ab1-Sample4 it was 313 ± 8 mOsm/kg. Considering the method variation of ± 10 mOsm/kg, osmolality values for both these lots are comparable for each storage conditions.

As a part of syringeability testing, the break-loose force and gliding force were measured for 6M samples stored at +5 ± 3 °C and +25 ± 2 °C (3M actual storage). All PFS showed low break loose forces (5.9 N to 7.5 N), except for Ab1-Sample3 - 5 °C sample which did not show a break-loose point. The average gliding force for all PFS sample was also low (4.1 N to 5.9 N), except for Ab1-Sample3 - 25 °C sample which had a higher gliding force of 12.9 N. These values of gliding force are in close agreement with theoretical value of 5.71 N force needed using Hagen-Poiseuille Equation for delivering Ab1 (150 mg/mL) solution with a dynamic viscosity of 0.02 Pa.s (20 centipoise) through a standard 27G needle.

The sub-visible particle analysis by MFI revealed that of total particles having size ≥ 5 μm about 97.5 to 98.7% of that were of protein-like for all 6M samples stored at +5 ± 3°C and +25 ± 2°C except for lot# Ab1-Sample3 +25 °C sample for which the fraction of protein-like was 92.7% which could have been generated due to excessive heat stress during storage. Ab1-Sample4 was also submitted to excessive heat stress and doesn't appear to show the same result. However, further testing beyond n=1 would be necessary in order to obtain conclusive results. Also, more orthogonal particle analysis methods may contribute to a more detailed and reliable characterization of particle size distribution beyond the particle size range covered by MFI.

Based on available data, it can be concluded that Ab1 DP in PFS (300 mg/2 mL), are found to be:

1) Stable for 18 months when stored refrigerated at +5 ± 3°C
2) Stable for 3 months when stored +25 ± 2°C
3) Not stable for 1 month when stored at +40 ± 2°C

## Claims

1. A stable liquid pharmaceutical formulation comprising an anti-OX40 antagonist antibody or fragment thereof, a buffer and one or more stabilizing or tonicity agents.

2. The pharmaceutical formulation of claim 1, wherein said anti-OX40 antagonist antibody or fragment thereof is present within said pharmaceutical formulation at a concentration between about 100 mg/mL and about 200 mg/mL.

3. The pharmaceutical formulation of any one of the preceeding claims, wherein said pharmaceutical formulation has a pH between about 5.0 and about 7.0.

4. The pharmaceutical formulation of any one of the preceeding claims, wherein said pharmaceutical formulation has a pH of about 6.0 ± 0.5.

5. The pharmaceutical formulation of claim 1, wherein said buffer is selected from the group comprising acetate, histidine-HCl, citrate and phosphate, wherein said buffer is present within said pharmaceutical formulation at concentration between about 1 mM and about 50 mM.

6. The pharmaceutical formulation of claim 5, wherein said buffer is histidine-HCl or citrate, present within said pharmaceutical formulation at a concentration of about 25 mM.

7. The pharmaceutical formulation of claim 1, wherein said stabilizing or tonicity agent is selected from the group comprising sodium chloride, calcium chloride, mannitol, sucrose, polyethylene glycol 400, sorbitol, trehalose, EDTA, glycine, arginine, arginine-HCl, glutamine, glutathione, methionine, cysteine, proline, lysine, lysine-HCl, Polysorbate 20, Polysorbate 40, Polysorbate 80, Poloxamer, Poloxamer 188, Poloxamer 407.

8. The pharmaceutical formulation of claim 7, wherein said stabilizing or tonicity agent is sodium chloride present within said formulation at a concentration between about 100 mM and about 200 mM; and/or arginine-HCl present with said formulation at a concentration between about 100 mM and about 200 mM; and/or proline present within said formulation at a concentration between about 100 mM and about 200 mM; and/or lysine-HCl present within said formulation at a concentration between about 100 mM and about 200 mM; and/or mannitol present within said formulation at a concentration between about 0.5% and about 5%; and/or sucrose present within said formulation at a concentration between about 5% and about 10%; and/or methionine present within said formulation at a concentration between about 1 mM and about 20 mM, and/or Polysorbate 80 present within said formulation at a concentration between about 0.01% and about 0.1%.

**9.** The pharmaceutical formulation of any one of claims 1 to 8, wherein said pharmaceutical formulation comprises said anti-OX40 antagonist antibody or fragment thereof present within said pharmaceutical formulation at a concentration of about 150 mg/mL, an histidine-HCl buffer present within said pharmaceutical formulation at a concentration of about 25 mM, arginine-HCl or sodium chloride present within said pharmaceutical formulation at a concentration of about 150 mM, optionally methionine present within said pharmaceutical formulation at a concentration of about 10 mM, and Polysorbate 80 present within said pharmaceutical formulation at a concentration between about 0.03% and about 0.06%, and wherein said pharmaceutical formulation has pH between about 5 and about 7.

**10.** The pharmaceutical formulation of any one of claims 1 to 8, wherein said pharmaceutical formulation comprises said anti-OX40 antagonist antibody or fragment thereof present within said pharmaceutical formulation at a concentration of about 150 mg/mL, histidine-HCl buffer present within said pharmaceutical formulation at a concentration of about 25 mM, mannitol present within said pharmaceutical formulation at a concentration between about 1% and about 5% or sucrose present within said pharmaceutical formulation at a concentration of about 8.5%, optionally proline or lysine-HCl present within said pharmaceutical formulation at a concentration of about 150 mM, or arginine-HCl present within said pharmaceutical formulation at a concentration of about 50 mM, and Polysorbate 80 present within said pharmaceutical formulation at a concentration of about 0.036%, and wherein said pharmaceutical formulation has pH of about 6.

**11.** The pharmaceutical formulation of any one of claims 1 to 8, wherein said pharmaceutical formulation comprises said anti-OX40 antagonist antibody or fragment thereof present within said pharmaceutical formulation at a concentration of about 150 mg/mL, citrate buffer present within said pharmaceutical formulation at a concentration of about 25 mM, sodium chloride or arginine-HCl present within said pharmaceutical formulation at a concentration of about 150 mM, and Polysorbate 80 present within said pharmaceutical formulation at a concentration of about 0.036%, and wherein said pharmaceutical formulation has pH of about 6.

**12.** The pharmaceutical formulation of any one of claims 1 to 8, wherein said pharmaceutical formulation comprises said anti-OX40 antagonist antibody or fragment thereof present within said pharmaceutical formulation at a concentration of about 150 mg/mL, an histidine-HCl buffer present within said pharmaceutical formulation at a concentration of about 25 mM, proline or sodium chloride present within said pharmaceutical formulation at a concentration of about 150 mM, or arginine-HCl present within said pharmaceutical formulation at a concentration between about 50 mM and about 150 mM, optionally methionine present within said pharmaceutical formulation at a concentration of about 10 mM or mannitol present within said pharmaceutical formulation at a concentration between about 2% and about 3.5%, and Polysorbate 80 present within said pharmaceutical formulation at a concentration between about 0.03% and about 0.06%, wherein said pharmaceutical formulation has pH between about 5 and about 7, and wherein said pharmaceutical formulation is stable for at least about 6 months at a temperature of about 25°C.

**13.** The pharmaceutical formulation of any one of claims 1 to 8, wherein said pharmaceutical formulation comprises said anti-OX40 antagonist antibody or fragment thereof present within said pharmaceutical formulation at a concentration of about 150 mg/mL, histidine-HCl buffer present within said pharmaceutical formulation at a concentration of about 25 mM, arginine-HCl or sodium chloride present within said pharmaceutical formulation at a concentration of about 150 mM and Polysorbate 80 present within said pharmaceutical formulation at a concentration of about 0.036%, wherein said pharmaceutical formulation has pH of about 6 and wherein said pharmaceutical formulation is stable for at least about 18 months at a temperature of about 5°C.

**14.** The pharmaceutical formulation of any one of claims 1 to 13, wherein said pharmaceutical formulation is comprised within a prefilled syringe which comprises a glass barrel **characterized by** a staked needle having gauge between about 25G and about 30G and a siliconization level between about 0.2 mg and about 1 mg per prefilled syringe, and a rubber stopper.

**15.** The pharmaceutical formulation of claim 14, wherein said pharmaceutical formulation is comprised within a prefilled syringe which comprises a glass barrel **characterized by** a staked needle having gauge of about 27G and a siliconization level between about 0.3 mg and about 0.7 mg per prefilled syringe, and a halobutyl stopper.

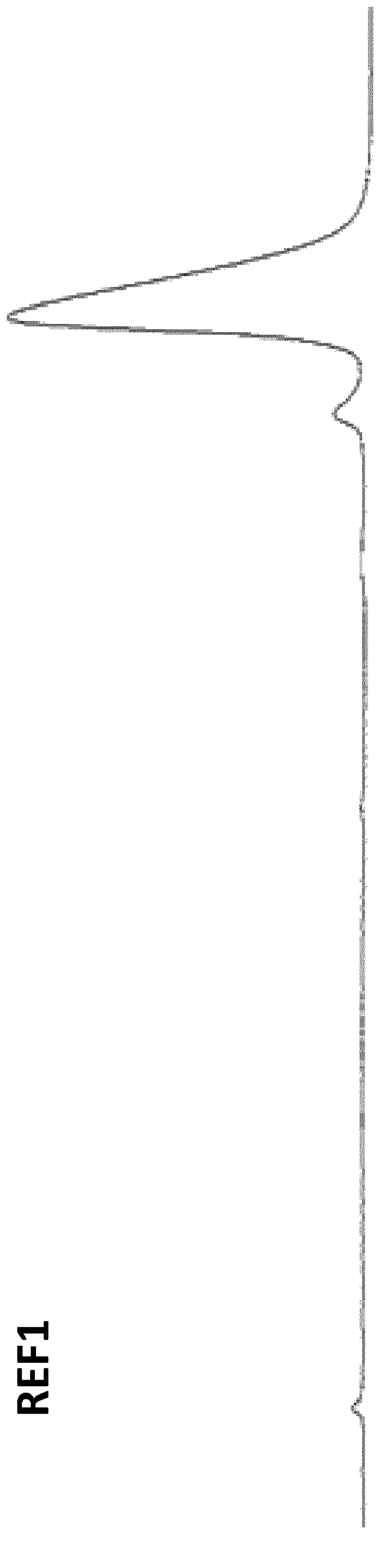


FIG. 1A

FIG. 1B

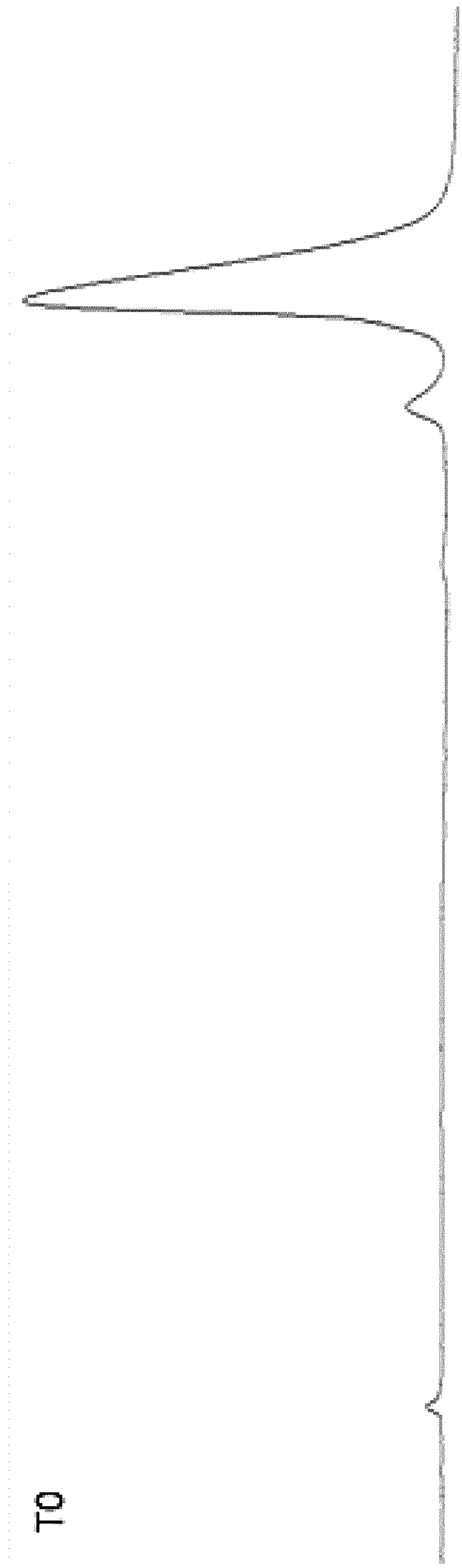

FIG. 1C

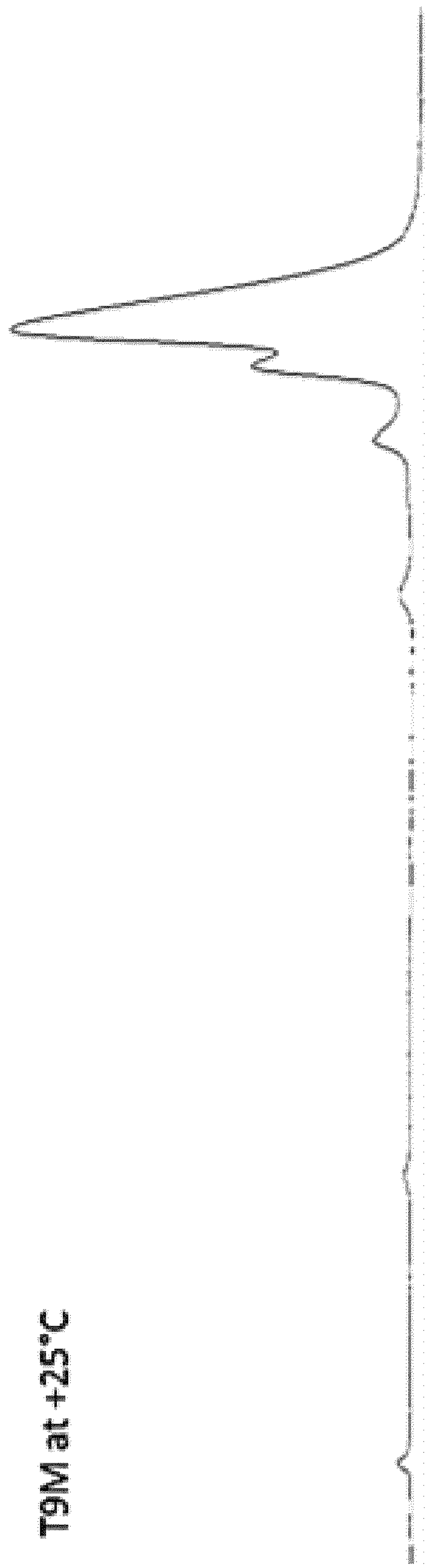

FIG. 1D

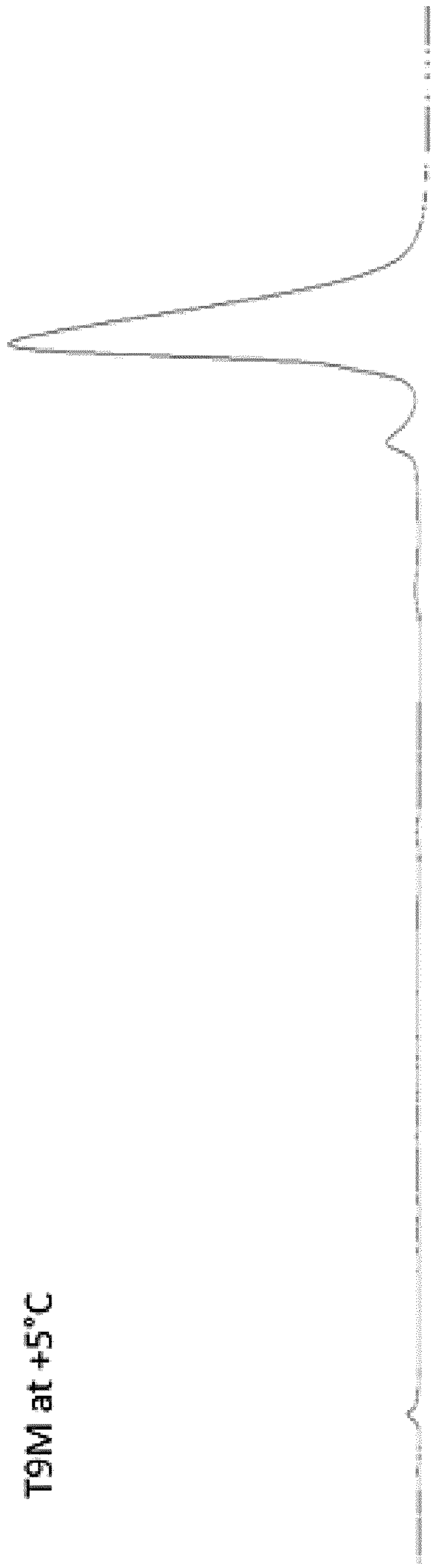

FIG. 2A

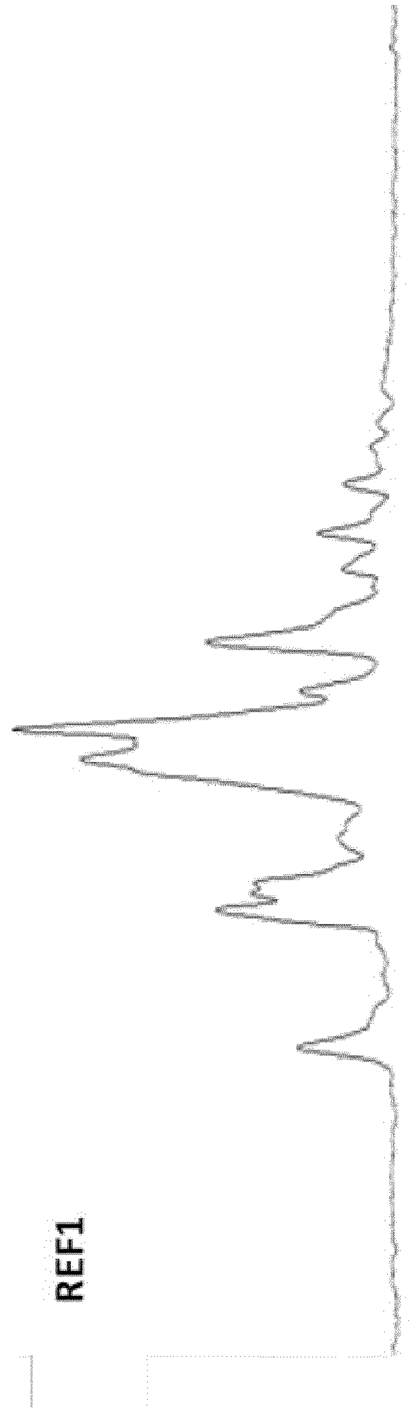

FIG. 2B

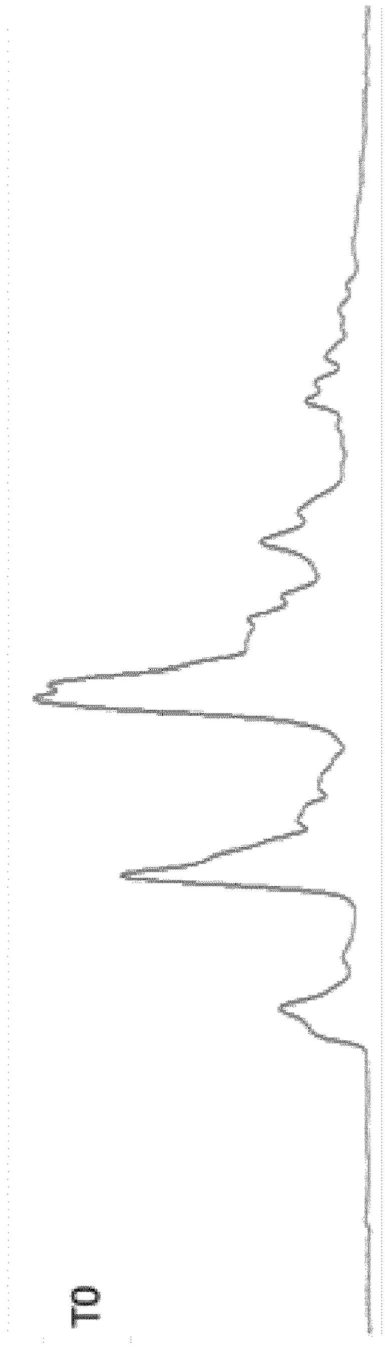

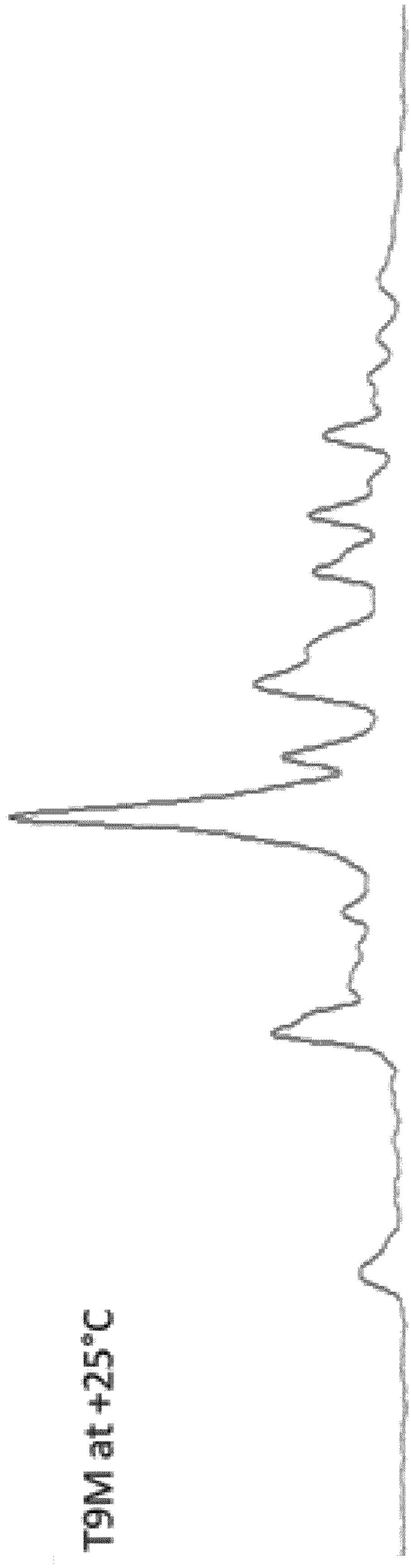


FIG. 2C

FIG. 2D

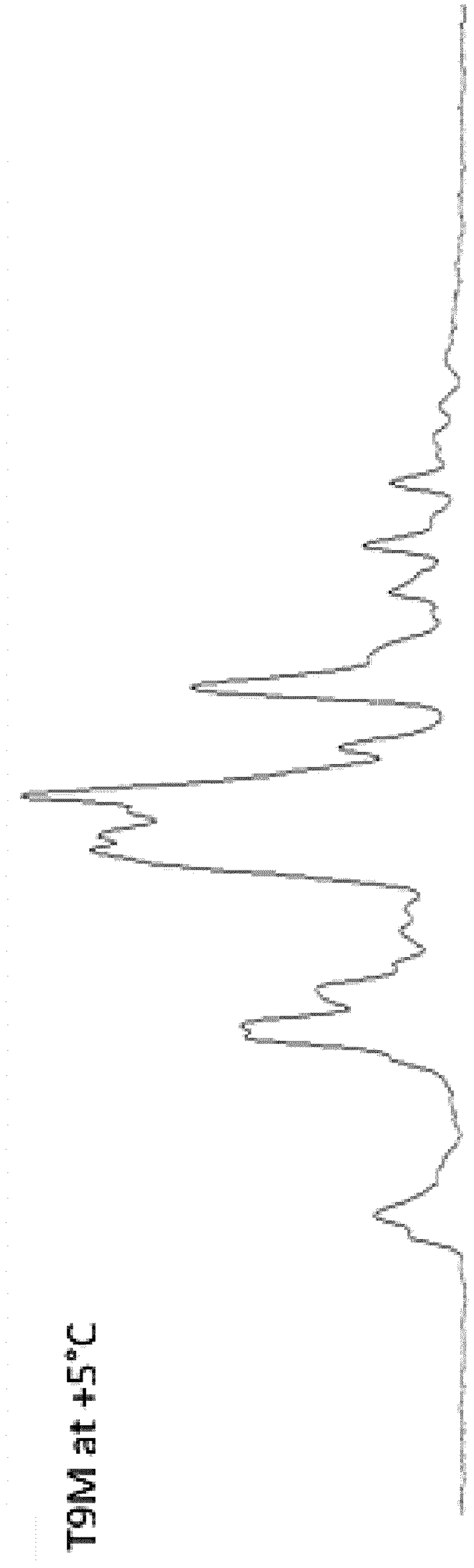

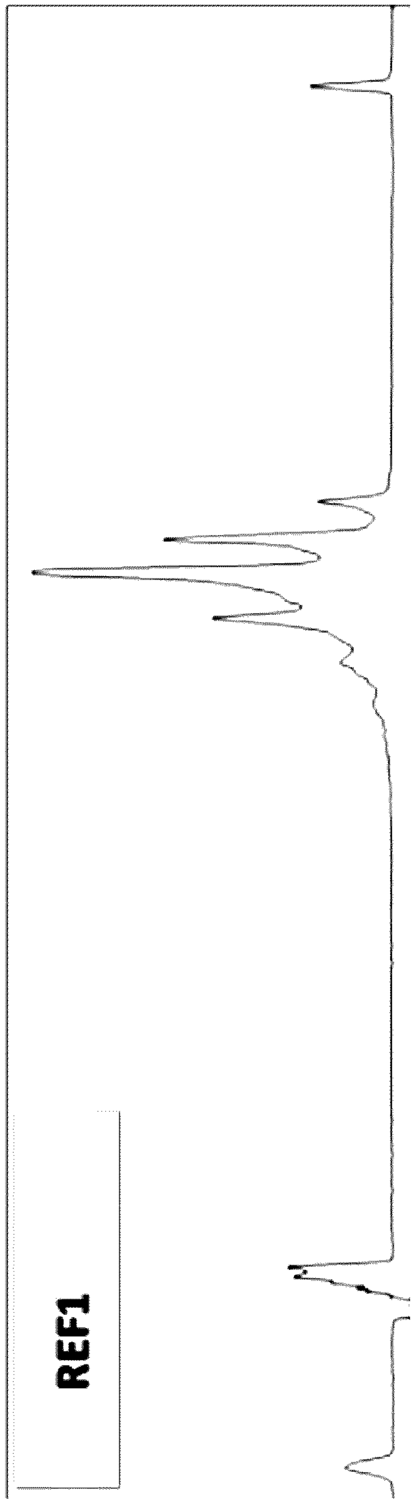


FIG. 3A

FIG. 3B

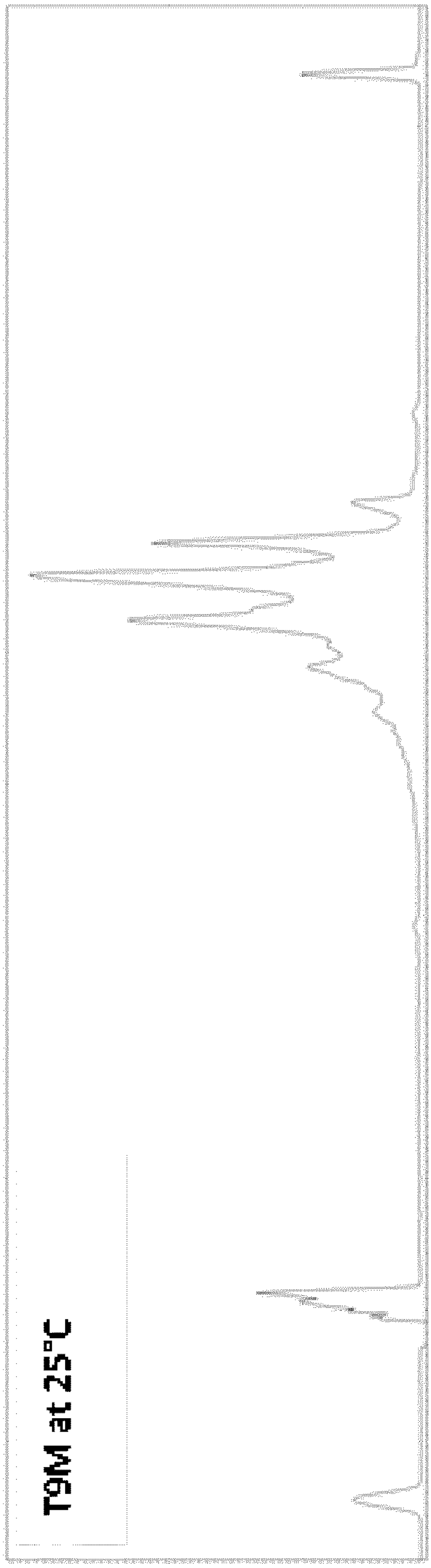

FIG. 3C

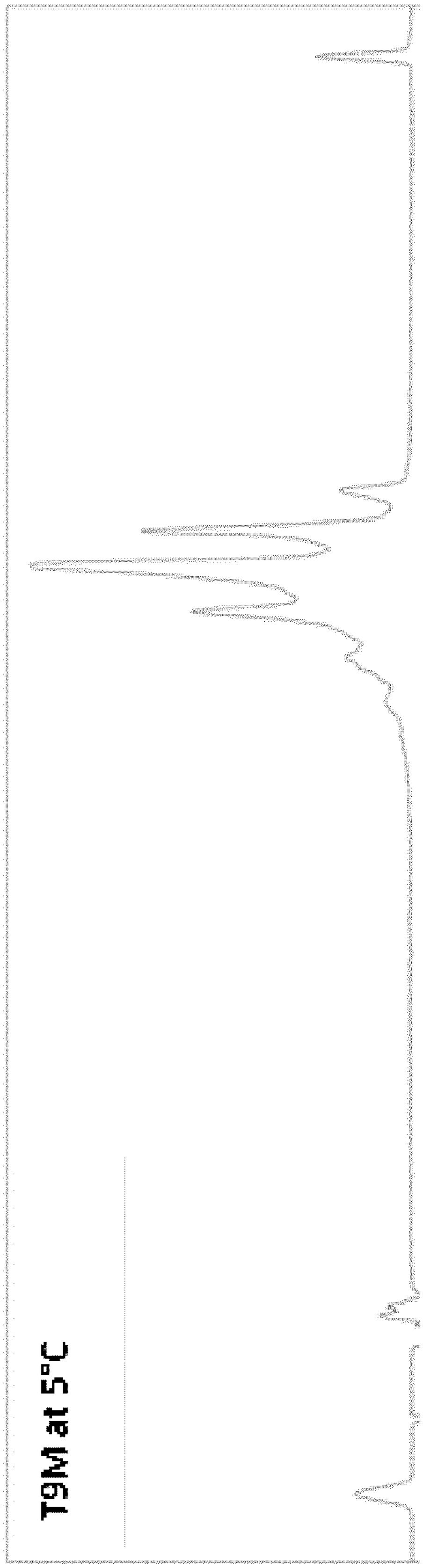

FIG. 4

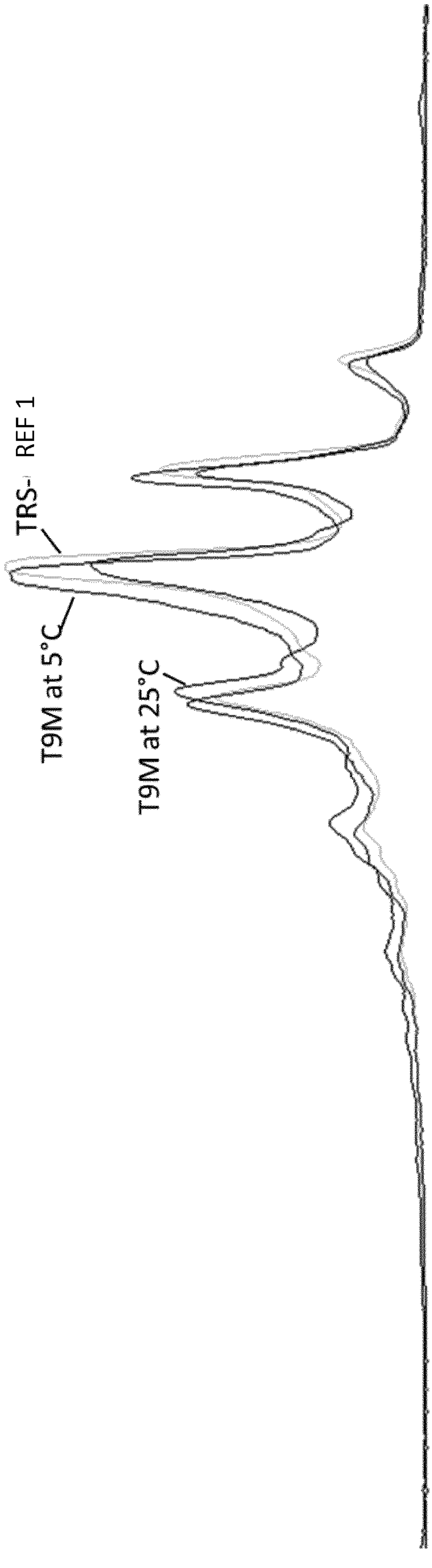

FIG. 5A

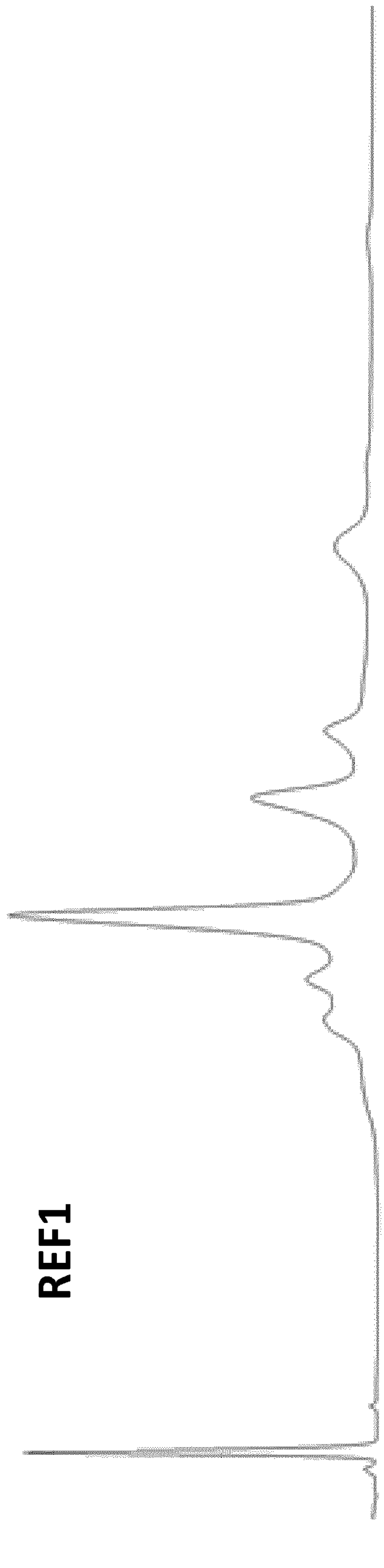

FIG. 5B

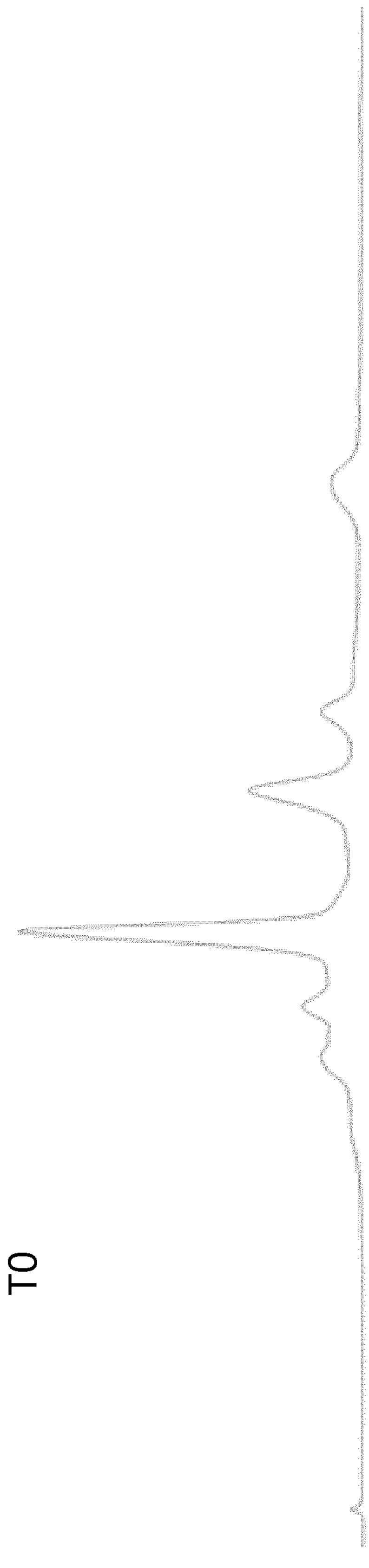

FIG. 5C

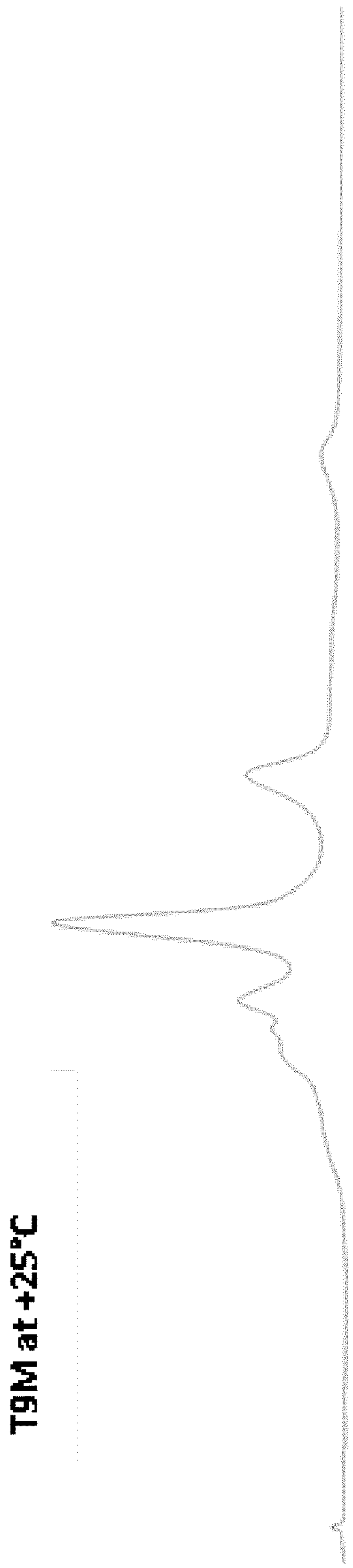

FIG. 5D

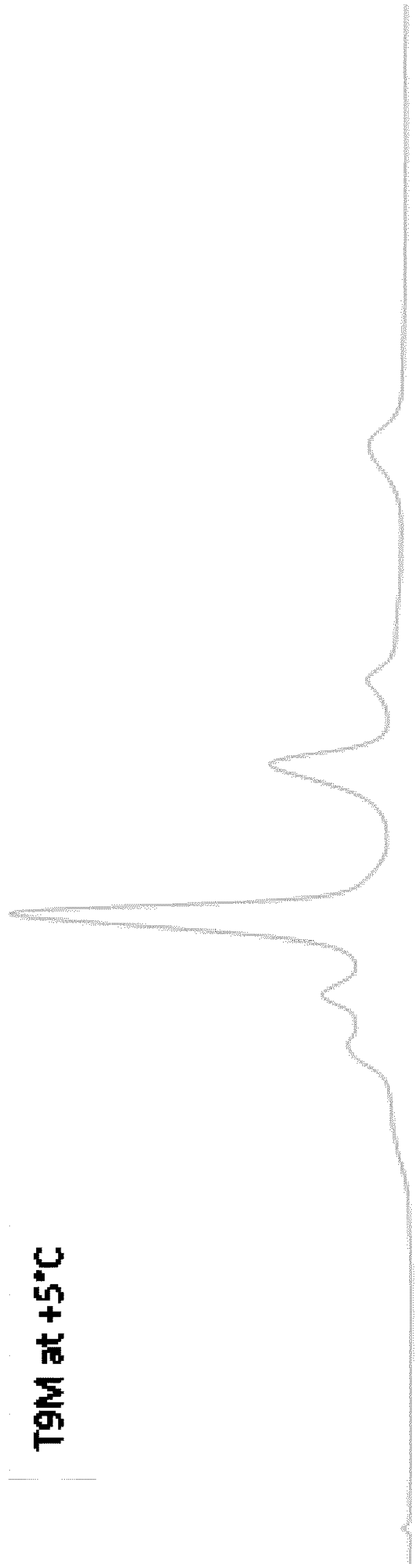

FIG. 6

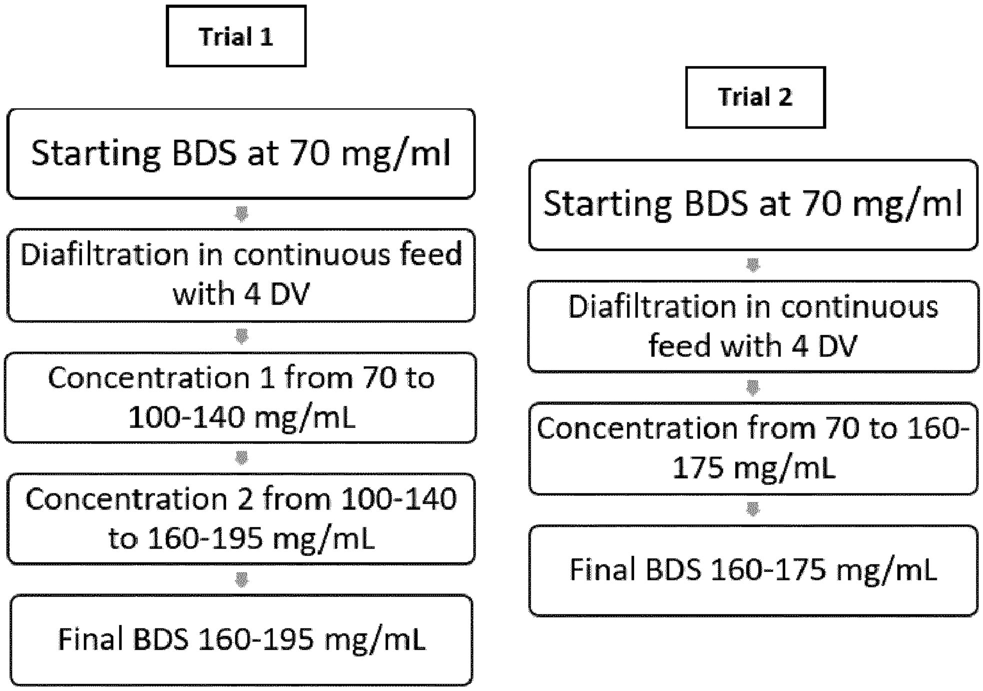
Trial 1
Starting BDS at 70 mg/ml
Diafiltration in continuous feed with 4 DV
Concentration 1 from 70 to 100-140 mg/mL
Concentration 2 from 100-140 to 160-195 mg/mL
Final BDS 160-195 mg/mL
Trial 2
Starting BDS at 70 mg/ml
Diafiltration in continuous feed with 4 DV
Concentration from 70 to 160-175 mg/mL
Final BDS 160-175 mg/mL

FIG. 7

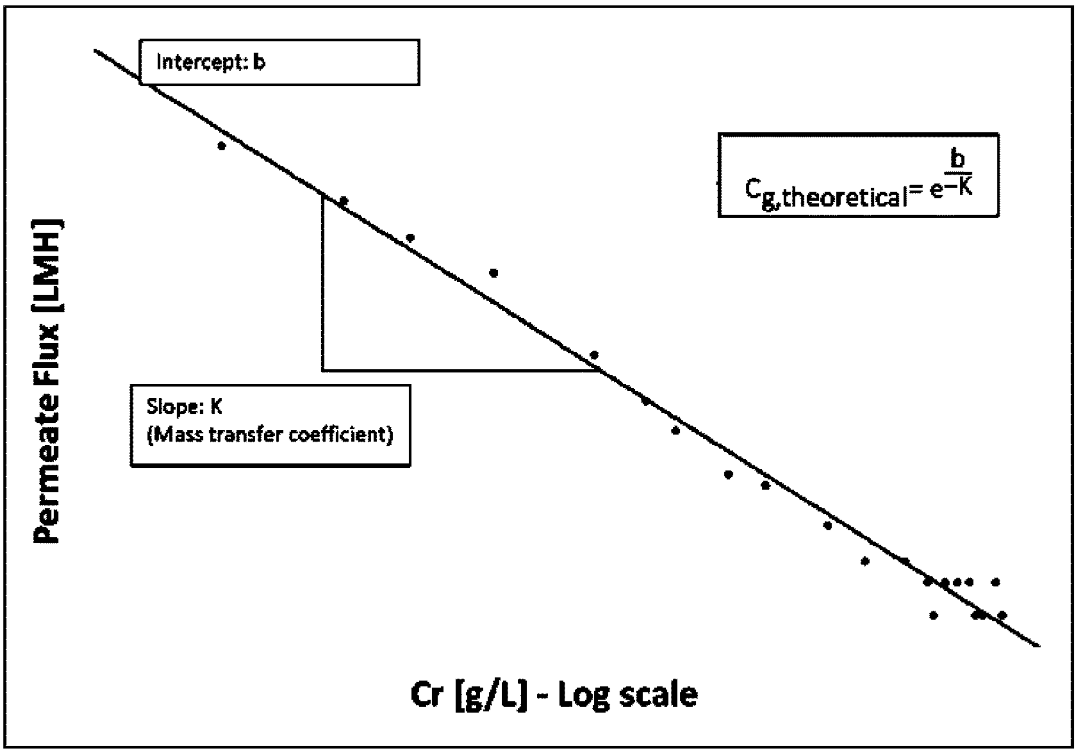
Intercept: b
Cg,theoretical= e^(b/-K)
Slope: K
(Mass transfer coefficient)
Permeate Flux [LMH]
Cr [g/L] - Log scale

FIG. 8A

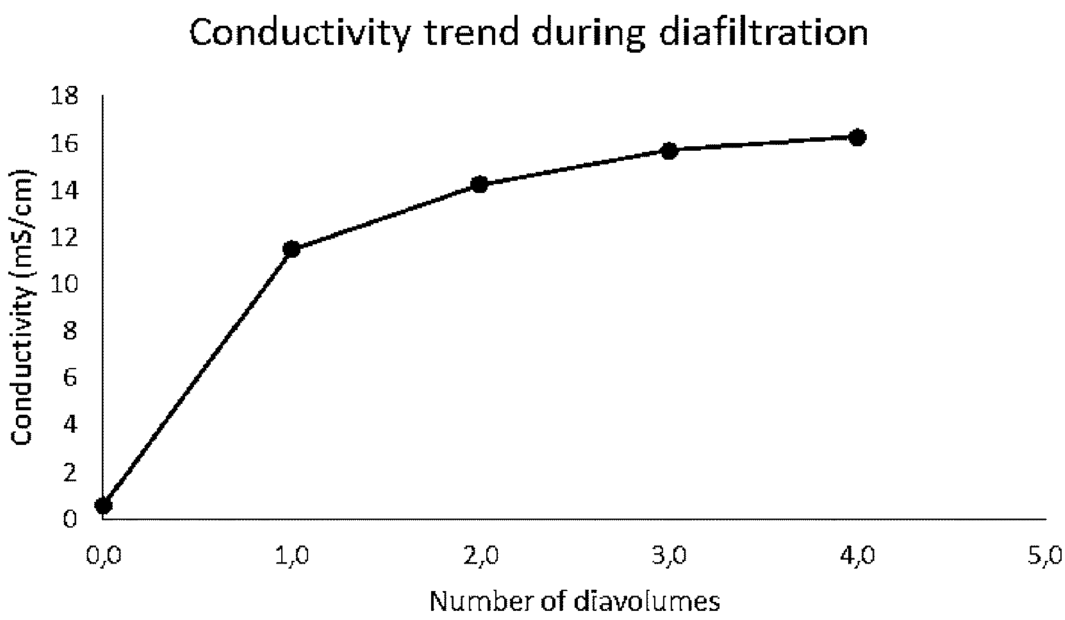
Conductivity trend during diafiltration
Conductivity (mS/cm)
18
16
14
12
10
8
6
4
2
0
0,0
1,0
2,0
3,0
4,0
5,0
Number of diavolumes

FIG. 8B

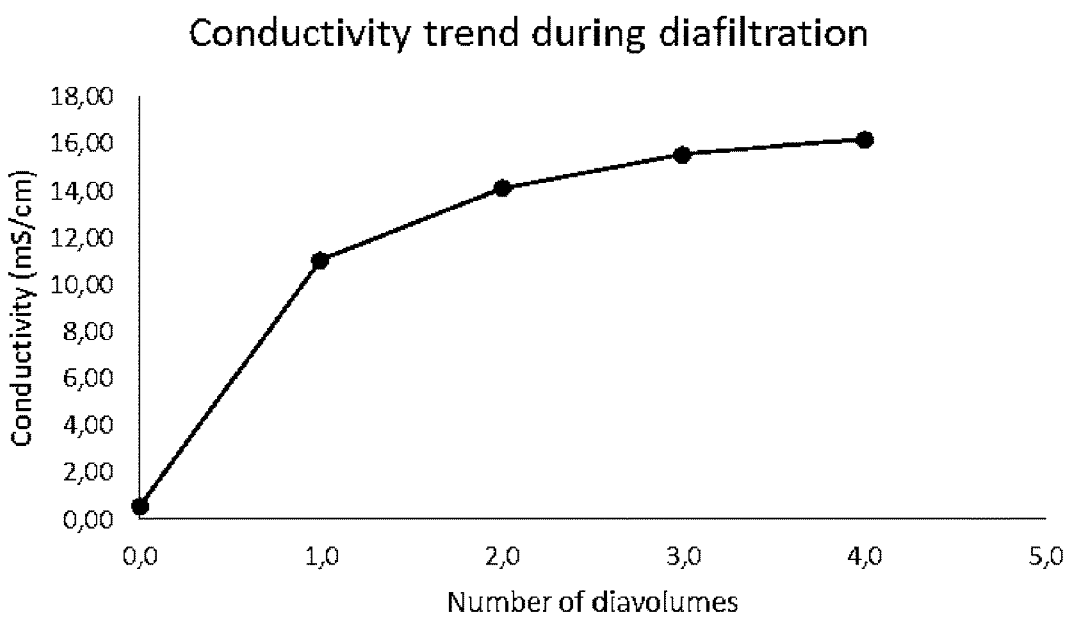
Conductivity trend during diafiltration
Conductivity (mS/cm)
18,00
16,00
14,00
12,00
10,00
8,00
6,00
4,00
2,00
0,00
0,0
1,0
2,0
3,0
4,0
5,0
Number of diavolumes

FIG. 8C

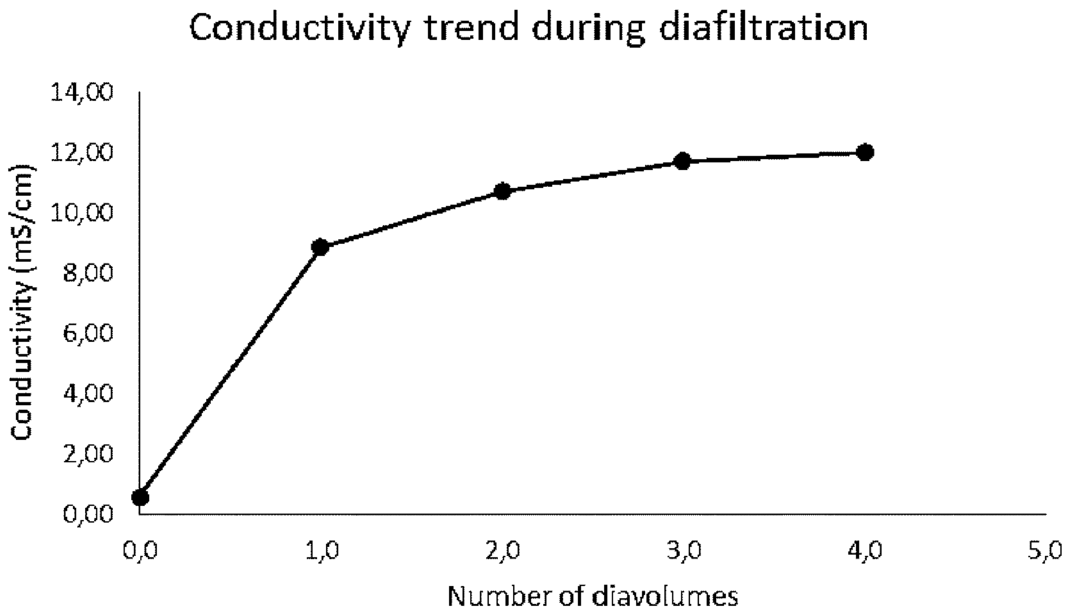
Conductivity trend during diafiltration
Conductivity (mS/cm)
14,00
12,00
10,00
8,00
6,00
4,00
2,00
0,00
0,0
1,0
2,0
3,0
4,0
5,0
Number of diavolumes

FIG. 8D

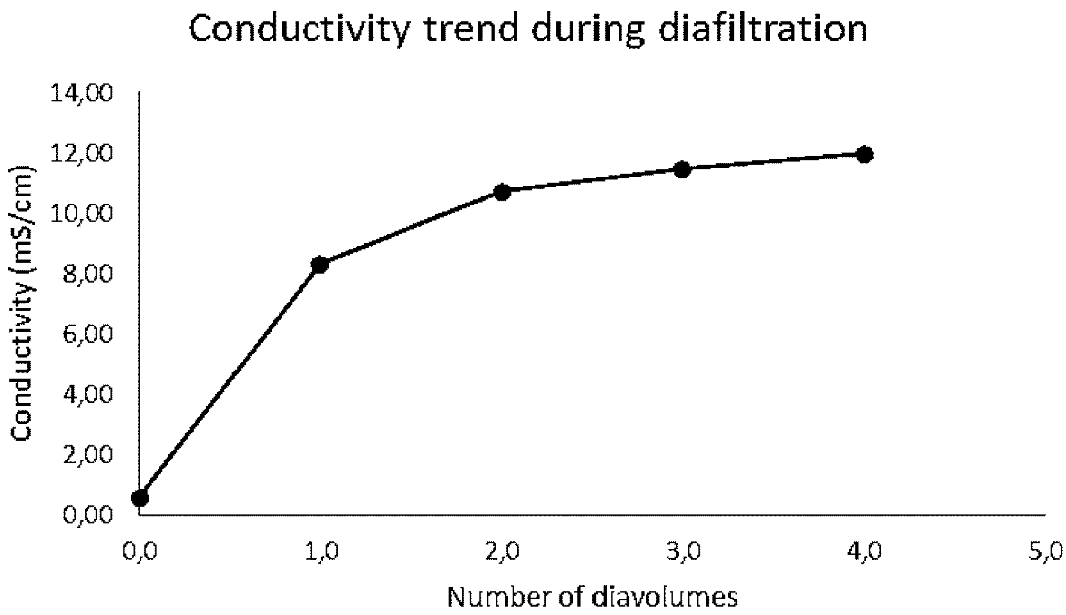
Conductivity trend during diafiltration
Conductivity (mS/cm)
14,00
12,00
10,00
8,00
6,00
4,00
2,00
0,00
0,0
1,0
2,0
3,0
4,0
5,0
Number of diavolumes

**FIG. 9A**

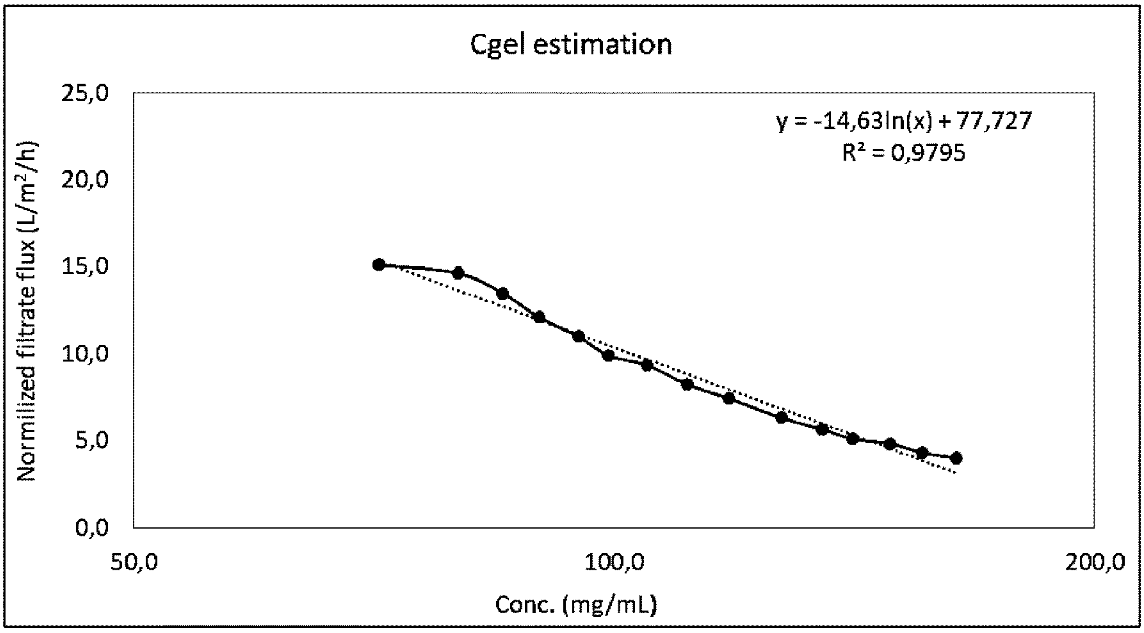
Cgel estimation
y = -14,63ln(x) + 77,727
R² = 0,9795
Normilized filtrate flux (L/m²/h)
25,0
20,0
15,0
10,0
5,0
0,0
50,0
100,0
200,0
Conc. (mg/mL)

**FIG. 9B**

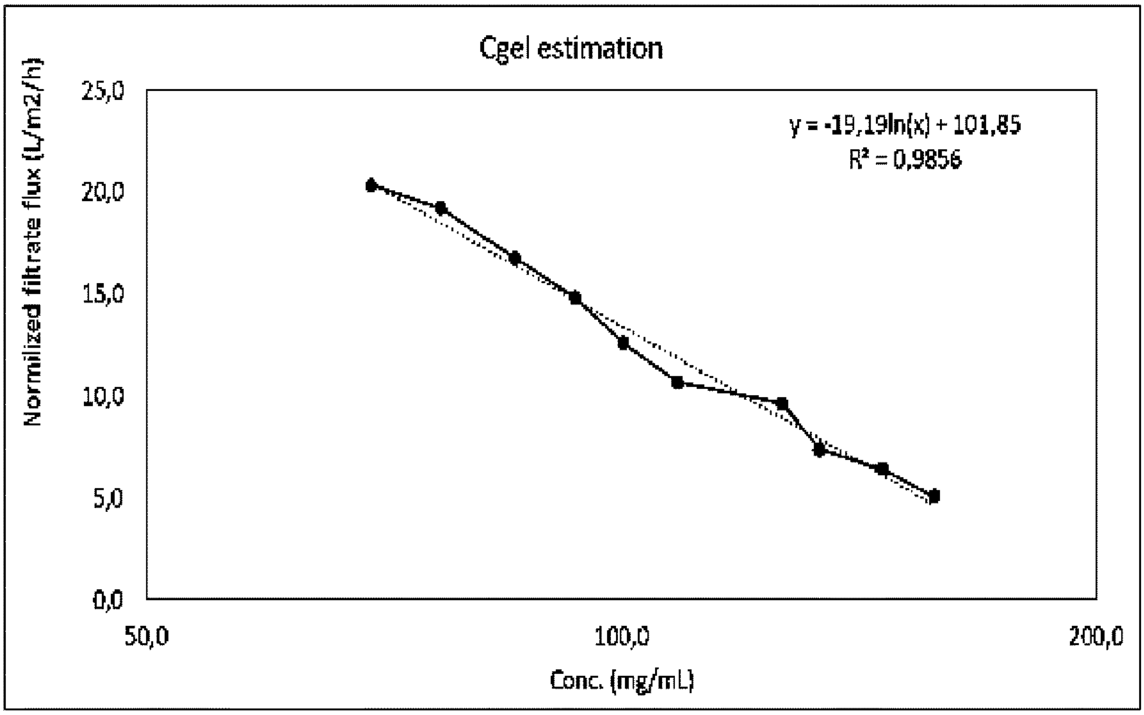
Cgel estimation
y = -19,19ln(x) + 101,85
R² = 0,9856
Normilized filtrate flux (L/m2/h)
25,0
20,0
15,0
10,0
5,0
0,0
50,0
100,0
200,0
Conc. (mg/mL)

FIG. 10

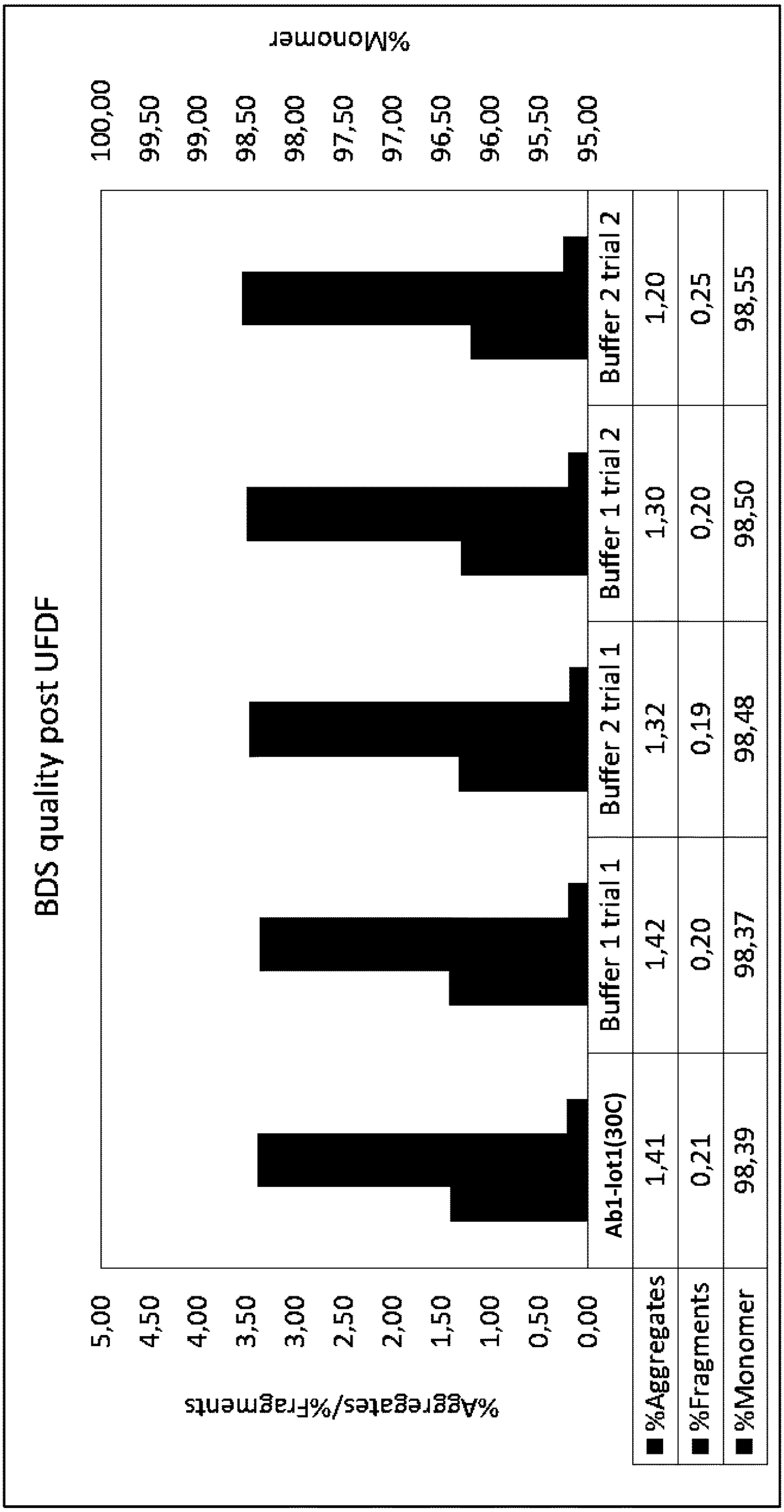
BDS quality post UFDF
%Aggregates/%Fragments
%Monomer
5,00
4,50
4,00
3,50
3,00
2,50
2,00
1,50
1,00
0,50
0,00
100,00
99,50
99,00
98,50
98,00
97,50
97,00
96,50
96,00
95,50
95,00
Ab1-lot1(30C) | Buffer 1 trial 1 | Buffer 2 trial 1 | Buffer 1 trial 2 | Buffer 2 trial 2
%Aggregates | 1,41 | 1,42 | 1,32 | 1,30 | 1,20
%Fragments | 0,21 | 0,20 | 0,19 | 0,20 | 0,25
%Monomer | 98,39 | 98,37 | 98,48 | 98,50 | 98,55

FIG. 11

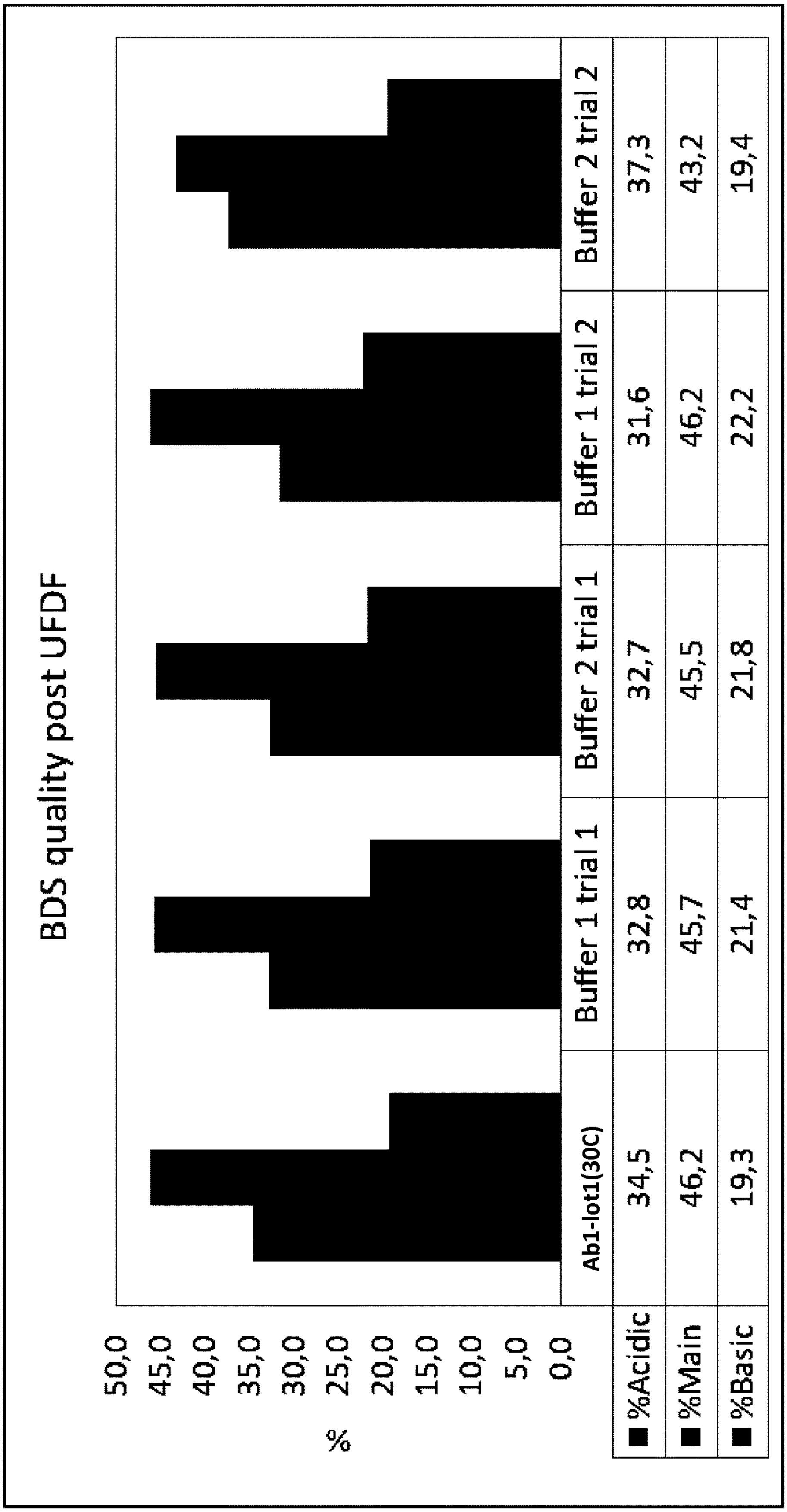
BDS quality post UFDF
%
50,0
45,0
40,0
35,0
30,0
25,0
20,0
15,0
10,0
5,0
0,0
Ab1-lot1(30C)
Buffer 1 trial 1
Buffer 2 trial 1
Buffer 1 trial 2
Buffer 2 trial 2
%Acidic 34,5 32,8 32,7 31,6 37,3
%Main 46,2 45,7 45,5 46,2 43,2
%Basic 19,3 21,4 21,8 22,2 19,4

FIG. 12

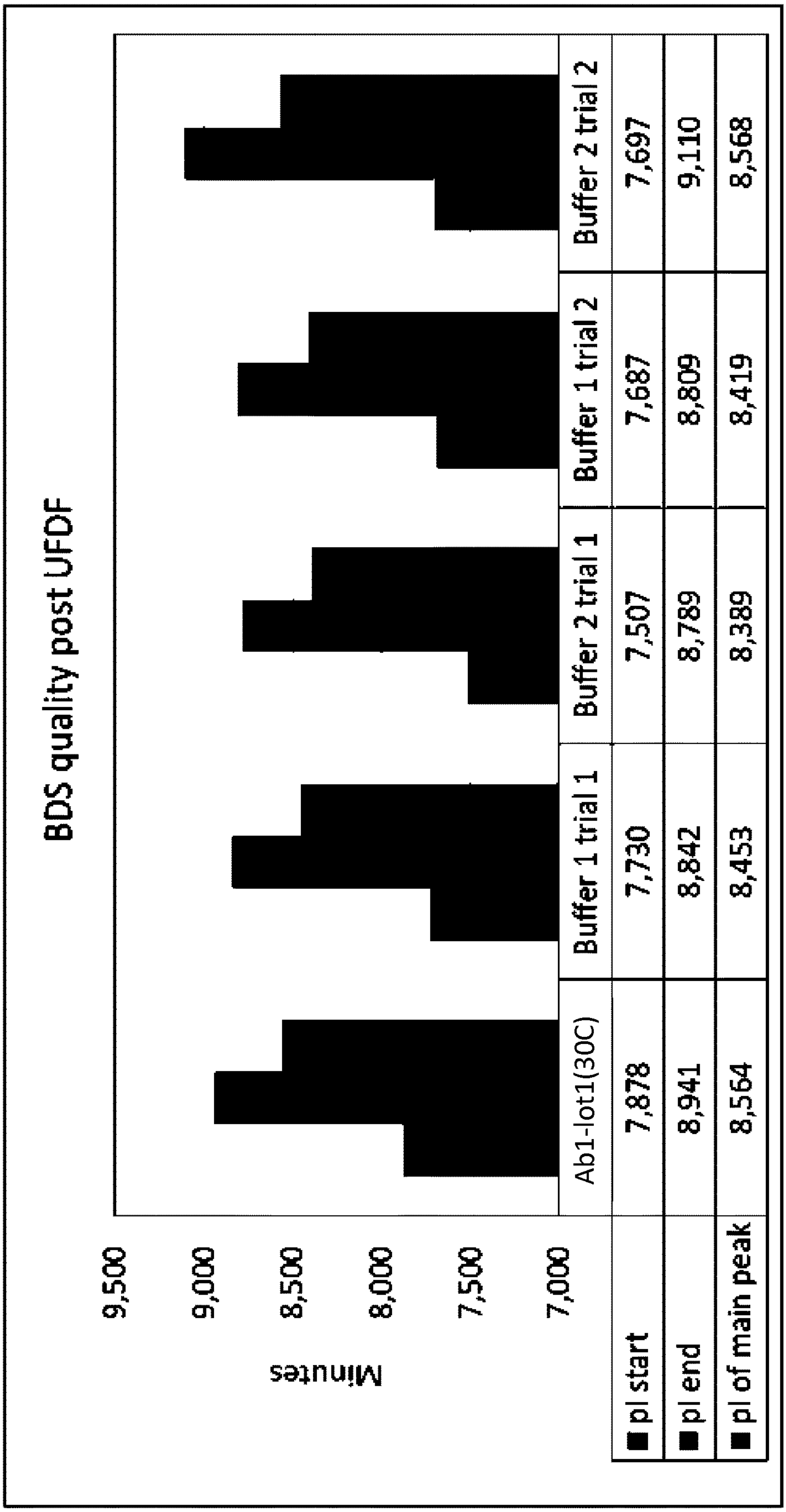
BDS quality post UFDF
Minutes
9,500
9,000
8,500
8,000
7,500
7,000
Ab1-lot1(30C) | Buffer 1 trial 1 | Buffer 2 trial 1 | Buffer 1 trial 2 | Buffer 2 trial 2
pI start | 7,878 | 7,730 | 7,507 | 7,687 | 7,697
pI end | 8,941 | 8,842 | 8,789 | 8,809 | 9,110
pI of main peak | 8,564 | 8,453 | 8,389 | 8,419 | 8,568

FIG. 13A

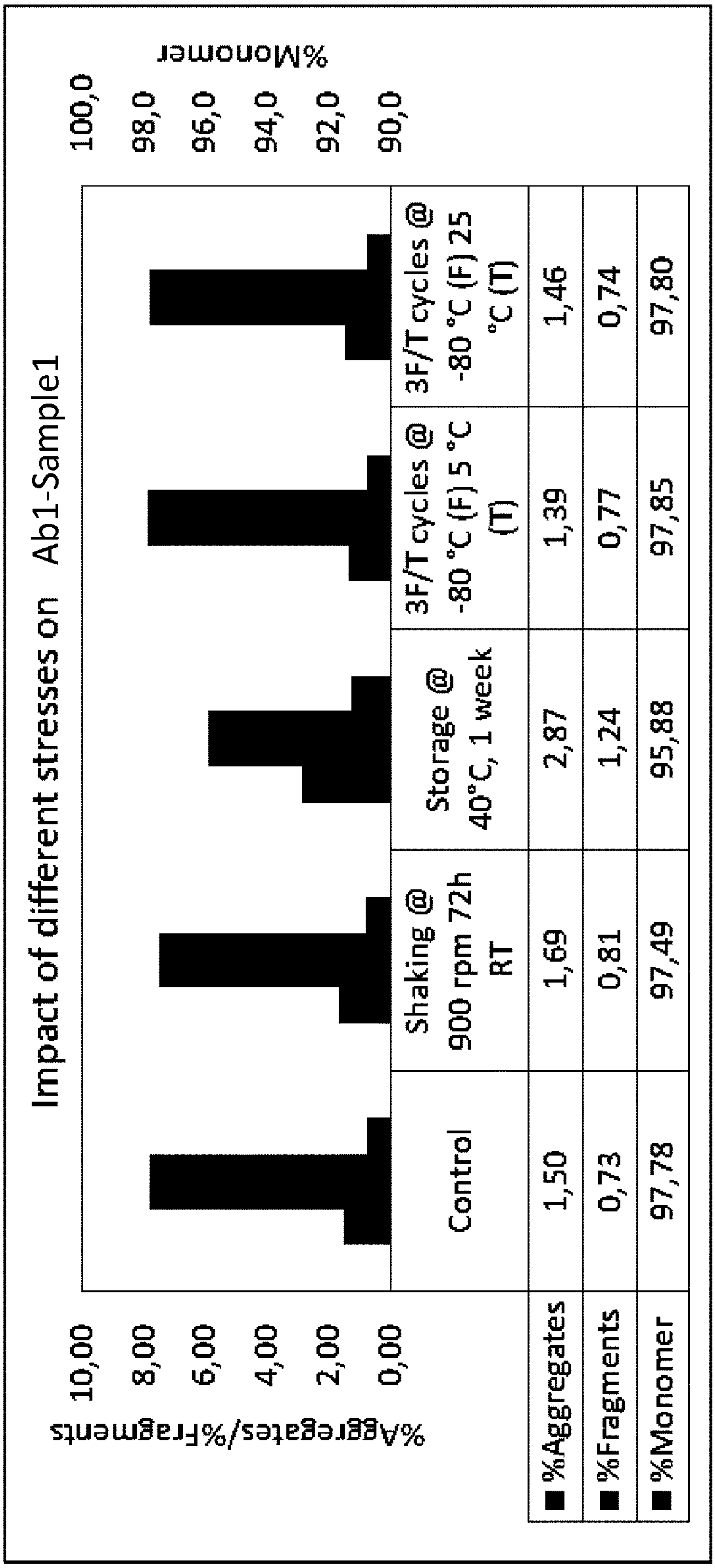
Impact of different stresses on Ab1-Sample1
%Aggregates/%Fragments
10,00
8,00
6,00
4,00
2,00
0,00
%Monomer
100,0
98,0
96,0
94,0
92,0
90,0
Control
Shaking @ 900 rpm 72h RT
Storage @ 40°C, 1 week
3F/T cycles @ -80 °C (F) 5 °C (T)
3F/T cycles @ -80 °C (F) 25 °C (T)
%Aggregates 1,50 1,69 2,87 1,39 1,46
%Fragments 0,73 0,81 1,24 0,77 0,74
%Monomer 97,78 97,49 95,88 97,85 97,80

FIG. 13B

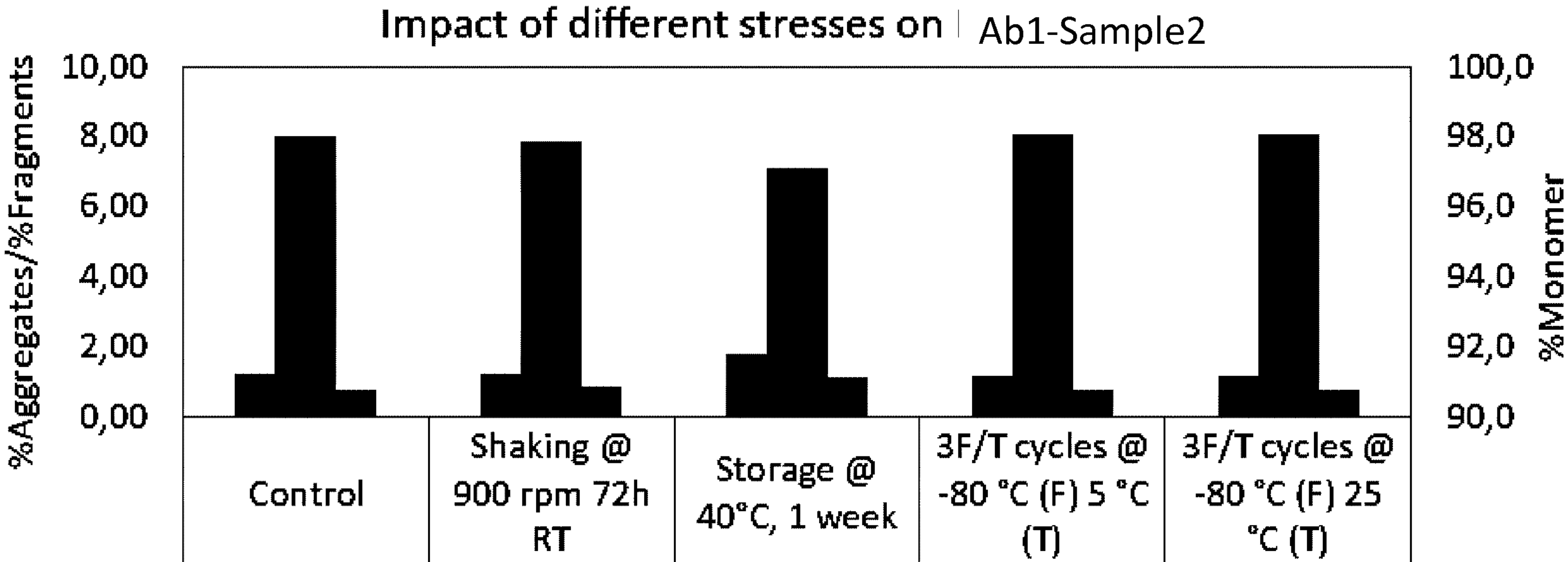


| | Control | Shaking @ 900 rpm 72h RT | Storage @ 40°C, 1 week | 3F/T cycles @ -80 °C (F) 5 °C (T) | 3F/T cycles @ -80 °C (F) 25 °C (T) |
|---|---|---|---|---|---|
| %Aggregates | 1,22 | 1,26 | 1,79 | 1,19 | 1,18 |
| %Fragments | 0,79 | 0,87 | 1,13 | 0,77 | 0,78 |
| %Monomer | 97,99 | 97,86 | 97,08 | 98,04 | 98,05 |

FIG. 14A

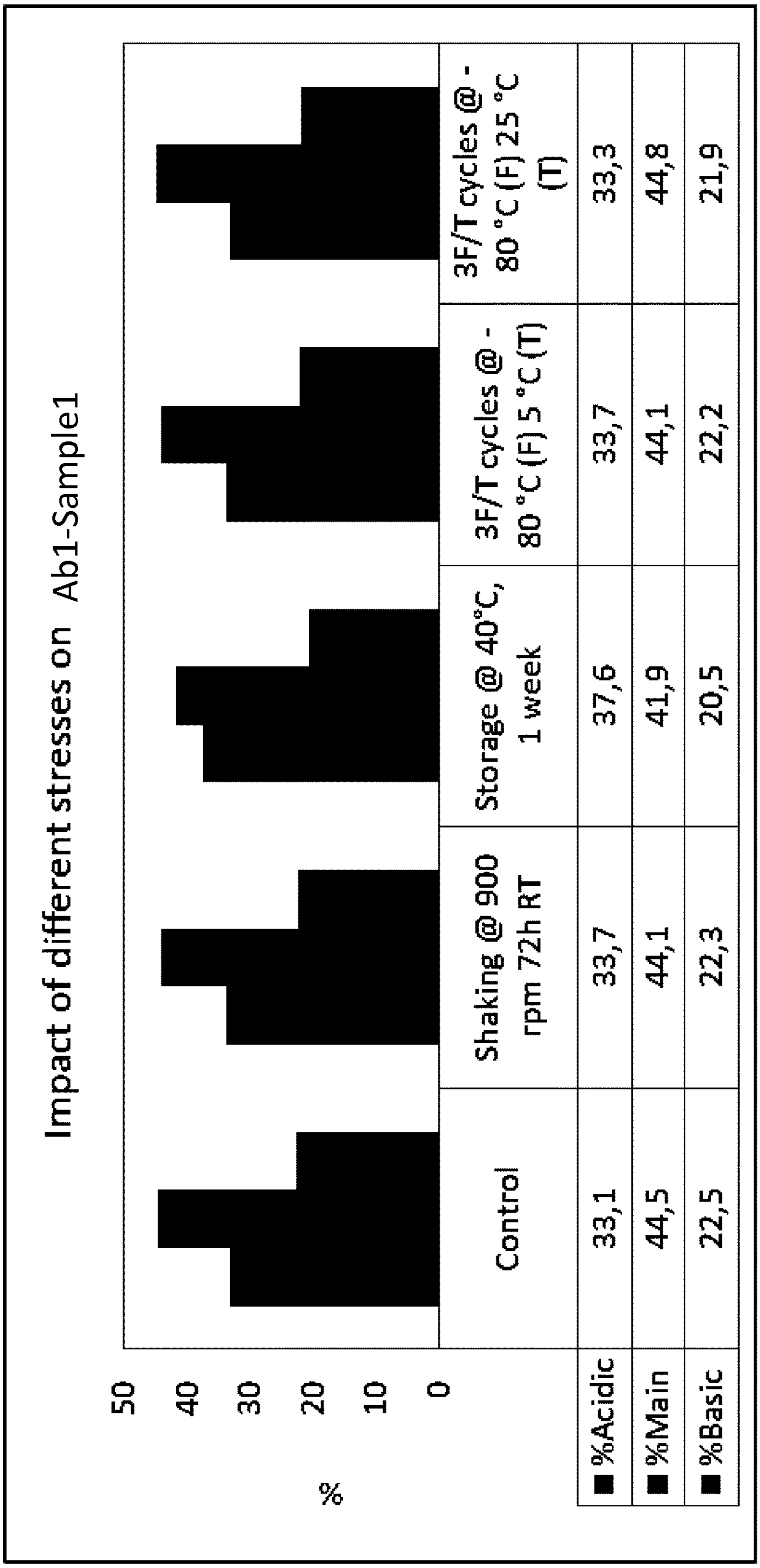
Impact of different stresses on Ab1-Sample1
%
50
40
30
20
10
0
Control
Shaking @ 900 rpm 72h RT
Storage @ 40°C, 1 week
3F/T cycles @ -80 °C (F) 5 °C (T)
3F/T cycles @ -80 °C (F) 25 °C (T)
%Acidic 33,1 33,7 37,6 33,7 33,3
%Main 44,5 44,1 41,9 44,1 44,8
%Basic 22,5 22,3 20,5 22,2 21,9

FIG. 14B

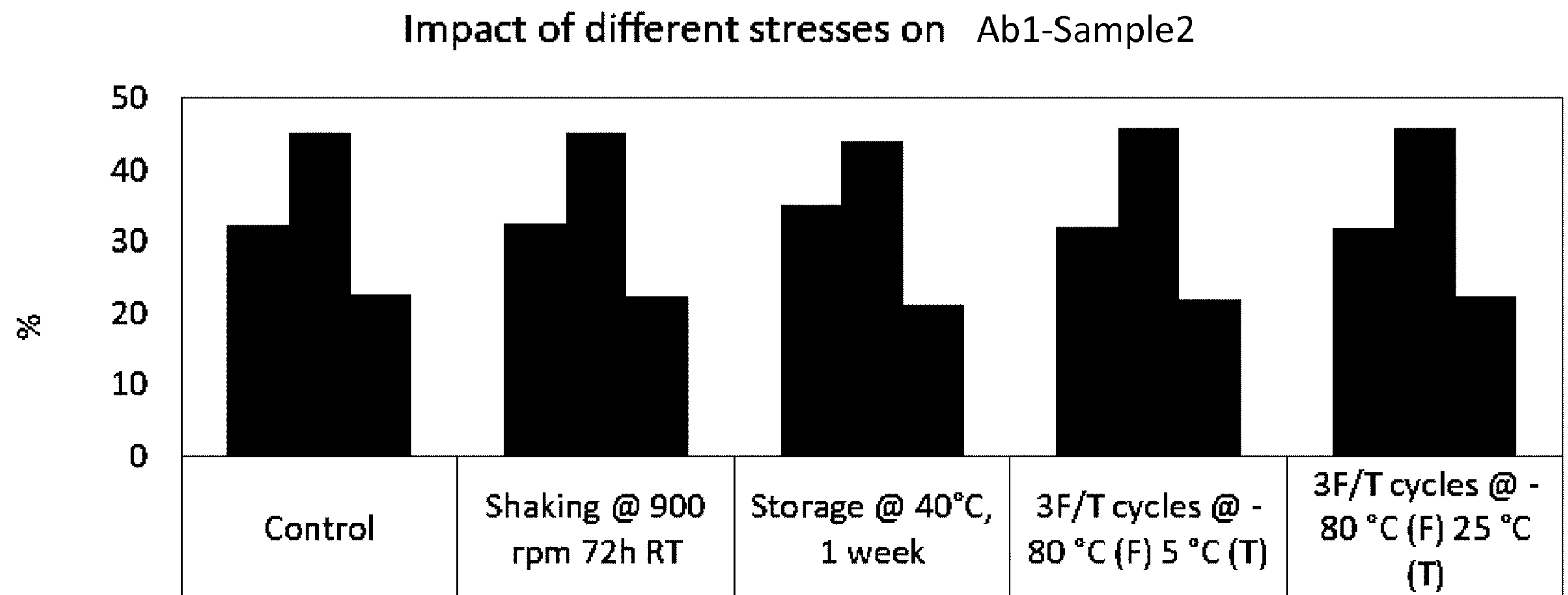


| | Control | Shaking @ 900 rpm 72h RT | Storage @ 40°C, 1 week | 3F/T cycles @ -80 °C (F) 5 °C (T) | 3F/T cycles @ -80 °C (F) 25 °C (T) |
|---|---|---|---|---|---|
| ■ %Acidic | 32,20 | 32,40 | 35,00 | 32,00 | 31,80 |
| ■ %Main | 45,10 | 45,20 | 44,00 | 45,90 | 46,00 |
| ■ %Basic | 22,70 | 22,50 | 21,10 | 22,00 | 22,30 |

FIG. 15

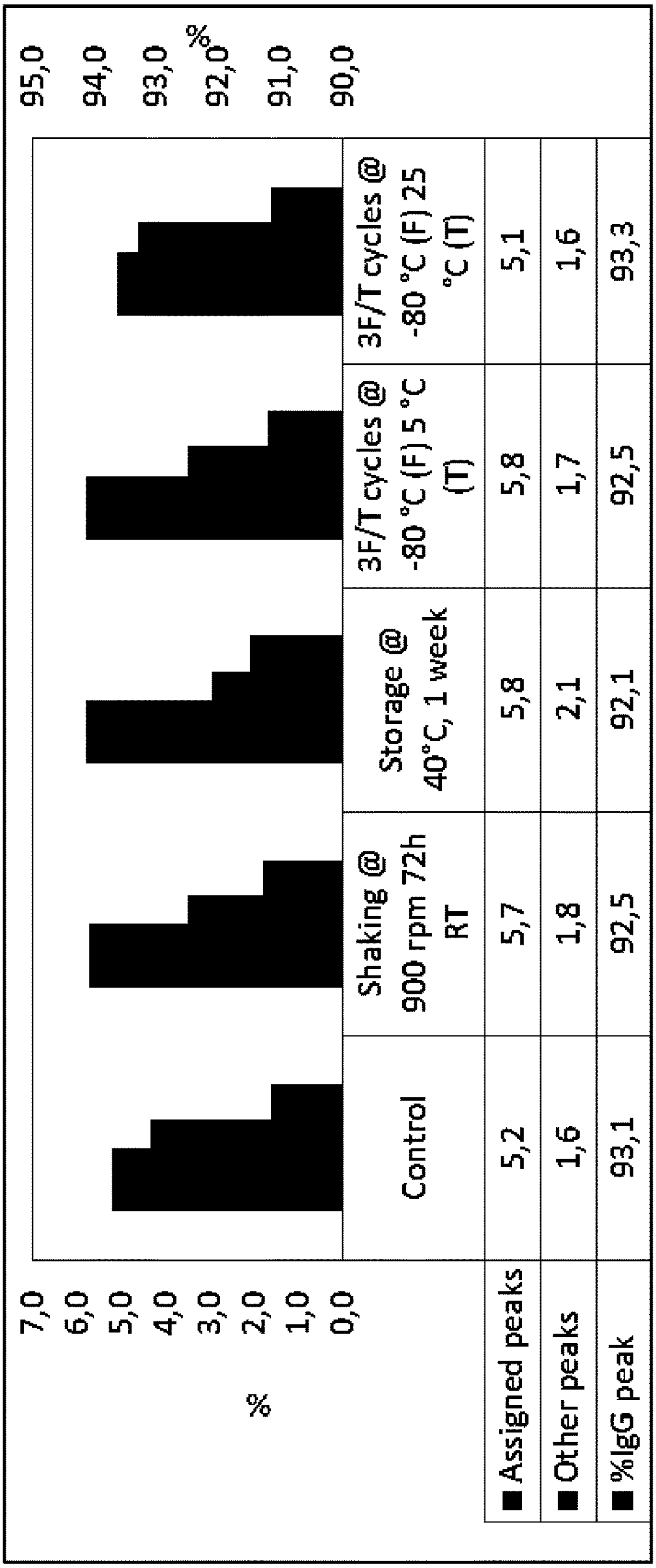


| | Control | Shaking @ 900 rpm 72h RT | Storage @ 40°C, 1 week | 3F/T cycles @ -80 °C (F) 5 °C (T) | 3F/T cycles @ -80 °C (F) 25 °C (T) |
|---|---|---|---|---|---|
| ■ Assigned peaks | 5,2 | 5,7 | 5,8 | 5,8 | 5,1 |
| ■ Other peaks | 1,6 | 1,8 | 2,1 | 1,7 | 1,6 |
| ■ %IgG peak | 93,1 | 92,5 | 92,1 | 92,5 | 93,3 |

**FIG. 16**

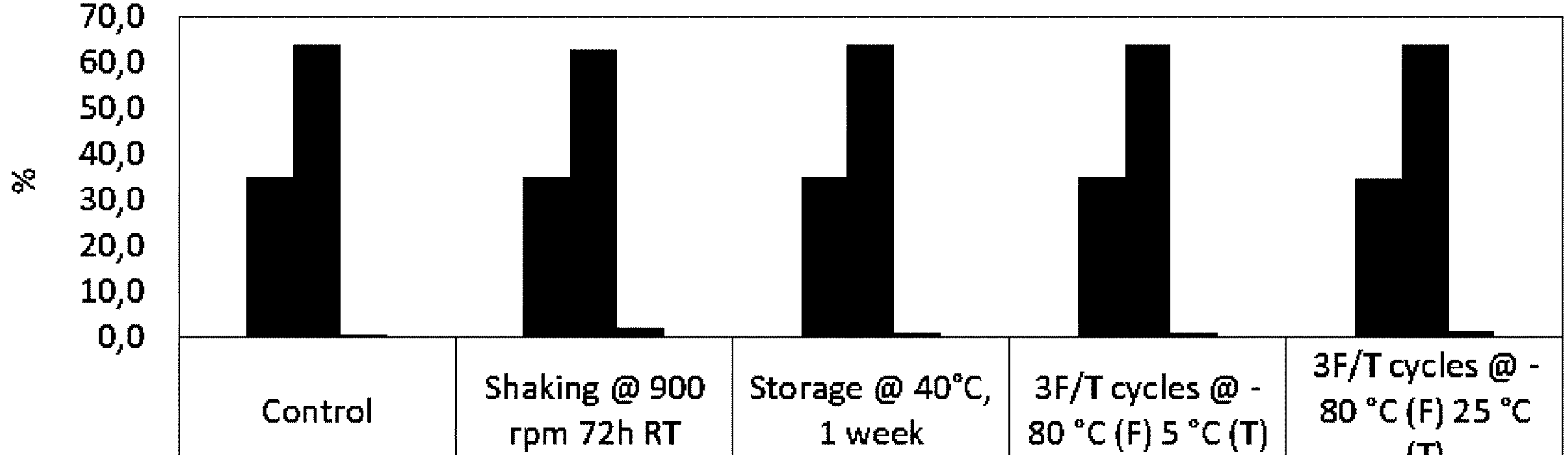


| | Control | Shaking @ 900 rpm 72h RT | Storage @ 40°C, 1 week | 3F/T cycles @ -80 °C (F) 5 °C (T) | 3F/T cycles @ -80 °C (F) 25 °C (T) |
|---|---|---|---|---|---|
| ■ %LC | 35,2 | 35,0 | 34,9 | 35,0 | 34,6 |
| ■ %HC | 64,1 | 62,7 | 64,1 | 64,0 | 64,0 |
| ■ Total other | 0,7 | 2,2 | 1,0 | 1,1 | 1,3 |

FIG. 17

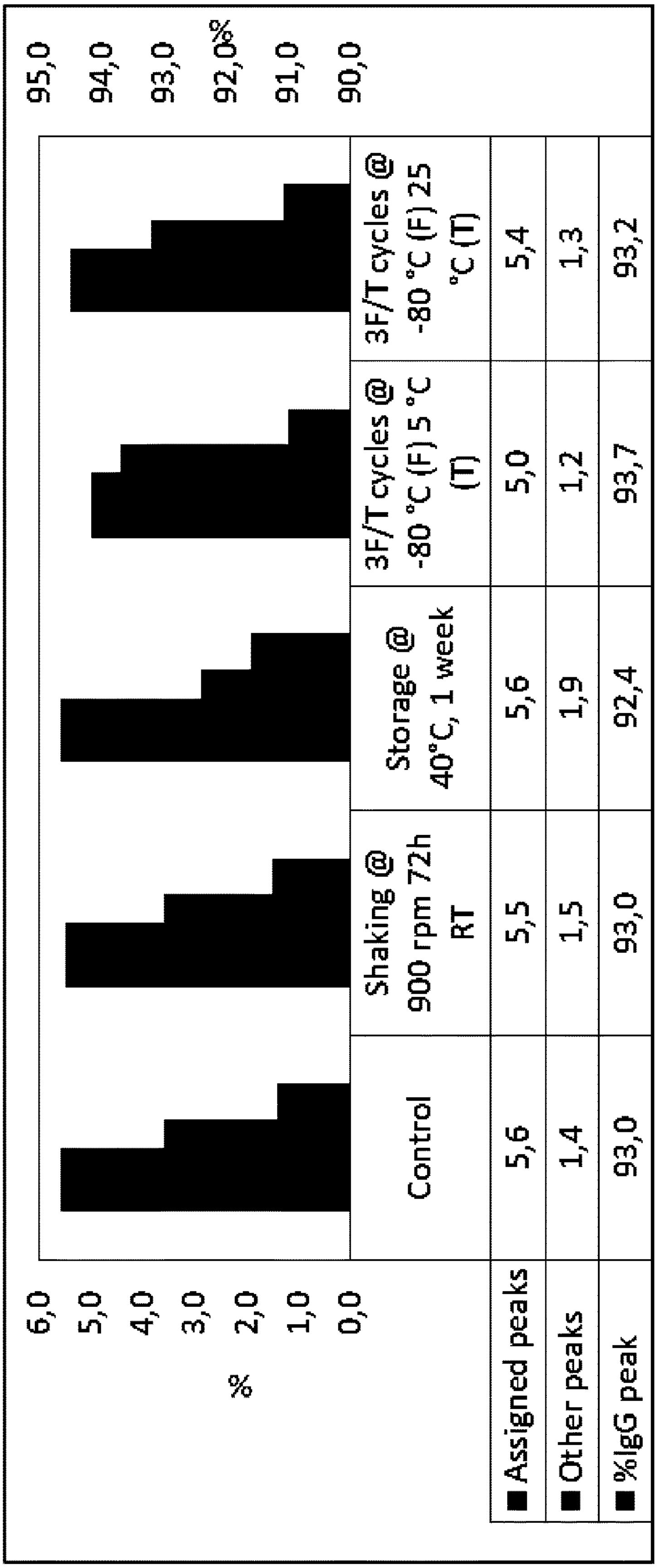


| | Control | Shaking @ 900 rpm 72h RT | Storage @ 40°C, 1 week | 3F/T cycles @ -80 °C (F) 5 °C (T) | 3F/T cycles @ -80 °C (F) 25 °C (T) |
|---|---|---|---|---|---|
| ■ Assigned peaks | 5,6 | 5,5 | 5,6 | 5,0 | 5,4 |
| ■ Other peaks | 1,4 | 1,5 | 1,9 | 1,2 | 1,3 |
| ■ %IgG peak | 93,0 | 93,0 | 92,4 | 93,7 | 93,2 |

# FIG. 18

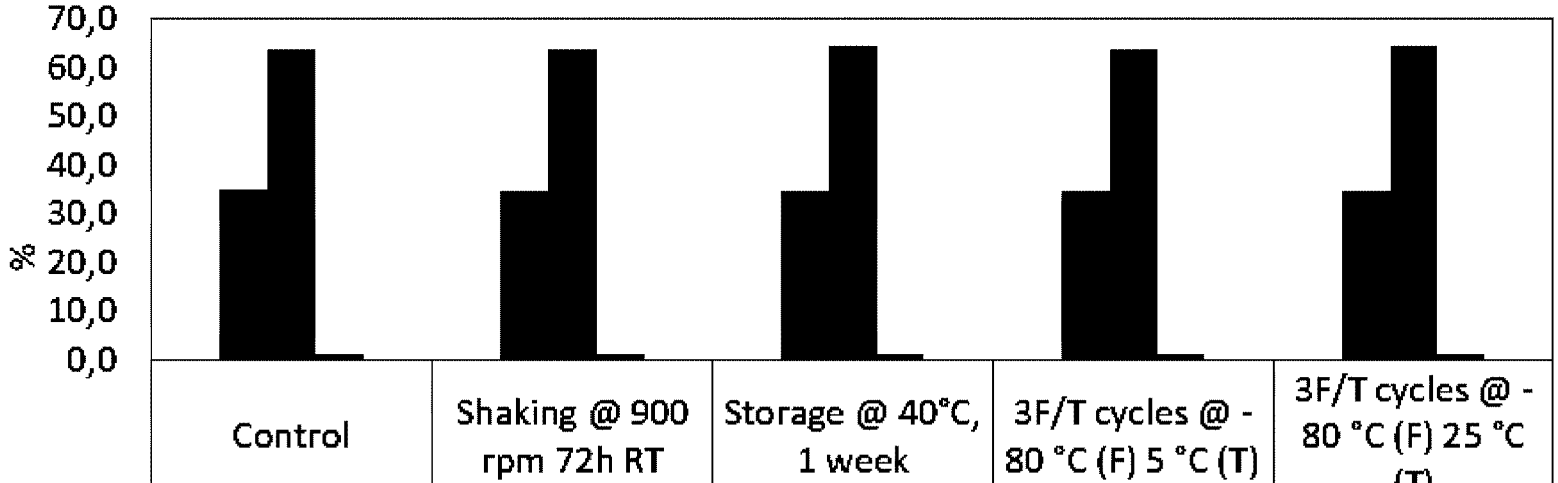


| | Control | Shaking @ 900 rpm 72h RT | Storage @ 40°C, 1 week | 3F/T cycles @ -80 °C (F) 5 °C (T) | 3F/T cycles @ -80 °C (F) 25 °C (T) |
|---|---|---|---|---|---|
| ■ %LC | 34,9 | 34,8 | 34,5 | 34,7 | 34,7 |
| ■ %HC | 63,9 | 63,9 | 64,3 | 63,9 | 64,3 |
| ■ Total other | 1,2 | 1,2 | 1,2 | 1,3 | 1,1 |

FIG. 19A

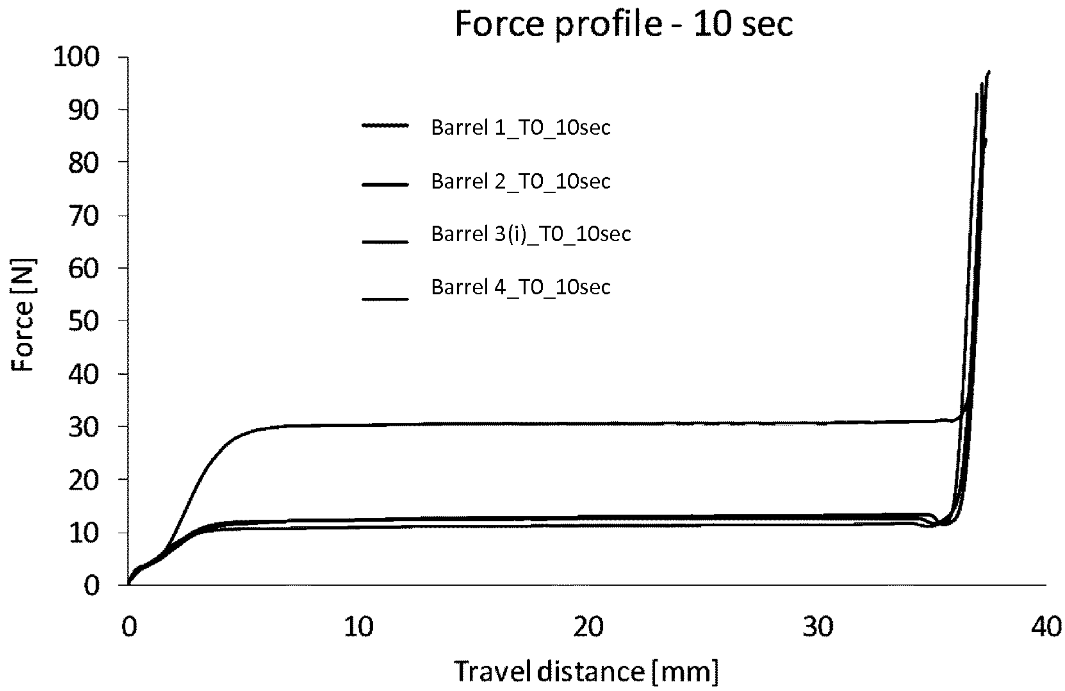


FIG. 19B

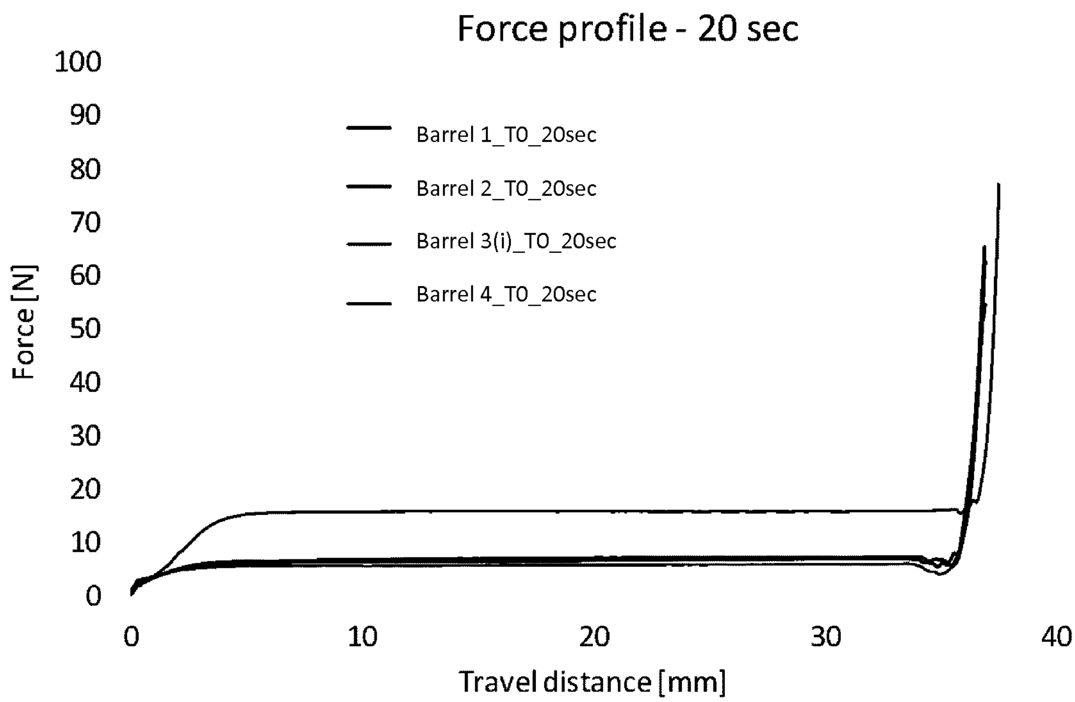

# FIG. 19C

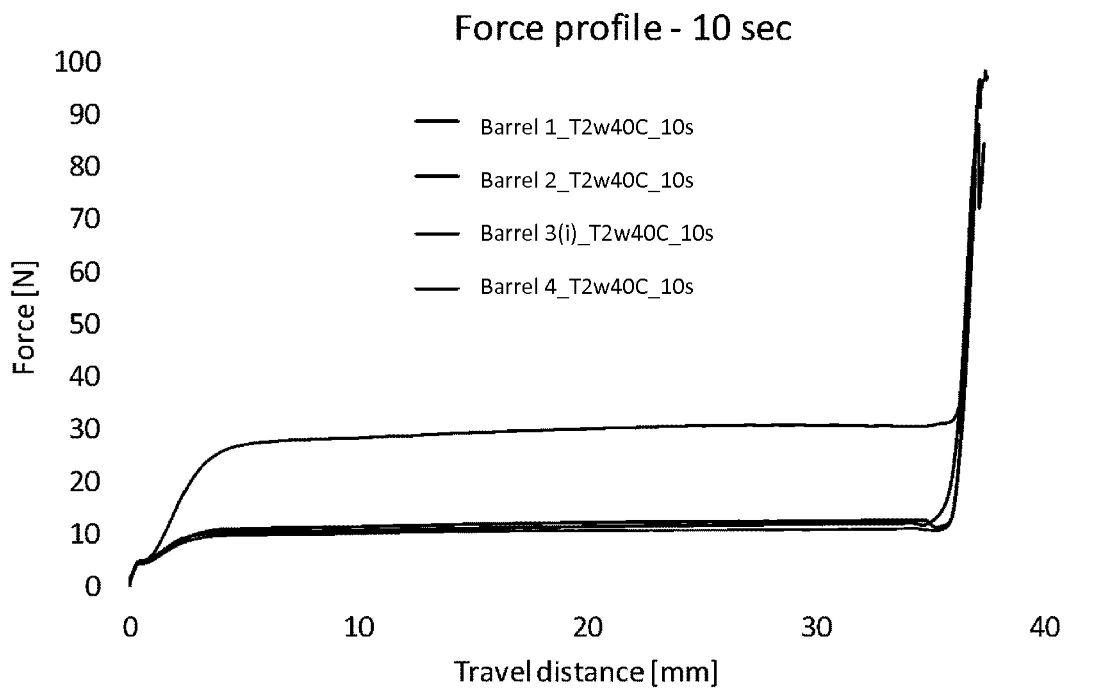


# FIG. 19D

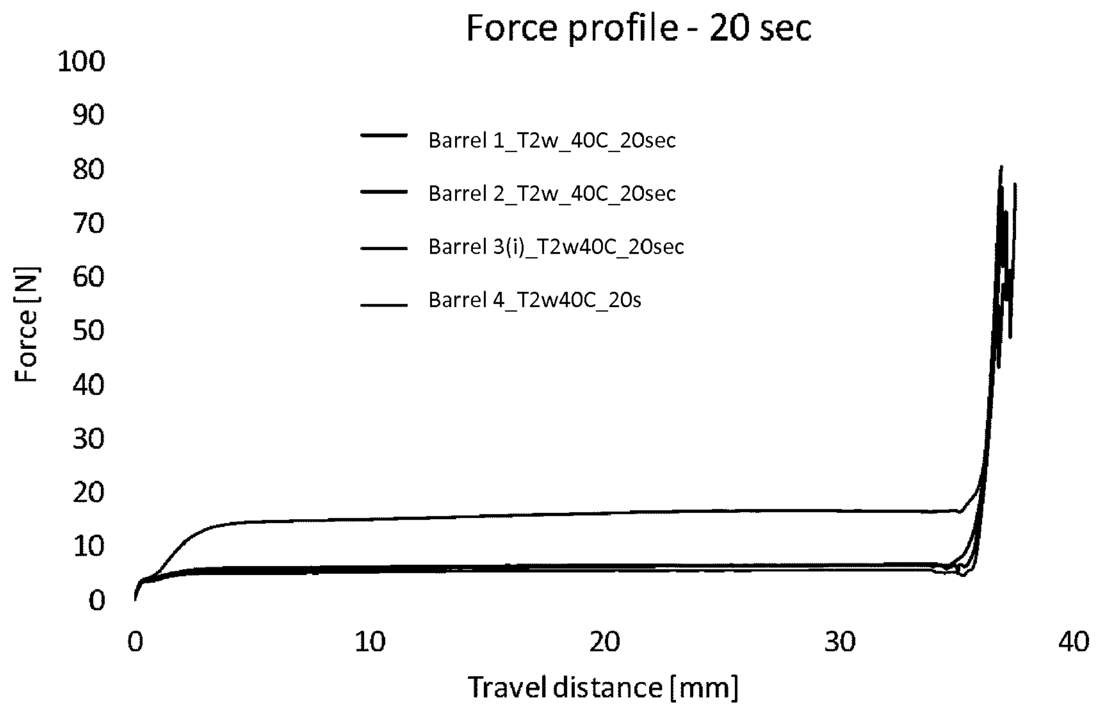

FIG. 20A

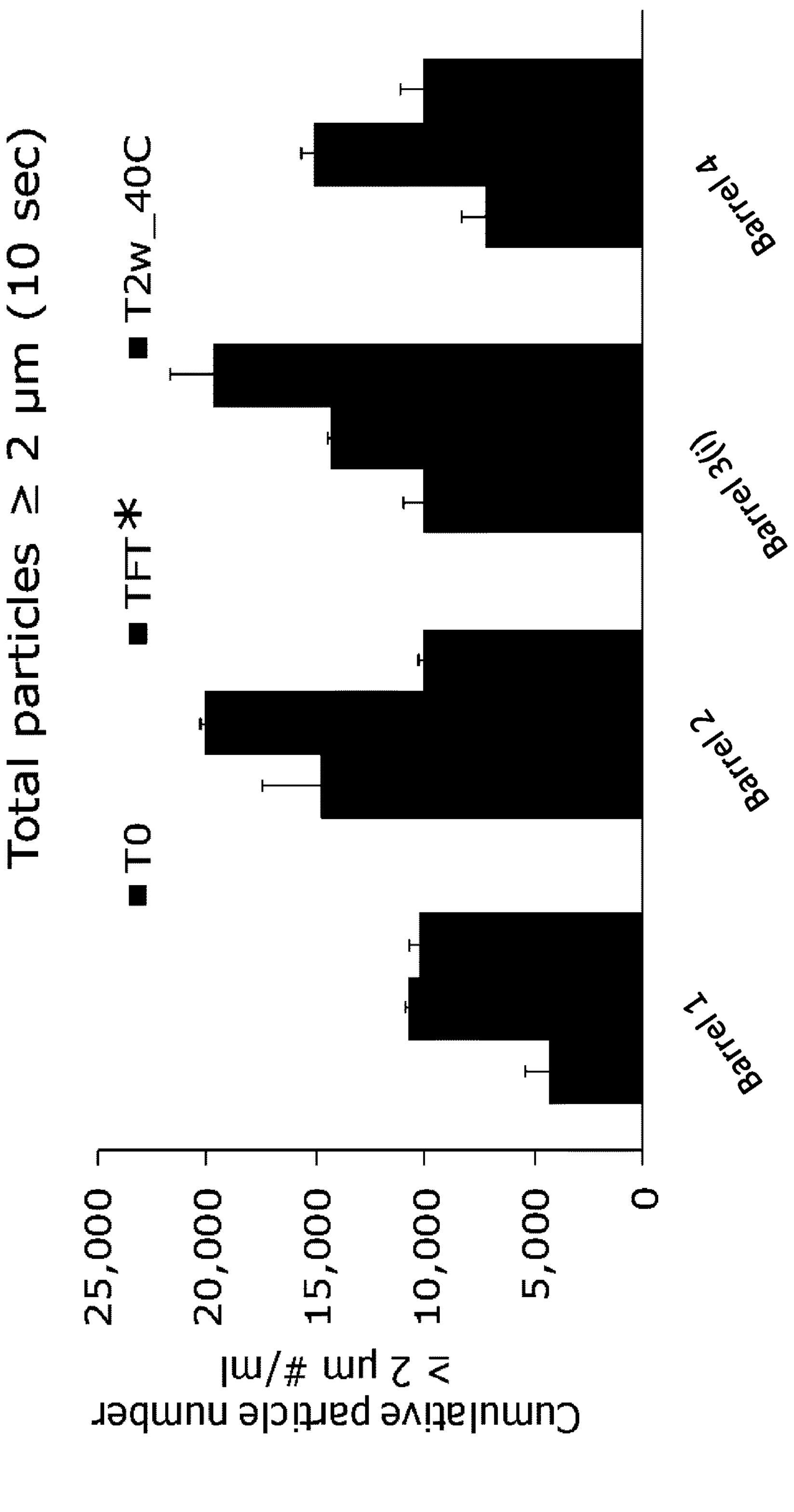
Total particles ≥ 2 µm (10 sec)
T0
TFT*
T2w_40C
Cumulative particle number ≥ 2 µm #/ml
25,000
20,000
15,000
10,000
5,000
0
Barrel 1
Barrel 2
Barrel 3(i)
Barrel 4

FIG. 20B

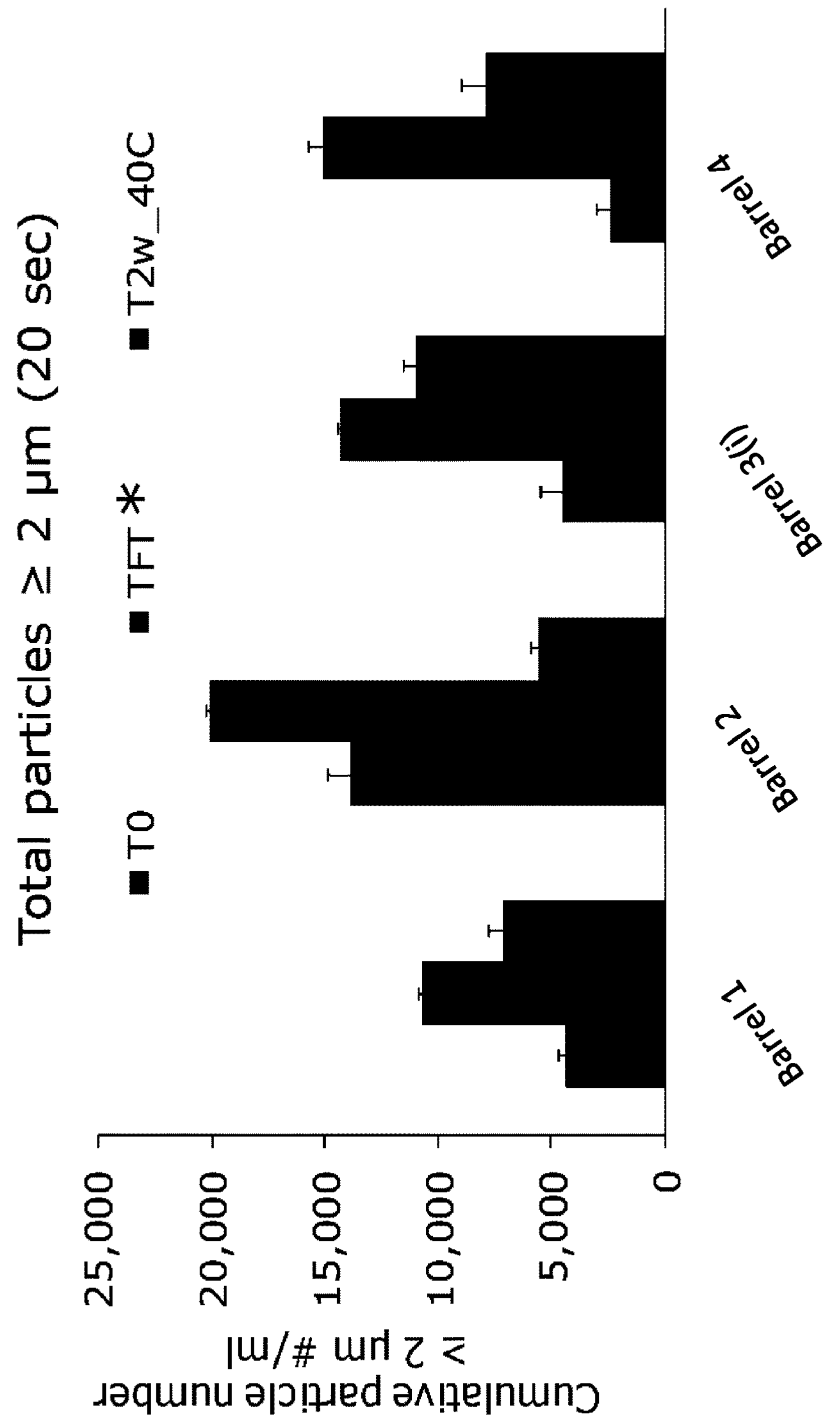
Total particles ≥ 2 µm (20 sec)
T0
TFT *
T2w_40C
Cumulative particle number
≥ 2 µm #/ml
25,000
20,000
15,000
10,000
5,000
0
Barrel 1
Barrel 2
Barrel 3(i)
Barrel 4

# FIG. 21A

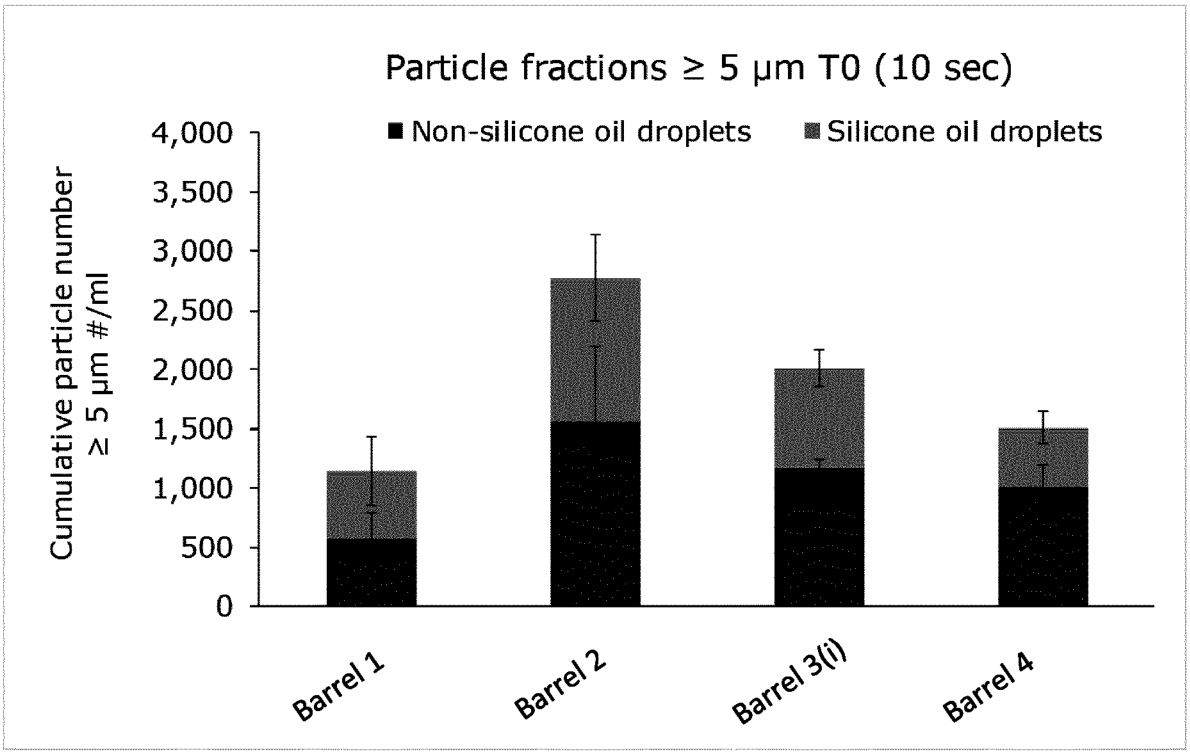


# FIG. 21B

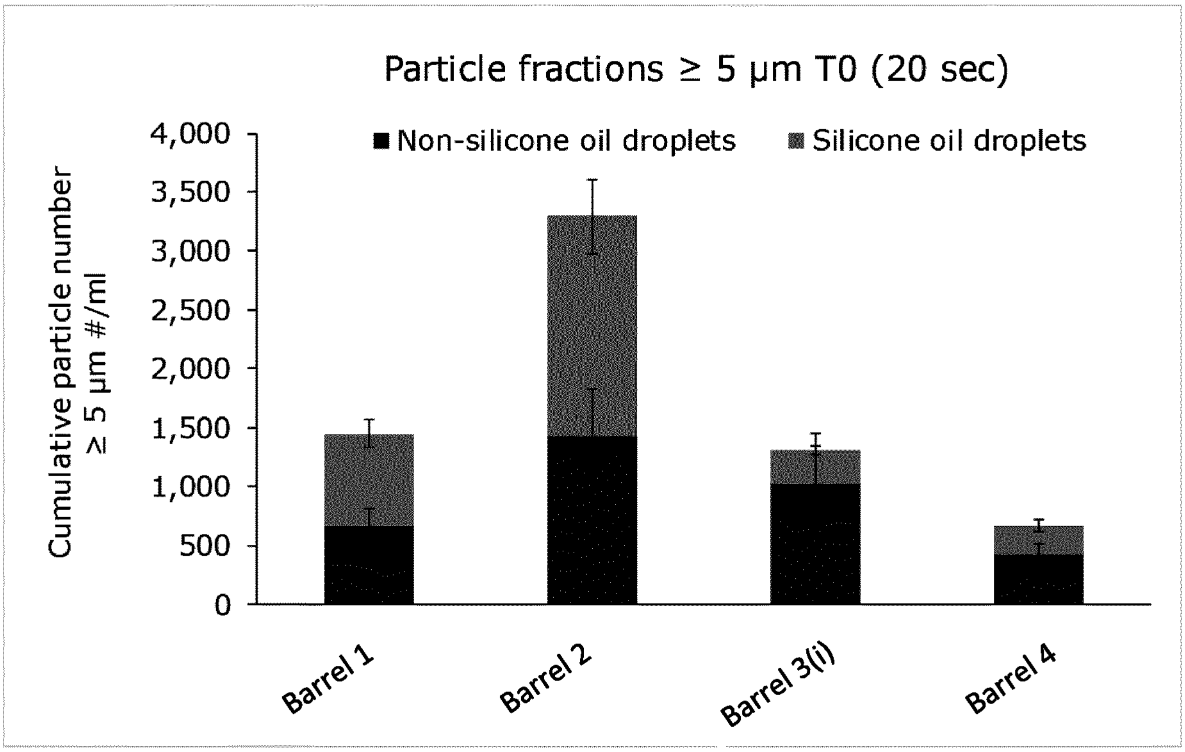

# FIG. 21C

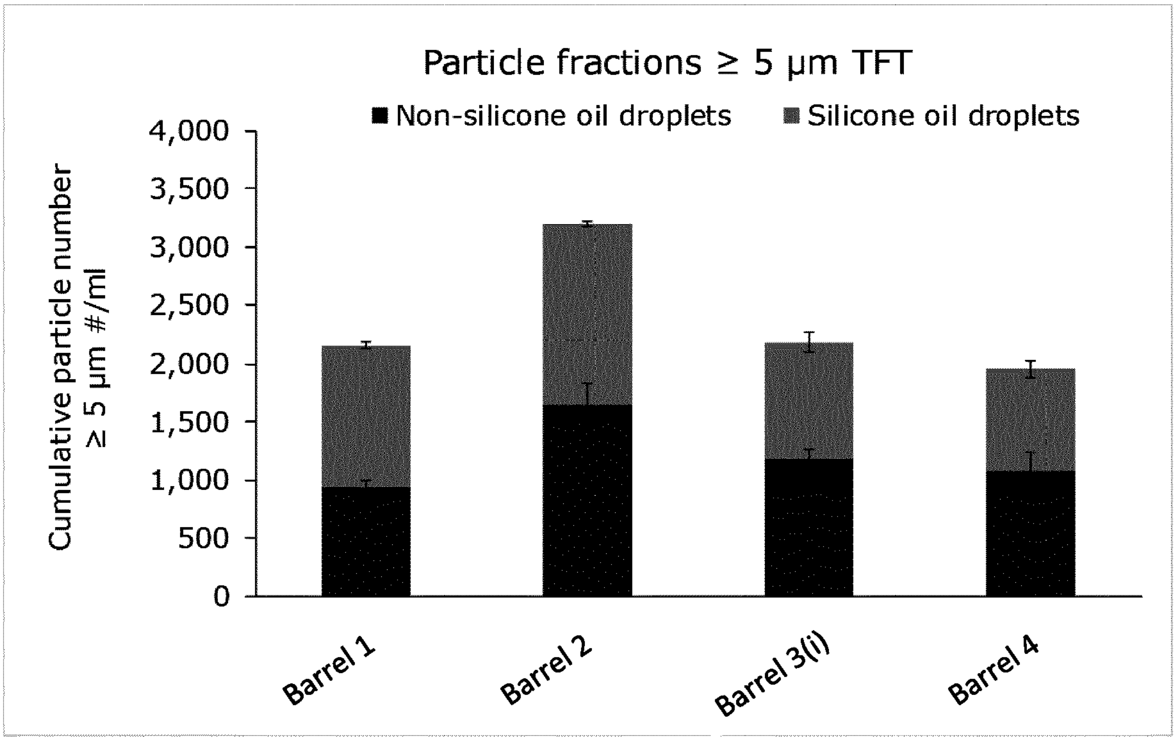


# FIG. 21D

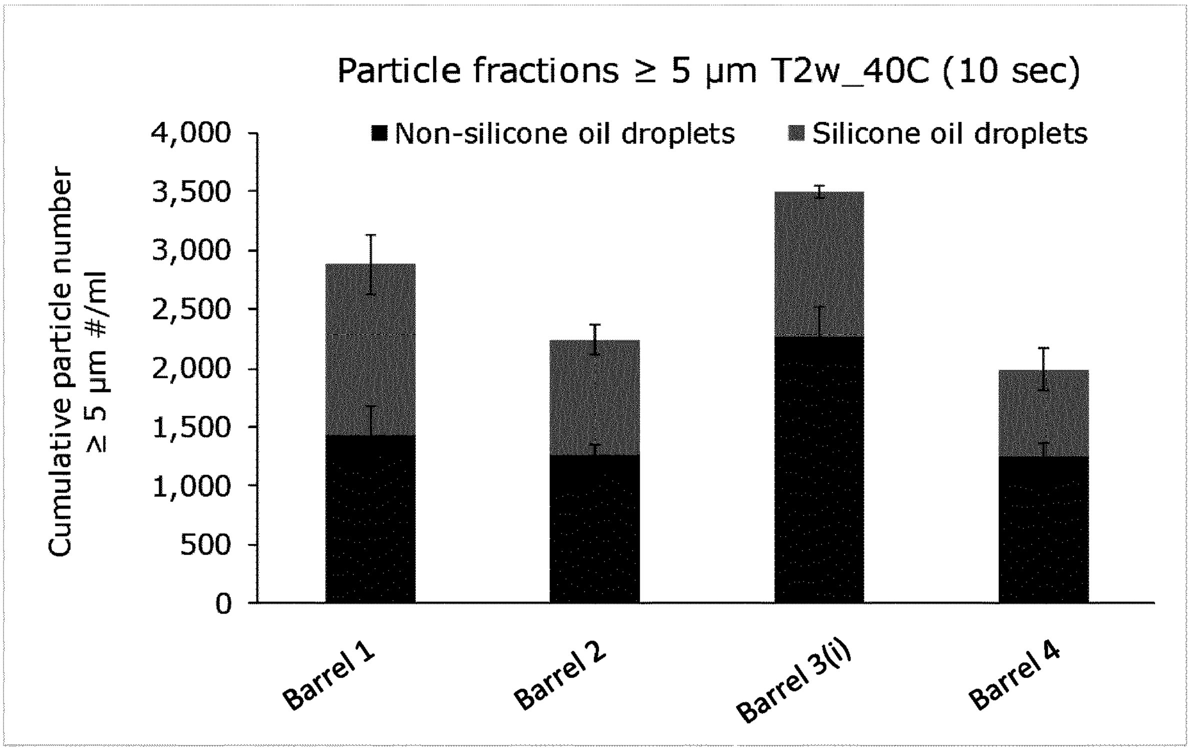

FIG. 21E

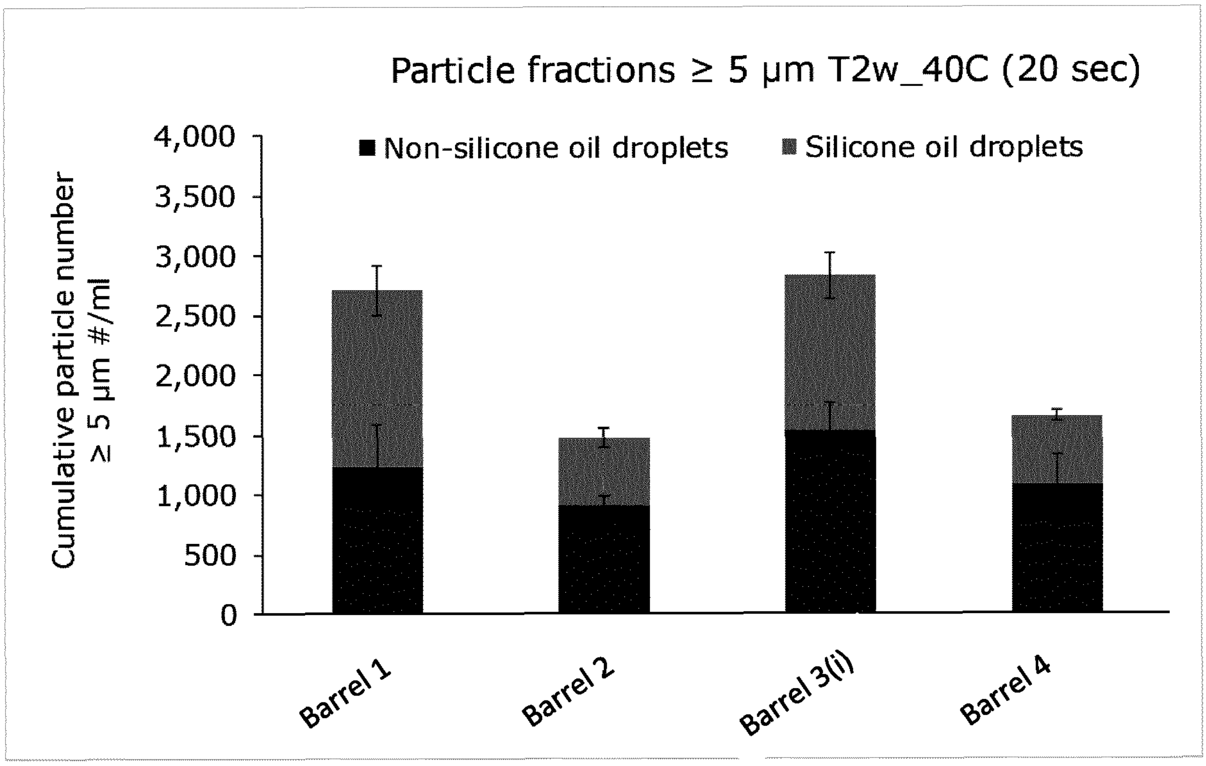


FIG. 22A

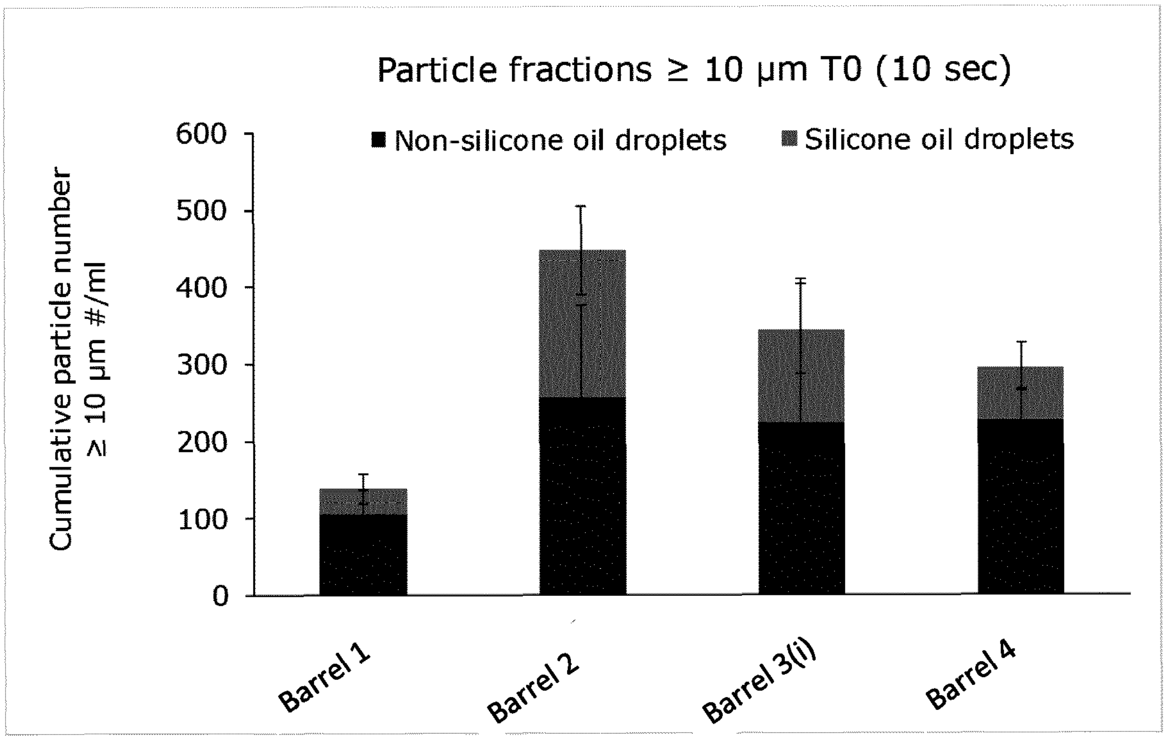

# FIG. 22B

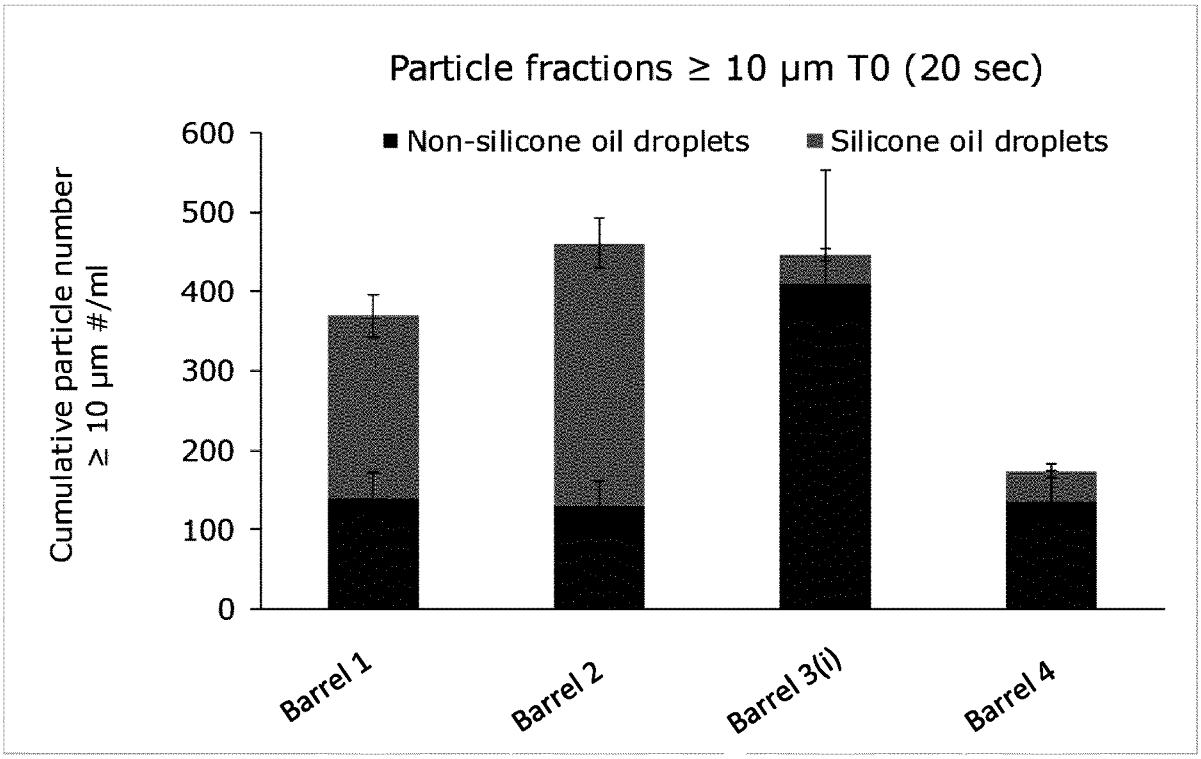


# FIG. 22C

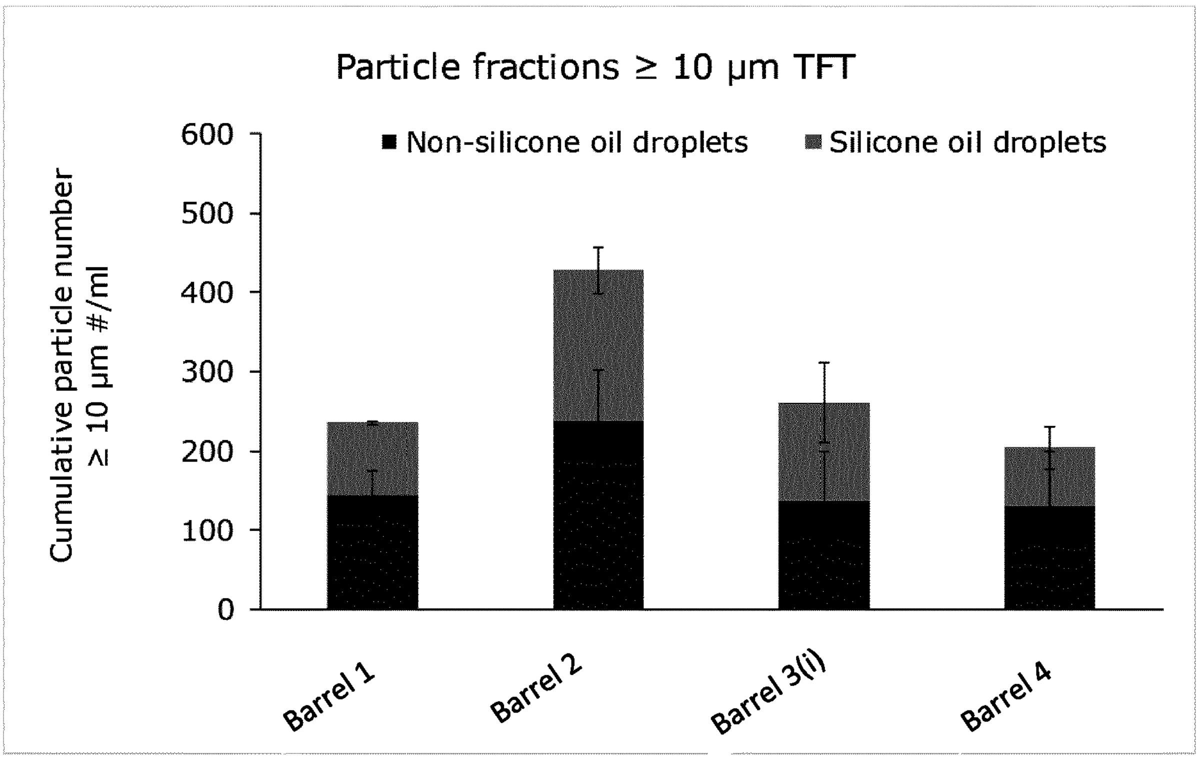

FIG. 22D

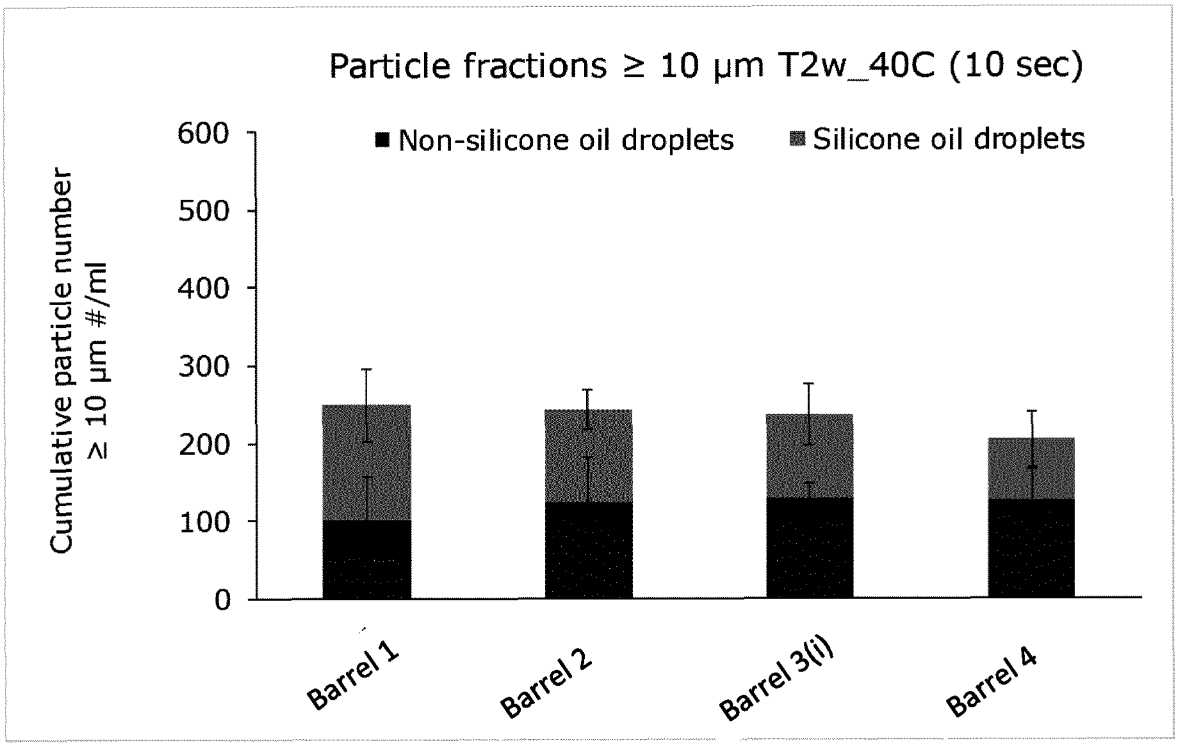


FIG. 22E

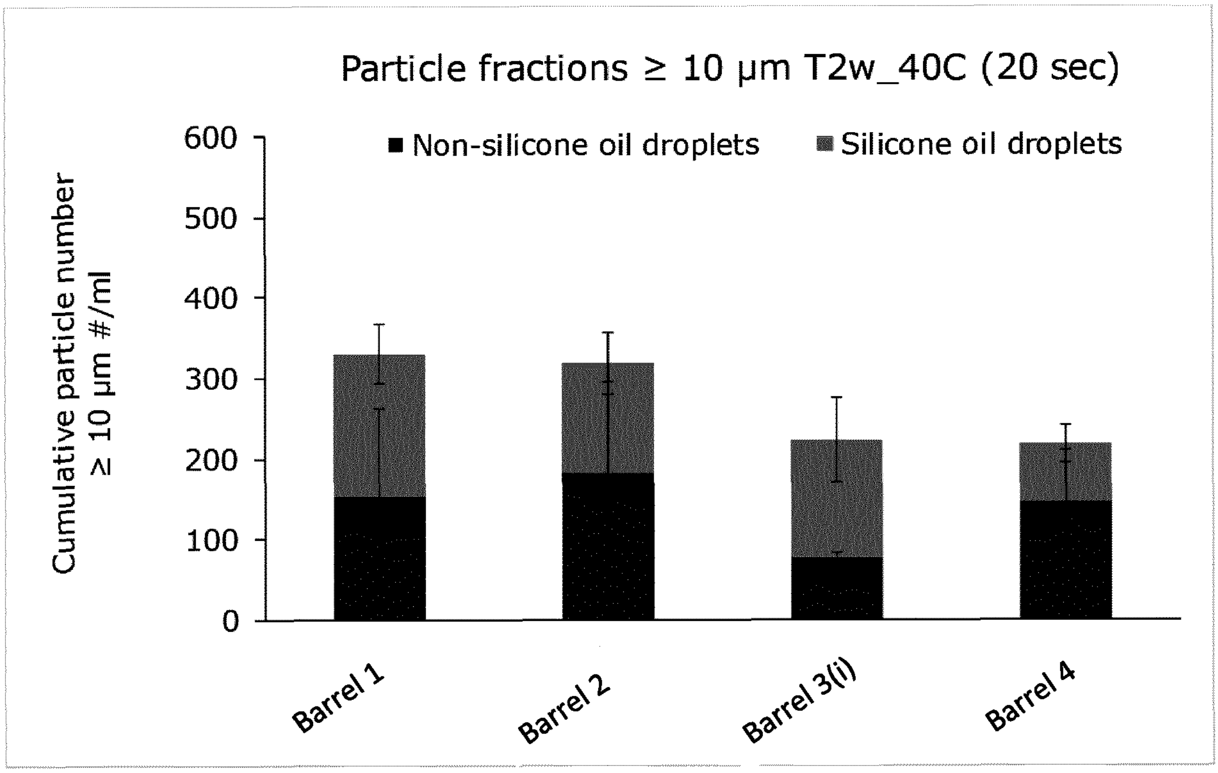

FIG. 23A

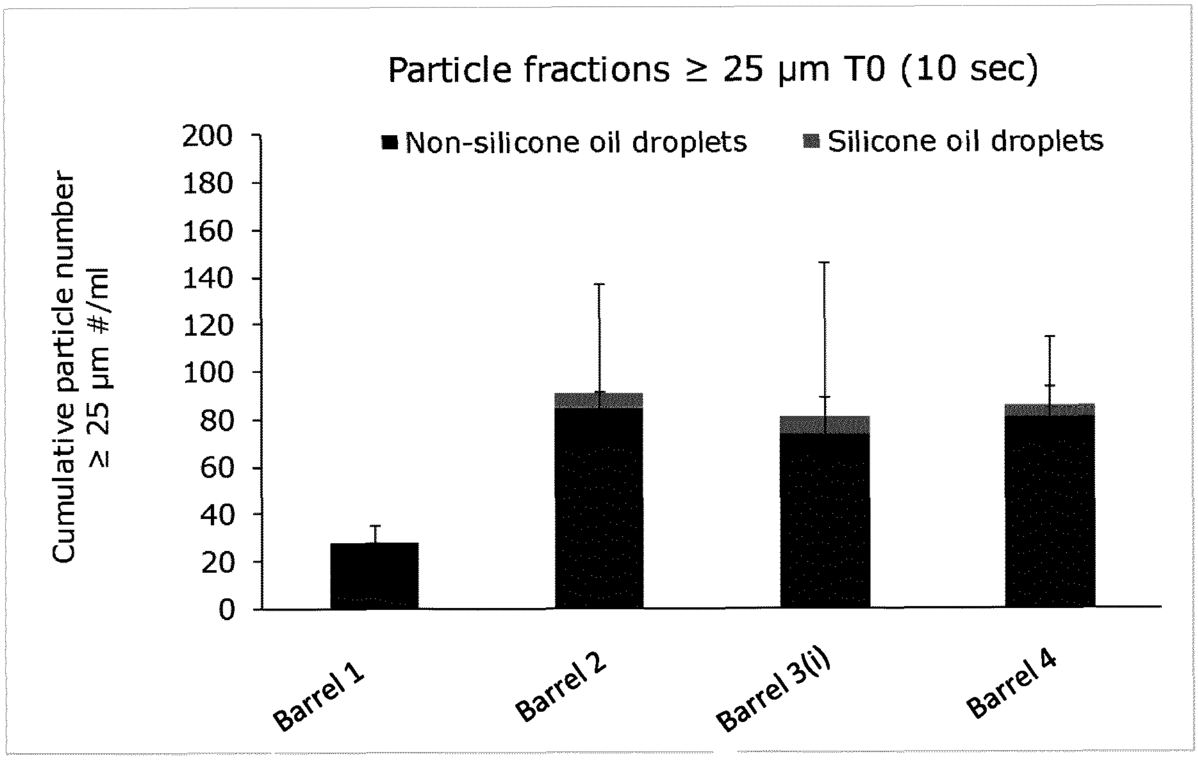


FIG. 23B

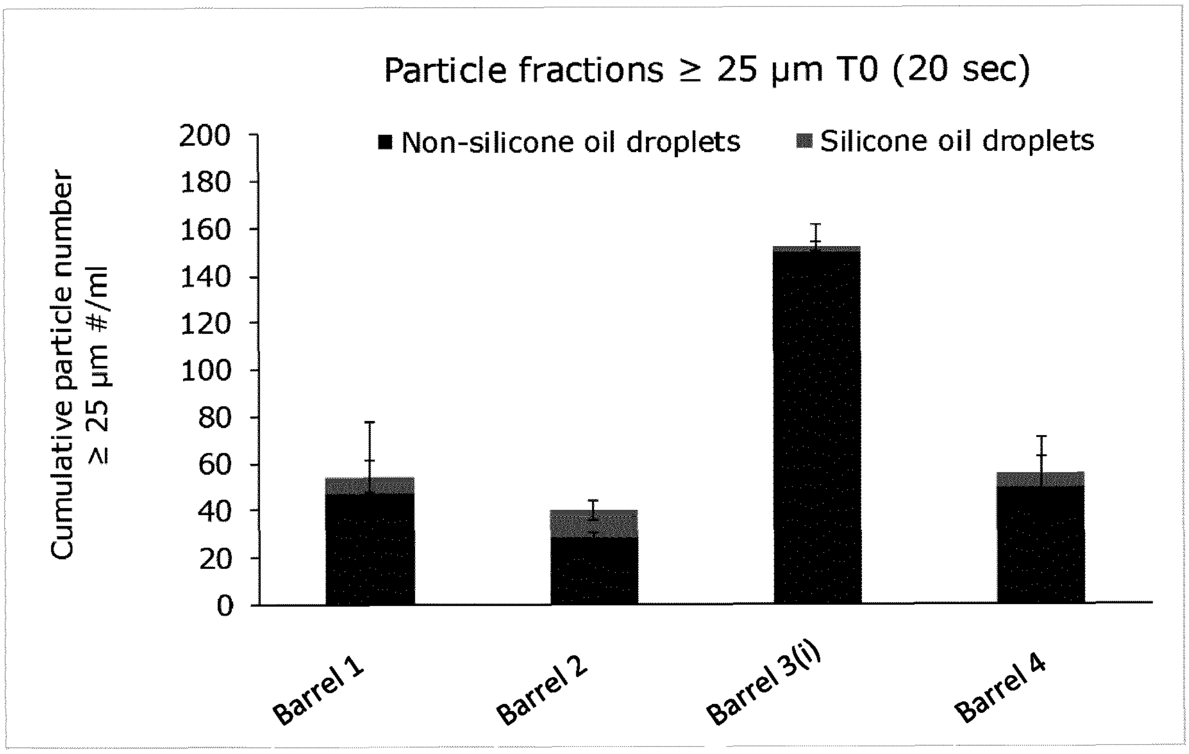

# FIG. 23C

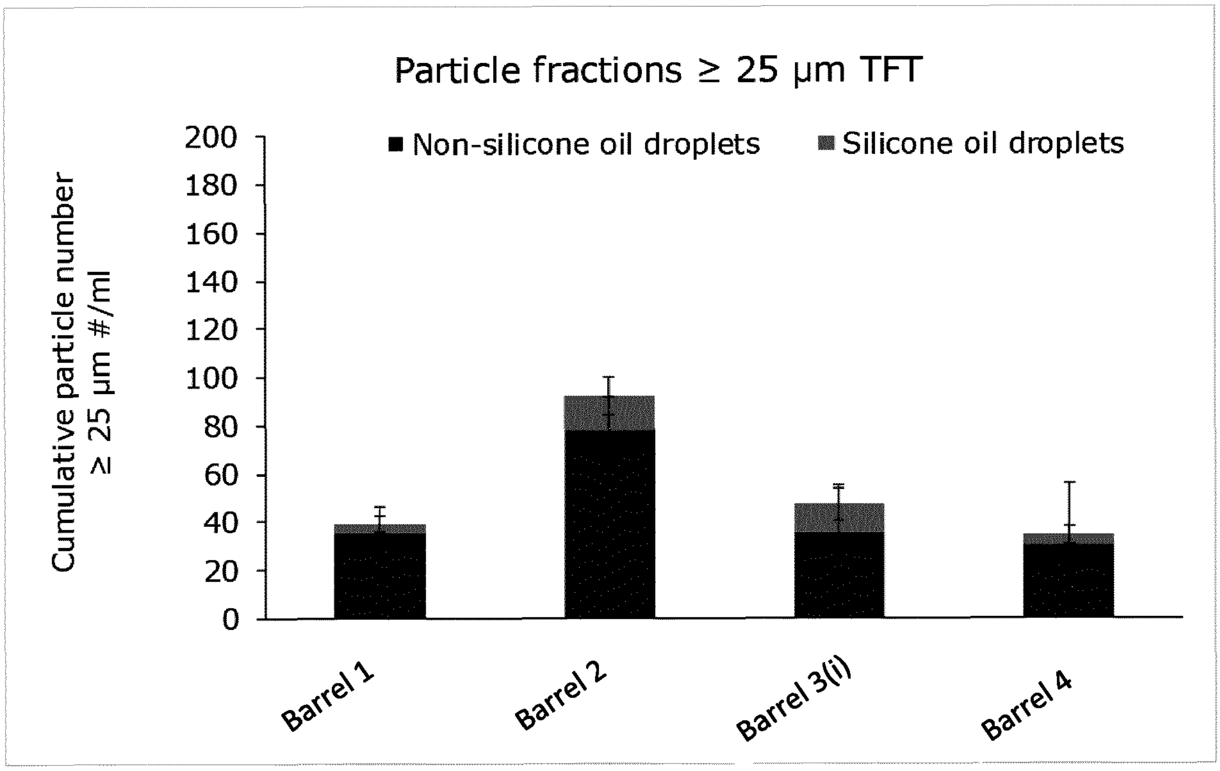


# FIG. 23D

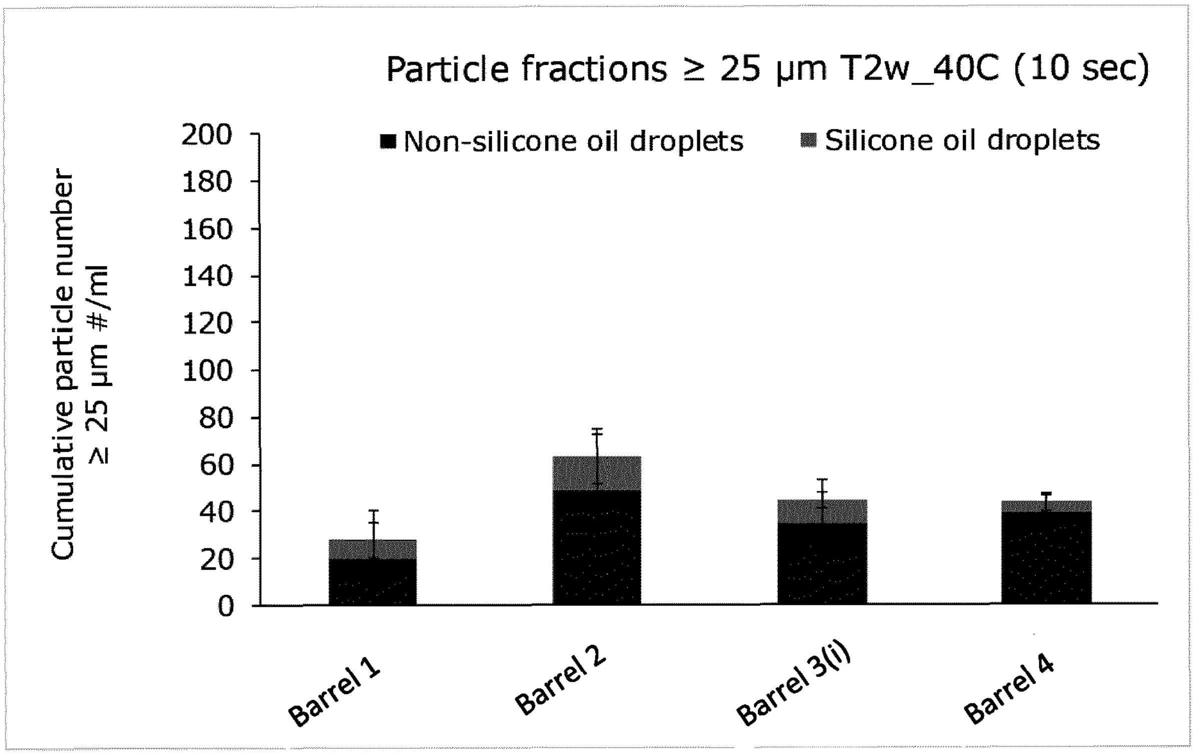

FIG. 23E

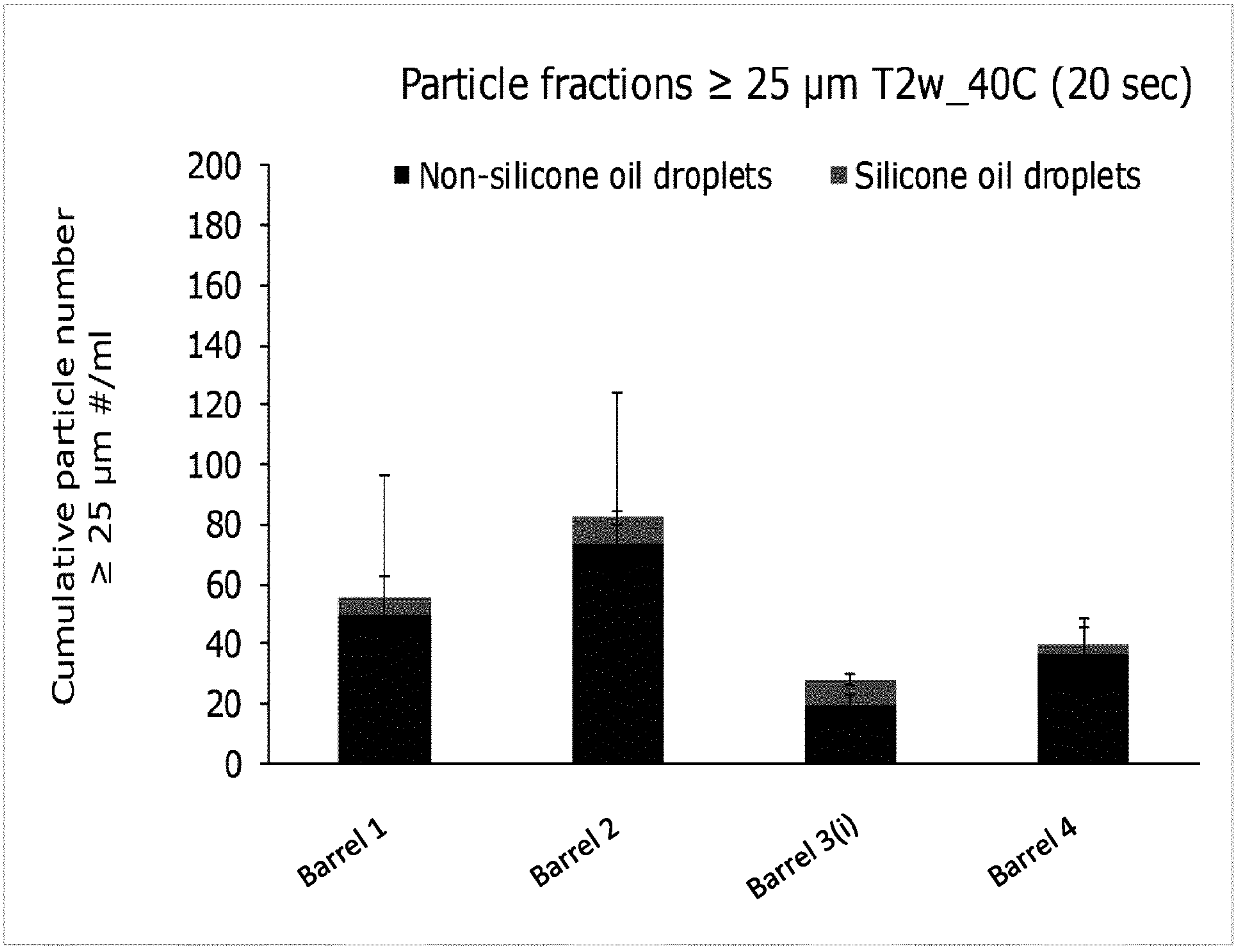
Particle fractions ≥ 25 µm T2w_40C (20 sec)
Non-silicone oil droplets
Silicone oil droplets
Cumulative particle number ≥ 25 µm #/ml
200
180
160
140
120
100
80
60
40
20
0
Barrel 1
Barrel 2
Barrel 3(i)
Barrel 4

FIG. 24A

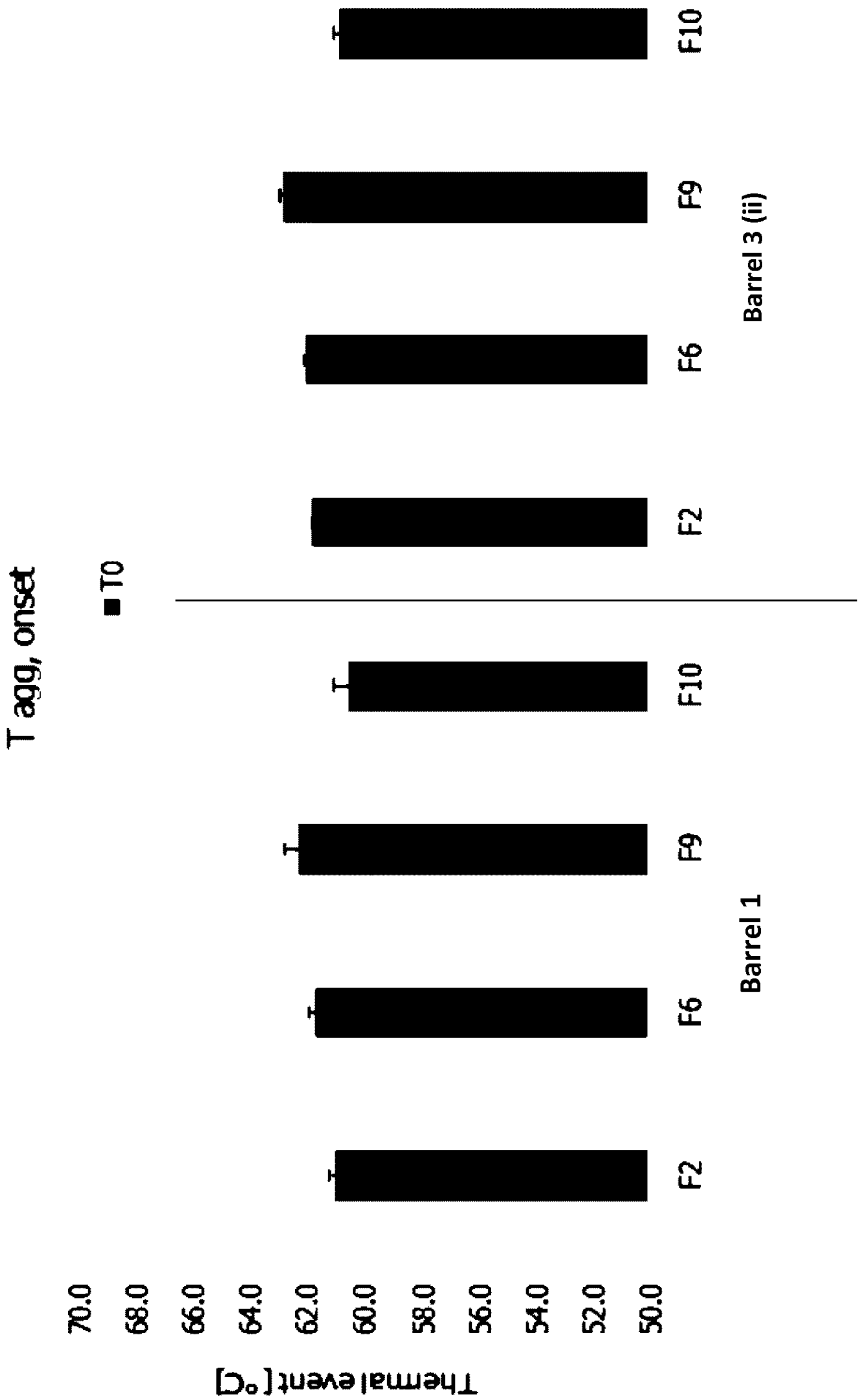
T agg, onset
T0
Themal event [°C]
70.0
68.0
66.0
64.0
62.0
60.0
58.0
56.0
54.0
52.0
50.0
F2
F6
F9
F10
Barrel 1
F2
F6
F9
F10
Barrel 3 (ii)

FIG. 24B

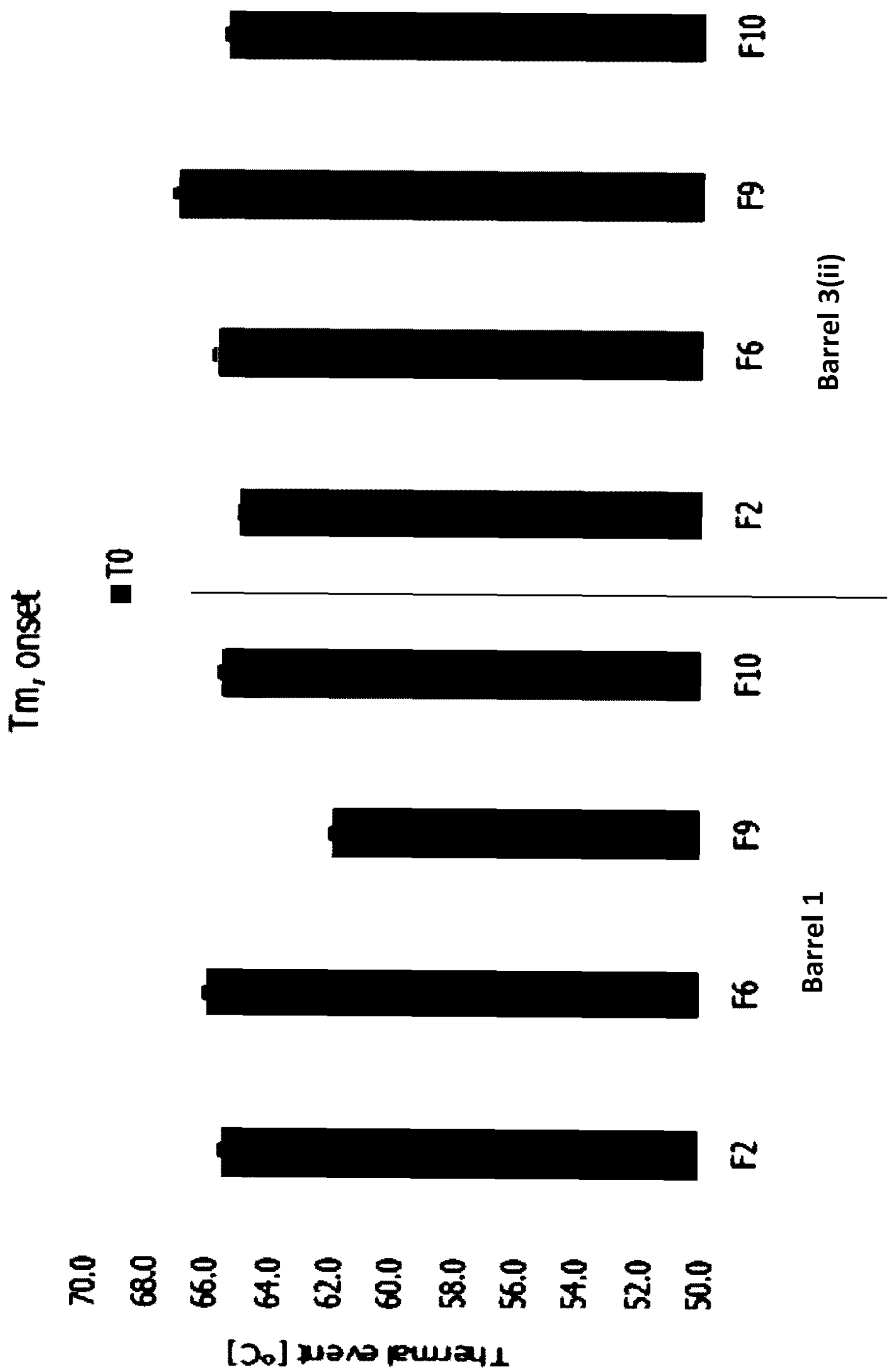
Tm, onset
T0
Thermal event [oC]
70.0
68.0
66.0
64.0
62.0
60.0
58.0
56.0
54.0
52.0
50.0
F2
F6
F9
F10
Barrel 1
F2
F6
F9
F10
Barrel 3(ii)

FIG. 24C

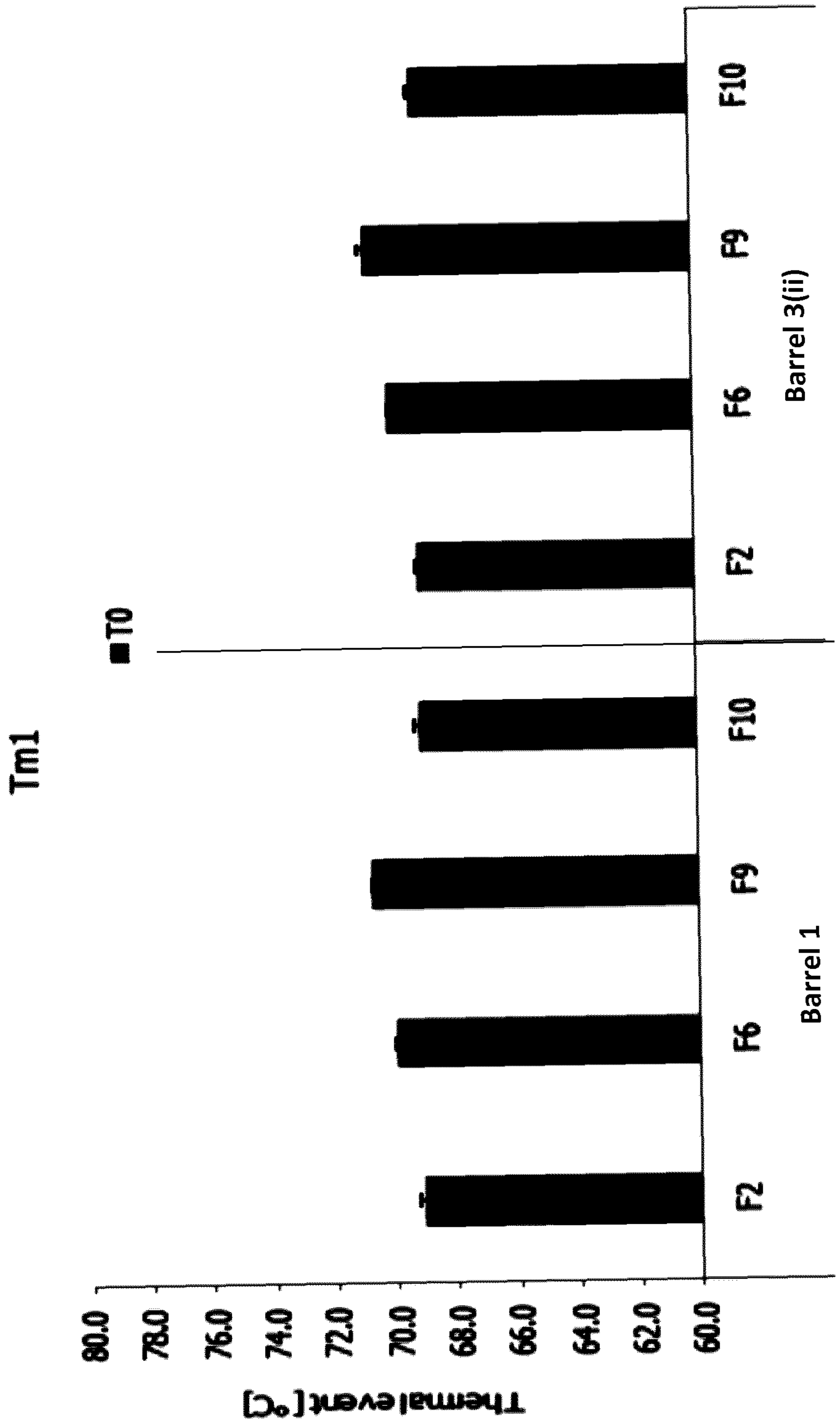
Tm1
T0
Themal event [°C]
80.0
78.0
76.0
74.0
72.0
70.0
68.0
66.0
64.0
62.0
60.0
F2
F6
F9
F10
Barrel 1
F2
F6
F9
F10
Barrel 3(ii)

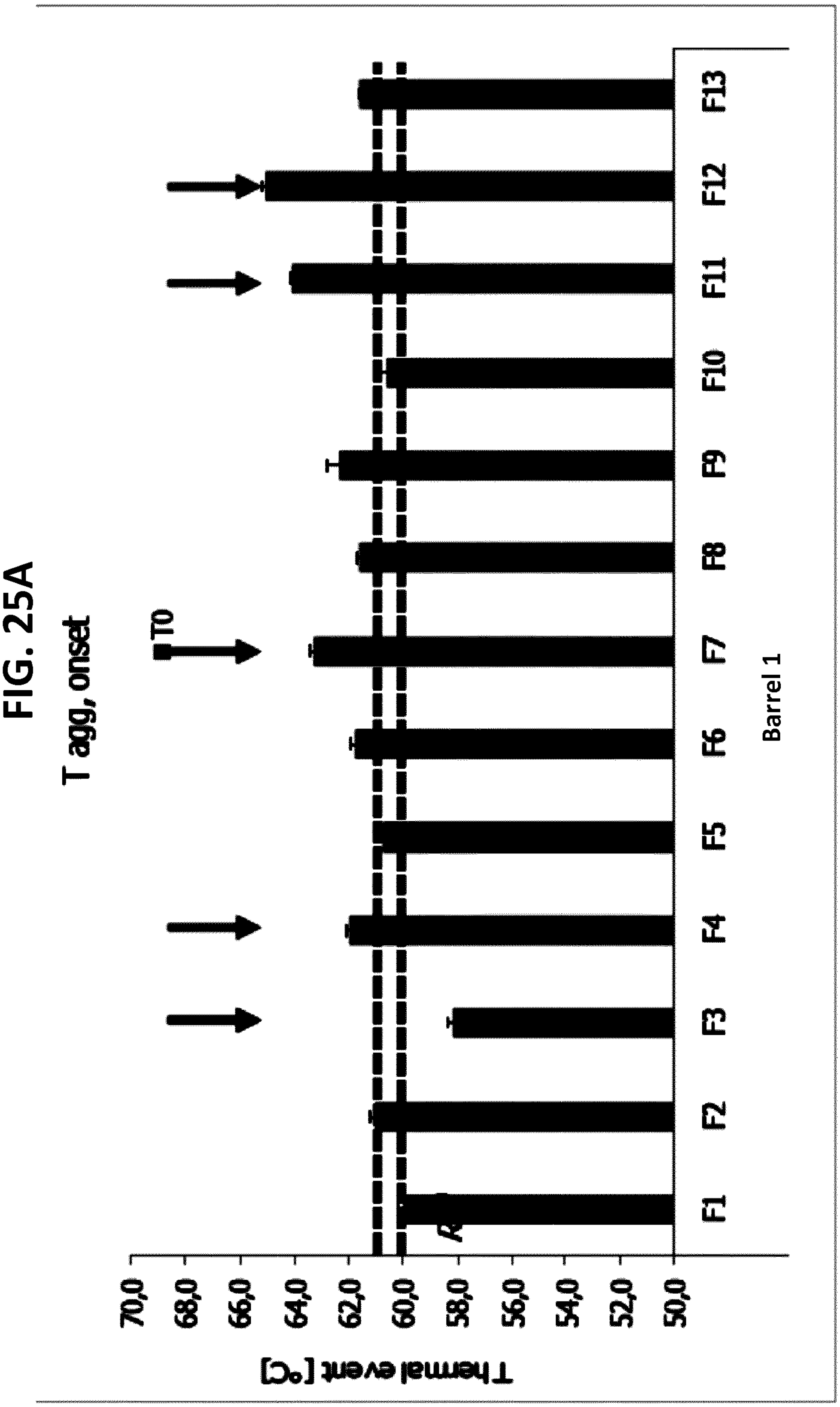
T agg, onset
T0
Thermal event [°C]
70,0
68,0
66,0
64,0
62,0
60,0
58,0
56,0
54,0
52,0
50,0
F1
F2
F3
F4
F5
F6
F7
F8
F9
F10
F11
F12
F13
Barrel 1

FIG. 25A

FIG. 25B

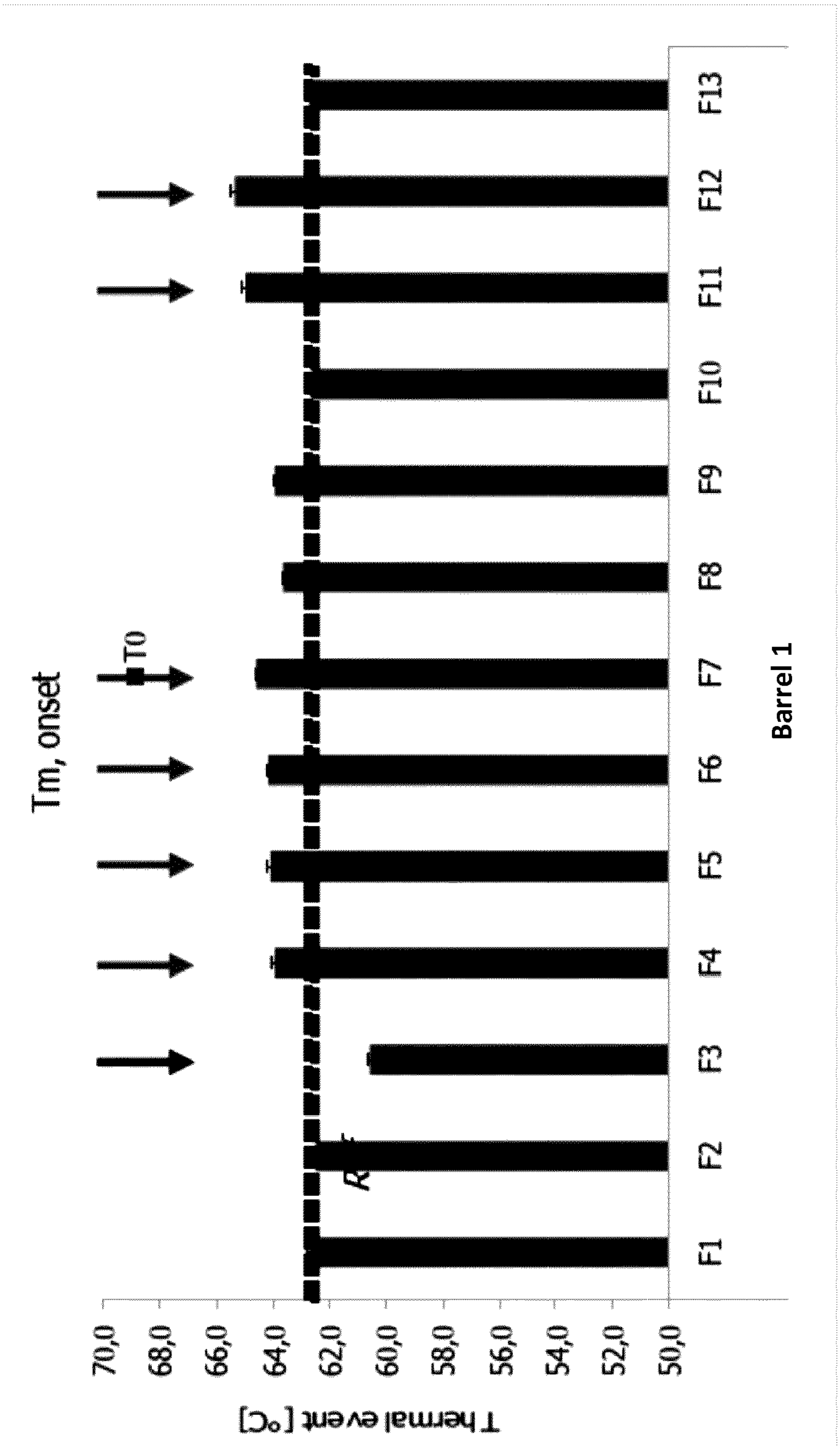
Tm, onset
T hermal event [°C]
70,0
68,0
66,0
64,0
62,0
60,0
58,0
56,0
54,0
52,0
50,0
T0
Ref
F1
F2
F3
F4
F5
F6
F7
F8
F9
F10
F11
F12
F13
Barrel 1

FIG. 25C

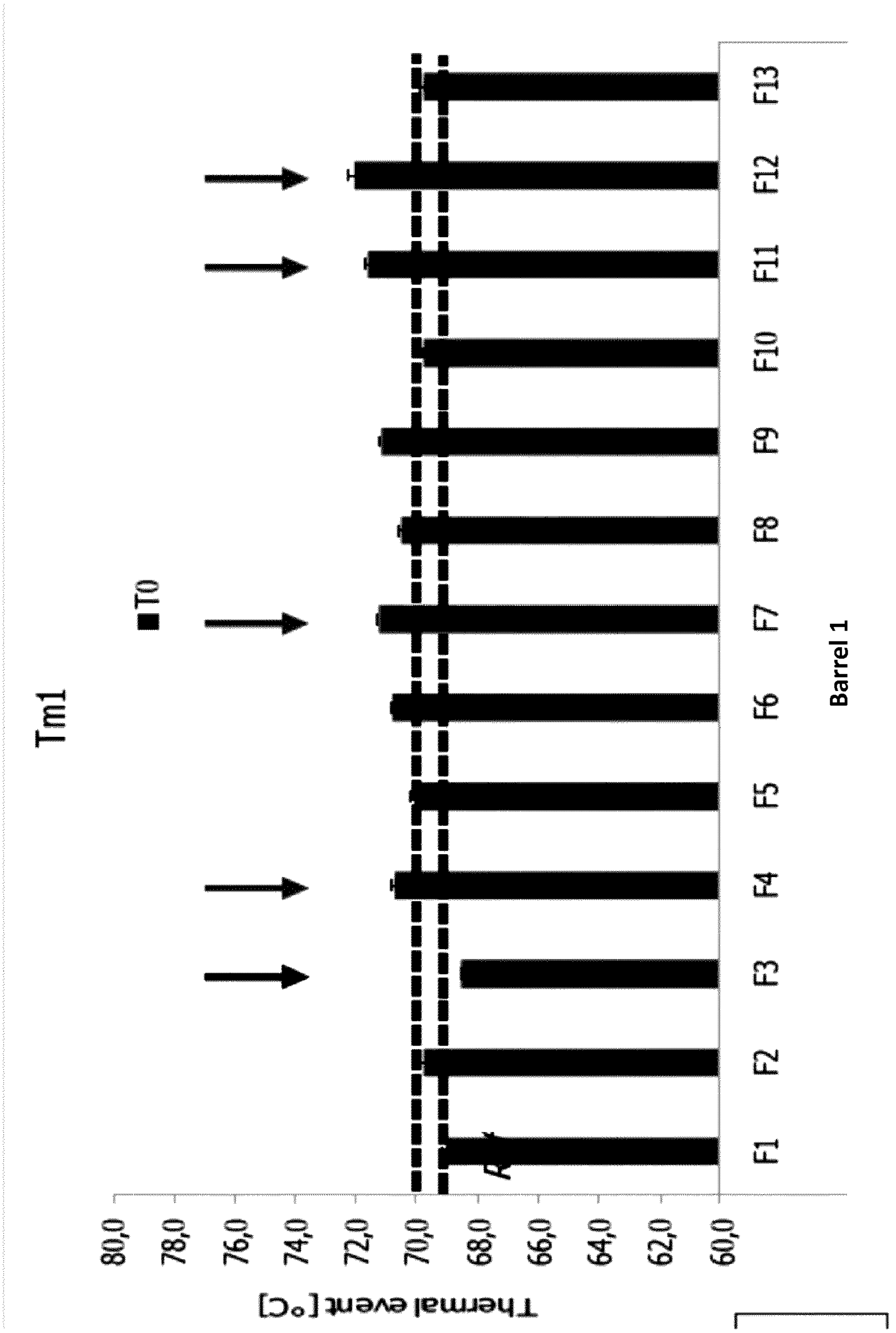
Tm1
T0
Thermal event [°C]
80,0
78,0
76,0
74,0
72,0
70,0
68,0
66,0
64,0
62,0
60,0
F1
F2
F3
F4
F5
F6
F7
F8
F9
F10
F11
F12
F13
Barrel 1

FIG. 27A

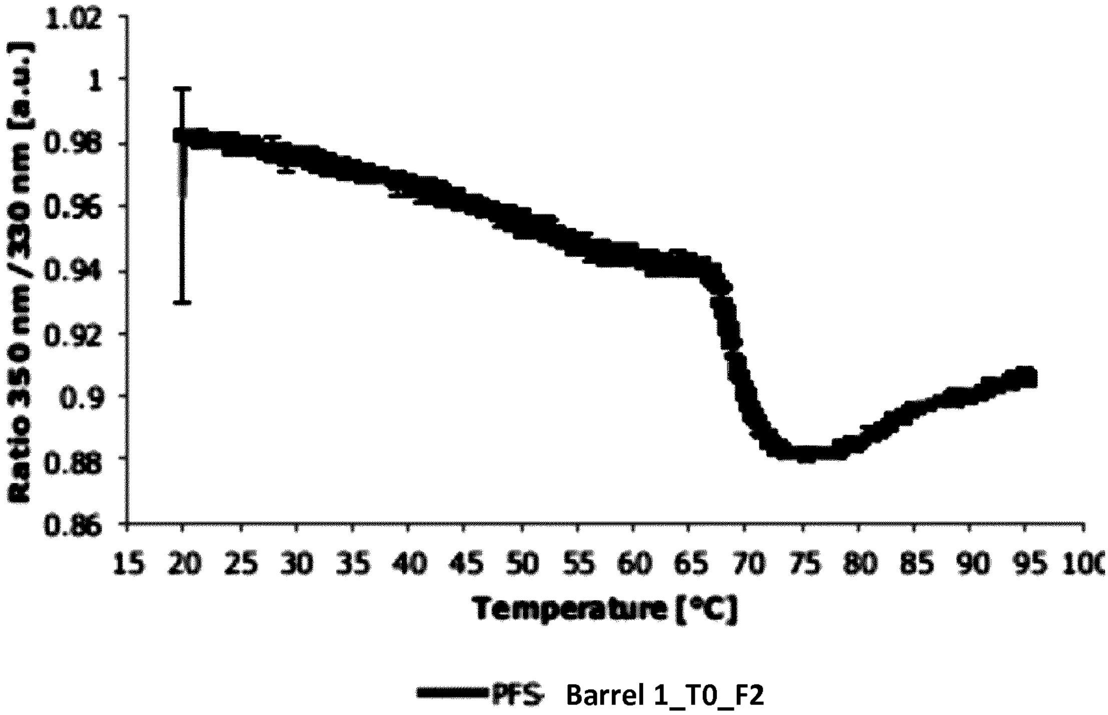
Ratio 350 nm/330 nm [a.u.]
1.02
1
0.98
0.96
0.94
0.92
0.9
0.88
0.86
15 20 25 30 35 40 45 50 55 60 65 70 75 80 85 90 95 100
Temperature [°C]
PFS Barrel 1_T0_F2

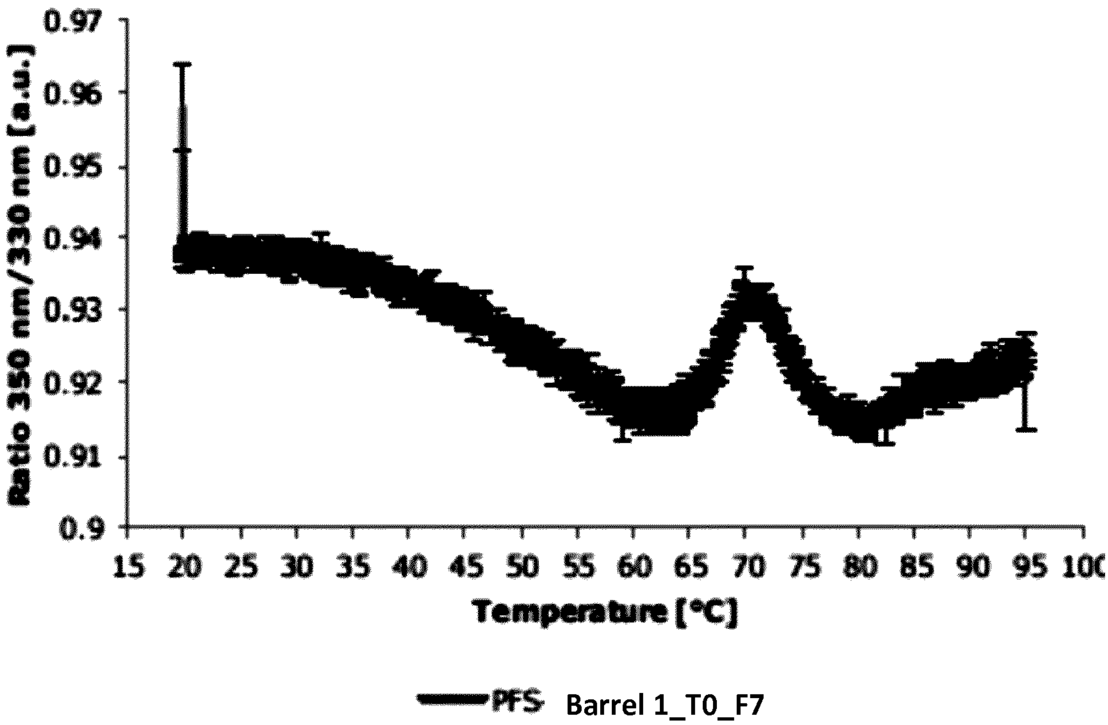
Ratio 350 nm/330 nm [a.u.]
0.97
0.96
0.95
0.94
0.93
0.92
0.91
0.9
15 20 25 30 35 40 45 50 55 60 65 70 75 80 85 90 95 100
Temperature [°C]
PFS Barrel 1_T0_F7

FIG. 26B

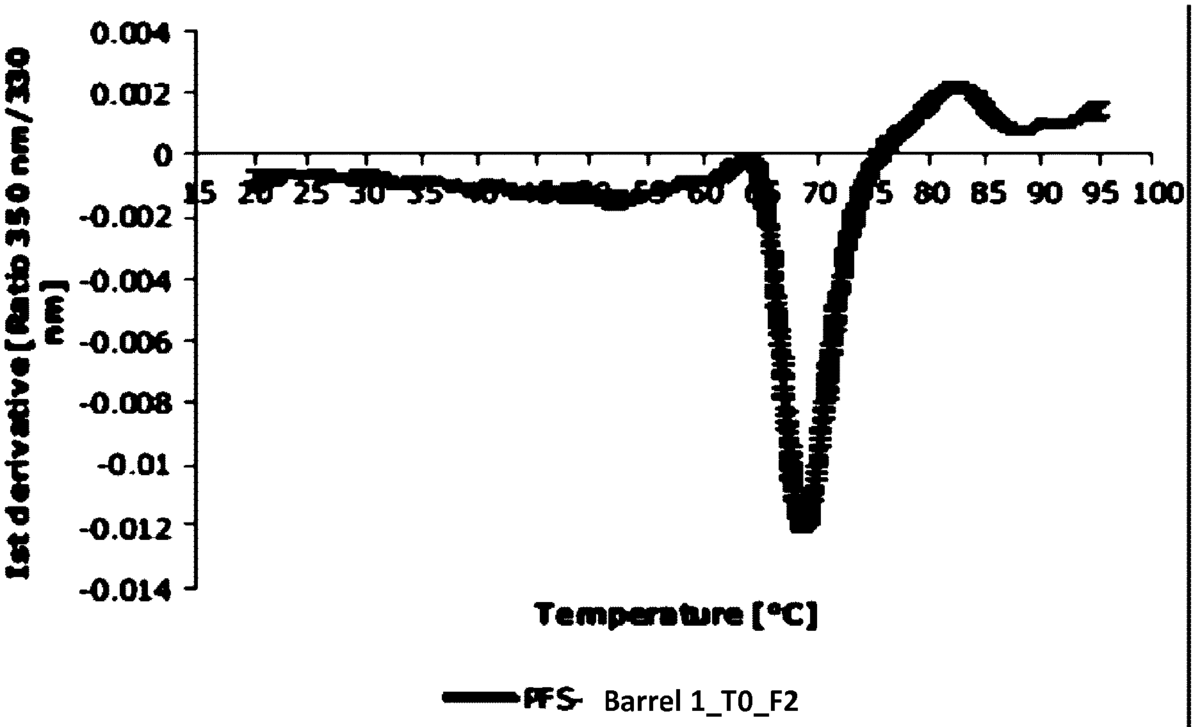

0.004
0.002
0
-0.002
-0.004
-0.006
-0.008
-0.01
-0.012
-0.014
15 20 25 30 35 40 45 50 55 60 65 70 75 80 85 90 95 100
1st derivative [Ratio 350 nm/330 nm]
Temperature [°C]
PFS- Barrel 1_T0_F2


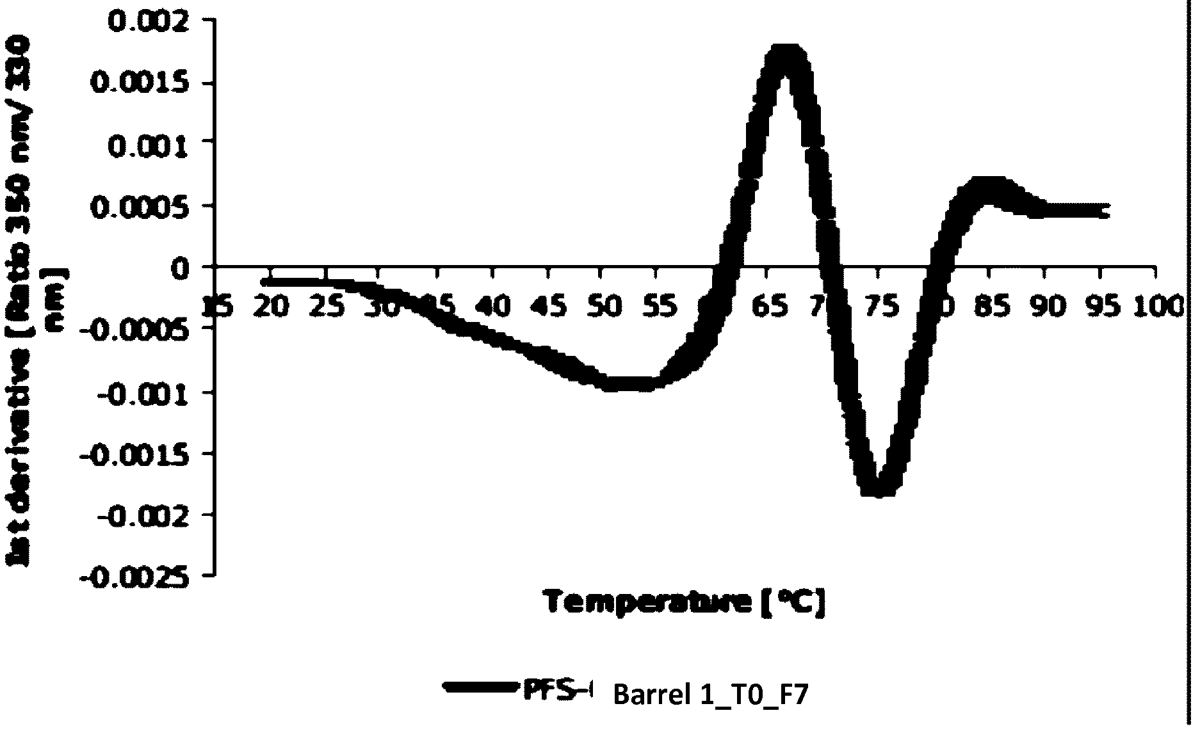

0.002
0.0015
0.001
0.0005
0
-0.0005
-0.001
-0.0015
-0.002
-0.0025
15 20 25 30 35 40 45 50 55 60 65 70 75 80 85 90 95 100
1st derivative [Ratio 350 nm/330 nm]
Temperature [°C]
PFS- Barrel 1_T0_F7

FIG. 26C

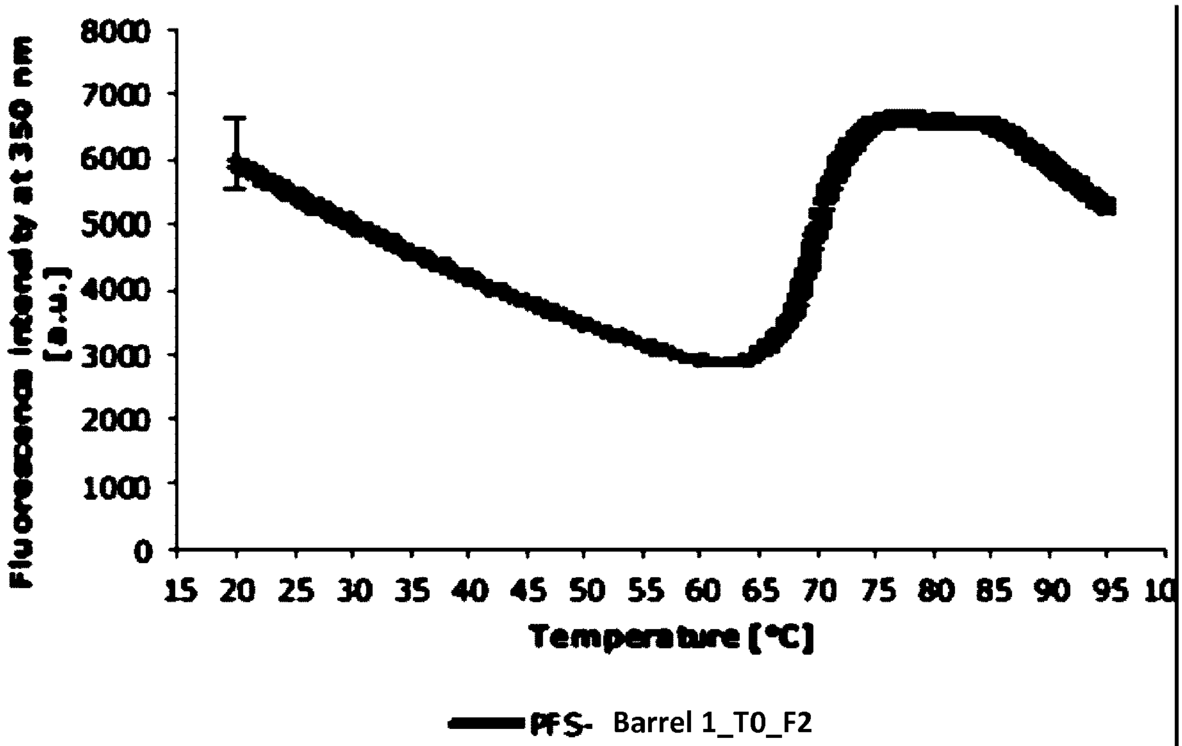
8000
7000
6000
5000
4000
3000
2000
1000
0
Fluorescence intensity at 350 nm [a.u.]
15 20 25 30 35 40 45 50 55 60 65 70 75 80 85 90 95 10
Temperature [°C]
PFS- Barrel 1_T0_F2

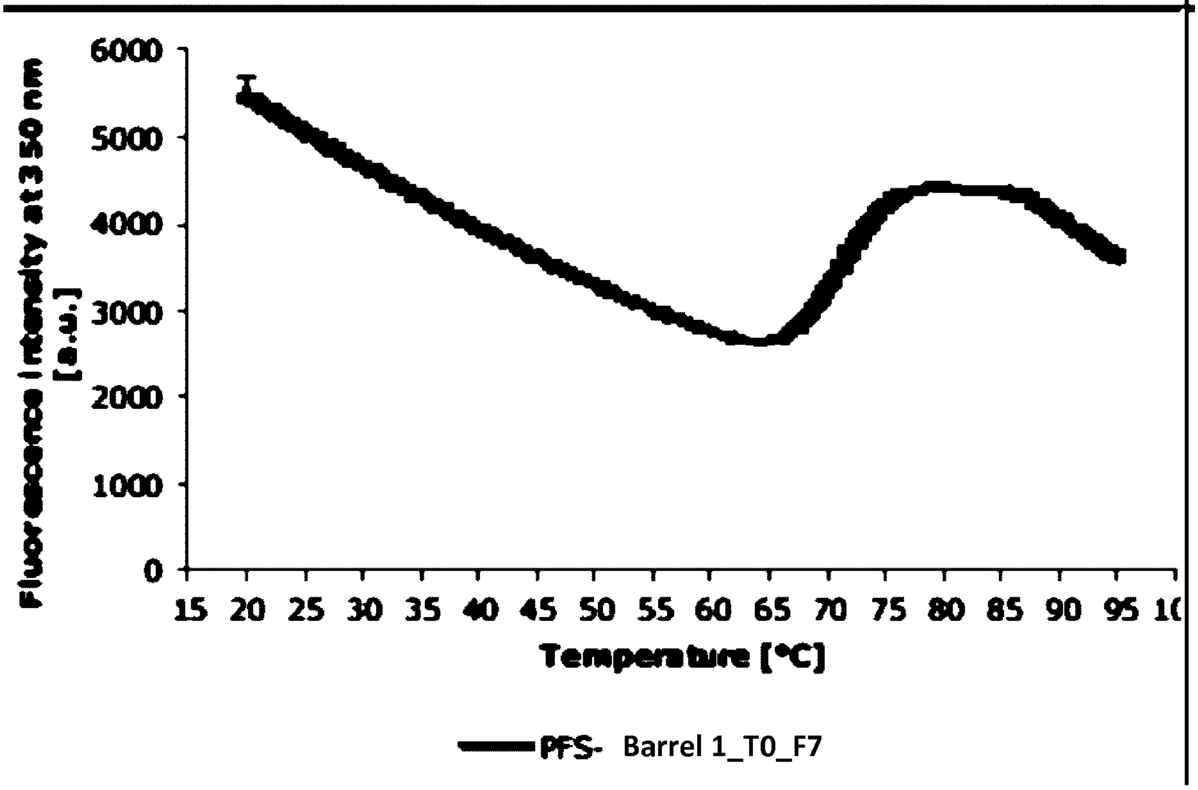
6000
5000
4000
3000
2000
1000
0
Fluorescence intensity at 350 nm [a.u.]
15 20 25 30 35 40 45 50 55 60 65 70 75 80 85 90 95 10
Temperature [°C]
PFS- Barrel 1_T0_F7

FIG. 26D

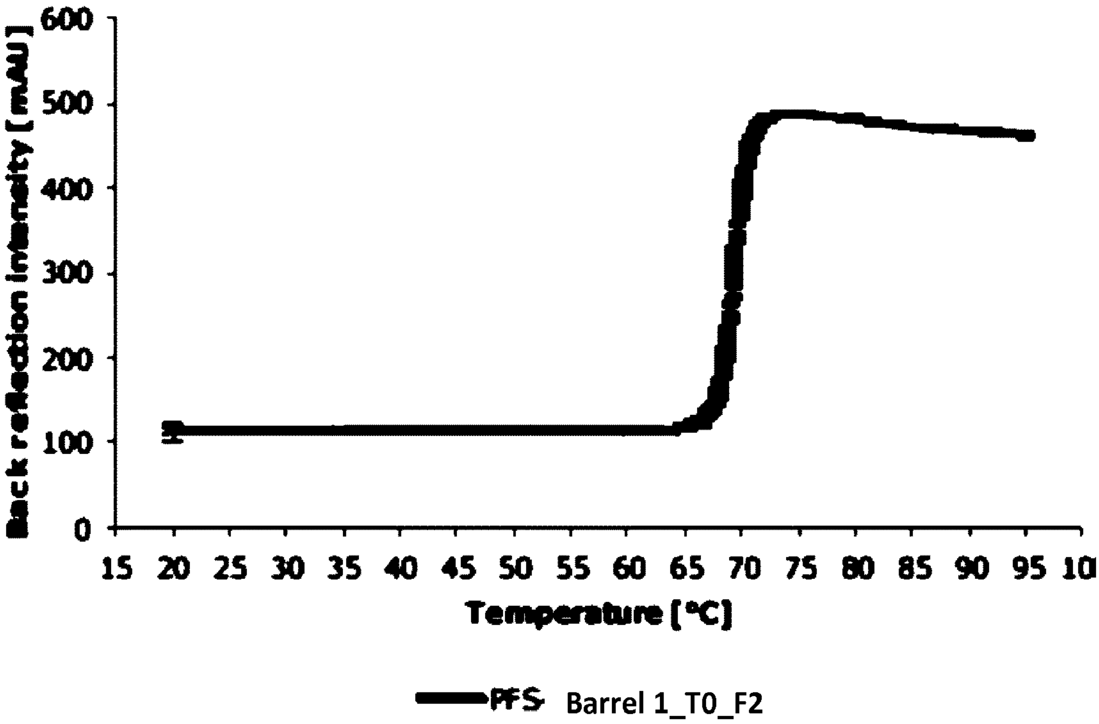
600
500
400
300
200
100
0
Back reflection intensity [mAU]
15 20 25 30 35 40 45 50 55 60 65 70 75 80 85 90 95 10
Temperature [°C]
PFS Barrel 1_T0_F2

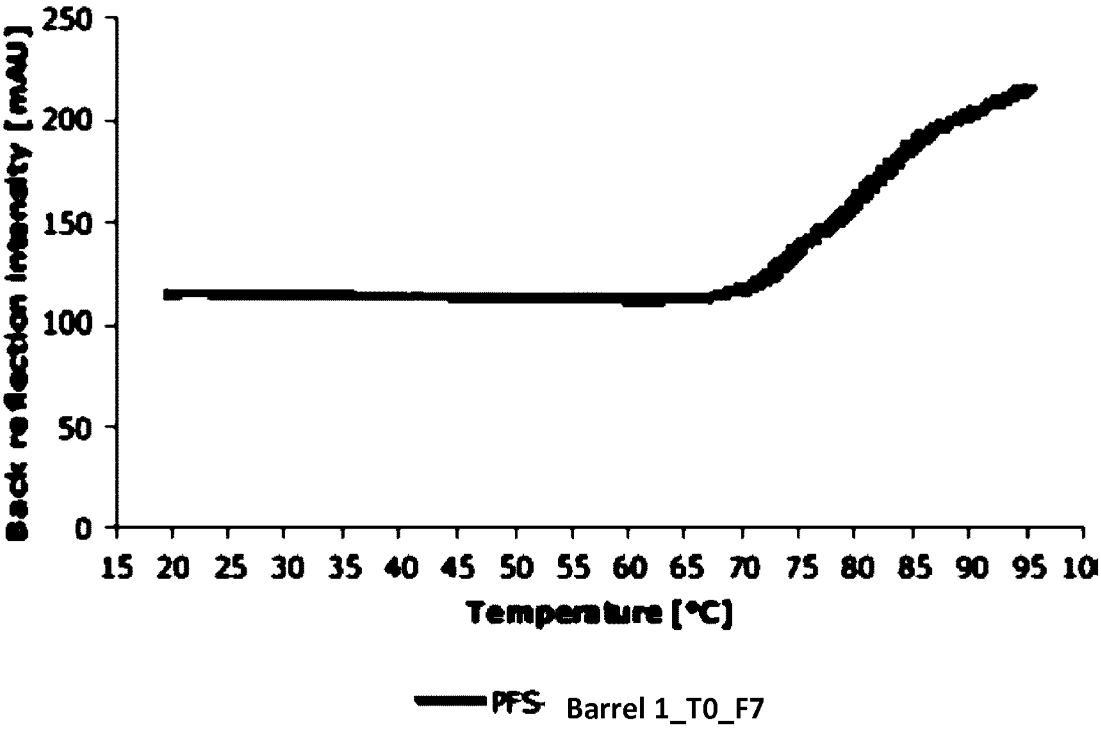
250
200
150
100
50
0
Back reflection intensity [mAU]
15 20 25 30 35 40 45 50 55 60 65 70 75 80 85 90 95 10
Temperature [°C]
PFS Barrel 1_T0_F7

FIG. 27

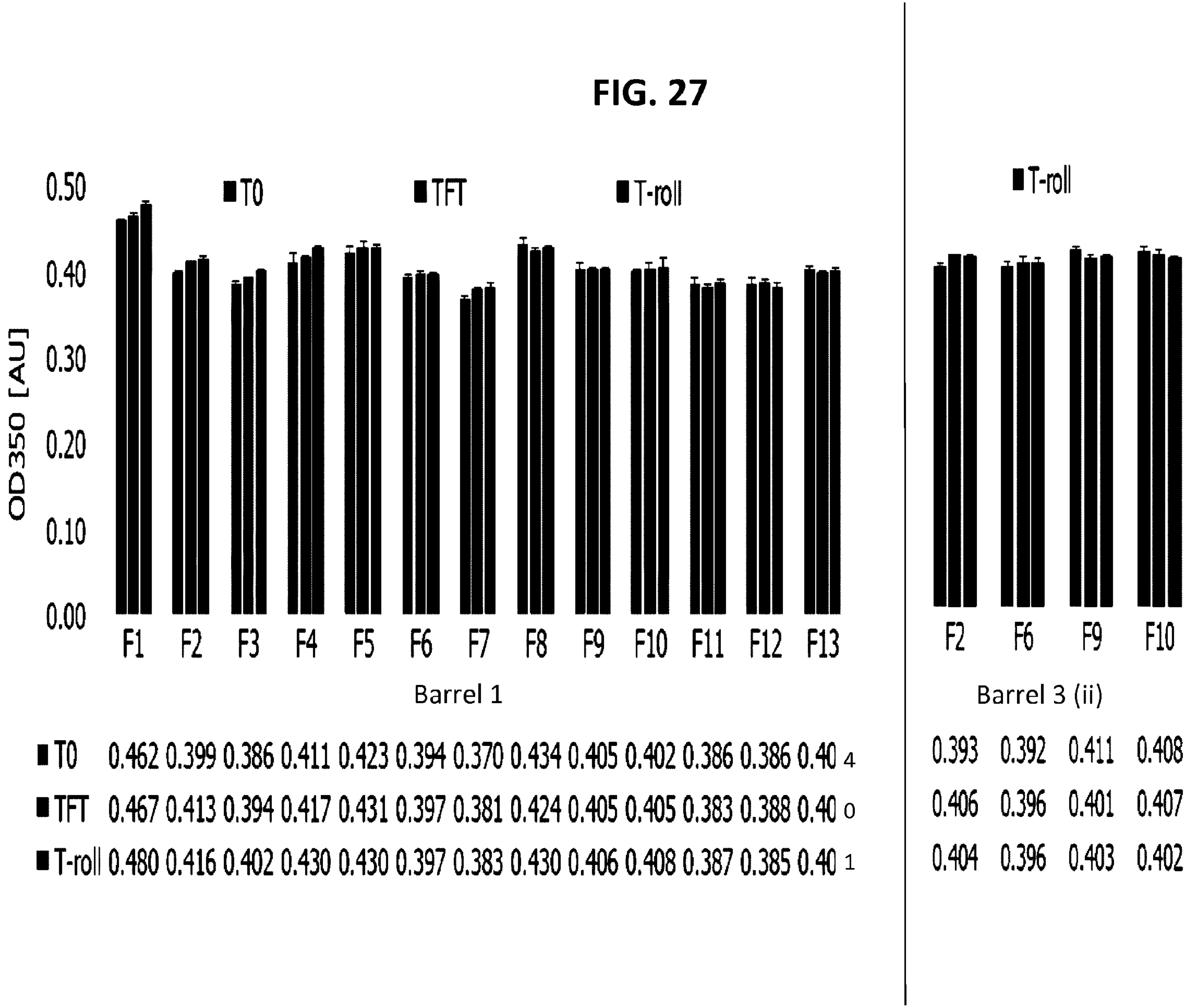
OD350 [AU]
0.50
0.40
0.30
0.20
0.10
0.00
T0
TFT
T-roll
F1 F2 F3 F4 F5 F6 F7 F8 F9 F10 F11 F12 F13
Barrel 1
T0 0.462 0.399 0.386 0.411 0.423 0.394 0.370 0.434 0.405 0.402 0.386 0.386 0.404
TFT 0.467 0.413 0.394 0.417 0.431 0.397 0.381 0.424 0.405 0.405 0.383 0.388 0.400
T-roll 0.480 0.416 0.402 0.430 0.430 0.397 0.383 0.430 0.406 0.408 0.387 0.385 0.401
T-roll
F2 F6 F9 F10
Barrel 3 (ii)
0.393 0.392 0.411 0.408
0.406 0.396 0.401 0.407
0.404 0.396 0.403 0.402

FIG. 28

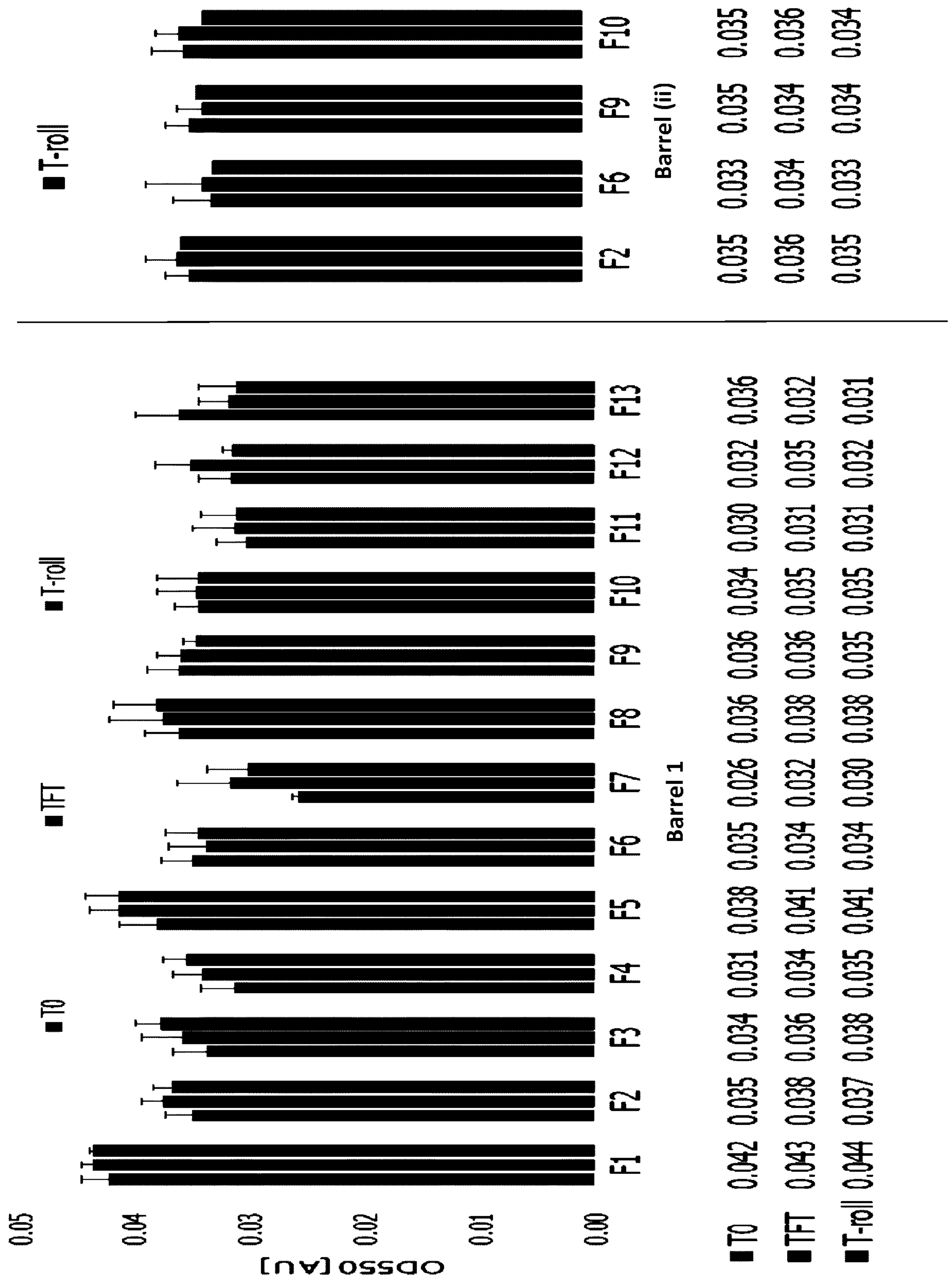
0.05
0.04
0.03
0.02
0.01
0.00
ODSSO [AU]
T0
TFT
T-roll
F1 F2 F3 F4 F5 F6 F7 F8 F9 F10 F11 F12 F13
Barrel 1
T0 0.042 0.035 0.034 0.031 0.038 0.035 0.026 0.036 0.036 0.034 0.030 0.032 0.036
TFT 0.043 0.038 0.036 0.034 0.041 0.034 0.032 0.038 0.036 0.035 0.031 0.035 0.032
T-roll 0.044 0.037 0.038 0.035 0.041 0.034 0.030 0.038 0.035 0.035 0.031 0.032 0.031
T-roll
F2 F6 F9 F10
Barrel (ii)
0.035 0.033 0.035 0.035
0.036 0.034 0.034 0.036
0.035 0.033 0.034 0.034

FIG. 29

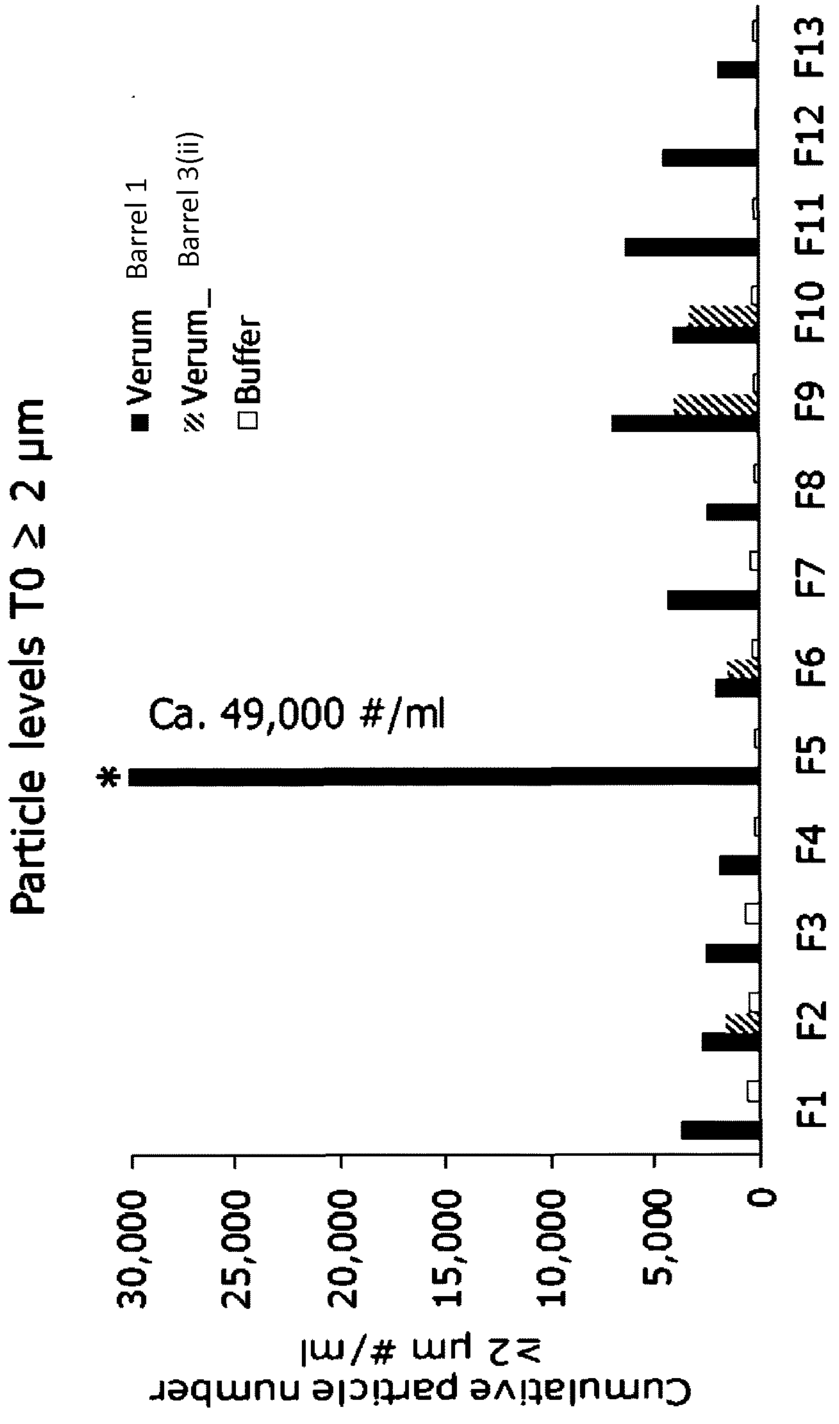
Particle levels T0 ≥ 2 µm
Ca. 49,000 #/ml
*
Verum Barrel 1
Verum_ Barrel 3(ii)
Buffer
Cumulative particle number
≥2 µm #/ml
30,000
25,000
20,000
15,000
10,000
5,000
0
F1
F2
F3
F4
F5
F6
F7
F8
F9
F10
F11
F12
F13

FIG. 30

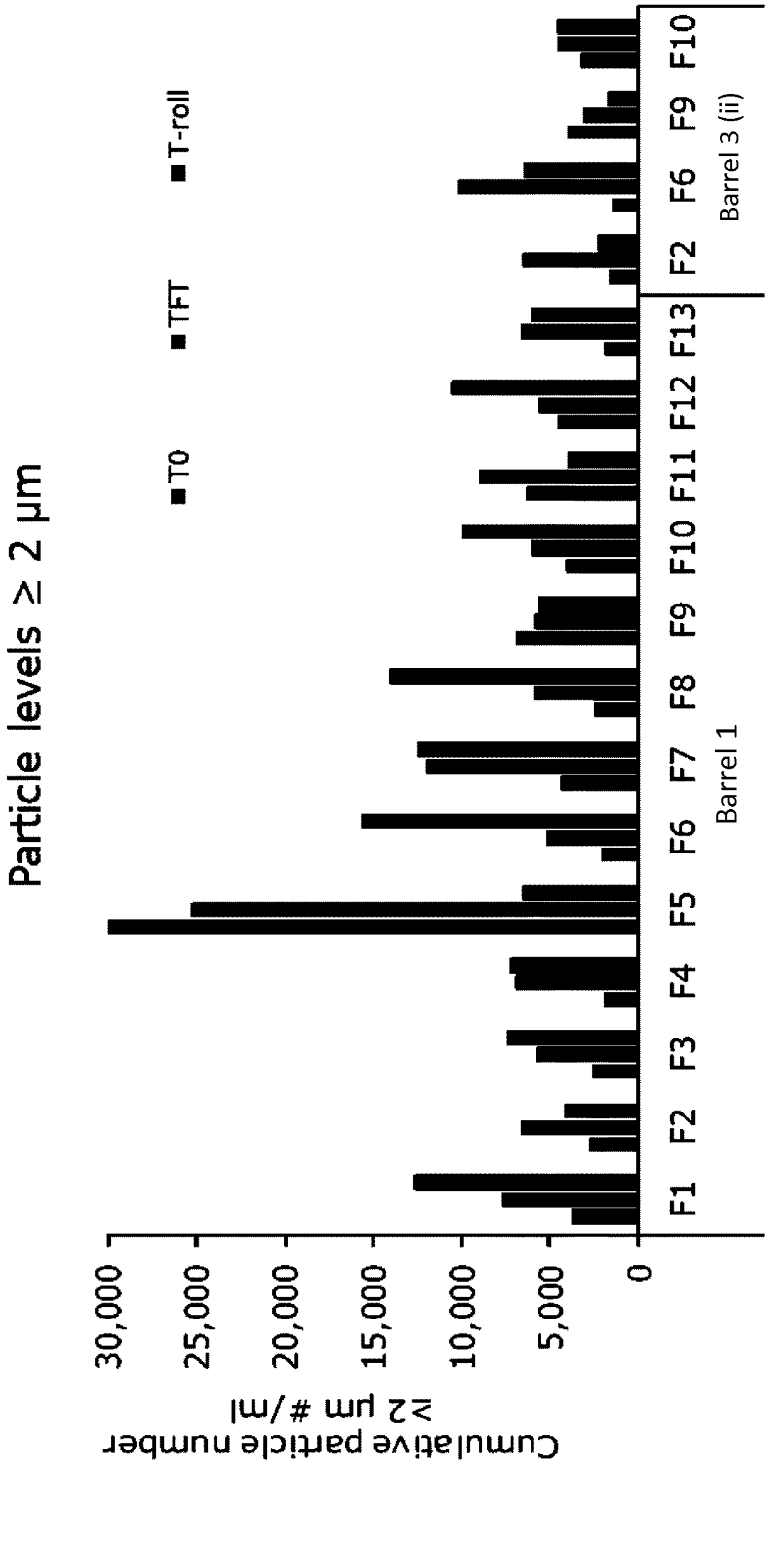
Particle levels ≥ 2 µm
T0
TFT
T-roll
Cumulative particle number
≥2 µm #/ml
30,000
25,000
20,000
15,000
10,000
5,000
0
F1
F2
F3
F4
F5
F6
F7
F8
F9
F10
F11
F12
F13
Barrel 1
F2
F6
F9
F10
Barrel 3 (ii)

FIG. 31A

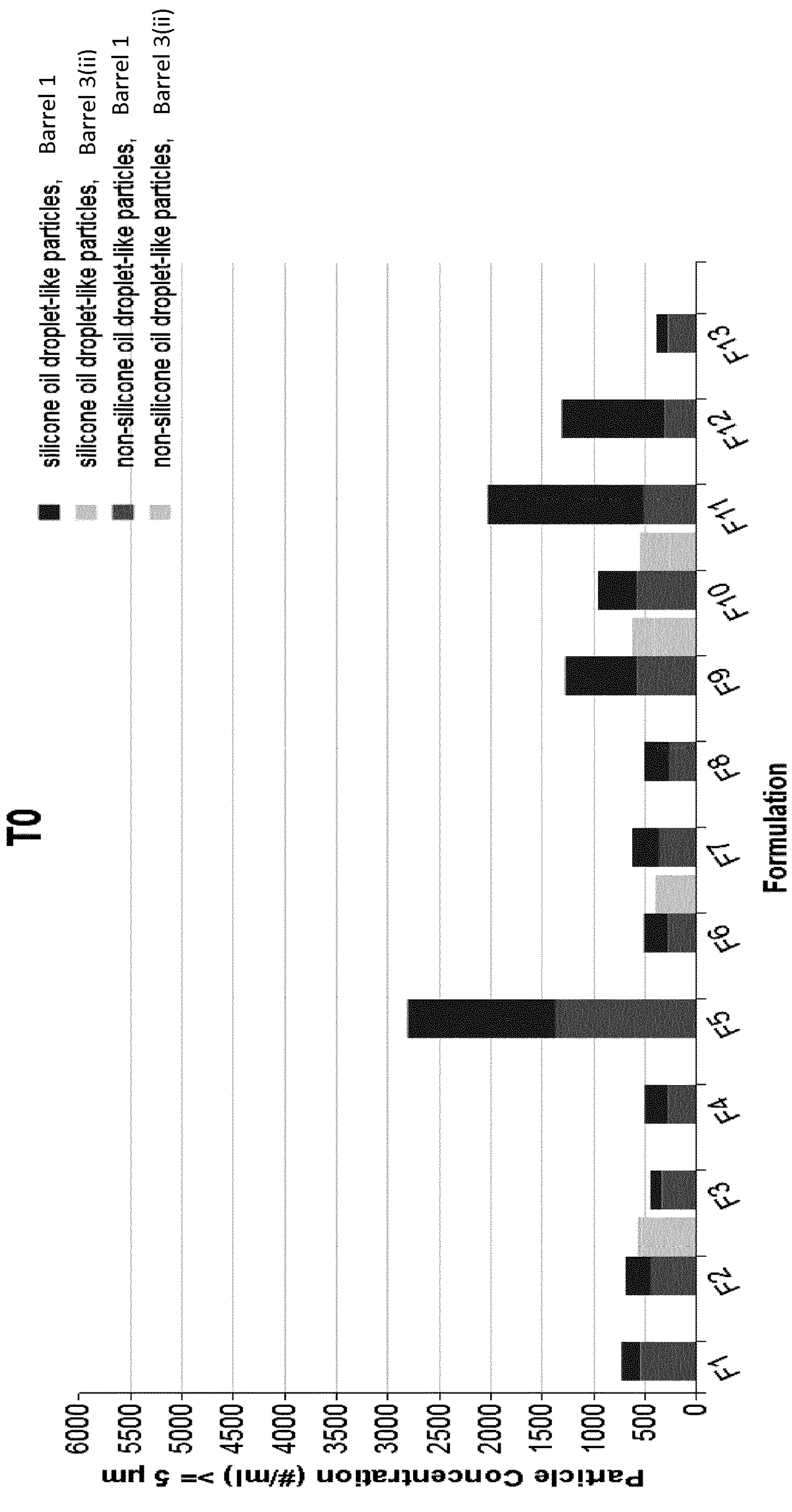
T0
silicone oil droplet-like particles, Barrel 1
silicone oil droplet-like particles, Barrel 3(ii)
non-silicone oil droplet-like particles, Barrel 1
non-silicone oil droplet-like particles, Barrel 3(ii)
Particle Concentration (#/ml) >= 5 µm
6000
5500
5000
4500
4000
3500
3000
2500
2000
1500
1000
500
0
F1
F2
F3
F4
F5
F6
F7
F8
F9
F10
F11
F12
F13
Formulation

FIG. 31B

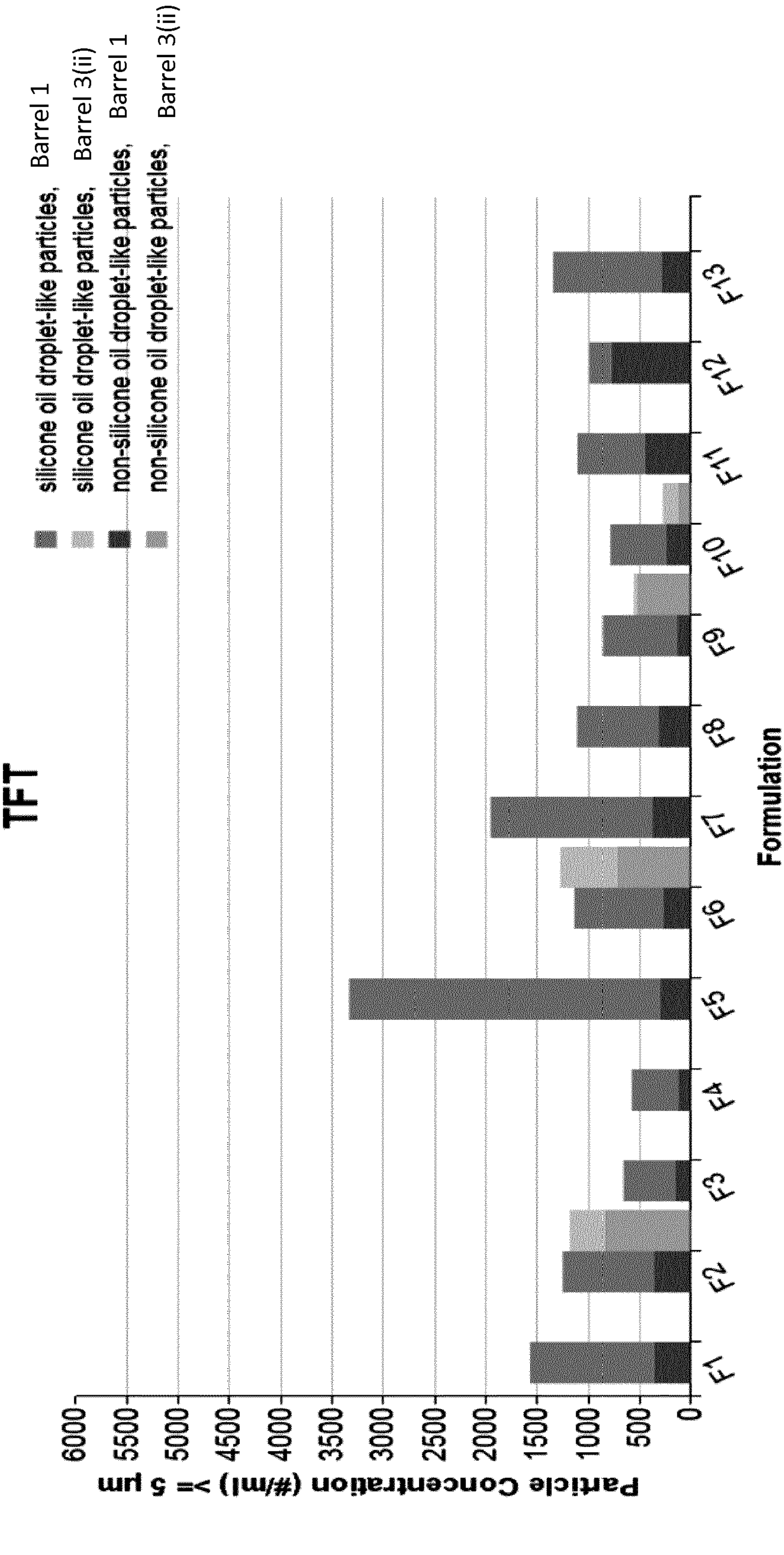
TFT
Particle Concentration (#/ml) >= 5 µm
6000
5500
5000
4500
4000
3500
3000
2500
2000
1500
1000
500
0
F1
F2
F3
F4
F5
F6
F7
F8
F9
F10
F11
F12
F13
Formulation
silicone oil droplet-like particles, Barrel 1
silicone oil droplet-like particles, Barrel 3(ii)
non-silicone oil droplet-like particles, Barrel 1
non-silicone oil droplet-like particles, Barrel 3(ii)

FIG. 31C

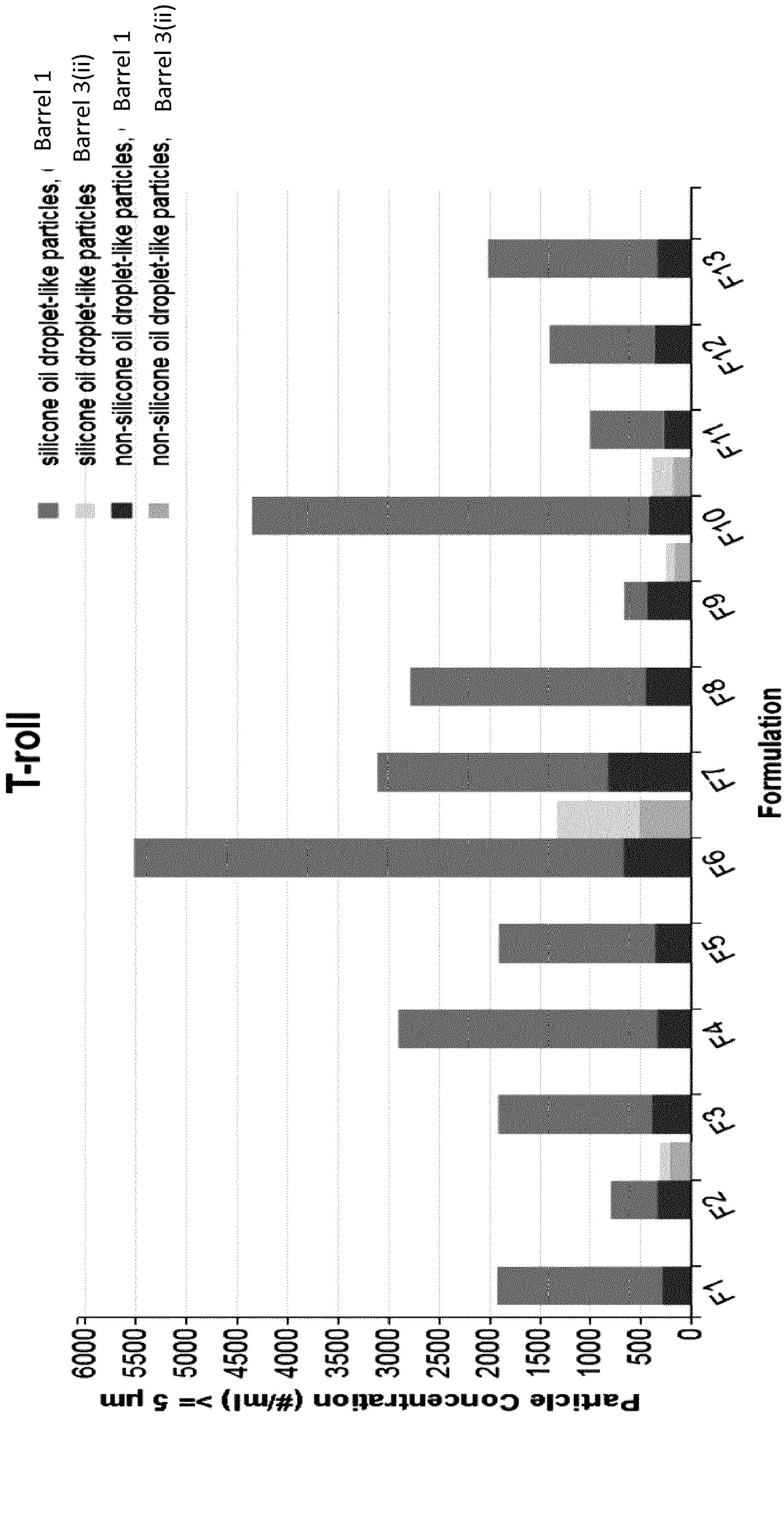
T-roll
silicone oil droplet-like particles, Barrel 1
silicone oil droplet-like particles Barrel 3(ii)
non-silicone oil droplet-like particles, Barrel 1
non-silicone oil droplet-like particles, Barrel 3(ii)
Particle Concentration (#/ml) >= 5 µm
6000
5500
5000
4500
4000
3500
3000
2500
2000
1500
1000
500
0
F1
F2
F3
F4
F5
F6
F7
F8
F9
F10
F11
F12
F13
Formulation

FIG. 32A

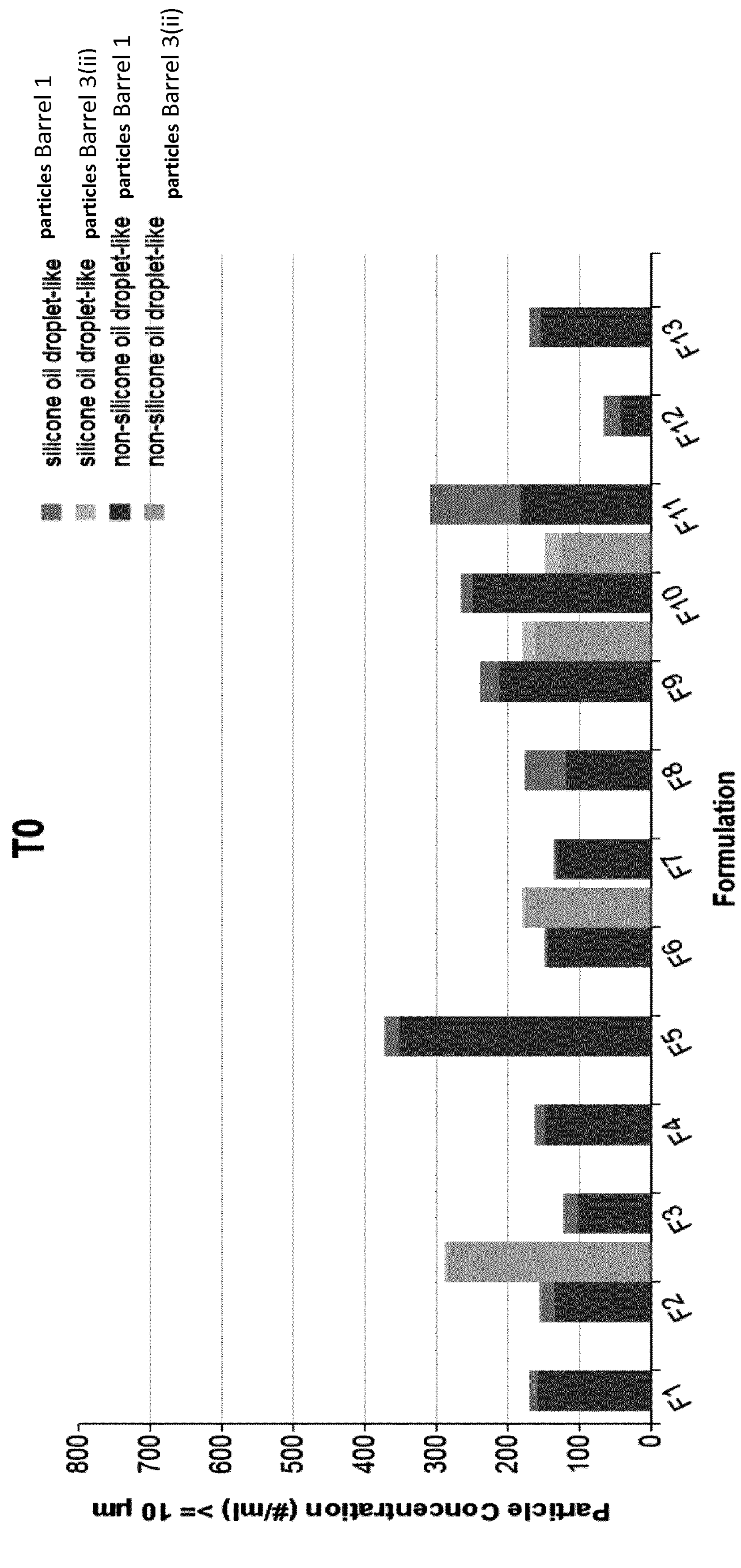
T0
silicone oil droplet-like particles Barrel 1
silicone oil droplet-like particles Barrel 3(ii)
non-silicone oil droplet-like particles Barrel 1
non-silicone oil droplet-like particles Barrel 3(ii)
Particle Concentration (#/ml) >= 10 µm
0
100
200
300
400
500
600
700
800
F1
F2
F3
F4
F5
F6
F7
F8
F9
F10
F11
F12
F13
Formulation

FIG. 32B

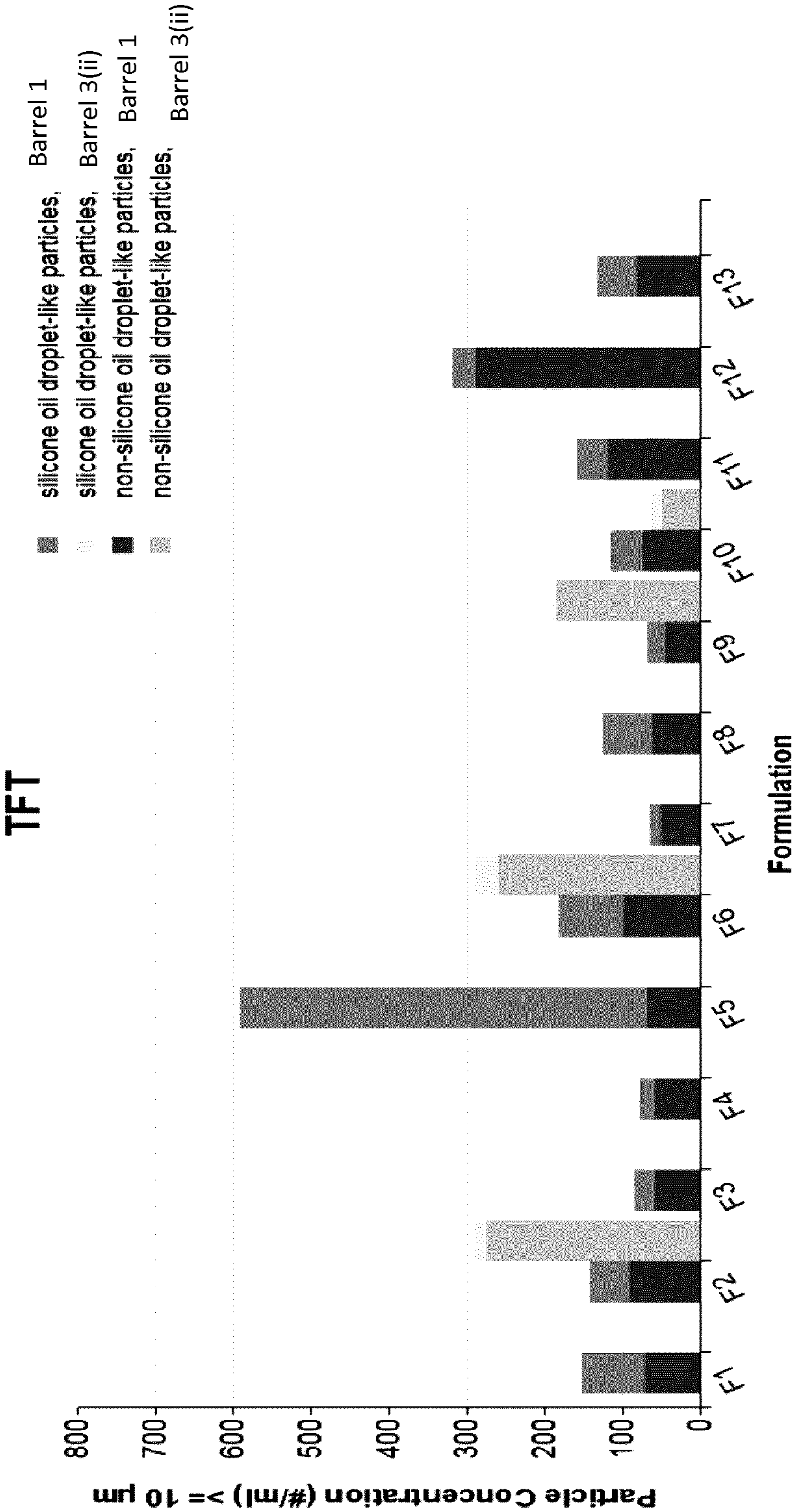
TFT
silicone oil droplet-like particles, Barrel 1
silicone oil droplet-like particles, Barrel 3(ii)
non-silicone oil droplet-like particles, Barrel 1
non-silicone oil droplet-like particles, Barrel 3(ii)
Particle Concentration (#/ml) >= 10 μm
0
100
200
300
400
500
600
700
800
F1
F2
F3
F4
F5
F6
F7
F8
F9
F10
F11
F12
F13
Formulation

FIG. 32C

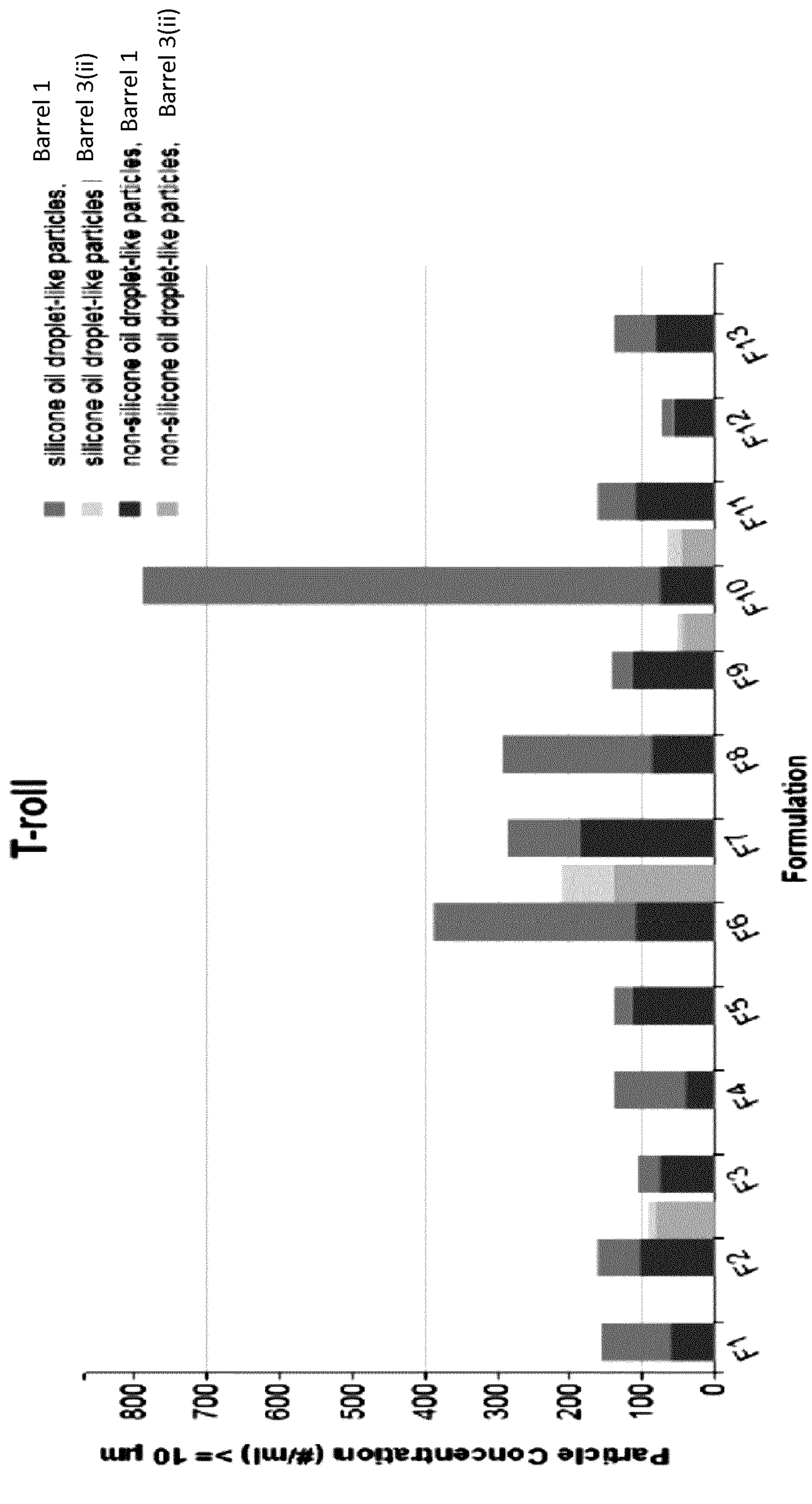
T-roll
silicone oil droplet-like particles, Barrel 1
silicone oil droplet-like particles Barrel 3(ii)
non-silicone oil droplet-like particles, Barrel 1
non-silicone oil droplet-like particles, Barrel 3(ii)
Particle Concentration (#/ml) >= 10 µm
0
100
200
300
400
500
600
700
800
F1
F2
F3
F4
F5
F6
F7
F8
F9
F10
F11
F12
F13
Formulation

FIG. 33A

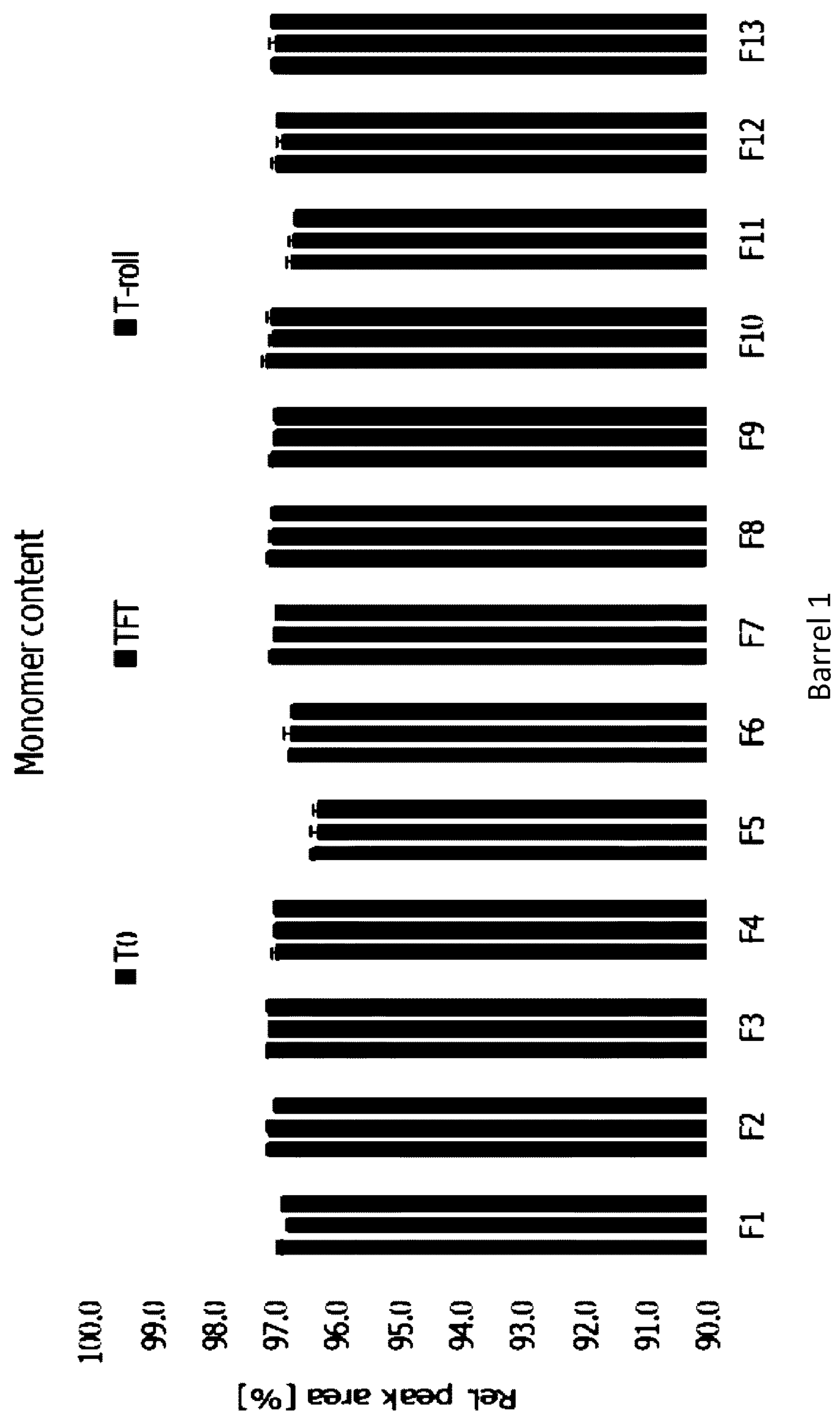
Monomer content
T0
TFT
T-roll
Rel. peak area [%]
100.0
99.0
98.0
97.0
96.0
95.0
94.0
93.0
92.0
91.0
90.0
F1
F2
F3
F4
F5
F6
F7
F8
F9
F10
F11
F12
F13
Barrel 1

FIG. 33B

Monomer content

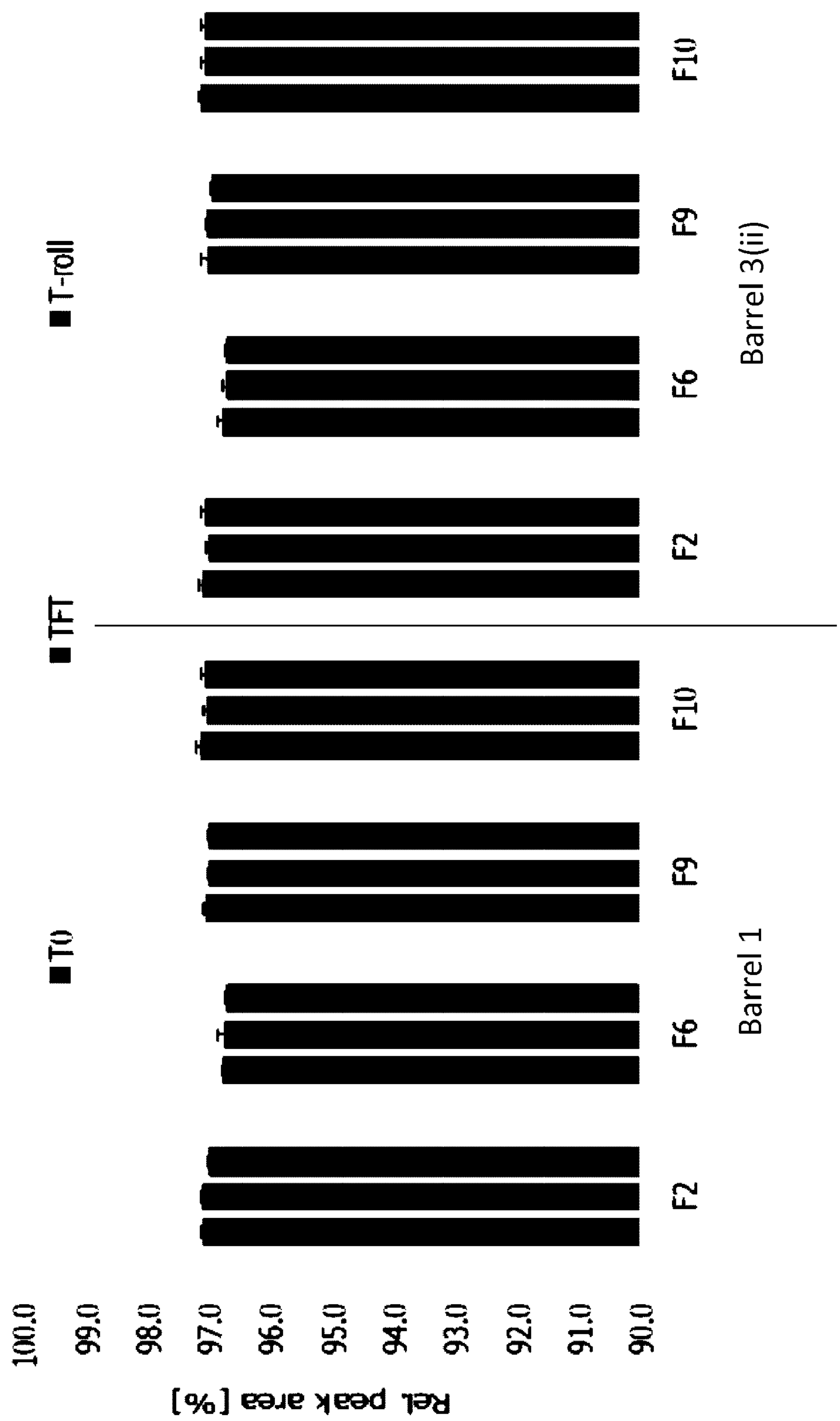

FIG. 34A

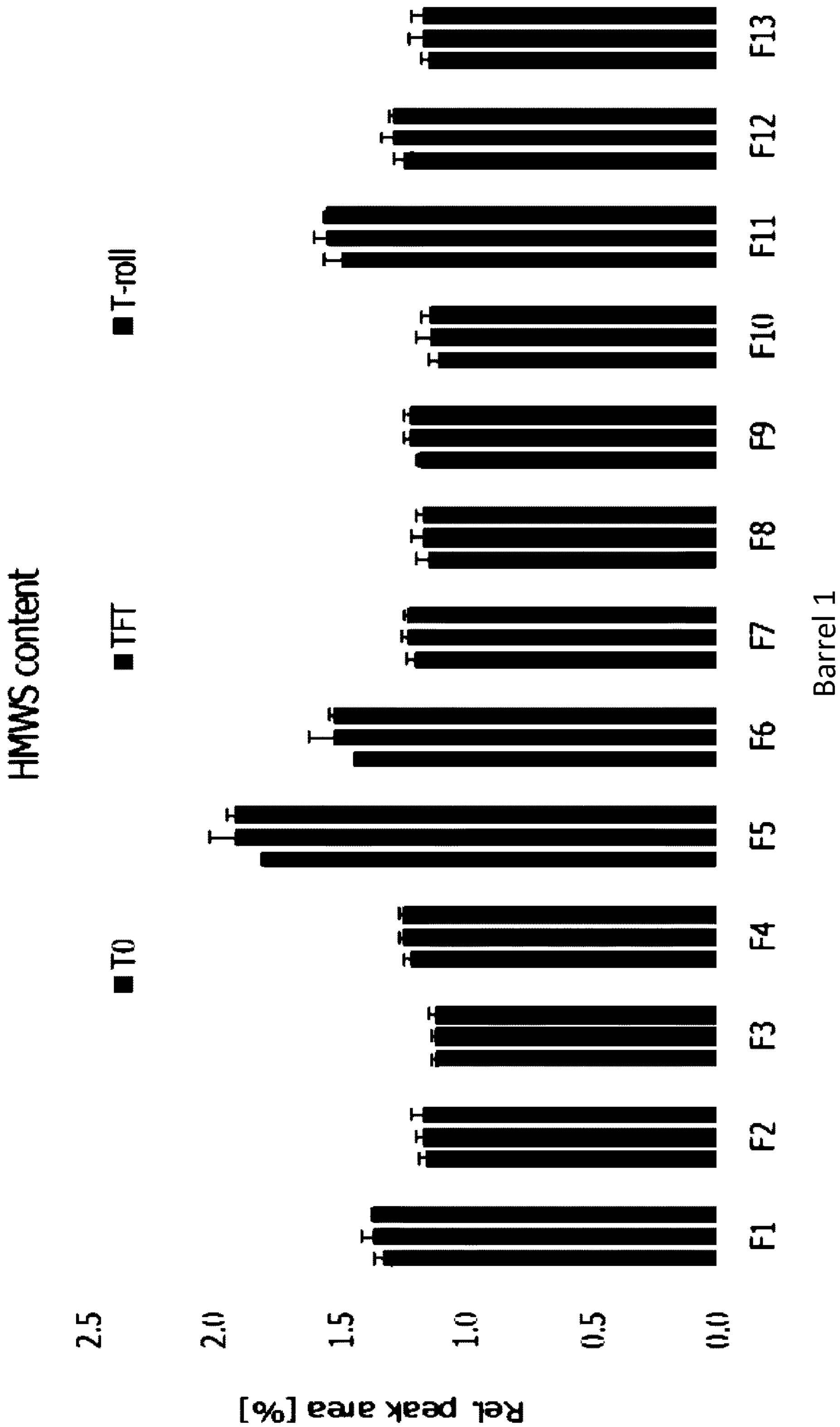
HMWS content
T0
TFT
T-roll
Rel. peak area [%]
2.5
2.0
1.5
1.0
0.5
0.0
F1
F2
F3
F4
F5
F6
F7
F8
F9
F10
F11
F12
F13
Barrel 1

FIG. 34B

HMWS content

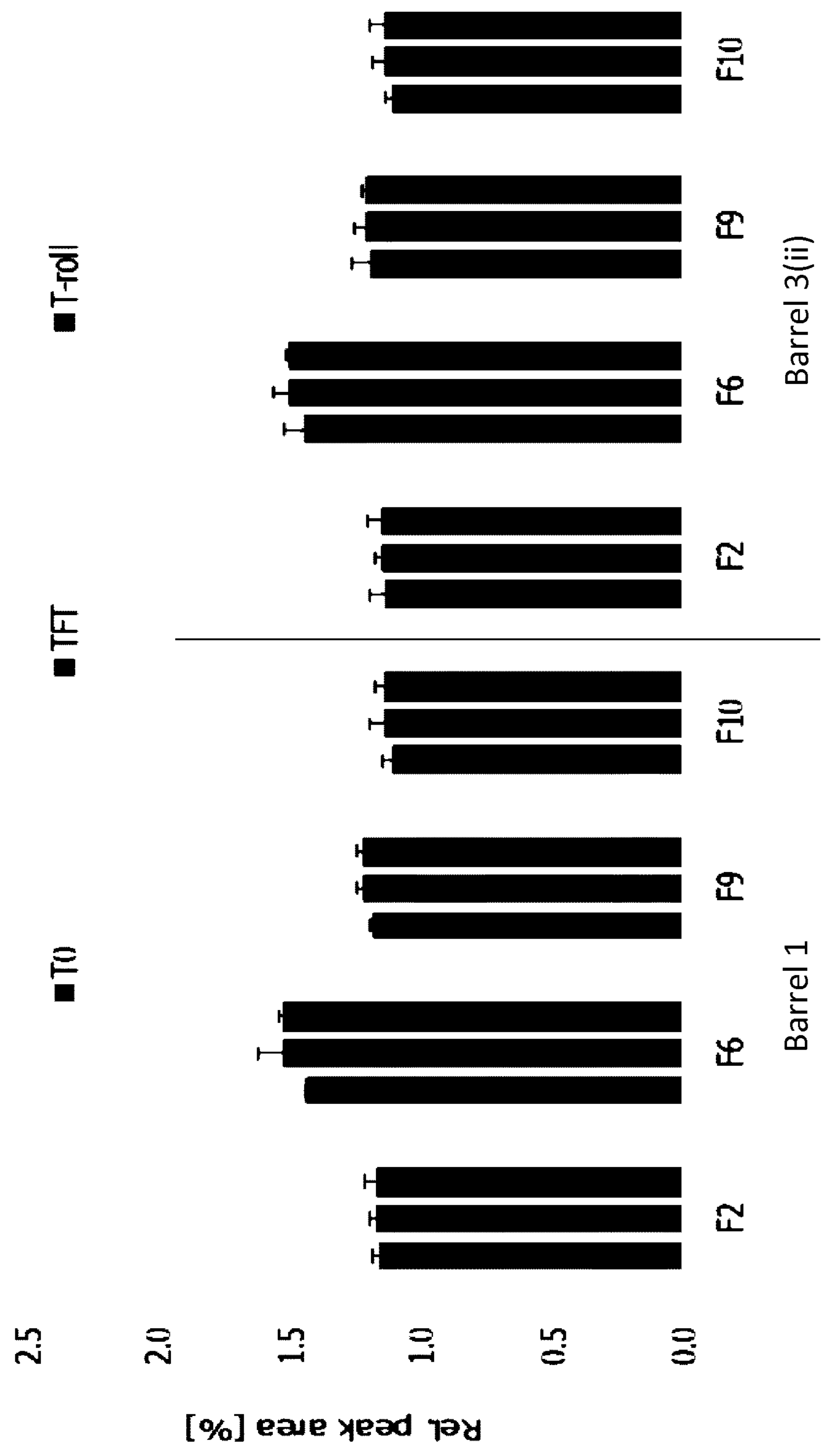

FIG. 35A

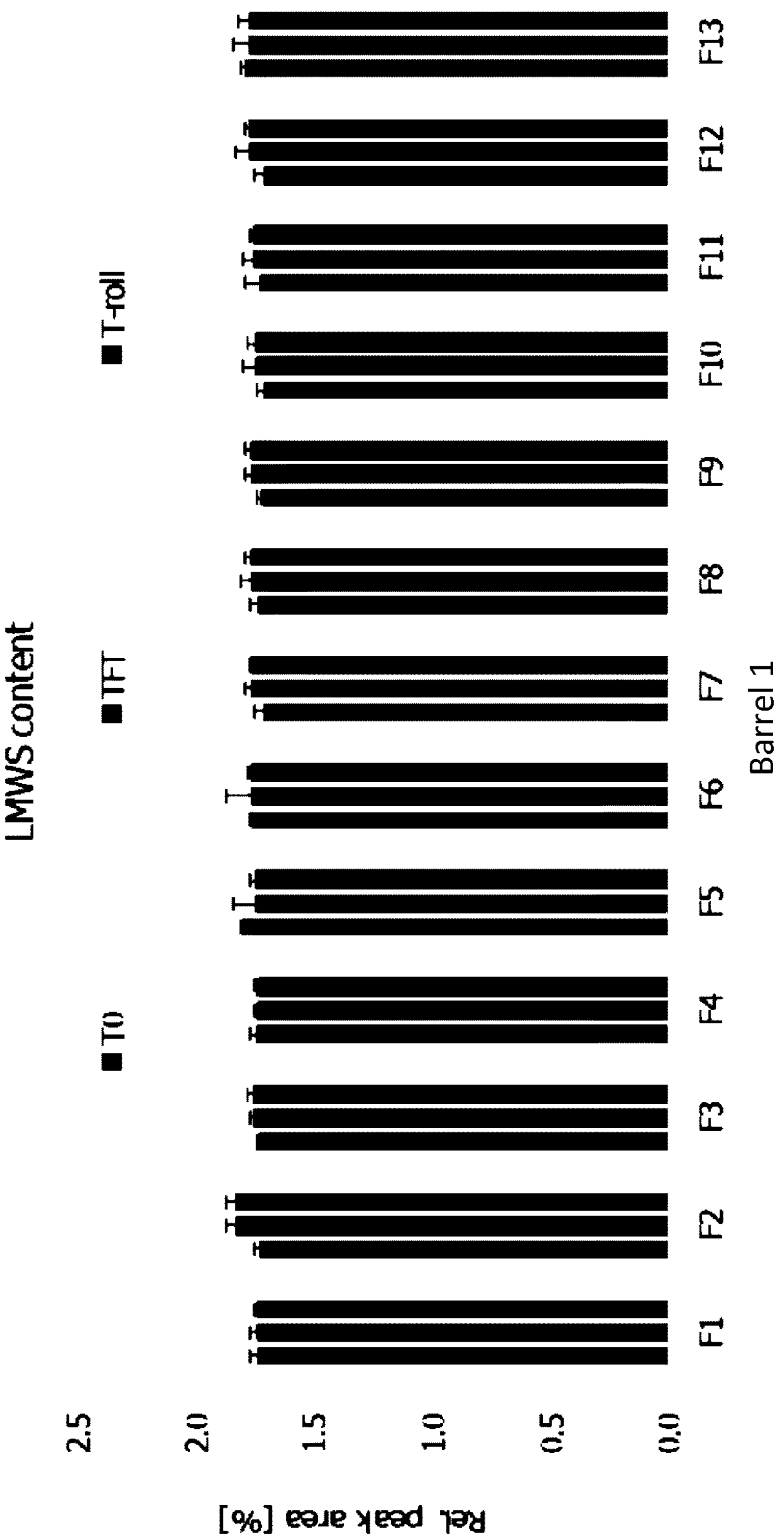
LMWS content
T0
TFT
T-roll
Rel. peak area [%]
0.0
0.5
1.0
1.5
2.0
2.5
F1
F2
F3
F4
F5
F6
F7
F8
F9
F10
F11
F12
F13
Barrel 1

FIG. 35B

LMWS content

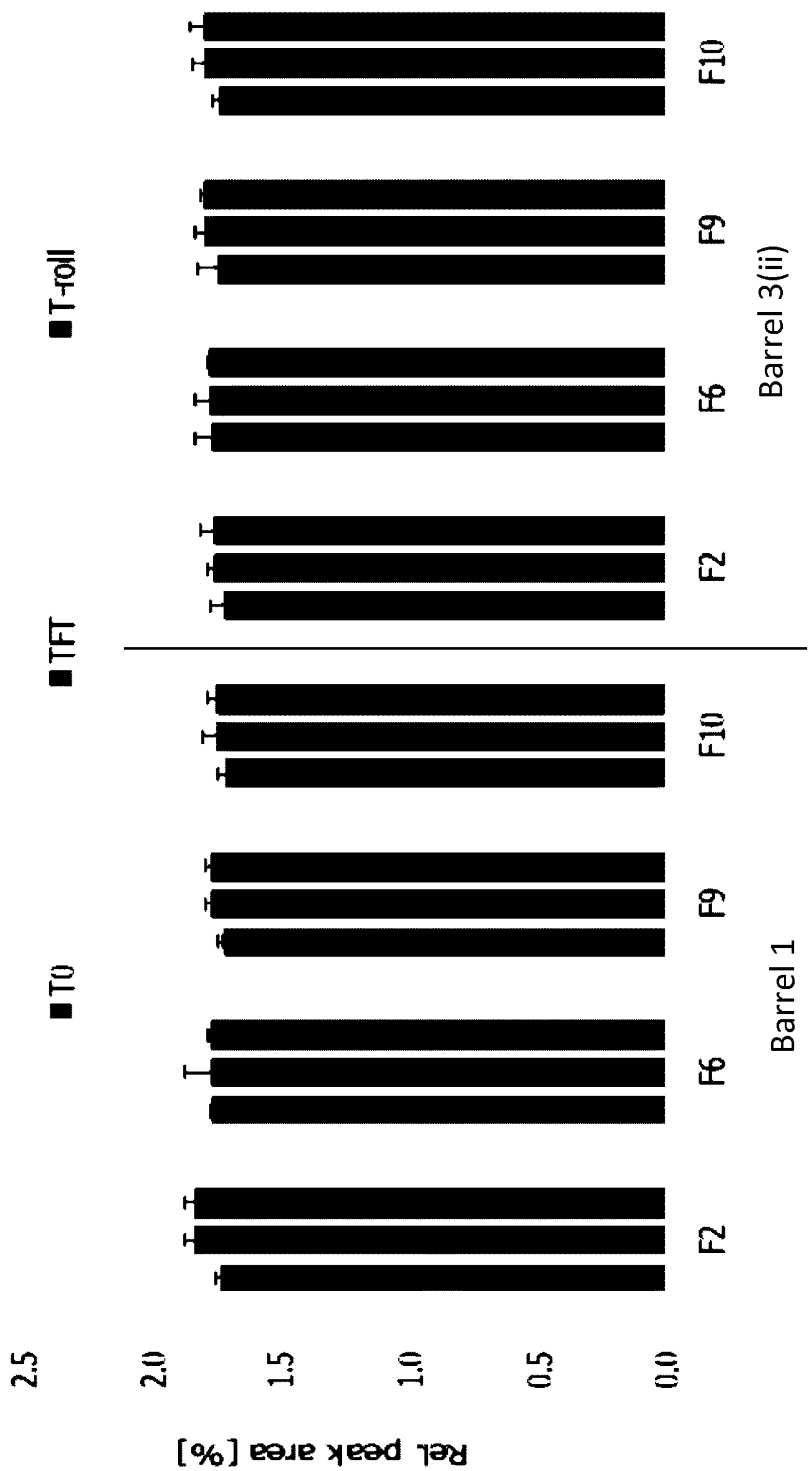

# FIG. 36

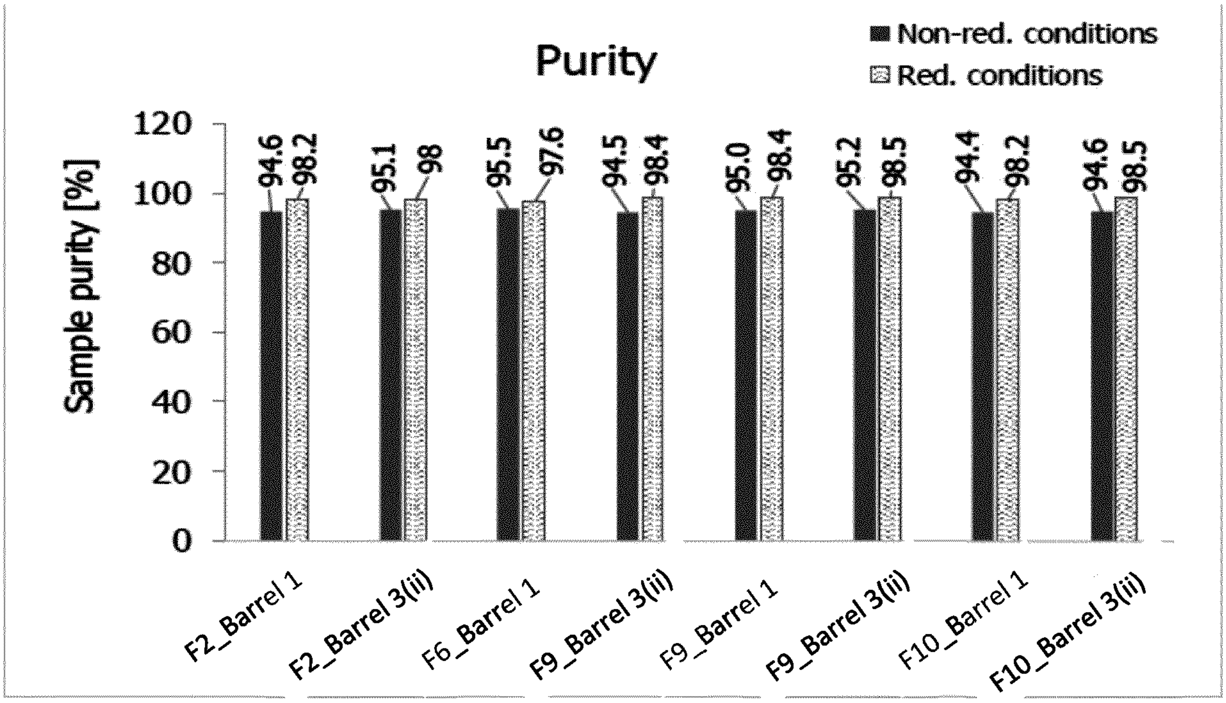
Purity
Non-red. conditions
Red. conditions
Sample purity [%]
120
100
80
60
40
20
0
94.6
98.2
95.1
98
95.5
97.6
94.5
98.4
95.0
98.4
95.2
98.5
94.4
98.2
94.6
98.5
F2_Barrel 1
F2_Barrel 3(ii)
F6_Barrel 1
F9_Barrel 3(ii)
F9_Barrel 1
F9_Barrel 3(ii)
F10_Barrel 1
F10_Barrel 3(ii)

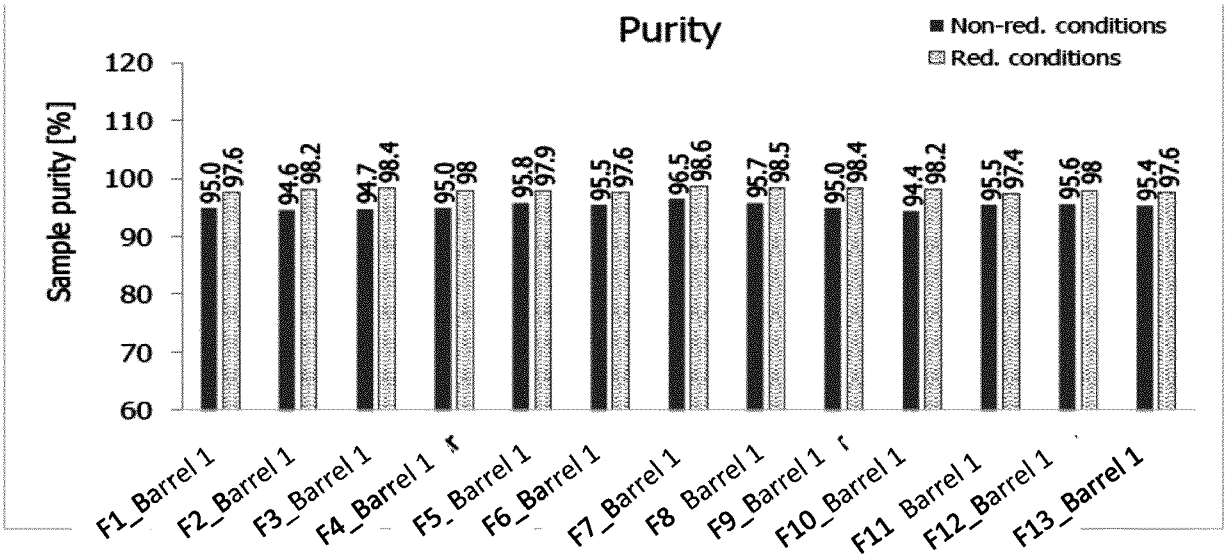
Purity
Non-red. conditions
Red. conditions
Sample purity [%]
120
110
100
90
80
70
60
95.0
97.6
94.6
98.2
94.7
98.4
95.0
98
95.8
97.9
95.5
97.6
96.5
98.6
95.7
98.5
95.0
98.4
94.4
98.2
95.5
97.4
95.6
98
95.4
97.6
F1_Barrel 1
F2_Barrel 1
F3_Barrel 1
F4_Barrel 1
F5_Barrel 1
F6_Barrel 1
F7_Barrel 1
F8 Barrel 1
F9_Barrel 1
F10_Barrel 1
F11 Barrel 1
F12_Barrel 1
F13_Barrel 1

FIG. 37A

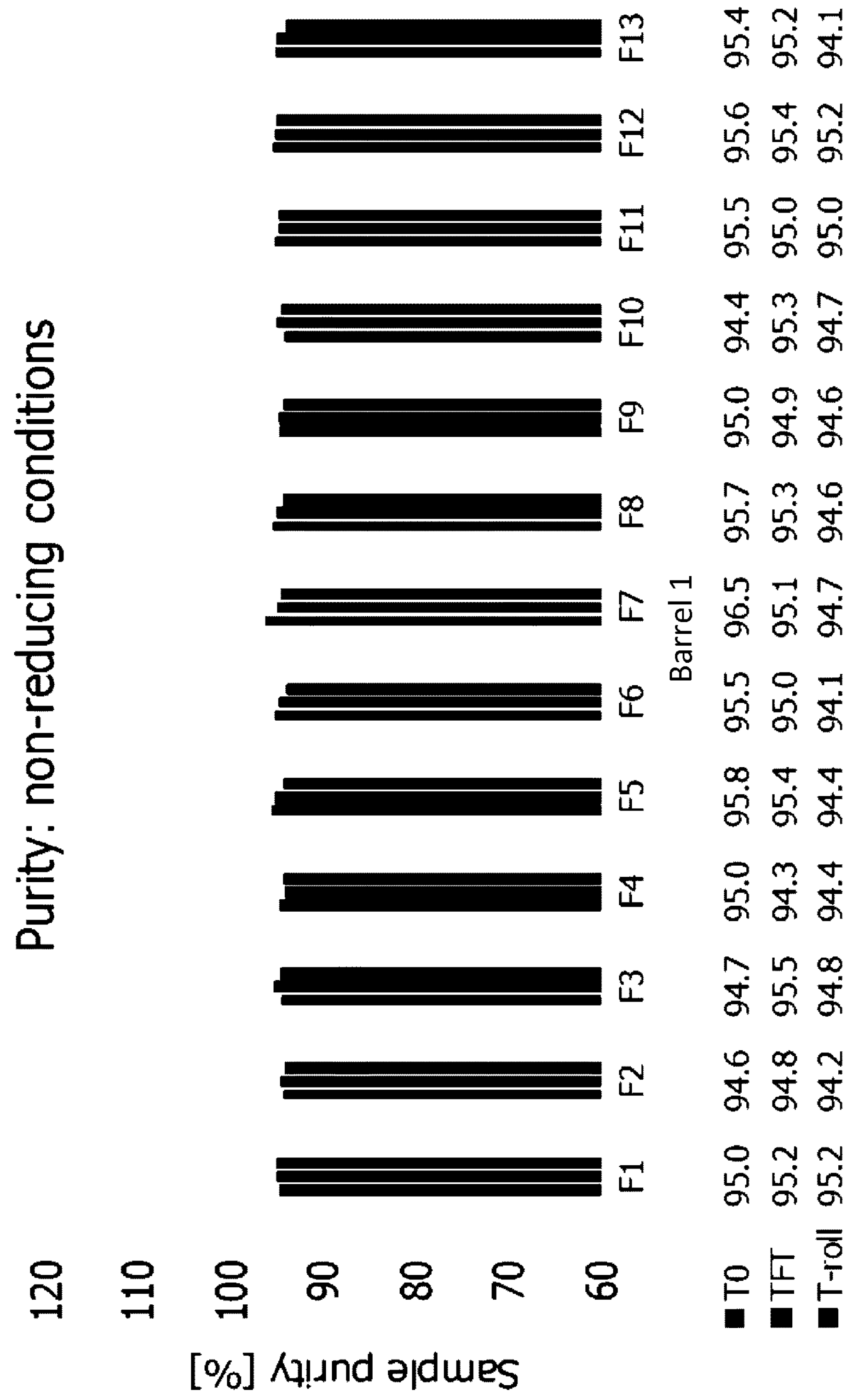
Purity: non-reducing conditions
Sample purity [%]
120
110
100
90
80
70
60
F1 F2 F3 F4 F5 F6 F7 F8 F9 F10 F11 F12 F13
Barrel 1
T0 95.0 94.6 94.7 95.0 95.8 95.5 96.5 95.7 95.0 94.4 95.5 95.6 95.4
TFT 95.2 94.8 95.5 94.3 95.4 95.0 95.1 95.3 94.9 95.3 95.0 95.4 95.2
T-roll 95.2 94.2 94.8 94.4 94.4 94.1 94.7 94.6 94.6 94.7 95.0 95.2 94.1

FIG. 37B

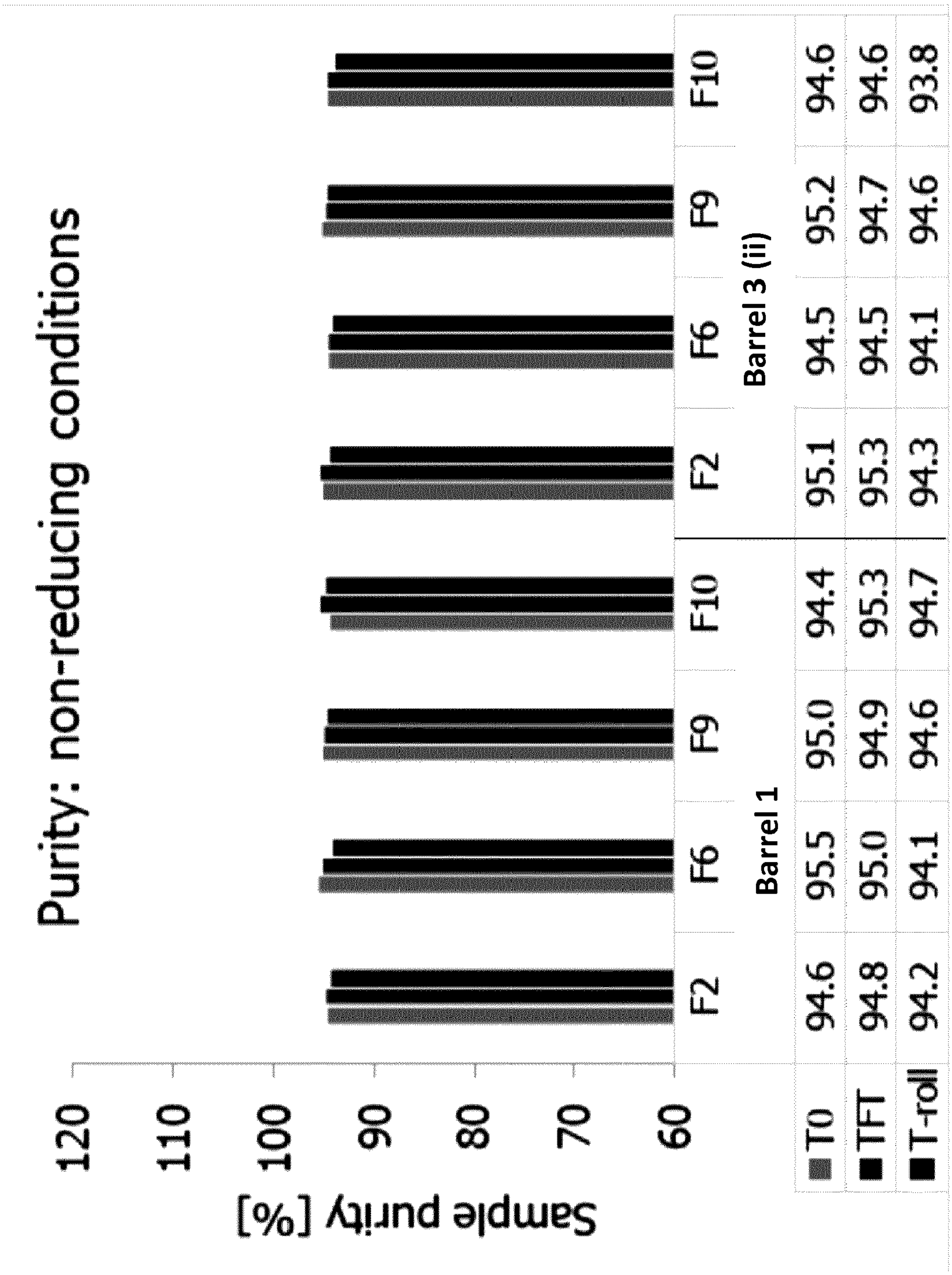
Purity: non-reducing conditions
Sample purity [%]
120
110
100
90
80
70
60
Barrel 1
Barrel 3 (ii)
F2
F6
F9
F10
F2
F6
F9
F10
T0 94.6 95.5 95.0 94.4 95.1 94.5 95.2 94.6
TFT 94.8 95.0 94.9 95.3 95.3 94.5 94.7 94.6
T-roll 94.2 94.1 94.6 94.7 94.3 94.1 94.6 93.8

FIG. 38A

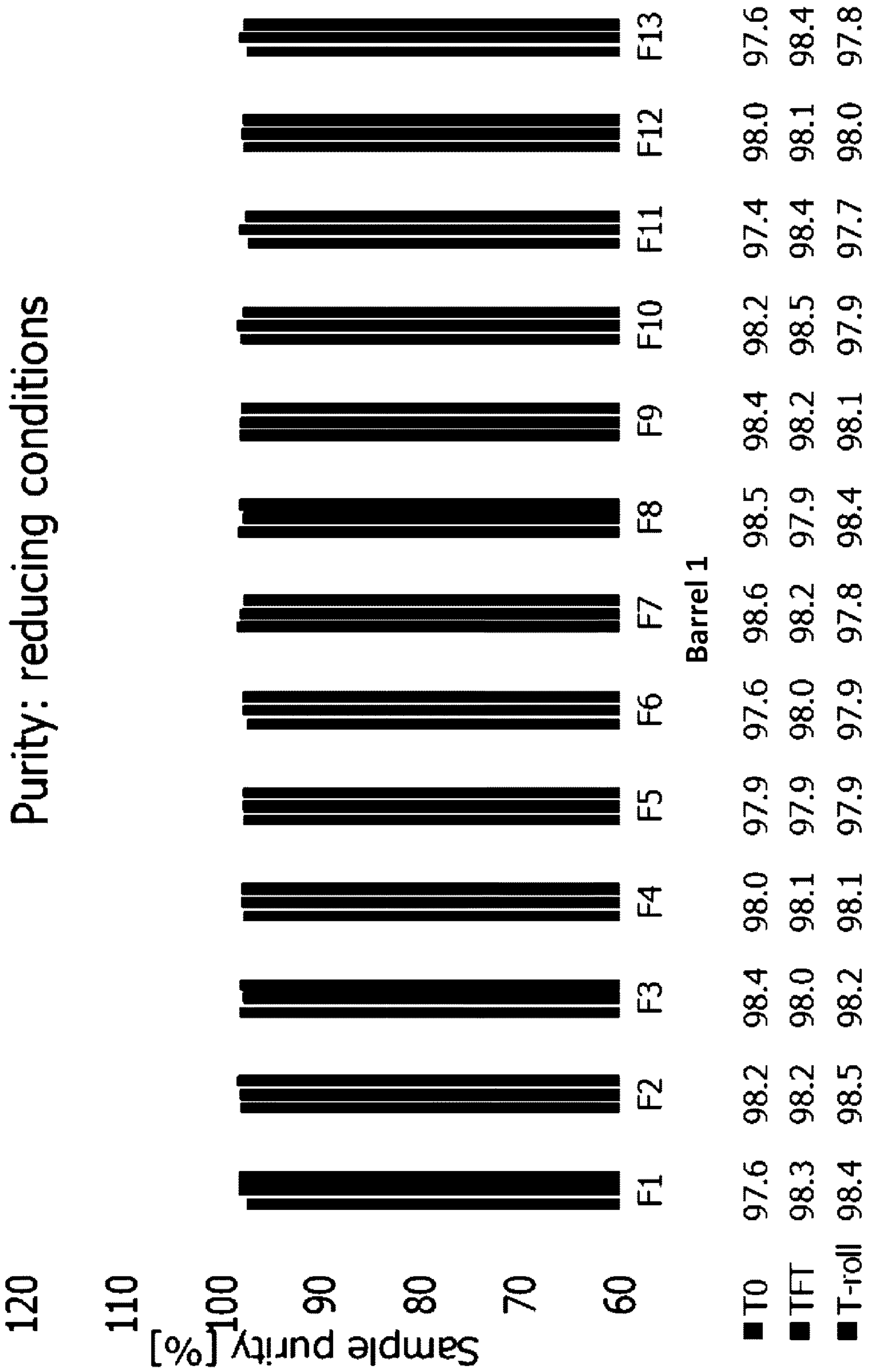
Purity: reducing conditions
Sample purity [%]
120
110
100
90
80
70
60
F1 F2 F3 F4 F5 F6 F7 F8 F9 F10 F11 F12 F13
Barrel 1
T0 97.6 98.2 98.4 98.0 97.9 97.6 98.6 98.5 98.4 98.2 97.4 98.0 97.6
TFT 98.3 98.2 98.0 98.1 97.9 98.0 98.2 97.9 98.2 98.5 98.4 98.1 98.4
T-roll 98.4 98.5 98.2 98.1 97.9 97.9 97.8 98.4 98.1 97.9 97.7 98.0 97.8

**FIG. 38B**

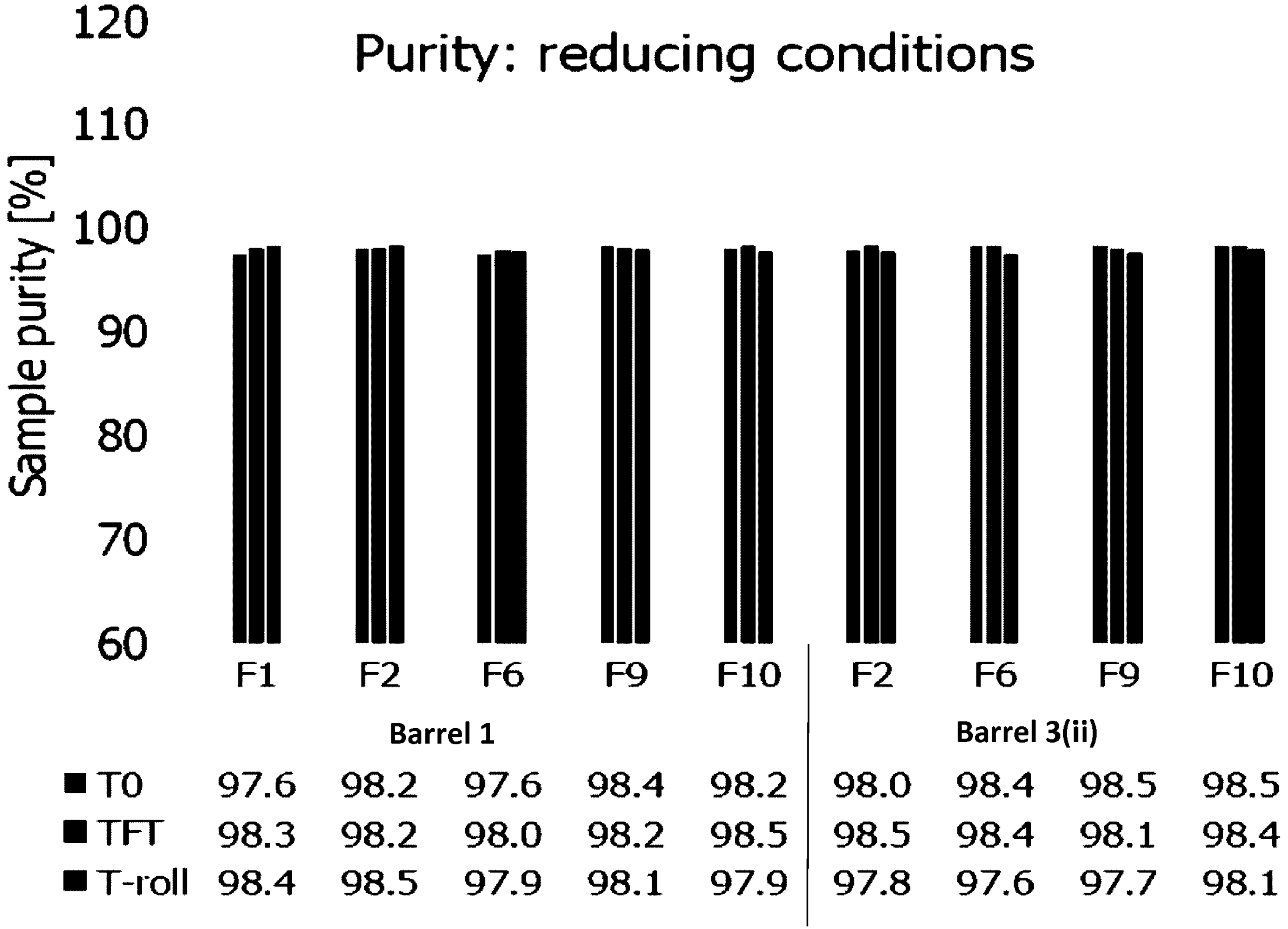


| | Barrel 1 F1 | Barrel 1 F2 | Barrel 1 F6 | Barrel 1 F9 | Barrel 1 F10 | Barrel 3(ii) F2 | Barrel 3(ii) F6 | Barrel 3(ii) F9 | Barrel 3(ii) F10 |
|---|---|---|---|---|---|---|---|---|---|
| ■ T0 | 97.6 | 98.2 | 97.6 | 98.4 | 98.2 | 98.0 | 98.4 | 98.5 | 98.5 |
| ■ TFT | 98.3 | 98.2 | 98.0 | 98.2 | 98.5 | 98.5 | 98.4 | 98.1 | 98.4 |
| ■ T-roll | 98.4 | 98.5 | 97.9 | 98.1 | 97.9 | 97.8 | 97.6 | 97.7 | 98.1 |

FIG. 39A

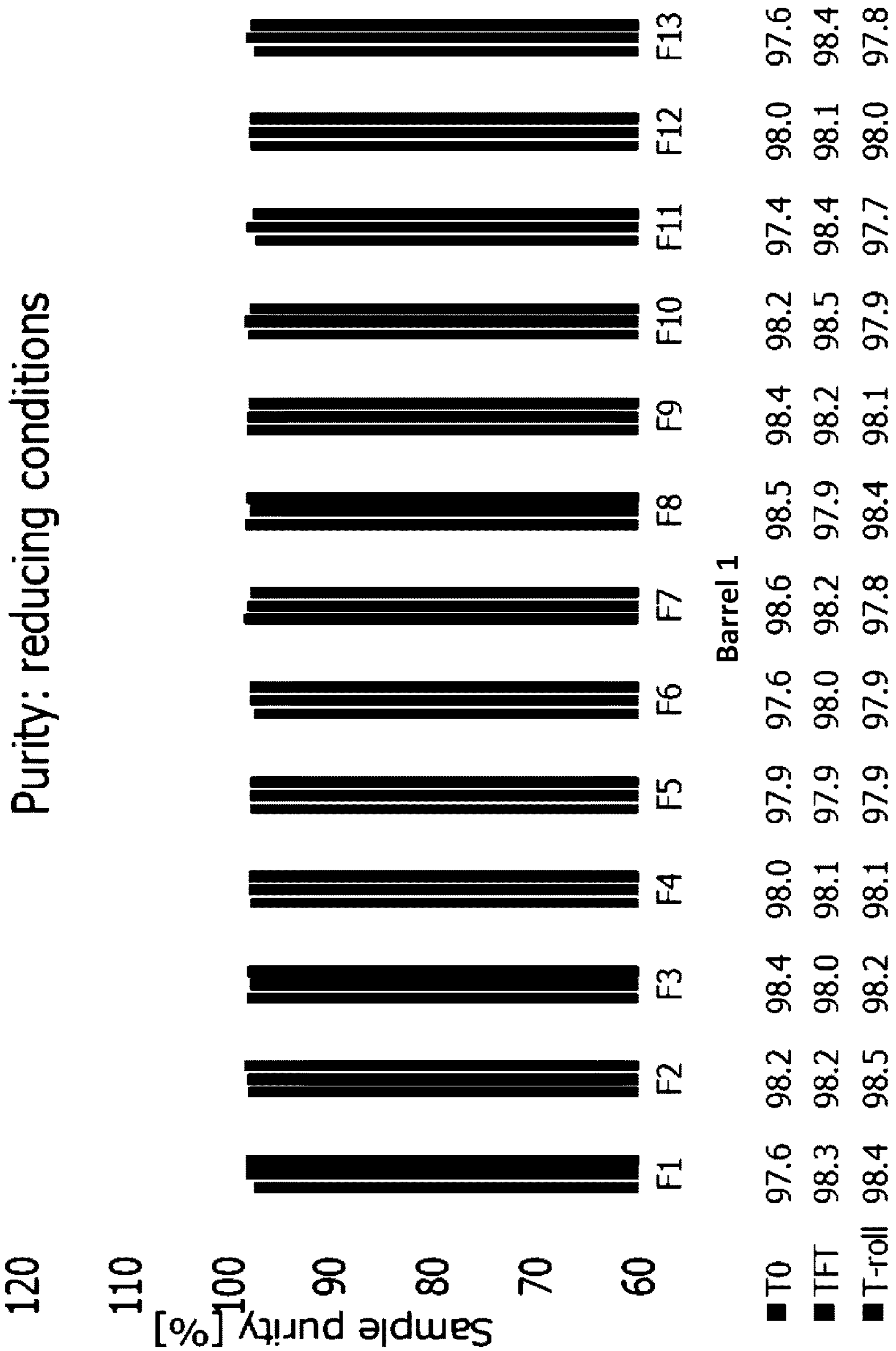
Purity: reducing conditions
Sample purity [%]
120
110
100
90
80
70
60
F1 F2 F3 F4 F5 F6 F7 F8 F9 F10 F11 F12 F13
Barrel 1
■ T0 97.6 98.2 98.4 98.0 97.9 97.6 98.6 98.5 98.4 98.2 97.4 98.0 97.6
■ TFT 98.3 98.2 98.0 98.1 97.9 98.0 98.2 97.9 98.2 98.5 98.4 98.1 98.4
■ T-roll 98.4 98.5 98.2 98.1 97.9 97.9 97.8 98.4 98.1 97.9 97.7 98.0 97.8

FIG. 39B

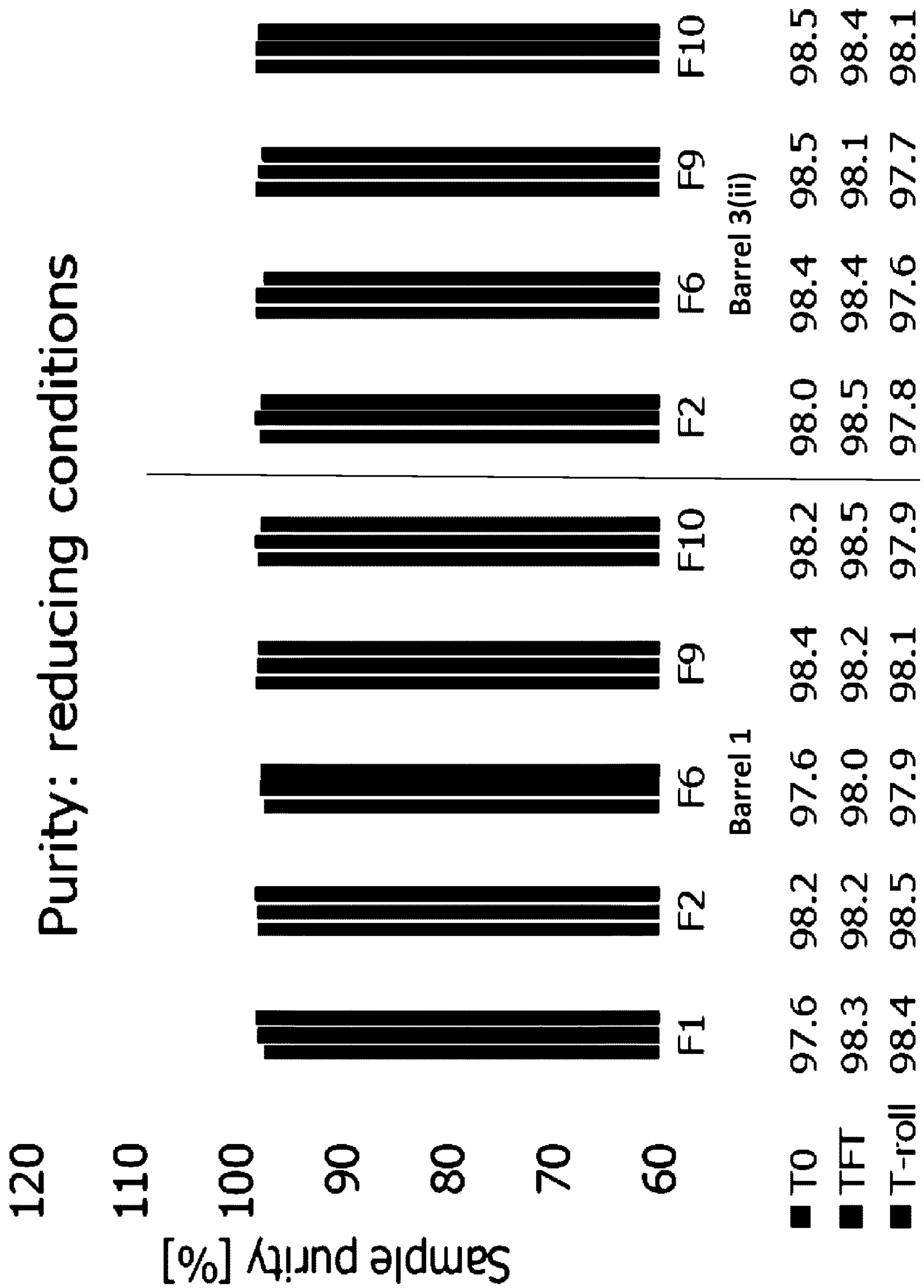
Purity: reducing conditions
Sample purity [%]
120
110
100
90
80
70
60
F1
F2
F6
F9
F10
F2
F6
F9
F10
Barrel 1
Barrel 3(ii)
T0 97.6 98.2 97.6 98.4 98.2 98.0 98.4 98.5 98.5
TFT 98.3 98.2 98.0 98.2 98.5 98.5 98.4 98.1 98.4
T-roll 98.4 98.5 97.9 98.1 97.9 97.8 97.6 97.7 98.1

FIG. 40

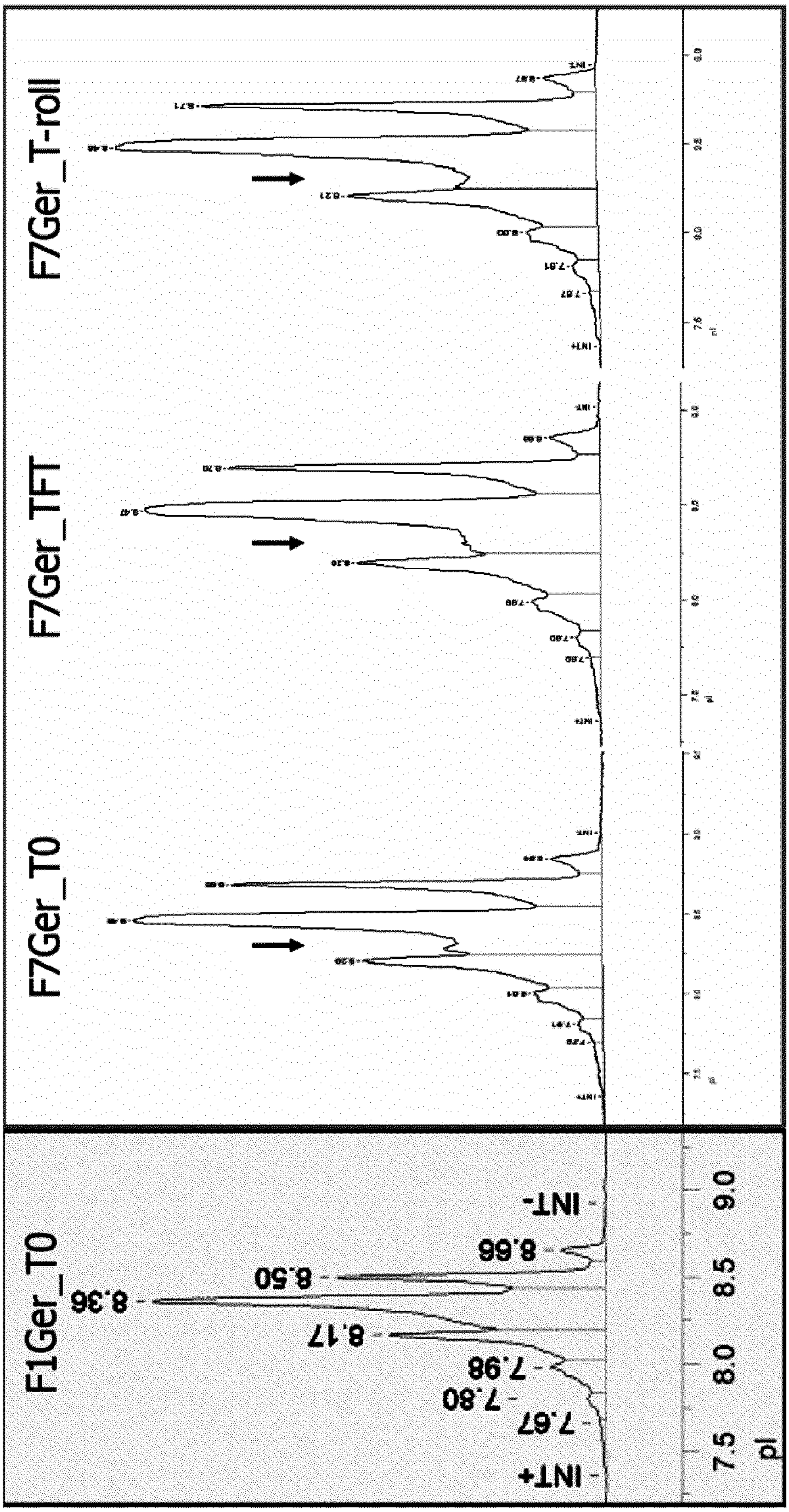
F1Ger_T0
F7Ger_T0
F7Ger_TFT
F7Ger_T-roll
INT+
7.67
7.80
7.98
8.17
8.36
8.50
8.66
INT-
7.5
8.0
8.5
9.0
pI

FIG. 41A

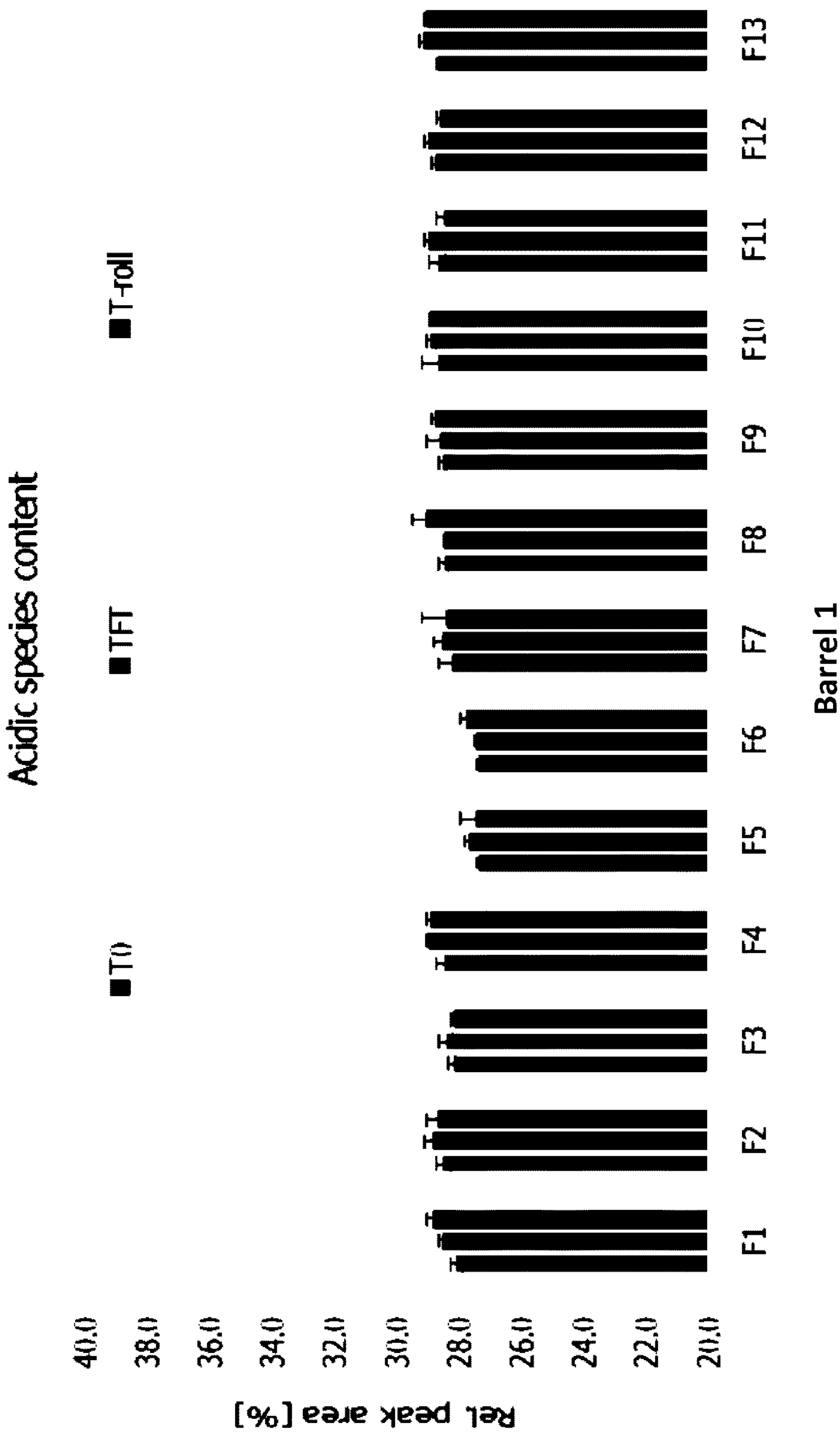
Acidic species content
T0
TFT
T-roll
Rel peak area [%]
40.0
38.0
36.0
34.0
32.0
30.0
28.0
26.0
24.0
22.0
20.0
F1
F2
F3
F4
F5
F6
F7
F8
F9
F10
F11
F12
F13
Barrel 1

FIG. 41B

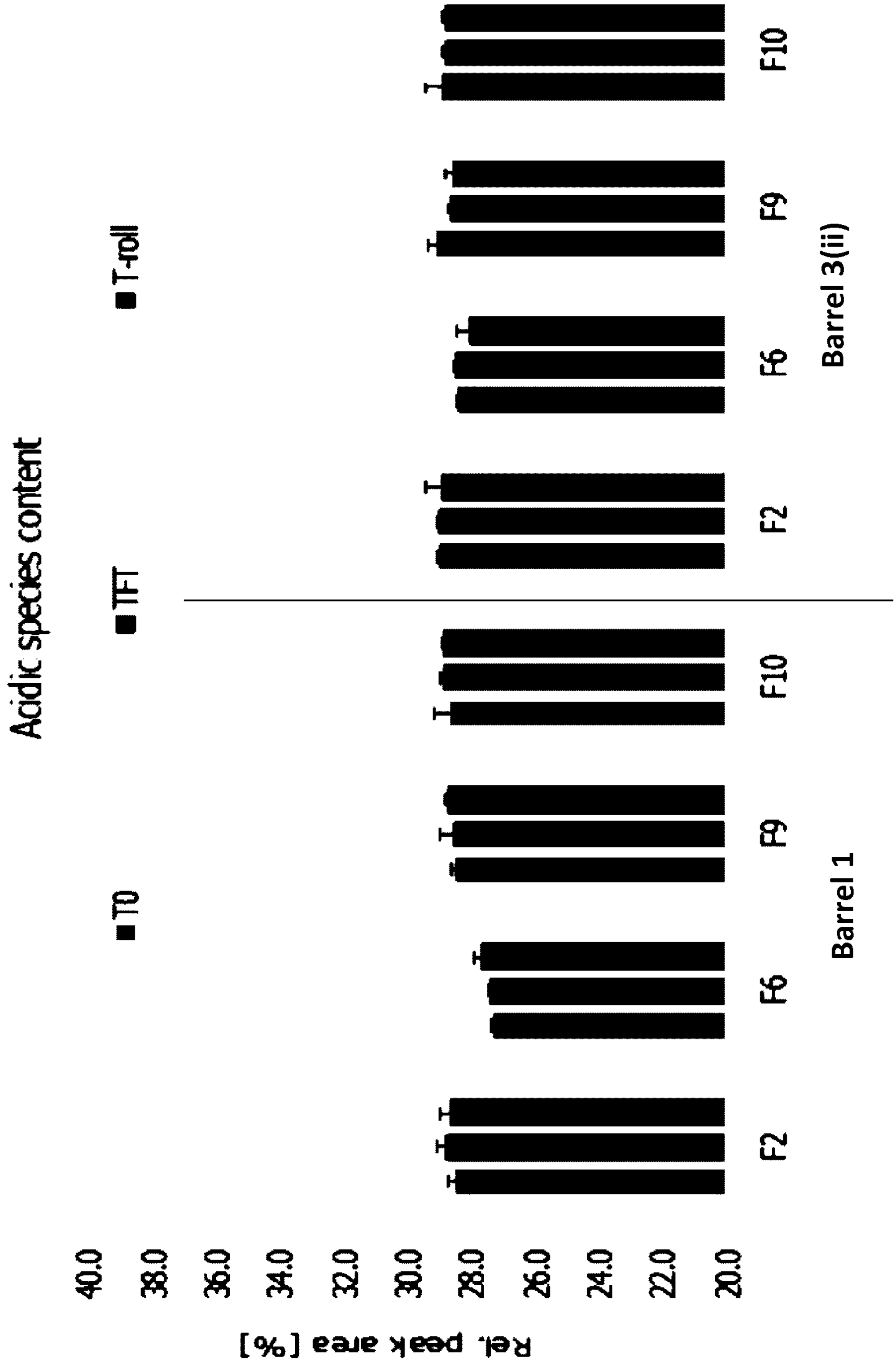
Acidic species content
T0
TFT
T-roll
Rel. peak area [%]
40.0
38.0
36.0
34.0
32.0
30.0
28.0
26.0
24.0
22.0
20.0
F2
F6
F9
F10
F2
F6
F9
F10
Barrel 1
Barrel 3(ii)

FIG. 42A

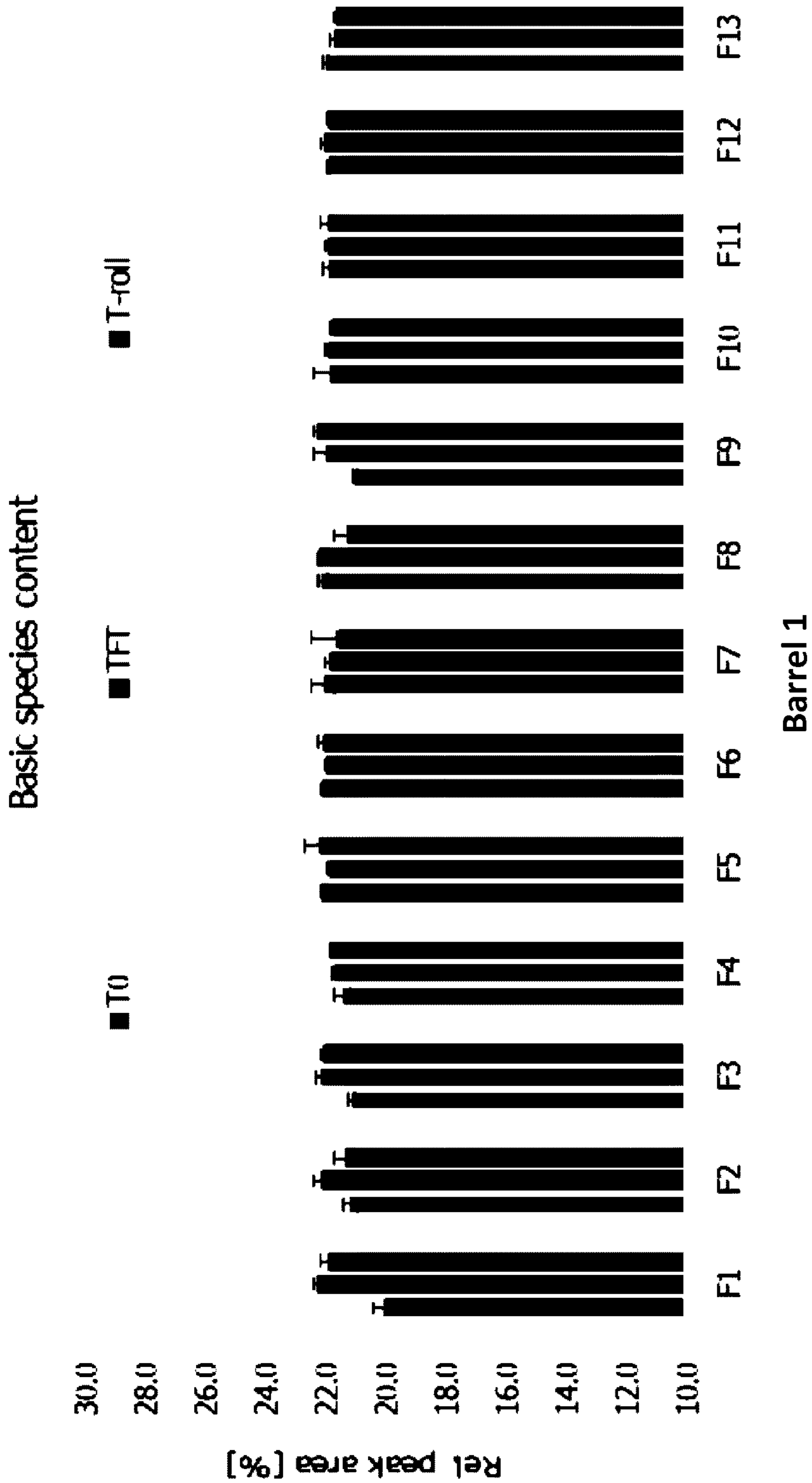
Basic species content
T0
TFT
T-roll
Rel peak area [%]
30.0
28.0
26.0
24.0
22.0
20.0
18.0
16.0
14.0
12.0
10.0
F1
F2
F3
F4
F5
F6
F7
F8
F9
F10
F11
F12
F13
Barrel 1

FIG. 42B

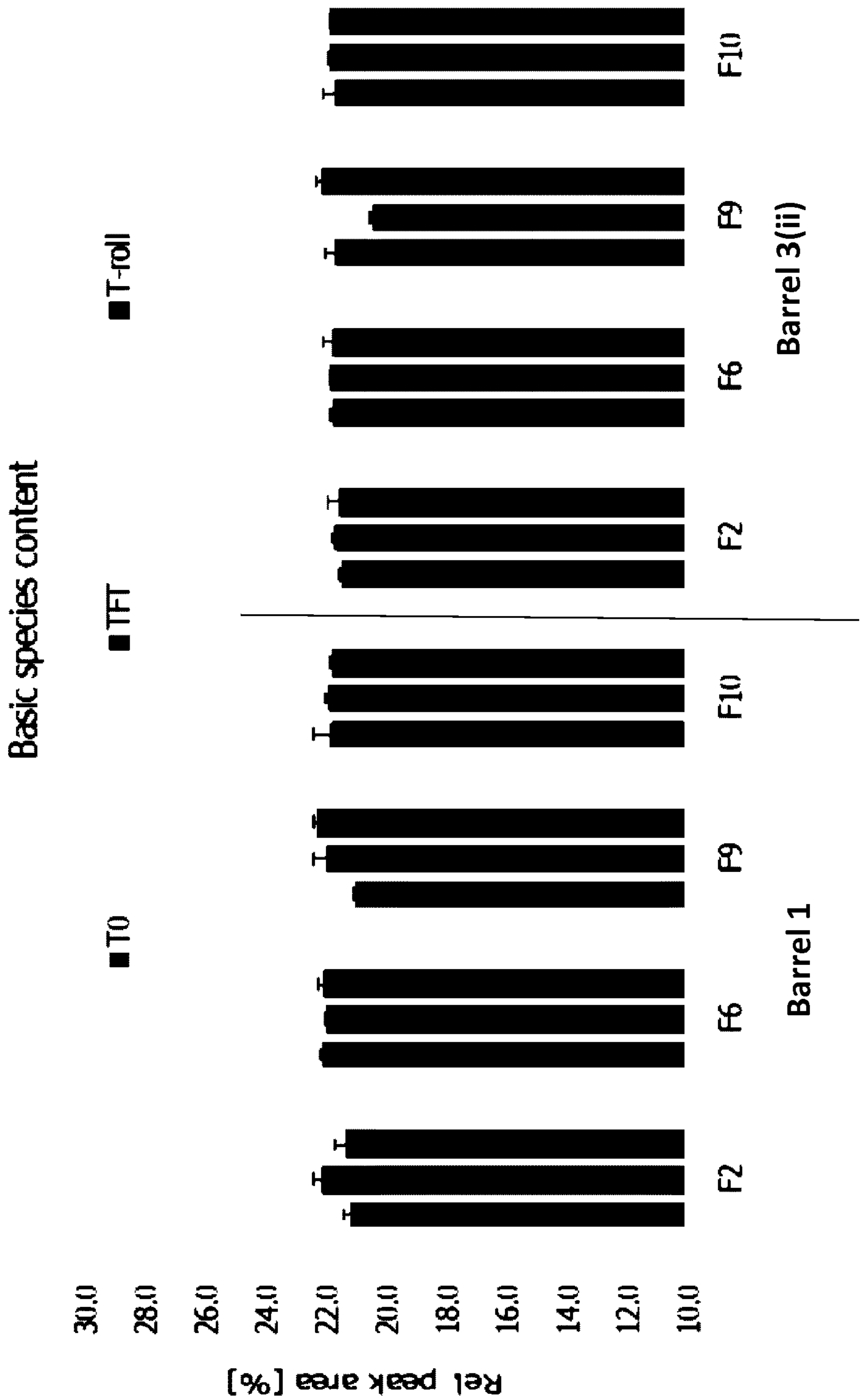
Basic species content
T0
TFT
T-roll
Rel peak area [%]
30.0
28.0
26.0
24.0
22.0
20.0
18.0
16.0
14.0
12.0
10.0
F2
F6
F9
F10
Barrel 1
F2
F6
F9
F10
Barrel 3(ii)

FIG. 43A

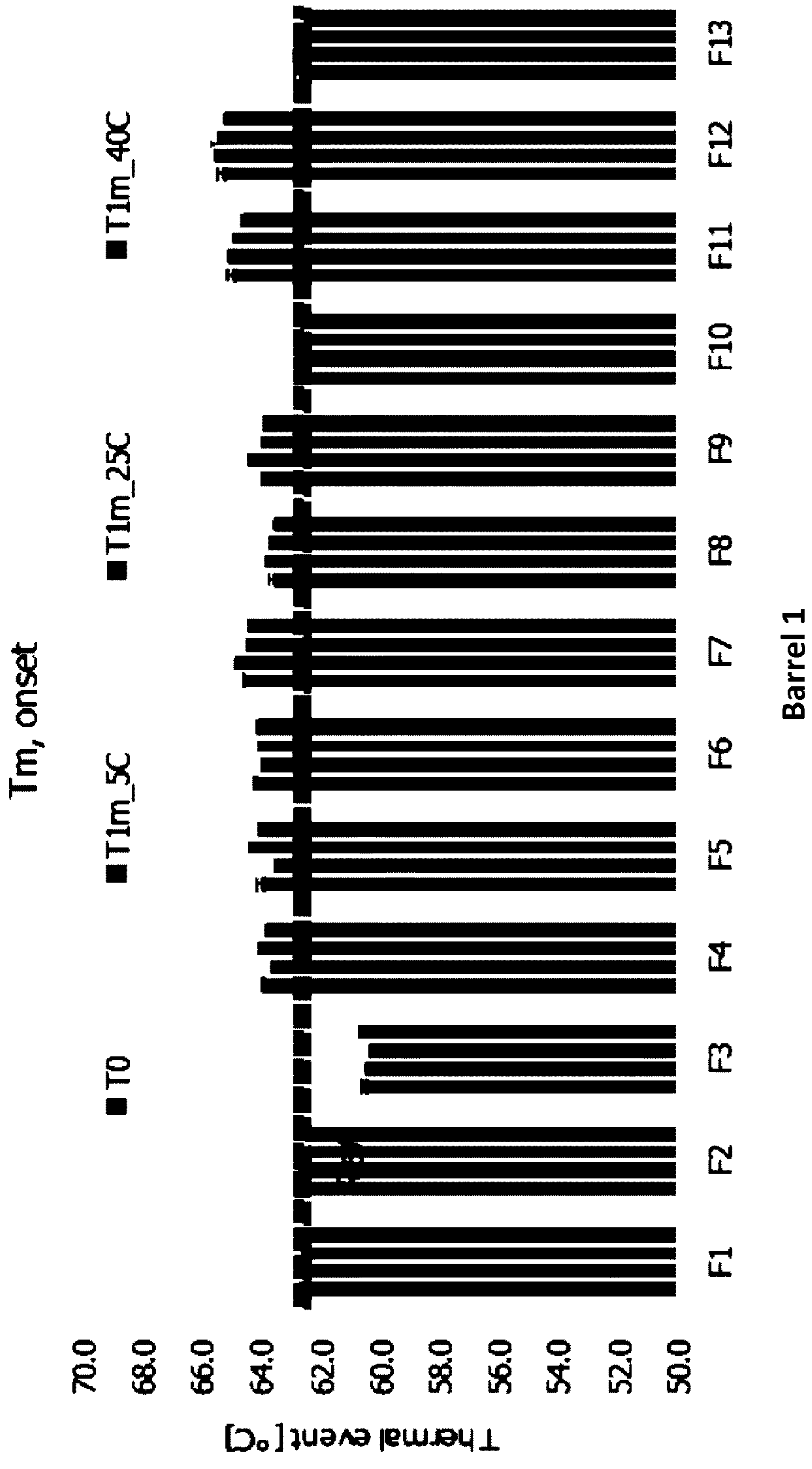
Tm, onset
T0
T1m_5C
T1m_25C
T1m_40C
Thermal event [°C]
70.0
68.0
66.0
64.0
62.0
60.0
58.0
56.0
54.0
52.0
50.0
F1
F2
F3
F4
F5
F6
F7
F8
F9
F10
F11
F12
F13
Barrel 1

FIG. 43B

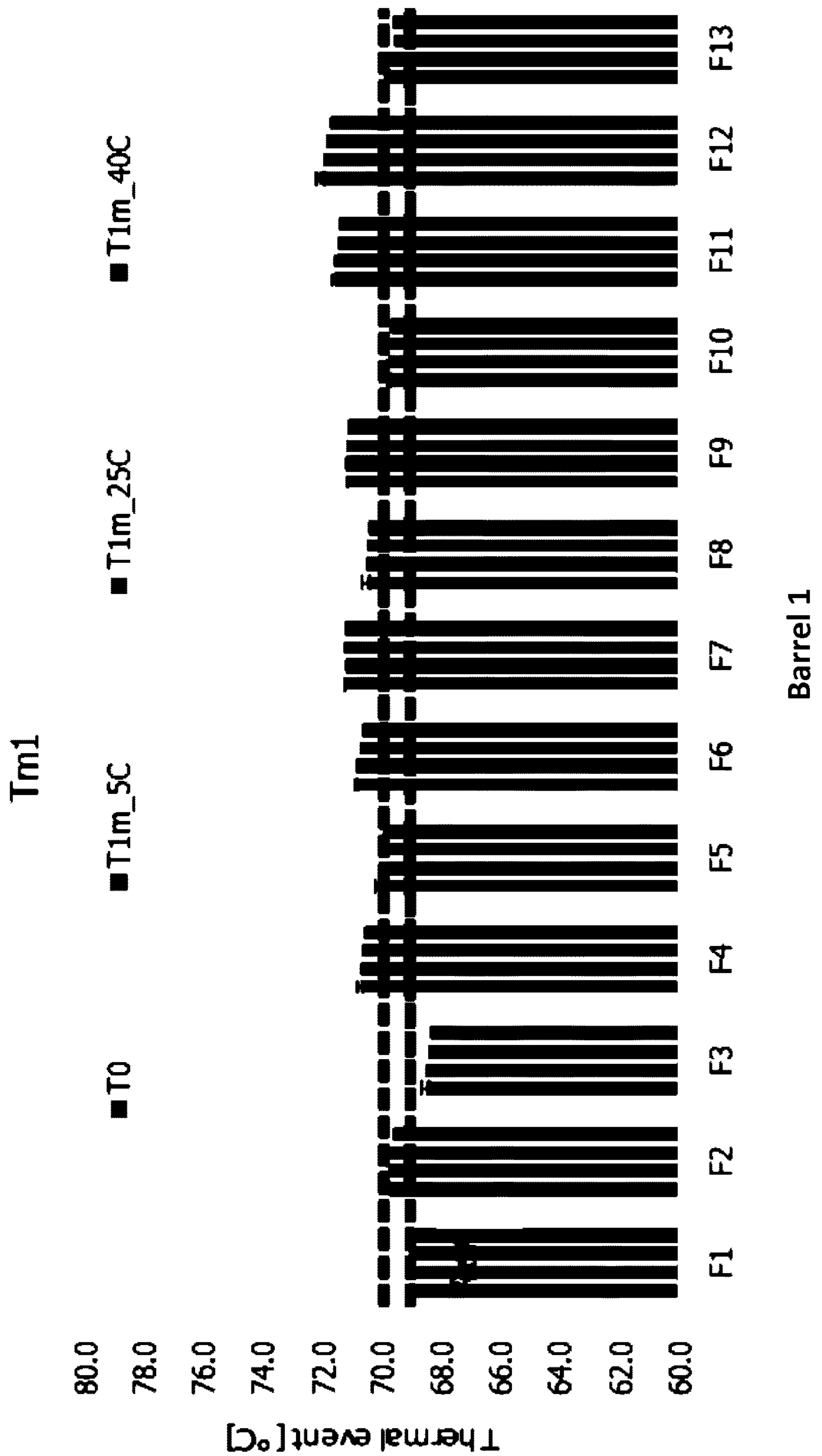
Tm1
T0
T1m_5C
T1m_25C
T1m_40C
Thermal event [°C]
80.0
78.0
76.0
74.0
72.0
70.0
68.0
66.0
64.0
62.0
60.0
F1
F2
F3
F4
F5
F6
F7
F8
F9
F10
F11
F12
F13
Barrel 1

FIG. 44

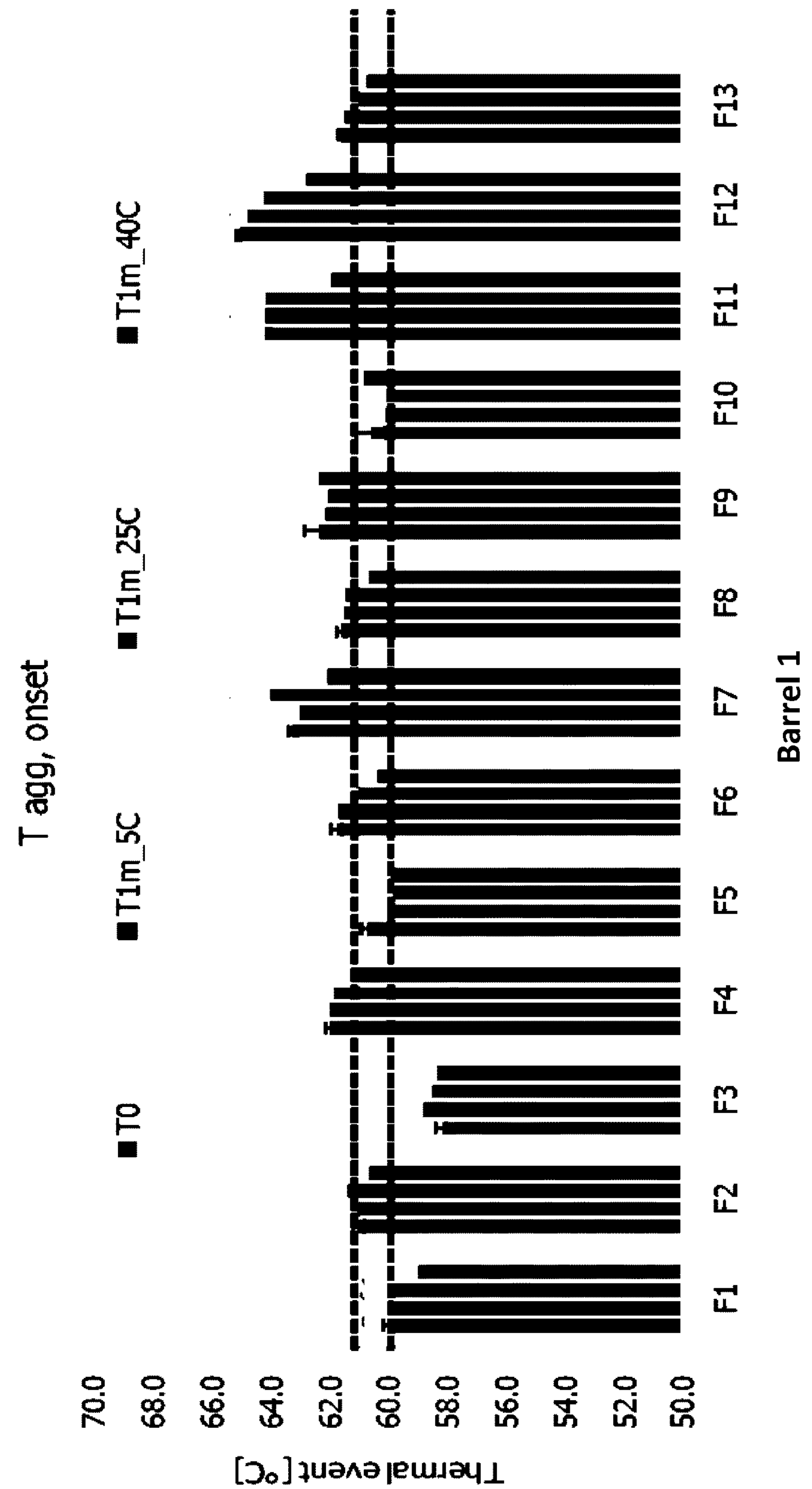
T agg, onset
T0
T1m_5C
T1m_25C
T1m_40C
Thermal event [°C]
70.0
68.0
66.0
64.0
62.0
60.0
58.0
56.0
54.0
52.0
50.0
F1
F2
F3
F4
F5
F6
F7
F8
F9
F10
F11
F12
F13
Barrel 1

FIG. 45A

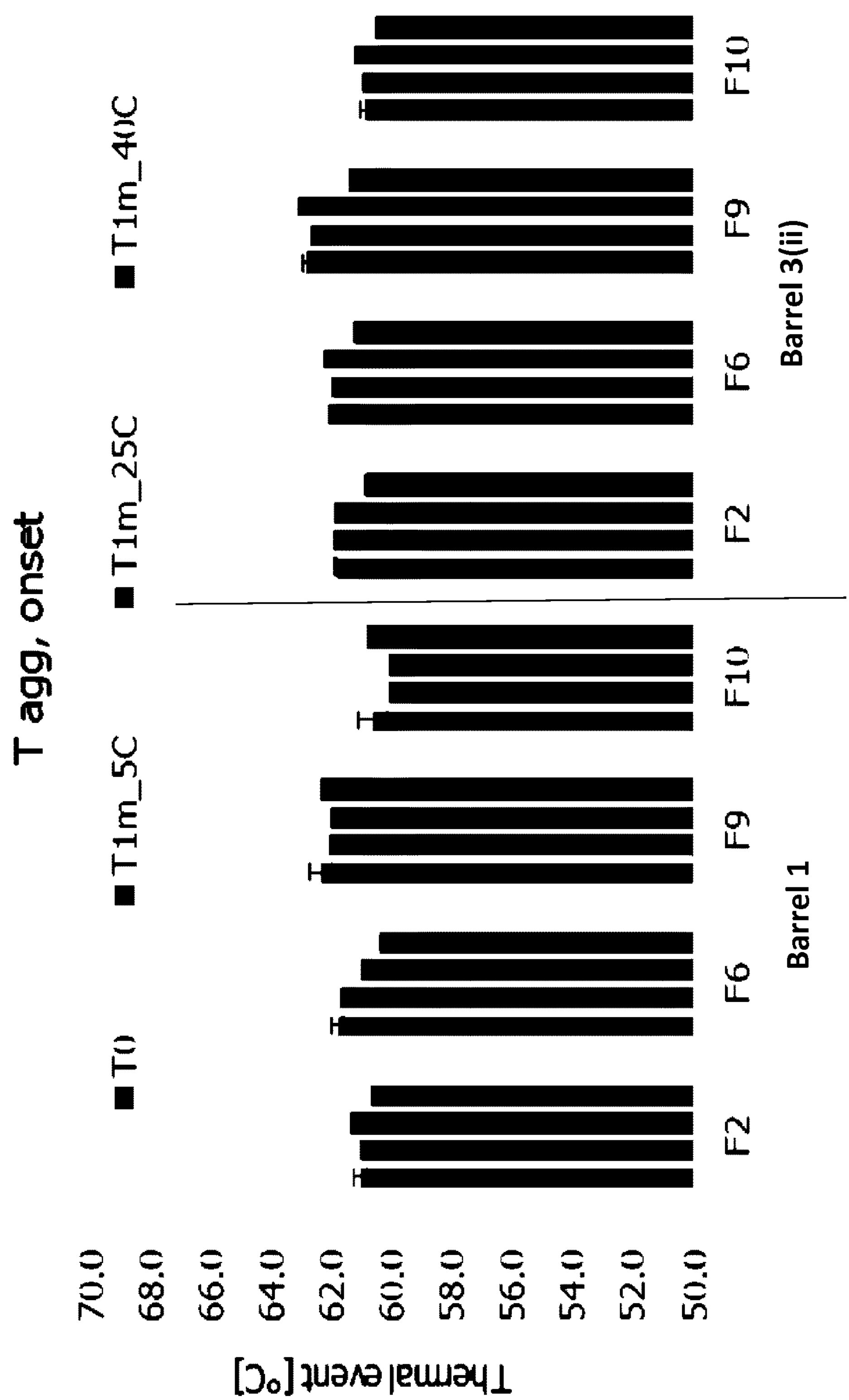
T agg, onset
T0
T1m_5C
T1m_25C
T1m_40C
Thermal event [°C]
70.0
68.0
66.0
64.0
62.0
60.0
58.0
56.0
54.0
52.0
50.0
F2
F6
F9
F10
F2
F6
F9
F10
Barrel 1
Barrel 3(ii)

FIG. 45B

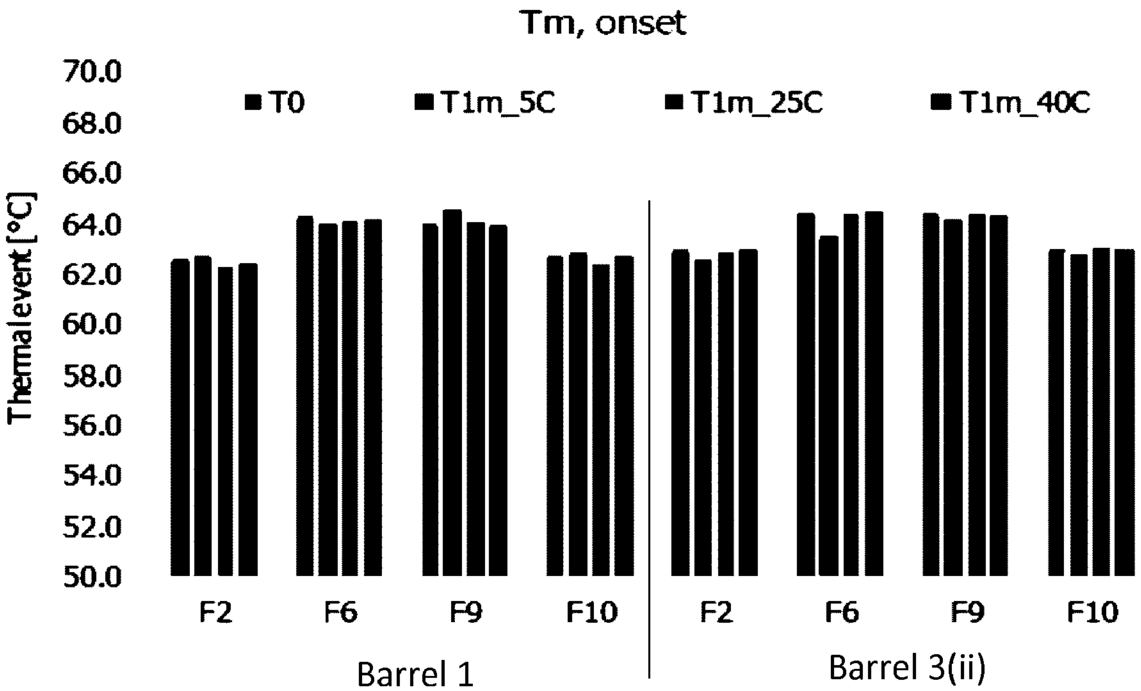
Tm, onset
70.0
68.0
66.0
64.0
62.0
60.0
58.0
56.0
54.0
52.0
50.0
T0
T1m_5C
T1m_25C
T1m_40C
Thermal event [°C]
F2
F6
F9
F10
F2
F6
F9
F10
Barrel 1
Barrel 3(ii)

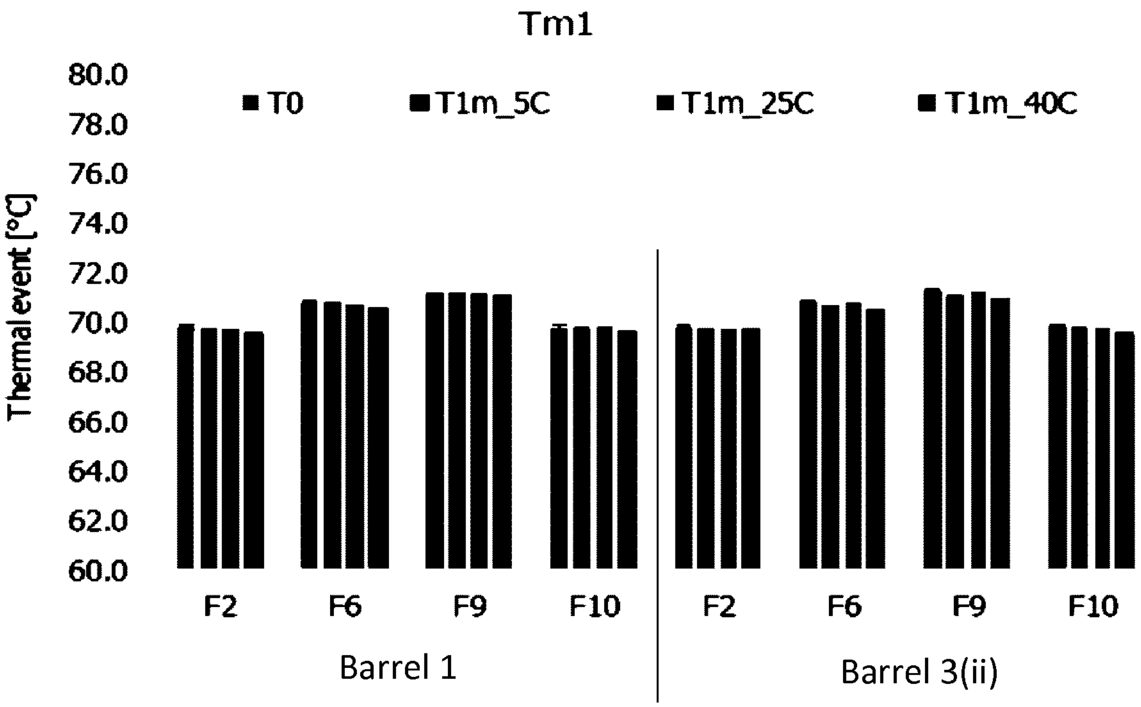
Tm1
80.0
78.0
76.0
74.0
72.0
70.0
68.0
66.0
64.0
62.0
60.0
T0
T1m_5C
T1m_25C
T1m_40C
Thermal event [°C]
F2
F6
F9
F10
F2
F6
F9
F10
Barrel 1
Barrel 3(ii)

FIG. 46A

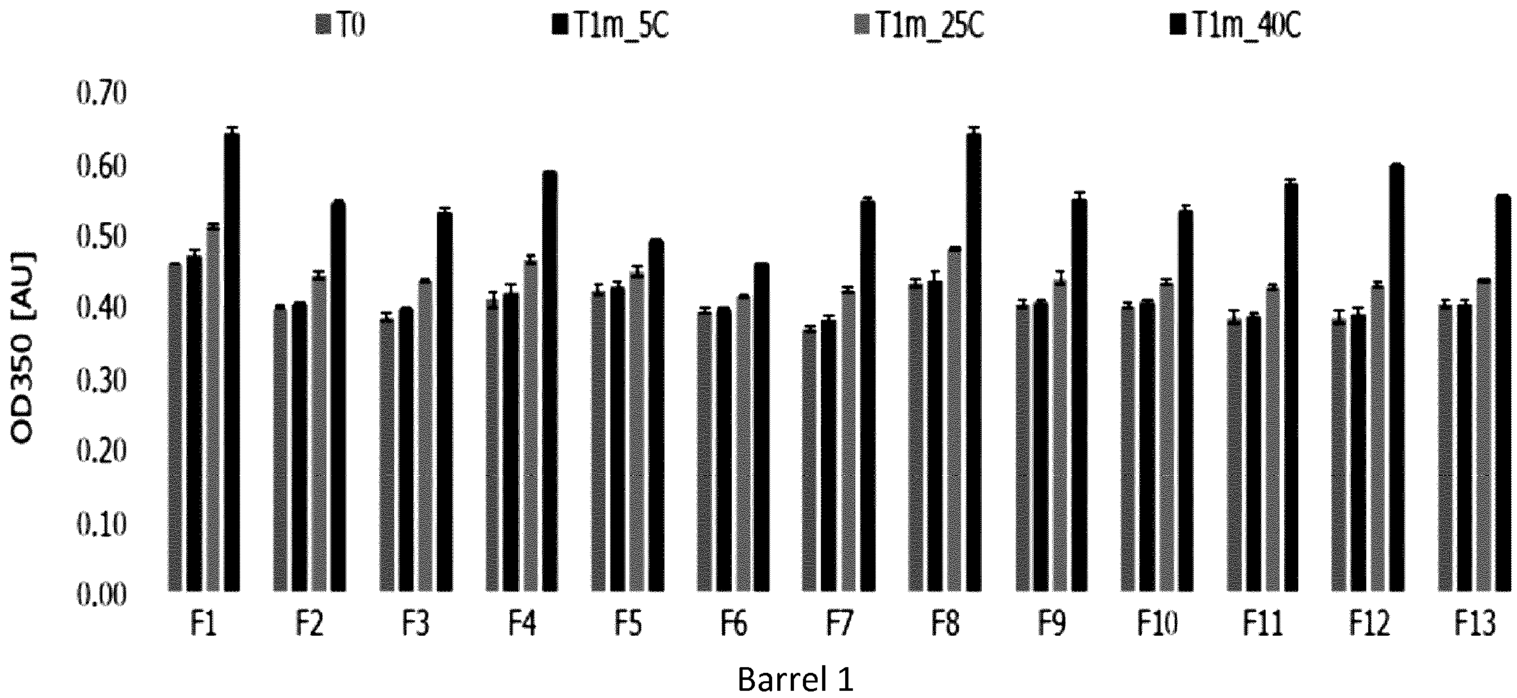


| | F1 | F2 | F3 | F4 | F5 | F6 | F7 | F8 | F9 | F10 | F11 | F12 | F13 |
|---|---|---|---|---|---|---|---|---|---|---|---|---|---|
| T0 | 0.462 | 0.399 | 0.386 | 0.411 | 0.423 | 0.394 | 0.370 | 0.434 | 0.405 | 0.402 | 0.386 | 0.386 | 0.404 |
| T1m_5C | 0.471 | 0.403 | 0.397 | 0.421 | 0.427 | 0.396 | 0.382 | 0.436 | 0.407 | 0.405 | 0.388 | 0.391 | 0.405 |
| T1m_25C | 0.512 | 0.444 | 0.436 | 0.466 | 0.449 | 0.414 | 0.424 | 0.481 | 0.441 | 0.435 | 0.428 | 0.431 | 0.439 |
| T1m_40C | 0.644 | 0.544 | 0.532 | 0.588 | 0.491 | 0.461 | 0.548 | 0.642 | 0.552 | 0.535 | 0.573 | 0.596 | 0.554 |

FIG. 46B

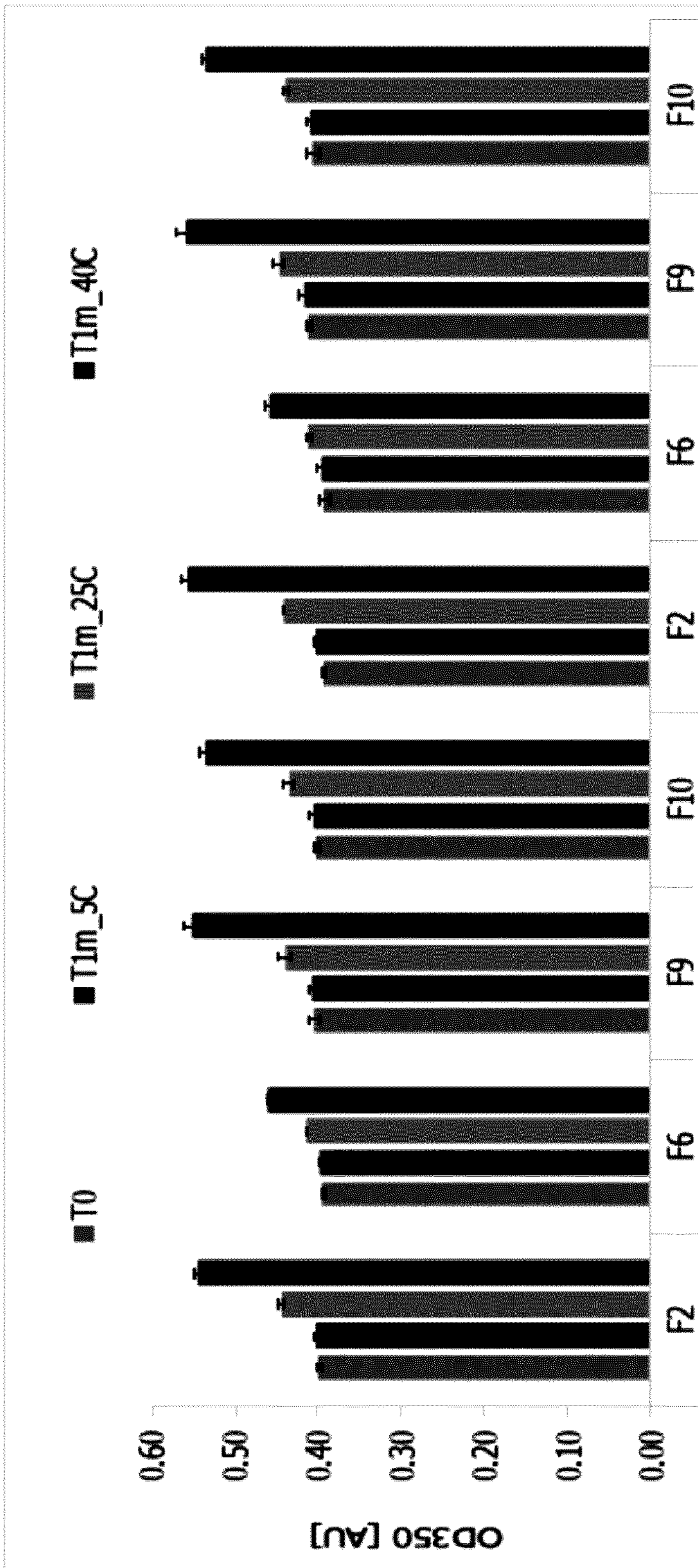


| | Barrel 1 | | | | Barrel 3(ii) | | | |
|---|---|---|---|---|---|---|---|---|
| | F2 | F6 | F9 | F10 | F2 | F6 | F9 | F10 |
| T0 | 0.399 | 0.394 | 0.405 | 0.402 | 0.393 | 0.392 | 0.411 | 0.408 |
| T1m_5C | 0.403 | 0.396 | 0.407 | 0.405 | 0.402 | 0.395 | 0.417 | 0.410 |
| T1m_25C | 0.444 | 0.414 | 0.441 | 0.435 | 0.442 | 0.412 | 0.447 | 0.439 |
| T1m_40C | 0.544 | 0.461 | 0.552 | 0.535 | 0.557 | 0.459 | 0.558 | 0.535 |

FIG. 47A

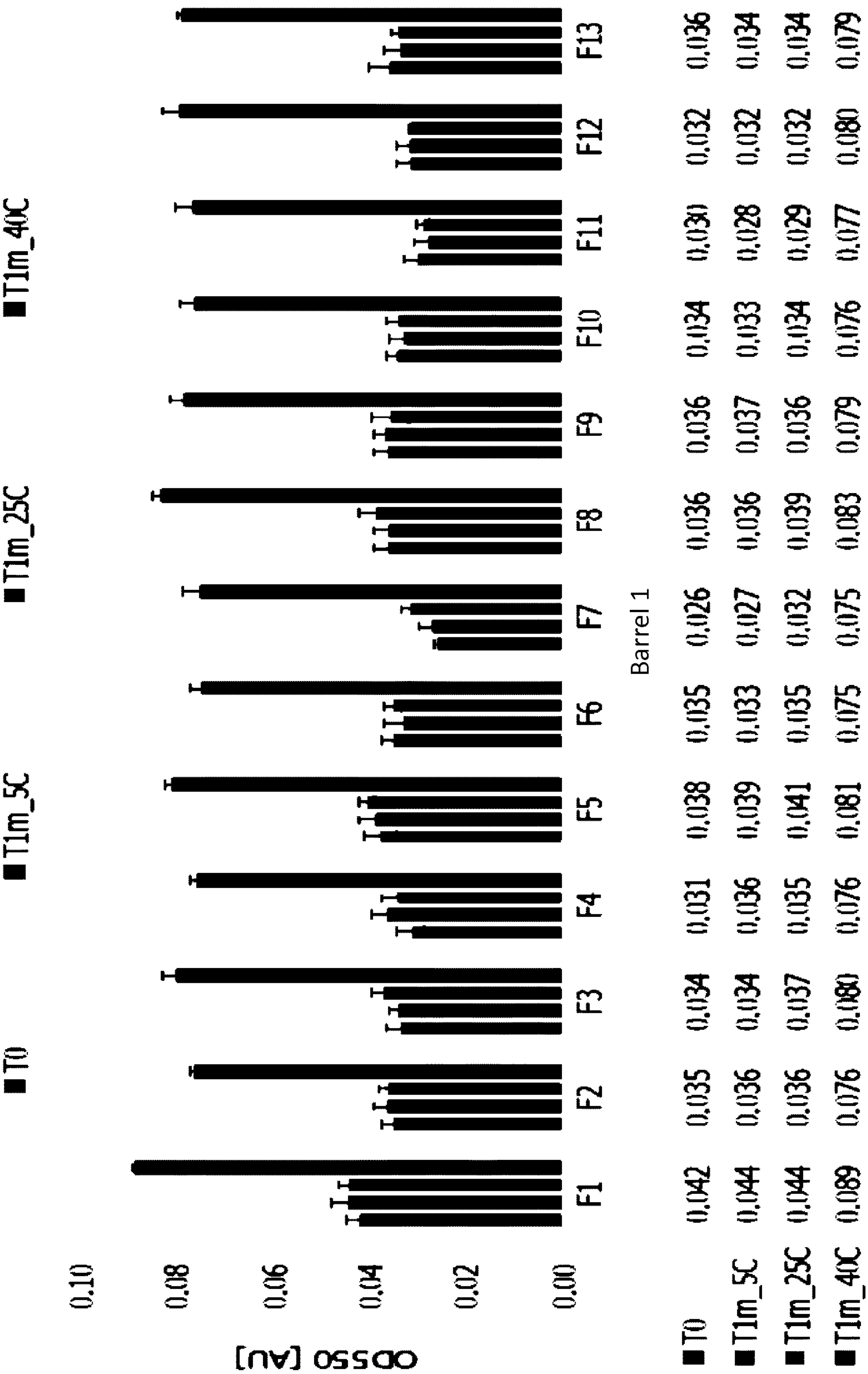
T0
T1m_5C
T1m_25C
T1m_40C
OD550 [AU]
0.10
0.08
0.06
0.04
0.02
0.00
Barrel 1
F1 F2 F3 F4 F5 F6 F7 F8 F9 F10 F11 F12 F13
T0 0.042 0.035 0.034 0.031 0.038 0.035 0.026 0.036 0.036 0.034 0.030 0.032 0.036
T1m_5C 0.044 0.036 0.034 0.036 0.039 0.033 0.027 0.036 0.037 0.033 0.028 0.032 0.034
T1m_25C 0.044 0.036 0.037 0.035 0.041 0.035 0.032 0.039 0.036 0.034 0.029 0.032 0.034
T1m_40C 0.089 0.076 0.080 0.076 0.081 0.075 0.075 0.083 0.079 0.076 0.077 0.080 0.079

FIG. 47B

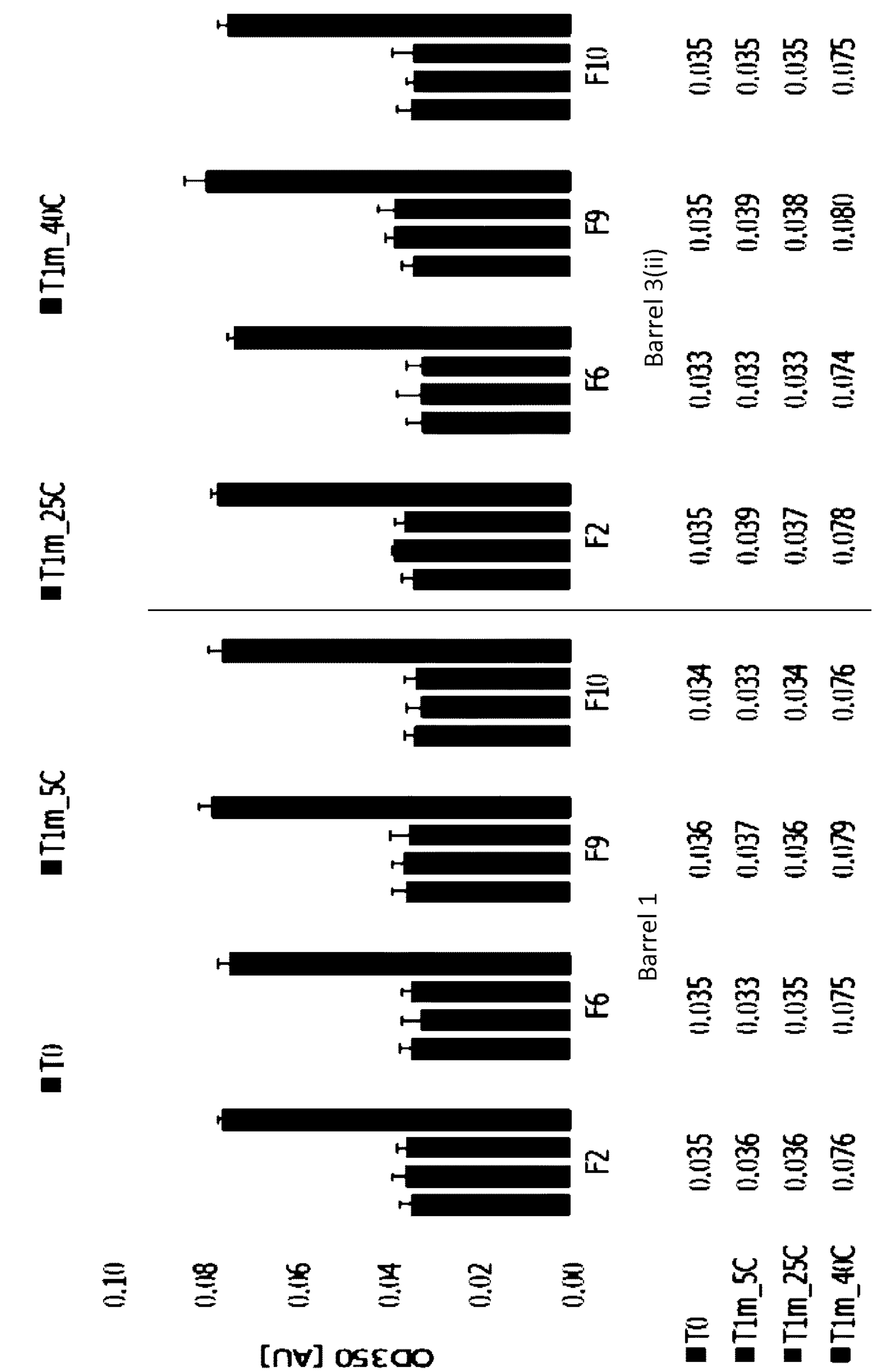
T0
T1m_5C
T1m_25C
T1m_40C
OD350 [AU]
0.10
0.08
0.06
0.04
0.02
0.00
Barrel 1
F2 0.035 0.036 0.036 0.076
F6 0.035 0.033 0.035 0.075
F9 0.036 0.037 0.036 0.079
F10 0.034 0.033 0.034 0.076
Barrel 3(ii)
F2 0,035 0,039 0,037 0,078
F6 0,033 0,033 0,033 0,074
F9 0,035 0,039 0,038 0,080
F10 0,035 0,035 0,035 0,075

FIG. 48

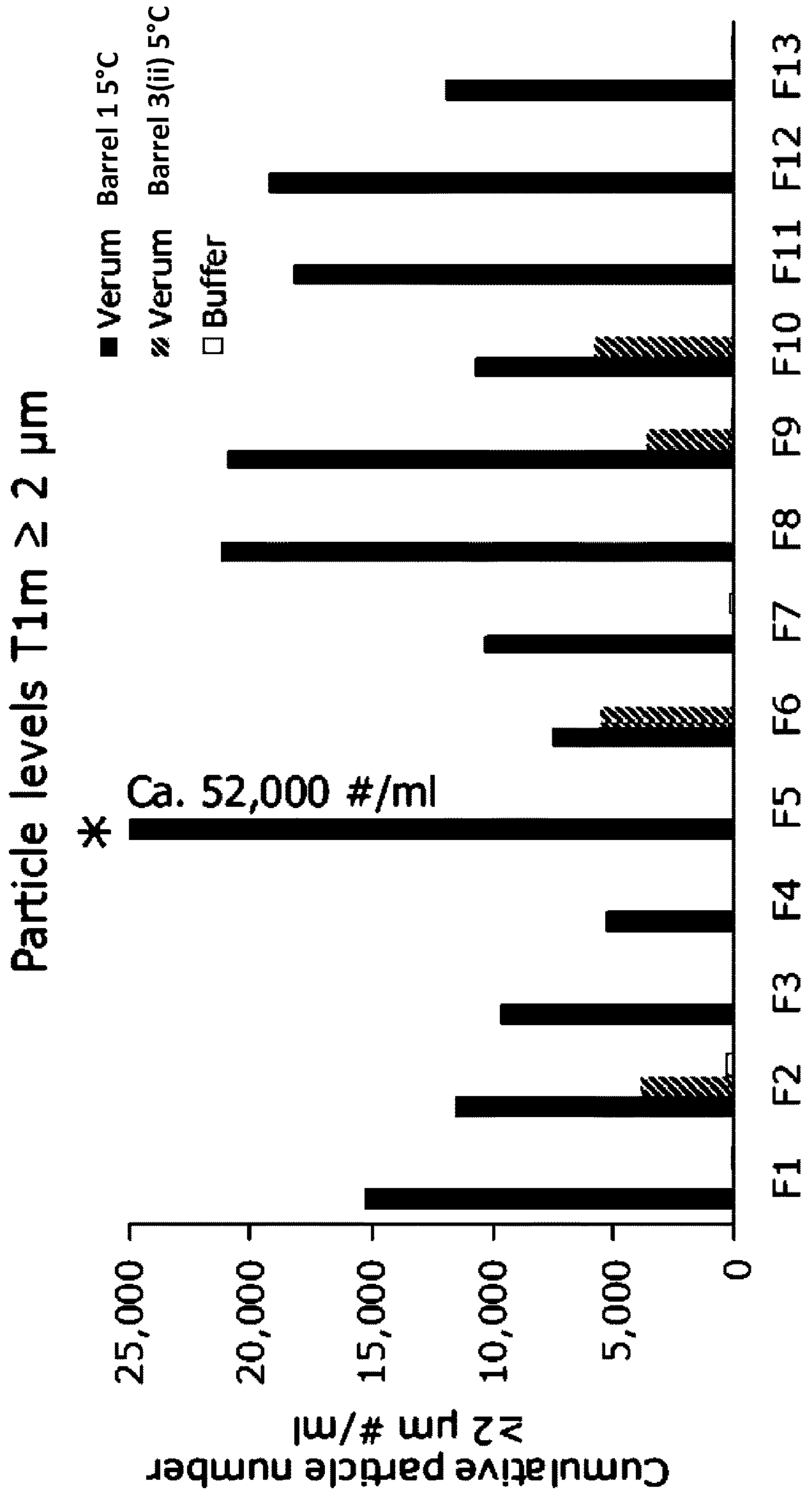
Particle levels T1m ≥ 2 µm
Verum Barrel 1 5°C
Verum Barrel 3(ii) 5°C
Buffer
Ca. 52,000 #/ml
Cumulative particle number
≥2 µm #/ml
25,000
20,000
15,000
10,000
5,000
0
F1
F2
F3
F4
F5
F6
F7
F8
F9
F10
F11
F12
F13

FIG. 49

T1m

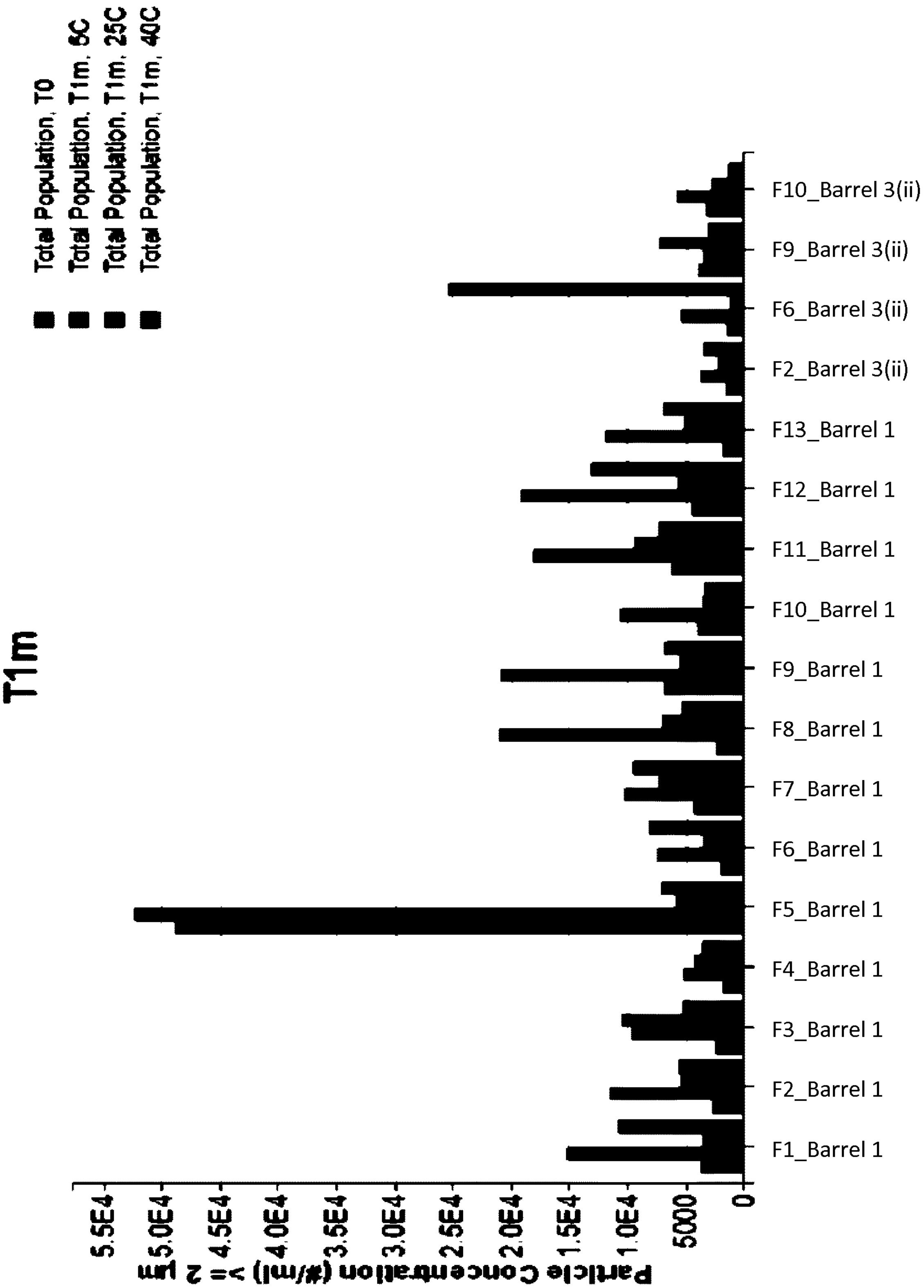
Total Population, T0
Total Population, T1m, 5C
Total Population, T1m, 25C
Total Population, T1m, 40C
Particle Concentration (#/ml) >= 2 µm
5.5E4
5.0E4
4.5E4
4.0E4
3.5E4
3.0E4
2.5E4
2.0E4
1.5E4
1.0E4
5000
0
F1_Barrel 1
F2_Barrel 1
F3_Barrel 1
F4_Barrel 1
F5_Barrel 1
F6_Barrel 1
F7_Barrel 1
F8_Barrel 1
F9_Barrel 1
F10_Barrel 1
F11_Barrel 1
F12_Barrel 1
F13_Barrel 1
F2_Barrel 3(ii)
F6_Barrel 3(ii)
F9_Barrel 3(ii)
F10_Barrel 3(ii)

FIG. 50A

T0

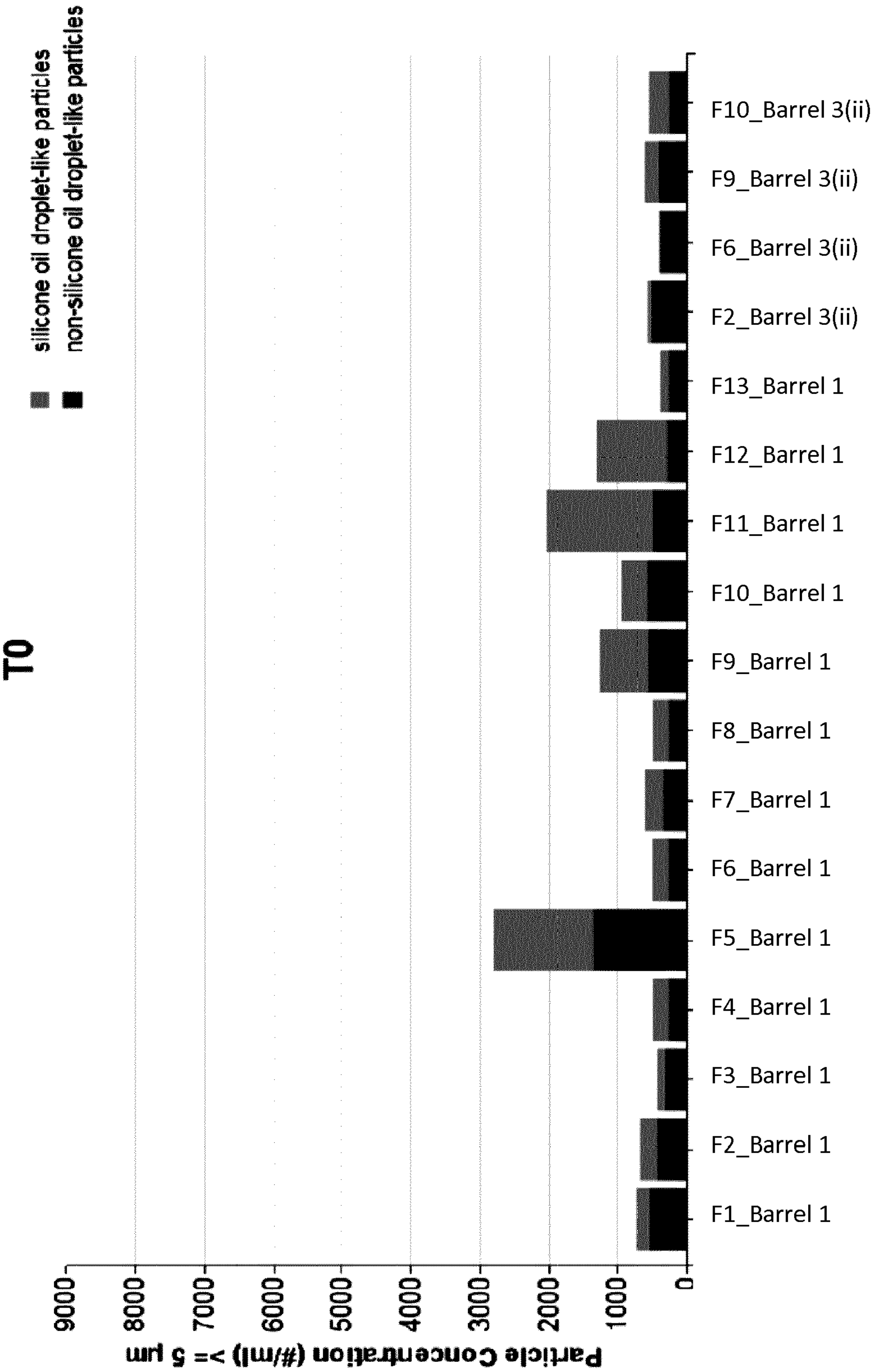
silicone oil droplet-like particles
non-silicone oil droplet-like particles
Particle Concentration (#/ml) >= 5 µm
9000
8000
7000
6000
5000
4000
3000
2000
1000
0
F1_Barrel 1
F2_Barrel 1
F3_Barrel 1
F4_Barrel 1
F5_Barrel 1
F6_Barrel 1
F7_Barrel 1
F8_Barrel 1
F9_Barrel 1
F10_Barrel 1
F11_Barrel 1
F12_Barrel 1
F13_Barrel 1
F2_Barrel 3(ii)
F6_Barrel 3(ii)
F9_Barrel 3(ii)
F10_Barrel 3(ii)

FIG. 50B

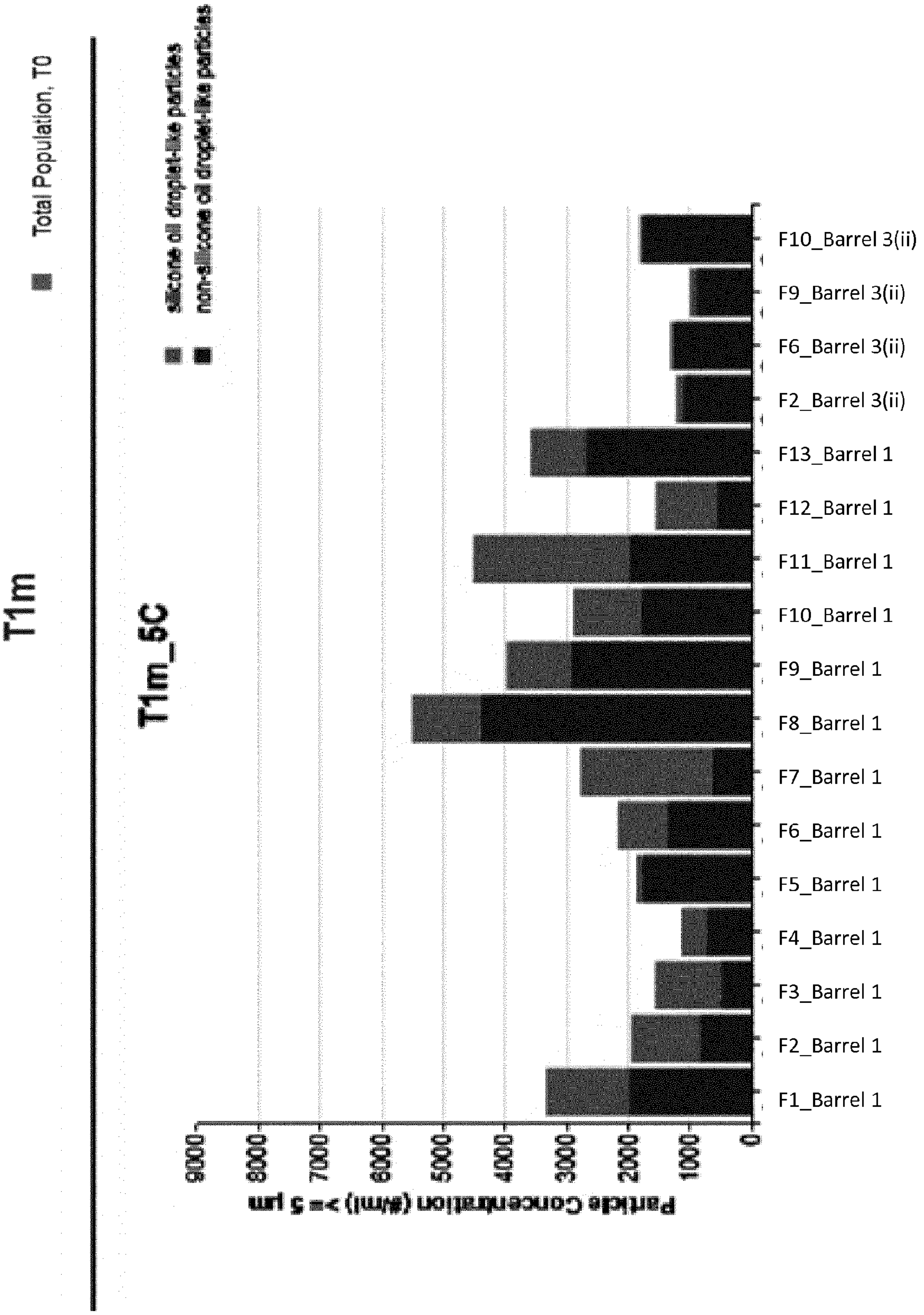
T1m
Total Population, T0
T1m_5C
silicone oil droplet-like particles
non-silicone oil droplet-like particles
Particle Concentration (#/ml) >= 5 µm
9000
8000
7000
6000
5000
4000
3000
2000
1000
0
F1_Barrel 1
F2_Barrel 1
F3_Barrel 1
F4_Barrel 1
F5_Barrel 1
F6_Barrel 1
F7_Barrel 1
F8_Barrel 1
F9_Barrel 1
F10_Barrel 1
F11_Barrel 1
F12_Barrel 1
F13_Barrel 1
F2_Barrel 3(ii)
F6_Barrel 3(ii)
F9_Barrel 3(ii)
F10_Barrel 3(ii)

FIG. 50C

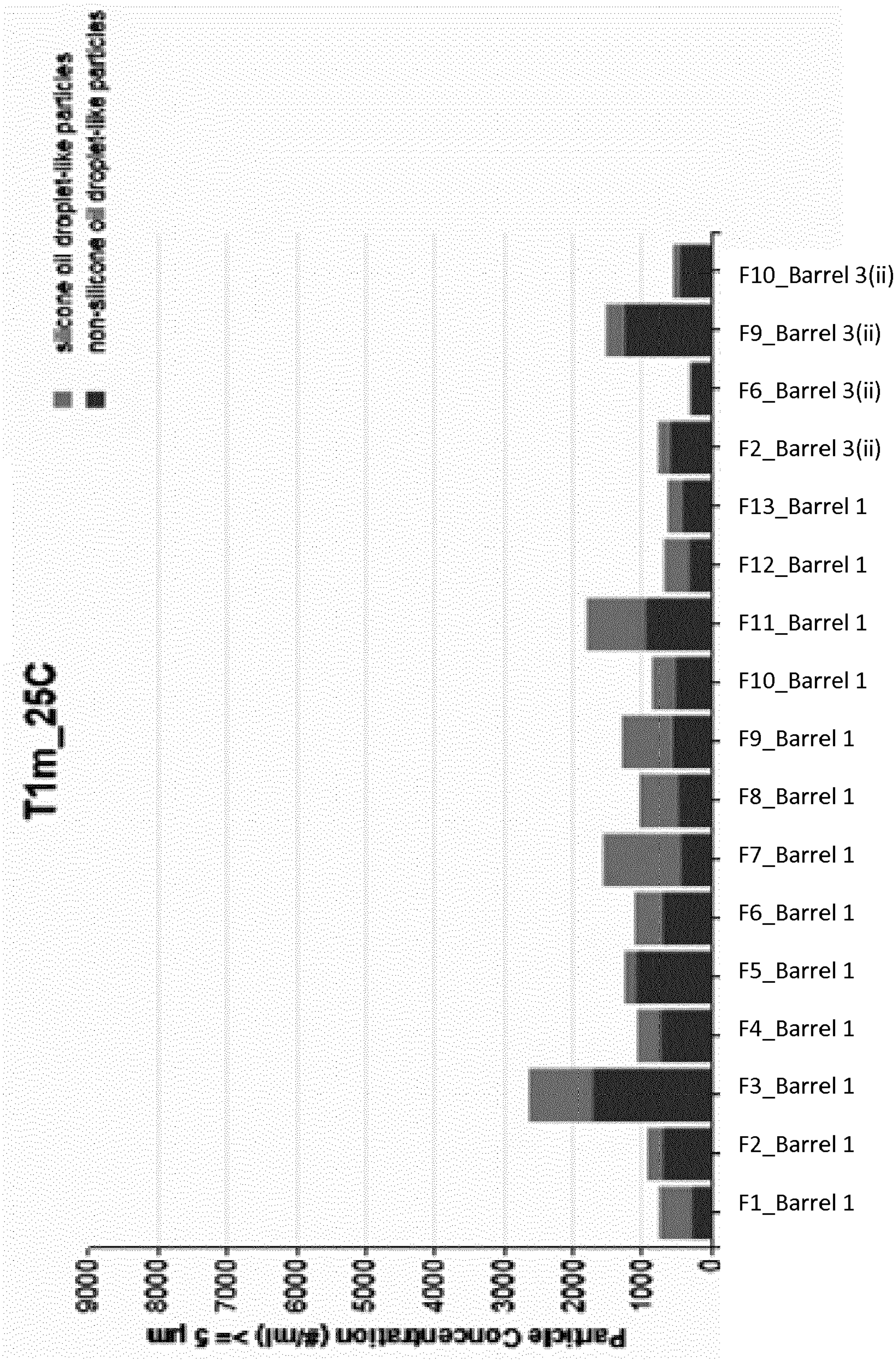
T1m_25C
silicone oil droplet-like particles
non-silicone oil droplet-like particles
Particle Concentration (#/ml) >= 5 µm
9000
8000
7000
6000
5000
4000
3000
2000
1000
0
F10_Barrel 3(ii)
F9_Barrel 3(ii)
F6_Barrel 3(ii)
F2_Barrel 3(ii)
F13_Barrel 1
F12_Barrel 1
F11_Barrel 1
F10_Barrel 1
F9_Barrel 1
F8_Barrel 1
F7_Barrel 1
F6_Barrel 1
F5_Barrel 1
F4_Barrel 1
F3_Barrel 1
F2_Barrel 1
F1_Barrel 1

FIG. 50D

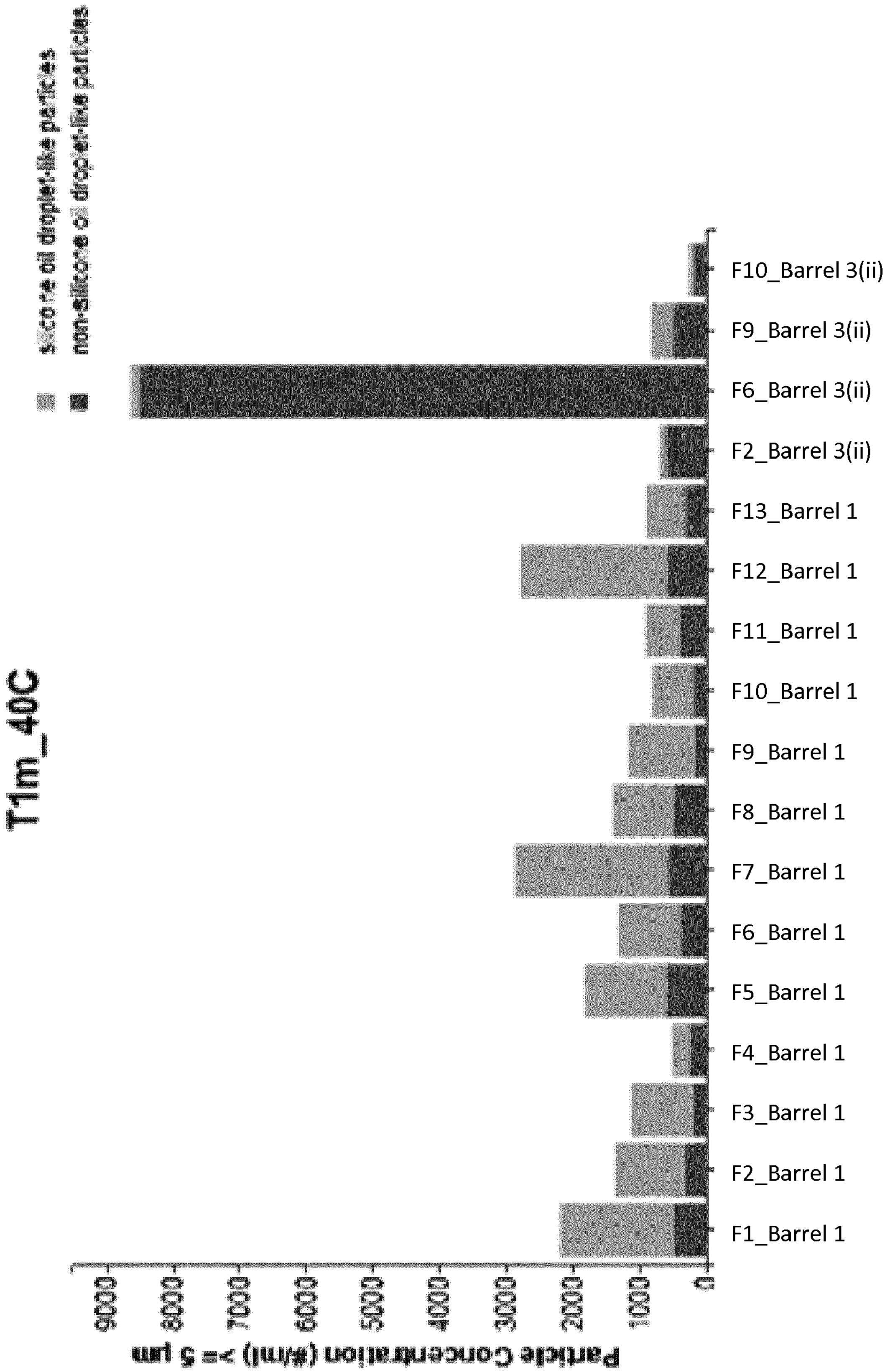
T1m_40C
silicone oil droplet-like particles
non-silicone oil droplet-like particles
Particle Concentration (#/ml) >= 5 μm
9000
8000
7000
6000
5000
4000
3000
2000
1000
0
F1_Barrel 1
F2_Barrel 1
F3_Barrel 1
F4_Barrel 1
F5_Barrel 1
F6_Barrel 1
F7_Barrel 1
F8_Barrel 1
F9_Barrel 1
F10_Barrel 1
F11_Barrel 1
F12_Barrel 1
F13_Barrel 1
F2_Barrel 3(ii)
F6_Barrel 3(ii)
F9_Barrel 3(ii)
F10_Barrel 3(ii)

FIG. 51A

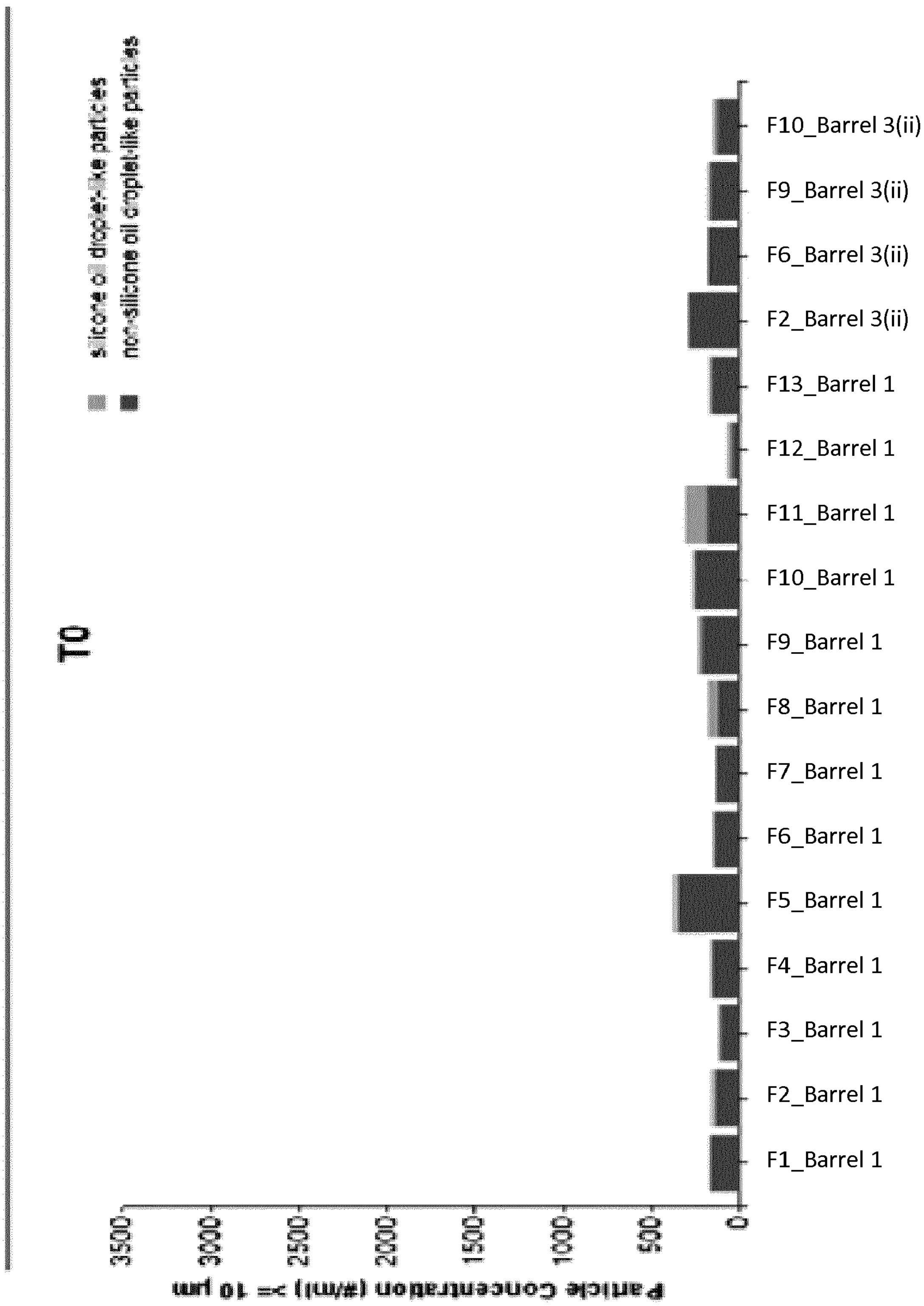
T0
silicone oil droplet-like particles
non-silicone oil droplet-like particles
Particle Concentration (#/ml) >= 10 µm
0
500
1000
1500
2000
2500
3000
3500
F1_Barrel 1
F2_Barrel 1
F3_Barrel 1
F4_Barrel 1
F5_Barrel 1
F6_Barrel 1
F7_Barrel 1
F8_Barrel 1
F9_Barrel 1
F10_Barrel 1
F11_Barrel 1
F12_Barrel 1
F13_Barrel 1
F2_Barrel 3(ii)
F6_Barrel 3(ii)
F9_Barrel 3(ii)
F10_Barrel 3(ii)

FIG. 51B

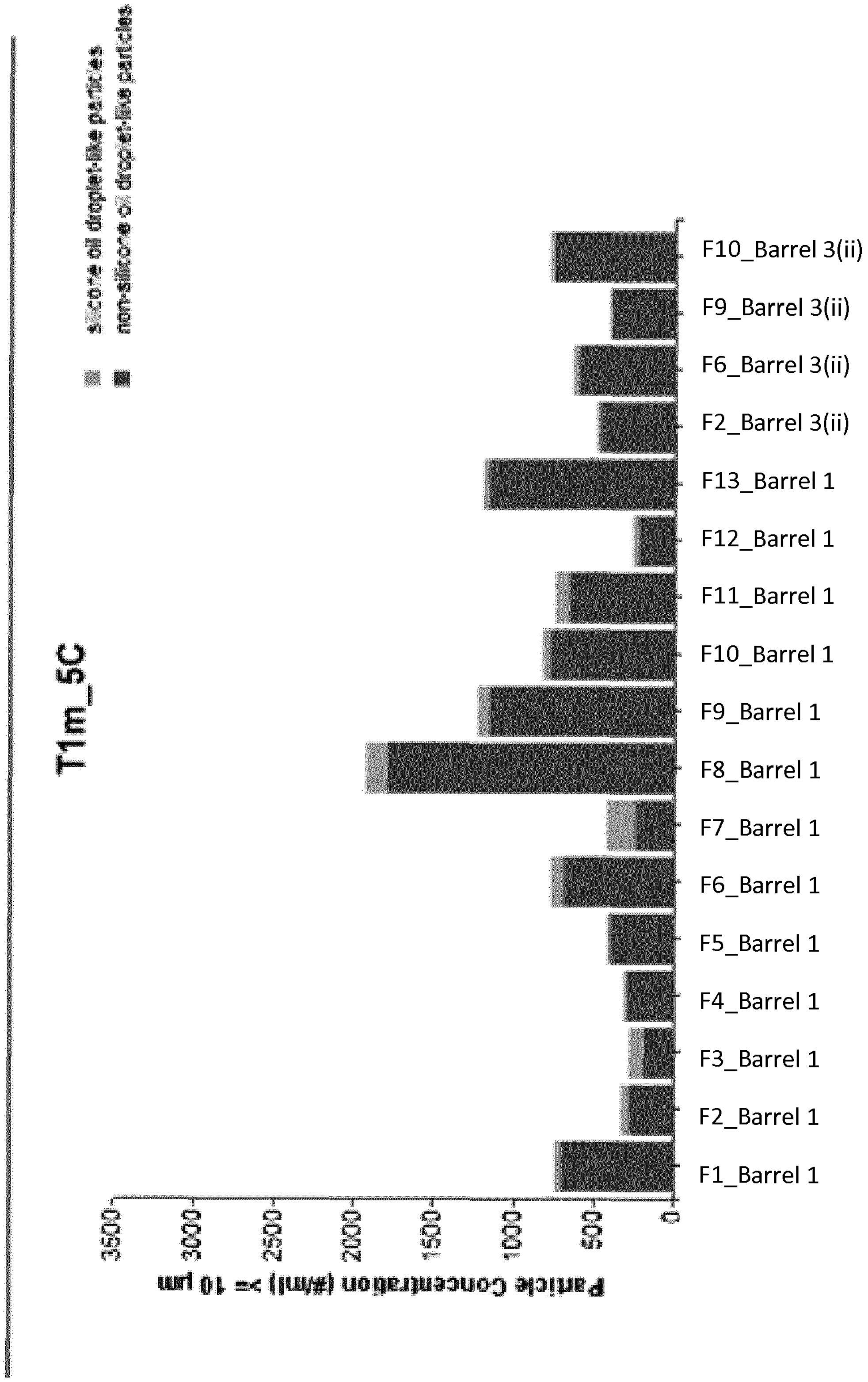
T1m_5C
silicone oil droplet-like particles
non-silicone oil droplet-like particles
Particle Concentration (#/ml) >= 10 µm
3500
3000
2500
2000
1500
1000
500
0
F1_Barrel 1
F2_Barrel 1
F3_Barrel 1
F4_Barrel 1
F5_Barrel 1
F6_Barrel 1
F7_Barrel 1
F8_Barrel 1
F9_Barrel 1
F10_Barrel 1
F11_Barrel 1
F12_Barrel 1
F13_Barrel 1
F2_Barrel 3(ii)
F6_Barrel 3(ii)
F9_Barrel 3(ii)
F10_Barrel 3(ii)

FIG. 51C

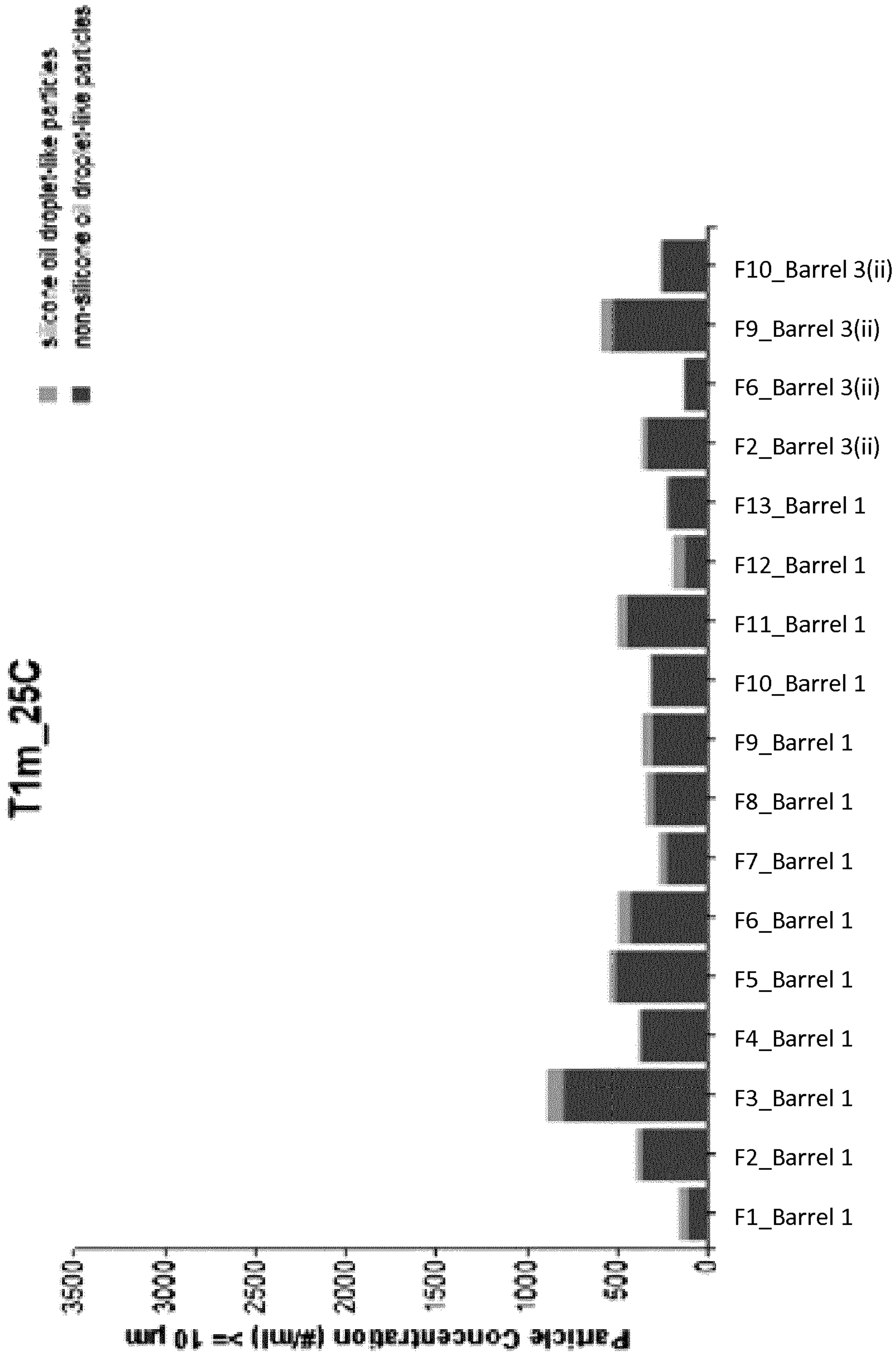
T1m_25C
silicone oil droplet-like particles
non-silicone oil droplet-like particles
Particle Concentration (#/ml) >= 10 µm
3500
3000
2500
2000
1500
1000
500
0
F1_Barrel 1
F2_Barrel 1
F3_Barrel 1
F4_Barrel 1
F5_Barrel 1
F6_Barrel 1
F7_Barrel 1
F8_Barrel 1
F9_Barrel 1
F10_Barrel 1
F11_Barrel 1
F12_Barrel 1
F13_Barrel 1
F2_Barrel 3(ii)
F6_Barrel 3(ii)
F9_Barrel 3(ii)
F10_Barrel 3(ii)

FIG. 51D

T1m_40C

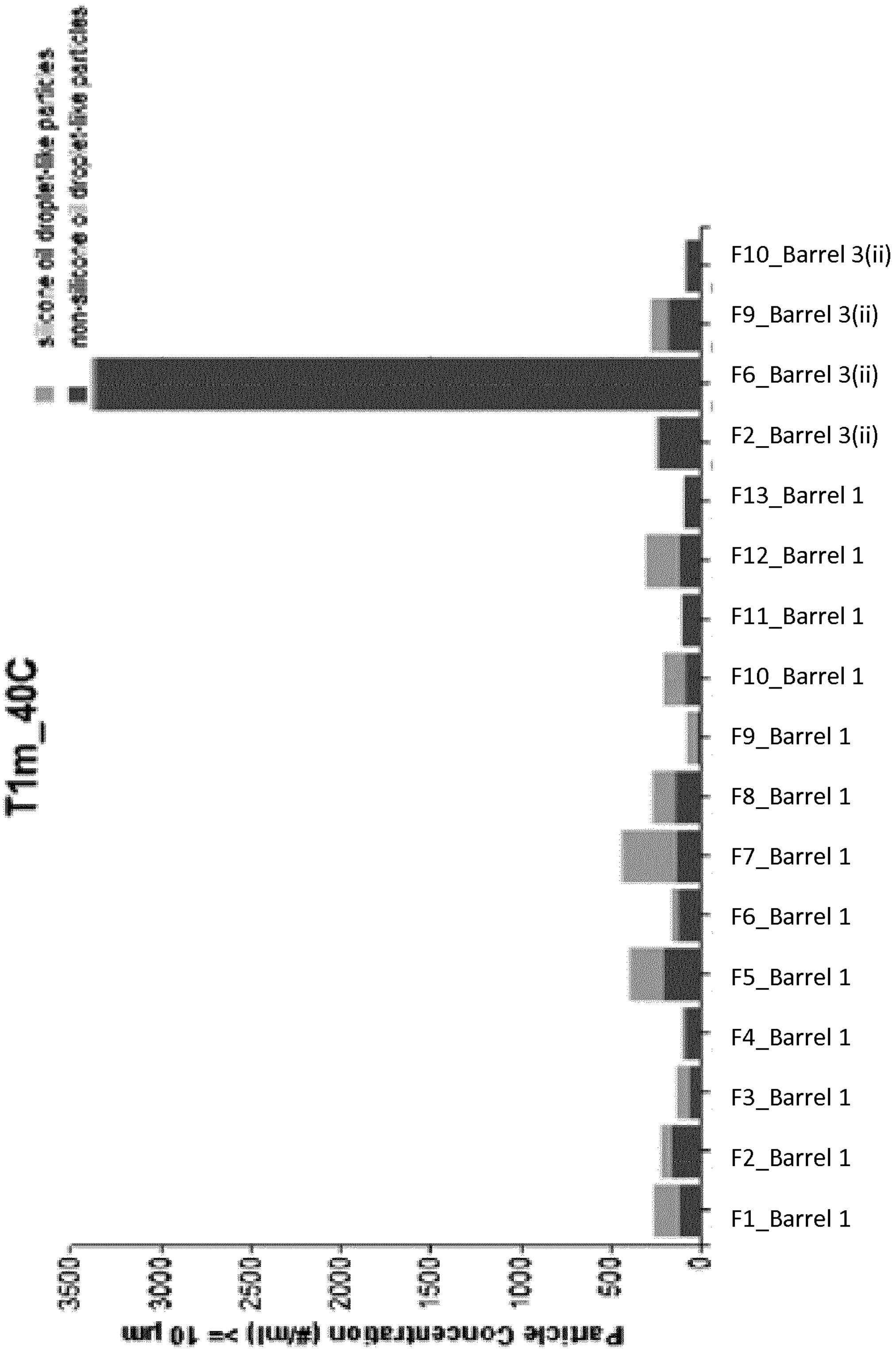

FIG. 52A

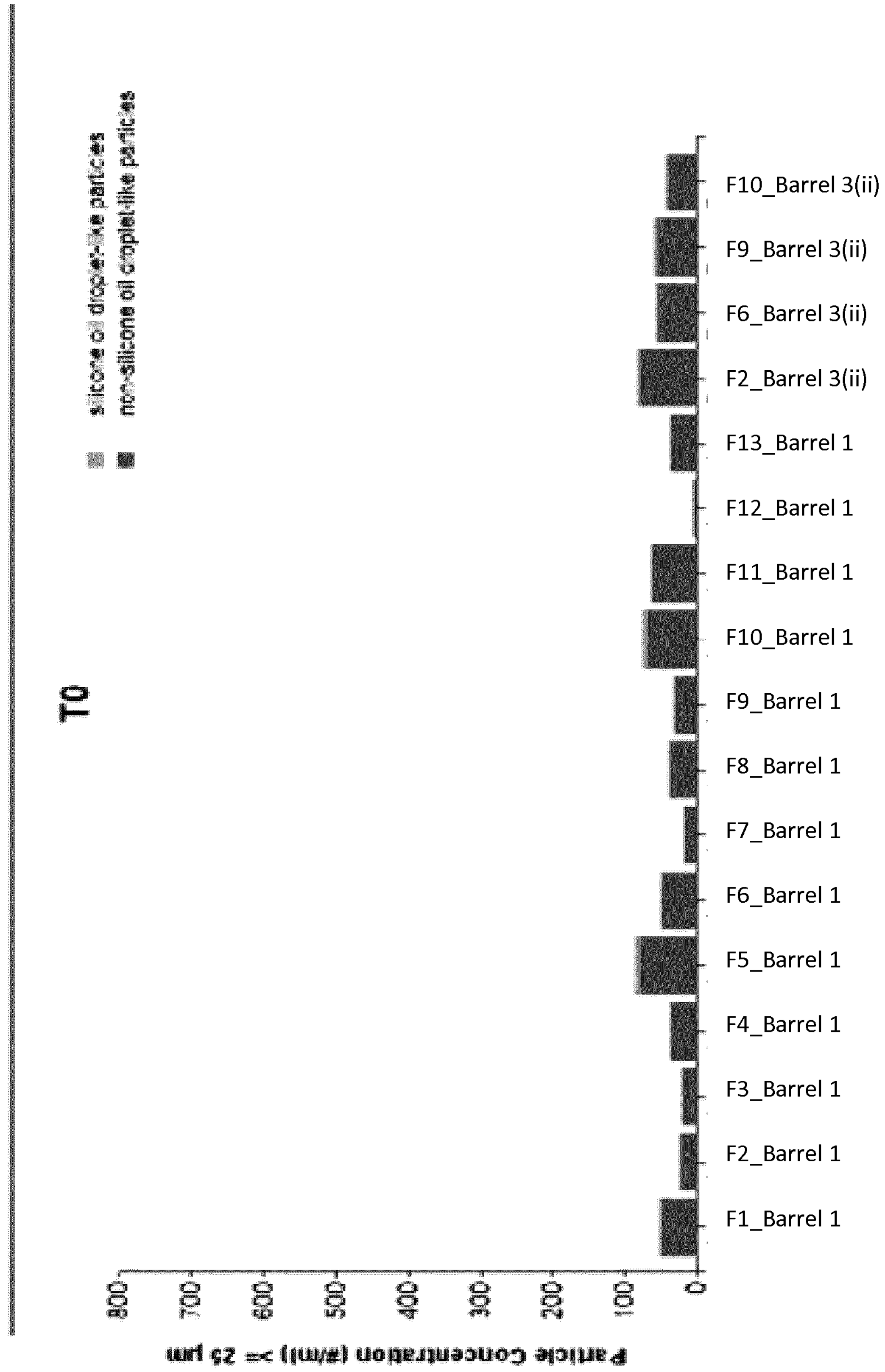
T0
silicone oil droplet-like particles
non-silicone oil droplet-like particles
Particle Concentration (#/ml) >= 25 µm
800
700
600
500
400
300
200
100
0
F1_Barrel 1
F2_Barrel 1
F3_Barrel 1
F4_Barrel 1
F5_Barrel 1
F6_Barrel 1
F7_Barrel 1
F8_Barrel 1
F9_Barrel 1
F10_Barrel 1
F11_Barrel 1
F12_Barrel 1
F13_Barrel 1
F2_Barrel 3(ii)
F6_Barrel 3(ii)
F9_Barrel 3(ii)
F10_Barrel 3(ii)

FIG. 52B

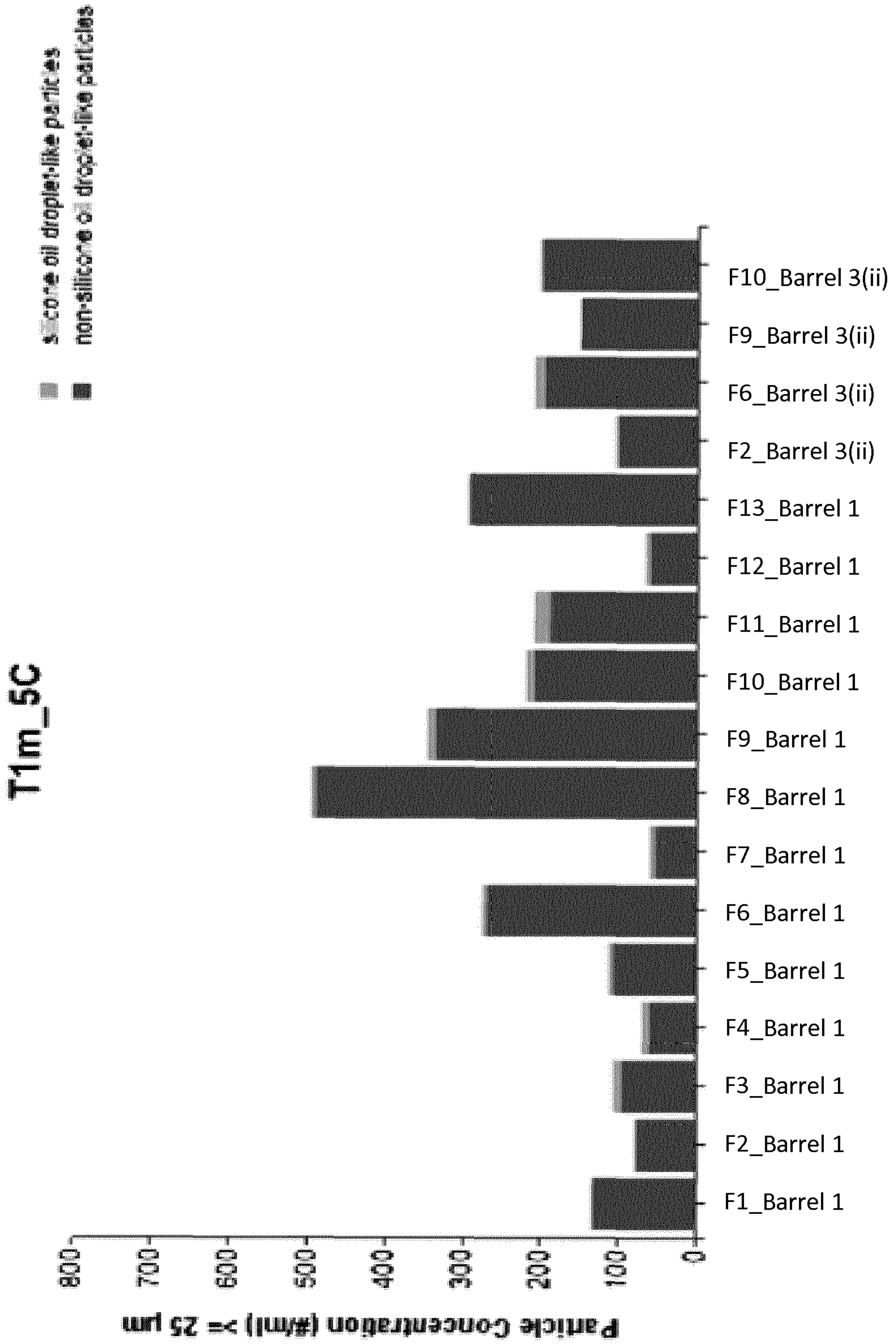
T1m_5C
silicone oil droplet-like particles
non-silicone oil droplet-like particles
Particle Concentration (#/ml) >= 25 µm
0
100
200
300
400
500
600
700
800
F1_Barrel 1
F2_Barrel 1
F3_Barrel 1
F4_Barrel 1
F5_Barrel 1
F6_Barrel 1
F7_Barrel 1
F8_Barrel 1
F9_Barrel 1
F10_Barrel 1
F11_Barrel 1
F12_Barrel 1
F13_Barrel 1
F2_Barrel 3(ii)
F6_Barrel 3(ii)
F9_Barrel 3(ii)
F10_Barrel 3(ii)

FIG. 52C

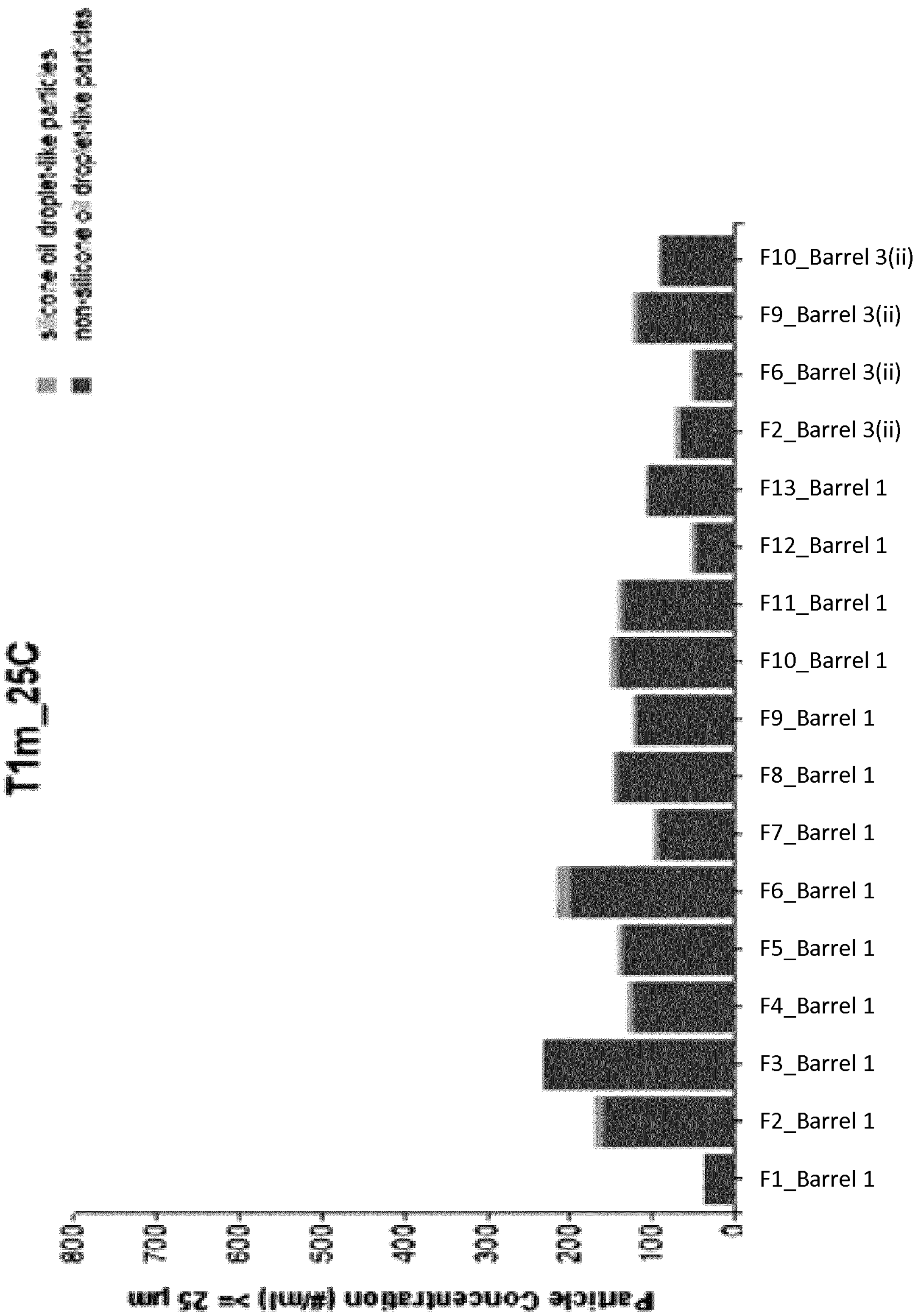
T1m_25C
silicone oil droplet-like particles
non-silicone oil droplet-like particles
Particle Concentration (#/ml) >= 25 µm
800
700
600
500
400
300
200
100
0
F1_Barrel 1
F2_Barrel 1
F3_Barrel 1
F4_Barrel 1
F5_Barrel 1
F6_Barrel 1
F7_Barrel 1
F8_Barrel 1
F9_Barrel 1
F10_Barrel 1
F11_Barrel 1
F12_Barrel 1
F13_Barrel 1
F2_Barrel 3(ii)
F6_Barrel 3(ii)
F9_Barrel 3(ii)
F10_Barrel 3(ii)

FIG. 52D

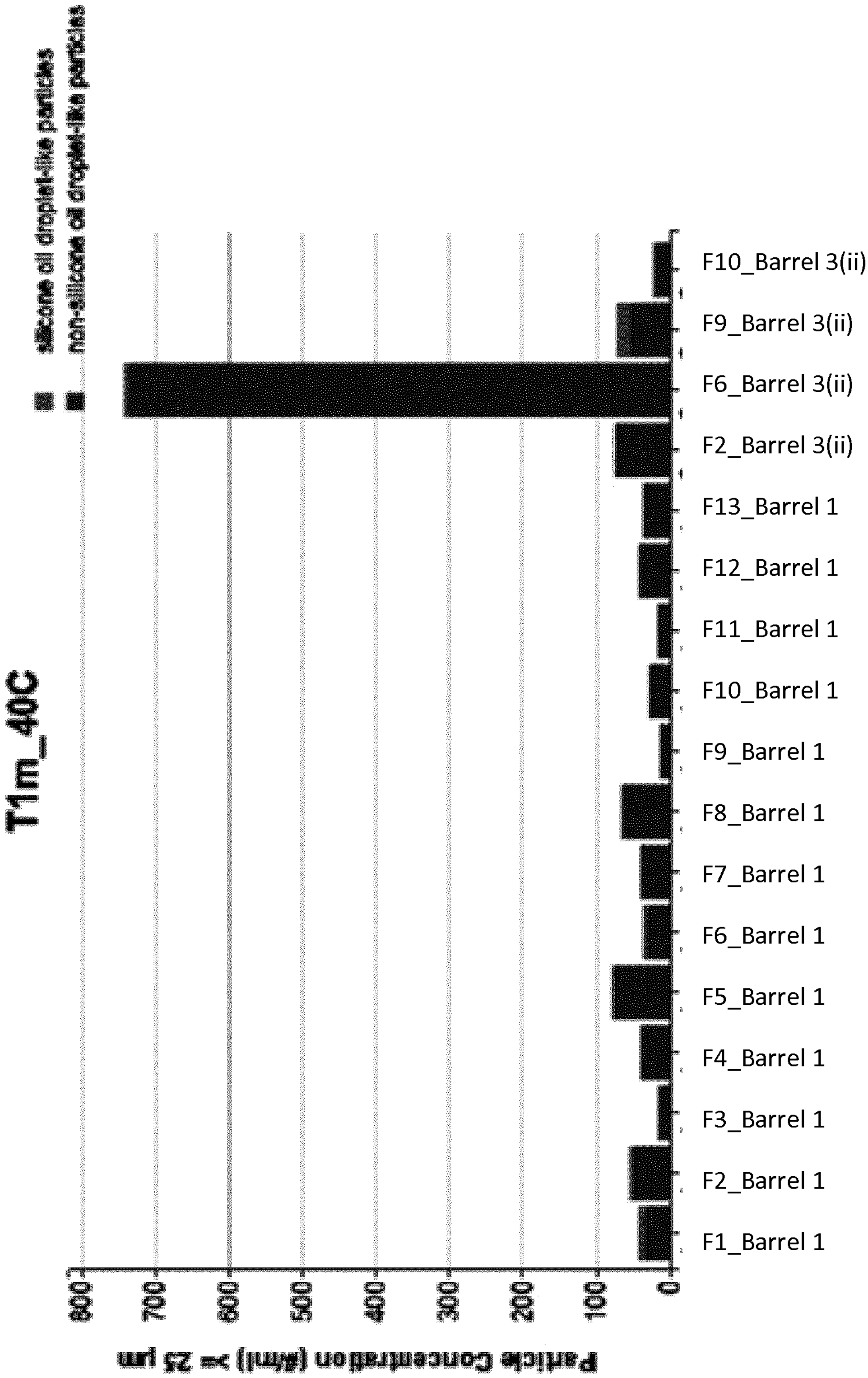
T1m_40C
silicone oil droplet-like particles
non-silicone oil droplet-like particles
Particle Concentration (#/ml) >= 25 µm
800
700
600
500
400
300
200
100
0
F1_Barrel 1
F2_Barrel 1
F3_Barrel 1
F4_Barrel 1
F5_Barrel 1
F6_Barrel 1
F7_Barrel 1
F8_Barrel 1
F9_Barrel 1
F10_Barrel 1
F11_Barrel 1
F12_Barrel 1
F13_Barrel 1
F2_Barrel 3(ii)
F6_Barrel 3(ii)
F9_Barrel 3(ii)
F10_Barrel 3(ii)

FIG. 53A

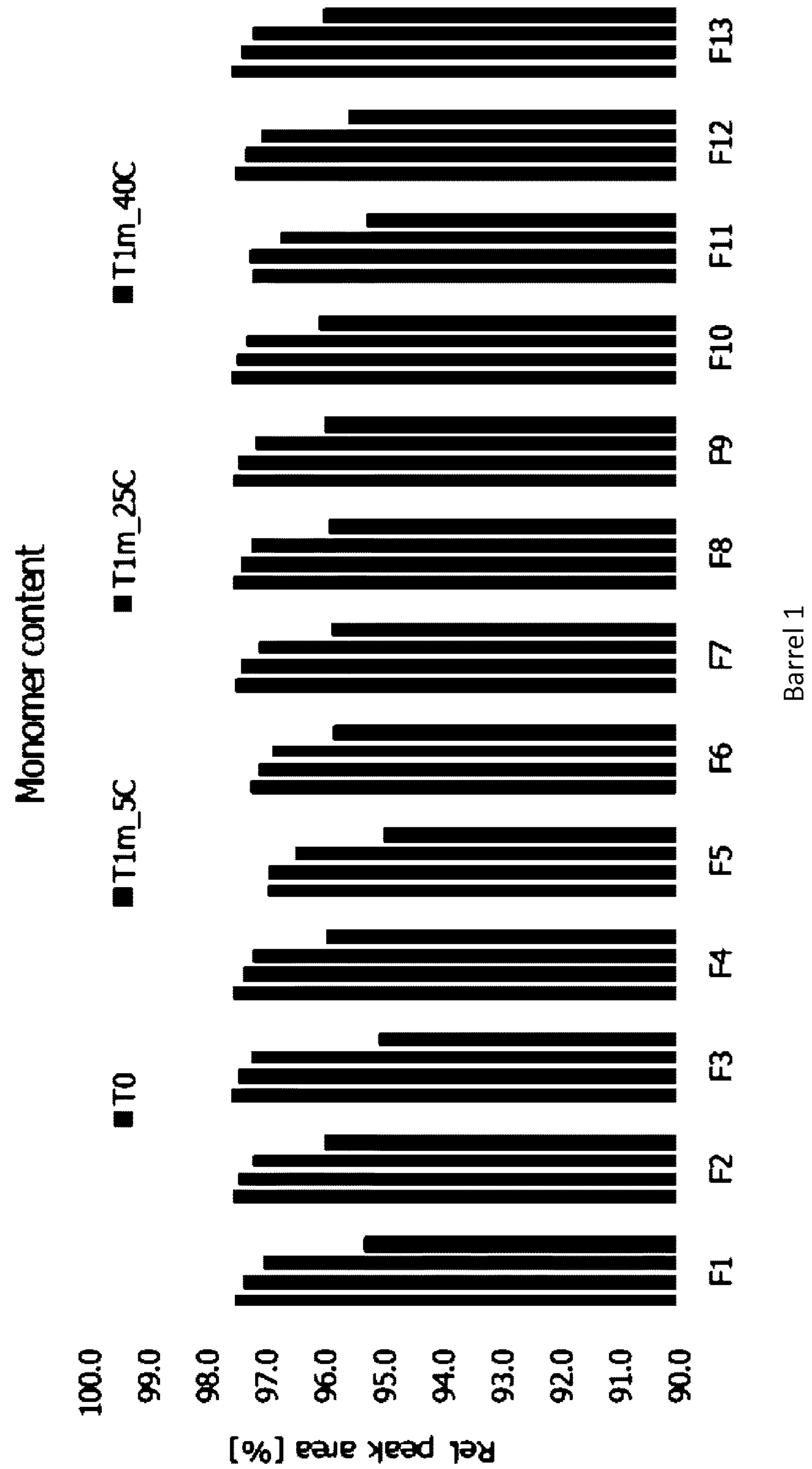
Monomer content
T0
T1m_5C
T1m_25C
T1m_40C
Rel peak area [%]
100.0
99.0
98.0
97.0
96.0
95.0
94.0
93.0
92.0
91.0
90.0
F1
F2
F3
F4
F5
F6
F7
F8
F9
F10
F11
F12
F13
Barrel 1

FIG. 53B

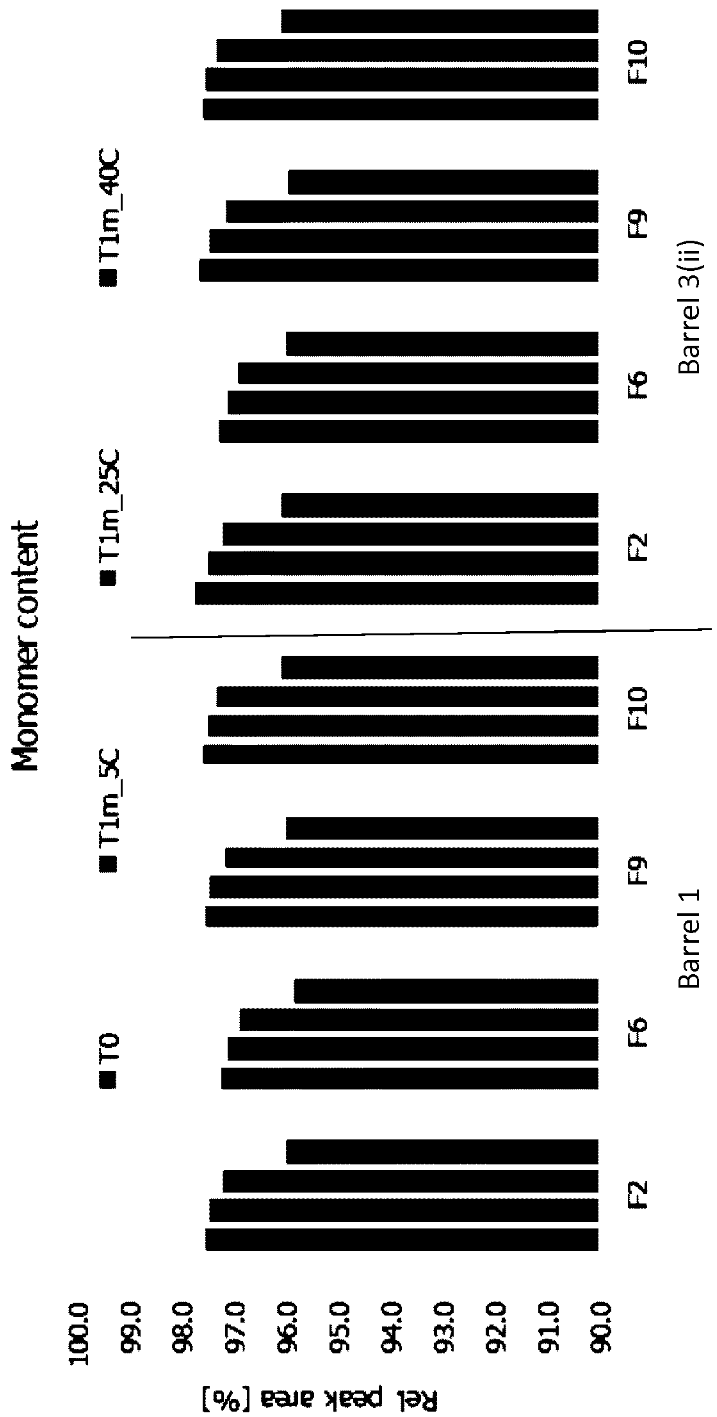
Monomer content
T0
T1m_5C
T1m_25C
T1m_40C
Rel peak area [%]
100.0
99.0
98.0
97.0
96.0
95.0
94.0
93.0
92.0
91.0
90.0
F2
F6
F9
F10
Barrel 1
F2
F6
F9
F10
Barrel 3(ii)

FIG. 54A

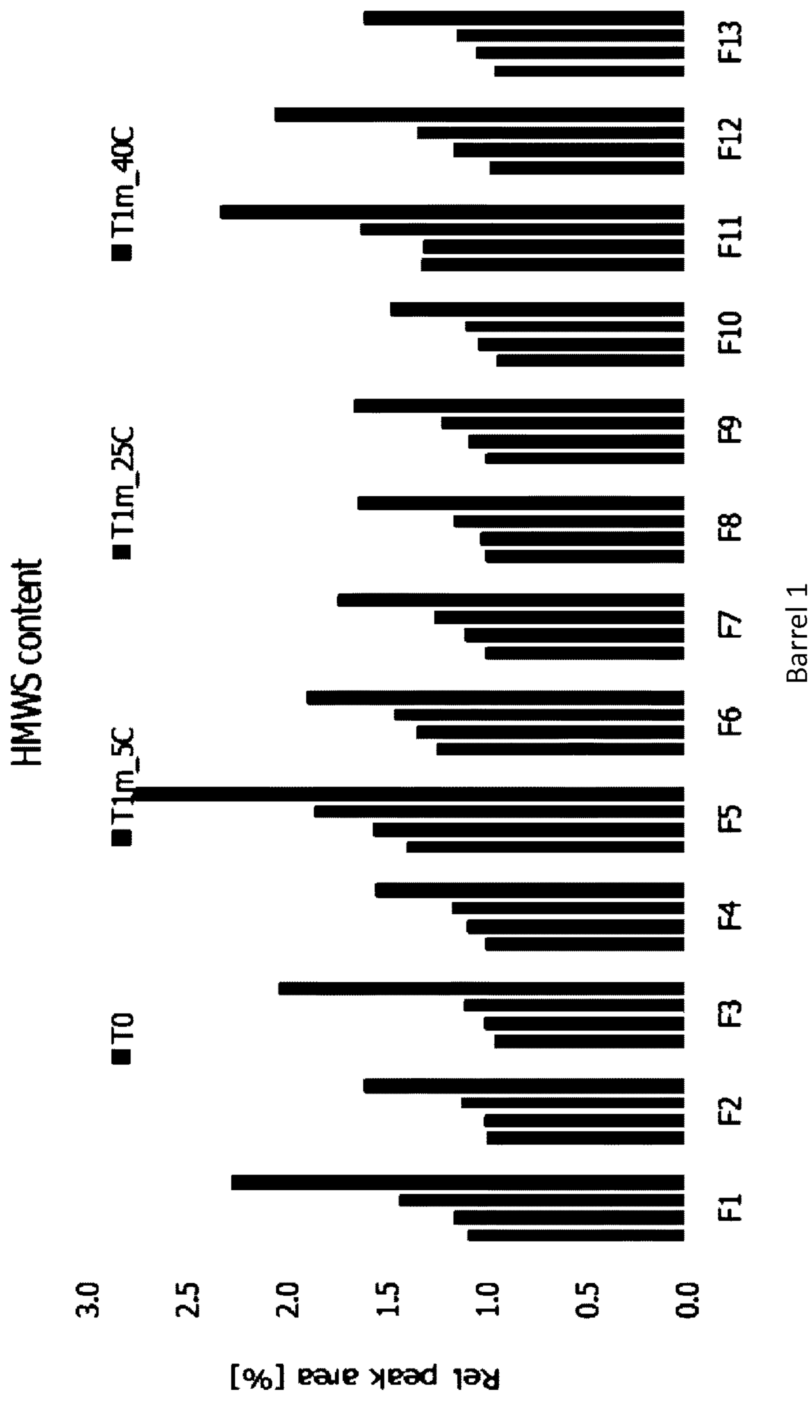
HMWS content
T0
T1m_5C
T1m_25C
T1m_40C
Rel peak area [%]
3.0
2.5
2.0
1.5
1.0
0.5
0.0
F1
F2
F3
F4
F5
F6
F7
F8
F9
F10
F11
F12
F13
Barrel 1

FIG. 54B

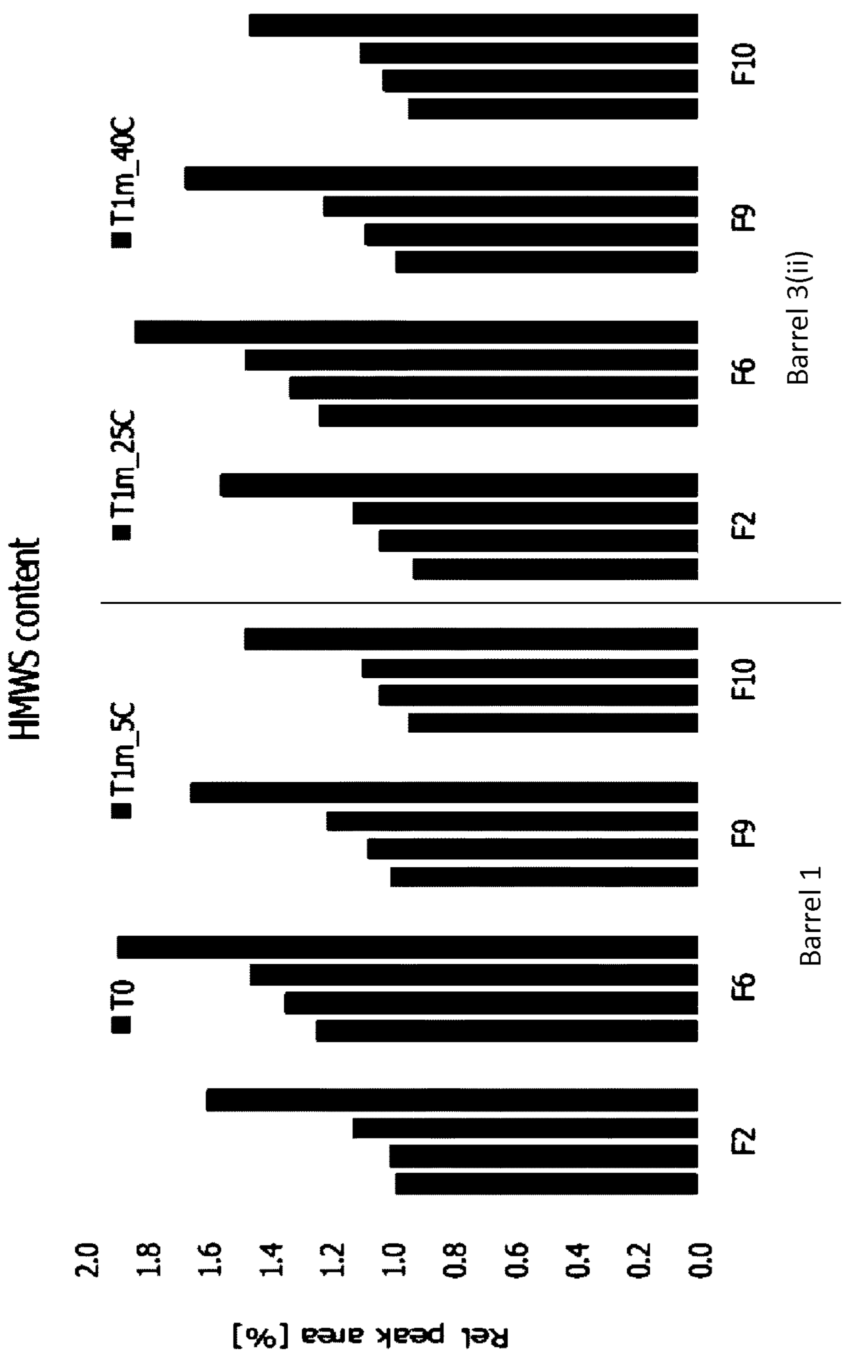
HMWS content
T0
T1m_5C
T1m_25C
T1m_40C
Rel peak area [%]
2.0
1.8
1.6
1.4
1.2
1.0
0.8
0.6
0.4
0.2
0.0
F2
F6
F9
F10
F2
F6
F9
F10
Barrel 1
Barrel 3(ii)

FIG. 55A

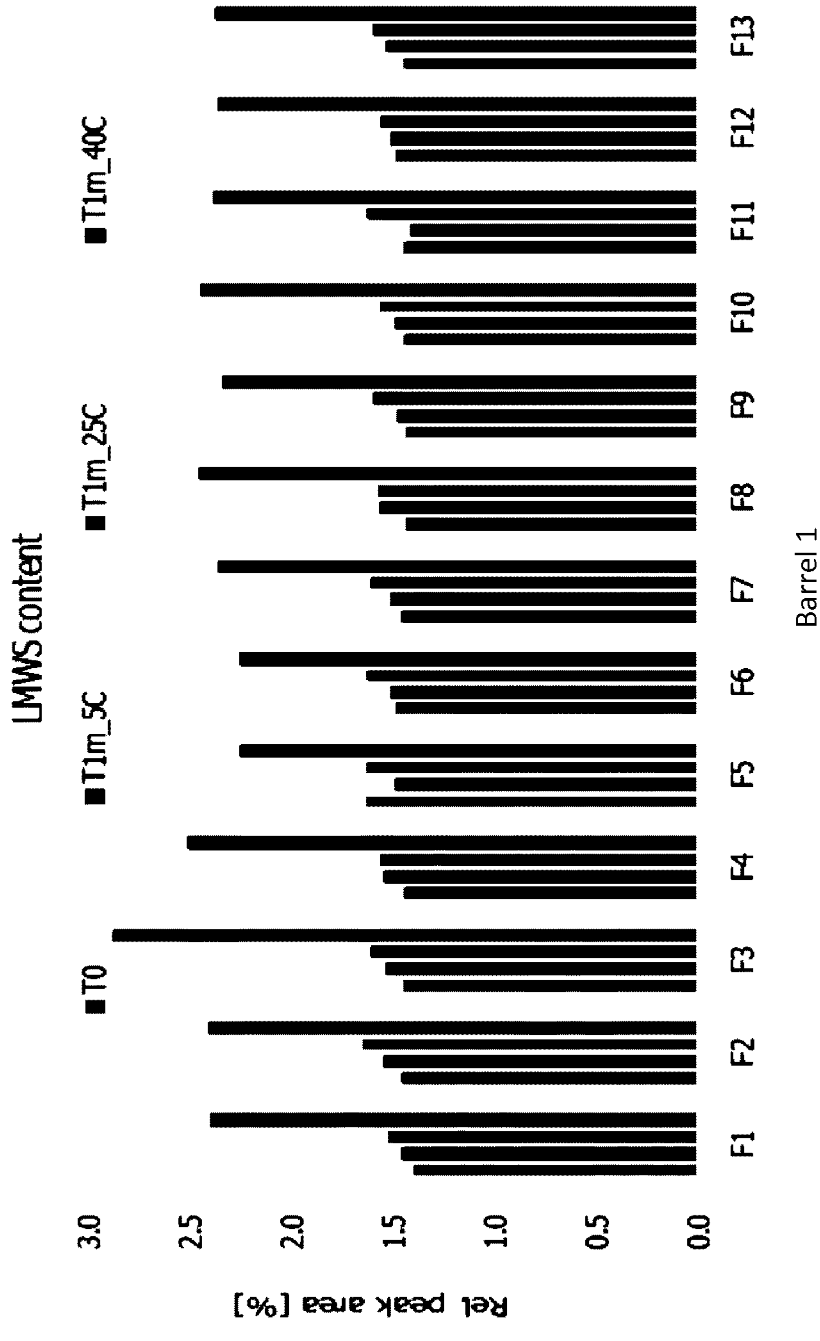
LMWS content
T0
T1m_5C
T1m_25C
T1m_40C
Rel peak area [%]
3.0
2.5
2.0
1.5
1.0
0.5
0.0
F1
F2
F3
F4
F5
F6
F7
F8
F9
F10
F11
F12
F13
Barrel 1

FIG. 55B

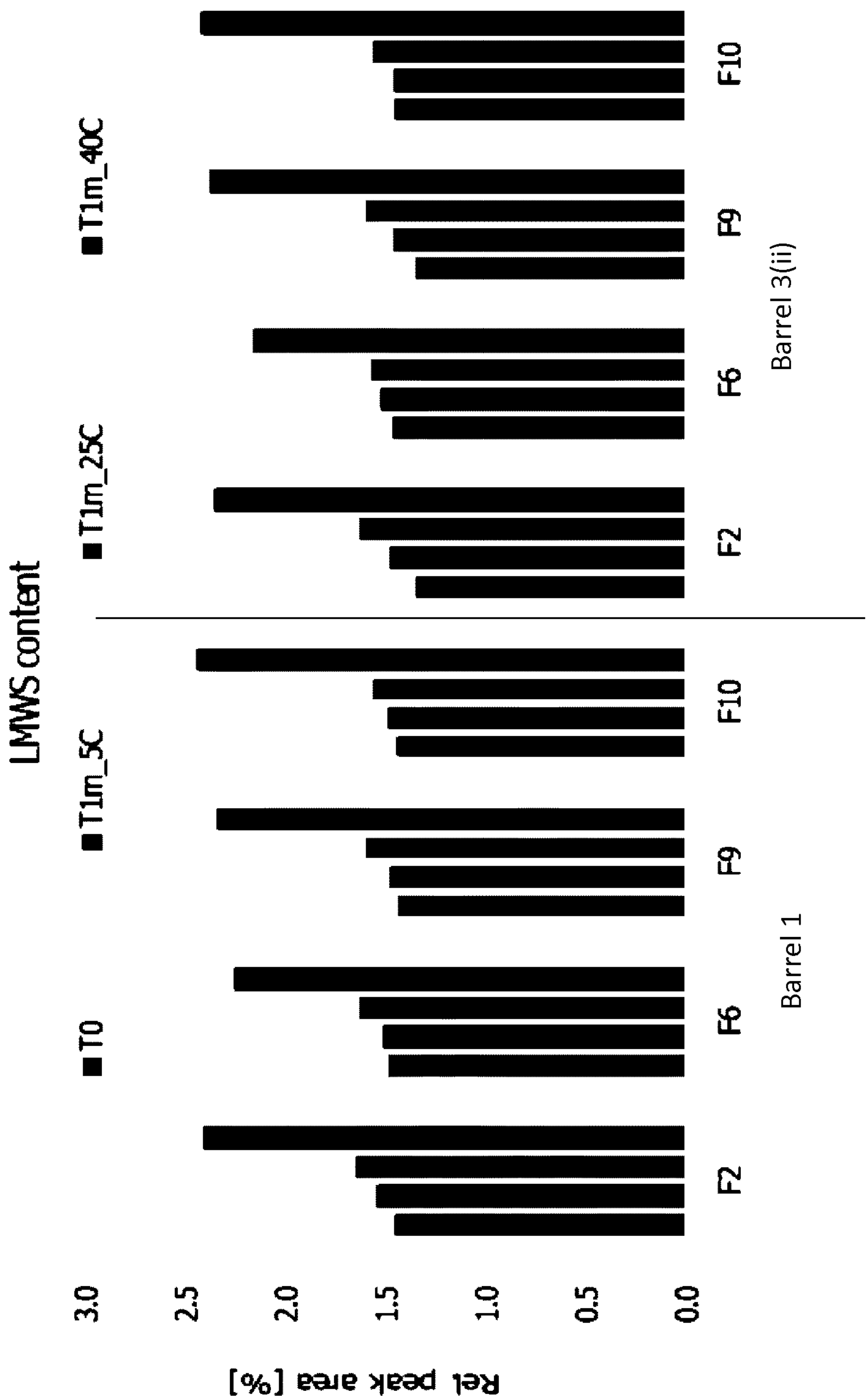
LMWS content
T0
T1m_5C
T1m_25C
T1m_40C
Rel peak area [%]
0.0
0.5
1.0
1.5
2.0
2.5
3.0
F2
F6
F9
F10
F2
F6
F9
F10
Barrel 1
Barrel 3(ii)

## FIG. 56A

Purity: non-reducing conditions

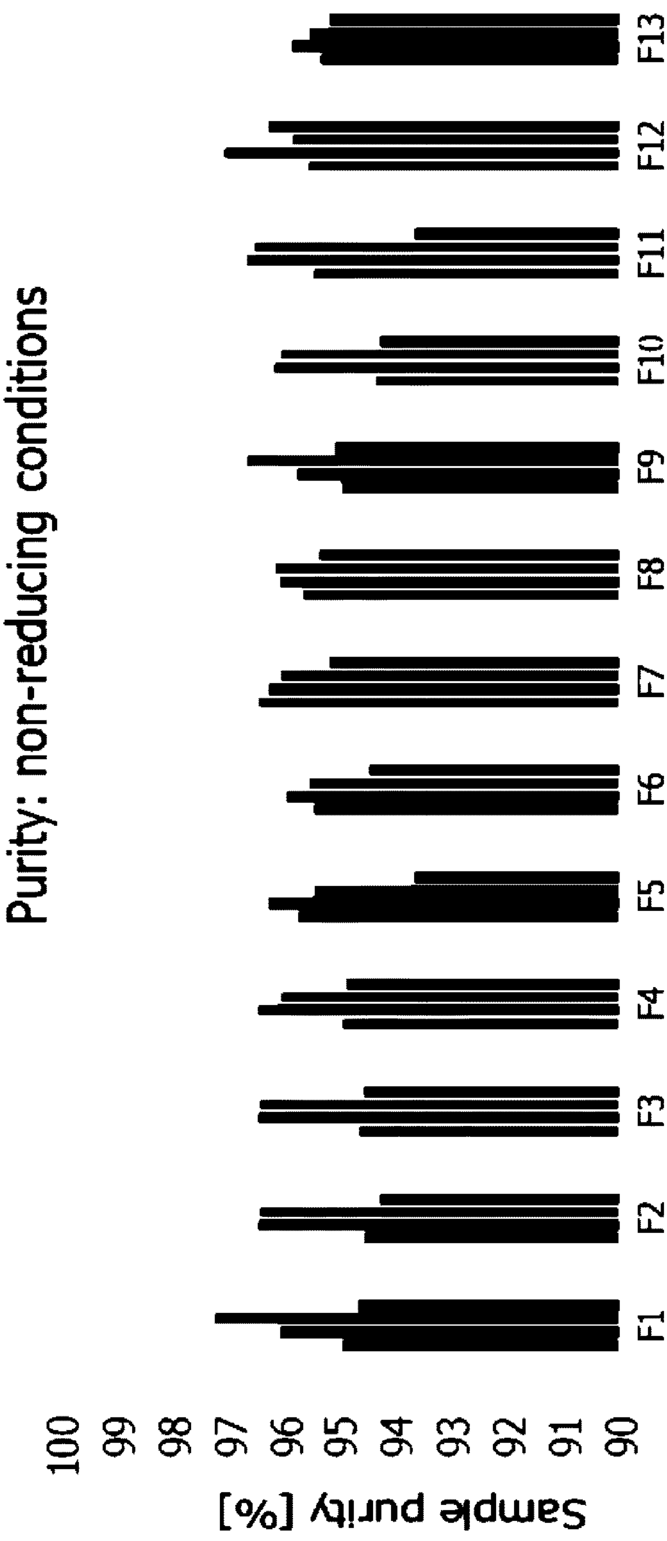


| | F1 | F2 | F3 | F4 | F5 | F6 | F7 | F8 | F9 | F10 | F11 | F12 | F13 |
|---|---|---|---|---|---|---|---|---|---|---|---|---|---|
| ■ T0 | 95 | 94.6 | 94.7 | 95 | 95.8 | 95.5 | 96.5 | 95.7 | 95 | 94.4 | 95.5 | 95.6 | 95.4 |
| ■ 5°C | 96.1 | 96.5 | 96.5 | 96.5 | 96.3 | 96 | 96.3 | 96.1 | 95.8 | 96.2 | 96.7 | 97.1 | 95.9 |
| ■ 25°C | 97.3 | 96.5 | 96.5 | 96.1 | 95.5 | 95.6 | 96.1 | 96.2 | 96.7 | 96.1 | 96.6 | 95.9 | 95.6 |
| ■ 40°C | 94.7 | 94.3 | 94.6 | 94.9 | 93.7 | 94.5 | 95.2 | 95.4 | 95.1 | 94.3 | 93.7 | 96.3 | 95.2 |

FIG. 56B

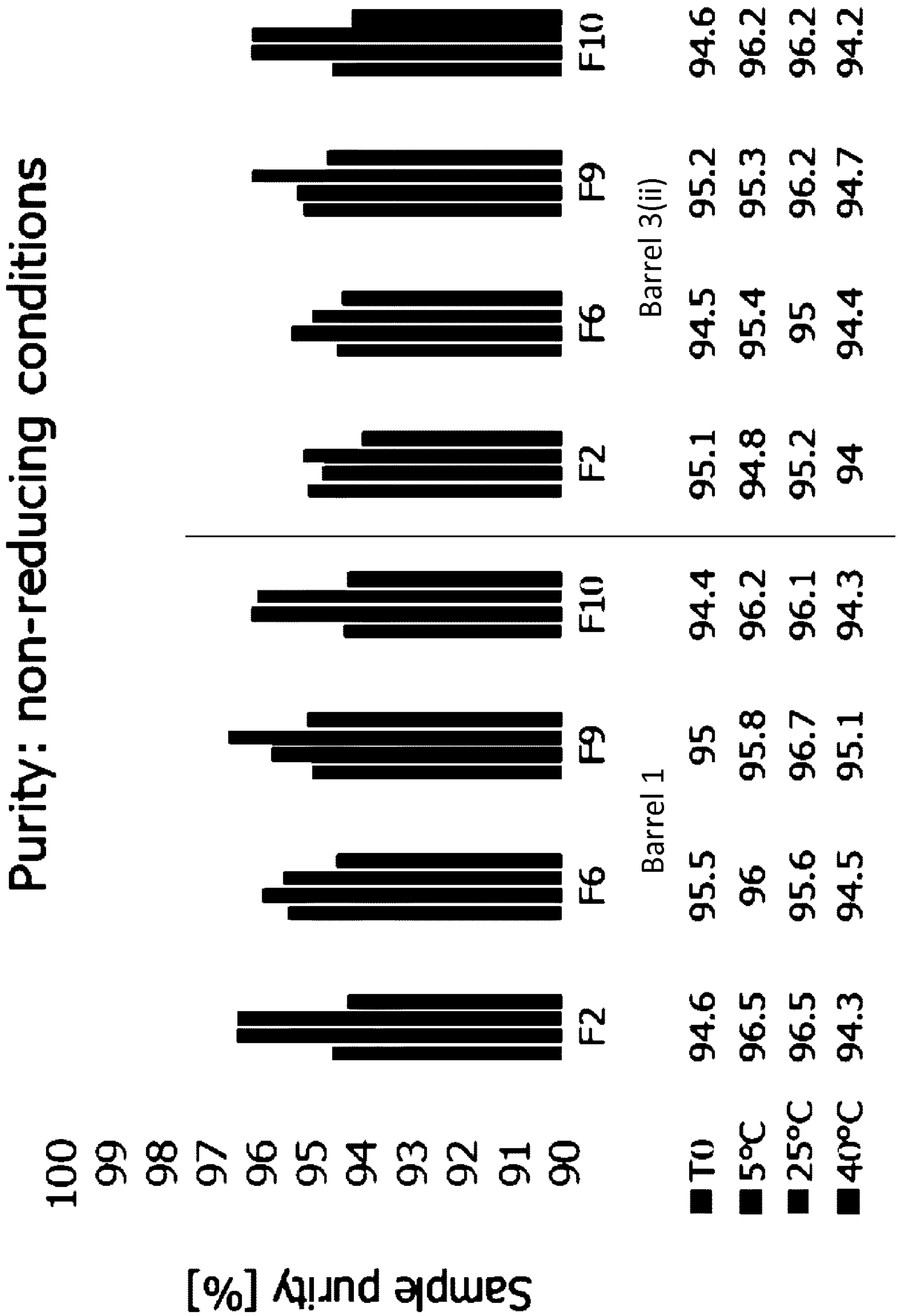
Purity: non-reducing conditions
Sample purity [%]
100
99
98
97
96
95
94
93
92
91
90
Barrel 1
Barrel 3(ii)
F2 F6 F9 F10 F2 F6 F9 F10
■T0 94.6 95.5 95 94.4 95.1 94.5 95.2 94.6
■5°C 96.5 96 95.8 96.2 94.8 95.4 95.3 96.2
■25°C 96.5 95.6 96.7 96.1 95.2 95 96.2 96.2
■40°C 94.3 94.5 95.1 94.3 94 94.4 94.7 94.2

FIG. 57A

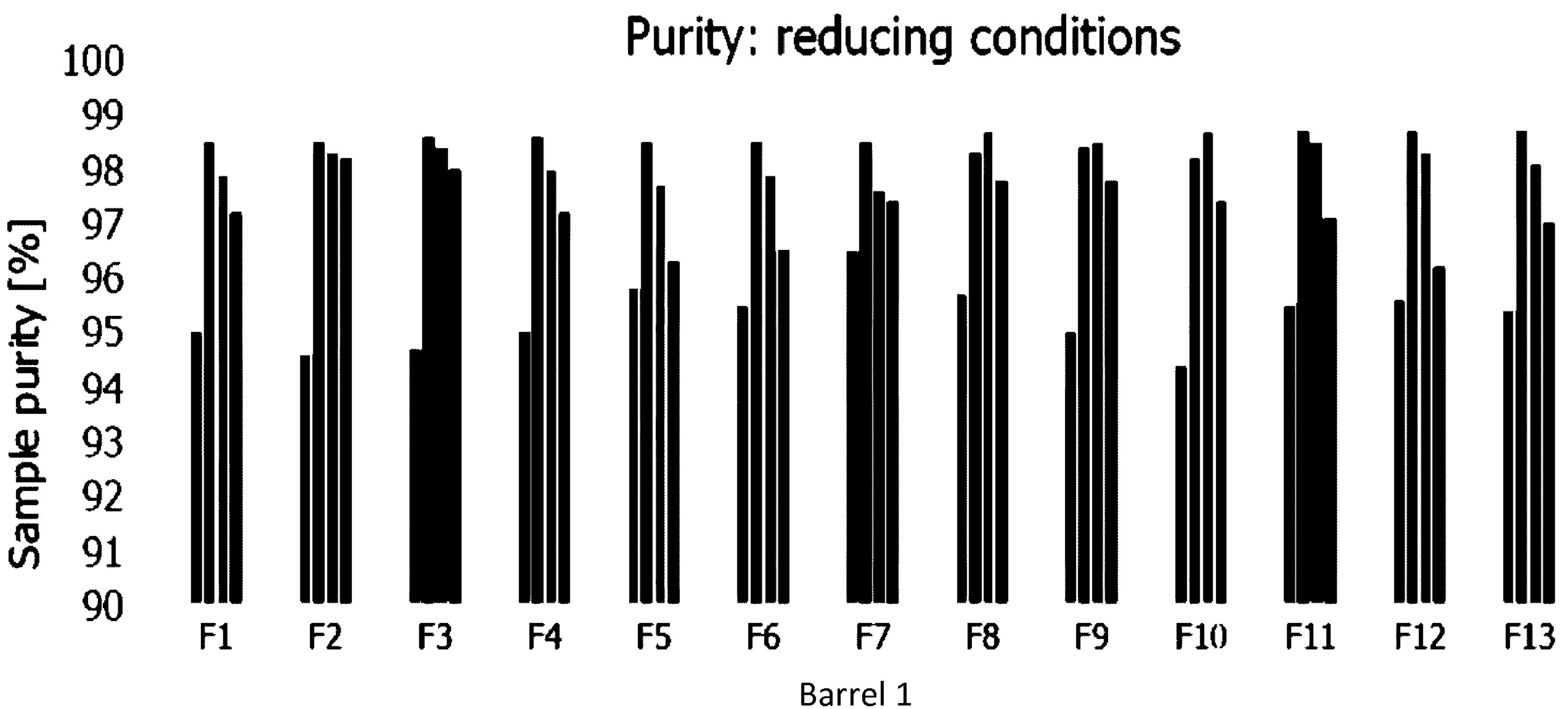


| | F1 | F2 | F3 | F4 | F5 | F6 | F7 | F8 | F9 | F10 | F11 | F12 | F13 |
|---|---|---|---|---|---|---|---|---|---|---|---|---|---|
| ■ T0 | 95 | 94.6 | 94.7 | 95 | 95.8 | 95.5 | 96.5 | 95.7 | 95 | 94.4 | 95.5 | 95.6 | 95.4 |
| ■ 5°C | 98.5 | 98.5 | 98.6 | 98.6 | 98.5 | 98.5 | 98.5 | 98.3 | 98.4 | 98.2 | 98.7 | 98.7 | 98.7 |
| ■ 25°C | 97.9 | 98.3 | 98.4 | 98 | 97.7 | 97.9 | 97.6 | 98.7 | 98.5 | 98.7 | 98.5 | 98.3 | 98.1 |
| ■ 40°C | 97.2 | 98.2 | 98 | 97.2 | 96.3 | 96.5 | 97.4 | 97.8 | 97.8 | 97.4 | 97.1 | 96.2 | 97 |

FIG. 57B

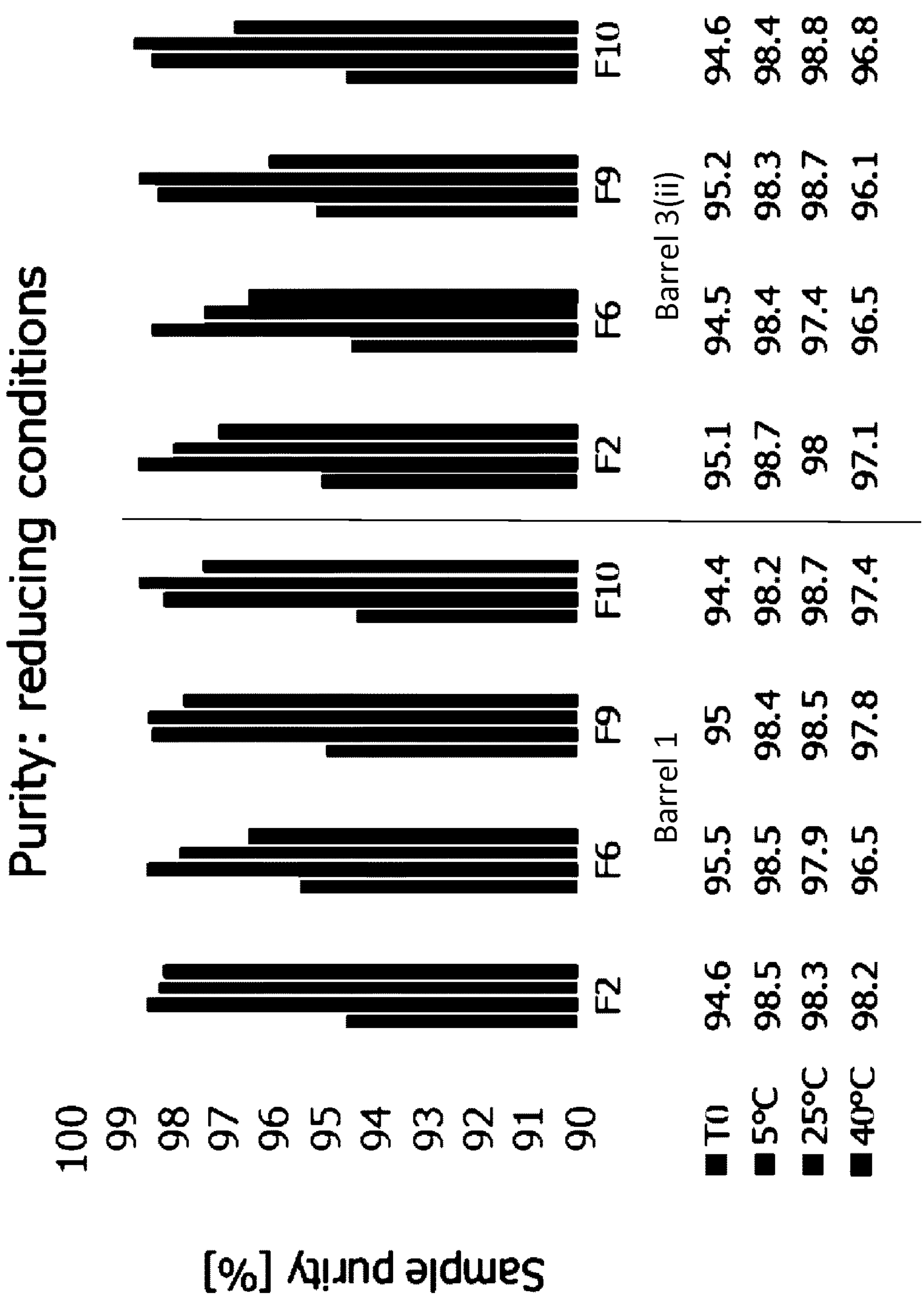
Purity: reducing conditions
Sample purity [%]
100
99
98
97
96
95
94
93
92
91
90
F2
F6
F9
F10
F2
F6
F9
F10
Barrel 1
Barrel 3(ii)
T0 94.6 95.5 95 94.4 95.1 94.5 95.2 94.6
5°C 98.5 98.5 98.4 98.2 98.7 98.4 98.3 98.4
25°C 98.3 97.9 98.5 98.7 98 97.4 98.7 98.8
40°C 98.2 96.5 97.8 97.4 97.1 96.5 96.1 96.8

FIG. 58A

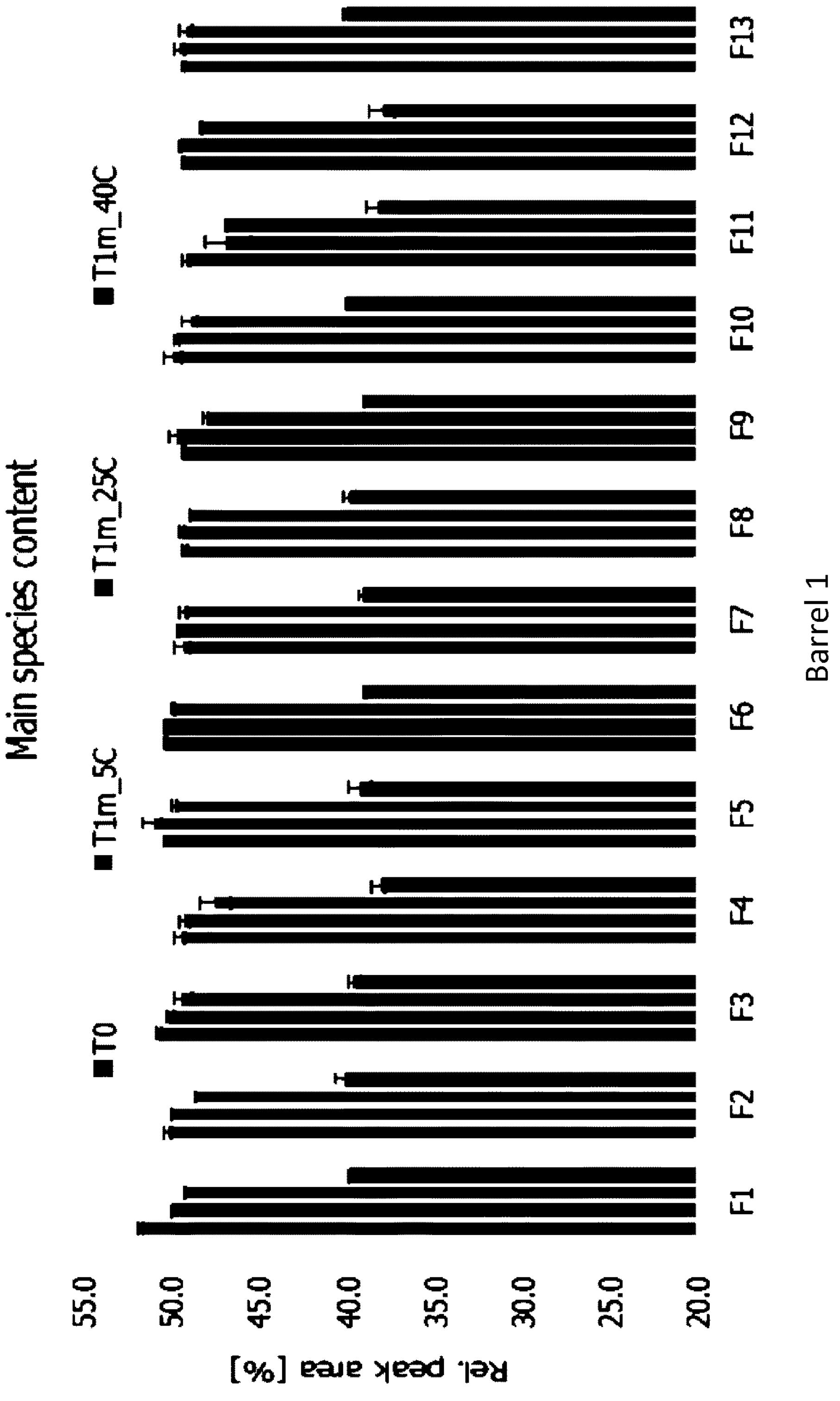
Main species content
T0
T1m_5C
T1m_25C
T1m_40C
Rel. peak area [%]
55.0
50.0
45.0
40.0
35.0
30.0
25.0
20.0
F1
F2
F3
F4
F5
F6
F7
F8
F9
F10
F11
F12
F13
Barrel 1

FIG. 58B

Main species content

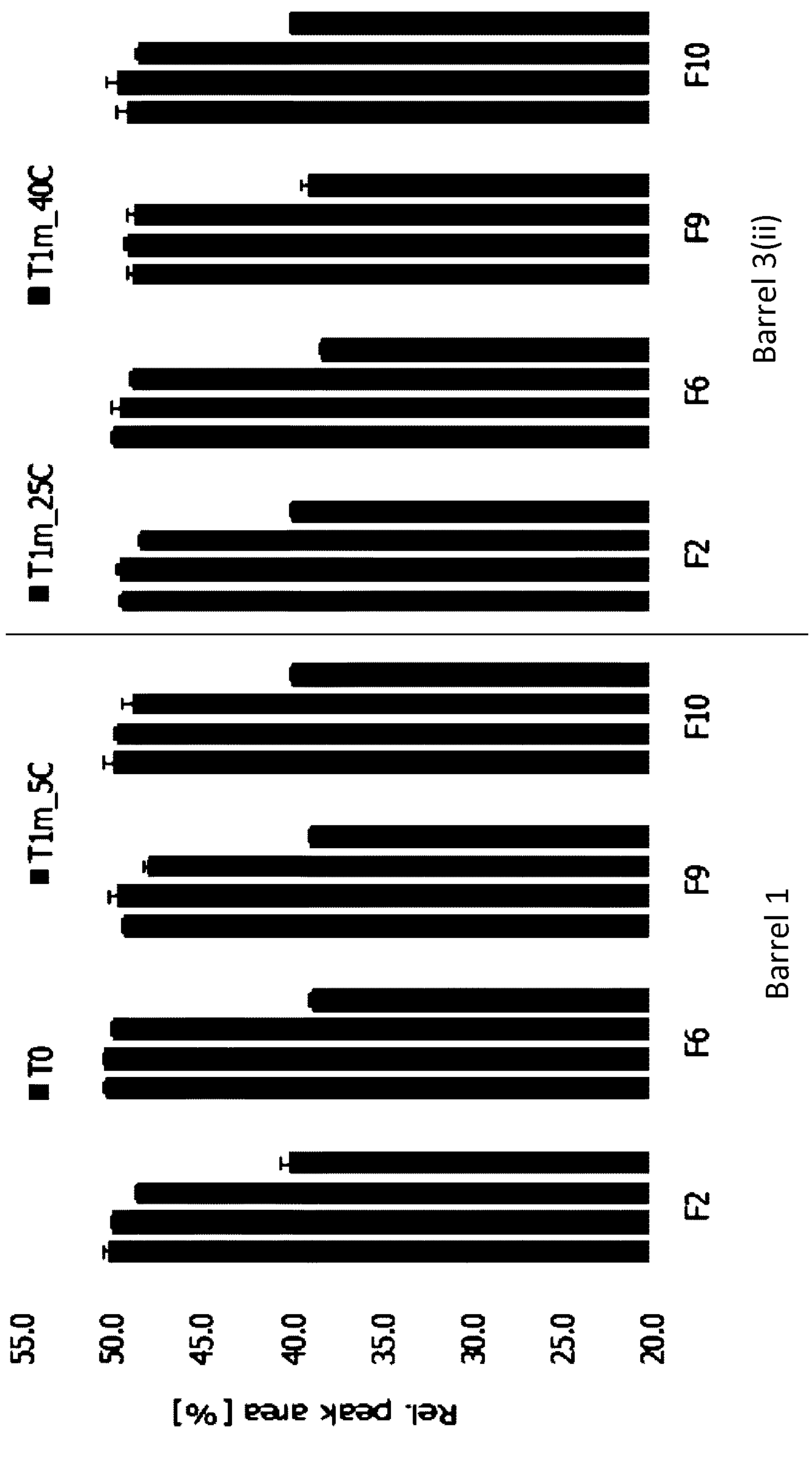

FIG. 59

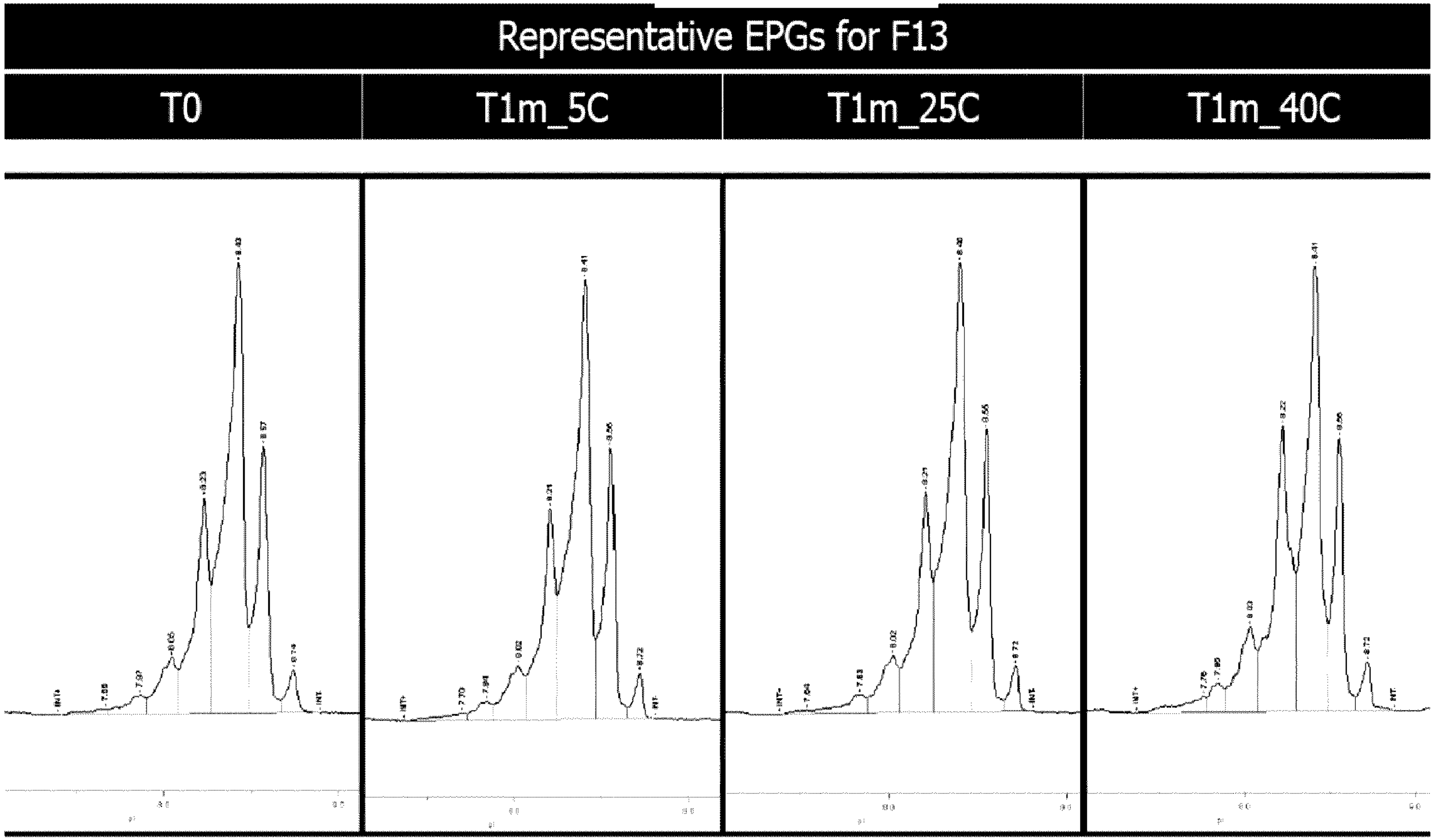

FIG. 60A

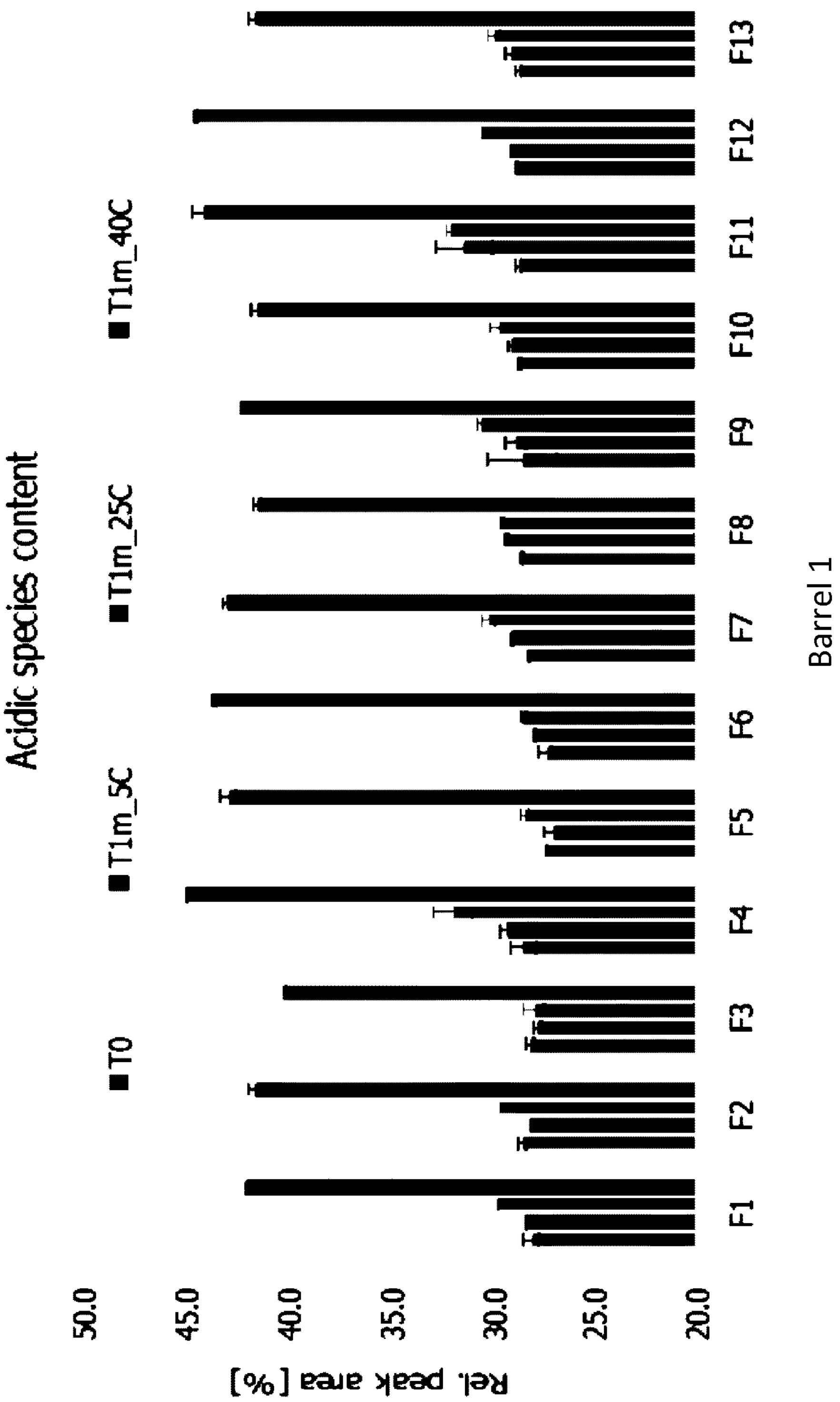
Acidic species content
T0
T1m_5C
T1m_25C
T1m_40C
Rel. peak area [%]
50.0
45.0
40.0
35.0
30.0
25.0
20.0
F1
F2
F3
F4
F5
F6
F7
F8
F9
F10
F11
F12
F13
Barrel 1

FIG. 60B

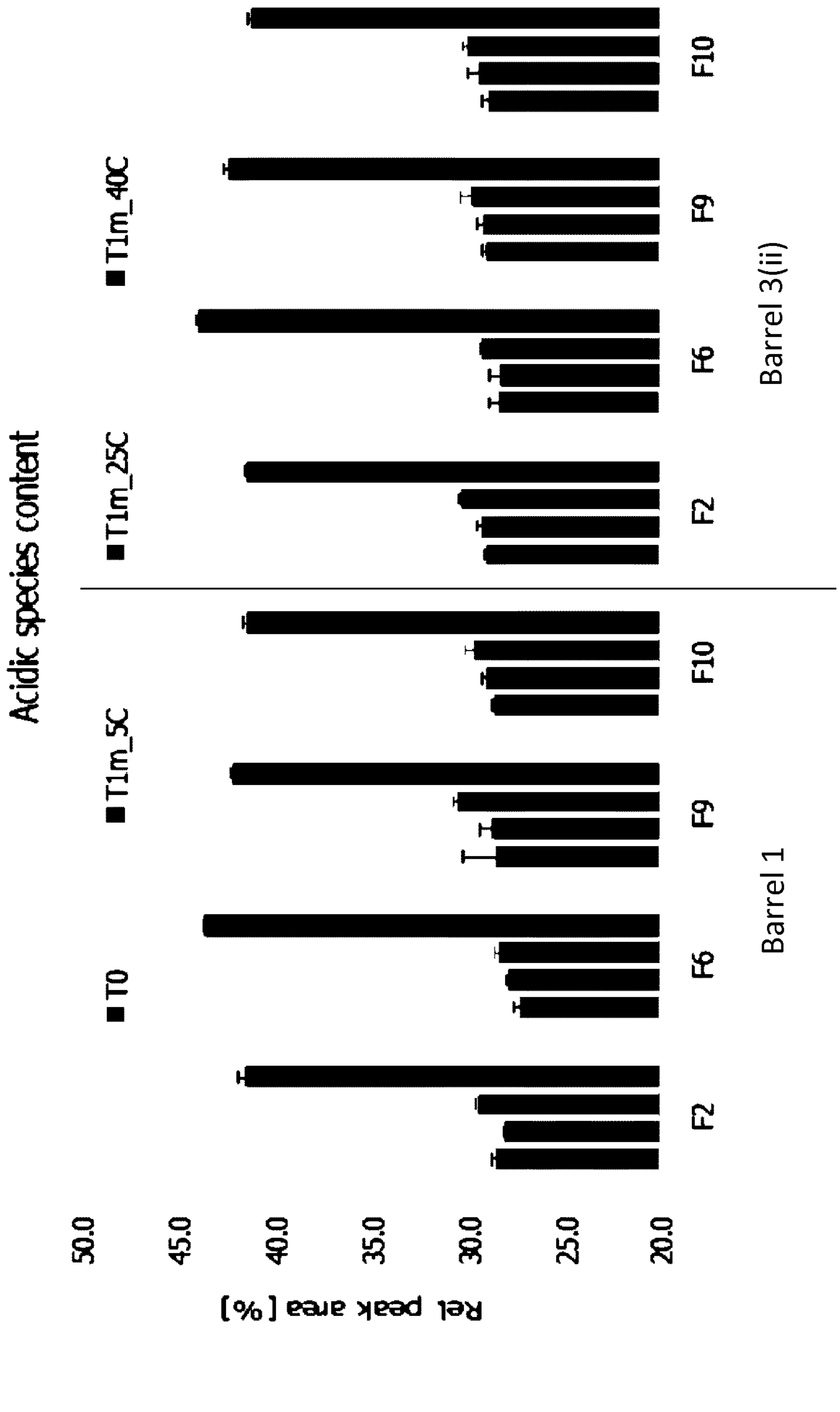
Acidic species content
T0
T1m_5C
T1m_25C
T1m_40C
Rel peak area [%]
50.0
45.0
40.0
35.0
30.0
25.0
20.0
F2
F6
F9
F10
F2
F6
F9
F10
Barrel 1
Barrel 3(ii)

FIG. 61A

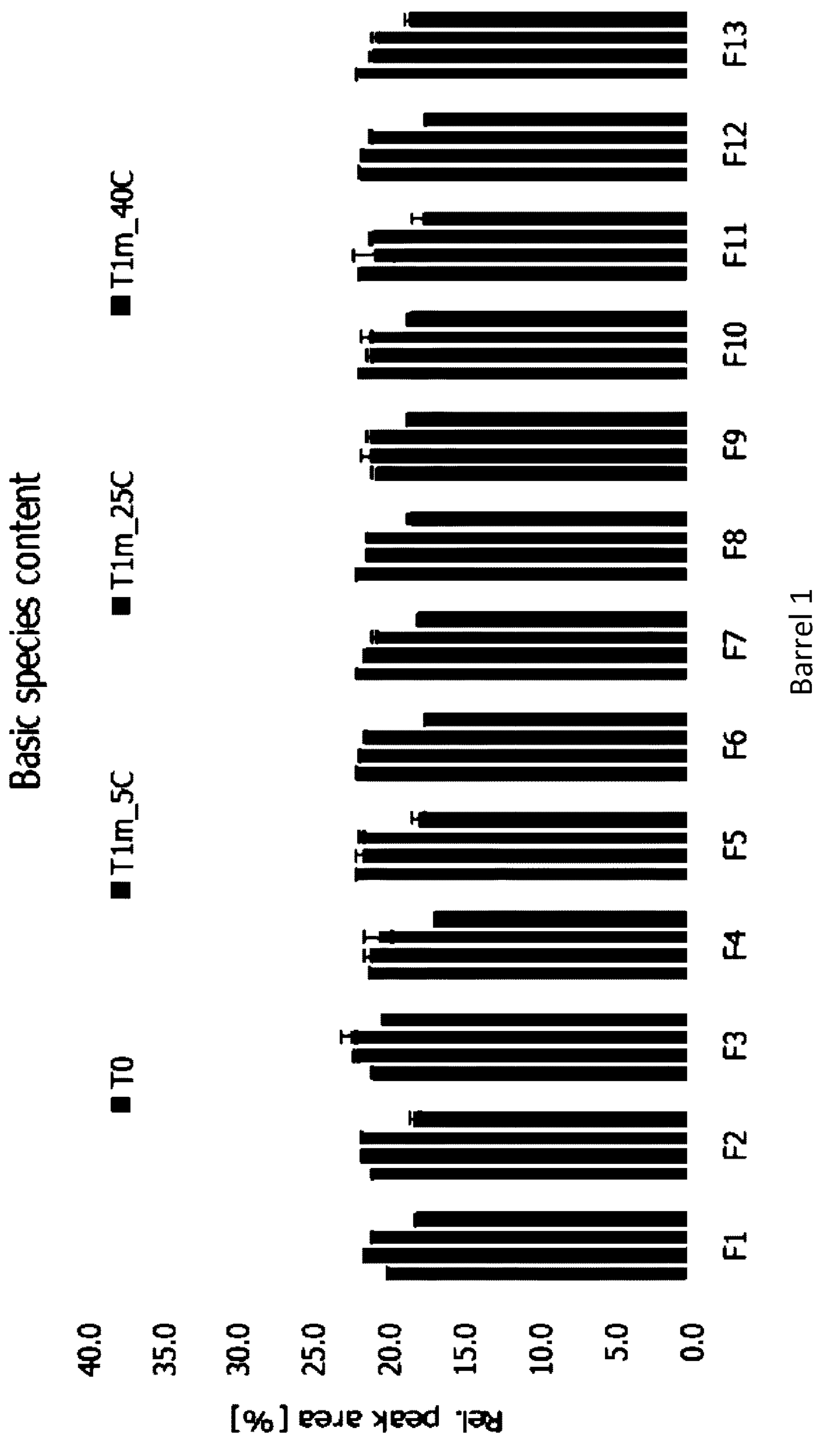
Basic species content
T0
T1m_5C
T1m_25C
T1m_40C
Rel. peak area [%]
40.0
35.0
30.0
25.0
20.0
15.0
10.0
5.0
0.0
F1
F2
F3
F4
F5
F6
F7
F8
F9
F10
F11
F12
F13
Barrel 1

FIG. 61B

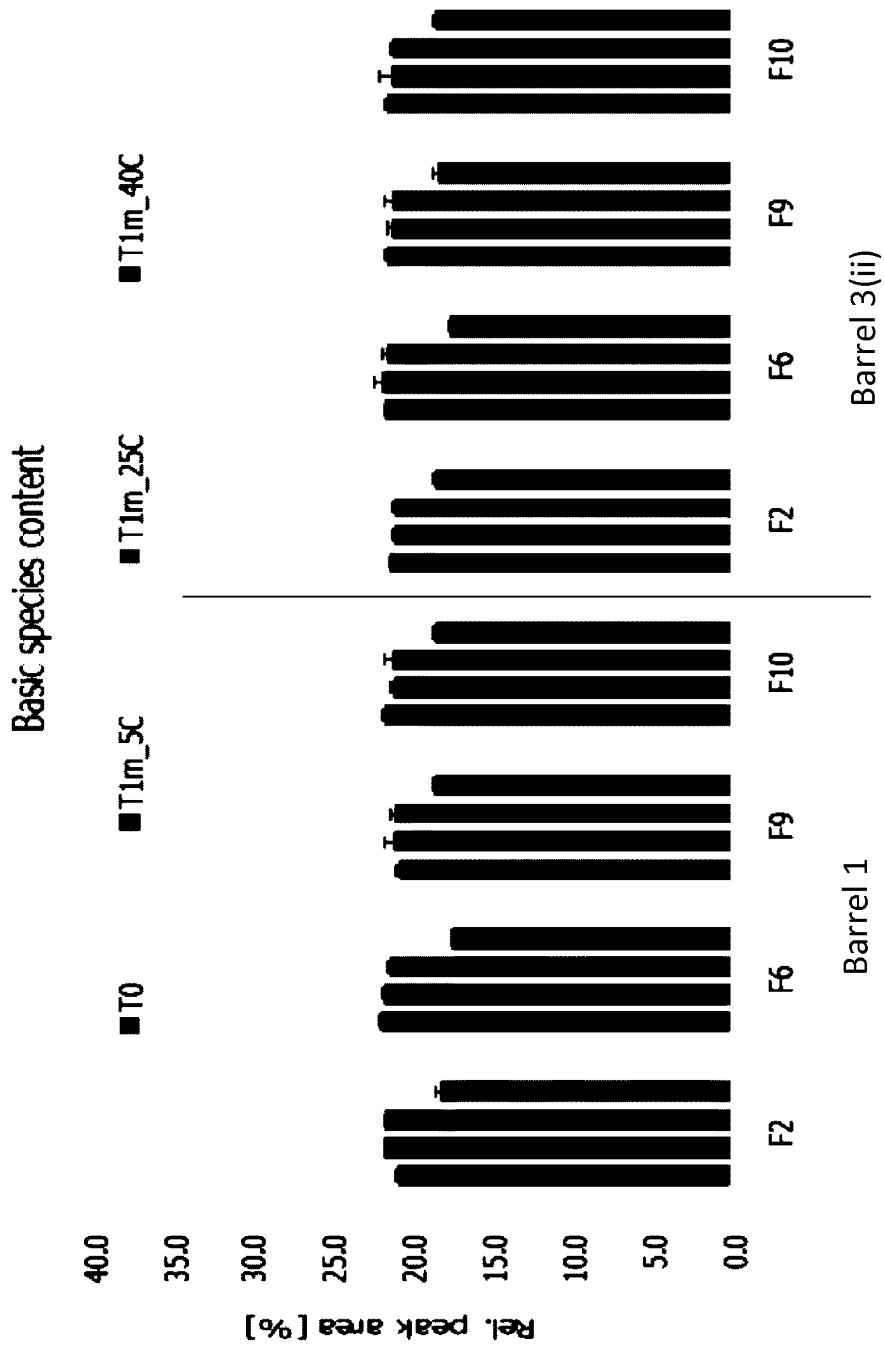
Basic species content
T0
T1m_5C
T1m_25C
T1m_40C
Rel. peak area [%]
40.0
35.0
30.0
25.0
20.0
15.0
10.0
5.0
0.0
F2
F6
F9
F10
F2
F6
F9
F10
Barrel 1
Barrel 3(ii)

FIG. 62A

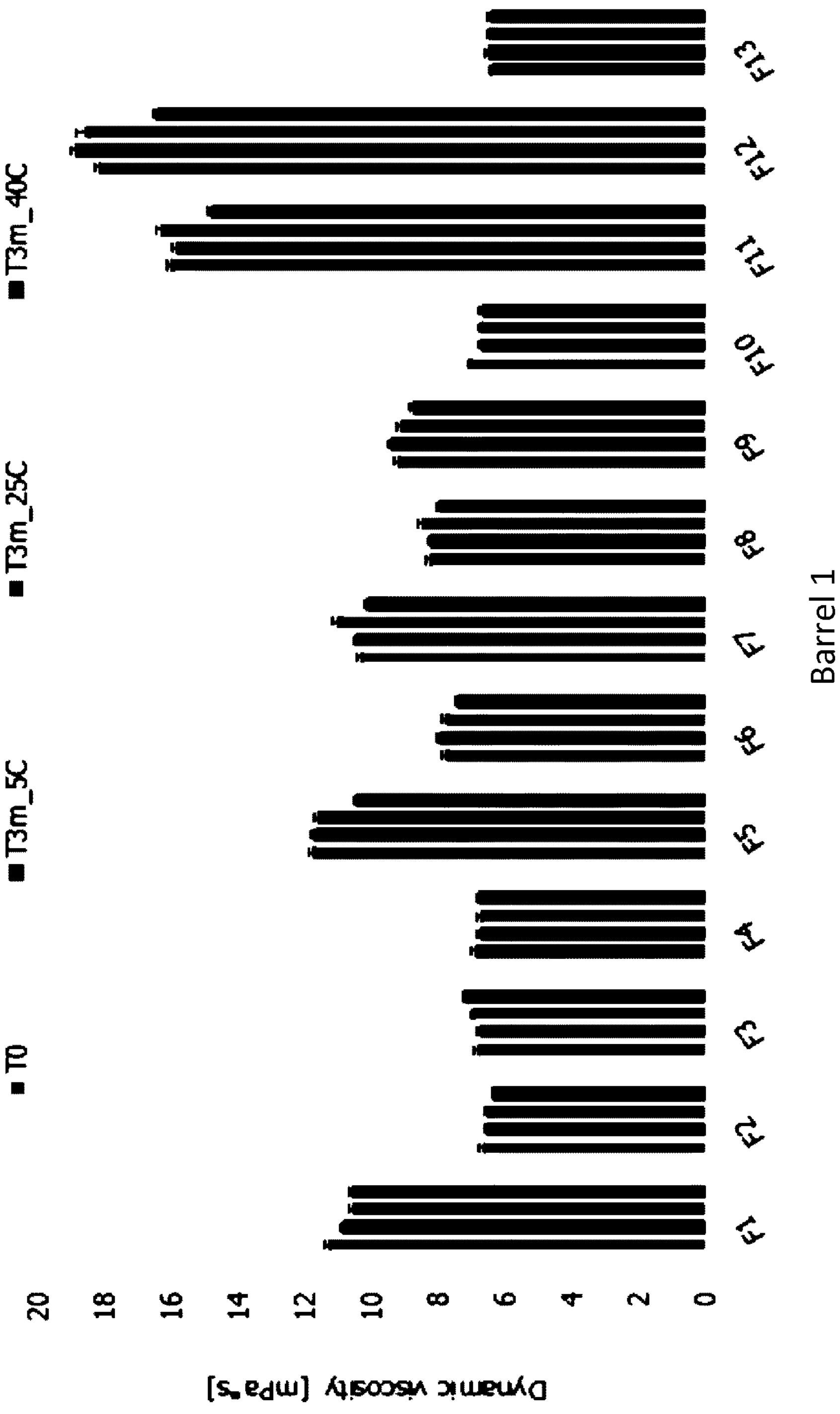

T0
T3m_5C
T3m_25C
T3m_40C
Dynamic viscosity [mPa*s]
20
18
16
14
12
10
8
6
4
2
0
F1
F2
F3
F4
F5
F6
F7
F8
F9
F10
F11
F12
F13
Barrel 1

FIG. 62B

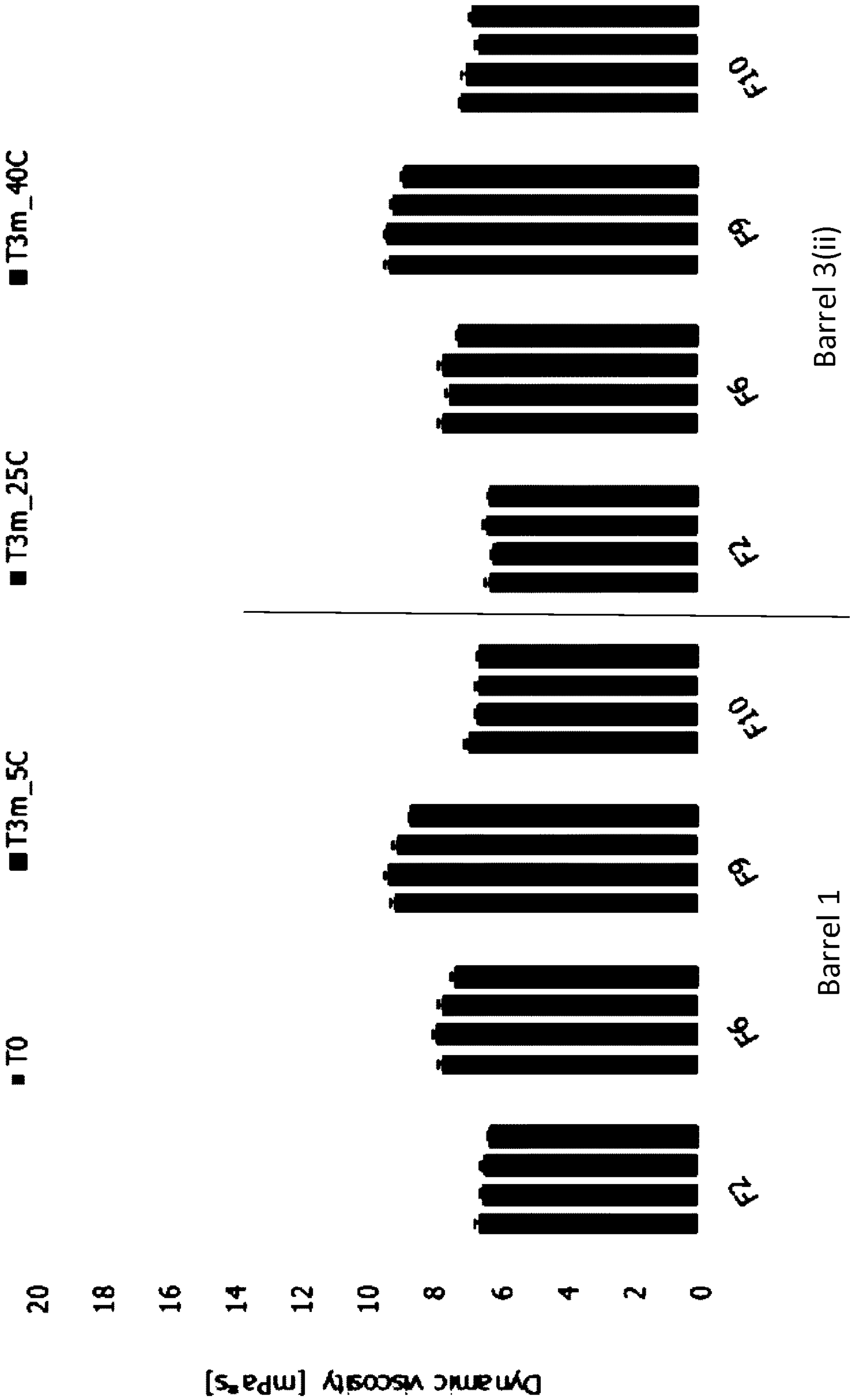
T0
T3m_5C
T3m_25C
T3m_40C
Dynamic viscosity [mPa*s]
20
18
16
14
12
10
8
6
4
2
0
F2
F6
F9
F10
F2
F6
F9
F10
Barrel 1
Barrel 3(ii)

FIG. 63A

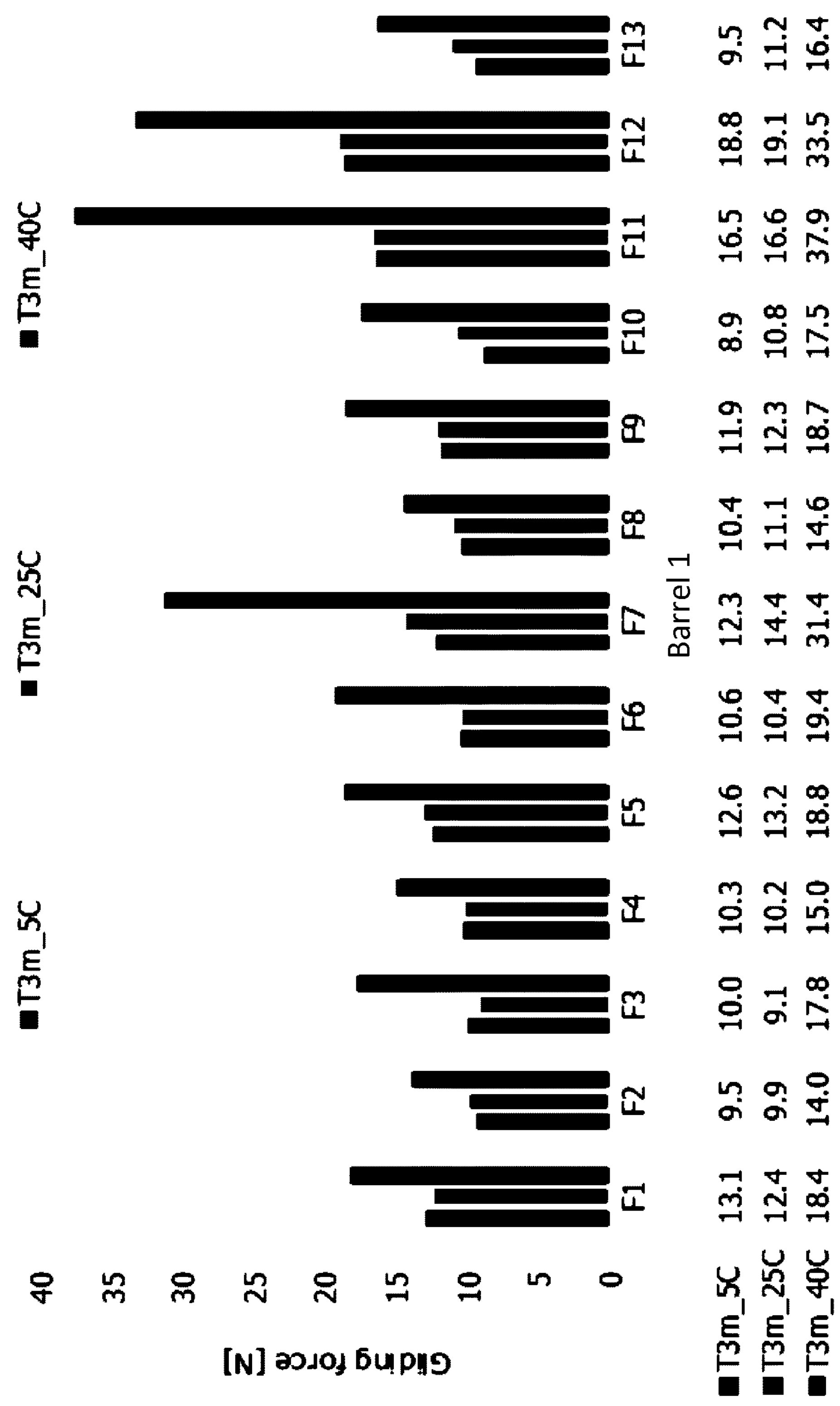
T3m_5C
T3m_25C
T3m_40C
Gliding force [N]
40
35
30
25
20
15
10
5
0
F1
F2
F3
F4
F5
F6
F7
F8
F9
F10
F11
F12
F13
Barrel 1
T3m_5C 13.1 9.5 10.0 10.3 12.6 10.6 12.3 10.4 11.9 8.9 16.5 18.8 9.5
T3m_25C 12.4 9.9 9.1 10.2 13.2 10.4 14.4 11.1 12.3 10.8 16.6 19.1 11.2
T3m_40C 18.4 14.0 17.8 15.0 18.8 19.4 31.4 14.6 18.7 17.5 37.9 33.5 16.4

FIG. 63B

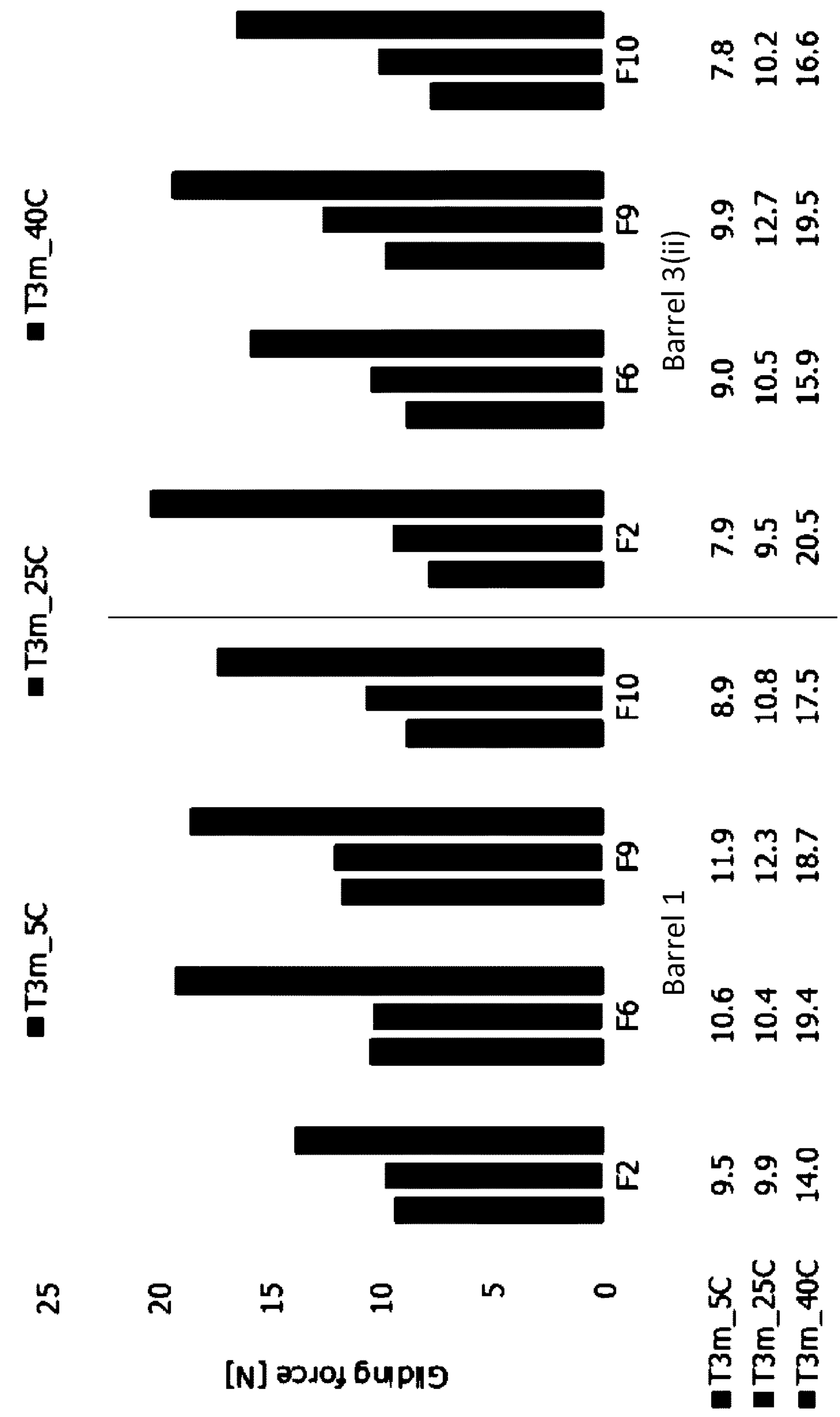

T3m_5C
T3m_25C
T3m_40C
Gliding force [N]
25
20
15
10
5
0
F2
F6
F9
F10
F2
F6
F9
F10
Barrel 1
Barrel 3(ii)
T3m_5C 9.5 10.6 11.9 8.9 7.9 9.0 9.9 7.8
T3m_25C 9.9 10.4 12.3 10.8 9.5 10.5 12.7 10.2
T3m_40C 14.0 19.4 18.7 17.5 20.5 15.9 19.5 16.6

FIG. 64A

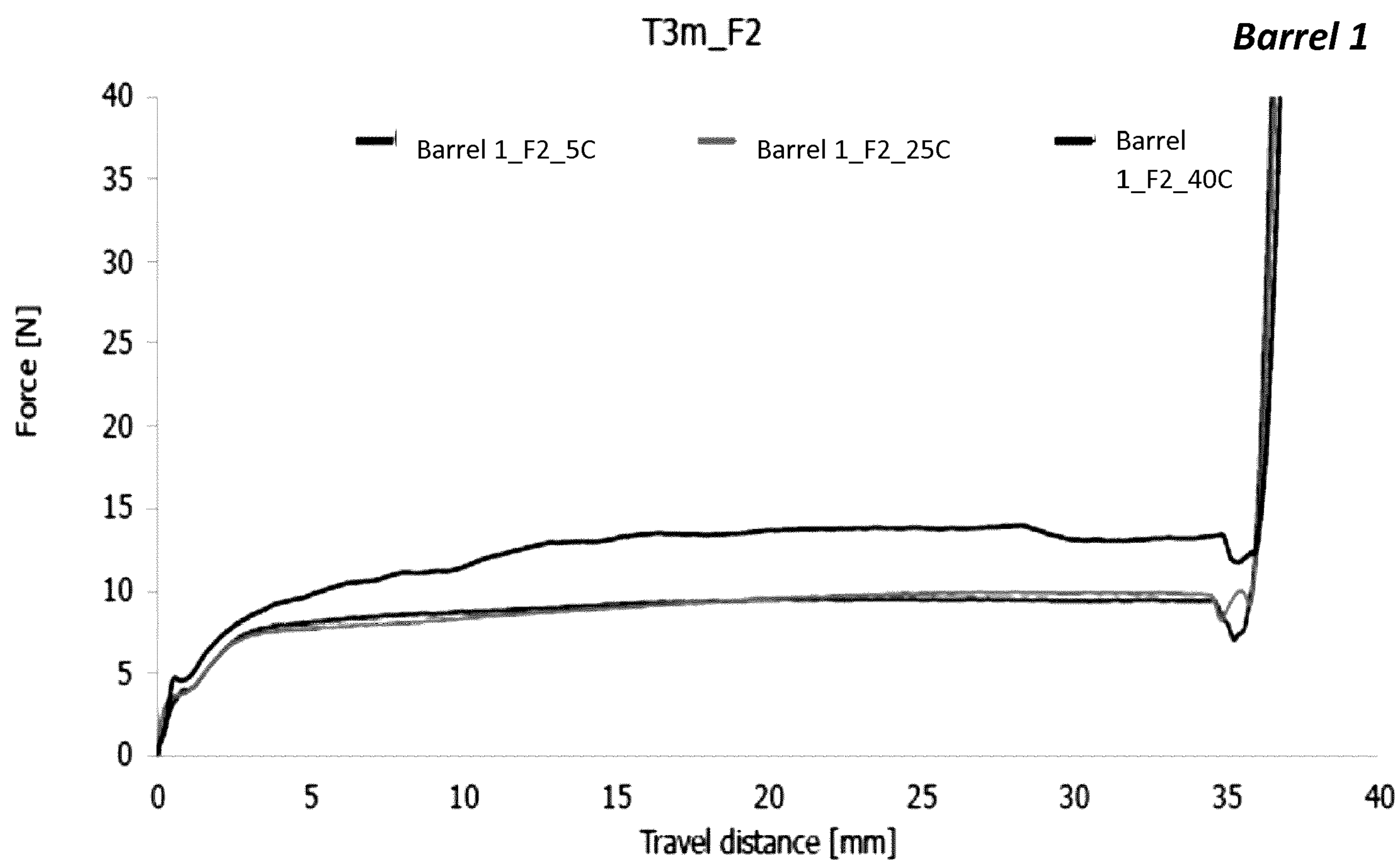
T3m_F2
Barrel 1
Force [N]
40
35
30
25
20
15
10
5
0
0
5
10
15
20
25
30
35
40
Travel distance [mm]
Barrel 1_F2_5C
Barrel 1_F2_25C
Barrel 1_F2_40C

FIG. 64B

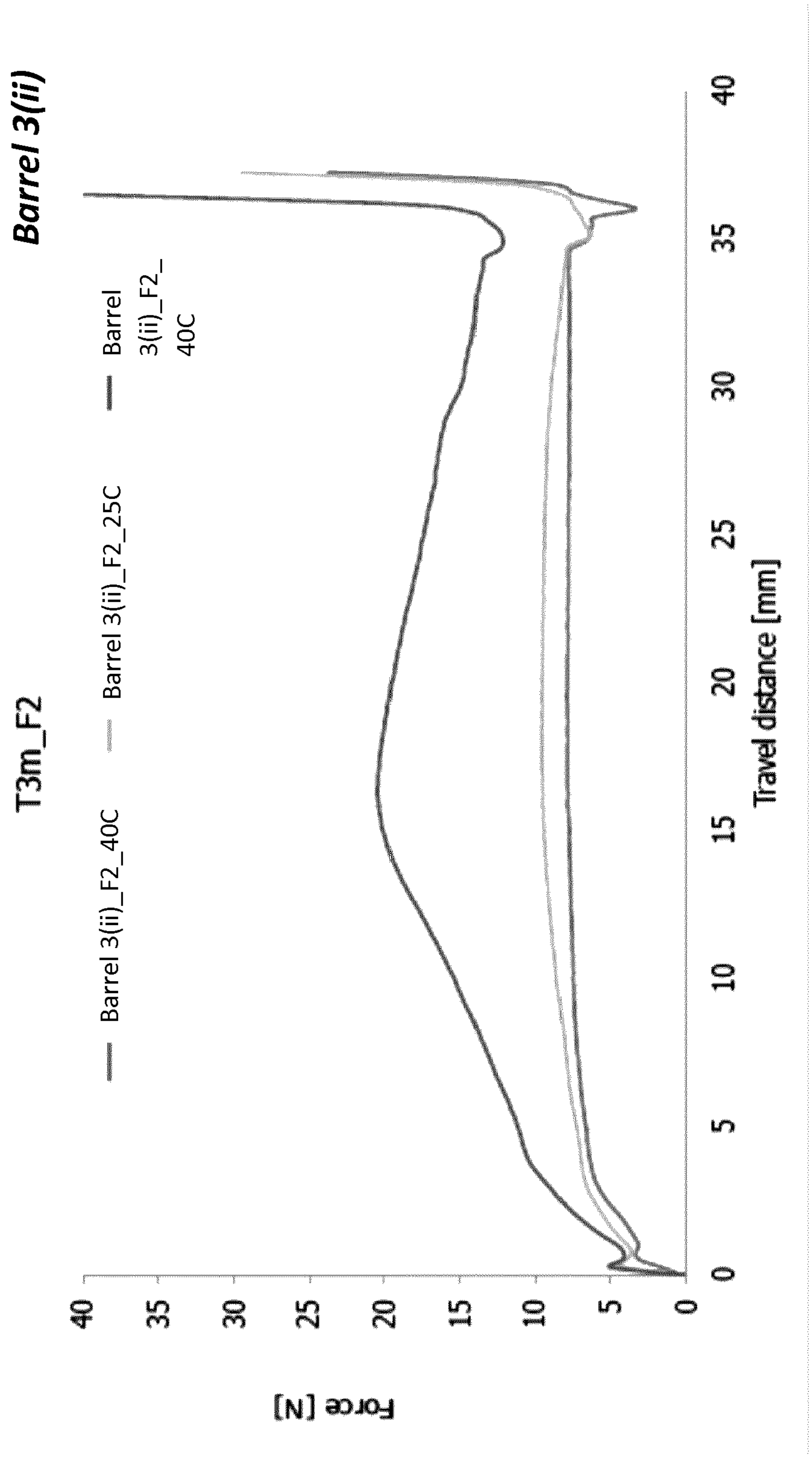
T3m_F2
Barrel 3(ii)
Barrel 3(ii)_F2_40C
Barrel 3(ii)_F2_25C
Barrel 3(ii)_F2_40C
Force [N]
0
5
10
15
20
25
30
35
40
Travel distance [mm]

FIG. 64C

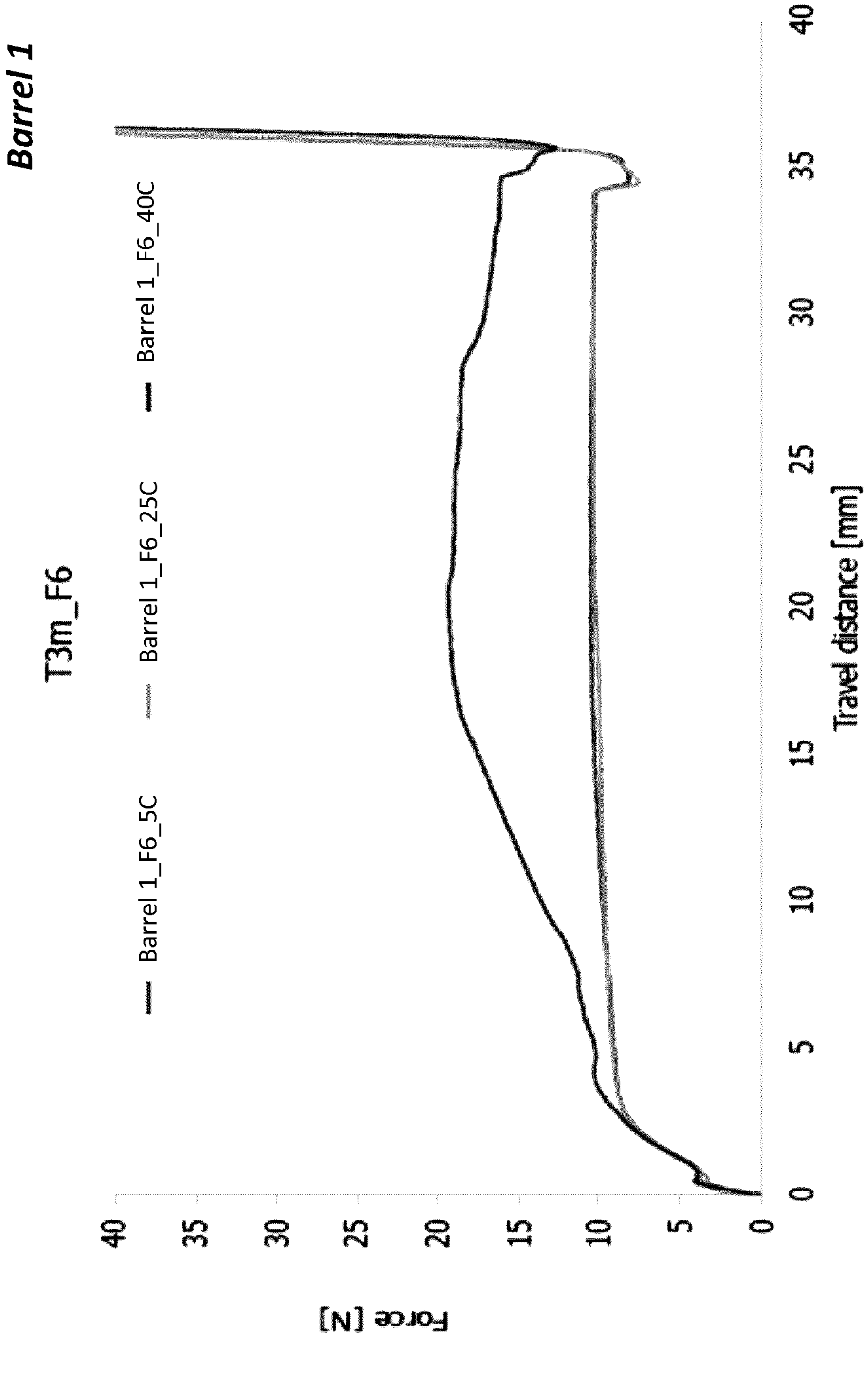
Barrel 1
T3m_F6
Barrel 1_F6_5C
Barrel 1_F6_25C
Barrel 1_F6_40C
Force [N]
Travel distance [mm]
0
5
10
15
20
25
30
35
40

FIG. 64D

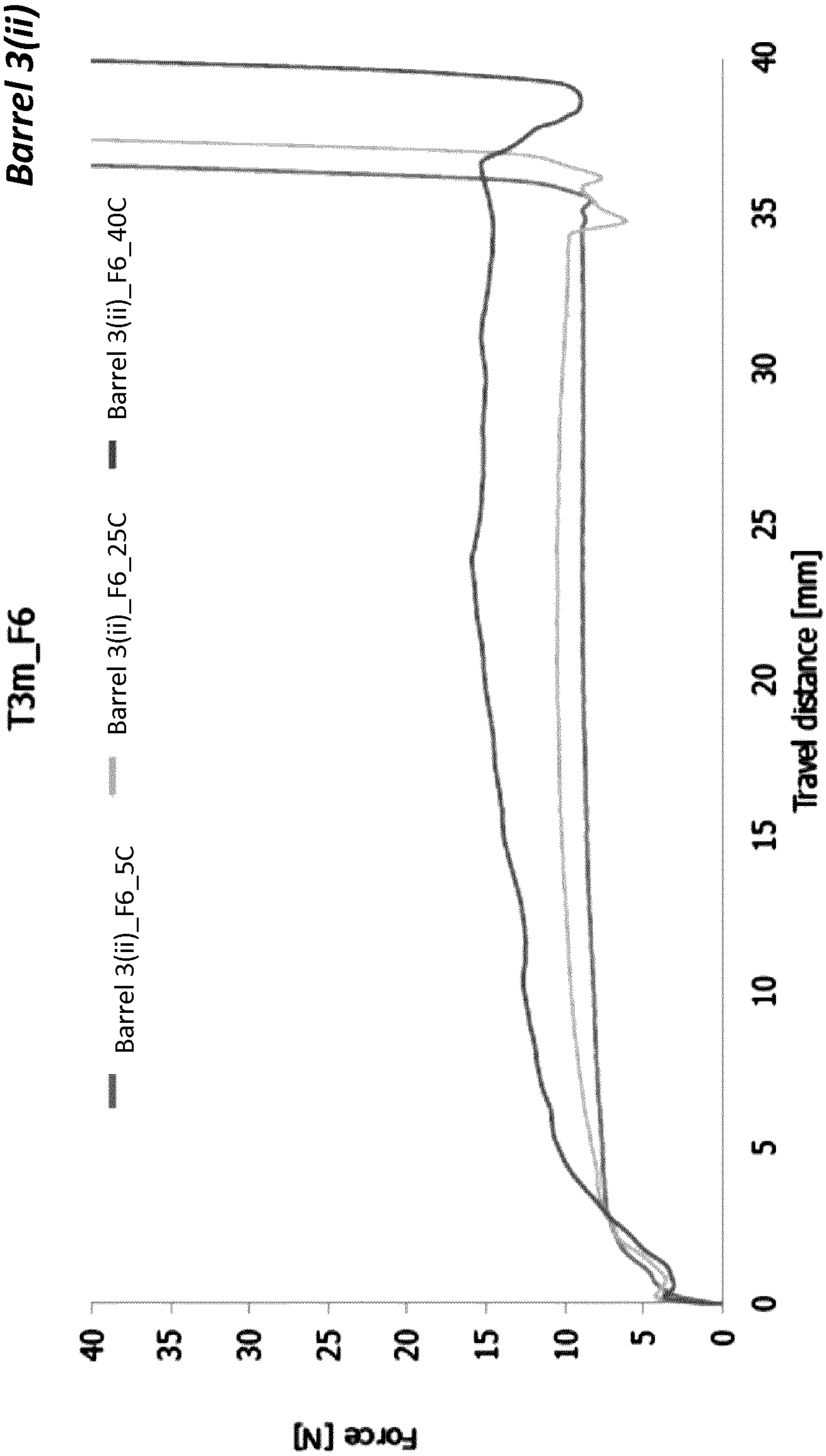
Barrel 3(ii)
T3m_F6
Barrel 3(ii)_F6_5C
Barrel 3(ii)_F6_25C
Barrel 3(ii)_F6_40C
Force [N]
Travel distance [mm]
0
5
10
15
20
25
30
35
40
0
5
10
15
20
25
30
35
40

FIG. 65A

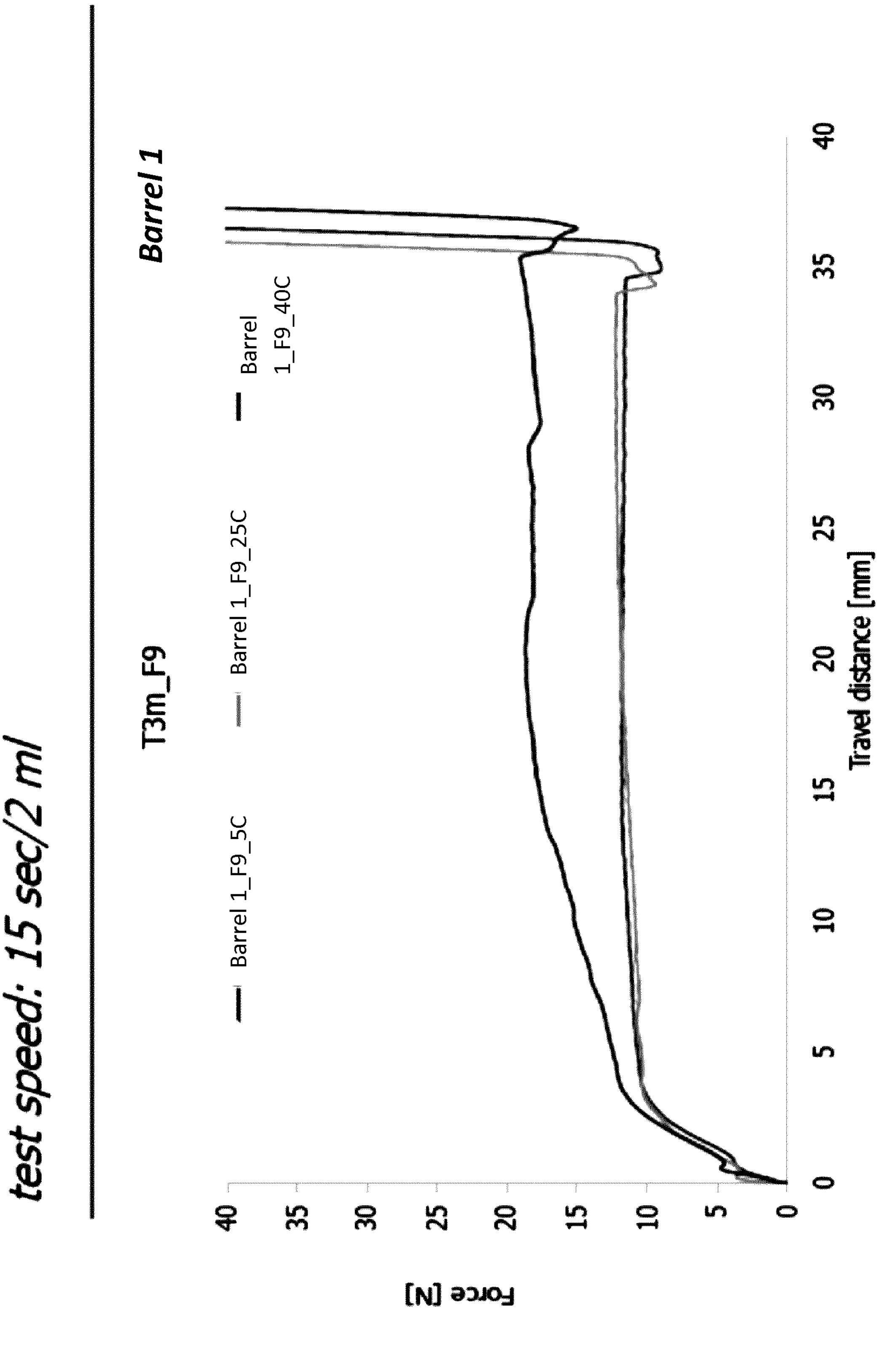
test speed: 15 sec/2 ml
T3m_F9
Barrel 1
Barrel 1_F9_5C
Barrel 1_F9_25C
Barrel 1_F9_40C
Force [N]
Travel distance [mm]
0
5
10
15
20
25
30
35
40

**FIG. 65B**

*All PFS 27G ½"*

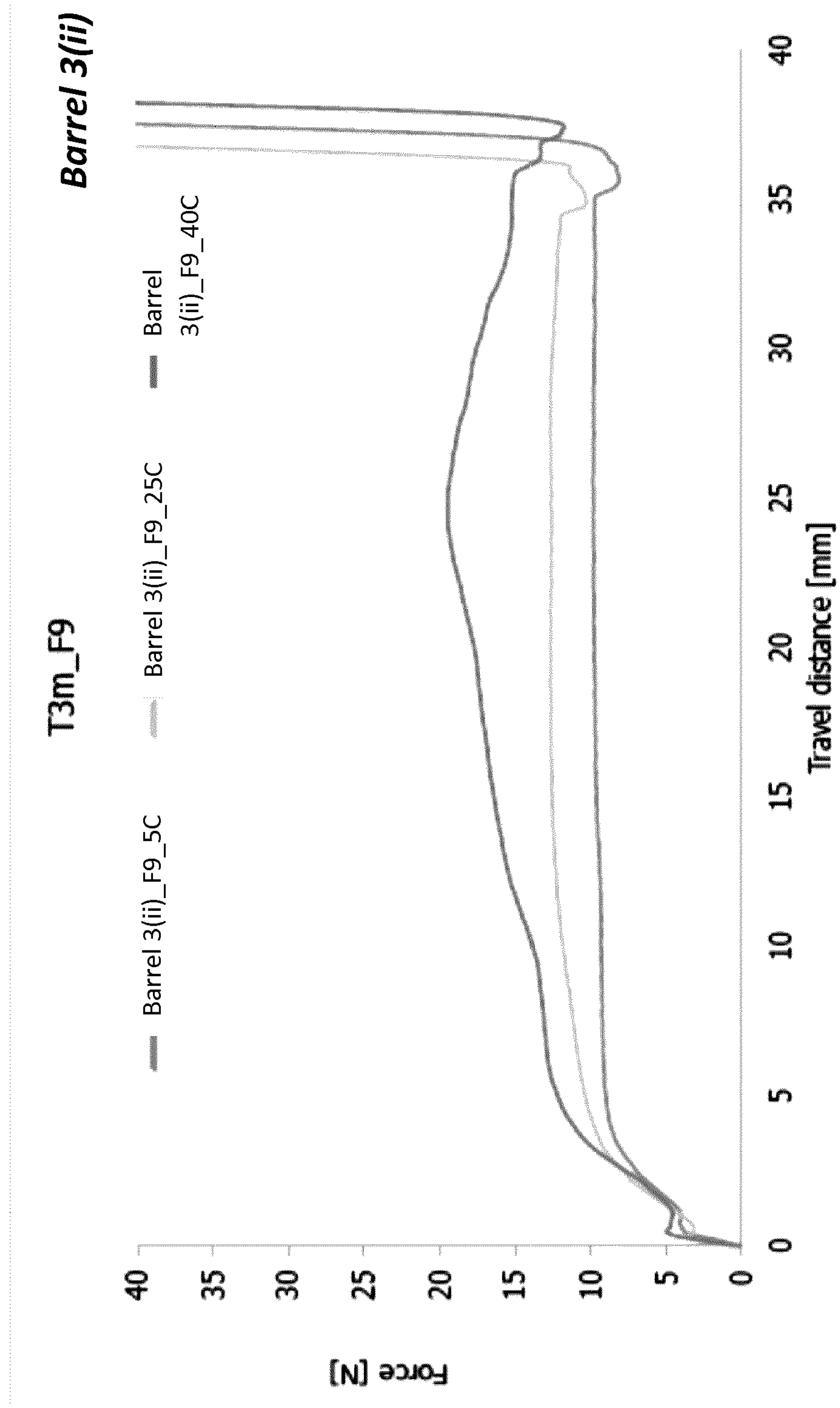

FIG. 65C

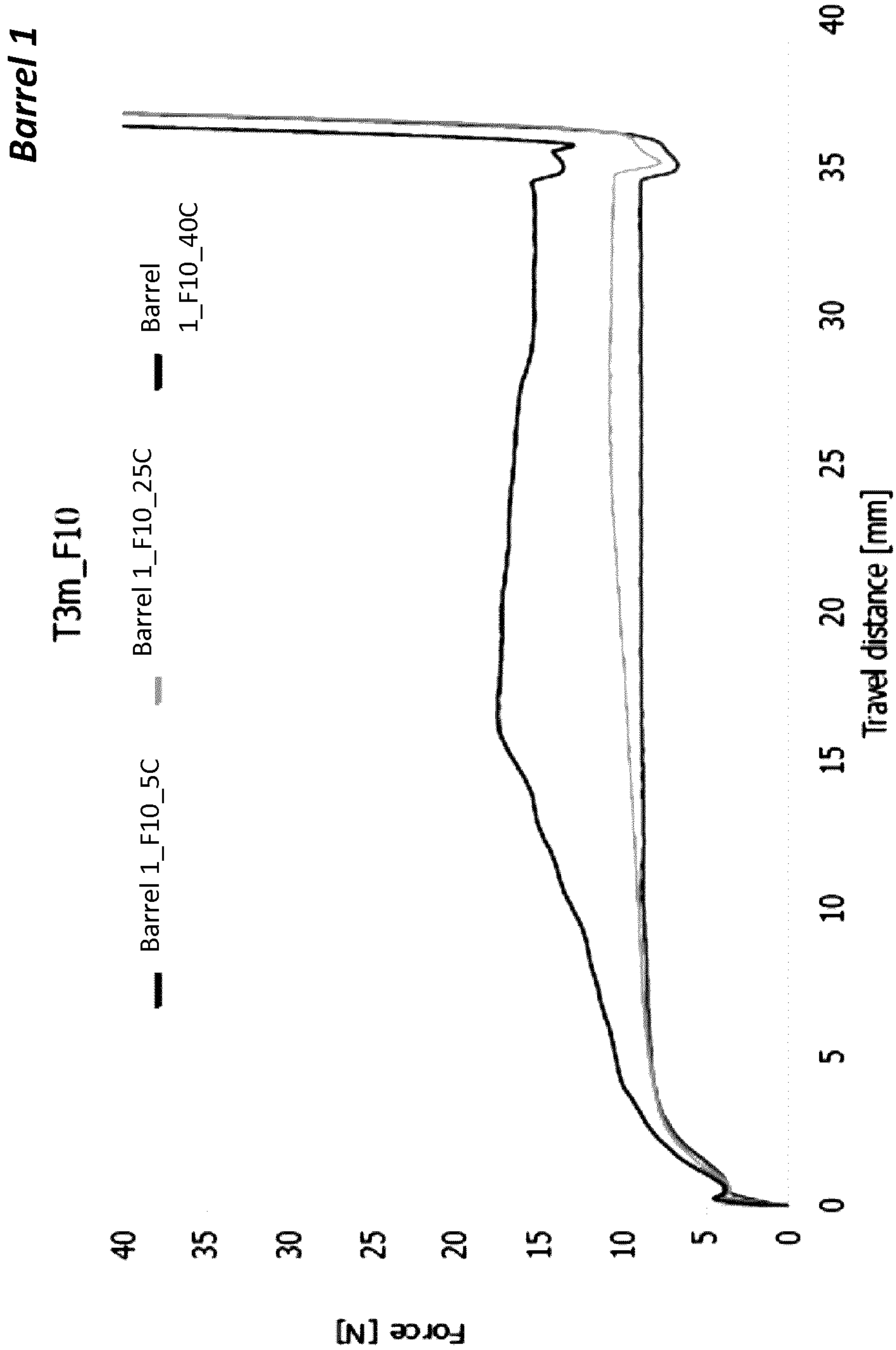
Barrel 1
T3m_F10
Barrel 1_F10_5C
Barrel 1_F10_25C
Barrel 1_F10_40C
Force [N]
0
5
10
15
20
25
30
35
40
Travel distance [mm]
0
5
10
15
20
25
30
35
40

FIG. 65D

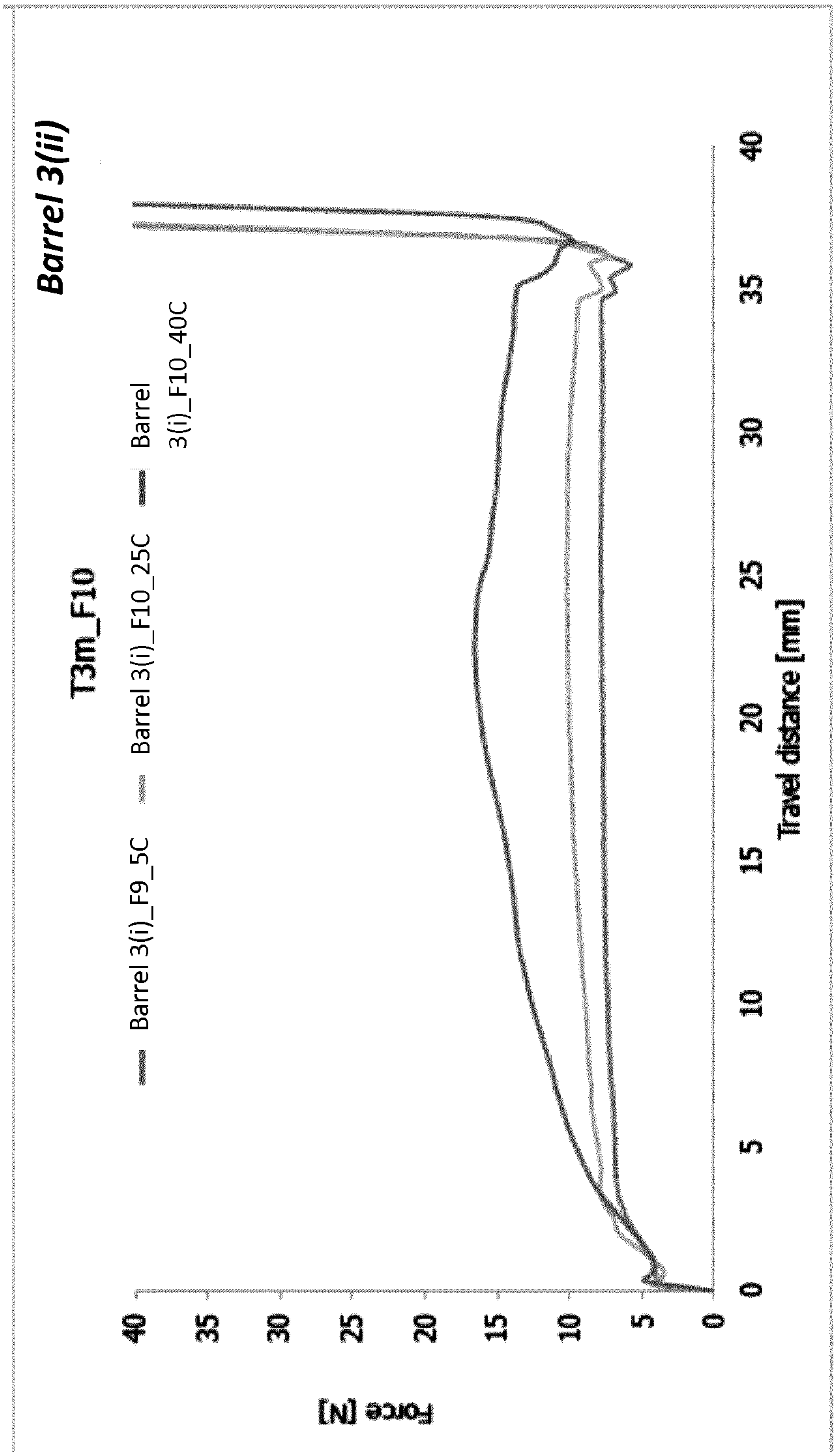
Barrel 3(ii)
T3m_F10
Barrel 3(i)_F9_5C
Barrel 3(i)_F10_25C
Barrel 3(i)_F10_40C
Force [N]
Travel distance [mm]
0
5
10
15
20
25
30
35
40

FIG. 66A

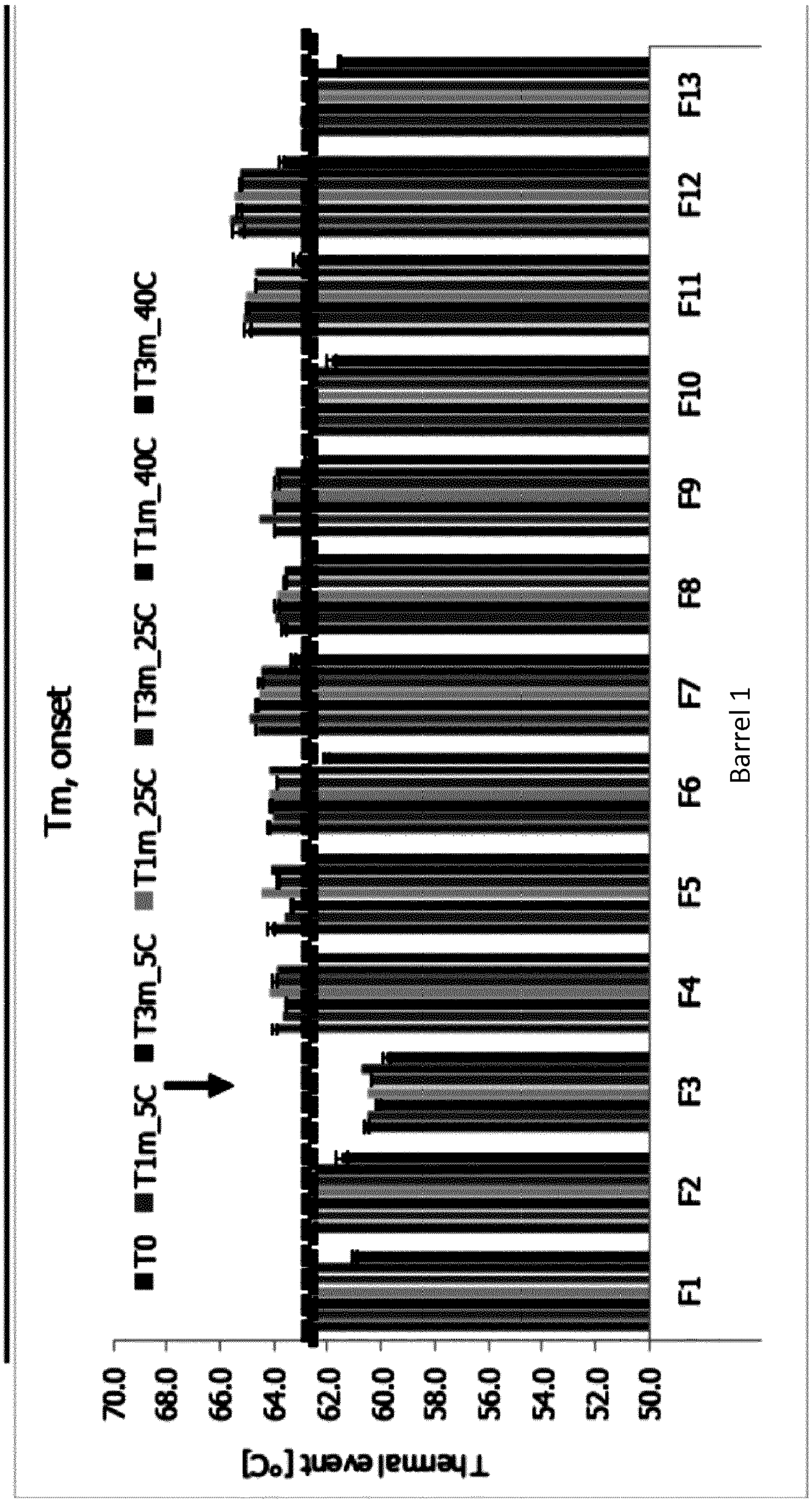
Tm, onset
T0
T1m_5C
T3m_5C
T1m_25C
T3m_25C
T1m_40C
T3m_40C
Themal event [°C]
70.0
68.0
66.0
64.0
62.0
60.0
58.0
56.0
54.0
52.0
50.0
F1
F2
F3
F4
F5
F6
F7
F8
F9
F10
F11
F12
F13
Barrel 1

FIG. 66B

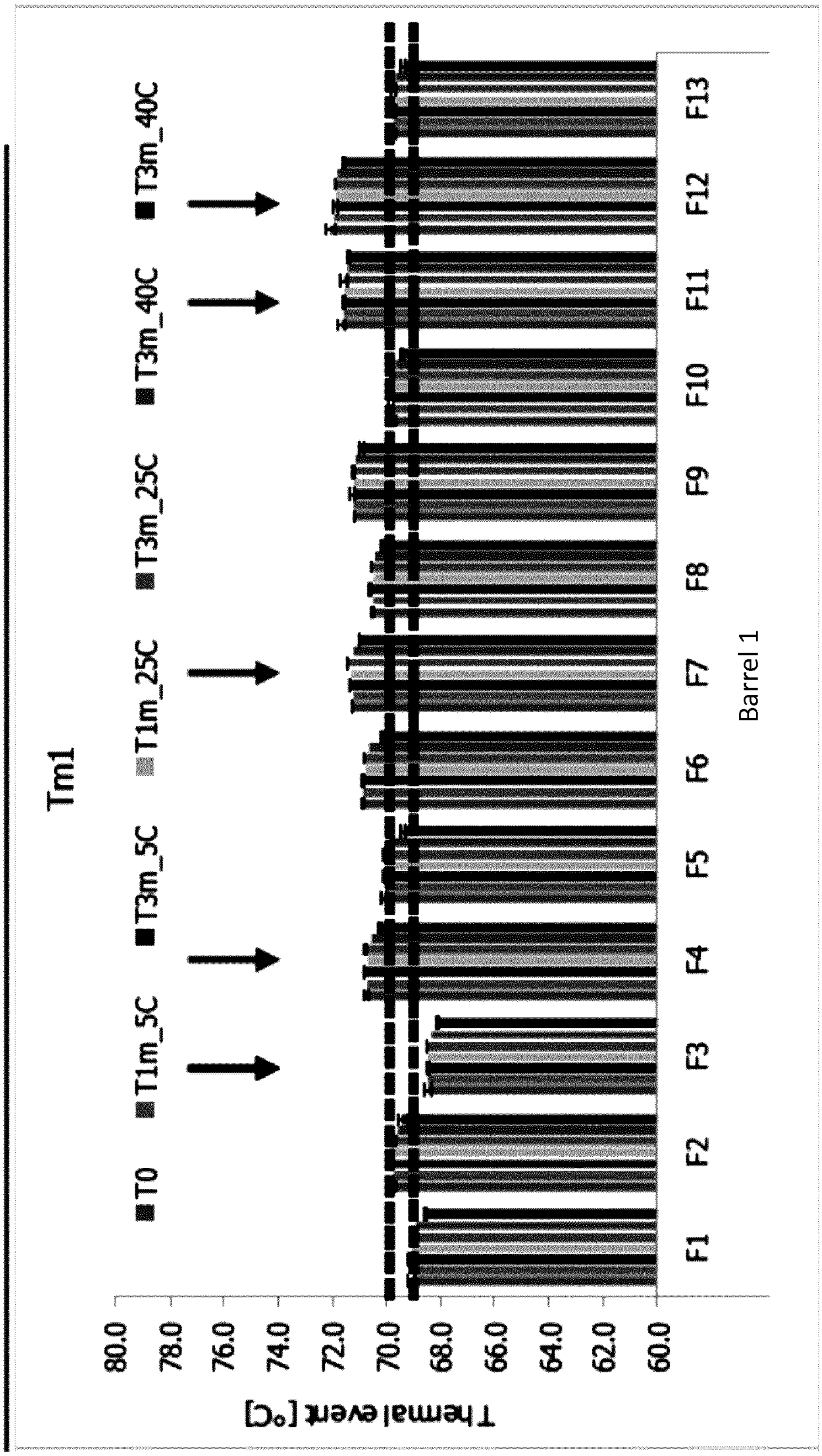
Tm1
T0
T1m_5C
T3m_5C
T1m_25C
T3m_25C
T3m_40C
T3m_40C
Themal event [°C]
80.0
78.0
76.0
74.0
72.0
70.0
68.0
66.0
64.0
62.0
60.0
F1
F2
F3
F4
F5
F6
F7
F8
F9
F10
F11
F12
F13
Barrel 1

FIG. 67

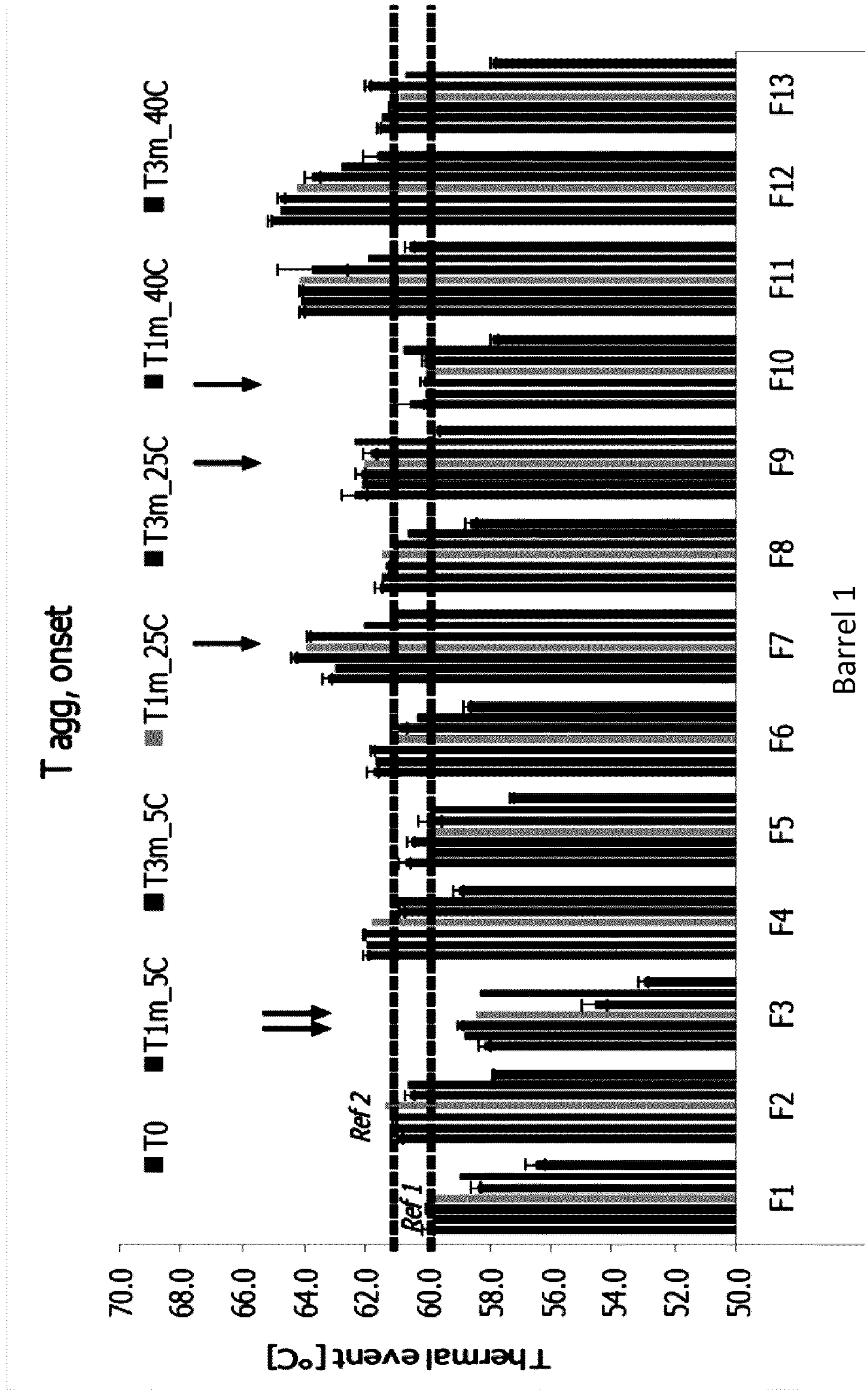
T agg, onset
T0
T1m_5C
T3m_5C
T1m_25C
T3m_25C
T1m_40C
T3m_40C
Thermal event [°C]
70.0
68.0
66.0
64.0
62.0
60.0
58.0
56.0
54.0
52.0
50.0
Ref 1
Ref 2
F1
F2
F3
F4
F5
F6
F7
F8
F9
F10
F11
F12
F13
Barrel 1

FIG. 68A

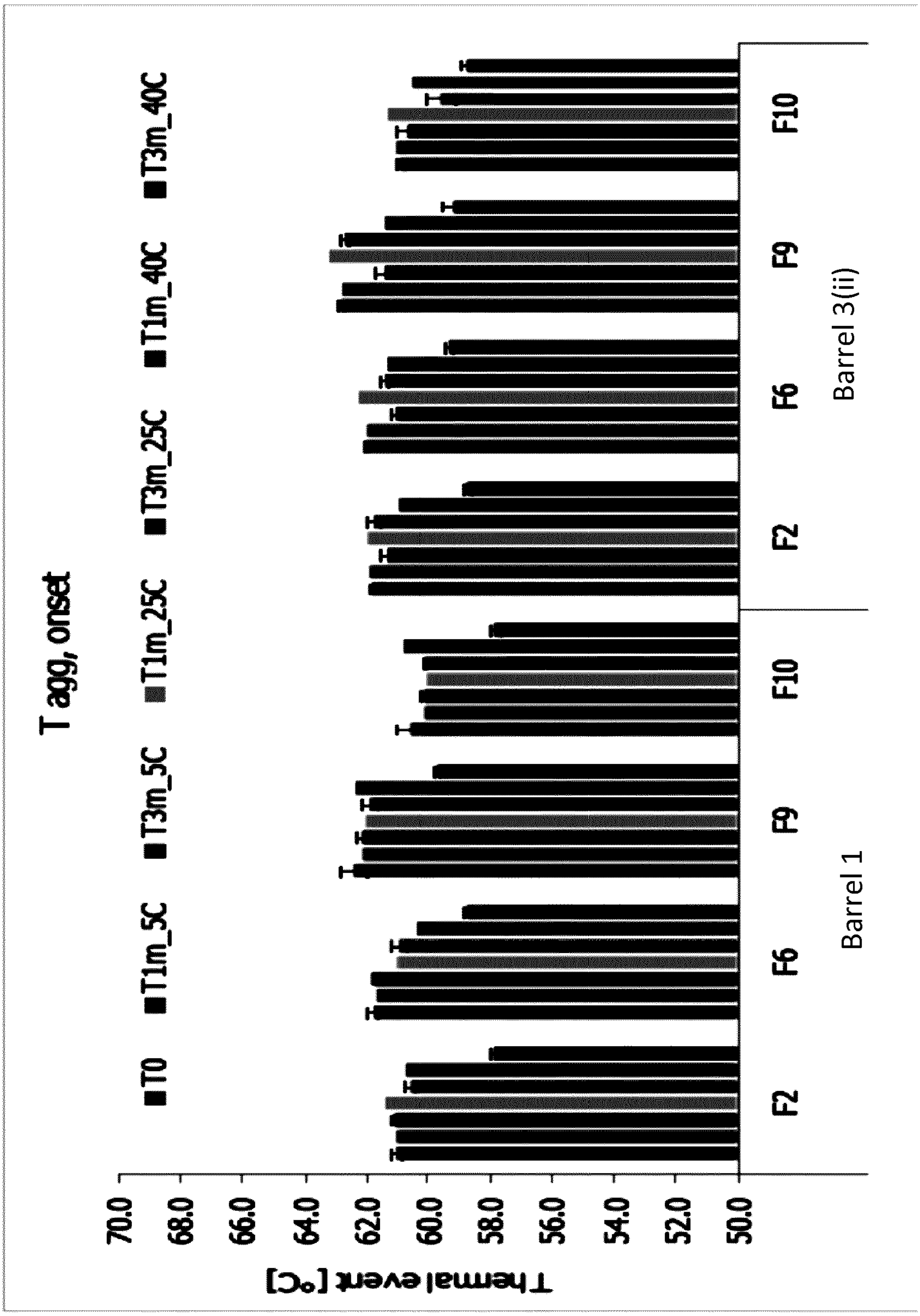
T agg, onset
T0
T1m_5C
T3m_5C
T1m_25C
T3m_25C
T1m_40C
T3m_40C
Thermal event [°C]
70.0
68.0
66.0
64.0
62.0
60.0
58.0
56.0
54.0
52.0
50.0
F2
F6
F9
F10
F2
F6
F9
F10
Barrel 1
Barrel 3(ii)

FIG. 68B

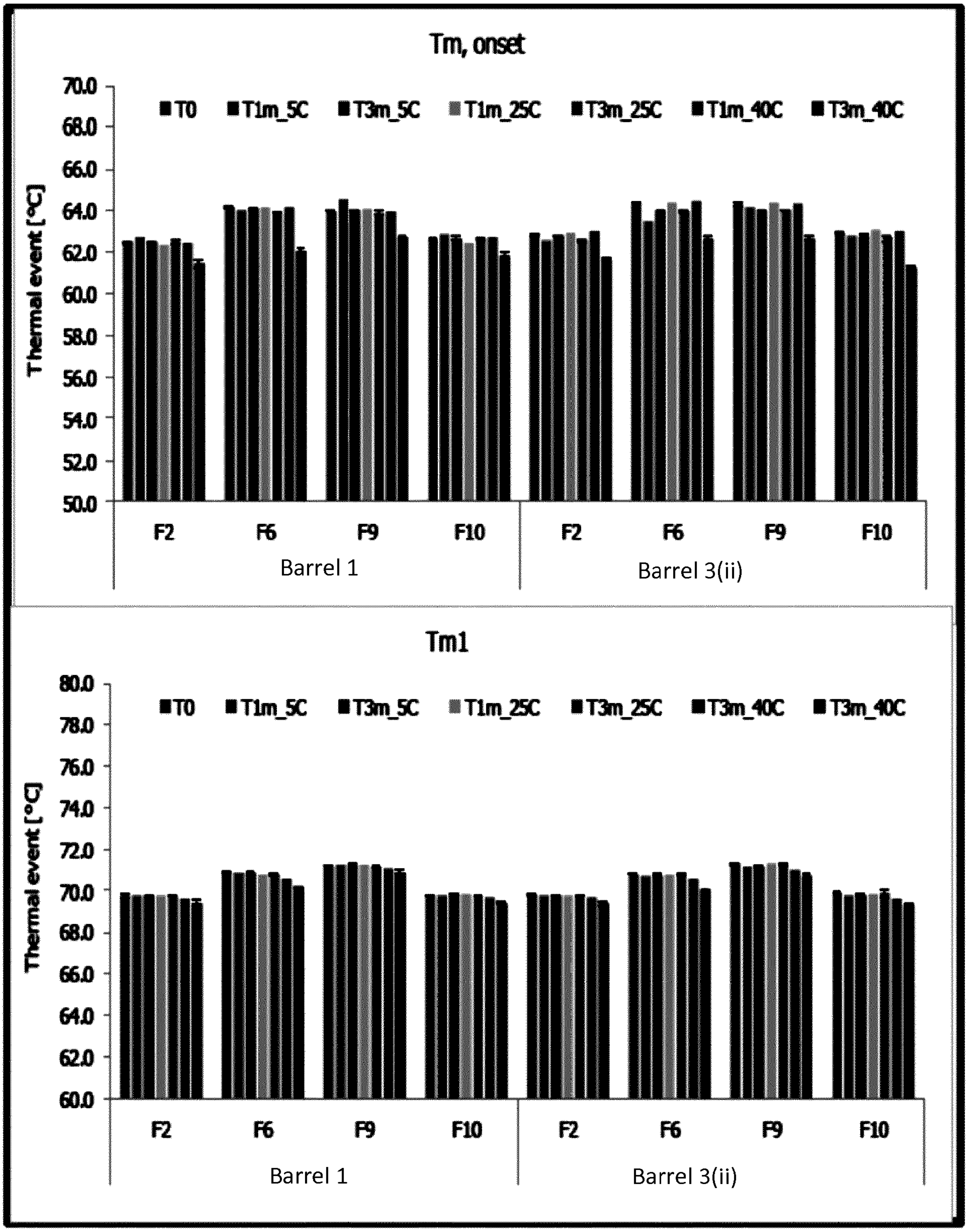
Tm, onset
T0
T1m_5C
T3m_5C
T1m_25C
T3m_25C
T1m_40C
T3m_40C
Thermal event [°C]
70.0
68.0
66.0
64.0
62.0
60.0
58.0
56.0
54.0
52.0
50.0
F2
F6
F9
F10
Barrel 1
Barrel 3(ii)
Tm1
T0
T1m_5C
T3m_5C
T1m_25C
T3m_25C
T3m_40C
T3m_40C
Thermal event [°C]
80.0
78.0
76.0
74.0
72.0
70.0
68.0
66.0
64.0
62.0
60.0
F2
F6
F9
F10
Barrel 1
Barrel 3(ii)

# FIG. 69A

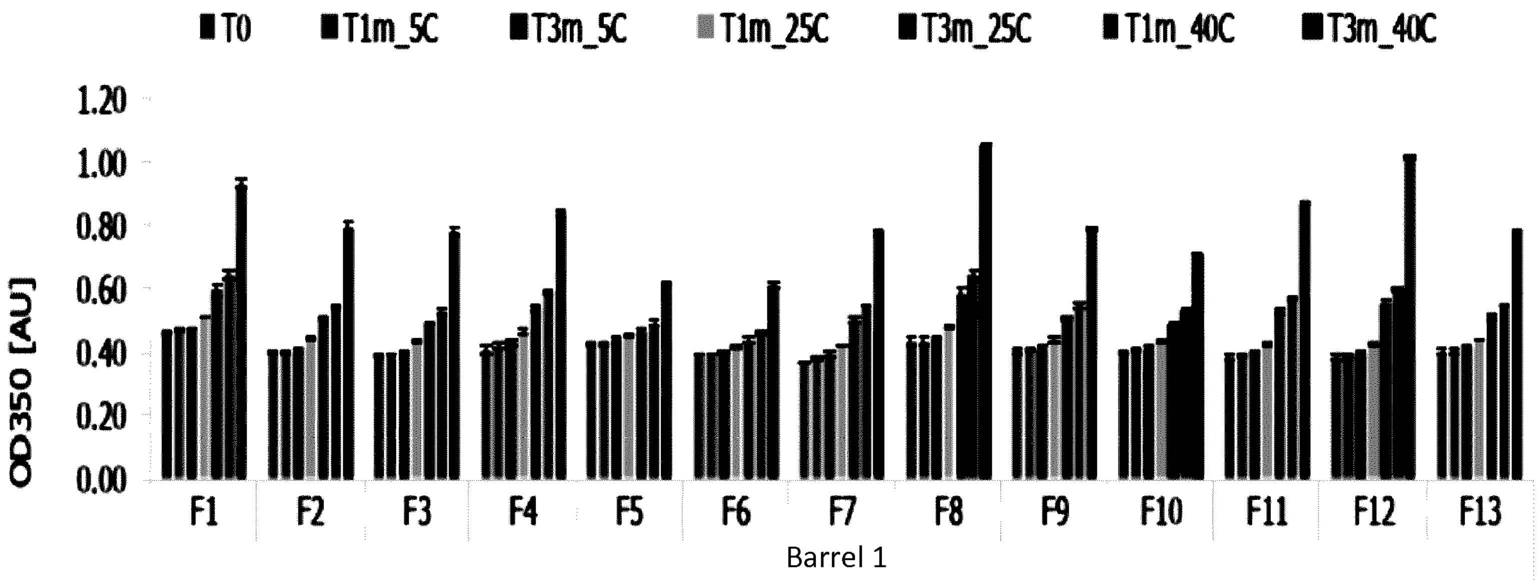


| | F1 | F2 | F3 | F4 | F5 | F6 | F7 | F8 | F9 | F10 | F11 | F12 | F13 |
|---|---|---|---|---|---|---|---|---|---|---|---|---|---|
| T0 | 0.462 | 0.399 | 0.386 | 0.411 | 0.423 | 0.394 | 0.370 | 0.434 | 0.405 | 0.402 | 0.386 | 0.386 | 0.404 |
| T1m_5C | 0.471 | 0.403 | 0.397 | 0.421 | 0.427 | 0.396 | 0.382 | 0.436 | 0.407 | 0.405 | 0.388 | 0.391 | 0.405 |
| T3m_5C | 0.478 | 0.412 | 0.403 | 0.431 | 0.439 | 0.400 | 0.394 | 0.439 | 0.416 | 0.418 | 0.399 | 0.403 | 0.414 |
| T1m_25C | 0.512 | 0.444 | 0.436 | 0.466 | 0.449 | 0.414 | 0.424 | 0.481 | 0.441 | 0.435 | 0.428 | 0.431 | 0.439 |
| T3m_25C | 0.598 | 0.509 | 0.486 | 0.540 | 0.469 | 0.437 | 0.501 | 0.586 | 0.505 | 0.489 | 0.534 | 0.557 | 0.513 |
| T1m_40C | 0.644 | 0.544 | 0.532 | 0.588 | 0.491 | 0.461 | 0.548 | 0.642 | 0.552 | 0.535 | 0.573 | 0.596 | 0.554 |
| T3m_40C | 0.931 | 0.796 | 0.782 | 0.841 | 0.618 | 0.613 | 0.776 | 1.056 | 0.787 | 0.715 | 0.869 | 1.016 | 0.782 |

# FIG. 69B

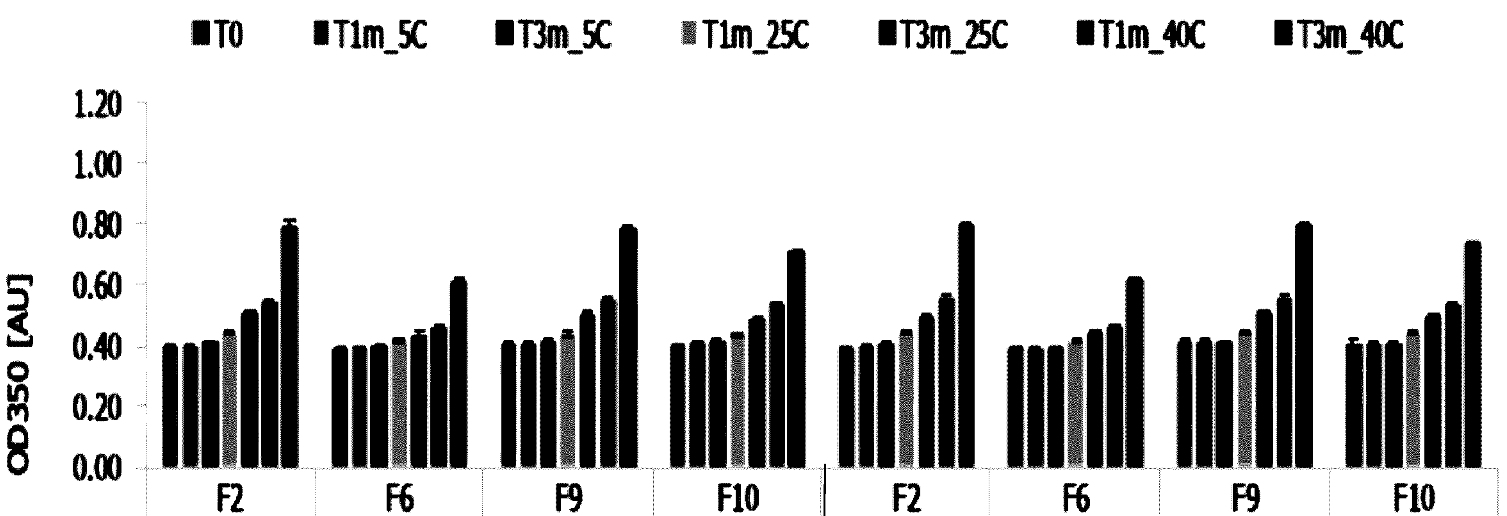


| | Barrel 1 | | | | Barrel 3(ii) | | | |
|---|---|---|---|---|---|---|---|---|
| | F2 | F6 | F9 | F10 | F2 | F6 | F9 | F10 |
| T0 | 0.399 | 0.394 | 0.405 | 0.402 | 0.393 | 0.392 | 0.411 | 0.408 |
| T1m_5C | 0.403 | 0.396 | 0.407 | 0.405 | 0.402 | 0.395 | 0.417 | 0.410 |
| T3m_5C | 0.412 | 0.400 | 0.416 | 0.418 | 0.405 | 0.396 | 0.413 | 0.410 |
| T1m_25C | 0.444 | 0.414 | 0.441 | 0.435 | 0.442 | 0.412 | 0.447 | 0.439 |
| T3m_25C | 0.509 | 0.437 | 0.505 | 0.489 | 0.497 | 0.440 | 0.507 | 0.496 |
| T1m_40C | 0.544 | 0.461 | 0.552 | 0.535 | 0.557 | 0.459 | 0.558 | 0.535 |
| T3m_40C | 0.796 | 0.613 | 0.787 | 0.715 | 0.797 | 0.619 | 0.802 | 0.737 |

**FIG. 70A**

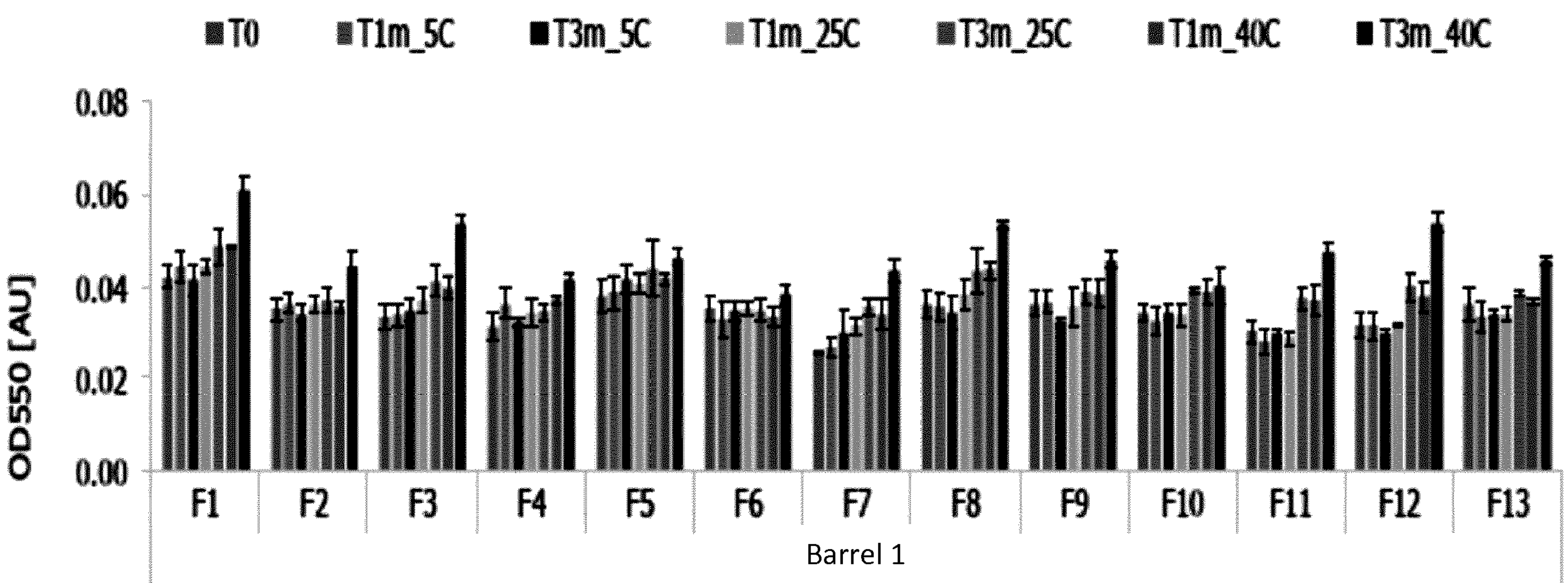


| | F1 | F2 | F3 | F4 | F5 | F6 | F7 | F8 | F9 | F10 | F11 | F12 | F13 |
|---|---|---|---|---|---|---|---|---|---|---|---|---|---|
| T0 | 0.042 | 0.035 | 0.034 | 0.031 | 0.038 | 0.035 | 0.026 | 0.036 | 0.036 | 0.034 | 0.030 | 0.032 | 0.036 |
| T1m_5C | 0.044 | 0.036 | 0.034 | 0.036 | 0.039 | 0.033 | 0.027 | 0.036 | 0.037 | 0.033 | 0.028 | 0.032 | 0.034 |
| T3m_5C | 0.042 | 0.034 | 0.035 | 0.033 | 0.041 | 0.035 | 0.030 | 0.034 | 0.033 | 0.034 | 0.030 | 0.030 | 0.034 |
| T1m_25C | 0.044 | 0.036 | 0.037 | 0.035 | 0.041 | 0.035 | 0.032 | 0.039 | 0.036 | 0.034 | 0.029 | 0.032 | 0.034 |
| T3m_25C | 0.049 | 0.037 | 0.041 | 0.035 | 0.044 | 0.035 | 0.036 | 0.043 | 0.039 | 0.039 | 0.037 | 0.040 | 0.039 |
| T1m_40C | 0.049 | 0.036 | 0.040 | 0.037 | 0.042 | 0.034 | 0.034 | 0.044 | 0.039 | 0.039 | 0.037 | 0.038 | 0.037 |
| T3m_40C | 0.061 | 0.044 | 0.054 | 0.041 | 0.046 | 0.038 | 0.043 | 0.054 | 0.046 | 0.040 | 0.047 | 0.054 | 0.046 |

# FIG. 70B

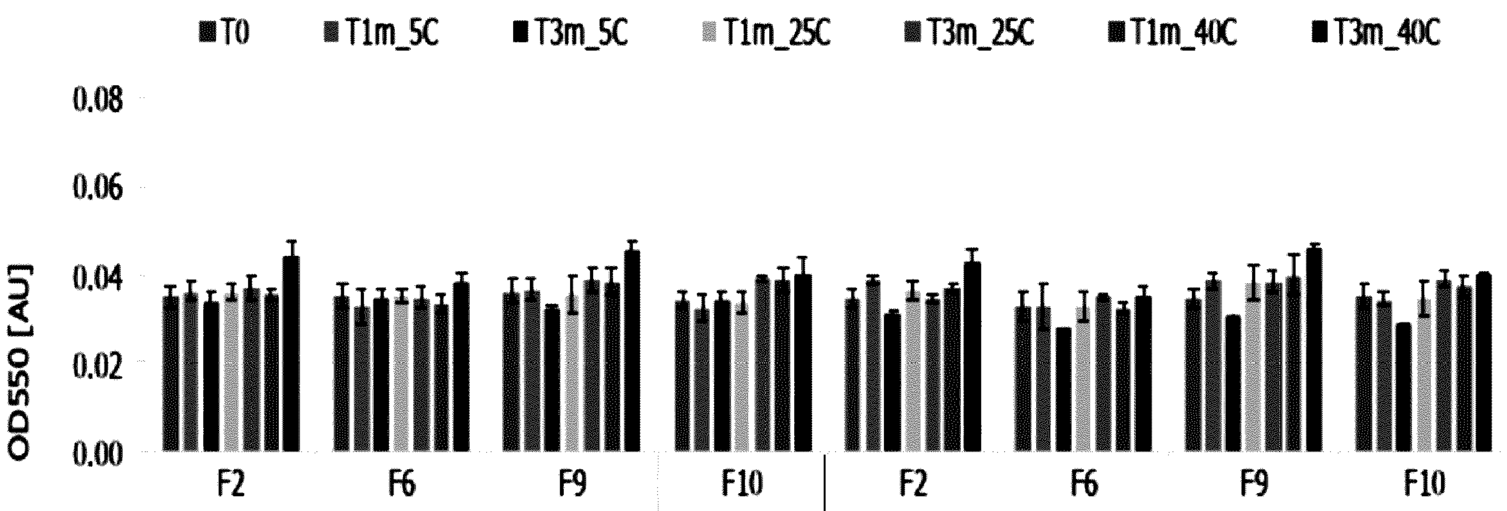


| | Barrel 1 | | | | Barrel 3(ii) | | | |
|---|---|---|---|---|---|---|---|---|
| | F2 | F6 | F9 | F10 | F2 | F6 | F9 | F10 |
| T0 | 0.035 | 0.035 | 0.036 | 0.034 | 0.035 | 0.033 | 0.035 | 0.035 |
| T1m_5C | 0.036 | 0.033 | 0.037 | 0.033 | 0.039 | 0.033 | 0.039 | 0.035 |
| T3m_5C | 0.034 | 0.035 | 0.033 | 0.034 | 0.031 | 0.028 | 0.031 | 0.029 |
| T1m_25C | 0.036 | 0.035 | 0.036 | 0.034 | 0.037 | 0.033 | 0.038 | 0.035 |
| T3m_25C | 0.037 | 0.035 | 0.039 | 0.039 | 0.035 | 0.035 | 0.038 | 0.039 |
| T1m_40C | 0.036 | 0.034 | 0.039 | 0.039 | 0.037 | 0.033 | 0.040 | 0.037 |
| T3m_40C | 0.044 | 0.038 | 0.046 | 0.040 | 0.043 | 0.035 | 0.046 | 0.040 |

FIG. 71

T3m

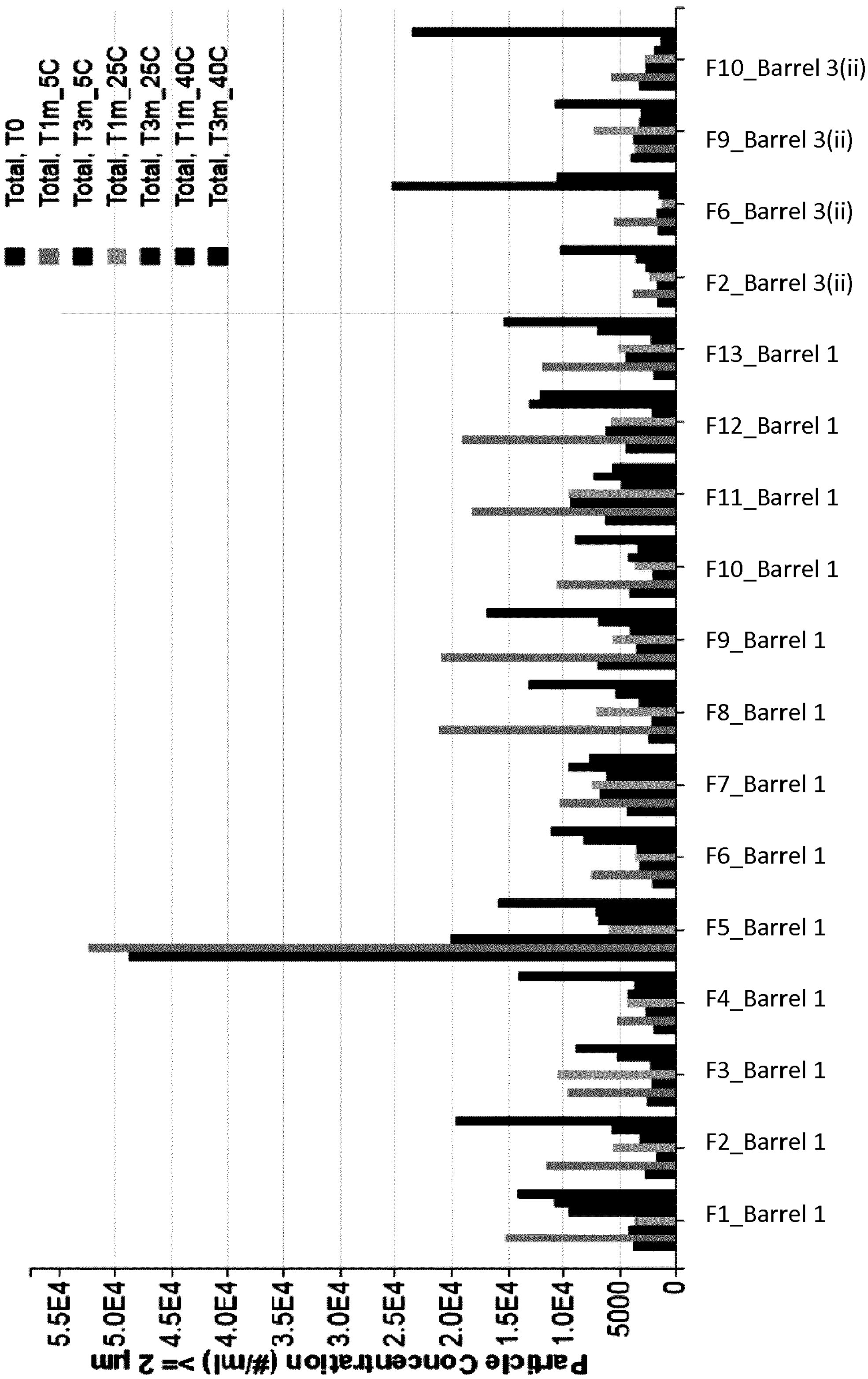
Total, T0
Total, T1m_5C
Total, T3m_5C
Total, T1m_25C
Total, T3m_25C
Total, T1m_40C
Total, T3m_40C
Particle Concentration (#/ml) >= 2 µm
5.5E4
5.0E4
4.5E4
4.0E4
3.5E4
3.0E4
2.5E4
2.0E4
1.5E4
1.0E4
5000
0
F1_Barrel 1
F2_Barrel 1
F3_Barrel 1
F4_Barrel 1
F5_Barrel 1
F6_Barrel 1
F7_Barrel 1
F8_Barrel 1
F9_Barrel 1
F10_Barrel 1
F11_Barrel 1
F12_Barrel 1
F13_Barrel 1
F2_Barrel 3(ii)
F6_Barrel 3(ii)
F9_Barrel 3(ii)
F10_Barrel 3(ii)

FIG. 72A

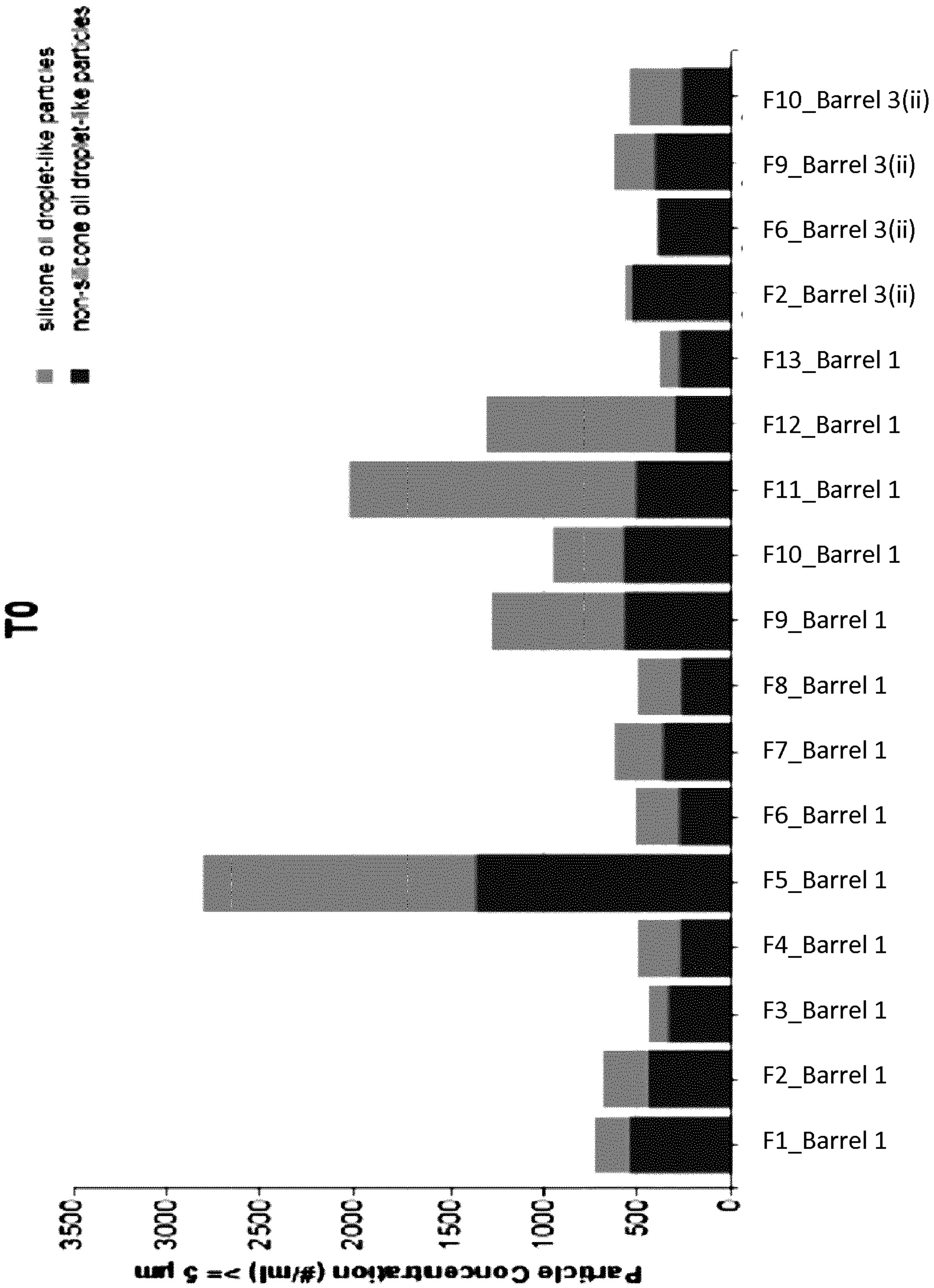
T0
silicone oil droplet-like particles
non-silicone oil droplet-like particles
Particle Concentration (#/ml) >= 5 µm
3500
3000
2500
2000
1500
1000
500
0
F1_Barrel 1
F2_Barrel 1
F3_Barrel 1
F4_Barrel 1
F5_Barrel 1
F6_Barrel 1
F7_Barrel 1
F8_Barrel 1
F9_Barrel 1
F10_Barrel 1
F11_Barrel 1
F12_Barrel 1
F13_Barrel 1
F2_Barrel 3(ii)
F6_Barrel 3(ii)
F9_Barrel 3(ii)
F10_Barrel 3(ii)

FIG. 72B

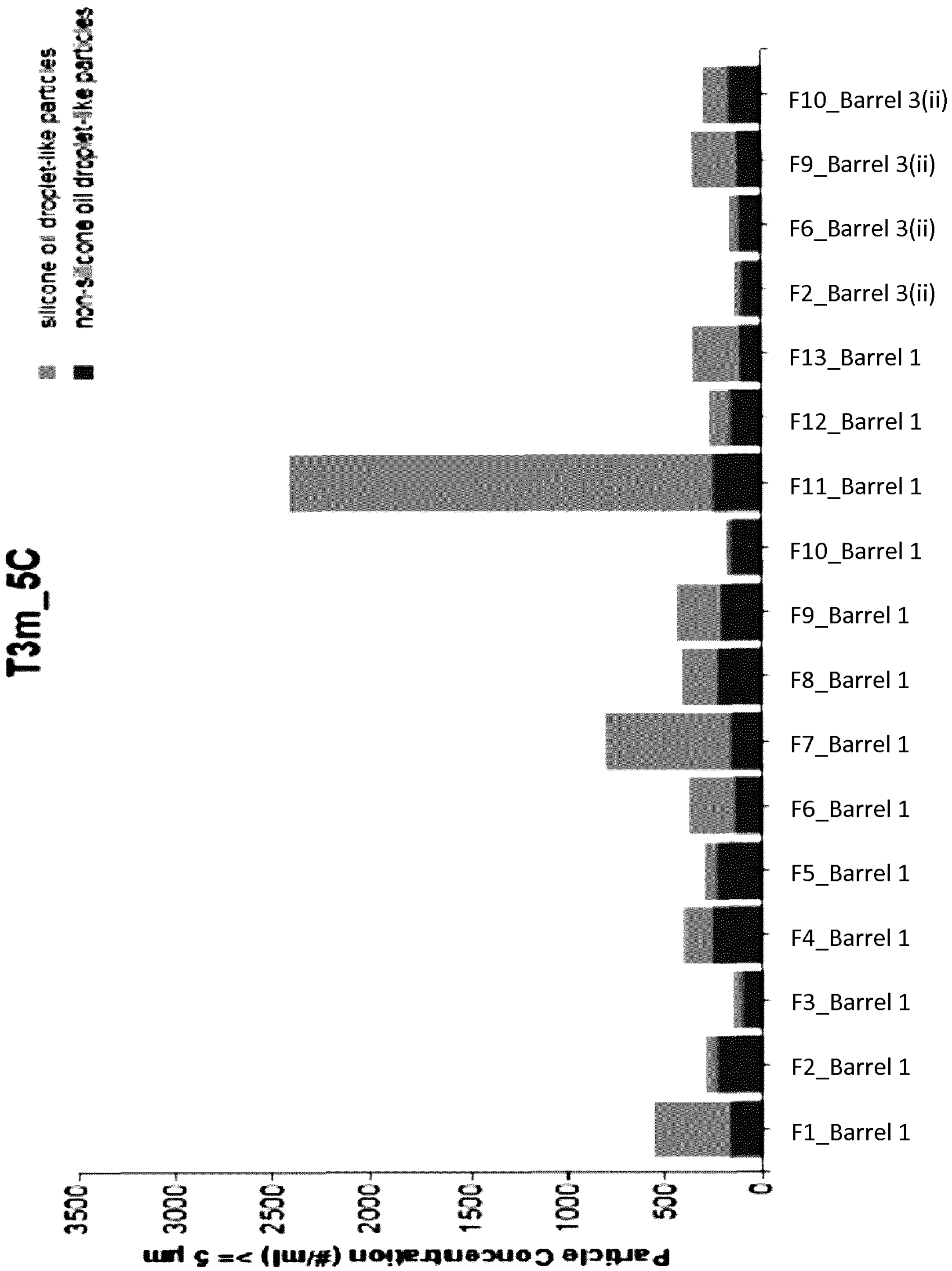
T3m_5C
silicone oil droplet-like particles
non-silicone oil droplet-like particles
Particle Concentration (#/ml) >= 5 µm
3500
3000
2500
2000
1500
1000
500
0
F1_Barrel 1
F2_Barrel 1
F3_Barrel 1
F4_Barrel 1
F5_Barrel 1
F6_Barrel 1
F7_Barrel 1
F8_Barrel 1
F9_Barrel 1
F10_Barrel 1
F11_Barrel 1
F12_Barrel 1
F13_Barrel 1
F2_Barrel 3(ii)
F6_Barrel 3(ii)
F9_Barrel 3(ii)
F10_Barrel 3(ii)

FIG. 72C

T3m_25C

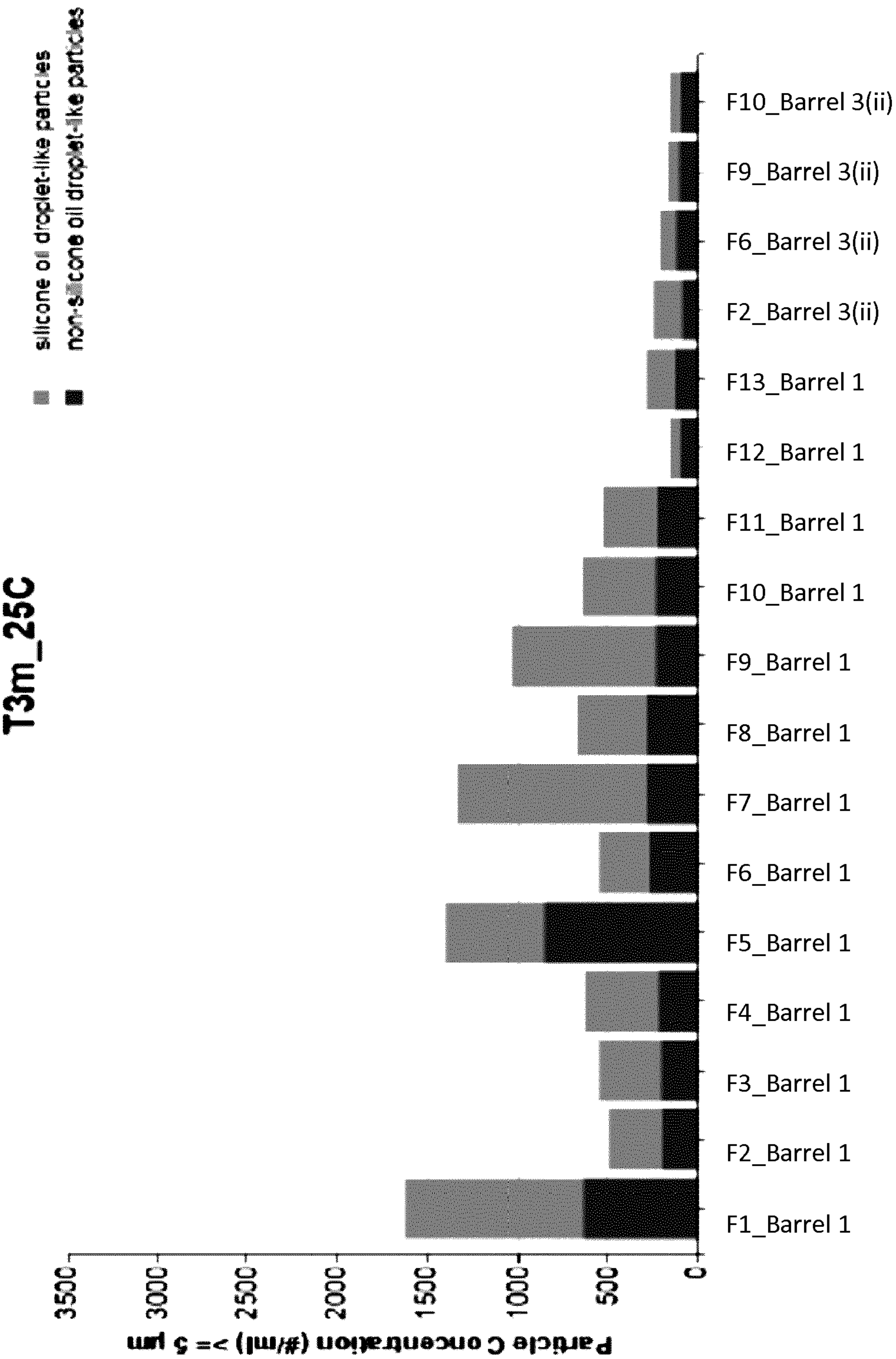

FIG. 72D
T3m_40C

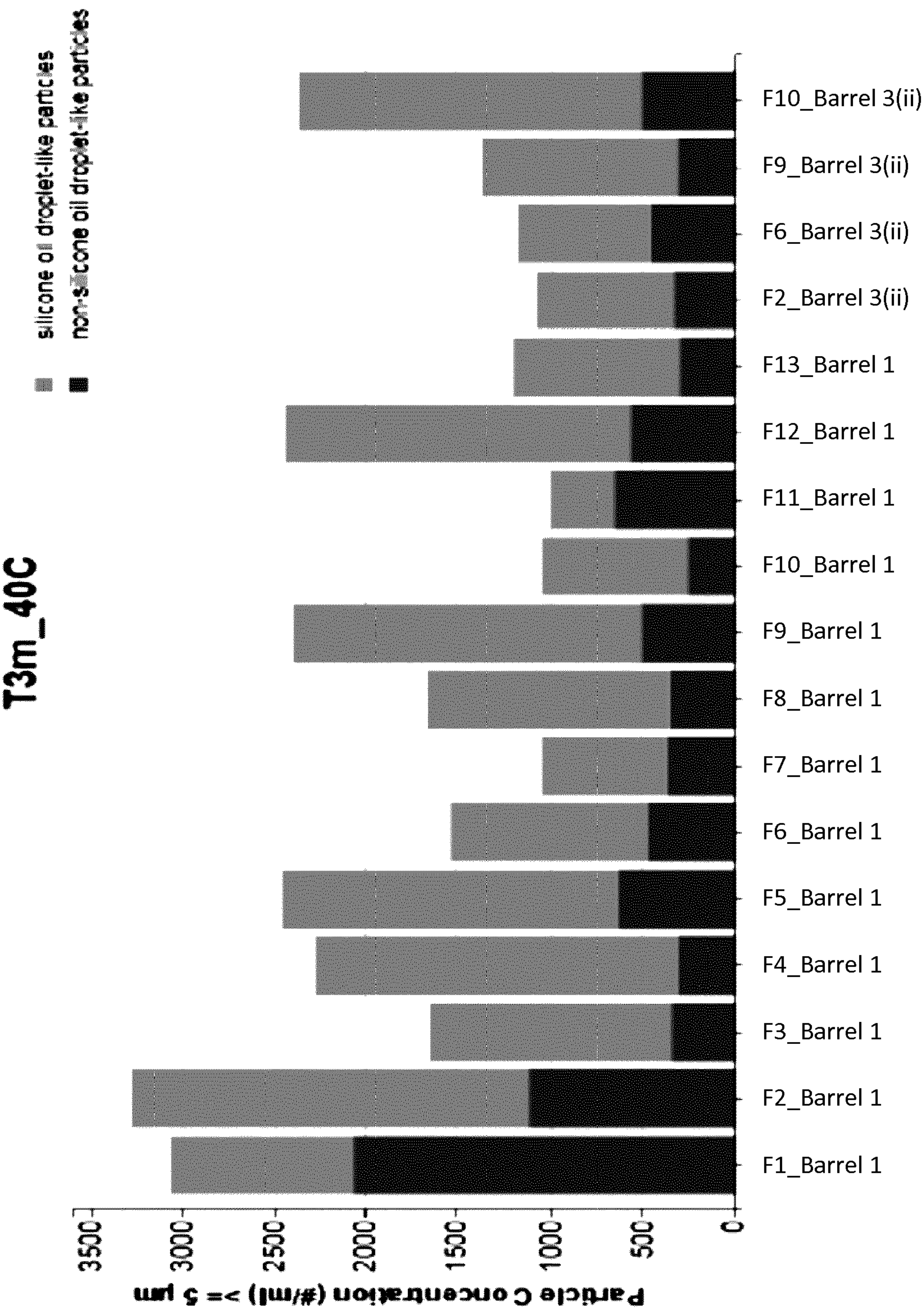

FIG. 73A

T0

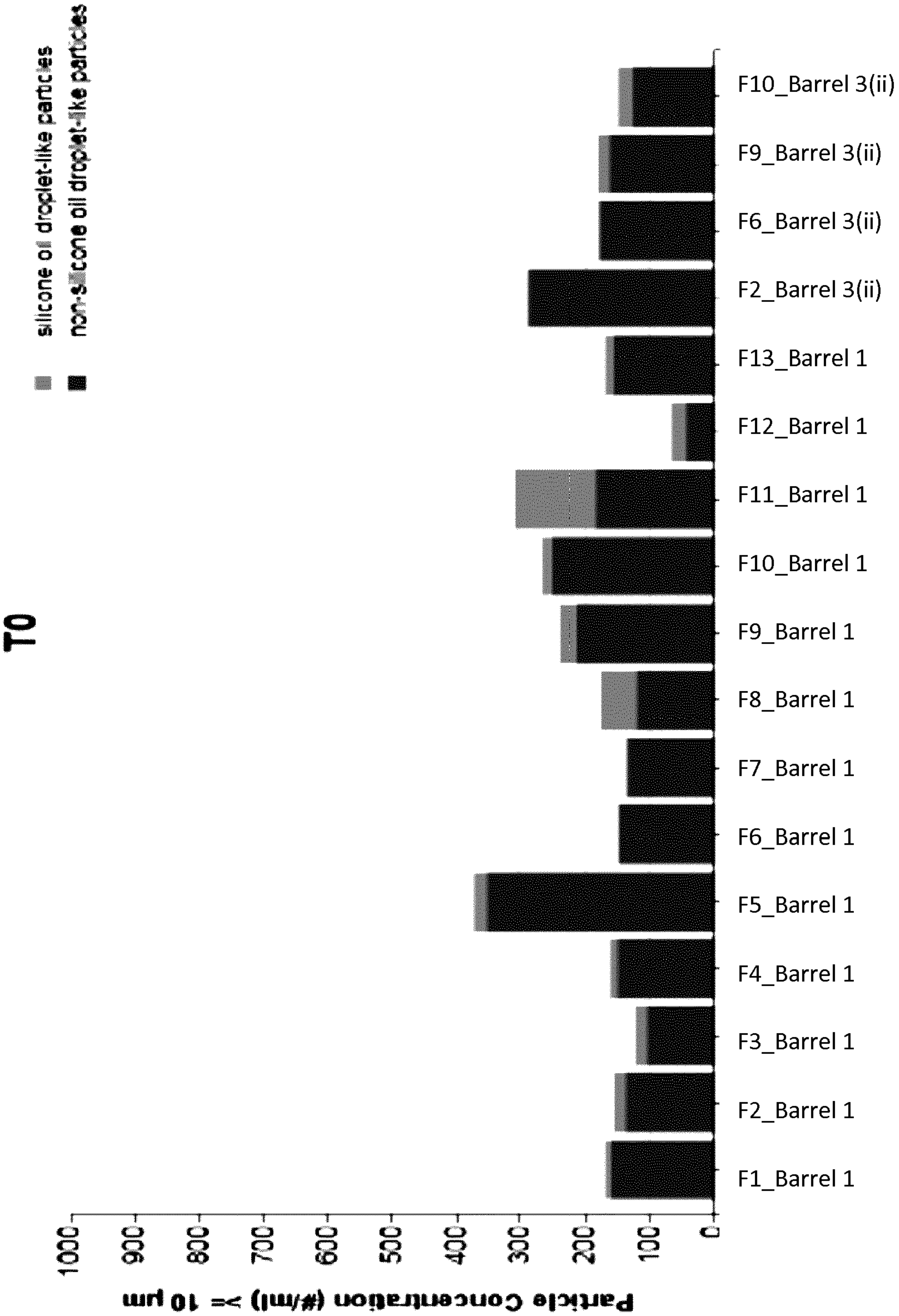
silicone oil droplet-like particles
non-silicone oil droplet-like particles
Particle Concentration (#/ml) >= 10 µm
0
100
200
300
400
500
600
700
800
900
1000
F1_Barrel 1
F2_Barrel 1
F3_Barrel 1
F4_Barrel 1
F5_Barrel 1
F6_Barrel 1
F7_Barrel 1
F8_Barrel 1
F9_Barrel 1
F10_Barrel 1
F11_Barrel 1
F12_Barrel 1
F13_Barrel 1
F2_Barrel 3(ii)
F6_Barrel 3(ii)
F9_Barrel 3(ii)
F10_Barrel 3(ii)

FIG. 73B

T3m_5C

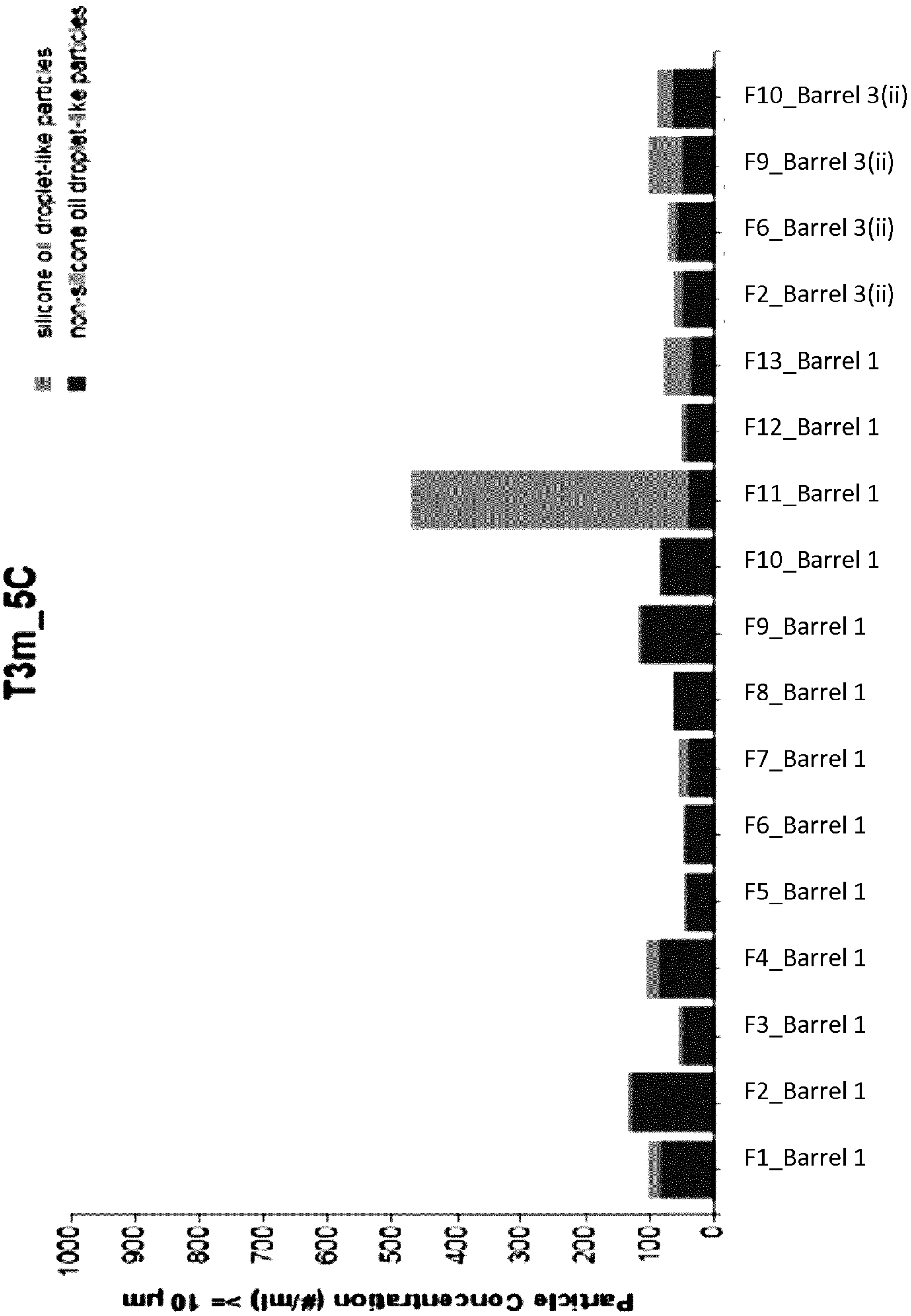

FIG. 73C

T3m_25C

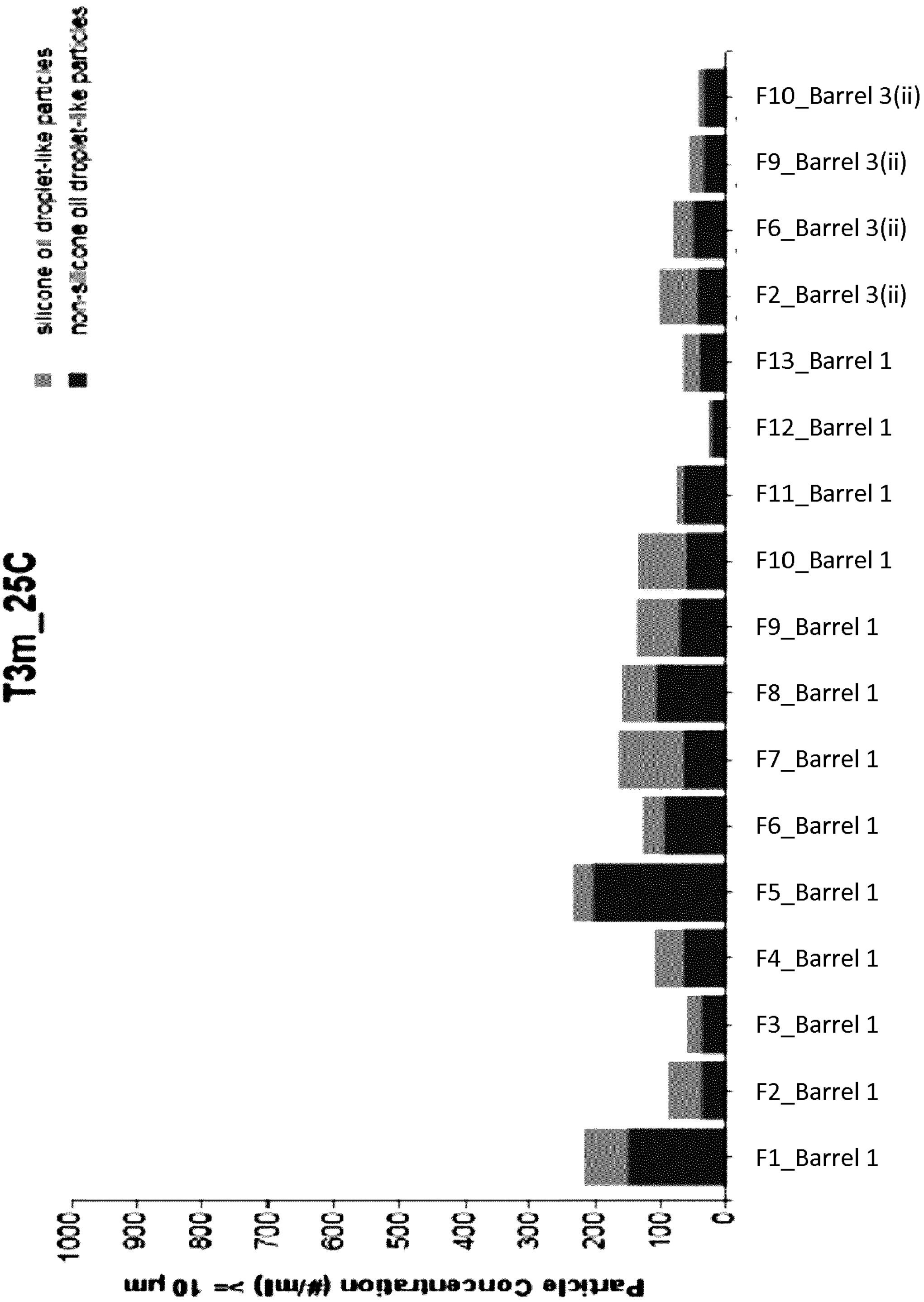

FIG.73D
T3m_40C

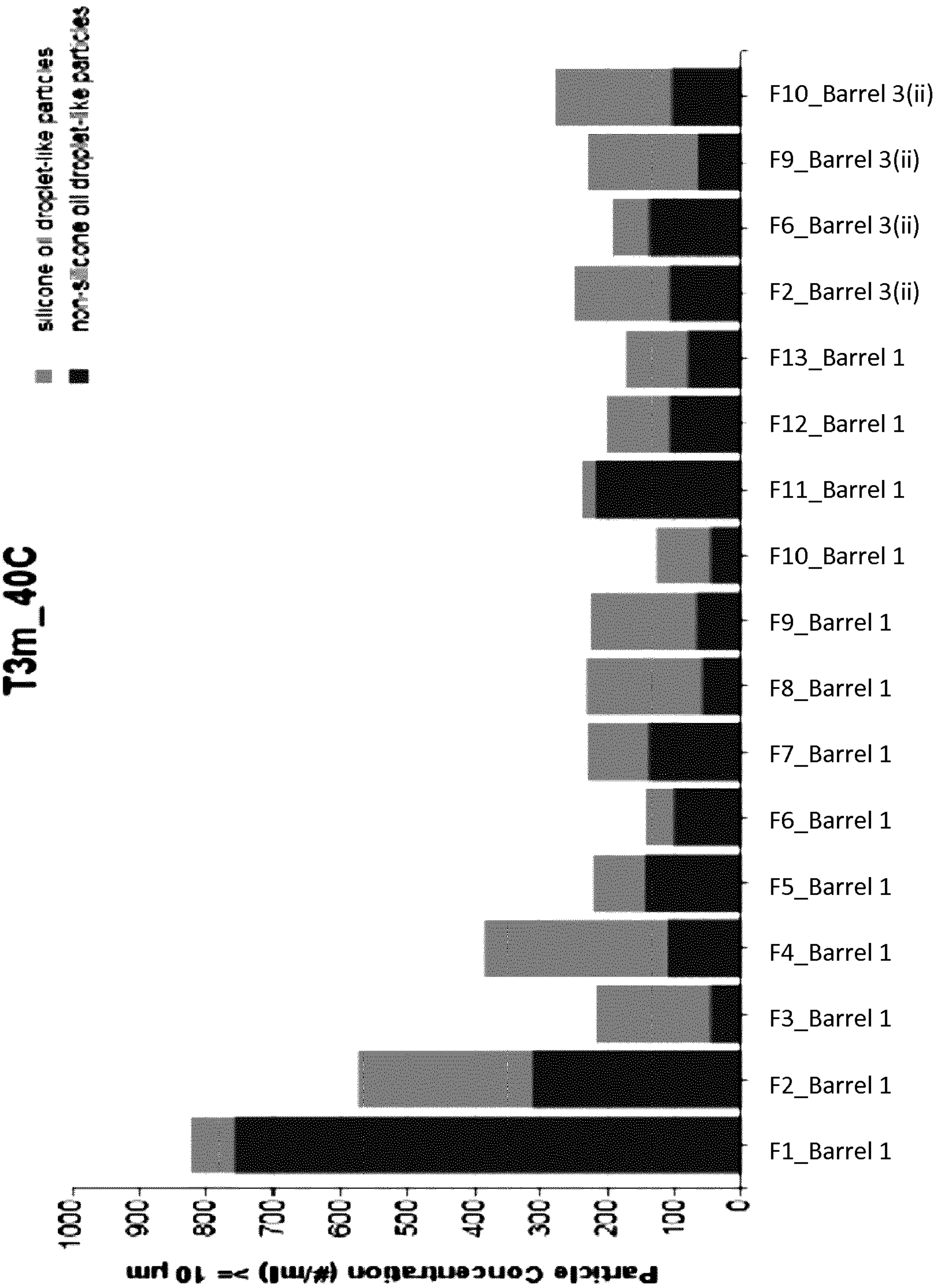

FIG. 74A

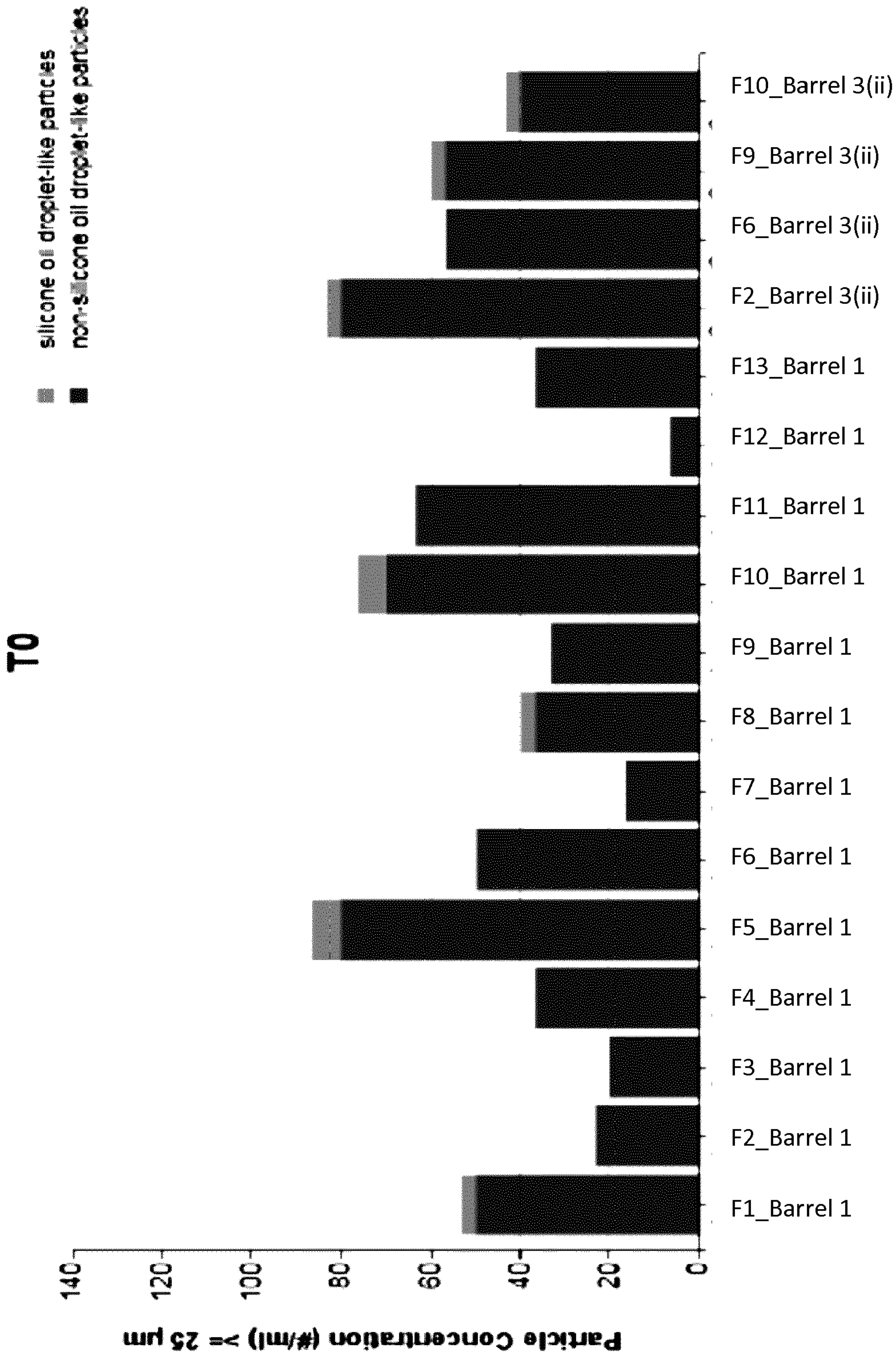
T0
silicone oil droplet-like particles
non-silicone oil droplet-like particles
Particle Concentration (#/ml) >= 25 µm
140
120
100
80
60
40
20
0
F1_Barrel 1
F2_Barrel 1
F3_Barrel 1
F4_Barrel 1
F5_Barrel 1
F6_Barrel 1
F7_Barrel 1
F8_Barrel 1
F9_Barrel 1
F10_Barrel 1
F11_Barrel 1
F12_Barrel 1
F13_Barrel 1
F2_Barrel 3(ii)
F6_Barrel 3(ii)
F9_Barrel 3(ii)
F10_Barrel 3(ii)

FIG. 74B

T3m_5C

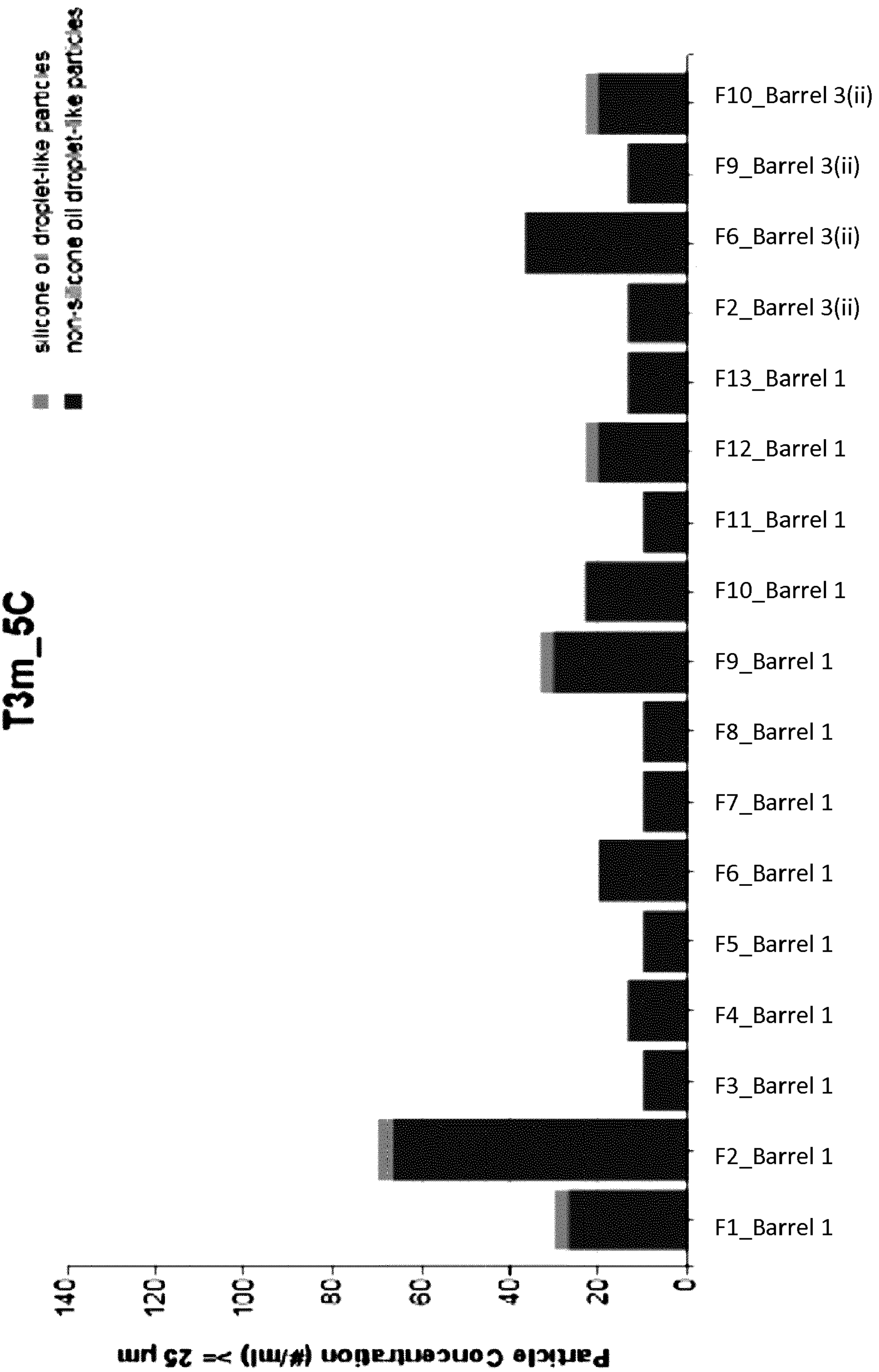

FIG.74C
T3m_25C

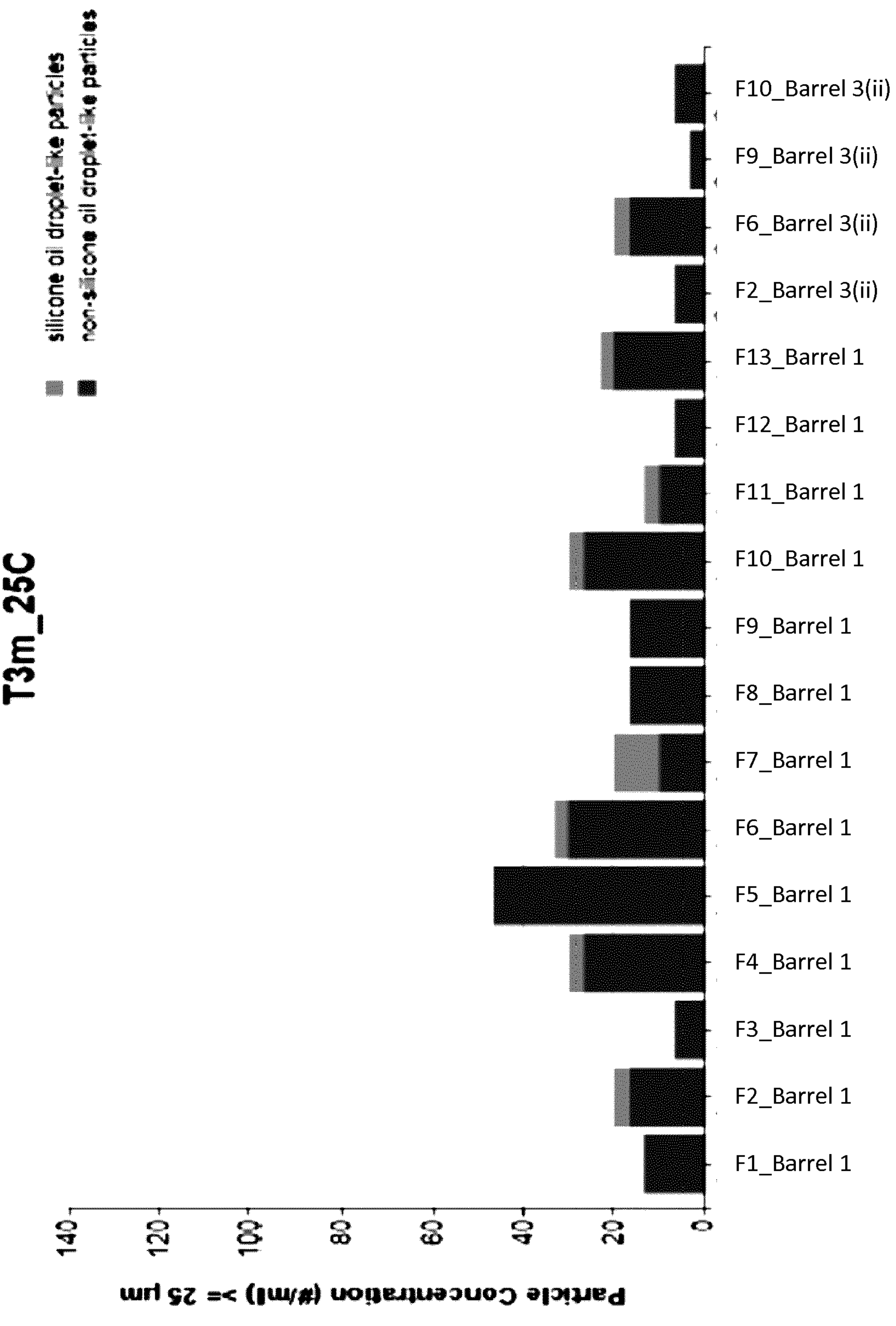

FIG. 74D

T3m_40C

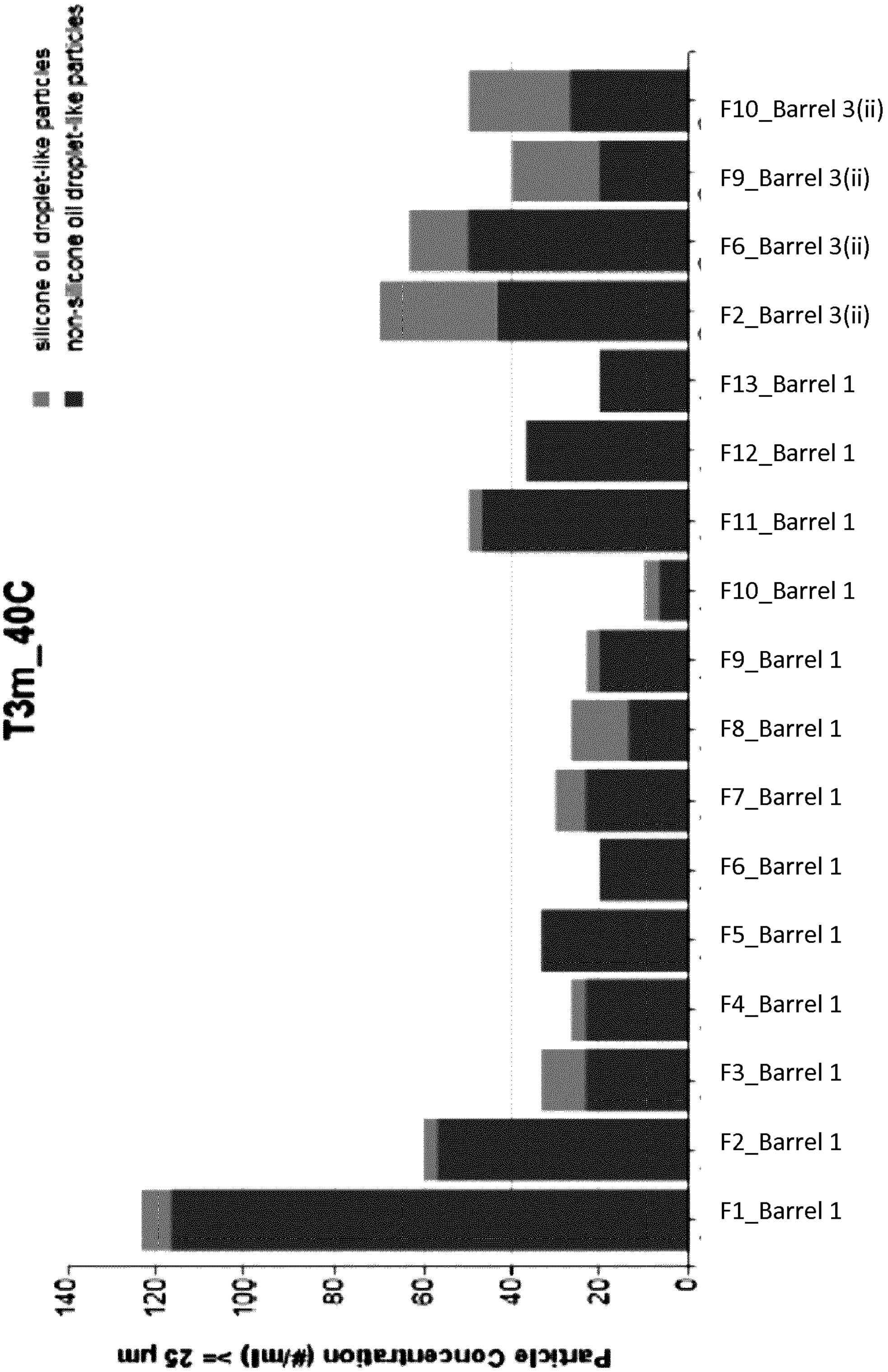

FIG. 75A

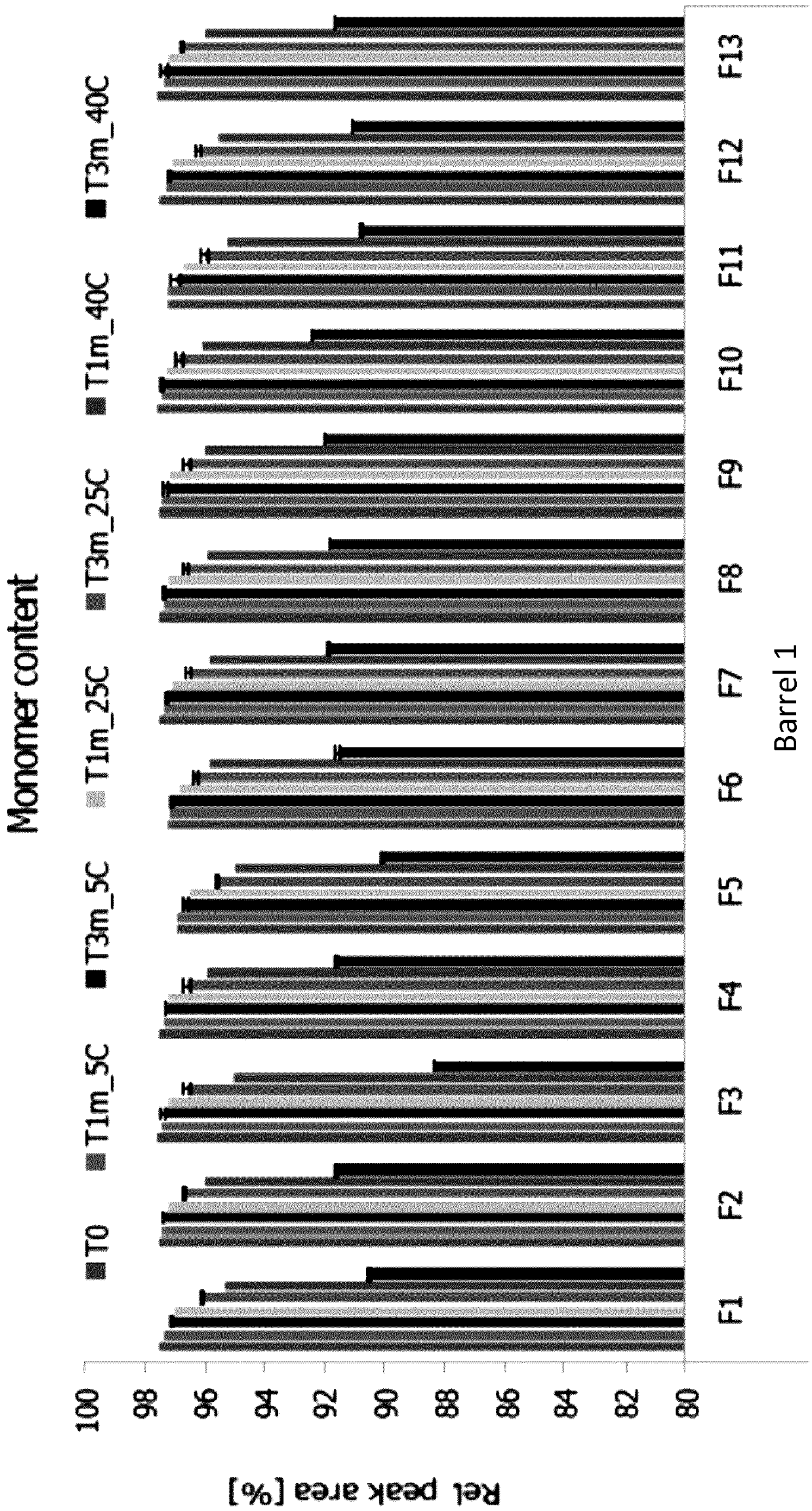
Monomer content
T0
T1m_5C
T3m_5C
T1m_25C
T3m_25C
T1m_40C
T3m_40C
Rel peak area [%]
100
98
96
94
92
90
88
86
84
82
80
F1
F2
F3
F4
F5
F6
F7
F8
F9
F10
F11
F12
F13
Barrel 1

FIG. 75B

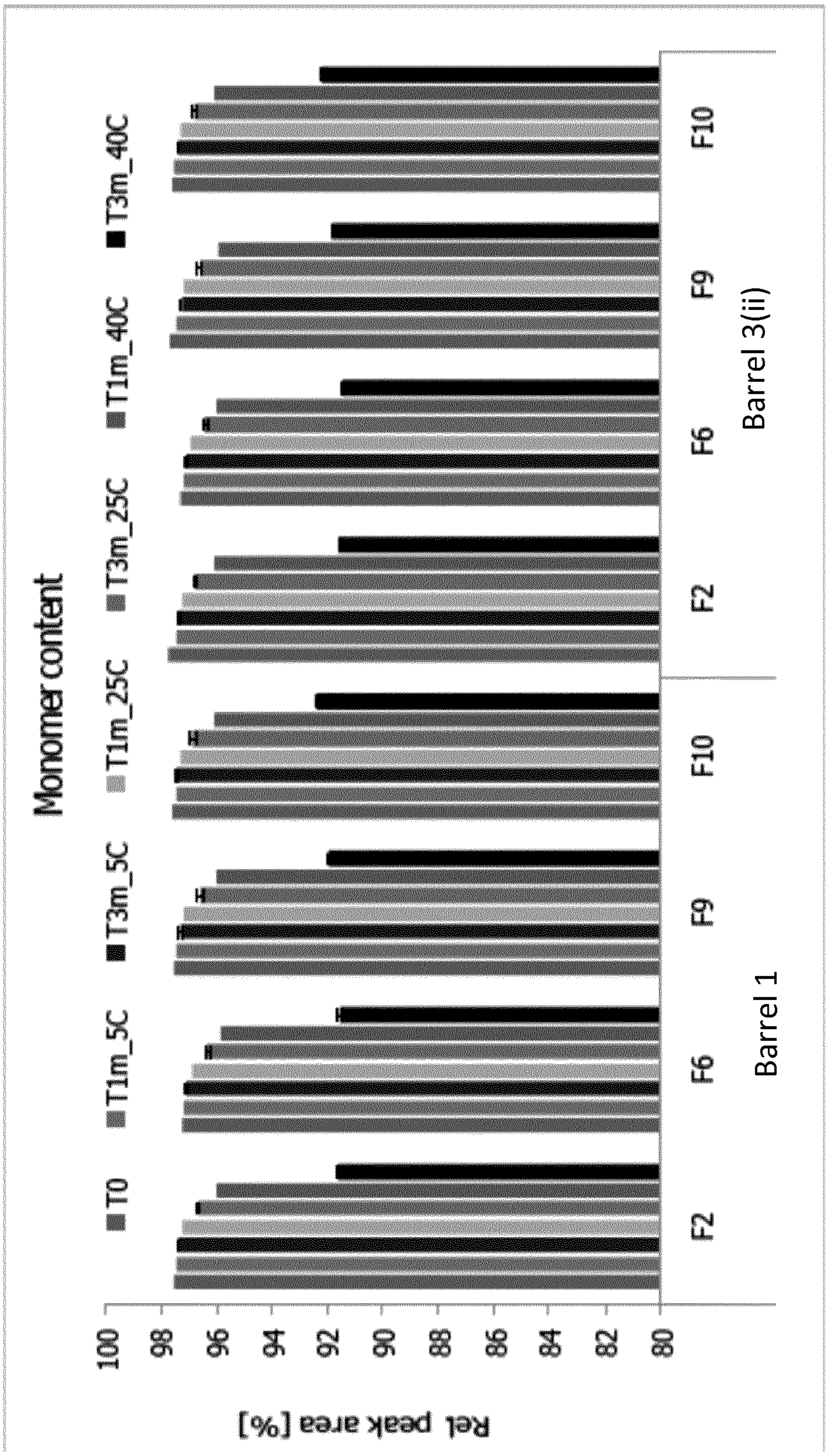
Monomer content
T0
T1m_5C
T3m_5C
T1m_25C
T3m_25C
T1m_40C
T3m_40C
Rel peak area [%]
100
98
96
94
92
90
88
86
84
82
80
F2
F6
F9
F10
F2
F6
F9
F10
Barrel 1
Barrel 3(ii)

FIG. 76A

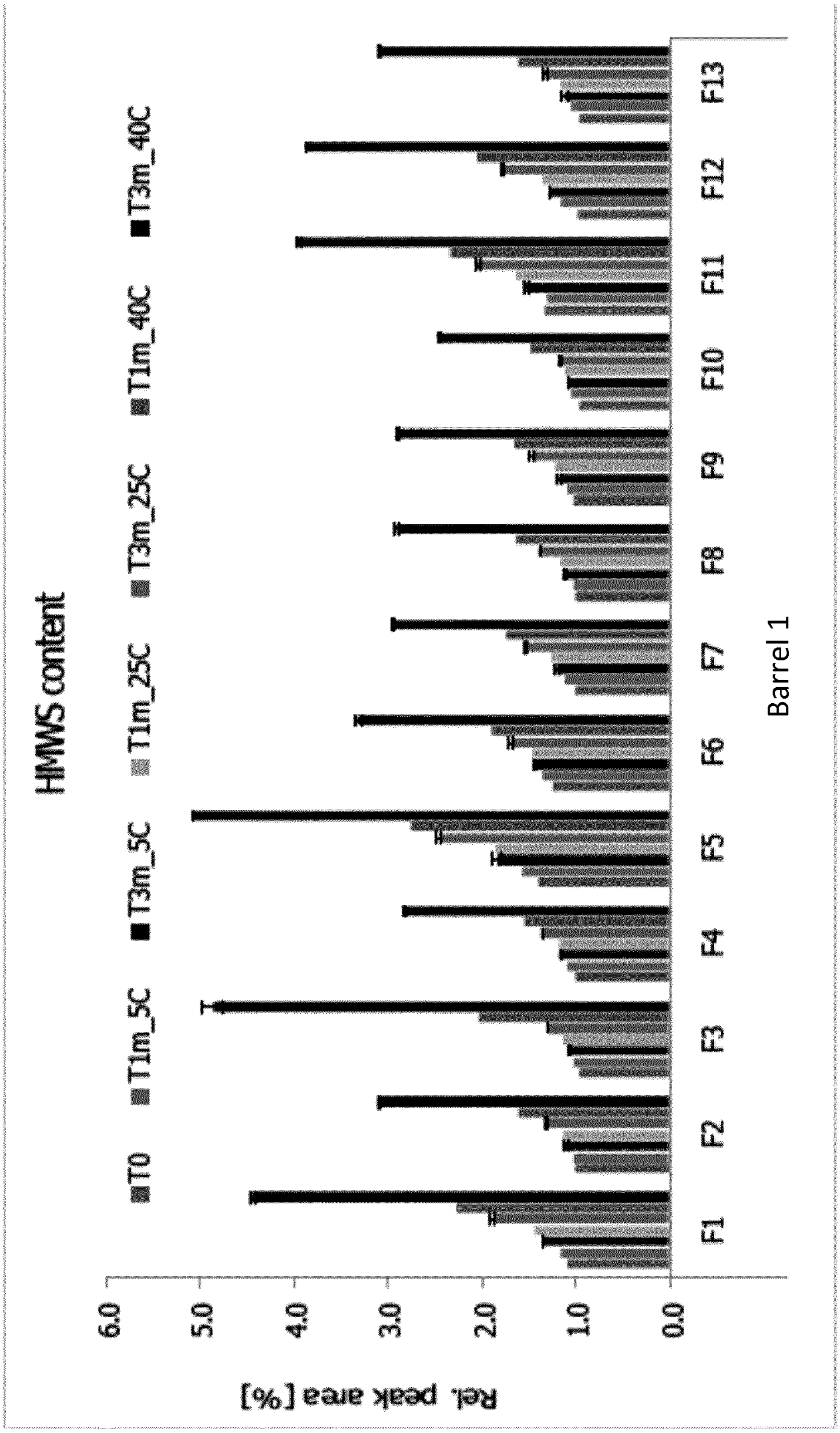
HMWS content
T0
T1m_5C
T3m_5C
T1m_25C
T3m_25C
T1m_40C
T3m_40C
Rel. peak area [%]
6.0
5.0
4.0
3.0
2.0
1.0
0.0
F1
F2
F3
F4
F5
F6
F7
F8
F9
F10
F11
F12
F13
Barrel 1

FIG. 76B

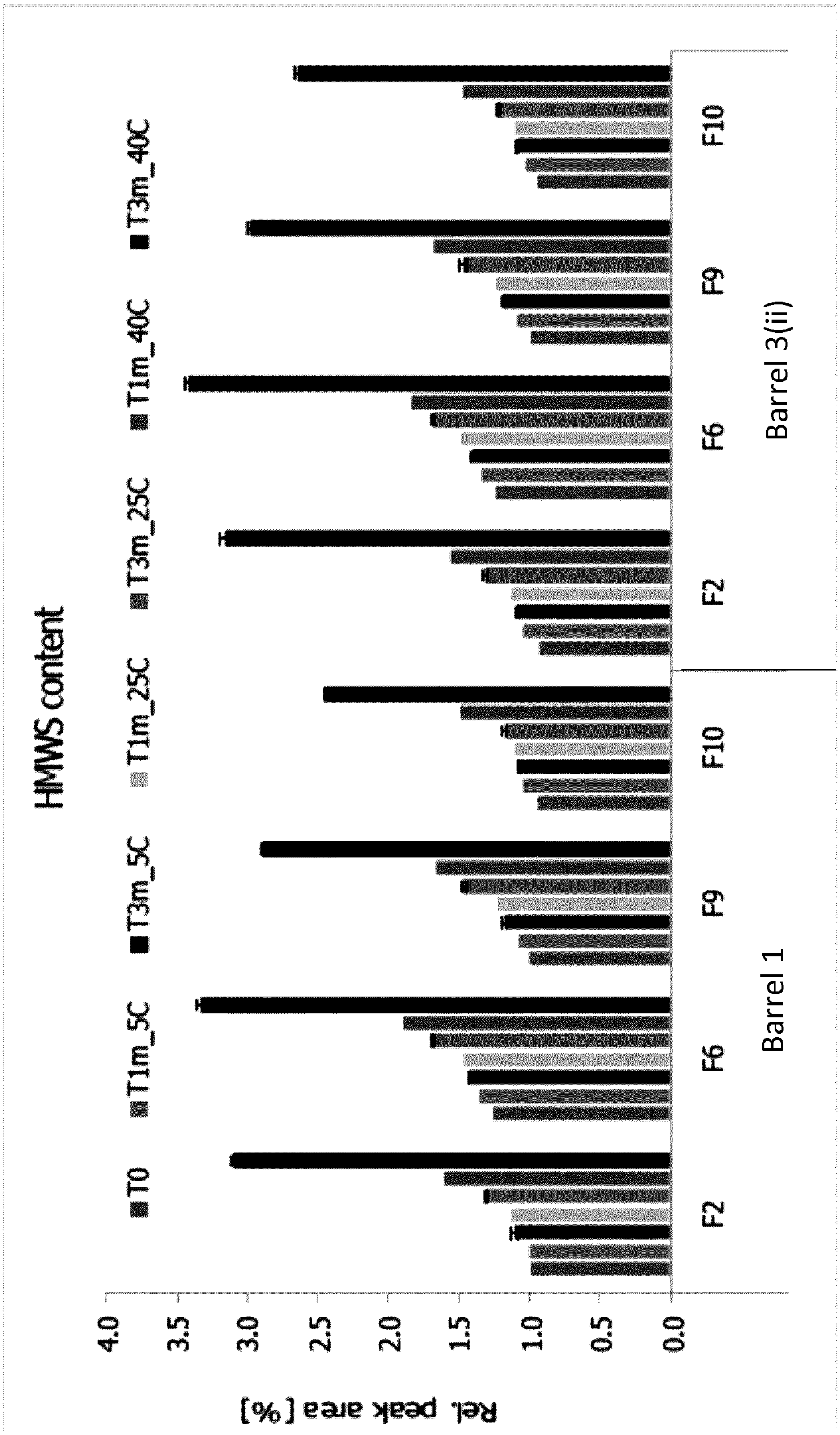
HMWS content
T0
T1m_5C
T3m_5C
T1m_25C
T3m_25C
T1m_40C
T3m_40C
Rel. peak area [%]
4.0
3.5
3.0
2.5
2.0
1.5
1.0
0.5
0.0
F2
F6
F9
F10
F2
F6
F9
F10
Barrel 1
Barrel 3(ii)

FIG. 77A

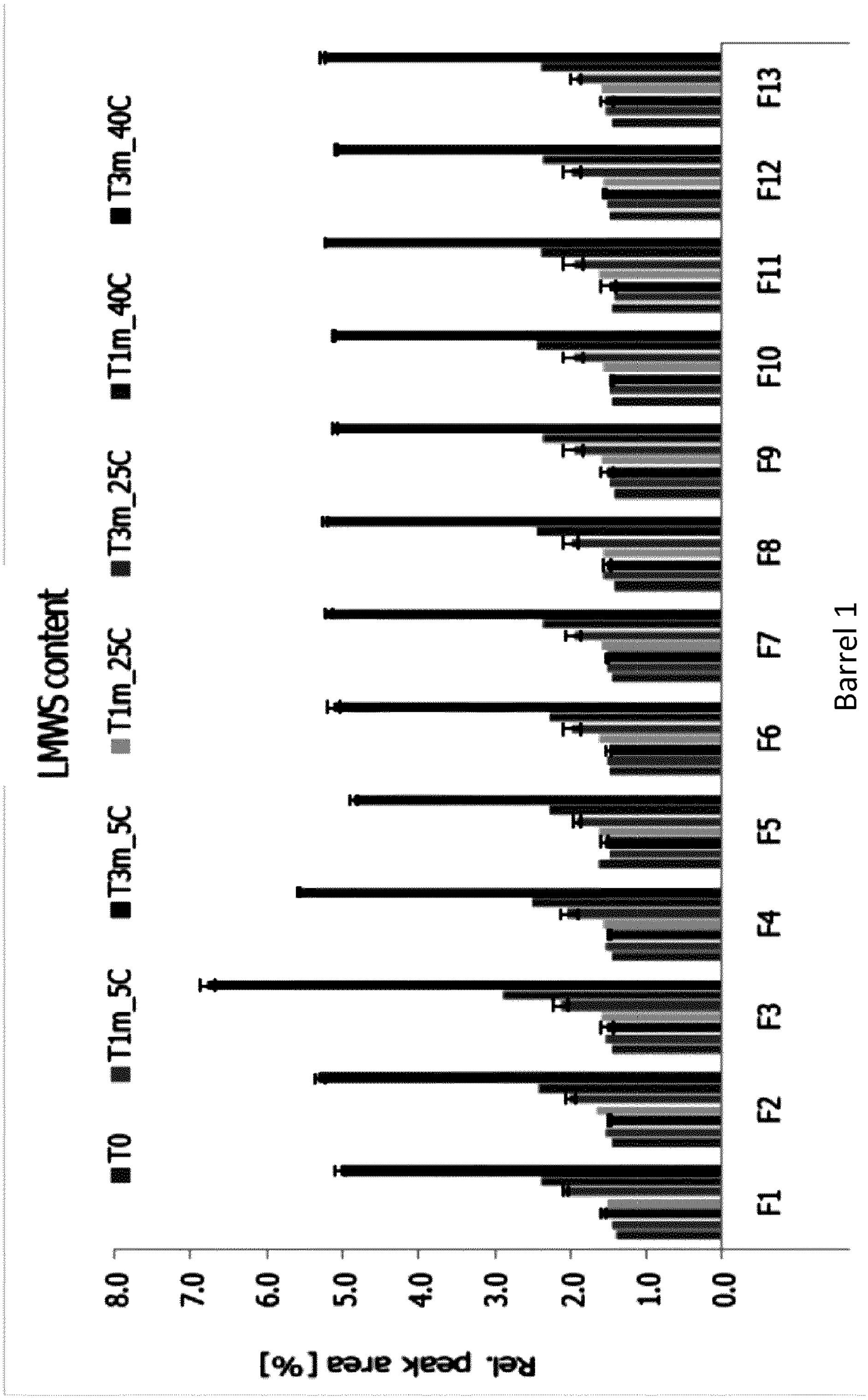
LMWS content
T0
T1m_5C
T3m_5C
T1m_25C
T3m_25C
T1m_40C
T3m_40C
Rel. peak area [%]
8.0
7.0
6.0
5.0
4.0
3.0
2.0
1.0
0.0
F1
F2
F3
F4
F5
F6
F7
F8
F9
F10
F11
F12
F13
Barrel 1

FIG. 77B

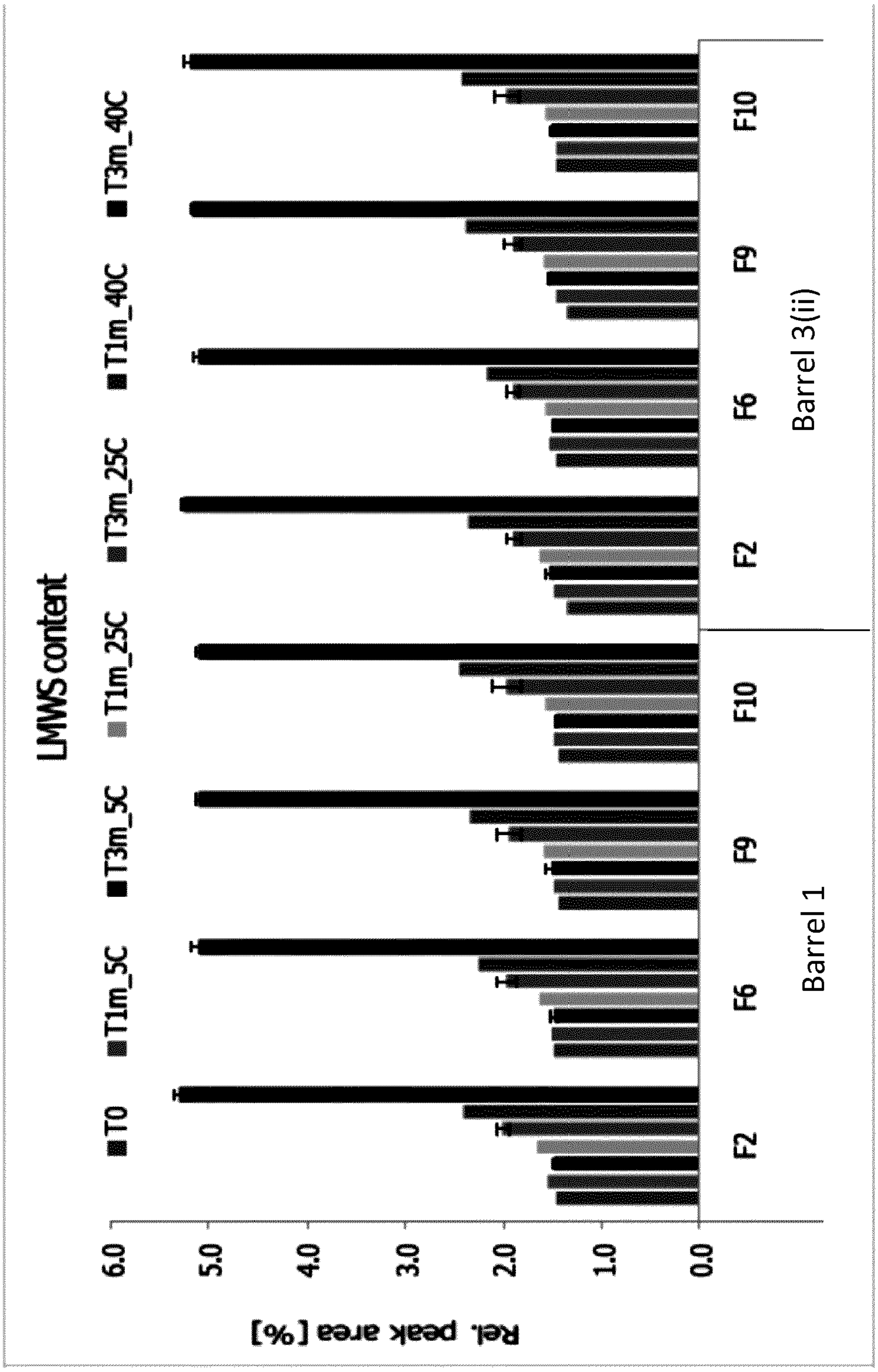
LMWS content
T0
T1m_5C
T3m_5C
T1m_25C
T3m_25C
T1m_40C
T3m_40C
Rel. peak area [%]
6.0
5.0
4.0
3.0
2.0
1.0
0.0
F2
F6
F9
F10
F2
F6
F9
F10
Barrel 1
Barrel 3(ii)

# FIG. 78A

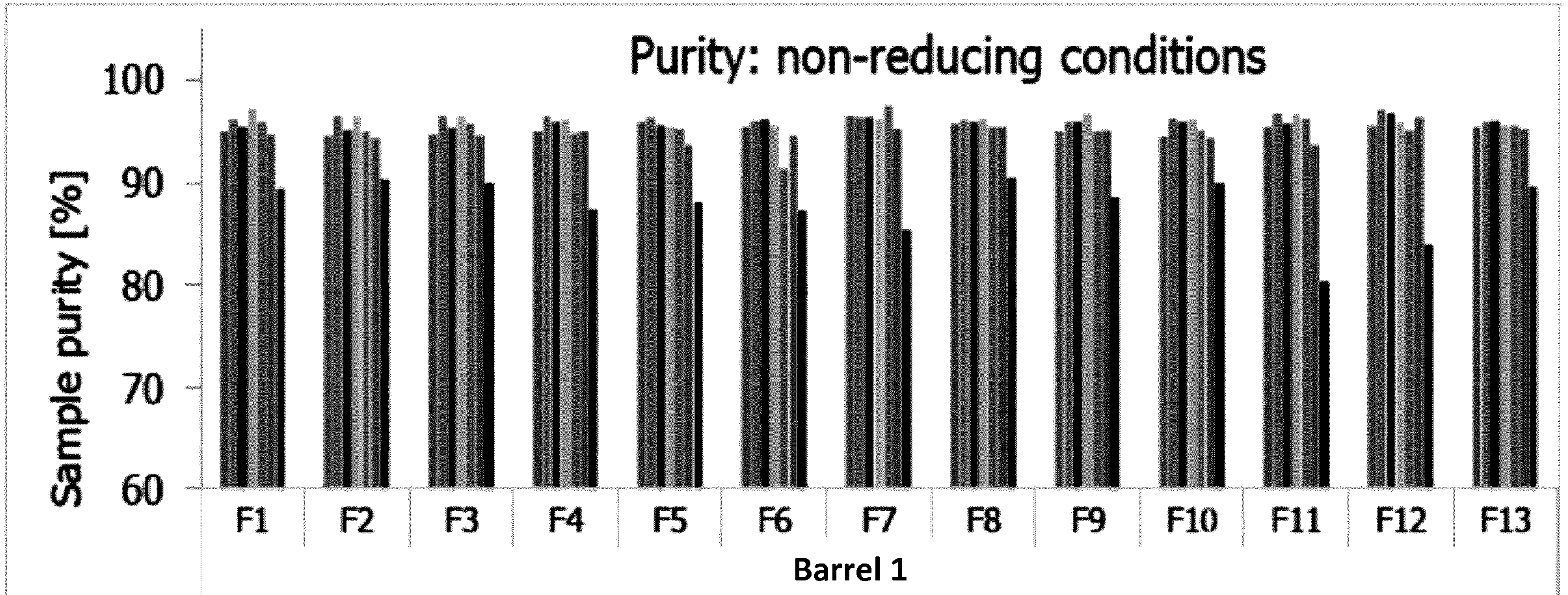


| | F1 | F2 | F3 | F4 | F5 | F6 | F7 | F8 | F9 | F10 | F11 | F12 | F13 |
|---|---|---|---|---|---|---|---|---|---|---|---|---|---|
| T0 | 95.0 | 94.6 | 94.7 | 95.0 | 95.8 | 95.5 | 96.5 | 95.7 | 95.0 | 94.4 | 95.5 | 95.6 | 95.4 |
| T1m_5C | 96.1 | 96.5 | 96.5 | 96.5 | 96.3 | 96.0 | 96.3 | 96.1 | 95.8 | 96.2 | 96.7 | 97.1 | 95.9 |
| T3m_5C | 95.4 | 95.1 | 95.3 | 95.9 | 95.6 | 96.1 | 96.3 | 95.9 | 95.9 | 95.8 | 95.7 | 96.7 | 96.0 |
| T1m_25C | 97.3 | 96.5 | 96.5 | 96.1 | 95.5 | 95.6 | 96.1 | 96.2 | 96.7 | 96.1 | 96.6 | 95.9 | 95.6 |
| T3m_25C | 95.8 | 95.0 | 95.7 | 94.8 | 95.2 | 91.4 | 97.5 | 95.4 | 94.9 | 95.1 | 96.2 | 95.1 | 95.6 |
| T1m_40C | 94.7 | 94.3 | 94.6 | 94.9 | 93.7 | 94.5 | 95.2 | 95.4 | 95.1 | 94.3 | 93.7 | 96.3 | 95.2 |
| T3m_40C | 89.5 | 90.3 | 90.0 | 87.4 | 88.0 | 87.3 | 85.4 | 90.5 | 88.6 | 89.9 | 80.3 | 83.9 | 89.6 |

# FIG. 78B

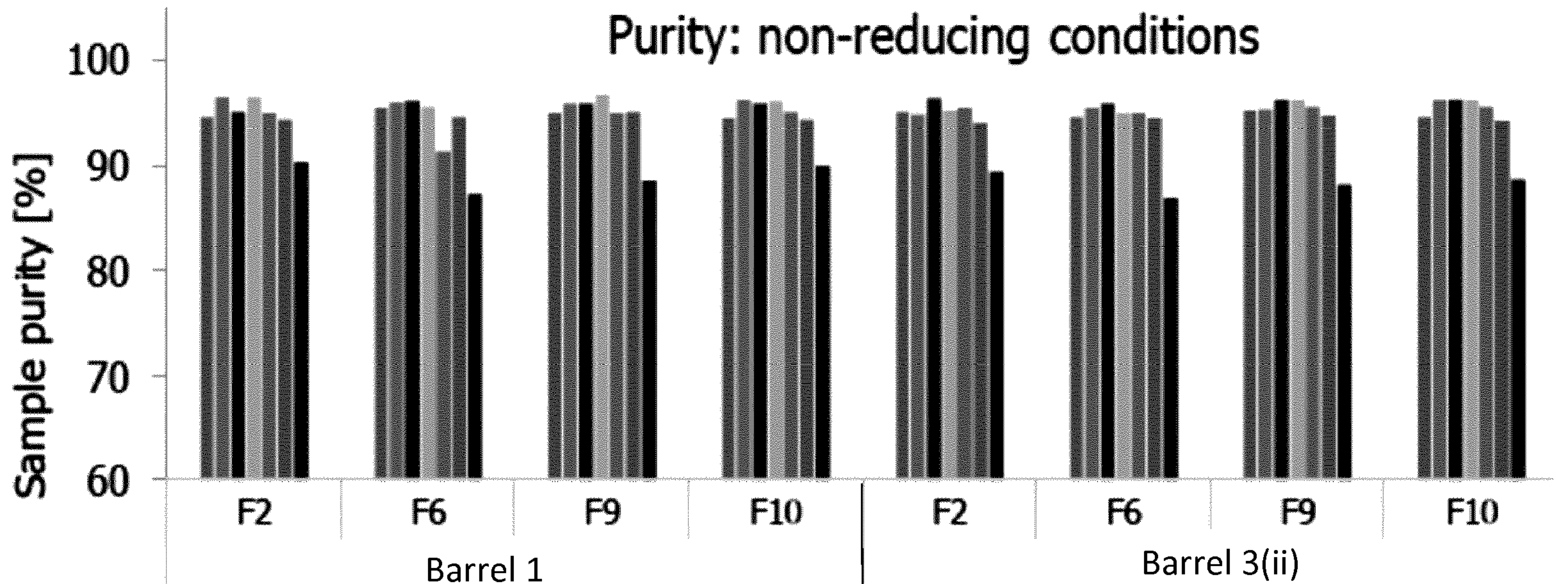


| | Barrel 1 F2 | Barrel 1 F6 | Barrel 1 F9 | Barrel 1 F10 | Barrel 3(ii) F2 | Barrel 3(ii) F6 | Barrel 3(ii) F9 | Barrel 3(ii) F10 |
|---|---|---|---|---|---|---|---|---|
| T0 | 94.6 | 95.5 | 95.0 | 94.4 | 95.1 | 94.5 | 95.2 | 94.6 |
| T1m_5C | 96.5 | 96.0 | 95.8 | 96.2 | 94.8 | 95.4 | 95.3 | 96.2 |
| T3m_5C | 95.1 | 96.1 | 95.9 | 95.8 | 96.3 | 95.8 | 96.2 | 96.2 |
| T1m_25C | 96.5 | 95.6 | 96.7 | 96.1 | 95.2 | 95.0 | 96.2 | 96.2 |
| T3m_25C | 95.0 | 91.4 | 94.9 | 95.1 | 95.5 | 94.9 | 95.6 | 95.6 |
| T1m_40C | 94.3 | 94.5 | 95.1 | 94.3 | 94.0 | 94.4 | 94.7 | 94.2 |
| T3m_40C | 90.3 | 87.3 | 88.6 | 89.9 | 89.5 | 86.9 | 88.2 | 88.7 |

FIG. 79A

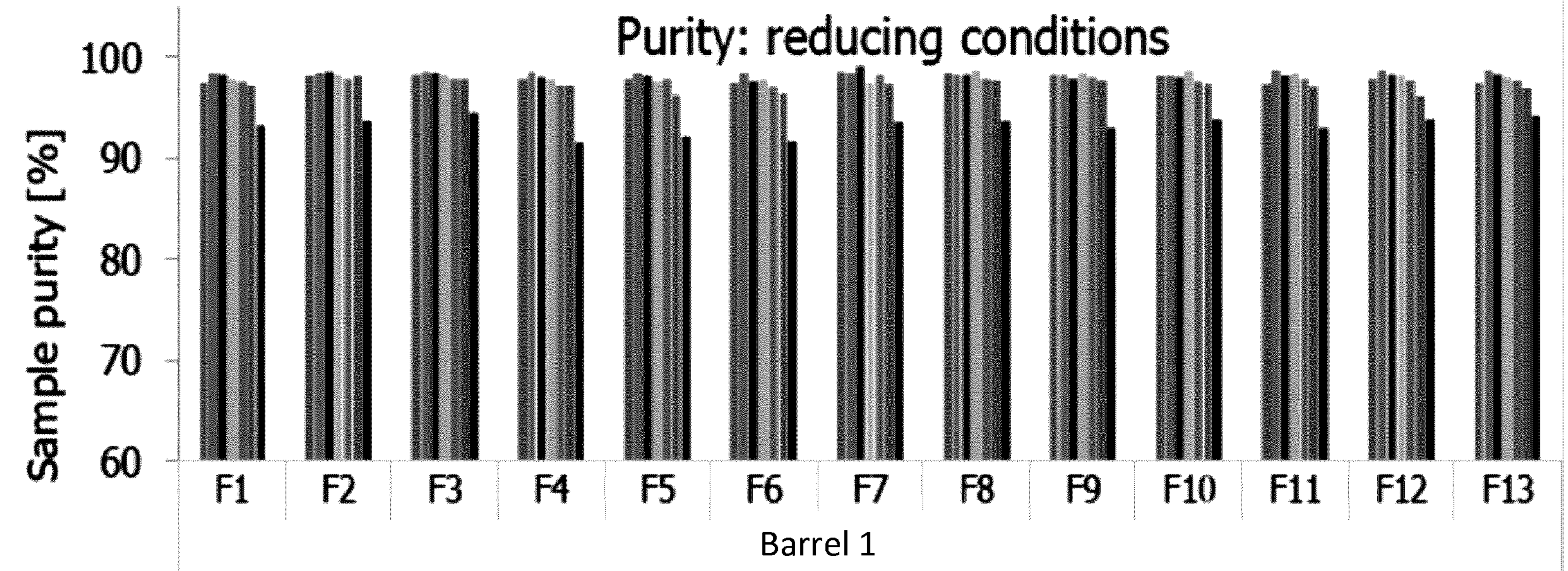


| | F1 | F2 | F3 | F4 | F5 | F6 | F7 | F8 | F9 | F10 | F11 | F12 | F13 |
|---|---|---|---|---|---|---|---|---|---|---|---|---|---|
| T0 | 97.6 | 98.2 | 98.4 | 98.0 | 97.9 | 97.6 | 98.6 | 98.5 | 98.4 | 98.2 | 97.4 | 98.0 | 97.6 |
| T1m_5C | 98.5 | 98.5 | 98.6 | 98.6 | 98.5 | 98.5 | 98.5 | 98.3 | 98.4 | 98.2 | 98.7 | 98.7 | 98.7 |
| T3m_5C | 98.4 | 98.6 | 98.5 | 98.1 | 98.2 | 97.7 | 99.2 | 98.4 | 97.9 | 98.1 | 98.2 | 98.4 | 98.4 |
| T1m_25C | 97.9 | 98.3 | 98.4 | 98.0 | 97.7 | 97.9 | 97.6 | 98.7 | 98.5 | 98.7 | 98.5 | 98.3 | 98.1 |
| T3m_25C | 97.7 | 97.9 | 98.0 | 97.3 | 97.9 | 97.1 | 98.3 | 98.0 | 98.1 | 97.7 | 97.9 | 97.8 | 97.8 |
| T1m_40C | 97.2 | 98.2 | 98.0 | 97.2 | 96.3 | 96.5 | 97.4 | 97.8 | 97.8 | 97.4 | 97.1 | 96.2 | 97.0 |
| T3m_40C | 93.3 | 93.7 | 94.5 | 91.5 | 92.2 | 91.6 | 93.6 | 93.7 | 93.0 | 93.9 | 93.0 | 93.8 | 94.3 |

# FIG. 79B

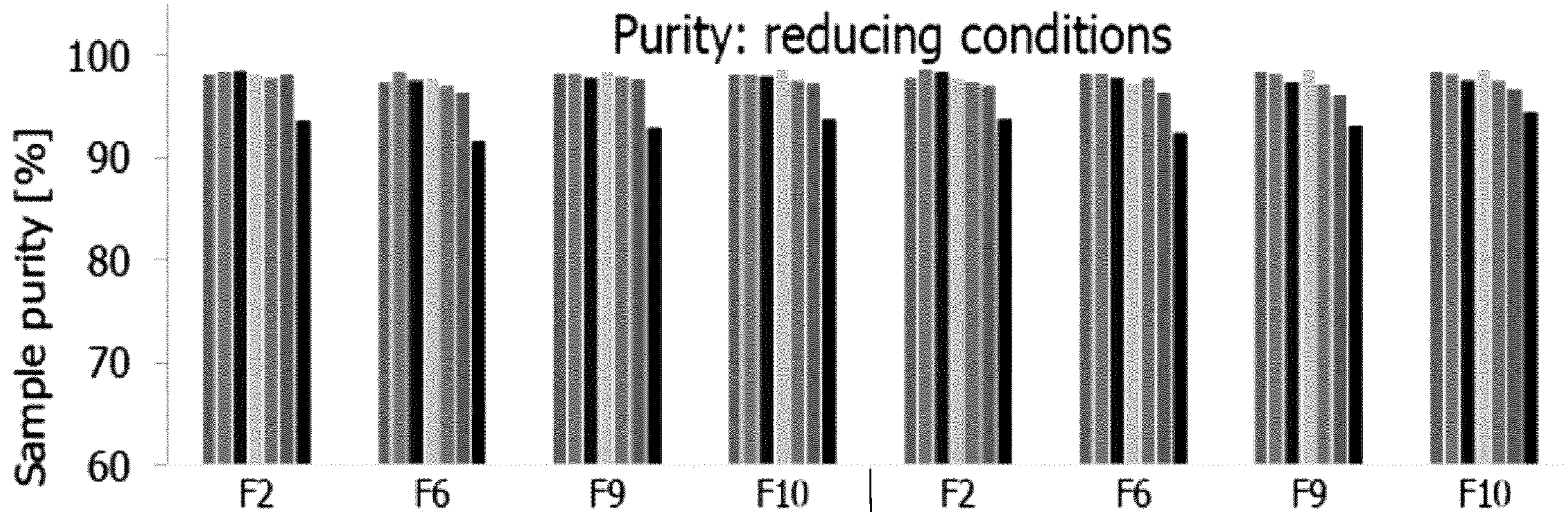


| | Barrel 1 F2 | Barrel 1 F6 | Barrel 1 F9 | Barrel 1 F10 | Barrel 3(ii) F2 | Barrel 3(ii) F6 | Barrel 3(ii) F9 | Barrel 3(ii) F10 |
|---|---|---|---|---|---|---|---|---|
| T0 | 98.2 | 97.6 | 98.4 | 98.2 | 98.0 | 98.4 | 98.5 | 98.5 |
| T1m_5C | 98.5 | 98.5 | 98.4 | 98.2 | 98.7 | 98.4 | 98.3 | 98.4 |
| T3m_5C | 98.6 | 97.7 | 97.9 | 98.1 | 98.5 | 97.9 | 97.5 | 97.7 |
| T1m_25C | 98.3 | 97.9 | 98.5 | 98.7 | 98.0 | 97.4 | 98.7 | 98.8 |
| T3m_25C | 97.9 | 97.1 | 98.1 | 97.7 | 97.6 | 98.0 | 97.2 | 97.7 |
| T1m_40C | 98.2 | 96.5 | 97.8 | 97.4 | 97.1 | 96.5 | 96.1 | 96.8 |
| T3m_40C | 93.7 | 91.6 | 93.0 | 93.9 | 93.9 | 92.5 | 93.2 | 94.5 |

FIG. 80A

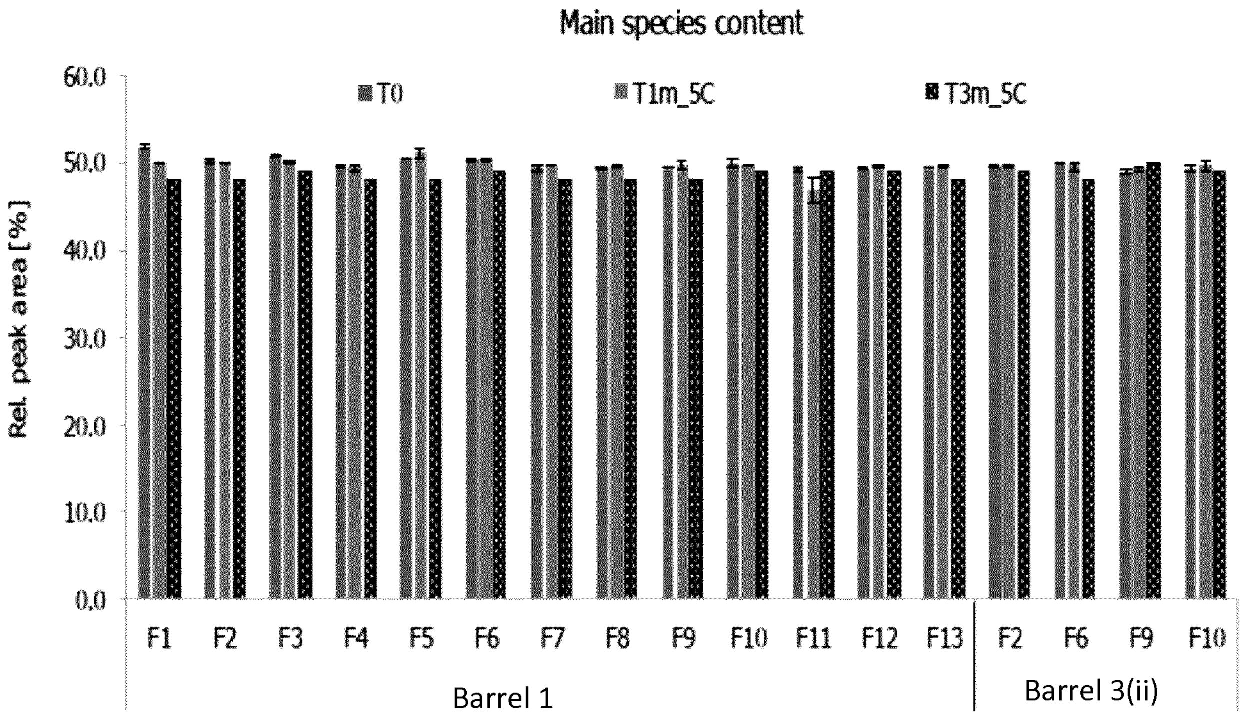
Main species content
T0
T1m_5C
T3m_5C
Rel. peak area [%]
60.0
50.0
40.0
30.0
20.0
10.0
0.0
F1
F2
F3
F4
F5
F6
F7
F8
F9
F10
F11
F12
F13
F2
F6
F9
F10
Barrel 1
Barrel 3(ii)

# FIG. 80B

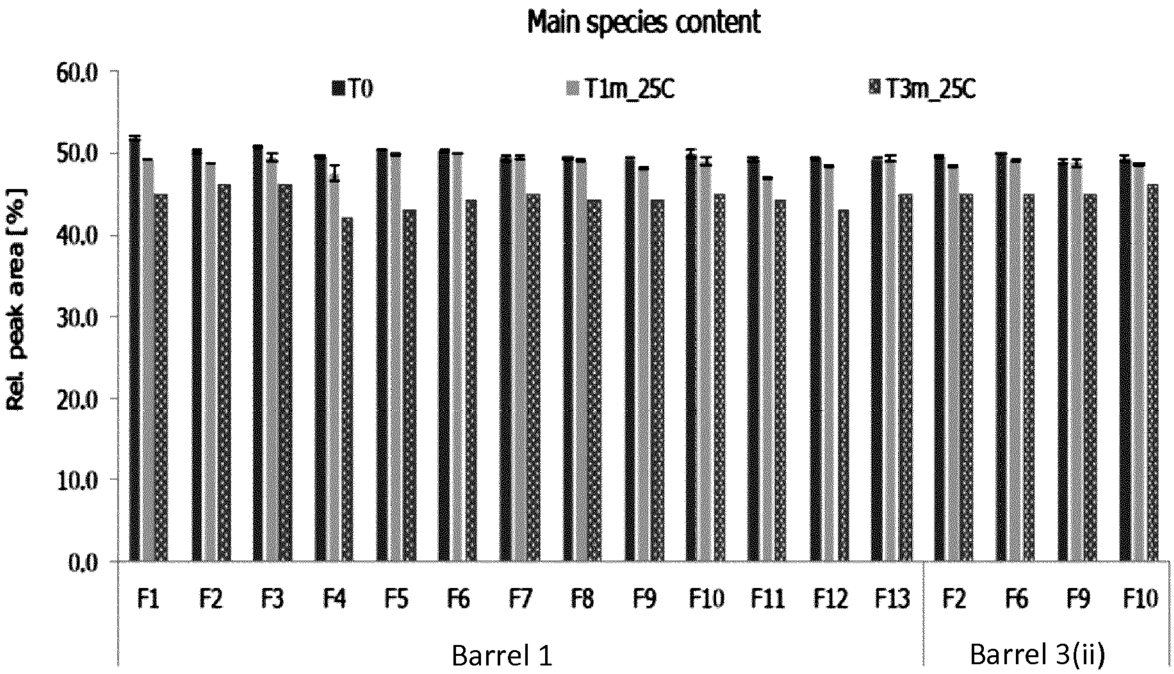


# FIG. 80C

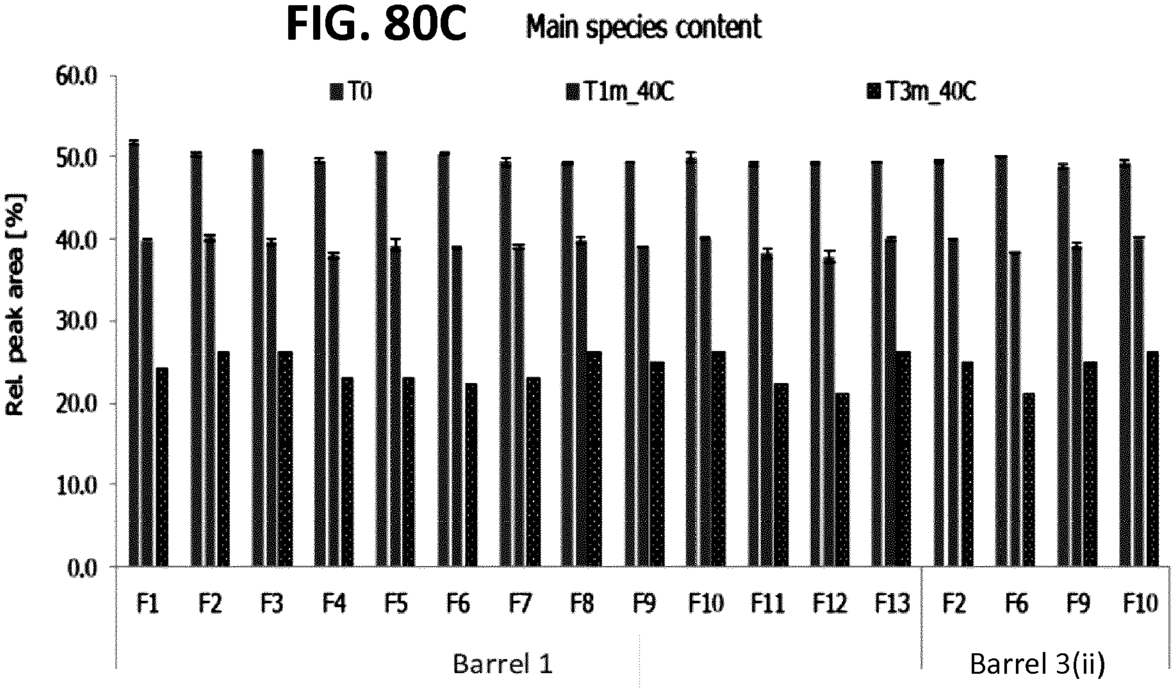

FIG. 81A

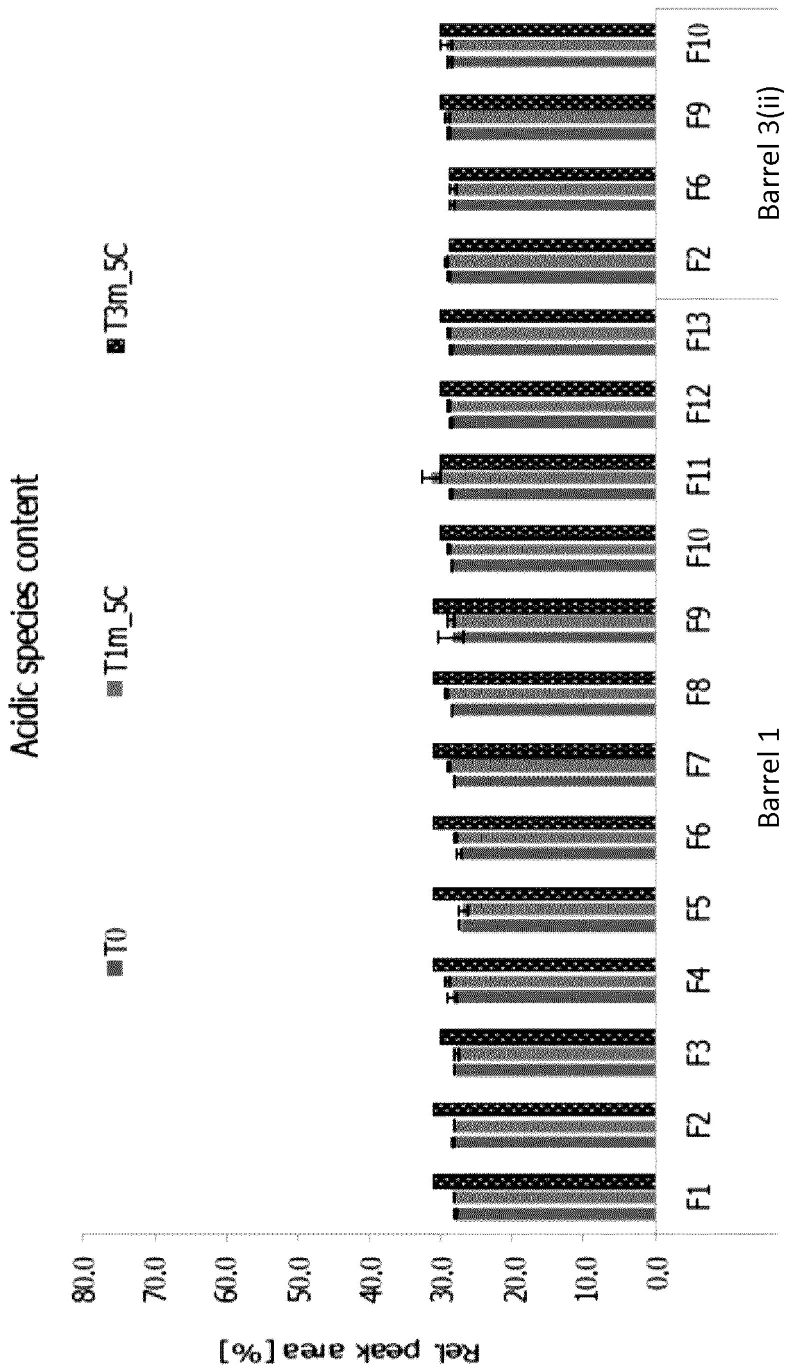
Acidic species content
T0
T1m_5C
T3m_5C
Rel. peak area [%]
80.0
70.0
60.0
50.0
40.0
30.0
20.0
10.0
0.0
F1
F2
F3
F4
F5
F6
F7
F8
F9
F10
F11
F12
F13
F2
F6
F9
F10
Barrel 1
Barrel 3(ii)

# FIG. 81B

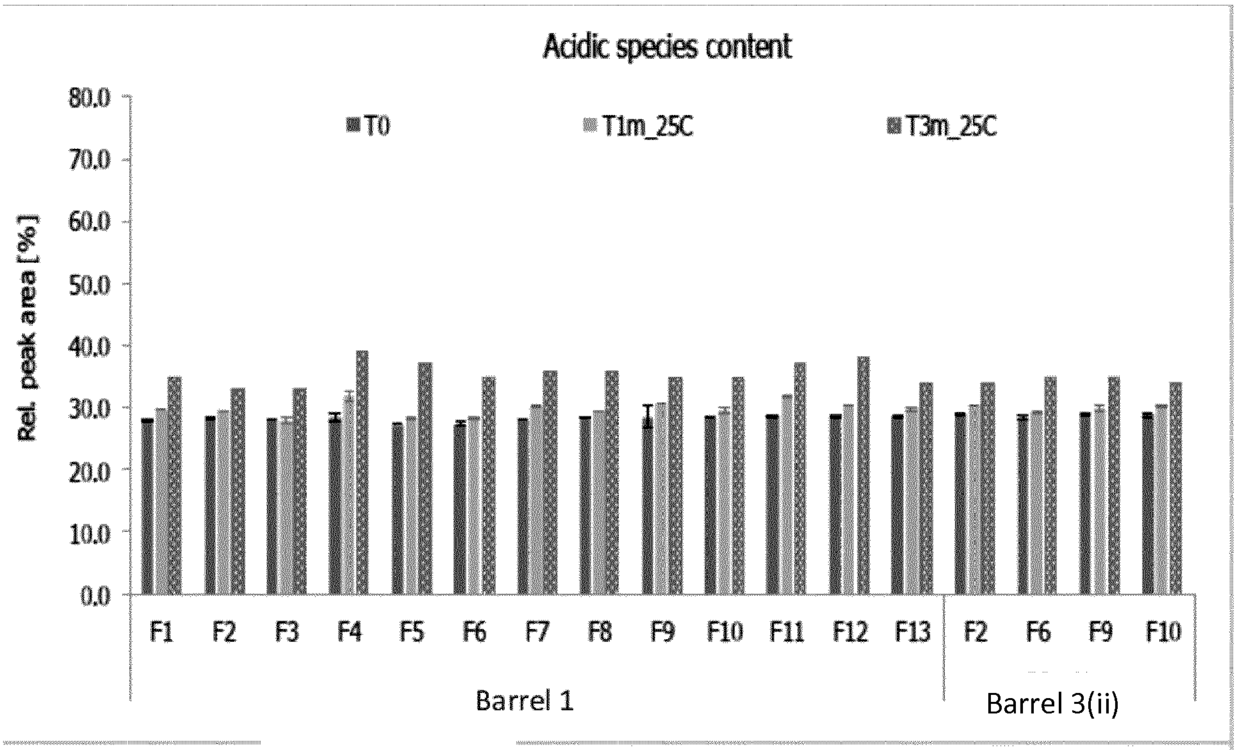


# FIG. 81C

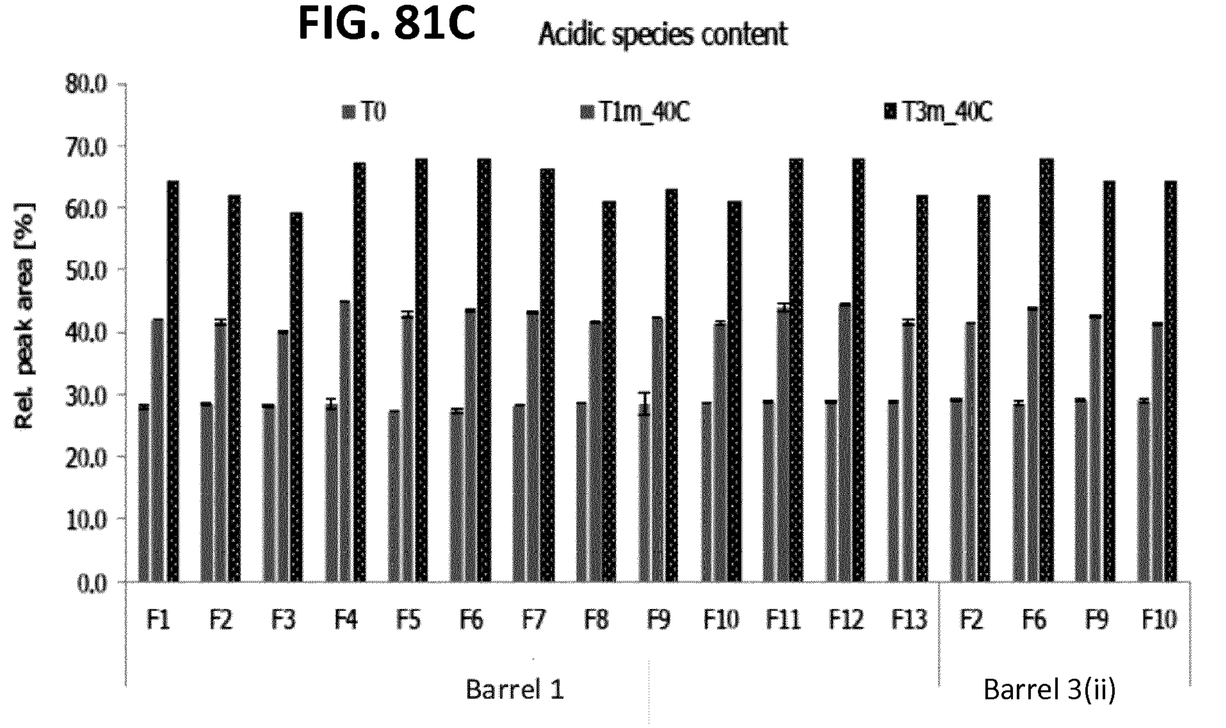

FIG. 82A

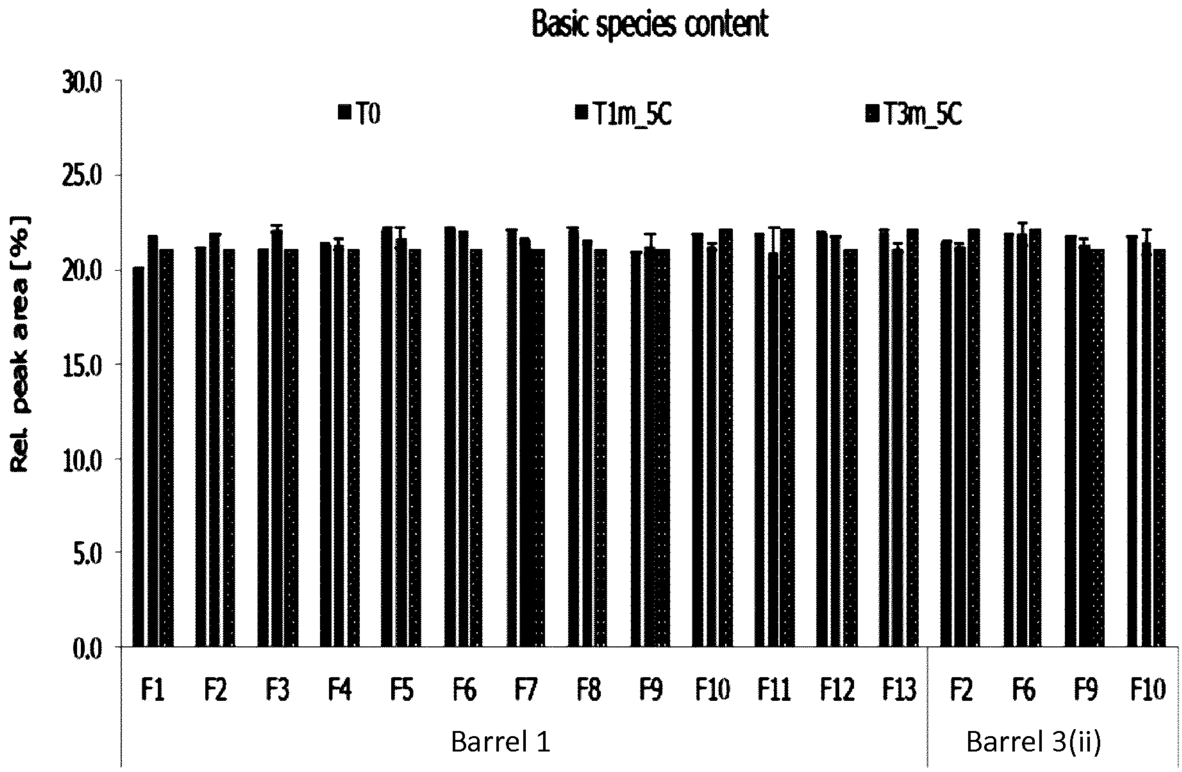
Basic species content
30.0
25.0
20.0
15.0
10.0
5.0
0.0
Rel. peak area [%]
T0
T1m_5C
T3m_5C
F1
F2
F3
F4
F5
F6
F7
F8
F9
F10
F11
F12
F13
F2
F6
F9
F10
Barrel 1
Barrel 3(ii)

FIG. 82B

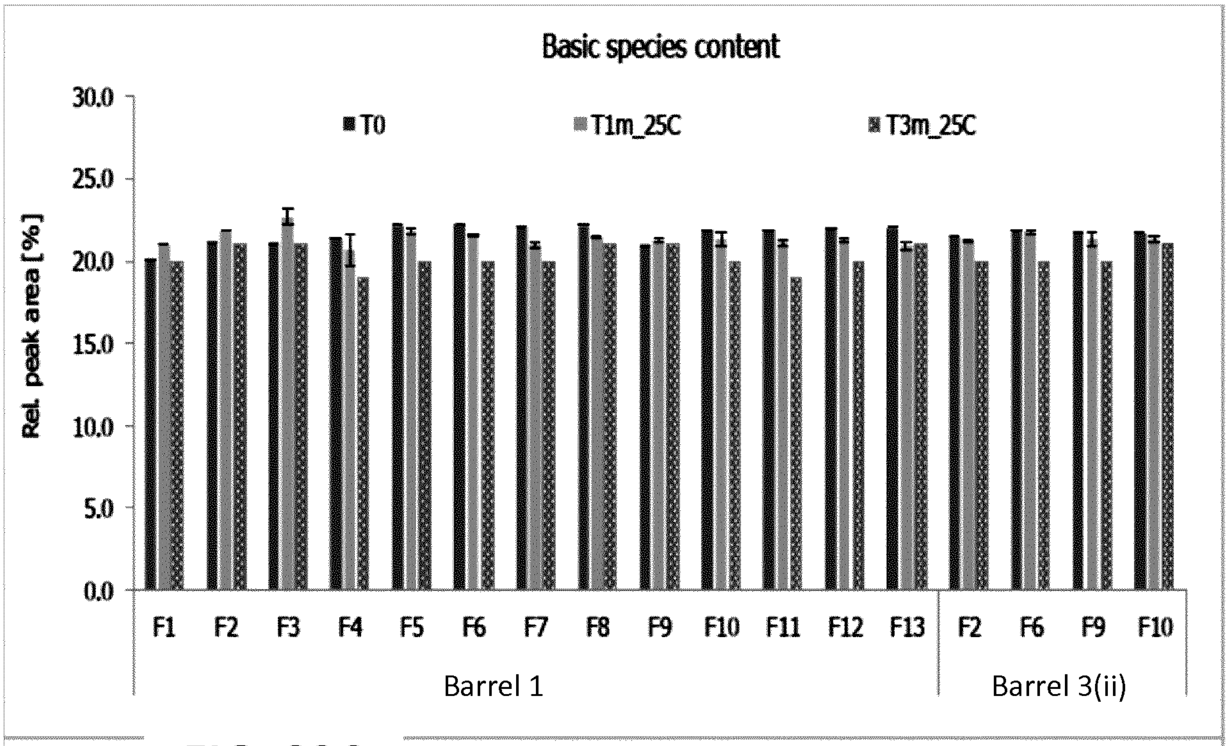


FIG. 82C

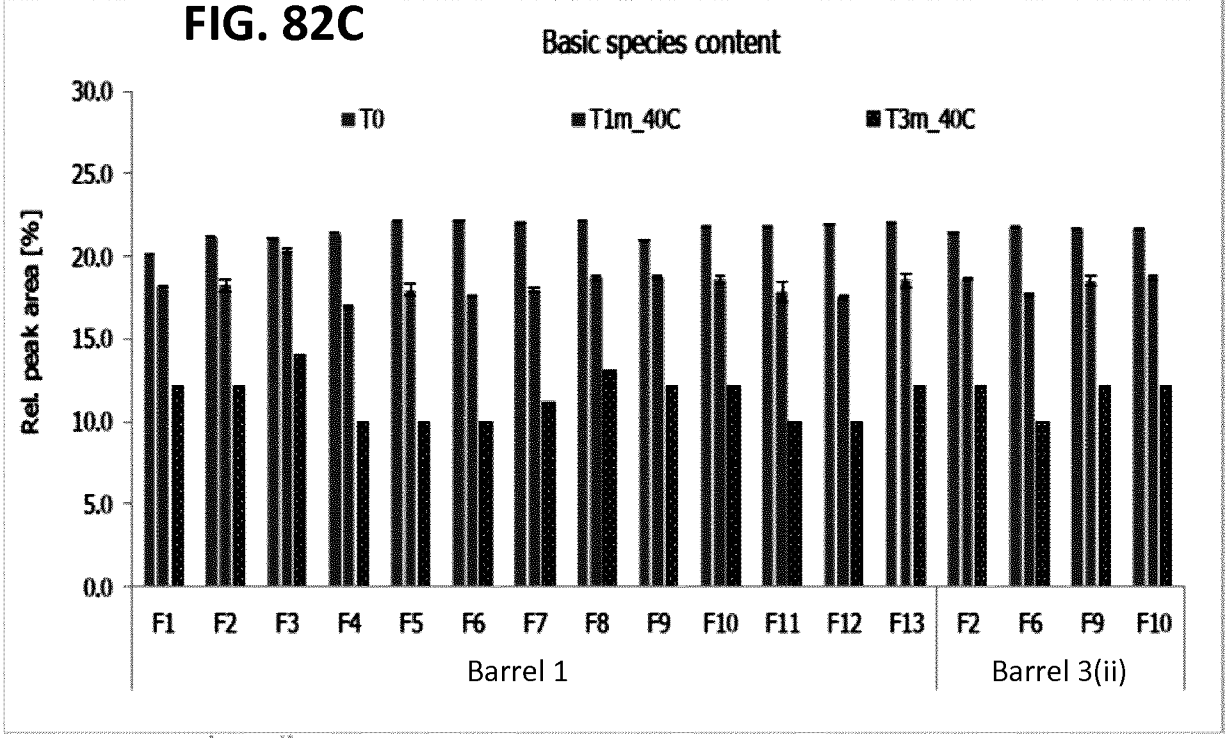

FIG. 83A

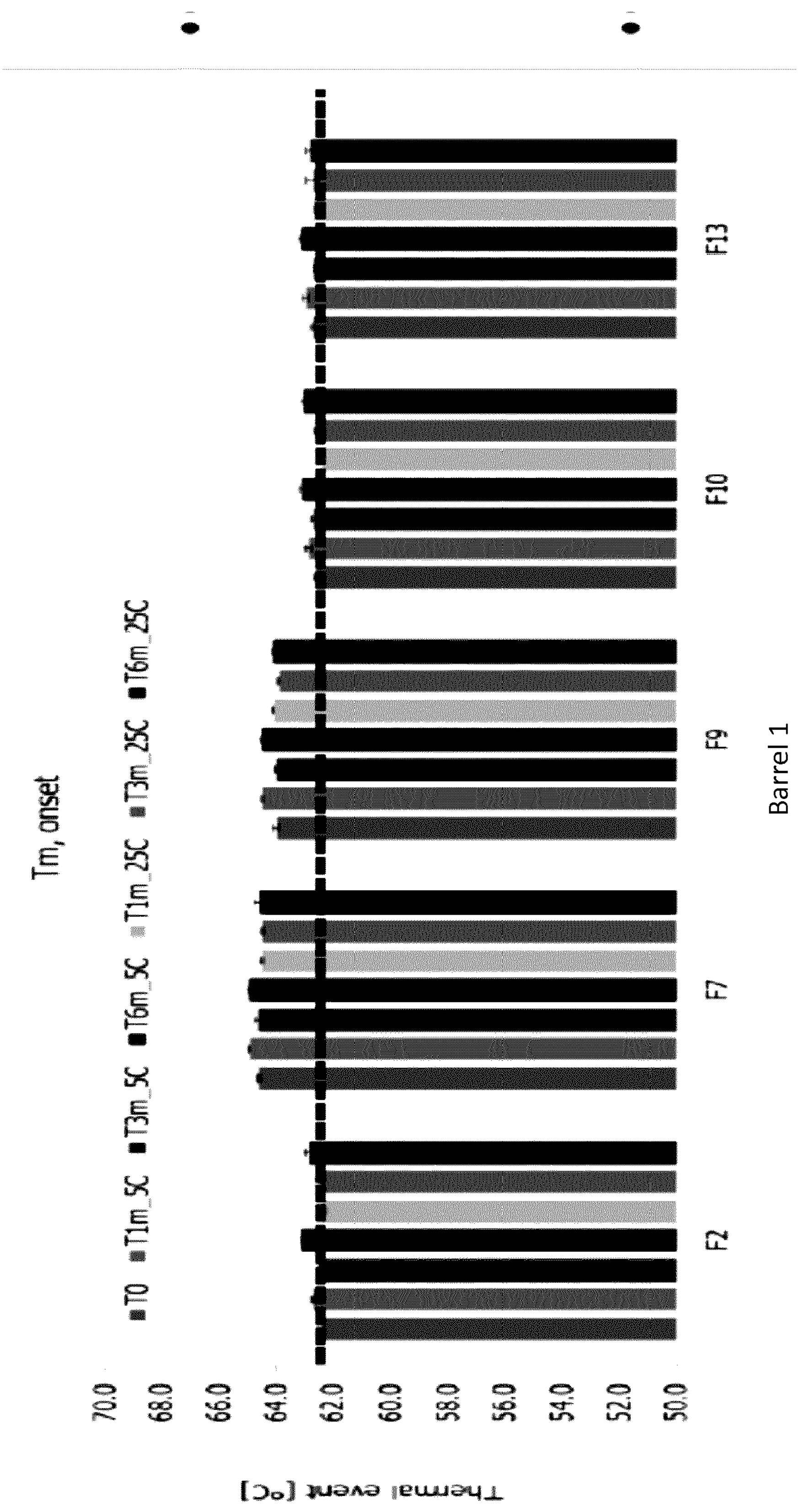
Tm, onset
T0
T1m_5C
T3m_5C
T6m_5C
T1m_25C
T3m_25C
T6m_25C
70.0
68.0
66.0
64.0
62.0
60.0
58.0
56.0
54.0
52.0
50.0
Thermal event [oC]
F2
F7
F9
F10
F13
Barrel 1

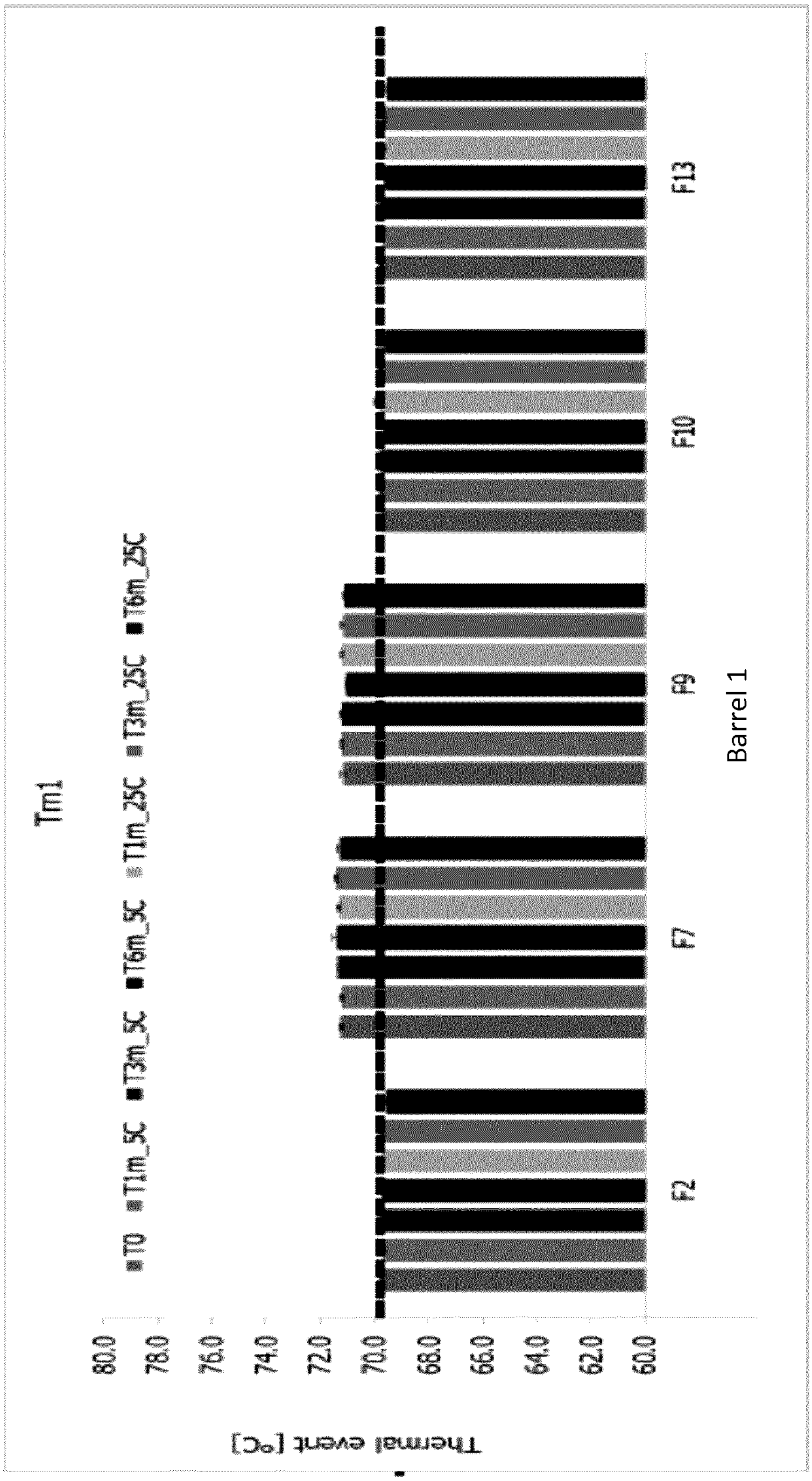
Tm1
T0
T1m_5C
T3m_5C
T6m_5C
T1m_25C
T3m_25C
T6m_25C
80.0
78.0
76.0
74.0
72.0
70.0
68.0
66.0
64.0
62.0
60.0
Thermal event [ºC]
F2
F7
F9
F10
F13
Barrel 1

FIG. 83B

FIG. 84

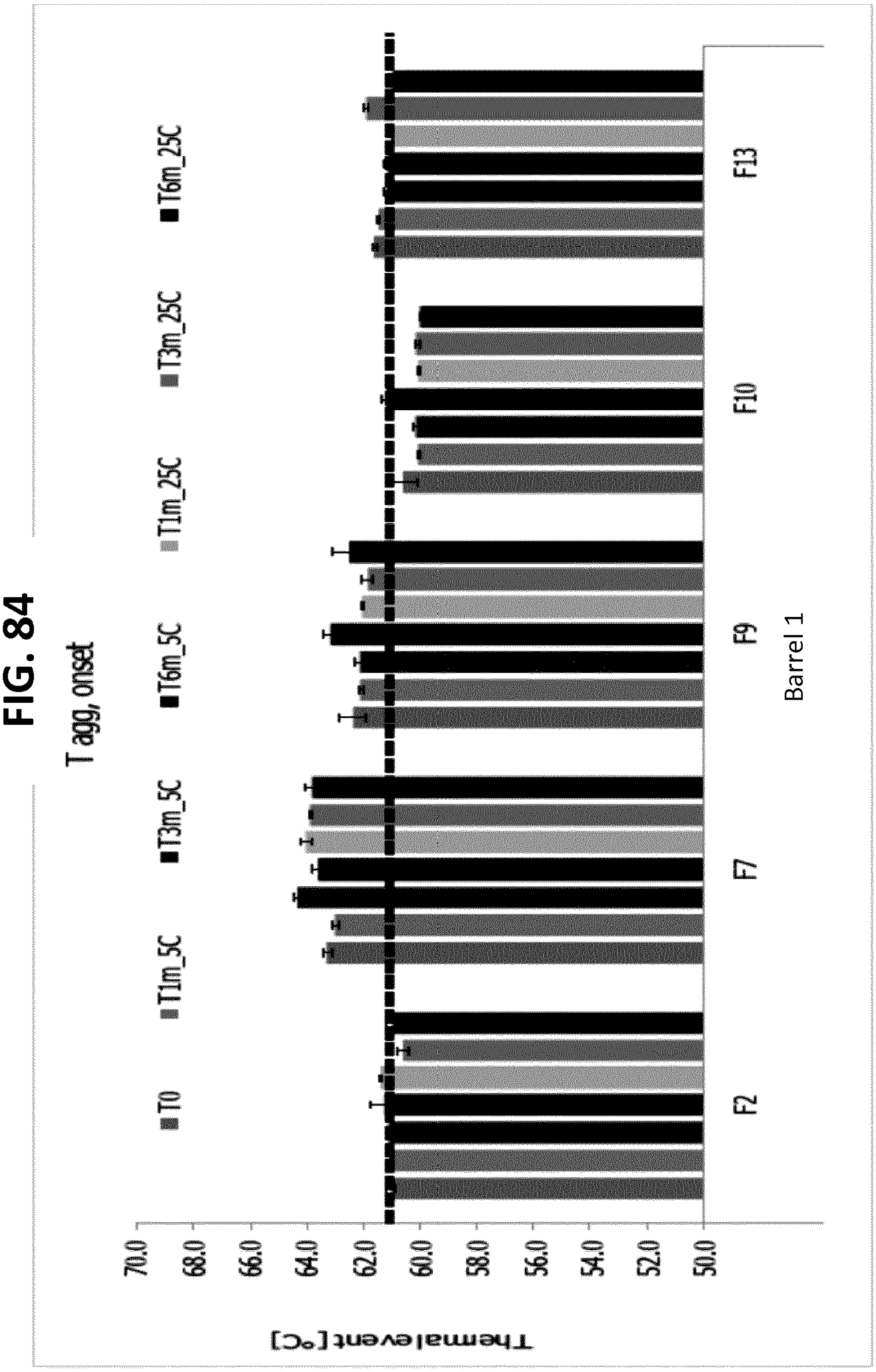
T agg, onset
T0
T1m_5C
T3m_5C
T6m_5C
T1m_25C
T3m_25C
T6m_25C
Themalevent [°C]
70.0
68.0
66.0
64.0
62.0
60.0
58.0
56.0
54.0
52.0
50.0
F2
F7
F9
F10
F13
Barrel 1

FIG. 85

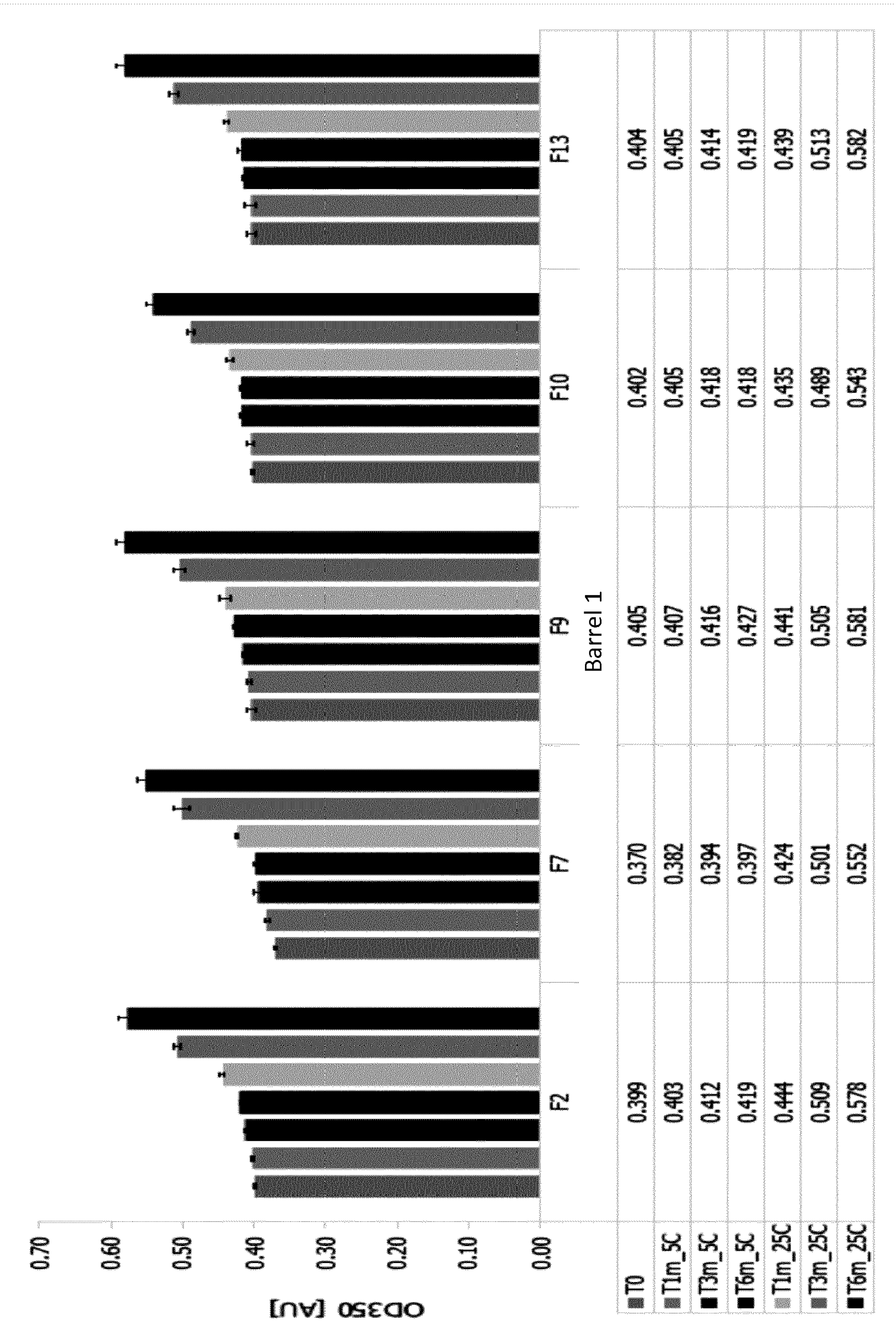


| | F2 | F7 | F9 | F10 | F13 |
|---|---|---|---|---|---|
| T0 | 0.399 | 0.370 | 0.405 | 0.402 | 0.404 |
| T1m_5C | 0.403 | 0.382 | 0.407 | 0.405 | 0.405 |
| T3m_5C | 0.412 | 0.394 | 0.416 | 0.418 | 0.414 |
| T6m_5C | 0.419 | 0.397 | 0.427 | 0.418 | 0.419 |
| T1m_25C | 0.444 | 0.424 | 0.441 | 0.435 | 0.439 |
| T3m_25C | 0.509 | 0.501 | 0.505 | 0.489 | 0.513 |
| T6m_25C | 0.578 | 0.552 | 0.581 | 0.543 | 0.582 |

FIG. 86

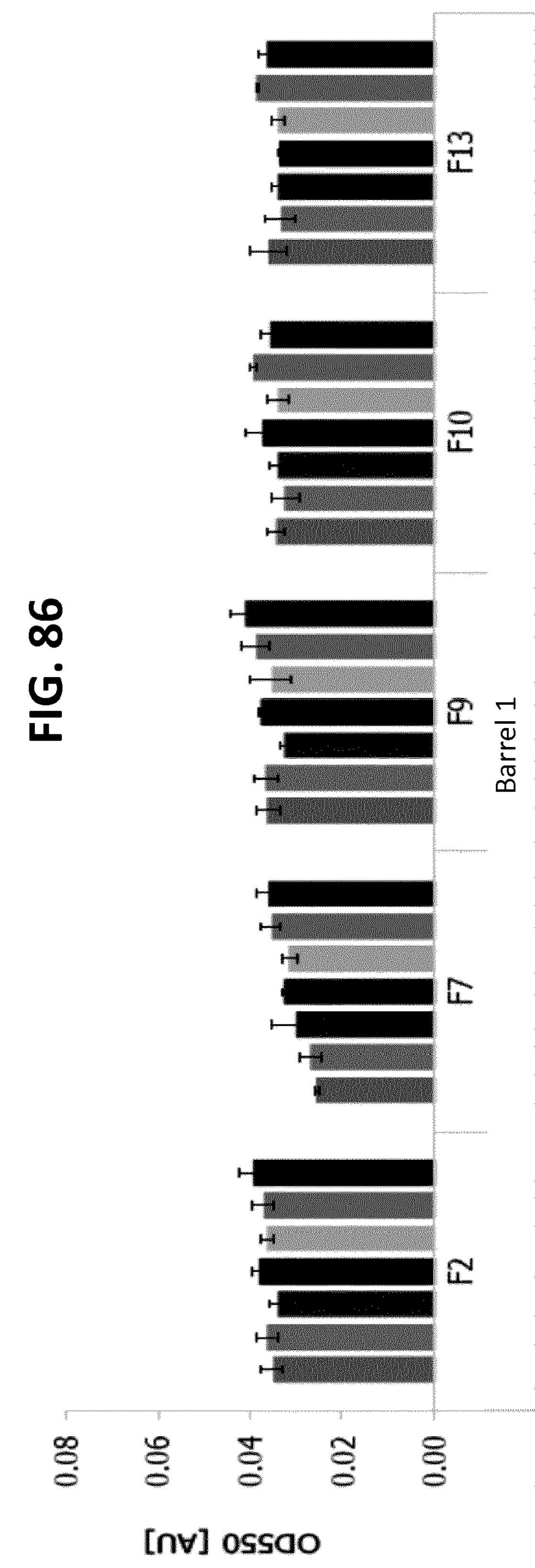


| | F2 | F7 | F9 | F10 | F13 |
|---|---|---|---|---|---|
| T0 | 0.035 | 0.026 | 0.036 | 0.034 | 0.036 |
| T1m_5C | 0.036 | 0.027 | 0.037 | 0.033 | 0.034 |
| T3m_5C | 0.034 | 0.030 | 0.033 | 0.034 | 0.034 |
| T6m_5C | 0.038 | 0.033 | 0.038 | 0.038 | 0.034 |
| T1m_25C | 0.036 | 0.032 | 0.036 | 0.034 | 0.034 |
| T3m_25C | 0.037 | 0.036 | 0.039 | 0.039 | 0.039 |
| T6m_25C | 0.039 | 0.036 | 0.041 | 0.036 | 0.036 |

# FIG.87A

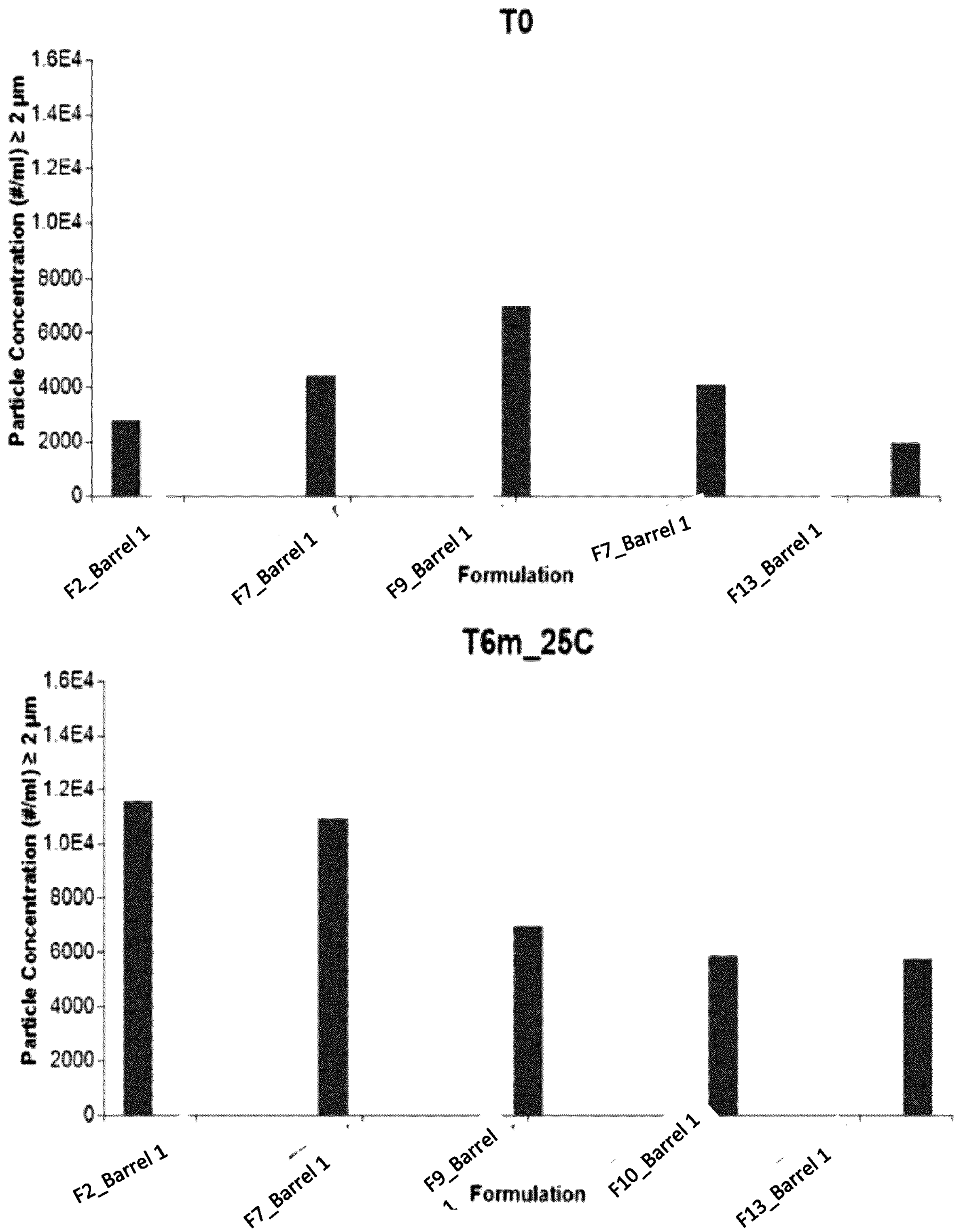
T0
Particle Concentration (#/ml) ≥ 2 µm
1.6E4
1.4E4
1.2E4
1.0E4
8000
6000
4000
2000
0
F2_Barrel 1
F7_Barrel 1
F9_Barrel 1
F7_Barrel 1
F13_Barrel 1
Formulation
T6m_25C
Particle Concentration (#/ml) ≥ 2 µm
1.6E4
1.4E4
1.2E4
1.0E4
8000
6000
4000
2000
0
F2_Barrel 1
F7_Barrel 1
F9_Barrel 1
F10_Barrel 1
F13_Barrel 1
Formulation

FIG. 87B

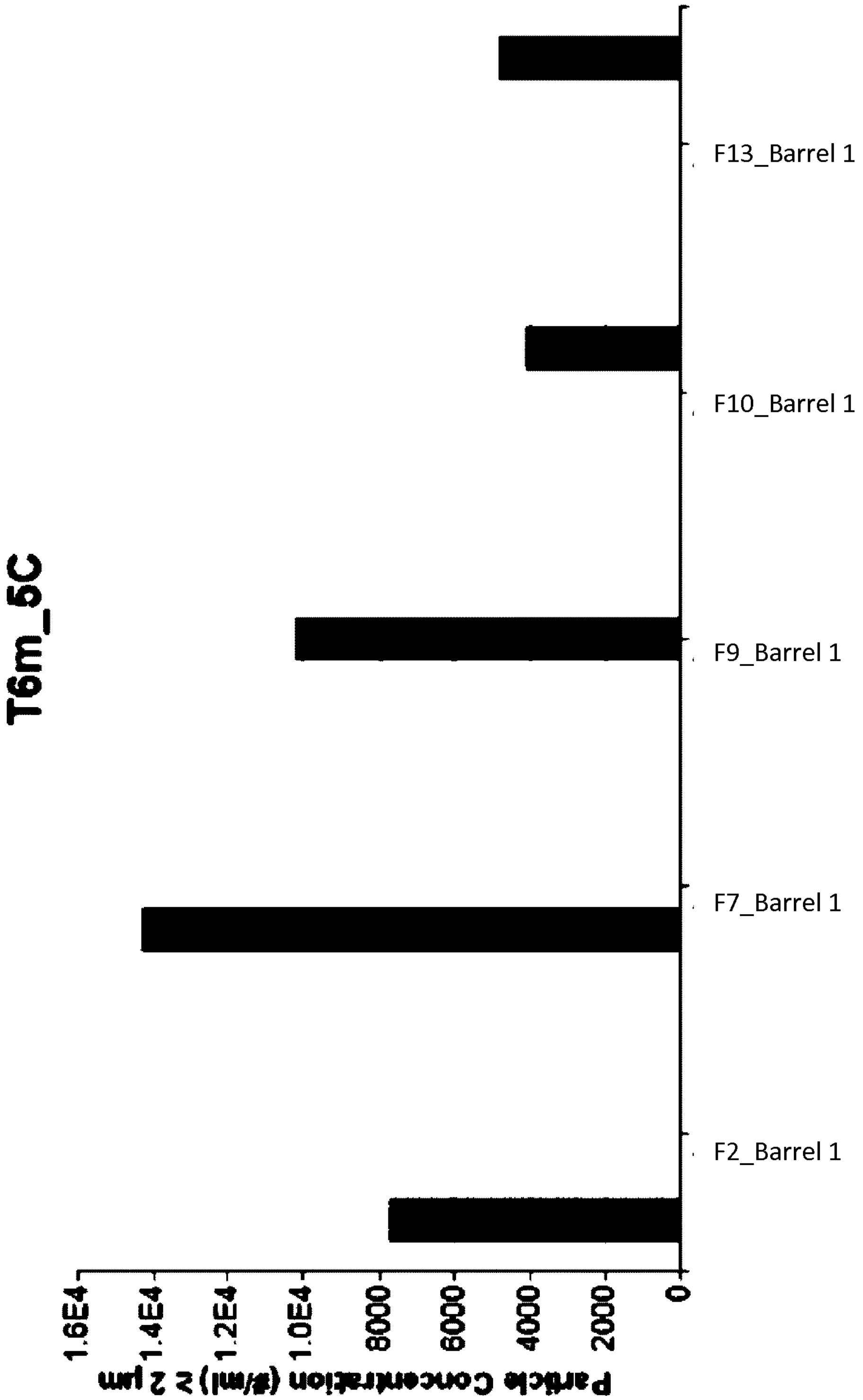
T6m_5C
Particle Concentration (#/ml) ≥ 2 μm
1.6E4
1.4E4
1.2E4
1.0E4
8000
6000
4000
2000
0
F2_Barrel 1
F7_Barrel 1
F9_Barrel 1
F10_Barrel 1
F13_Barrel 1

FIG.88A

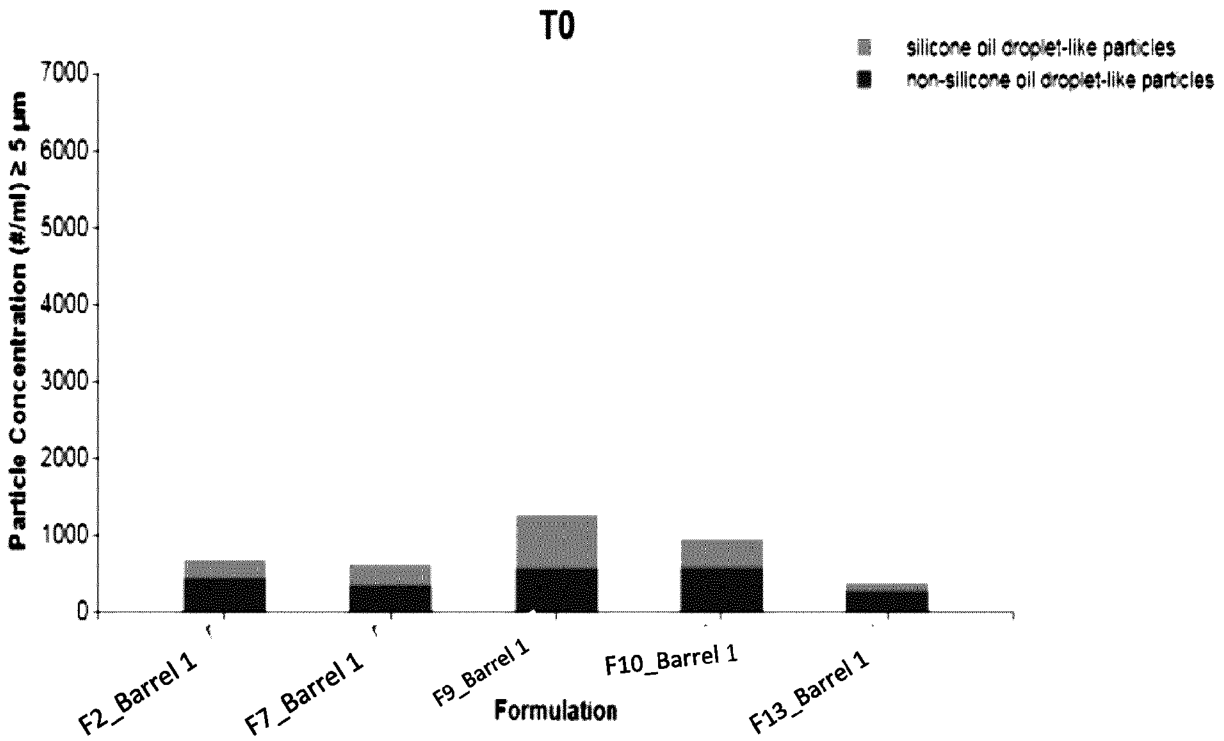


FIG. 88B T6m_25C

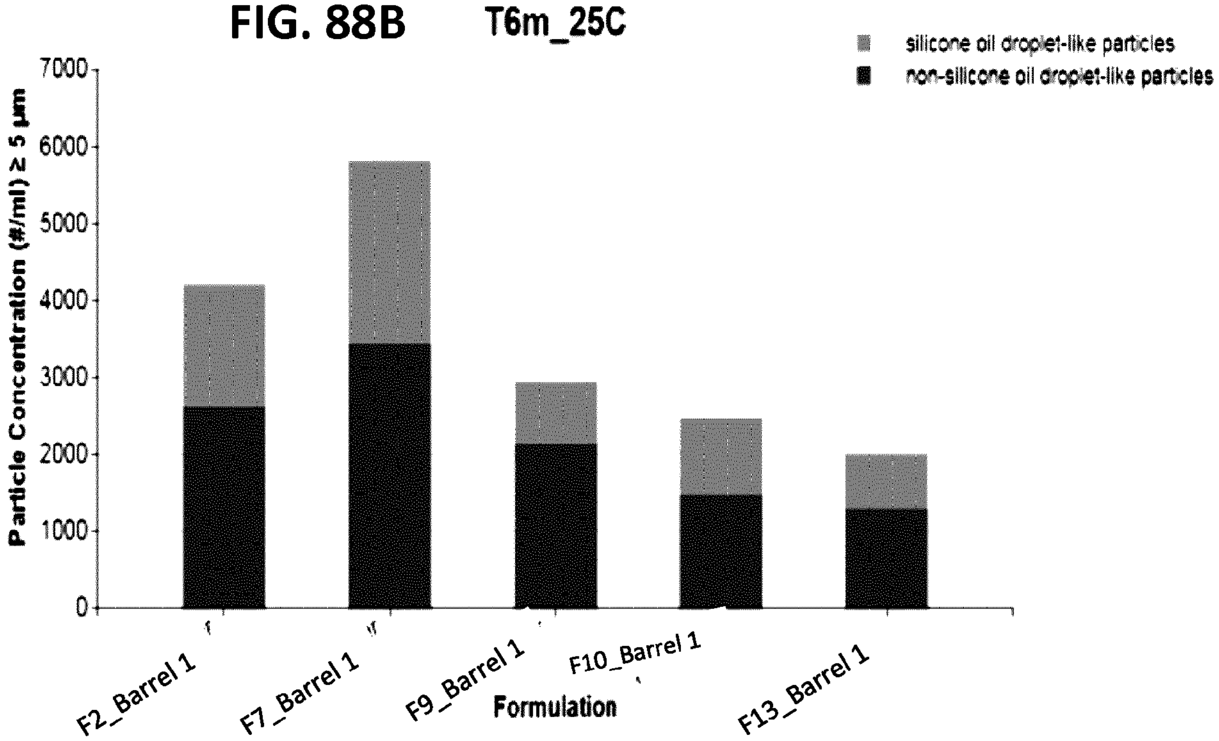

FIG.88C

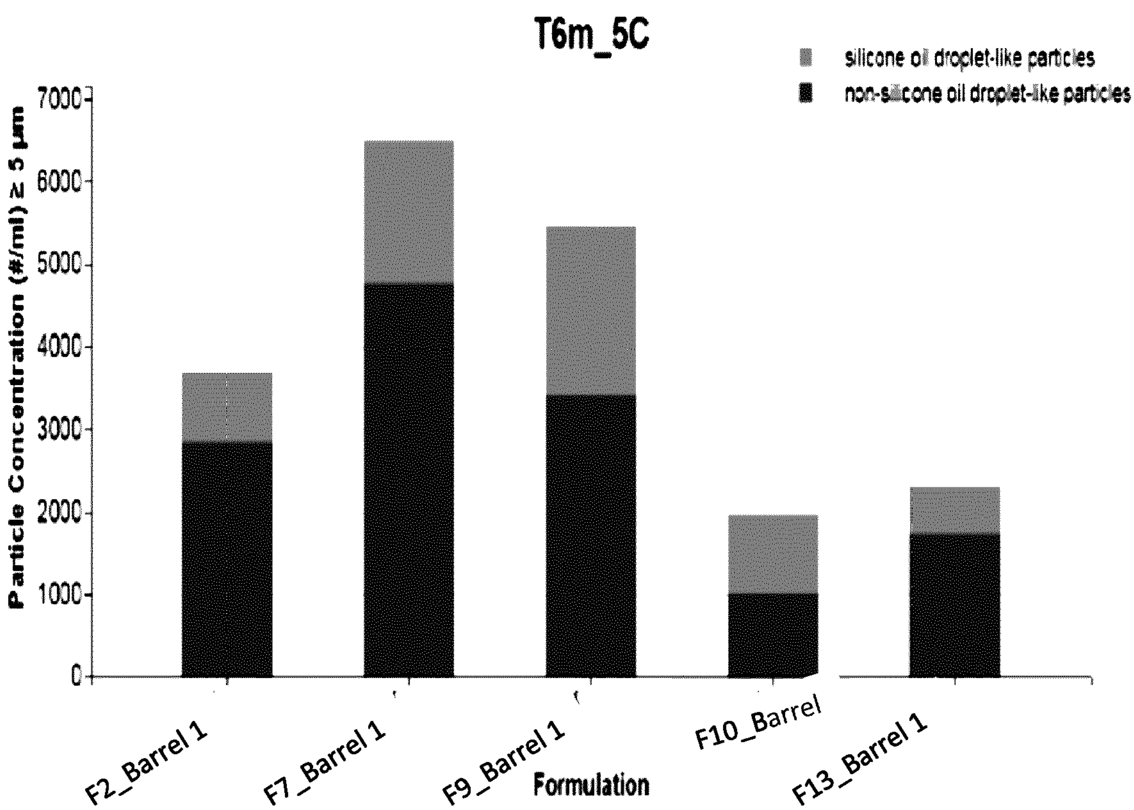
T6m_5C
silicone oil droplet-like particles
non-silicone oil droplet-like particles
Particle Concentration (#/ml) ≥ 5 µm
7000
6000
5000
4000
3000
2000
1000
0
F2_Barrel 1
F7_Barrel 1
F9_Barrel 1
F10_Barrel
F13_Barrel 1
Formulation

FIG. 89A

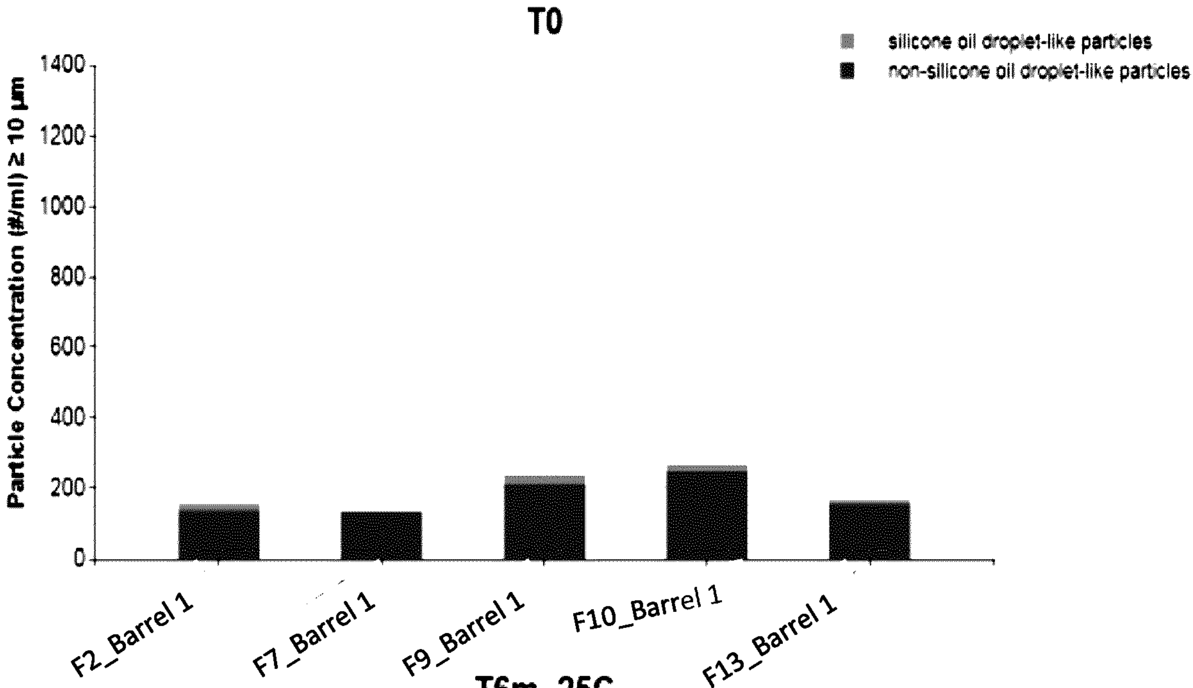


FIG. 89B

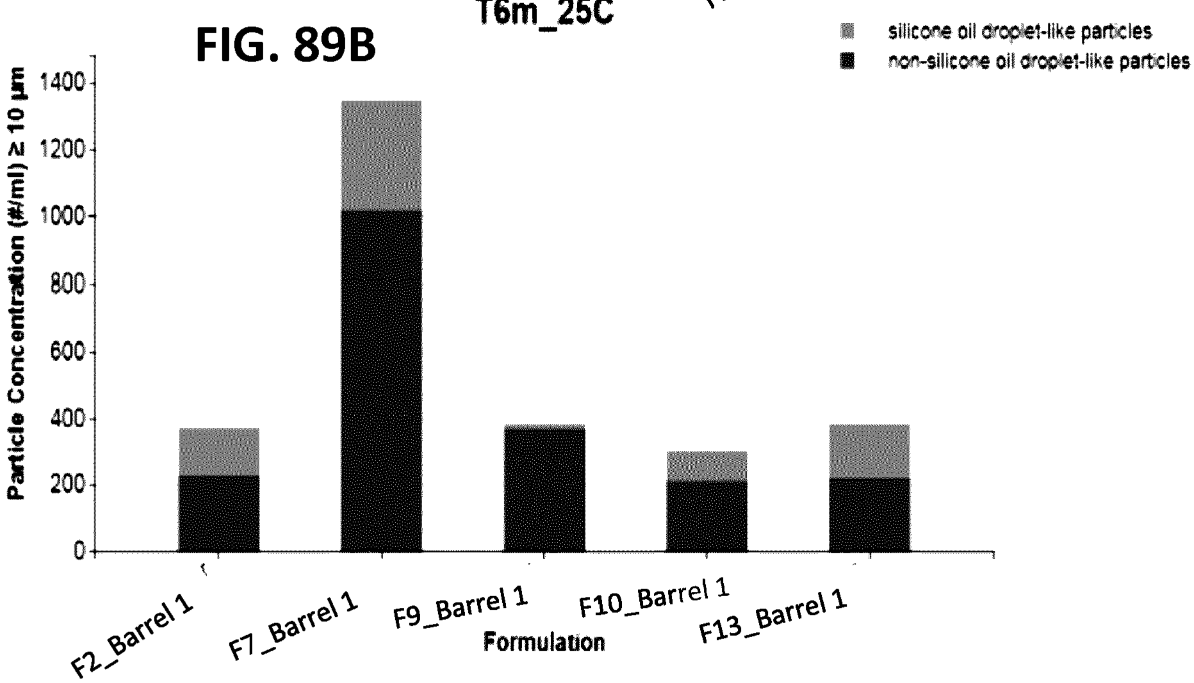

FIG. 89C

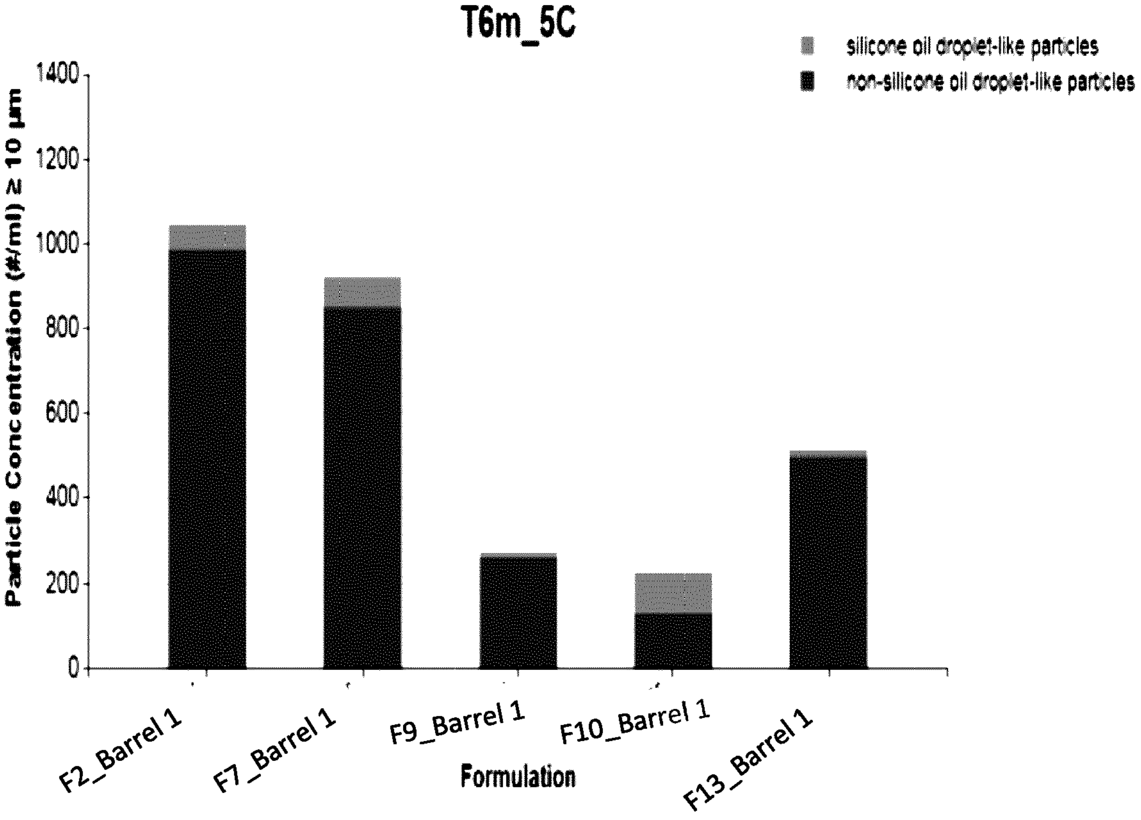
T6m_5C
silicone oil droplet-like particles
non-silicone oil droplet-like particles
Particle Concentration (#/ml) ≥ 10 µm
1400
1200
1000
800
600
400
200
0
F2_Barrel 1
F7_Barrel 1
F9_Barrel 1
F10_Barrel 1
F13_Barrel 1
Formulation

FIG. 90A

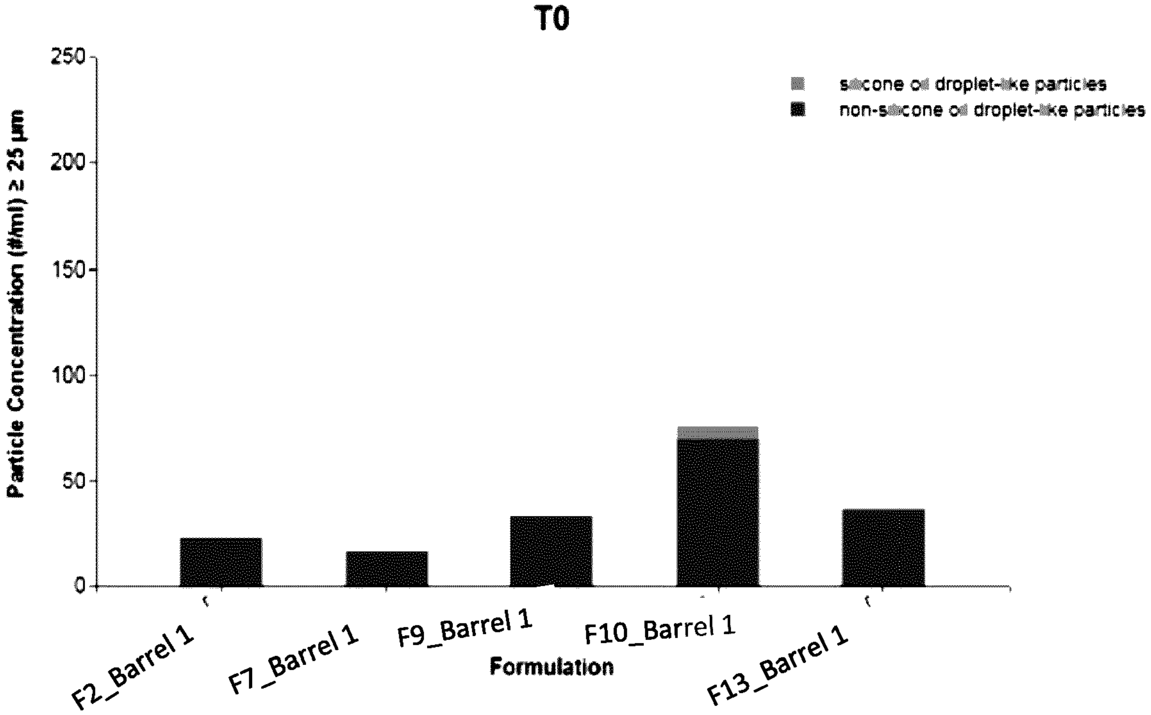


FIG. 90B

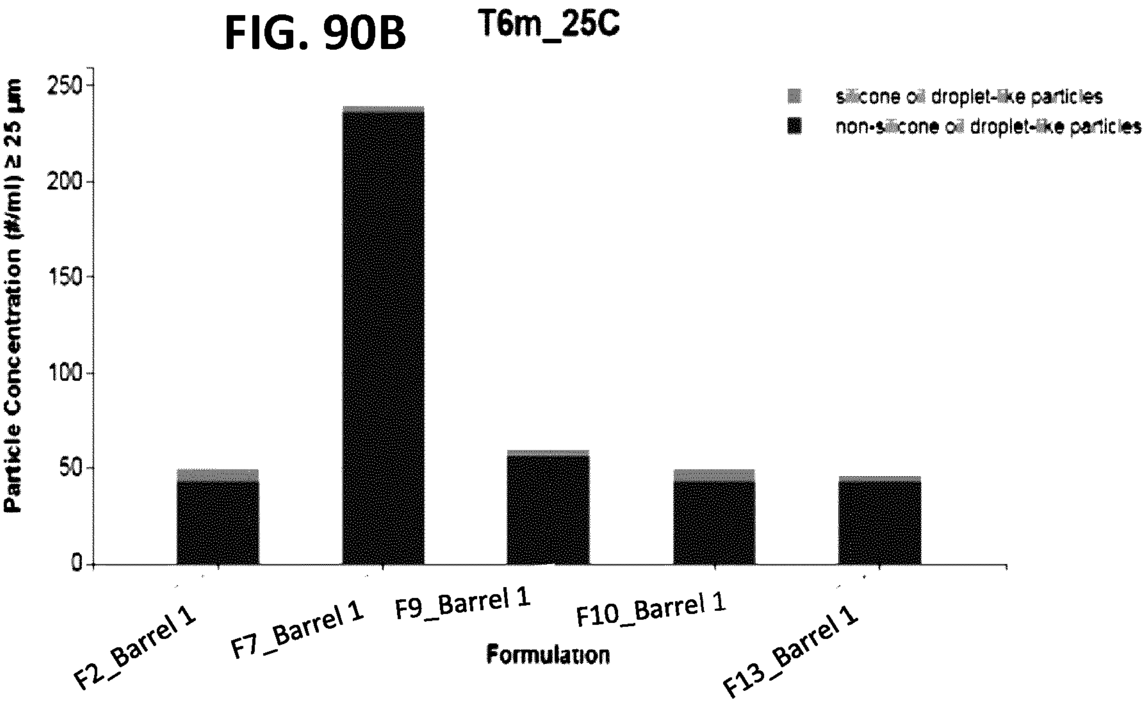

FIG. 90C

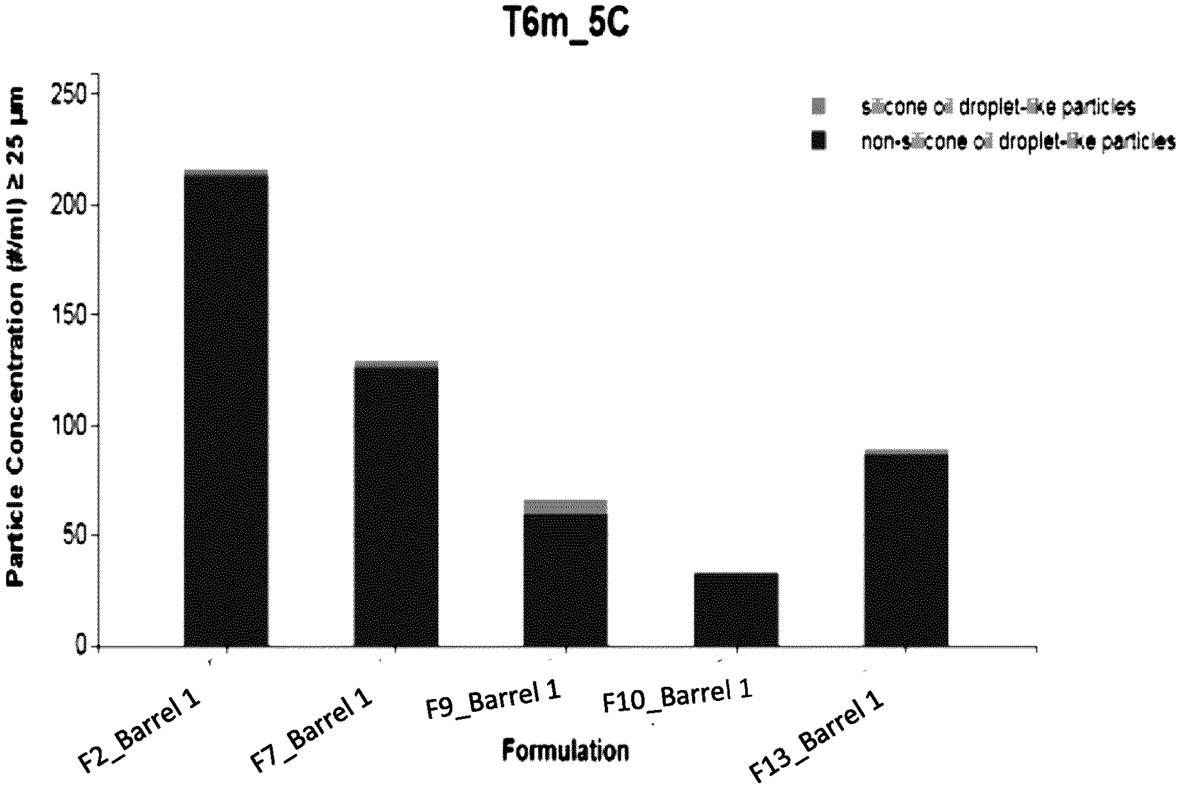
T6m_5C
Particle Concentration (#/ml) ≥ 25 µm
250
200
150
100
50
0
silicone oil droplet-like particles
non-silicone oil droplet-like particles
F2_Barrel 1
F7_Barrel 1
F9_Barrel 1
F10_Barrel 1
F13_Barrel 1
Formulation

FIG. 91

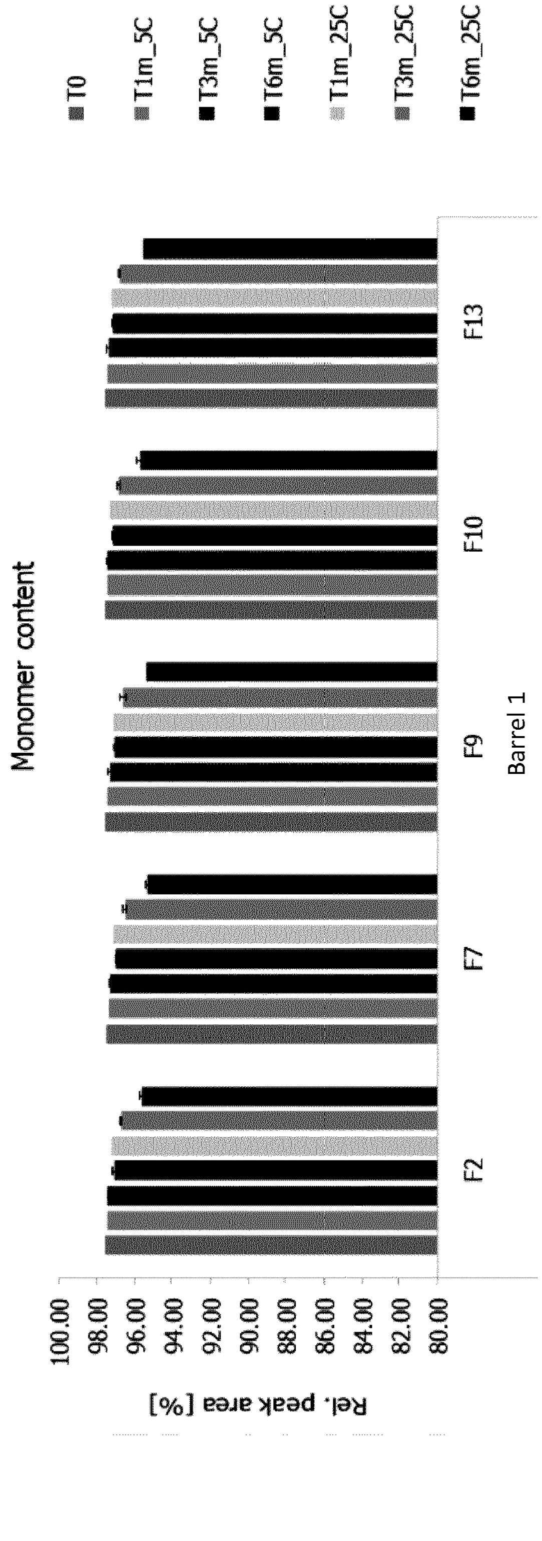
Monomer content
Rel. peak area [%]
100.00
98.00
96.00
94.00
92.00
90.00
88.00
86.00
84.00
82.00
80.00
F2
F7
F9
F10
F13
Barrel 1
T0
T1m_5C
T3m_5C
T6m_5C
T1m_25C
T3m_25C
T6m_25C

FIG. 92

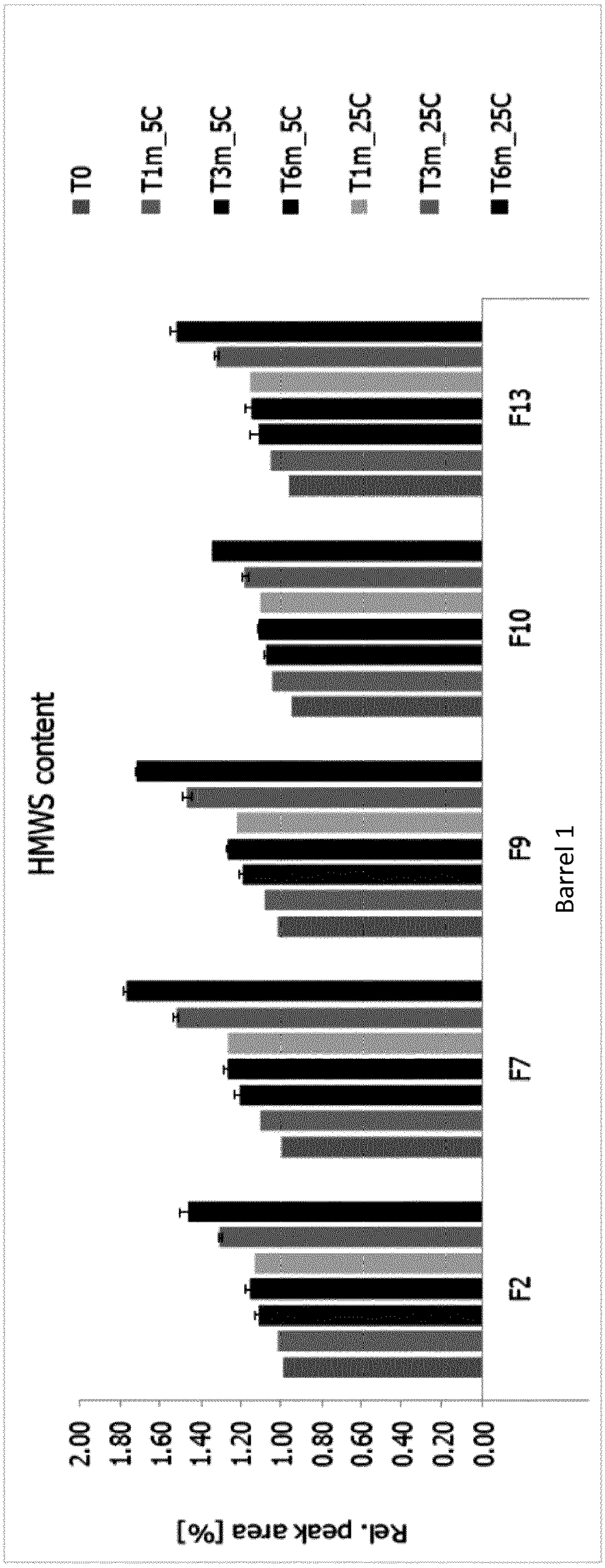
HMWS content
Rel. peak area [%]
2.00
1.80
1.60
1.40
1.20
1.00
0.80
0.60
0.40
0.20
0.00
F2
F7
F9
F10
F13
Barrel 1
T0
T1m_5C
T3m_5C
T6m_5C
T1m_25C
T3m_25C
T6m_25C

# FIG. 93

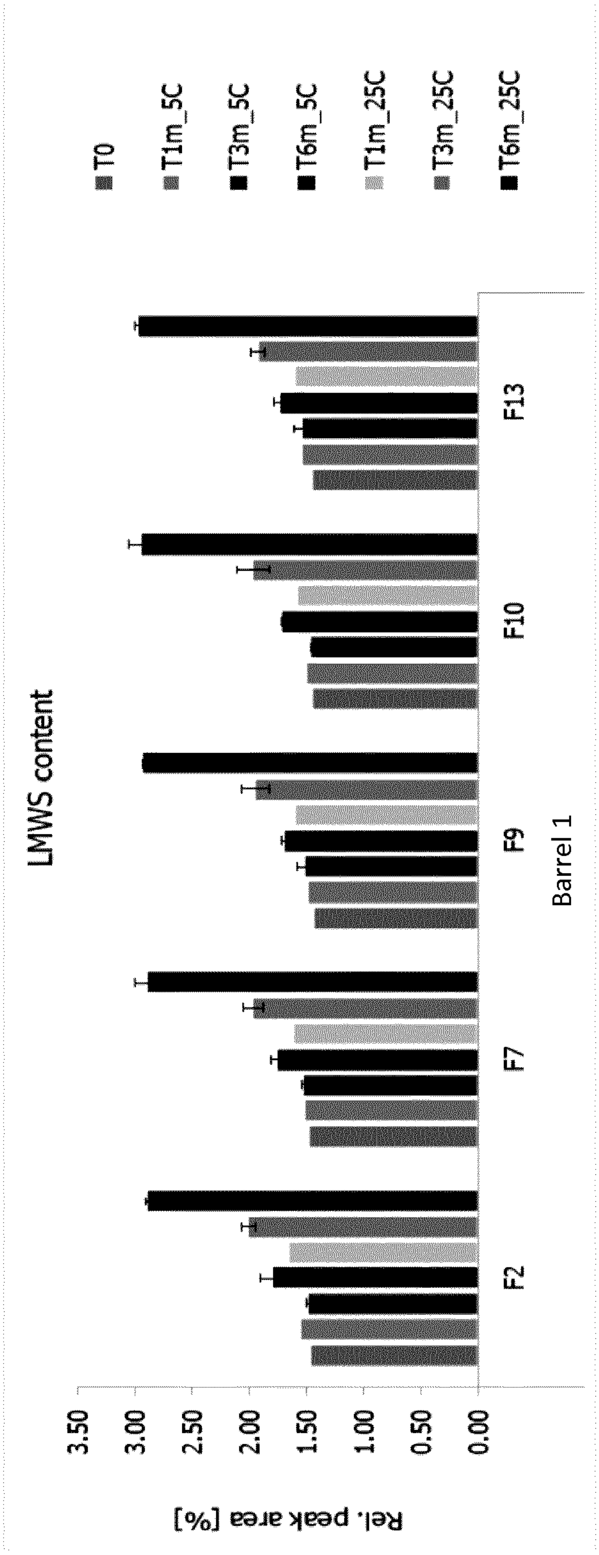
LMWS content
Rel. peak area [%]
3.50
3.00
2.50
2.00
1.50
1.00
0.50
0.00
F2
F7
F9
F10
F13
Barrel 1
T0
T1m_5C
T3m_5C
T6m_5C
T1m_25C
T3m_25C
T6m_25C

FIG. 94

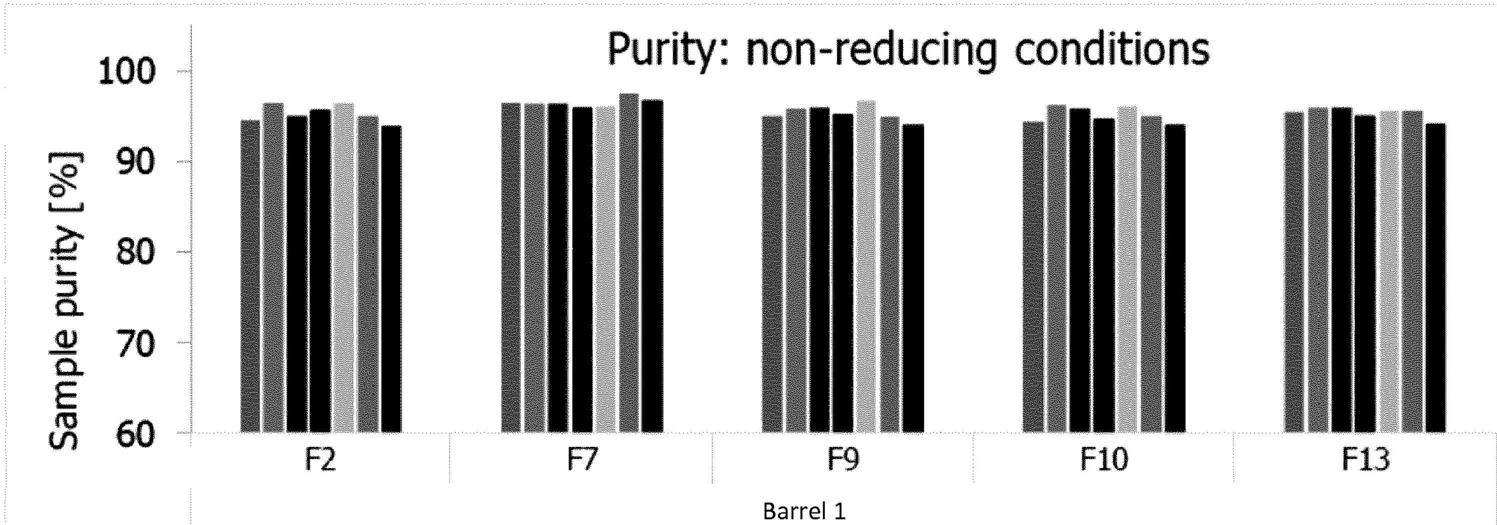


| | F2 | F7 | F9 | F10 | F13 |
|---|---|---|---|---|---|
| T0 | 94.6 | 96.5 | 95.0 | 94.4 | 95.4 |
| T1m_5C | 96.5 | 96.3 | 95.8 | 96.2 | 95.9 |
| T3m_5C | 95.1 | 96.3 | 95.9 | 95.8 | 96.0 |
| T6m_5C | 95.7 | 95.9 | 95.2 | 94.7 | 95.1 |
| T1m_25C | 96.5 | 96.1 | 96.7 | 96.1 | 95.6 |
| T3m_25C | 95.0 | 97.5 | 94.9 | 95.1 | 95.6 |
| T6m_25C | 93.9 | 96.7 | 94.0 | 94.0 | 94.1 |

# FIG. 95

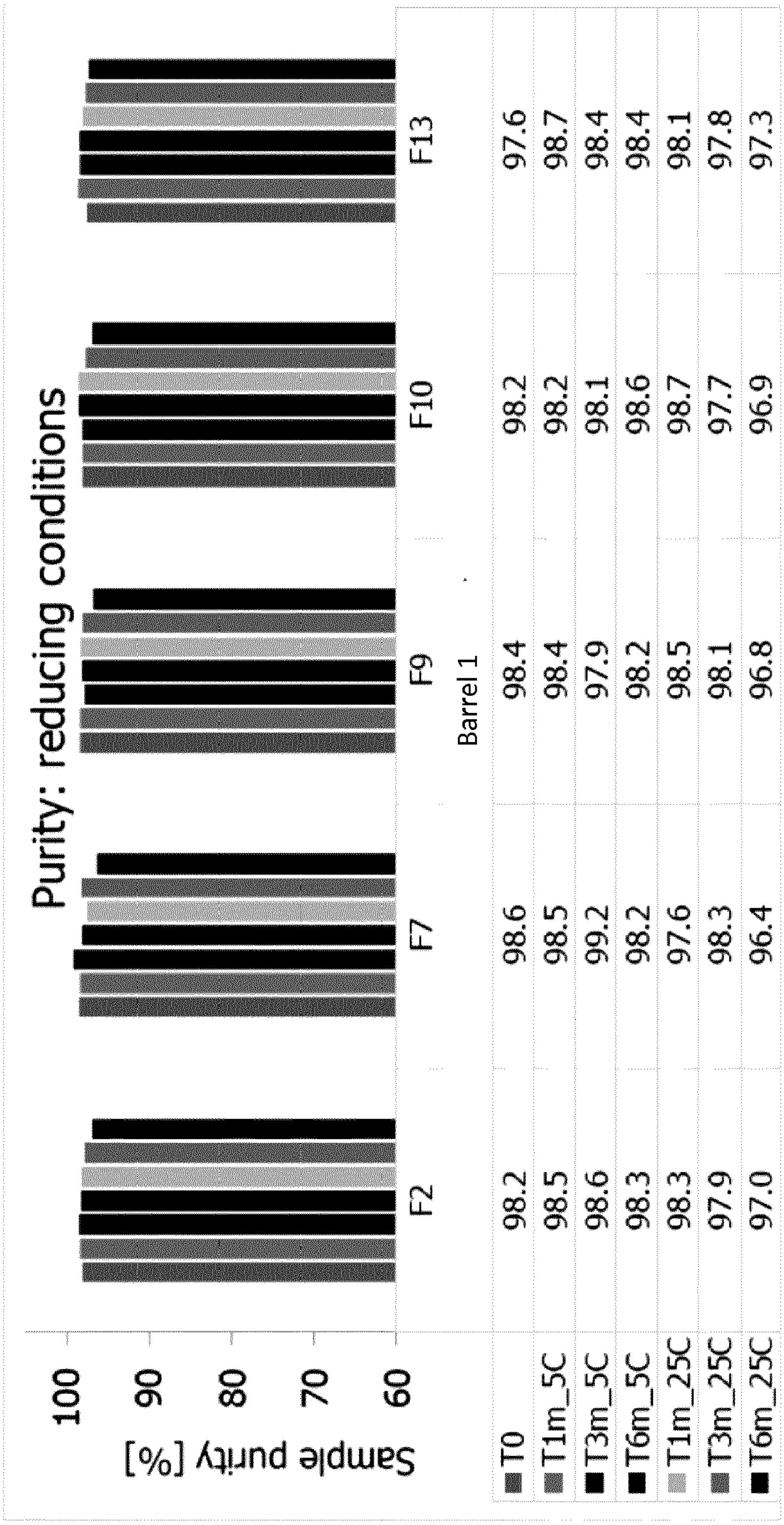


| | F2 | F7 | F9 | F10 | F13 |
|---|---|---|---|---|---|
| T0 | 98.2 | 98.6 | 98.4 | 98.2 | 97.6 |
| T1m_5C | 98.5 | 98.5 | 98.4 | 98.2 | 98.7 |
| T3m_5C | 98.6 | 99.2 | 97.9 | 98.1 | 98.4 |
| T6m_5C | 98.3 | 98.2 | 98.2 | 98.6 | 98.4 |
| T1m_25C | 98.3 | 97.6 | 98.5 | 98.7 | 98.1 |
| T3m_25C | 97.9 | 98.3 | 98.1 | 97.7 | 97.8 |
| T6m_25C | 97.0 | 96.4 | 96.8 | 96.9 | 97.3 |

FIG. 96

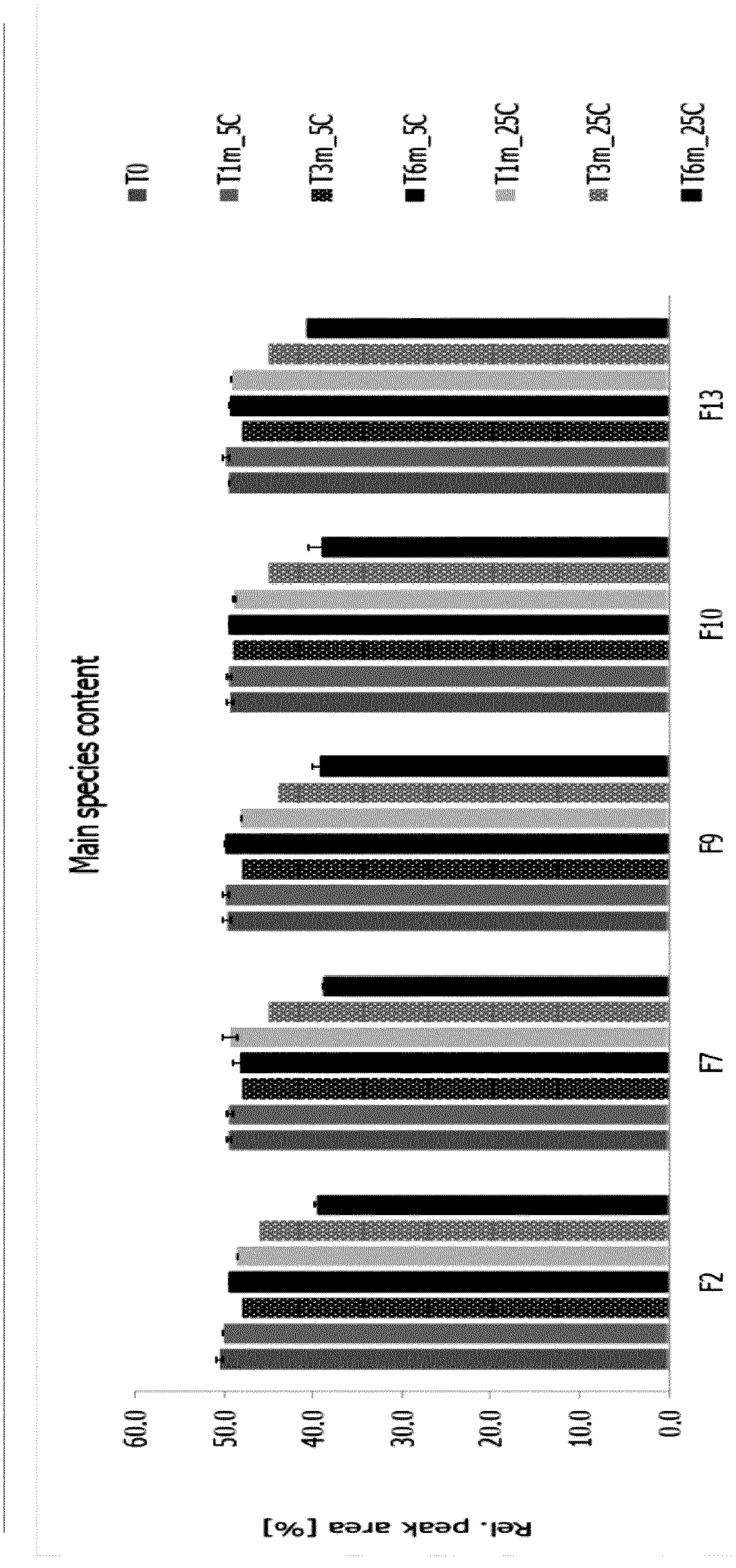

Main species content
Rel. peak area [%]
60.0
50.0
40.0
30.0
20.0
10.0
0.0
F2
F7
F9
F10
F13
T0
T1m_5C
T3m_5C
T6m_5C
T1m_25C
T3m_25C
T6m_25C

FIG. 97

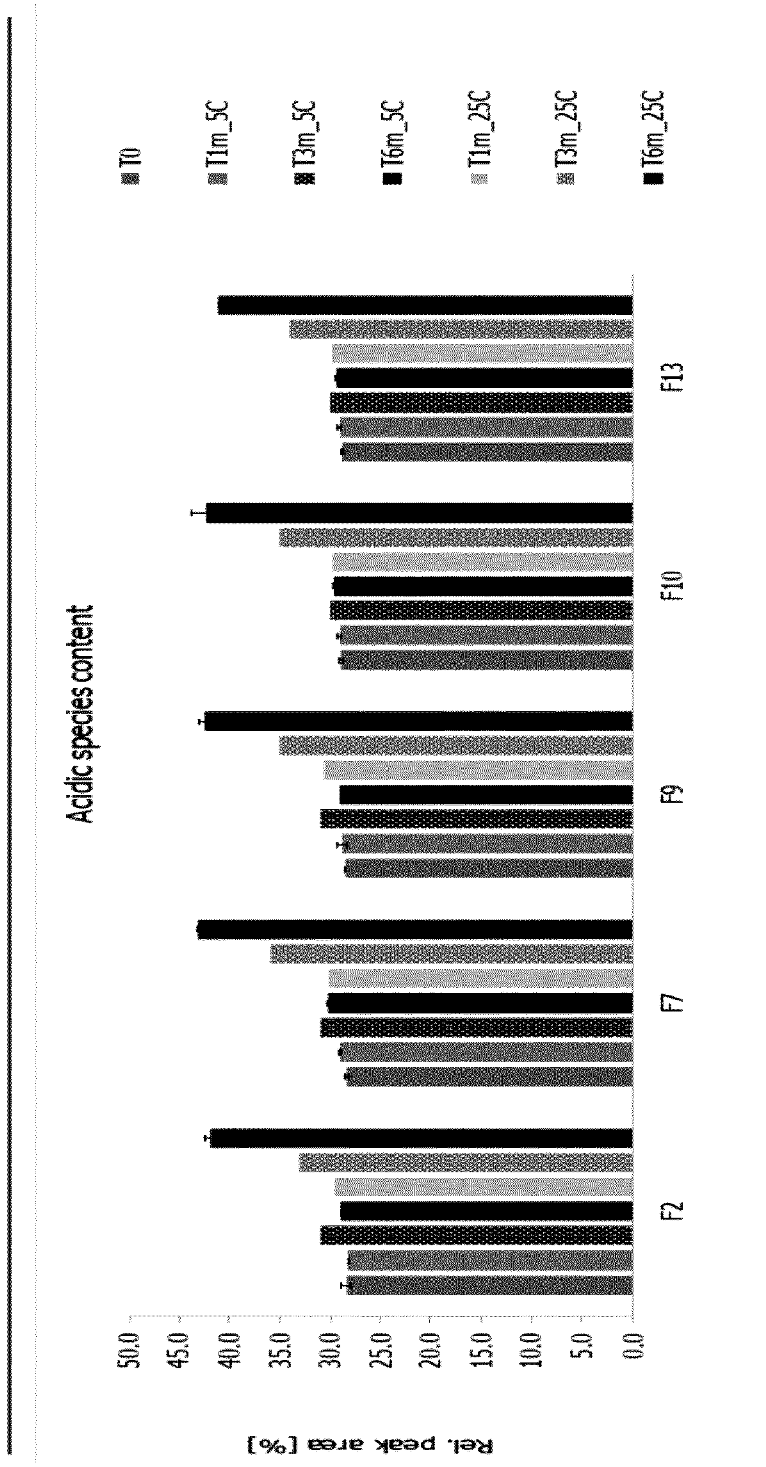
Acidic species content
Rel. peak area [%]
50.0
45.0
40.0
35.0
30.0
25.0
20.0
15.0
10.0
5.0
0.0
F2
F7
F9
F10
F13
T0
T1m_5C
T3m_5C
T6m_5C
T1m_25C
T3m_25C
T6m_25C

FIG. 98

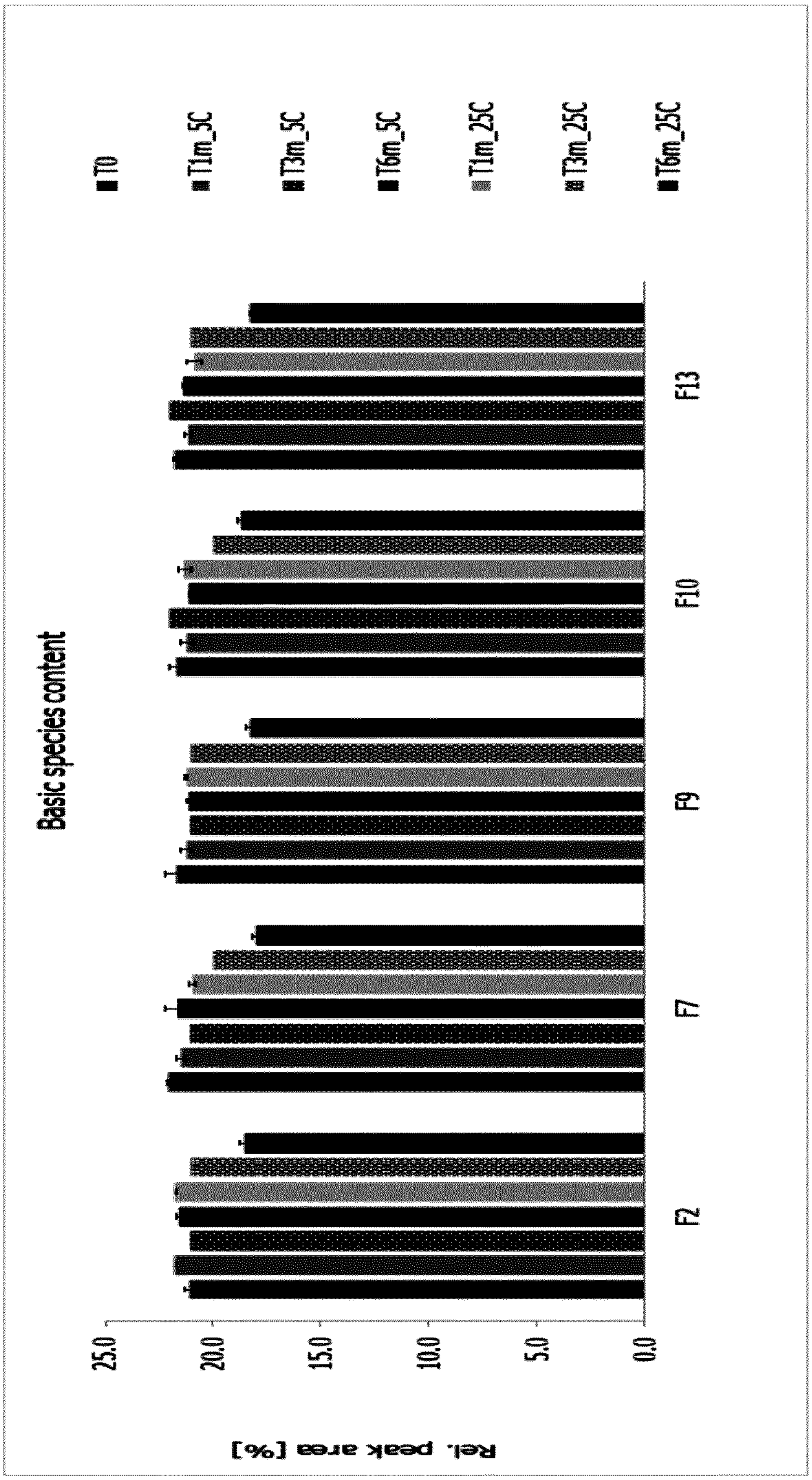
Basic species content
Rel. peak area [%]
25.0
20.0
15.0
10.0
5.0
0.0
F2
F7
F9
F10
F13
T0
T1m_5C
T3m_5C
T6m_5C
T1m_25C
T3m_25C
T6m_25C

Europäisches Patentamt
European Patent Office
Office européen des brevets

# EUROPEAN SEARCH REPORT

Application Number
EP 19 18 7338

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/096418 A2 (UCB PHARMA SA; LAWSON ALASTAIR DAVID GRIFFITHS [GB] ET AL.) 26 August 2010 (2010-08-26) * claims 1-28,36 * * page 25, lines 19-22 * ----- | 1-15 | INV. A61K9/08 A61K9/00 A61K39/395 A61K47/26 |
| X | WO 2017/134292 A1 (GLENMARK PHARMACEUTICALS SA [CH]) 10 August 2017 (2017-08-10) * page 7, lines 3-17; claims 1-10; example 1 * ----- | 1-15 | |
| X | Anonymous: "Anti-OX40 Antagonist Antibody", , 22 June 2017 (2017-06-22), XP055658970, Retrieved from the Internet: URL:https://web.archive.org/web/20170622014410/http://bpsbioscience.com/immunotherapy/antibodies/anti-ox40-antibody-72063-1 [retrieved on 2020-01-17] * the whole document * ----- | 1,5 | |
| | | | TECHNICAL FIELDS SEARCHED (IPC) |
| X | W Anonymous: "Anti-OX40 antagonist antibody Data Sheet,", BOPS Bioscienc, 23 August 2017 (2017-08-23), pages 511-521, XP055658980, Retrieved from the Internet: URL:https://web.archive.org/web/20170823182436if_/http://bpsbioscience.com/media/wysiwyg/72063-1_Lot_161005.pdf [retrieved on 2020-01-17] * the whole document * ----- -/-- | 1,5 | A61K C07K |

The present search report has been drawn up for all claims

2

| Place of search | Date of completion of the search | Examiner |
|---|---|---|
| Munich | 17 January 2020 | Madalinska, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 19 18 7338

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | WO 2019/229155 A1 (GLENMARK PHARMCEUTICALS S A [CH]) 5 December 2019 (2019-12-05) * page 115, lines 3-5; claims 1-15; example 3 * * page 35, lines 14-22 * ----- | 1-5,7,8 | |
| E | WO 2019/219978 A1 (GLENMARK PHARMACEUTICALS SA [CH]) 21 November 2019 (2019-11-21) * page 98, paragraph Investigational product * * page 40, line 29 - page 41, line 9 * ----- | 1-5,7,8 | |
| | | | TECHNICAL FIELDS SEARCHED (IPC) |

The present search report has been drawn up for all claims

| Place of search | Date of completion of the search | Examiner |
|---|---|---|
| Munich | 17 January 2020 | Madalinska, K |

2

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

page 2 of 2

## ANNEX TO THE EUROPEAN SEARCH REPORT ON EUROPEAN PATENT APPLICATION NO. EP 19 18 7338

This annex lists the patent family members relating to the patent documents cited in the above-mentioned European search report. The members are as contained in the European Patent Office EDP file on
The European Patent Office is in no way liable for these particulars which are merely given for the purpose of information.

17-01-2020

| Patent document cited in search report | Publication date | Patent family member(s) | Publication date |
|---|---|---|---|
| WO 2010096418 A2 | 26-08-2010 | AR 075504 A1 | 06-04-2011 |
| | | AU 2010216152 A1 | 18-08-2011 |
| | | BR PI1008692 A2 | 08-03-2016 |
| | | CA 2751477 A1 | 26-08-2010 |
| | | CL 2011001994 A1 | 24-02-2012 |
| | | CN 102741287 A | 17-10-2012 |
| | | CO 6410312 A2 | 30-03-2012 |
| | | DK 2398498 T3 | 07-01-2019 |
| | | EA 201171060 A1 | 30-07-2012 |
| | | EP 2398498 A2 | 28-12-2011 |
| | | ES 2699716 T3 | 12-02-2019 |
| | | HK 1177470 A1 | 18-12-2015 |
| | | HR P20181932 T1 | 25-01-2019 |
| | | HU E042114 T2 | 28-06-2019 |
| | | IL 214282 A | 30-04-2017 |
| | | JP 5782385 B2 | 24-09-2015 |
| | | JP 2012521191 A | 13-09-2012 |
| | | JP 2015171362 A | 01-10-2015 |
| | | KR 20110128301 A | 29-11-2011 |
| | | LT 2398498 T | 10-01-2019 |
| | | MA 33122 B1 | 01-03-2012 |
| | | MY 157473 A | 15-06-2016 |
| | | NZ 594315 A | 31-05-2013 |
| | | PE 20120211 A1 | 24-03-2012 |
| | | PL 2398498 T3 | 29-03-2019 |
| | | PT 2398498 T | 03-12-2018 |
| | | SG 173620 A1 | 29-09-2011 |
| | | SG 2014014708 A | 29-05-2014 |
| | | SG 10201505038T A | 30-07-2015 |
| | | SI 2398498 T1 | 28-02-2019 |
| | | TN 2011000374 A1 | 27-03-2013 |
| | | TW 201031753 A | 01-09-2010 |
| | | UA 107341 C2 | 25-12-2014 |
| | | US 2010254978 A1 | 07-10-2010 |
| | | US 2013330344 A1 | 12-12-2013 |
| | | US 2016355598 A1 | 08-12-2016 |
| | | UY 32451 A | 30-09-2010 |
| | | WO 2010096418 A2 | 26-08-2010 |
| | | ZA 201105500 B | 31-10-2012 |
| WO 2017134292 A1 | 10-08-2017 | NONE | |
| WO 2019229155 A1 | 05-12-2019 | NONE | |
| WO 2019219978 A1 | 21-11-2019 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 9209965 W
- WO 9413804 A


### Non-patent literature cited in the description


- **P. FURRER.** *European Pharmaceutical Review,* 18 April 2013
- **A. MCAULEY et al.** *BioPharm International,* 01 June 2009, vol. 22 (6
- **M. RIOS.** *BioProcess Int.,* 12 May 2015
- **BENSMANA M et al.** *Nucleic Acids Res.,* 1988, vol. 16 (7), 3108
- **WARD ES et al.** *Nature,* 1989, vol. 341, 544-546
- **BIRD RE et al.** *Science,* 1988, vol. 242, 423-426
- **HUSTON JS et al.** *Proc. Natl. Acad. Sci. USA,* 1988, vol. 85, 5879-83
- **TOMLINSON I ; HOLLINGER P.** *Methods Enzymol.,* 2000, vol. 326, 461-79
- **HOLLIGER P et al.** *Proc. Natl. Acad. Sci. USA,* 1993, vol. 90, 6444-48
- **COLOMA MJ ; MORRISON SL.** *Nature Biotechnology,* 1997, vol. 15 (2), 159-163
- Peptide and Protein Drug Delivery. Marcel Dekker, Inc, 1991, 247-301
- **JONES A.** *Adv Drug Delivery Rev,* 1993, vol. 10, 29-90
- **LEDERMANN JA et al.** *Int J Cancer,* 1999, vol. 47, 659-664
- **BAGSHAWE KD.** *Antibody, Immunoconjugates and Radiopharmaceuticals,* 1991, vol. 4, 915-922
- European Pharmacopoeia